# EUROPEAN PATENT SPECIFICATION

(11) **EP 3 227 460 B1**
(45) Date of publication and mention of the grant of the patent: **27.01.2021**
(21) Application number: 15816095.2
(22) Date of filing: 01.12.2015
(51) Int. Cl.: C12Q 1/6886

(54) **NOVEL RNA-BIOMARKER SIGNATURE FOR DIAGNOSIS OF PROSTATE CANCER**
NEUARTIGE RNA-BIOMARKERSIGNATUR ZUR DIAGNOSE VON PROSTATAKREBS
NOUVELLE SIGNATURE DE BIOMARQUEUR RNA POUR LE DIAGNOSTIC DU CANCER DE LA PROSTATE

(30) Priority: 01.12.2014 EP 14195709
(43) Date of publication of application: 11.10.2017
(73) Proprietor: Fraunhofer-Gesellschaft zur Förderung der angewandten Forschung e.V., 80686 München (DE)
(72) Inventor: HORN, Friedemann, 04316 Leipzig (DE); HACKERMÜLLER, Jörg, 04103 Leipzig (DE); CHRIST, Sabina, 04103 Leipzig (DE); REICHE, Kristin, 04416 Markkleeberg (DE); WIRTH, Manfred, 01307 Dresden (DE); FRÖHNER, Michael, 01307 Dresden (DE); FÜSSEL, Susanne, 01307 Dresden (DE)
(74) Representative: CH Kilger Anwaltspartnerschaft mbB
(86) International application number: PCT/EP2015/078183
(87) International publication number: WO 2016/087430

(56) References cited:
- WO-A1-2013/028788
- WO-A2-01/60860
- US-B1- 7 914 988
- SHANCHENG REN ET AL: "RNA-seq analysis of prostate cancer in the Chinese population identifies recurrent gene fusions, cancer-associated long noncoding RNAs and aberrant alternative splicings", CELL RESEARCH, vol. 22, no. 5, 21 February 2012 (2012-02-21), pages 806-821, XP055193081, ISSN: 1001-0602, DOI: 10.1038/cr.2012.30
- JOHN R PRENSNER ET AL: "Transcriptome sequencing across a prostate cancer cohort identifies PCAT-1, an unannotated lincRNA implicated in disease progression", NATURE BIOTECHNOLOGY, vol. 29, no. 8, 31 July 2011 (2011-07-31), pages 742-749, XP055106101, ISSN: 1087-0156, DOI: 10.1038/nbt.1914
- E. DAVID CRAWFORD ET AL: "Diagnostic Performance of PCA3 to Detect Prostate Cancer in Men with Increased Prostate Specific Antigen: A Prospective Study of 1,962 Cases", THE JOURNAL OF UROLOGY, vol. 188, no. 5, 1 November 2012 (2012-11-01), pages 1726-1731, XP055112423, ISSN: 0022-5347, DOI: 10.1016/j.juro.2012.07.023

## Description

### Field of the invention

The present invention is in the field of biology and chemistry. In particular, the invention is in the field of molecular biology. More particular, the invention relates to the analysis of RNA transcripts. Most particularly, the invention is in the field of diagnosing prostate cancer.

### Background

Prostate cancer is the most frequently diagnosed cancer in men. In 2012, the annual number of newly diagnosed prostate cancer cases was reported as approximately 240,000 cases in the United States and approximately 360,000 in the European Union, 68,000 of which in Germany. In the United States, lifetime risks for prostate cancer diagnosis and for dying of prostate cancer are currently estimated at 15.9% and 2.8%, respectively. Despite widespread screening for prostate cancer and major advances in the treatment of metastatic disease, prostate cancer remains the second most common cause of cancer death for men with over 250,000 deaths each year in the Western world.

Currently, testing of prostate-specific antigen (PSA) serum levels and the digital rectal examination represent the two major screening methods. Patients showing abnormal results usually are advised to have a prostate biopsy performed. This has however significant consequences. The lack of specificity of PSA screening which produces high numbers of false positives results in unnecessary prostate biopsies performed annually on millions of men worldwide (overdiagnosis). In addition, taking biopsies carries a substantial risk for infectious complications. Therefore, there is an urgent need for a more sensitive and specific diagnostic assay for early prostate cancer diagnosis to improve prostate cancer screening and to avoid the high numbers of unnecessarily taken prostate biopsies. The present invention addresses this problem by providing a set of biomarkers for the screening and diagnosis of prostate cancer.

Schlegel et al. (WO0160860A2) discloses methods for the diagnosis of prostate cancer assessing whether a patient is afflicted with or has higher than normal risk for developing prostate cancer by detecting the level of expression of a marker in a patient sample.

Russo (WO2013/028788A1) refers to methods for detecting biomarkers in urine microvescicles associated to medical conditions of the prostate gland.

US7914988B1 refers method for preparing a reference model for cancer relapse prediction via analysing expression pattern of signature genes.

### Summary of the invention

Transcripts differentially expressed in tumour and control tissues were identified by Next Generation Sequencing of 64 samples of prostate cancer patients and controls and validated by microarray and qRT-PCR analyses of 256 and 56 samples, respectively. The invention describes RNA biomarkers, which had not so far been found to be suitable for use in the diagnosis of prostate cancer.

The invention relates to a method for the diagnosis of prostate cancer comprising the steps of analysing the expression levels of at least two nucleic acids in a sample of a patient, wherein said at least two nucleic acids are selected from at least two groups of nucleic acids, wherein said groups consist of:
- Group 1:: SEQ ID NO 1
- Group 2:: a splice variant of Ensembl gene ID ENSG00000245750.3 selected from the group consisting of SEQ ID NOs 4, 5, 6, 7, 8, 9 and 10
- Group 3:: a splice variant of Ensembl gene ID ENSG00000255545.3 selected from the group SEQ ID NOs 26, 27, 28 and 29
- Group 4:: SEQ ID NOs 3 and 11
- Group 5:: SEQ ID NO 12
- Group 6:: SEQ ID NO 24
- Group 7:: SEQ ID NO 36
- Group 8:: SEQ ID NO 38
wherein, if the combination of the expression levels of said at least two nucleic acids or the expression level of said nucleic acids from all Groups 1-8 are above a threshold value, the sample is designated as prostate cancer positive.

Herein described is a set of nucleic acids that hybridize under stringent conditions to the nucleic acids in the groups above.

The invention further relates to the use of the method for the diagnosis of prostate cancer.

The invention also relates to the use of a kit for the diagnosis of prostate cancer comprising:
a. at least two primers or probes, which hybridize under stringent conditions to at least two nucleic acids, wherein said at least two nucleic acids are selected from at least two groups of nucleic acids, wherein said groups consist of:
   - Group 1:: SEQ ID NO 1
   - Group 2:: a splice variant of Ensembl gene ID ENSG00000245750.3 selected from the group consisting of SEQ ID NOs 4, 5, 6, 7, 8, 9 and 10
   - Group 3:: a splice variant of Ensembl gene ID ENSG00000255545.3 selected from the group SEQ ID NOs 26, 27, 28 and 29
   - Group 4:: SEQ ID NOs 3 and 11
   - Group 5:: SEQ ID NO 12
   - Group 6:: SEQ ID NO 24
   - Group 7:: SEQ ID NO 36
   - Group 8:: SEQ ID NO 38
b. instructions for combining said at least two primers or probes.

### Definitions

The following definitions are provided for specific terms, which are used in the application text.

As used herein, "nucleic acid(s)" or "nucleic acid molecule" generally refers to any ribonucleic acid or deoxyribonucleic acid, which may be unmodified or modified DNA.

"Nucleic acids" include, without limitation, single- and double-stranded nucleic acids. As used herein, the term "nucleic acid(s)" also includes DNA as described above that contain one or more modified bases. Thus, DNA with backbones modified for stability or for other reasons are "nucleic acids". The term "nucleic acids" as it is used herein encompasses such chemically, enzymatically or metabolically modified forms of nucleic acids, as well as the chemical forms of DNA characteristic of viruses and cells, including for example, simple and complex cells.

The terms "level" or "expression level" in the context of the present invention relate to the level at which a biomarker is present in a sample of a patient. The expression level of a biomarker is generally measured by comparing its expression level to the expression level of one or several housekeeping genes in a sample for normalisation. The sample from the patient is designated as prostate cancer positive if the expression level of the biomarker exceeds the expression level of the same biomarker in an appropriate control (for example a healthy tissue) by a set threshold value.

The term, "analysing a sample for the presence and/or level of nucleic acids" or "specifically estimate levels of nucleic acids", as used herein, relates to the means and methods useful for assessing and quantifying the levels of nucleic acids. One useful method is for instance quantitative reverse transcription PCR. Likewise, the level of RNA can also be analysed for example by northern blot, next generation sequencing or after amplification by using spectrometric techniques that include measuring the absorbance at 260 and 280 nm.

As used herein, the term "amplified", when applied to a nucleic acid sequence, refers to a process whereby one or more copies of a particular nucleic acid sequence is generated from a nucleic acid template sequence, preferably by the method of polymerase chain reaction. Other methods of amplification include, but are not limited to, ligase chain reaction (LCR), polynucleotide-specific based amplification (NSBA), or any other method known in the art.

The term "correlating", as used herein in reference to the use of diagnostic and prognostic marker(s), refers to comparing the presence or amount of the marker(s) in a sample from a patient to its presence or expression level in a sample from a person known to suffer from, or is at risk of suffering from, a given condition. A marker expression level in a patient sample can be compared to a level known to be associated with a specific diagnosis.

As used herein, the term "diagnosis" refers to the identification of the disease, in this case prostate cancer, at any stage of its development, and also includes the determination of predisposition of a subject to develop the disease.

The term "Ensembl gene ID ENSG00000245750.3" relates to a gene ID sequence annotation by Ensembl. Transcripts that belong to the same gene ID may differ in splice events, exons, and can give rise to very different proteins. These are isoforms, arising from alternative splicing. The Ensembl gene ID has several equivalents in other annotation systems such as for example RP11-279F6.1, or locus (hg19) Chr15: 69,755,365-69,863,775 (+). Any equivalent to this Ensembl annotation can be used in its place.

The term "Ensembl gene ID ENSG00000255545.3" relates to a gene ID sequence annotation by Ensembl. Transcripts that belong to the same gene ID may differ in splice events, exons, and can give rise to very different proteins. These are isoforms, arising from alternative splicing. The Ensembl gene ID has several equivalents in other annotation systems such as for example RP11-627G23.1, or locus (hg19) Chr11: 134,306,367-134,375,555 (+). Any equivalent to this Ensembl annotation can be used in its place.

As used herein, the term "fluorescent dye" refers to any chemical that absorbs light energy of a specific wavelength and re-emits light at a different wavelength. Fluorescent dyes suitable for labelling nucleic acids include for example FAM (5-or 6-carboxyfluorescein), VIC, NED, Fluorescein, FITC, IRD-700/800, CY3, CY5, CY3.5, CY5.5, HEX, TET, TAMRA, JOE, ROX, BODIPY TMR, Oregon Green, Rhodamine Green, Rhodamine Red, Texas Red, Yakima Yellow, Alexa Fluor, PET and the like.

As used herein, "isolated" when used in reference to a nucleic acid means that a naturally occurring sequence has been removed from its normal cellular (e.g. chromosomal) environment or is synthesised in a non-natural environment (e.g. artificially synthesised). Thus, an "isolated" sequence may be in a cell-free solution or placed in a different cellular environment.

As used herein, a "kit" is a packaged combination optionally including instructions for use of the combination and/or other reactions and components for such use. If the kit contains nucleic acids, the kit may also comprise synthetic or non-natural variants of said nucleic acids. A synthetic or non-natural nucleic acid is to be understood as a nucleic acid comprising any chemical, biochemical or biological modification, such that the nucleic acid does not appear in nature in this form. Such modifications include, but are not limited to, labelling with a fluorescent dye or a quencher moiety, a biotin tag, as well as modification(s) in the backbone of a nucleic acid, or any other modification that distinguishes the element from its natural counterpart. The same applies also to other natural compounds such as proteins, lipids and the like.

The term "patient" as used herein refers to a living human or non-human organism that is receiving medical care or that should receive medical care due to a disease, or is suspected of having a disease. This includes persons with no defined illness who are being investigated for signs of pathology. Thus the methods and assays described herein are applicable to both, human and veterinary disease.

The term "primer" as used herein, refers to an nucleic acid, whether occurring naturally as in a purified restriction digest or produced synthetically, which is capable of acting as a point of initiation of synthesis when placed under conditions in which synthesis of a primer extension product, which is complementary to a nucleic acid strand, is induced, i.e., in the presence of nucleotides and an inducing agent such as a DNA polymerase and at a suitable temperature and pH. The primer may be either single-stranded or double-stranded and must be sufficiently long to prime the synthesis of the desired extension product in the presence of the inducing agent. The exact length of the primer will depend upon many factors, including temperature, source of primer and the method used. Preferably, primers have a length of from about 15-100 bases, more preferably about 20-50, most preferably about 20-40 bases. The factors involved in determining the appropriate length of primer are readily known to one of ordinary skill in the art. Optionally, the primer can be a synthetic element, in the sense that it comprises a chemical, biochemical or biological modification. Such modifications include, but are not limited to, labelling with a fluorescent dye or a quencher moiety, or a modification in the backbone of a nucleic acid, or any other modification that distinguishes the primer from its natural nucleic acid counterpart.

The term "probe" refers to any element that can be used to specifically detect a biological entity, such as a nucleic acid, a protein or a lipid. Besides the portion of the probe that allows it to specifically bind to the biological entity, the probe also comprises at least one modification that allows its detection in an assay. Such modifications include, but are not limited to labels such as for fluorescent dyes, a specifically introduced radioactive element, or a biotin tag. The probe can also comprise a modification in its structure, such as a locked nucleic acid.

The term "sample" as used herein refers to a sample of bodily fluid or tissue obtained for the purpose of diagnosis, prognosis, or evaluation of a subject of interest, such as a patient. Preferred test samples include blood, serum, plasma, cerebrospinal fluid, urine, saliva, sputum, and pleural effusions. In addition, one of skill in the art would realize that some test samples would be more readily analysed following a fractionation or purification procedure, for example, separation of whole blood into serum or plasma components.

Thus, in a preferred embodiment of the invention the sample is selected from the group comprising a blood sample, a serum sample, a plasma sample, a cerebrospinal fluid sample, a saliva sample and a urine sample or an extract of any of the aforementioned samples as well as circulating tumour cells in blood or lymph, any tissue suspected to contain metastases as well as any source that may contain prostate tumour cells or parts thereof, including vesicles like exosomes, microvesicles, and others as well as free or protein-bound RNA molecules derived from prostate tumour cells. Preferably, the sample is a blood sample, most preferably a serum sample or a plasma sample. Importantly, urine (particularly after digital rectal examination) and ejaculate belong to the most preferable samples. Tissue samples may also be biopsy material or tissue samples obtained during surgery.

The term "area under the curve (AUC)" as used herein describes the area under the curve of a receiver operating characteristic (ROC) or ROC curve. The AUC relates to how specific and sensitive a biomarker is. A perfect marker (AUC=1.0) would yield a point in the upper left corner or coordinate (0,1) of the ROC space, representing 100% sensitivity (no false negatives) and 100% specificity (no false positives).

The term "p-value" relate to the probability of obtaining the observed sample results (or a more extreme result) when the null hypothesis is actually true, *i.e.* there are no differences between means for groups. The smaller the p-value, the higher the likelihood that the alternative hypothesis explains the observed results better than the null hypothesis.

The term "adjusted p-value" refers to p-values which have been adjusted for multiple comparisons (number of genes/probes tested). The method applied is detailed in the experimental section.

### Detailed description of the invention

The invention describes a method of diagnosis of prostate cancer. This method comprises analysing a sample taken from a patient and specifically determine the level of a combination of biomarkers in said patient sample. The result for each biomarker is then correlated to a threshold value and in the case it is above that threshold value, said patient sample is designated as prostate cancer positive.

The invention relates to several groups of sequences comprising SEQ ID NOs 1 to 42. The sequences are listed below. Due to space constraints, only the first 100 nucleotides are listed. The remaining part of the sequence can be found in the sequence protocol.

In particular the invention relates to 8 distinct groups of sequences:
- Group 1:: SEQ ID NO 1
- Group 2:: a splice variant of Ensembl gene ID ENSG00000245750.3 selected from the group consisting of SEQ ID NOs 4, 5, 6, 7, 8, 9 and 10
- Group 3:: a splice variant of Ensembl gene ID ENSG00000255545.3 selected from the group SEQ ID NOs 26, 27, 28 and 29
- Group 4:: SEQ ID NOs 3 and 11
- Group 5:: SEQ ID NO 12
- Group 6:: SEQ ID NO 24
- Group 7:: SEQ ID NO 36
- Group 8:: SEQ ID NO 38

The nucleic acid sequences of SEQ ID NO 3 and 11 correspond to spliced transcripts from the locus (hg19): Chr2: 1,550,437-1,629,191 (-).

The transcripts SEQ ID NO: 36 and 38 are respectively 38 342 nucleotides and 346 832 nucleotides in length. The inventors surprisingly found that these two nucleic acids correspond to single long transcripts. The detection of the expression level of any part of these long transcripts is therefore suited for the method of the present invention.

There are two types of sequences. First, some transcripts are known sequences that are already annotated in relevant databases. They are identified by their respective annotations. Second, new transcripts were identified that are not yet annotated. They are designated here as follows: XLOC_followed by a number. These designations provide information about the genomic origins of the transcripts, but may not necessarily represent the whole sequence of a given transcript. The sequences as detected may in some cases be longer or shorter. In the case of XLOC transcripts, if fragments are detected, these fragments may be as small as 1000, 500, 400, 300, 200, 150, 100, 50, 40, 30, 20, 10, 9, 8, 7, 6 or 5 nucleotides.

**Table 1: List of SEQ ID NOs. SEQ ID NOs 1 to 42 are listed together with the corresponding transcript and gene annotations. The first 100 nucleotides of each SEQ ID NO are shown.**

| SEQ ID | Transcript | Gene/transcript annotation | Sequence |
|---|---|---|---|
| | | Retro-RPL7 | |
| 1 | 1 | Ensemble-ID gene: ENSG00000242899.1 Locus (hg19): Chr3: 131,962,301-131,963,125 (-) | |
| | | Ensemble-ID transcript(s): ENST00000479738.1 | |
| 2 | 2 | XLOC_133897 | |
| | | Ensemble-ID gene: none Locus (hg19): chr20: 45,377,600-45,380,719 (-) | |
| | | Ensemble-ID transcript(s): none | |
| | | Includes GenBank entries: AK128800.1, BC065739.1 | |
| 3 | 3 | AC144450.2 | |
| | | Ensemble-ID gene: ENSG00000203635.2 Locus (hg19): Chr2: 1,624,282-1,629,191 (-) | |
| | | Ensemble-ID transcript(s): ENST00000366424 | |
| | | RP11-27 9F6.1 | |
| 4 | 4 | Ensemble-ID gene: ENSG00000245750.3 Locus (hg19): Chr15: 69,755,365-69,863,775 (+) | |
| | | Ensemble ID trancript: ENST00000558633 | |
| | | | |
| 5 | 4 | ENST00000558309 | |
| | | | |
| 6 | 4 | ENST00000560882 | |
| | | | |
| 7 | 4 | ENST00000559029 | |
| | | | |
| 8 | 4 | ENST00000558781 | |
| | | | |
| 9 | 4 | ENST00000498938 | |
| | | | |
| 10 | 4 | ENST00000559477 | |
| | | AC144450.1 | |
| 11 | 5 | Ensemble-ID gene: ENSG00000228613.1 Locus (hg19): Chr2: 1,550,437-1, 623, 885 (-) | |
| | | Ensemble-ID transcript(s): ENST00000438247.1 | |
| | | AC012531.25 | |
| 12 | 6 | Ensemble-ID gene: ENSG00000260597.1 Locus (hg19): Chr12: 54,413,694-54,416,373 (+) | |
| | | Ensemble-ID transcript(s): ENST00000562848.1 | |
| 13 | | XLOC_068574 | |
| | 7 | Ensemble-ID gene: none | |
| | | Locus (hg19): chr14: 62,653,302-62,655,723 (+) | |
| | | Ensemble-ID transcript(s): none | |
| | | RP1-207H1.3 | |
| 14 | 8 | Ensemble-ID gene: ENSG00000231150.1 Locus (hg19): chr6:38,890,805-38,920,875 (-) | |
| | | Ensemble ID transcript: ENST00000416948.1 | |
| | | | |
| 15 | 8 | ENST00000453417.1 | |
| | | | |
| 16 | 8 | ENST00000418399.1 | |
| | | XLOC_016724 | |
| 17 | 9 | Ensemble-ID gene: none | |
| | | Locus (hg19): chr1: 177,827,793-177,841,757 (-) | |
| 18 | | RP11-314013.1 | |
| | | Ensemble-ID gene: ENSG00000260896 Locus (hg19): Chr16: 80,862,632-80, 926, 492 (-) | |
| | 10 | Ensemble ID transcript: ENST00000562231 | |
| | | | |
| 19 | 10 | ENST00000569356 | |
| | | ENST00000561519 | |
| 20 | 10 | | |
| 21 | 10 | ENST00000563626 | |
| 22 | 11 | XLOC_167596 | |
| | | Ensemble-ID gene: none | |
| | | Locus (hg19): chr4: 67,964,836-67,975,652 (-) | |
| 23 | 12 | XLOC_167595 | |
| | | Ensemble-ID gene: | |
| | | Locus (hg19): chr4: 67,946,236-67,964,614 (-) | |
| 24 | | XLOC_156132 | |
| | 13 | Ensemble-ID gene: none | |
| | | Locus (hg19): chr3: 193,632,725-193,636,178 (-) | |
| 25 | | XLOC_156120 | |
| | 14 | Ensemble-ID gene: none | |
| | | Locus (hg19): chr3: 193,580,748-193,608,459 (-) | |
| | | RP11-627G23.1 | |
| | | Ensemble-ID gene: ENSG00000255545.3 | |
| 26 | 15 | Locus (hg19): Chr11: 134,306,367-134,375,555 (+) | |
| | | Ensemble ID transcript: ENST00000533390 | |
| | | | |
| 27 | 15 | ENST00000531319 | |
| 28 | 15 | ENST00000528482 | |
| 29 | 15 | ENST00000532886 | |
| 30 | | XLOC_047797 | |
| | 16 | Ensemble-ID gene: none | |
| | | Locus (hg19): chr12: 75,378,181-75,383,176 (+) | |
| | | ANKRD34B | |
| 31 | 17 | Ensemble-ID gene: ENSG00000189127.3 Locus (hg19): Chr5: 79,852,574-79,866,307 (-) | |
| | | Ensemble ID transcript: ENST00000338682 | |
| 32 | 17 | ENST00000508916 | |
| | | | |
| 33 | | XLOC_243739 | |
| | 18 | Ensemble-ID gene: none | |
| | | Locus (hgl9): chr9: 79,530,077-79,542,427 (-) | |
| 34 | 19 | XLOC_198292 | |
| | | Ensemble-ID gene: none | |
| | | Locus (hg19): chr6: 148,396,831-148,428,362 (+) | |
| 35 | 20 | XLOC_068639 | |
| | | Ensemble-ID gene: none | |
| | | Locus (hg19): chr14: 62,931,844-62,933,233 (+) | |
| 36 | 21 | XLOC_172083 | |
| | | Ensemble-ID gene: none | |
| | | Locus (hg19): chr4: 169,961,616-169, 999, 957 (-) | |
| 37 | 22 | XLOC_172082 | |
| | | Ensemble-ID gene: none | |
| | | Locus (hgl9): chr4: 169,947,628-169,961,481 (-) | |
| 38 | 23 | XLOC_112832 | |
| | | Ensemble-ID gene: none | |
| | | Locus (hg19): chr2: 123,297,707-123,644,538 (+) | |
| 39 | 24 | XLOC_243747 | |
| | | Ensemble-ID gene: none | |
| | | Locus (hg19): chr9: 79,622,778-79, 633, 361 (-) | |
| 40 | 25 | XLOC_243744 | |
| | | Ensemble-ID gene: none | |
| | | Locus (hg19): chr9: 79,601,892-79,606,132 (-) | |
| 41 | 26 | XLOC_ 126289 | |
| | | Ensemble-ID gene: none | |
| | | Locus (hg19): chr2: 180,988,687-180, 989, 287 (-) | |
| 42 | 27 | XLOC_172084 | |
| | | Ensemble-ID gene: none | |
| | | Locus (hg19): chr4: 169,983,995-169,984,246 (-) | |

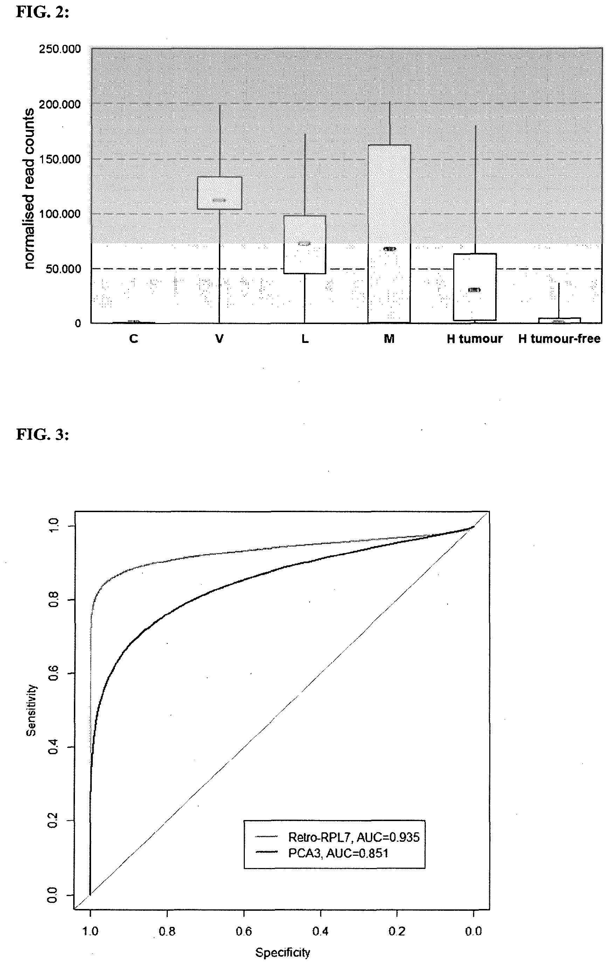

The biomarker PCA3 is routinely used for prostate carcinoma (PCa) diagnosis. As expected therefore, PCA3 expression levels were indicative of PCa in the subjects tested by next generation sequencing by the inventors (FIG. 2). However, it was found that the biomarker had its highest expression level in very low risk tumours (V) and decreased as the risk factor of tumours grew. This finding makes PCA3 an unreliable marker for medium- and high-risk tumours and shows the need for better prostate cancer biomarkers.

Many of the novel biomarkers found by the inventors are significantly better in terms of specificity and sensitivity than PCA3. Retro-RPL7 (SEQ ID NO: 1) for example yielded an area under the ROC curve (AUC) value of 0.935, compared to 0.851 for PCA3 (FIG. 3).

A combination of the biomarkers with SEQ ID NO:1 and SEQ ID NO: 12 yielded an AUC of 0.985 (see table 7).

The following groups of biomarkers have been found to be highly indicative in combination for prostate cancer:
- Group 1:: SEQ ID NO 1
- Group 2:: a splice variant of Ensembl gene ID ENSG00000245750.3 selected from the group consisting of SEQ ID NOs 4, 5, 6, 7, 8, 9 and 10
- Group 3:: a splice variant of Ensembl gene ID ENSG00000255545.3 selected from the group SEQ ID NOs 26, 27, 28 and 29
- Group 4:: SEQ ID NOs 3 and 11
- Group 5:: SEQ ID NO 12
- Group 6:: SEQ ID NO 24
- Group 7:: SEQ ID NO 36
- Group 8:: SEQ ID NO 38

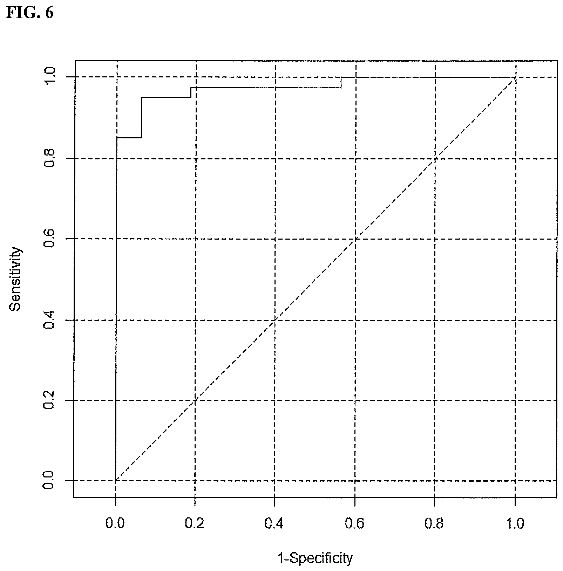

A combination of biomarkers selected from all eight groups results in an AUC of 1.0 (see figure 6).

Hence the invention relates to a method for the diagnosis of prostate cancer comprising the steps of analysing the expression level of at least two nucleic acids in a sample of a patient, wherein said at least two nucleic acids are selected from at least two groups of nucleic acids, wherein said groups consist of:
- Group 1:: SEQ ID NO 1
- Group 2:: a splice variant of Ensembl gene ID ENSG00000245750.3 selected from the group consisting of SEQ ID NOs 4, 5, 6, 7, 8, 9 and 10
- Group 3:: a splice variant of Ensembl gene ID ENSG00000255545.3 selected from the group SEQ ID NOs 26, 27, 28 and 29
- Group 4:: SEQ ID NOs 3 and 11
- Group 5:: SEQ ID NO 12
- Group 6:: SEQ ID NO 24
- Group 7:: SEQ ID NO 36
- Group 8:: SEQ ID NO 38
wherein, if the expression level of said at least two nucleic acids is above a threshold value, the sample is designated as prostate cancer positive.

The invention further relates to a method for the diagnosis of prostate cancer comprising the steps of
a. analysing the expression level of at least two nucleic acids in a sample of a patient, wherein said at least two nucleic acids are selected from at least two groups of nucleic acids, wherein said groups consist of:
   - Group 1:: SEQ ID NO 1
   - Group 2:: a splice variant of Ensembl gene ID ENSG00000245750.3 selected from the group consisting of SEQ ID NOs 4, 5, 6, 7, 8, 9 and 10
   - Group 3:: a splice variant of Ensembl gene ID ENSG00000255545.3 selected from the group SEQ ID NOs 26, 27, 28 and 29
   - Group 4:: SEQ ID NOs 3 and 11
   - Group 5:: SEQ ID NO 12
   - Group 6:: SEQ ID NO 24
   - Group 7:: SEQ ID NO 36
   - Group 8:: SEQ ID NO 38
b. wherein, if the combination of the expression level of -said at least two nucleic acids or the expression level of said nucleic acids from all Groups 1-8 is above a threshold value, the sample is designated as prostate cancer.

Preferred Prostate Cancer Therapeutic Agents comprise: Docetaxel (Taxotere®); Cabazitaxel (Jevtana®); Mitoxantrone (Novantrone®); Estramustine (Emcyt®); Doxorubicin (Adriamycin®); Etoposide (VP-16); Vinblastine (Velban®); Paclitaxel (Taxol®); Carboplatin (Paraplatin®); Abiraterone acetate, Bicalutamide, Casodex, Degarelix, Enzalutamide, Goserelin acetate, Leuprolide acetate, Prednisone, Sipuleucel-T, Radium 223 dichloride and/or Vinorelbine (Navelbine®)

In a preferred embodiment, the invention relates to a method for the diagnosis of prostate cancer comprising the steps of analysing the expression levels of the at least two nucleic acids, wherein at least one of said at least two nucleic acids is selected from group 2. SEQ ID NO 4, 5, 6, 7, 8, 9 and 10 and/or group 1. SEQ ID NO 1.

In another embodiment of the invention the expression level of at least 3 nucleic acids is analysed, wherein said nucleic acids are selected from at least 3 different groups. In an alternative embodiment of the invention the expression level of at least 4 nucleic acids is analysed, wherein said nucleic acids are selected from at least 4 different groups. In an alternative embodiment of the invention the expression level of at least 5 nucleic acids is analysed, wherein said nucleic acids are selected from at least 5 different groups. In an alternative embodiment of the invention the expression level of at least 6 nucleic acids is analysed, wherein said nucleic acids are selected from at least 6 different groups. In an alternative embodiment of the invention the expression level of at least 7 nucleic acids is analysed, wherein said nucleic acids are selected from at least 7 different groups. In the most preferred embodiment the expression level of at least one nucleic acid from each group is analysed.

The sample could be selected from the group comprising prostate tissue, biopsy material, lymph nodes, urine, ejaculate, blood, blood serum, blood plasma, circulating tumour cells in blood or lymph, any tissue suspected to contain metastases as well as any source that may contain prostate tumour cells or parts thereof, including vesicles like exosomes, micro vesicles, and others as well as free or protein-bound RNA molecules derived from prostate tumour cells. More preferably, the sample is urine, and most preferably, the sample is urine obtained from a patient after a digital rectal examination (DRE).

Any suitable method for the quantification of nucleic acids may be used to analyse the expression levels of the nucleic acids. In one embodiment the quantification of nucleic acids is performed using a fluorescence based assay. In a preferred embodiment the analysis of the expression level is performed by measuring the fluorescence of a labelled primer, labelled probe or a fluorescent detection agent.

In another embodiment of the invention the quantification is performed using an assay based on nucleic acid amplification. In a preferred embodiment the quantification is performed using PCR or sequencing methods. In a most preferred embodiment the quantification of nucleic acid is performed using qRT-PCR.

In an alternative embodiment the proteins, which are encoded by the nucleic acids or their reverse complements are analysed and quantified.

The invention further relates to the use of a kit for the diagnosis of prostate cancer comprising at least two primers or probes, which hybridize under stringent conditions to at least two nucleic acids, wherein said at least two nucleic acids are selected from at least two groups of nucleic acids, wherein said groups consist of
- Group 1:: SEQ ID NO 1
- Group 2:: a splice variant of Ensembl gene ID ENSG00000245750.3 selected from the group consisting of SEQ ID NOs 4, 5, 6, 7, 8, 9 and 10
- Group 3:: a splice variant of Ensembl gene ID ENSG00000255545.3 selected from the group SEQ ID NOs 26, 27, 28 and 29
- Group 4:: SEQ ID NOs 3 and 11
- Group 5:: SEQ ID NO 12
- Group 6:: SEQ ID NO 24
- Group 7:: SEQ ID NO 36
- Group 8:: SEQ ID NO 38

The kit may contain more than two nucleic acids. In a preferred embodiment the kit also contains reagents for performing the analysis of the expression level. In a preferred embodiment the kit additionally comprises reagents for nucleic acid amplification and/or nucleic acid quantification and/or nucleic acid detection. In another embodiment the kit comprises control samples.

The experimental results demonstrate high specificity and sensitivity of the novel biomarker combinations for the detection of PCa.

Ideally, the expression levels of transcripts of the nucleic acids of the groups are compared to the expression level of transcript of one or several other genes in the sample, such as of housekeeping genes. Examples of suitable housekeeping genes are shown below in Table 2:

**Table 2:**

| | **Housekeeping name** |
|---|---|
| Housekeeping | GAPDH - Glyceraldehyde 3-phosphate dehydrogenase |
| | HPRT1 - hypoxanthine phosphoribosyltransferase 1 |
| | HMBS - hydroxymethylbilane synthase |
| | TBP Tata box binding protein |

The threshold value is the minimal expression difference between the test sample and the control sample at which the sample is designated as cancer-positive.

Ideally the expression level difference of the biomarker between the test sample and the control sample is 1,5 fold (± 20%), 2 fold (± 20%), 3 fold (± 20%), 4 fold (± 20%) and most preferably 5-fold (± 20%) or more. The p-value (T test) is < 2x10⁻⁵. The FDR is preferably < 5x10-4.

For the selected groups of genes, the threshold value is preferably a 2 fold (± 20%) expression increase between tumour and control sample.

The analysis of RNA biomarker levels can be accomplished by a number of methods, for instance PCR-based methods like quantitative reverse transcription PCR.

The invention relates to a method of amplification to specifically determine the level of biomarkers. Herein, the sample is mixed with forward and reverse primers that are specific for nucleic acids of at least sequences selected from the following groups of sequences:
- Group 1:: SEQ ID NO 1
- Group 2:: a splice variant of Ensembl gene ID ENSG00000245750.3 selected from the group consisting of SEQ ID NO: 4, 5, 6, 7, 8, 9 and 10
- Group 3:: a splice variant of Ensembl gene ID ENSG00000255545.3 selected from the group SEQ ID NO: 26, 27, 28 and 29
- Group 4:: SEQ ID NO 3 and 11
- Group 5:: SEQ ID NO 12
- Group 6:: SEQ ID NO 24
- Group 7:: SEQ ID NO 36
- Group 8:: SEQ ID NO 38
and amplification cycles are performed. Probes or primers are designed such that they hybridize under stringent conditions to said target sequence. The at least two amplification reactions may be performed in parallel or in form of a multiplex amplification.

In one embodiment, the analysis in the method is performed by measuring the fluorescence of a labelled primer, labelled probe or a fluorescent detection agent. More preferably, the analysis of the expression level is performed by qRT-PCR.

In a further embodiment the analysis of the expression level of the at least two nucleic acids is performed using a sequencing method.

The invention also relates to a quantification of the expression levels of the biomarkers included in the combinations. After amplification, quantification is straightforward and can be accomplished by a number of methods. In the case when primers are used wherein at least one primer has a fluorescent dye attached, quantification is possible using the fluorescent signal from the dye. Various primer systems and dyes are available, such as SYBR green, Multiplex probes, TaqMan probes, molecular beacons and Scorpion primers. Other possible means of quantification are for example northern blotting, next generation sequencing or absorbance measurements at 260 and 280 nm.

As will become clear from the examples below, the invention discloses biomarker combinations for prostate cancer, which allow a more accurate and sensitive diagnosis of the disease than current biomarkers.

### EXAMPLES

### Clinical cohort

Prostate carcinoma (PCa) patients who underwent radical prostatectomy (RPE) or surgery to remove a benign prostate hyperplasia (BPH) at the University Hospital of Dresden were included in a retrospective clinical cohort aiming at identifying novel biomarkers for PCa. Approval from the local ethics committee as well as informed consent from the patients were obtained according to the legal regulations. For PCa patients, data on the clinical follow-up for at least five years were collected.

Prostate tissue samples from a cohort of 40 PCa patients and 8 BPH patients were used for identification of diagnostically relevant biomarkers by genome-wide RNA sequencing.. Four PCa groups were defined based on staging according to Gleason (The Veteran's Administration Cooperative Urologic Research Group: histologic grading and clinical staging of prostatic carcinoma; in Tannenbaum, M. Urologic Pathology: The Prostate, Philadelphia: Lea and Febiger. Pp. 171-198) as well as the presence of metastases in the adjacent lymph nodes upon RPE (see Table 3).

**Table 3: PCa cohort for genome-wide RNA sequencing: The control group (C) consists of BPH samples. The very low risk (V) and low risk (L) groups compris samples from patients graded with Gleason Score (GS)<7 and =7, respectively, all without lymph node metastases (pN0). The medium risk (M) group comprise cases with GS<=7 and exhibiting lymph node metastases (pN+); and the high risk (H) group consist of tissues with GS>7. For the latter, pairs of tumour and tumour-free tissue samples obtained from the same patient were analysed.**

| Group | C | V | L | M | H | | | |
|---|---|---|---|---|---|---|---|---|
| Gleason score | BPH | GS<7 | GS=7 | GS<=7 | GS>7 | | | |
| lymph node metastasis | - | pN0 | pN0 | pN+ | pN0 | | pN+ | |
| tissue | control | tumour | tumour | tumour | tumour | tumour-free | tumour | tumour-free |
| number of samples | 8 | 8 | 8 | 8 | 8 | 8 | 8 | 8 |

Selected biomarker candidates were further validated by custom microarrays and quantitative reverse-transcription real-time PCR (qRT-PCR) on cohorts comprising 256 (40 control BPH, 216 tumour samples) and 56 patients (16 control BPH samples, 40 tumour samples), respectively.

### Prostate tissue samples

Prostate tissue samples were obtained from surgery carried out at the Dept. of Urology of the University Hospital of Dresden and stored in liquid nitrogen at the Comprehensive Cancer Centre of Dresden University. Prostate tissue samples obtained from radical prostatectomies (RPEs) of prostate carcinoma (PCa) patients were divided into tumour and tumour-free samples. Prostate tissue samples from patients with benign prostate hyperplasia (BPH) were used as non-tumour controls. Patient consent was always given.

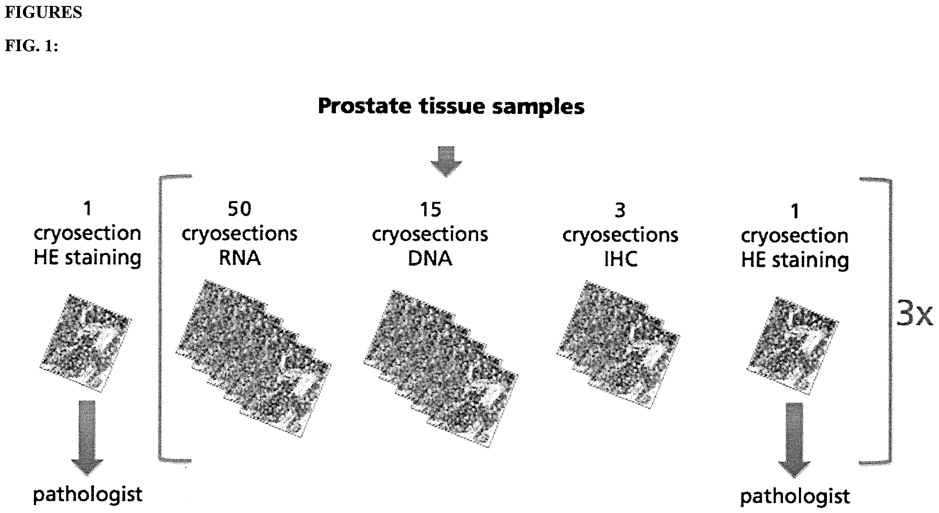

To verify the status of the samples and their tumour cell content, all samples were divided into series of cryosections. To this end, frozen tissue samples were embedded in Tissue-Tek OCT-compound (Sakura Finetek GmbH) and fixed on metal indenters by freezing. Cryosections were prepared using a cryomicrotome (Leica) equipped with a microtome blade C35 (FEATHER) cooled to -28°C. Every sample was cut into a total of 208 cryosections, 4 of which were HE-stained and evaluated by a pathologist with respect to their tumour cell content (FIG. 1). This yielded 3 stacks of consecutive cryosections, each of which was flanked by HE-stained sections. Only stacks that were flanked on either side by sections containing at least 60% or at most 5% tumour cells were used as tumour or tumour-free samples, respectively. 50 cryosections of the stacks chosen were then subjected to RNA preparation.

### RNA isolation

Total RNA was isolated from cryo-preserved tissue using Qiazol and the miRNeasy Mini Kit on the QIAcube (all from Qiagen) with manual subsequent DNase I digestion. RNA concentration was determined using a Nanodrop 1000 (Peqlab). RNA integrity was verified on an Agilent Bioanalyzer 2100 (Agilent Technologies, Palo Alto, CA), and only RNA samples with an RNA-Integrity-Number (RIN) of at least 6 were further processed.

### Genome-wide long-RNA next generation sequencing

Genome-wide long RNA sequencing was performed using a subset of the retrospective PCa cohort comprising 8 prostate tissue samples from benign prostate hyperplasia (BPH) as a control and 56 tumour tissue samples (including tumour and tumour free tissue pairs from samples with Gleason score >7). 1 µg of total RNA was depleted of ribosomal RNA using the Ribo-Zero rRNA Removal Kit (Epicentre). Sequencing libraries were prepared from 50 ng of rRNA-depleted RNA using ScriptSeq v2 RNA-Seq Library Preparation Kit (Epicentre). The di-tagged cDNA was purified using the Agencourt AMPure XP System Kit (Beckman Coulter). PCR was carried out through 10 cycles to incorporate index barcodes for sample multiplexing and amplify the cDNA libraries. The quality and concentration of the amplified libraries were determined using a DNA High Sensitivity Kit on an Agilent Bioanalyzer (Agilent Technologies). 4 ng each of 8 samples were pooled and size-selected on 2% agarose gels using agarose gel electrophoresis. The sample range between 150 bp and 600 bp was gel-excised and purified with the MinElute Gel Extraction Kit (Qiagen), according to manufacturer's instructions. The purified libraries were quantified on an Agilent Bioanalyzer using a DNA High Sensitivity Chip (Agilent Technologies). Every purified and size-selected library pool was then loaded onto an Illumina HiSeq2000 flow cell, distributing it among all lanes. Cluster generation was performed using TruSeq PE Cluster Kits v3 (Illumina Inc.) in an Illumina cBOT instrument following the manufacturer's protocol. Sequencing was performed on an Illumina HiSeq2000 sequencing machine (Illumina, Inc.). The details of the sequencing runs were as follows: paired-end sequencing strategy; 101 cycles for Read1, 7 cycles for index sequences, and 101 cycles for Read2.

### Analysis of sequencing data: Raw data preparation

Raw sequencing data comprising base call files (BCL files) was processed with CASAVA v1.8.1 (Illumina) resulting in FASTQ files. FASTQ files contain for each clinical sample all sequenced RNA fragments, in the following referred to as "reads". Specific adapter sequences were removed by using cutadapt (http://code.google.com/p/cutadapt/).

### Analysis of sequencing data: Genome mapping and transcript assembling:

Reads were mapped to the human genome (assembly hg19) using segemehl v0.1.4-382 and TopHat v2.0.9. Novel transcripts, i.e. transcripts not annotated in Gencode v17, were assembled using Cufflinks v2.1.1 and Cuffmerge v2.1.1. All novel transcripts and all known Gencode v17 transcripts were combined into a comprehensive annotation set.

### Analysis of sequencing data: Statistical analysis

Htseq-count v0.5.4pl (http://www-huber.embl.de/users/anders/HTSeq/doc/count.html) was used to compute the read counts per transcript and gene that are contained in the comprehensive annotation set of novel and known transcripts. Differentially expressed transcripts and genes were identified using R and the Bioconductor libraries edgeR. Different RNA composition of the clinical samples was adjusted for by scaling library size for each sample (TMM method). A negative binomial log-linear model was fitted to the read counts for each transcript or gene, and coefficients distinct from zero identified by a likelihood ratio test. False discovery rate was controlled by Benjanimi-Hochberg adjustment.

### Validation by custom microarrays

Based on the sequencing results custom microarrays with 180 000 probes (Agilent SurePrint G3 Custom Exon Array, 4x180K, Design-ID 058029) were designed comprising mRNAs, long non coding RNAs (gencode v15), new transcripts and all differential expressed transcripts.

The microarray screening was performed using the retrospective PCa cohort comprising 40 prostate tissue samples from benign prostate hyperplasia as a control as a control, as well as 164 and 52 tumour and tumour-free tissue samples, respectively, of PCa patients after radical prostatectomy. Using the Quick Amp Labeling Kit (Agilent) cRNA was synthesized from 200 ng total RNA, and 1650 ng cRNA was hybridized on the arrays (Agilent Gene Expression Hybridization Kit).

### Validation by quantitative real-time PCR

For validation of the results obtained by next generation sequencing and microarray screening a total of 56 tissue samples (16 tumour free and 40 tumor samples) were screened using quantitative real-time PCR.

cDNA was synthesized from 100 ng total RNA using the High-Capacity Reverse transcription kit (Applied Biosystems) and random primers according to manufacturer's instructions. Subsequent PCR assays were run using 4 µl of the diluted cDNA. Quantitative real-time PCR was performed using custom- and pre-designed TaqMan Gene Expression Assays (Applied Biosystems) for housekeeping and target transcripts on an Applied Biosystems 7900HT Real-Time PCR System.
All samples were measured in triplicate and the means of these measurements were used for further calculations.

**Table 4: IDs of the Applied Biosystems TaqMan Gene Expression Assays used for qRT-PCR validation in prostate tissue samples.**

| | **Housekeeping/Target name** | **TaqMan Assay ID** |
|---|---|---|
| Housekeeping | GAPDH | Hs02758991_g1 |
| | HPRT1 | Hs02800695_m1 |
| | HMBS | Hs00609293_g1 |
| Target | SEQ ID NO 1 | AJ70L28 |
| | SEQ ID NO 9 | Hs01388451_m1 |
| | SEQ ID NO 3 | AJCSVRJ |
| | PCA3 | Hs01371939_g1 |

### Statistical analysis of the RT-qPCR results

Data normalization was carried out against the unregulated housekeeping genes GAPDH (SEQ ID NO 1) and HPRT1 (SEQ ID NO 3 and SEQ ID NO 9), respectively. For relative quantification, changes in gene expression of each sample were analysed relative to the median expression of the control samples. All statistical analyses were carried out using R statistical software.

The log2-transformed relative expression levels of the biomarkers were compared between tumor and control samples employing Student's t-test. Receiver-operating characteristic (ROC) curves, representing a measure of diagnostic power of each marker by the area under the curve (AUC), were calculated using the package pROC.

All samples were measured in triplicate and the means of these measurements were used for further calculations.

### Validation in DRE urine samples

### DRE urine sample collection and RNA isolation

Urine samples were collected after digital rectal examination (DRE) of the prostate (DRE urine). This routinely performed examination method allows getting urine samples including a certain amount of prostate cells. The DRE urine samples were centrifuged and washed two times using PBS. The resulting cell pellet was resuspended in 700µl Qiazol. Total RNA was isolated using the miRNeasy Mini Kit on the QIAcube (all from Qiagen) with manual subsequent DNase I digestion. RNA concentration was determined using a Nanodrop 1000 (Peqlab). RNA integrity was verified on an Agilent Bioanalyzer 2100 (Agilent Technologies, Palo Alto, CA).

### Quantitative real-time PCR screening of DRE urine samples

cDNA was synthesized from 2x50 ng total RNA using the Superscript III Reverse transcriptase (Applied Biosystems) and random primers according to manufacturer's instructions. Subsequent PCR assays were run using 4 µl of cDNA. Quantitative real-time PCR was performed using custom and pre-designed TaqMan Gene Expression Assays (Applied Biosystems) for housekeeping (PSA) and target transcripts on an Applied Biosystems 7900HT Real-Time PCR System.
All samples were measured in duplicate and the means of these measurements were used for further calculations.

### Genome-wide long-RNA next generation sequencing of DRE urine samples

For genome-wide long RNA sequencing total RNA from 7 DRE urine samples was precipitated using ethanol to concentrate the RNA amount and resuspended in 10µl RNase free water. The rRNA removal was performed with 4ng of total RNA using the Low input Ribo-Zero rRNA Removal Kit (Epicentre modified by clontech), resulting in 10µl rRNA depleted RNA. Sequencing libraries were prepared from 8µl rRNA-depleted RNA using the SMARTER stranded RNAseq Kit (Clontech). The di-tagged cDNA was purified using the Agencourt AMPure XP System Kit (Beckman Coulter). PCR was carried out through 18 cycles to incorporate index barcodes for sample multiplexing and amplify the cDNA libraries. The quality and concentration of the amplified libraries were determined using a DNA High Sensitivity Kit on an Agilent Bioanalyzer (Agilent Technologies). Samples were pooled and cluster generation was performed using 15pmol/l of the pooled library and the TruSeq PE Cluster Kit v4 (Illumina Inc.) in an Illumina cBOT instrument following the manufacturer's protocol. Sequencing was performed using the HiSeq SBS v4 sequencing reagents (250 cycles) on an Illumina HiSeq2500 sequencing machine (Illumina, Inc.). The details of the sequencing run were as follows: paired-end sequencing strategy; 126 cycles for Read1, 7 cycles for index sequences, and 126 cycles for Read2.

### Statistical analysis of the qRT-PCR results from DRE urines

For analysis of qRT-PCR results from DRE urine samples data normalization was carried out against the prostate specific antigen (PSA). For relative quantification, changes in gene expression of each sample were analysed relative to the median expression of the control samples. All statistical analyses were carried out using R statistical software.

**Table 5: IDs of the Applied Biosystems TaqMan Gene Expression Assays used for qRT-PCR validation.**

| | **Name** | **TaqMan Assay ID** |
|---|---|---|
| Housekeeping | GAPDH | Hs02758991_g1 |
| | HPRT1 | Hs02800695_m1 |
| Target | SeqID 1 | AJ70L28 |
| | PSA | Hs02576345_m1 |

### Results

The transcriptomes of 40 PCa tumour samples and 16 tumour-free samples obtained upon RPE and 8 BPH prostate tissue samples as benign, non-tumour controls were analysed using strand-specific, paired-end long RNA next generation sequencing (NGS). Approximately 150 cryosections per sample in at least three segments were prepared, aiming at an optimal data quality and robustness of the analysis. Upon pathological evaluation, only segments satisfying a maximal and minimal tumour cell count of 60% and 5% in tumour and tumour free samples, respectively, were retained for further analysis. The transcriptome sequencing (RNAseq) approach aimed at a comprehensive identification and quantification of RNAs expressed in normal or cancer prostate tissue. All classes of coding and long non-coding transcripts independent of polyadenylation status were sequenced. Large input masses of RNA were used to ensure high library complexity. Furthermore, on average 200 M paired-end reads 2 x 100 nt per library were sequenced, enabling the assembly of novel lowly expressed transcripts due to high coverage. This approach outperformed most comparable published studies that analyzed larger numbers of samples. In total, approx. 3000 novel transcripts that did not show an exonic overlap with transcripts annotated in Gencode v17 were assembled. At a false discovery rate of 0.01, 6442 differentially expressed genes across all contrasts were observed. Numbers of differentially expressed genes for specific contrasts are given in Table 4.

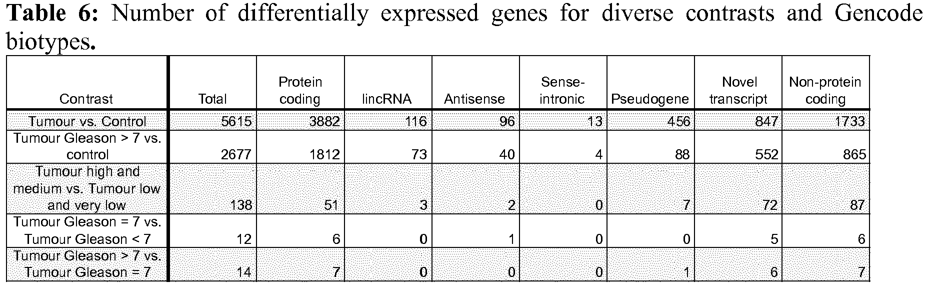

The results successfully reproduced the majority of transcripts previously reported to be differentially expressed between prostate tumour and normal tissue. In addition, a number of novel PCa-associated transcripts were identified, which can be used to develop assays for the diagnosis of PCa. Selected most promising transcripts were validated in a test cohort of PCA tumour and BPH control samples by qRT-PCR.

Several of these novel biomarker candidates significantly surpass the specificity and sensitivity of the biomarker PCA3, which is already used for PCa diagnosis. In the sequencing cohort, PCA3 proved to be clearly associated with PCa, yet with a strong tendency to a decline in the high-risk group (FIG. 2).

In the test cohort, the novel biomarker Retro-RPL7 (SEQ ID NO 1) yielded an area under ROC curve (AUC) value of 0.935, compared to 0.851 for PCA3 (FIG. 3). A combination of our novel biomarkers Retro-RPL7 and XLOC133897 showed an AUC of 0.975 (FIG. 6).

The experimental results demonstrate high specificity and sensitivity of the novel biomarkers for the detection of PCa. Therefore, assays can be set up based on the measurement of these newly discovered biomarkers alone or in combination (or in combination with already known markers) in all sources that may contain prostate tumour cells or parts thereof (including vesicles like exosomes, microvesicles, and others as well as free or protein-bound RNA molecules deriving from prostate tumour cells) to be used for the diagnosis of PCa. These sources include (but are not limited to) prostate tissue, biopsy material, lymph nodes, urine, ejaculate, blood, blood serum, blood plasma, circulating tumour cells in blood or lymph, as well as any tissue suspected to contain PCa metastases. Measurement of our RNA biomarkers can be done by any method suited to specifically estimate RNA levels, e.g. PCR-based methods like qRT-PCR. The assays can be applied for early diagnosis (screening) of PCa, for predicting the aggressiveness of the tumours (prognosis), and/or for aiding the choice of therapy.

Diagnostic assays based on these biomarkers may therefore dramatically decrease the high false-positive rates of current assays and thereby help to avoid unnecessary invasive prostate biopsies.

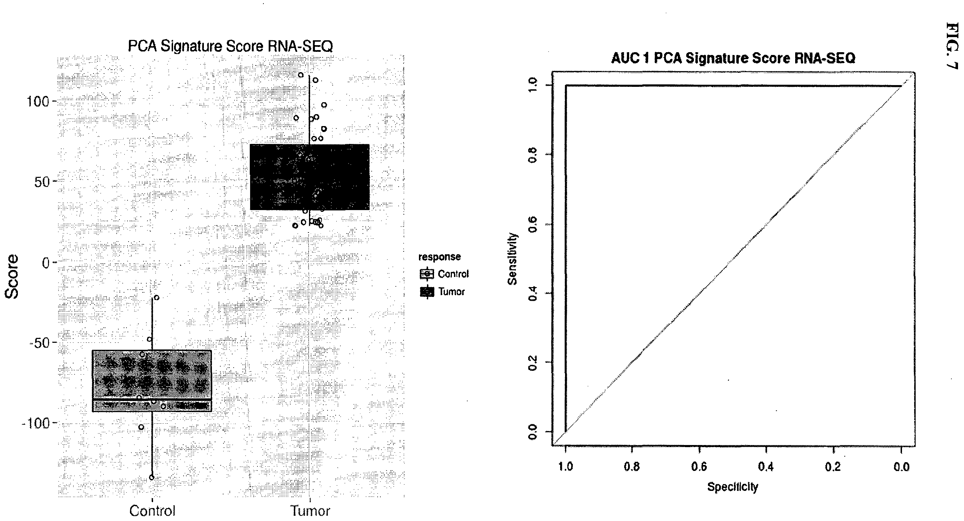

Combinations of biomarkers selected from the groups consisting of:
- Group 1:: SEQ ID NO 1
- Group 2:: a splice variant of Ensembl gene ID ENSG00000245750.3 selected from the group consisting of SEQ ID NO: 4, 5, 6, 7, 8, 9 and 10
- Group 3:: a splice variant of Ensembl gene ID ENSG00000255545.3 selected from the group SEQ ID NO: 26, 27, 28 and 29
- Group 4:: SEQ ID NO 3 and 11
- Group 5:: SEQ ID NO 12
- Group 6:: SEQ ID NO 24
- Group 7:: SEQ ID NO 36
- Group 8:: SEQ ID NO 38
have also been analysed for their diagnostic value in combination. The inventors found that the diagnostic value and the AUC greatly increased, when a combination of said groups was analyzed as shown in the following tables and Figure 7. This indicates that not only are the single novel biomarkers superior to previously known biomarkers, but that their combination surprisingly allows further substantial improvement of the PCa diagnosis.

**Table 7: AUC values of marker combinations involving sequences from group 5, compared to the AUC value of group 5 alone. Data derive from custom microarray analysis of 256 prostate tissue samples.**

| **Seq A** | | **Seq B** | **AUC value** |
|---|---|---|---|
| | | | |
| SeqID 12 | | | 0.817 |
| | | | |
| SeqID 12 | + | SeqID 1 | 0.985 |
| SeqID 12 | + | SeqID 3 | 0.959 |
| SeqID 12 | + | SeqID 11 | 0.96 |
| SeqID 12 | + | SeqID 4_10 | 0.989 |
| SeqID 12 | + | SeqID 38 | 0.896 |
| SeqID 12 | + | SeqID 36 | 0.944 |

**Table 8: AUC values of marker combinations involving sequences from group 2, compared to the AUC value of group 2 alone. Data derive from custom microarray analysis of 256 prostate tissue samples**

| **Seq A** | | **Seq B** | **AUC array** |
|---|---|---|---|
| | | | |
| SeqID 4_10 | | | 0.959 |
| | | | |
| SeqID 4_10 | + | SeqID 26_29 | 0.981 |
| SeqID 4_10 | + | SeqID 12 | 0.989 |
| SeqID 4_10 | + | SeqID 38 | 0.98 |
| SeqID 4_10 | + | SeqID 36 | 0.988 |

**Table 9: AUC values of marker combinations involving sequences from group 3, compared to the AUC value of group 3 alone. Data derive from custom microarray analysis of 256 prostate tissue samples.**

| **Seq A** | | **Seq B** | **AUC array** |
|---|---|---|---|
| | | | |
| SeqID 26_29 | | | 0.849 |
| | | | |
| SeqID 26_29 | + | SeqID 12 | 0.926 |
| SeqID 26_29 | + | SeqID 38 | 0.915 |
| SeqID 26_29 | + | SeqID 36 | 0.954 |
| SeqID 26_29 | + | SeqID 4_10 | 0.981 |
| SeqID 26_29 | + | SeqID 1 | 0.985 |
| SeqID 26_29 | + | SeqID 3 | 0.962 |
| SeqID 26_29 | + | SeqID 11 | 0.963 |

**Table 10: AUC values of marker combinations involving sequences from group 4, compared to the AUC value of group 4 alone. Data derive from custom microarray analysis of 256 prostate tissue samples**

| **Seq A** | | **Seq B** | **AUC array** |
|---|---|---|---|
| | | | |
| SeqID 3 | | | 0.935 |
| | | | |
| SeqID 3 | + | SeqID 1 | 0.984 |
| SeqID 3 | + | SeqID 4_10 | 0.981 |
| SeqID 3 | + | SeqID 11 | 0.946 |
| SeqID 3 | + | SeqID 12 | 0.959 |
| SeqID 3 | + | SeqID 26_29 | 0.962 |
| SeqID 3 | + | SeqID 36 | 0.974 |
| SeqID 3 | + | SeqID 38 | 0.958 |

**Table 11: AUC values of marker combinations involving sequences from group 1, compared to the AUC value of group 1 alone. Data derive from custom microarray analysis of 256 prostate tissue samples**

| **Seq A** | | **Seq B** | **AUC array** |
|---|---|---|---|
| | | | |
| SeqID 1 | | | 0.945 |
| | | | |
| SeqID 1 | + | SeqID 3 | 0.984 |
| SeqID 1 | + | SeqID 4_10 | 0.986 |
| SeqID 1 | + | SeqID 11 | 0.983 |
| SeqID 1 | + | SeqID 12 | 0.985 |
| SeqID 1 | + | SeqID 26_29 | 0.985 |
| SeqID 1 | + | SeqID 36 | 0.986 |
| SeqID 1 | + | SeqID 38 | 0.983 |

**Table 12: AUC values of marker combinations involving sequences from group 6, compared to the AUC value of group 6 alone. Data derive from RNA next-generation sequencing analysis of 64 prostate tissue samples.**

| **Seq A** | | **Seq B** | **AUC sequencing** |
|---|---|---|---|
| | | | |
| SeqID 24 | | | 0.919 |
| | | | |
| SeqID 24 | + | SeqID 1 | 1 |
| SeqID 24 | + | SeqID 3 | 0.984 |
| SeqID 24 | + | SeqID 11 | 0.975 |
| SeqID 24 | + | SeqID 12 | 1 |
| SeqID 24 | + | SeqID 4_10 | 0.988 |
| SeqID 24 | + | SeqID 26_29 | 0.962 |
| SeqID 24 | + | SeqID 36 | 0.959 |
| SeqID 24 | + | SeqID 38 | 0.966 |

**Table 13: AUC values of marker combinations involving sequences from group 7 and 8, compared to the AUC values of group 7 or 8 alone. Data derive from custom microarray analysis of 256 prostate tissue samples.**

| **Seq A** | | **Seq B** | **AUC value** |
|---|---|---|---|
| | | | |
| SeqID 38 | | | 0.806 |
| | | SeqID 36 | 0.88 |
| SeqID 38 | + | SeqID 36 | 0.958 |

The inventors also found that the expression of all groups could be detected in urine sample of patients, while being absent or low expressed in healthy patients (Figure 8). This is surprising because Fontenete et al., (Int. braz j urol. vol.37 no.6 Rio de Janeiro Nov./Dec. 2011) showed that the mRNA of PSA is not a suitable biomarker for prostate cancer in urine samples, as it was found to be overexpressed more frequently in healthy patients than in PCa patients in these samples. Therefore, it was not *a priori* evident that analysing the biomarker expression levels in urine samples could be used to reliably diagnose prostate cancer.

The advantages of a diagnostic assays based on these biomarker combinations allows a dramatically lower false-positive rate compared to current assays and measuring their expression levels in urine sample avoid having to perform unnecessary invasive prostate biopsies.

### Figure captions

Fig.1 : Verification of tissue sample quality: to determine the tumour cell content of the tissue samples, cryosections were prepared from the frozen samples as shown. HE: hematoxylin/eosin; IHC: immunohistochemistry. Verification of tissue sample quality: cryosections of 4µm were prepared from the frozen samples as shown for HE staining (to ensure tumour cell content of the tissue samples), for RNA and DNA isolation and for IHC. HE: hematoxylin/eosin; IHC: immunohistochemistry.
Fig.2: Box-blot of RNA-seq data for transcript PCA3. Results from RNA sequencing of the retrospective PCa cohort comprising 8 prostate tissue samples from benign prostate hyperplasia as a control (C), 8 PCa tumour samples each of groups V (very low risk; Gleason <7, pN0), L (low risk; Gleason score =7, pN0), and M (medium risk; Gleason score <=7, pN+), as well as 16 pairs of tumour and tumour-free tissue samples from group H (high risk; Gleason score >7).
Fig.3: ROC curves of Retro-RPL7 (SEQ ID NO 1) and PCA3 resulting from qRT-PCR analysis of 56 prostate tissue samples.

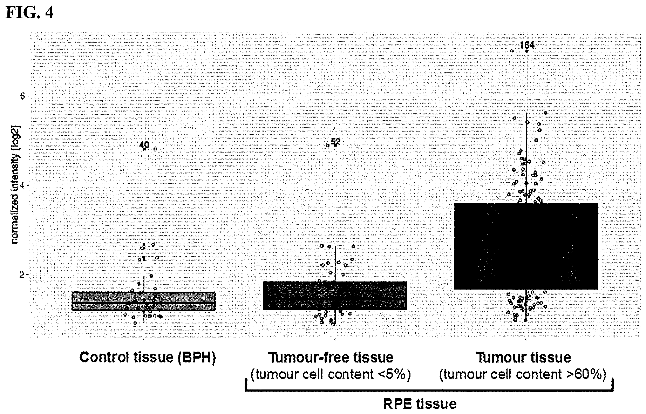

Fig.4: Box-blot of custom microarray data for SEQ ID NO 12. Results from custom microarray analysis of the retrospective PCa cohort comprising 40 prostate tissue samples from benign prostate hyperplasia (BPH) as a control, as well as 164 and 52 tumour and tumour-free tissue samples, respectively, of PCa patients after radical prostatectomy (RPE).

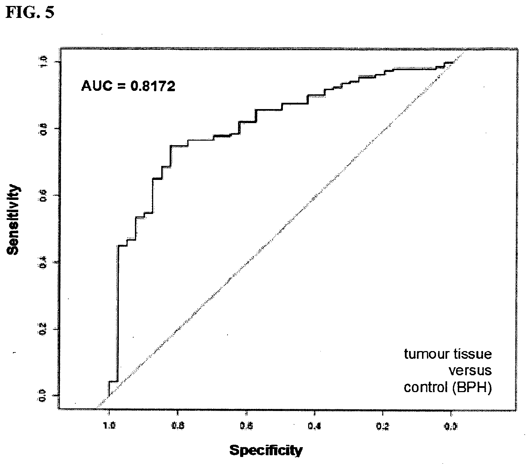

Fig.5: ROC curve of SEQ ID NO 12 resulting from custom microarray analysis of 256 prostate tissue samples as described in FIG.4.
Fig.6: Logit model based on a two-transcript signature. ROC curve is shown for the multivariate logit model comprising Retro-RPL7 (SEQ ID NO 1) and XLOC133897 (SEQ ID NO 2) (AUC=0.975).
Fig. 7: Biomarker signature: A) Data obtained by RNA next-generation sequencing from 8 control tissue samples (benign prostate hyperplasia, BPH) and 40 prostate carcinoma tissue samples for all transcripts of SEQ ID NOs 1, 3, 4-10, 11, 12, 24, 26-29, 36, and 38 were combined as a signature to yield higher specificity and sensitivity. Data are shown by box plot (left) and ROC curve (right). The resulting AUC value is 1.0. B) Data obtained by custom microarray analysis from 40 control tissue samples (benign prostate hyperplasia, BPH) and 164 prostate carcinoma tissue samples for all transcripts of SEQ ID NOs 1,3,4-10, 11, 12, 24, 26-29, and 38 were combined as a signature to yield higher specificity and sensitivity. Data are shown by box plot (left) and ROC curve (right). The resulting AUC value is 1.0.
Fig. 8: Biomarker detection in urine samples by RNA next-generation sequencing: Urine samples were obtained after digital rectal examination of patients by an urologist. RNA isolated from these samples was subjected to transcriptome-wide RNA sequencing using an Illumina HiSeq2500 next-generation sequencer. Reads were mapped to the genome by standard algorithms, and reads mapping to the genomic loci of the SEQ ID NOs shown were counted and normalized to reads derived from the gene locus of prostate-specific antigen (PSA) as a measure for the presence of prostate epithelium cells in the urine. Samples of patients diagnosed with and without prostate cancer were compared.

## Claims

1. A method for the diagnosis of prostate cancer, comprising the steps of
a) analysing the expression levels of at least two nucleic acids in a sample of a patient, wherein said at least two nucleic acids are selected from at least two groups of nucleic acids, wherein said groups consist of:
Group 1: SEQ ID NO 1
Group 2: a splice variant of Ensembl gene ID ENSG00000245750.3 selected from the group consisting of SEQ ID NO: 4, 5, 6, 7, 8, 9 and 10
Group 3: a splice variant of Ensembl gene ID ENSG00000255545.3 selected from the group SEQ ID NO: 26, 27, 28 and 29
Group 4: SEQ ID NO 3 and 11
Group 5: SEQ ID NO 12
Group 6: SEQ ID NO 24
Group 7: SEQ ID NO 36
Group 8: SEQ ID NO 38
b) wherein, if the combination of the expression level of said at least two nucleic acids or the expression level of said nucleic acids from all Groups 1-8 is above a defined threshold value, the sample is designated as prostate cancer positive.

2. The method according to claim 1, wherein at least one of said at least two nucleic acids is selected from group 1 SEQ ID NO 1 and/or group 2 SEQ ID NO 4, 5, 6, 7, 8, 9 and 10.

3. The method according to claim 1 or 2, wherein the expression level of at least 5 nucleic acids are analysed, wherein said nucleic acids are selected from at least 5 different groups.

4. The method according to claim 1, wherein the expression level of at least one nucleic acid from each of the eight groups is analyzed.

5. The method according to any of claims 1 to 4, wherein the sample is selected from the group comprising prostate tissue, biopsy material, lymph nodes, urine, ejaculate, blood, blood serum, blood plasma, circulating tumour cells in blood or lymph, any tissue suspected to contain metastases as well as any source that may contain prostate tumour cells or parts thereof, including vesicles like exosomes, micro vesicles, and others as well as free or protein-bound RNA molecules derived from prostate tumour cells.

6. The method according to any of claims 1 to 4, wherein the sample is a urine sample.

7. The method according to any of the preceding claims, wherein the analysis of the expression level is performed by measuring the fluorescence of a labelled primer, labelled probe or a fluorescent detection agent.

8. The method according to any of the preceding claims, wherein the analysis of the expression level is performed by qRT-PCR.

9. Use of a kit in the method according to any one of claims 1 to 8 comprising
a. at least two primers or probes, which hybridize under stringent conditions to at least two nucleic acids, wherein said at least two nucleic acids are selected from at least two groups of nucleic acids, wherein said groups consist of:
Group 1: SEQ ID NO 1
Group 2: a splice variant of Ensembl gene ID ENSG00000245750.3 selected from the group consisting of SEQ ID NO: 4, 5, 6, 7, 8, 9 and 10
Group 3: a splice variant of Ensembl gene ID ENSG00000255545.3 selected from the group SEQ ID NO: 26, 27, 28 and 29
Group 4: SEQ ID NO 3 and 11
Group 5: SEQ ID NO 12
Group 6: SEQ ID NO 24
Group 7: SEQ ID NO 36
Group 8: SEQ ID NO 38
b. instructions for combining said at least two primers or probes.

10. The kit for the use according to claim 9 comprising at least 8 primers or probes, wherein said at least 8 primers or probes hybridize under stringent conditions to at least one nucleic acid of each of the eight groups.

11. Prostate Cancer Therapeutic Agents for use in the treatment of prostate cancer, wherein said prostate cancer is diagnosed according to the method of claim 1.

12. The Prostate Cancer Therapeutic Agents for the use according to claim 11, wherein the Prostate Cancer Therapeutic Agents comprises: Docetaxel (Taxotere®); Cabazitaxel (Jevtana®); Mitoxantrone (Novantrone®); Estramustine (Emcyt®); Doxorubicin (Adriamycin®); Etoposide (VP-16); Vinblastine (Velban®); Paclitaxel (Taxol®); Carboplatin (Paraplatin®); Abiraterone acetate, Bicalutamide, Casodex, Degarelix, Enzalutamide, Goserelin acetate, Leuprolide acetate, Prednisone, Sipuleucel-T, Radium 223 dichloride and/or Vinorelbine (Navelbine®).

## Patentansprüche

1. Ein Verfahren zur Diagnose von Prostatakrebs, umfassend die Schritte:
a) Analysieren der Expressionsniveaus von mindestens zwei Nukleinsäuren in einer Probe eines Patienten, wobei die mindestens zwei Nukleinsäuren aus mindestens zwei Gruppen von Nukleinsäuren ausgewählt sind, wobei die Gruppen bestehen aus:
Gruppe 1: SEQ ID NO 1
Gruppe 2: eine Splicevariante der Ensemble Gene ID ENSG00000245750.3 ausgewählt aus der Gruppe umfassend SEQ ID NO: 4, 5, 6, 7, 8, 9 und 10
Gruppe 3: eine Splicevariante der Ensemble Gene ID ENSG00000255545.3 ausgewählt aus der Gruppe umfassend SEQ ID NO: 26, 27, 28 und 29
Gruppe 4: SEQ ID NO 3 und 11
Gruppe 5: SEQ ID NO 12
Gruppe 6: SEQ ID NO 24
Gruppe 7: SEQ ID NO 36
Gruppe 8: SEQ ID NO 38
b) wobei, wenn die Kombination des Expressionsniveaus der mindestens zwei Nukleinsäuren oder das Expressionsniveau der Nukleinsäuren aus allen Gruppen 1 bis 8 über einem definierten Schwellenwert liegt, wird die Probe als Prostatakrebspositiv bezeichnet.

2. Das Verfahren gemäß Anspruch 1, wobei mindestens eine der mindestens zwei Nukleinsäuren aus der Gruppe 1 SEQ ID NO 1 und/oder Gruppe 2 SEQ ID NO 4, 5, 6, 7, 8, 9 und 10 ausgewählt ist.

3. Das Verfahren gemäß Anspruch 1, wobei das Expressionsniveau von mindestens 5 Nukleinsäuren analysiert wird, wobei die Nukleinsäuren aus mindestes 5 verschiedenen Gruppen ausgewählt sind.

4. Das Verfahren gemäß Anspruch 1, wobei das Expressionsniveau mindestens einer Nukleinsäure aus jeder der acht Gruppen analysiert wird.

5. Das Verfahren gemäß einer der Ansprüche 1 bis 4, wobei die Probe ausgewählt ist aus der Gruppe umfassend Prostatagewebe, Biopsiematerial, Lymphknoten, Urin, Ejakulat, Blut, Blutserum, Blutplasma, zirkulierende Tumorzellen in Blut oder Lymphe, jedes Gewebe verdächtigt Metastasen zu enthalten, sowie jede Quelle, die Prostatatumorzellen oder Teile davon enthalten kann, einschließlich Vesikel wie Exosomen, Mikrovesikel und andere, sowie freie oder proteingebundene RNA-Moleküle, welche von Prostatatumorzellen stammen.

6. Das Verfahren gemäß eines der Ansprüche 1 bis 4, wobei die Probe eine Urinprobe ist.

7. Das Verfahren gemäß eines der vorhergehenden Ansprüche, wobei die Analyse des Expressionsniveaus durch das Messen der Fluoreszenz eines markierten Oligonukleotides, einer markierten Sonde oder eines fluoreszierenden Detektionsmittels durchgeführt.

8. Verfahren gemäß eines der vorhergehenden Ansprüche, wobei die Analyse des Expressionsniveaus mittels qRT-PCR durchgeführt wird.

9. Verwendung eines Kits in dem Verfahren gemäß eines der Ansprüche 1 bis 8, umfassend
a) mindestens zwei Oligonukleotide oder Sonden, die unter stringenten Bedingungen mit mindestens zwei Nukleinsäuren hybridisieren, wobei die mindestens zwei Nukleinsäuren aus mindestens zwei Gruppen von Nukleinsäuren ausgewählt sind, wobei die Gruppen bestehen aus:
Gruppe 1: SEQ ID NO 1
Gruppe 2: eine Splicevariante der Ensemble Gene ID ENSG00000245750.3 ausgewählt aus der Gruppe umfassend SEQ ID NO: 4, 5, 6, 7, 8, 9 und 10
Gruppe 3: eine Splicevariante der Ensemble Gene ID ENSG00000255545.3 ausgewählt aus der Gruppe umfassend SEQ ID NO: 26, 27, 28 und 29
Gruppe 4: SEQ ID NO 3 und 11
Gruppe 5: SEQ ID NO 12
Gruppe 6: SEQ ID NO 24
Gruppe 7: SEQ ID NO 36
Gruppe 8: SEQ ID NO 38
b) Anweisungen bezüglich des Kombinierens der mindestens zwei Oligonukleotide oder Sonden.

10. Kit zur Verwendung gemäß Anspruch 9, umfassend mindestens 8 Primer oder Sonden, wobei die mindestens 8 Primer oder Sonden unter stringenten Bedingungen mit mindestens einer Nukleinsäure jeder der acht Gruppen hybridisieren.

11. Prostatakrebs-Therapeutika zur Verwendung bei der Behandlung von Prostatakrebs, wobei der Prostatakrebs gemäß dem Verfahren nach Anspruch 1 diagnostiziert wird.

12. Prostatakrebstherapeutika zur Verwendung gemäß Anspruch 11, wobei die Prostatakrebstherapeutika umfassen: Docetaxel (Taxotere ®); Cabazitaxel (Jevtana®); Mitoxantron (Novantron®); Estramustin (Emcyt®); Doxorubicin (Adriamycin®); Etoposid (VP-16); Vinblastin (Velban®); Paclitaxel (Taxol®); Carboplatin (Paraplatin®); Abirateronacetat, Bicalutamid, Casodex, Degarelix, Enzalutamid, Goserelinacetat, Leuprolidacetat, Prednison, Sipuleucel-T, Radium 223-Dichlorid und / oder Vinorelbin (Navelbine®).

## Revendications

1. Méthode de diagnostic du cancer de la prostate, comprenant les étapes de
a) analyser les niveaux d'expression d'au moins deux acides nucléiques dans un échantillon d'un patient, dans lequel lesdits au moins deux acides nucléiques sont choisis parmi au moins deux groupes d'acides nucléiques, dans lequel lesdits groupes sont constitués de:
Groupe 1 : SEQ ID No. 1
Groupe 2 : variant d'épissage du gène Ensemble ID ENSG00000245750.3 sélectionné dans le groupe de SEQ ID NO : 4, 5, 6, 7, 8, 9 et 10
Groupe 3 : variant d'épissage du gène Ensemble ID ENSG00000255545.3 sélectionné dans le groupe de SEQ ID NO : 26, 27, 28 et 29
Groupe 4: SEQ ID NO 3 et 11
Groupe 5 : SEQ ID NO 12
Groupe 6 : SEQ ID NO 24
Groupe 7 : SEQ ID NO 36
Groupe 8 : SEQ ID NO 38
b) dans lequel, si la combinaison du niveau d'expression desdits au moins deux acides nucléiques ou le niveau d'expression desdits acides nucléiques de tous le groupes 1 a 8 est supérieur a un valeur seuil, l'échantillon est désigné comme positif au cancer de la prostate.

2. La méthode selon la revendication 1, dans laquelle au moins l'un desdits au moins deux acides nucléiques est choisi parmi le groupe 1 SEQ ID NO 1 et/ou le groupe 2 SEQ ID NO 4, 5, 6, 7, 8, 9 et 10.

3. La méthode selon la revendication 1 ou 2, dans lequel le niveau d'expression d'au moins 5 acides nucléiques est analysé, dans lequel lesdits acides nucléiques sont choisis parmi au moins 5 groupes différents.

4. La méthode selon la revendication 1, dans laquelle le niveau d'expression d'au moins un acide nucléique de chacun des huit groupes est analysé.

5. La méthode selon l'une quelconque des revendications 1 à 4, dans laquelle l'échantillon est choisi dans le groupe comprenant le tissu prostatique, le matériel de biopsie, les ganglions lymphatiques, l'urine, l'éjaculat, le sang, le sérum sanguin, le plasma sanguin, les cellules tumorales circulantes dans le sang ou la lymphe, tout tissu suspecté de contenir des métastases ainsi que toute source pouvant contenir des cellules tumorales de la prostate ou des parties de celles-ci, y compris des vésicules telles que des exosomes, des microvésicules et autres ainsi que des molécules d'ARN libres ou liées à des protéines dérivées de cellules tumorales de la prostate.

6. La méthode selon l'une quelconque des revendications 1 à 4, dans laquelle l'échantillon est un échantillon d'urine.

7. La méthode selon l'une quelconque des revendications précédentes, dans laquelle l'analyse du niveau d'expression est effectuée en mesurant la fluorescence d'une amorce marquée, d'une sonde marquée ou d'un agent de détection fluorescent.

8. La méthode selon l'une quelconque des revendications précédentes, dans laquelle l'analyse du niveau d'expression est effectuée par qRT-PCR.

9. Utilisation d'un kit dans la méthode selon l'une quelconque des revendications 1 à 8 comprenant :
a) au moins deux amorces ou sondes, qui s'hybrident dans des conditions strict à au moins deux acides nucléiques, dans lequel lesdits au moins deux acides nucléiques sont choisis parmi au moins deux groupes d'acides nucléiques, dans lequel lesdits groupes consistent en:
Groupe 1 : SEQ ID No. 1
Groupe 2 : variant d'épissage du gène Ensemble ID ENSG00000245750.3 sélectionné dans le groupe de SEQ ID NO : 4, 5, 6, 7, 8, 9 et 10
Groupe 3 : variant d'épissage du gène Ensemble ID ENSG00000255545.3 sélectionné dans le groupe de SEQ ID NO : 26, 27, 28 et 29
Groupe 4 : SEQ ID NO 3 et 11
Groupe 5 : SEQ ID NO 12
Groupe 6 : SEQ ID NO 24
Groupe 7 : SEQ ID NO 36
Groupe 8 : SEQ ID NO 38
b) des instructions pour combiner lesdites au moins deux amorces ou sondes.

10. Kit pour l'utilisation selon la revendication 9 comprenant au moins 8 amorces ou sondes, dans lequel lesdites au moins 8 amorces ou sondes s'hybrident dans des conditions strict à au moins un acide nucléique de chacun des huit groupes.

11. Agents thérapeutiques contre le cancer de la prostate à utiliser dans le traitement du cancer de la prostate, dans lesquels ledit cancer de la prostate est diagnostiqué selon la méthode de la revendication 1.

12. Agents thérapeutiques contre le cancer de la prostate pour l'utilisation selon la revendication 11, dans lesquels les agents thérapeutiques contre le cancer de la prostate comprennent: Docetaxel (Taxotere®); Cabazitaxel (Jevtana®); Mitoxantrone (Novantrone®); Estramustine (Emcyt®); La doxorubicine (Adriamycine®); Étoposide (VP-16); Vinblastine (Velban®); Paclitaxel (Taxol®); Carboplatine (Paraplatin®); Acétate d'abiratérone, bicalutamide, Casodex, Degarelix, Enzalutamide, Goséréline acétate, acétate de leuprolide, prednisone, sipuleucel-T, dichlorure de radium 223 et / ou Vinorelbine (Navelbine®).


### SEQUENCE LISTING

<110> Fraunhofer-Gesellschaft zur Förderung der angewandten Forschung e. V.
<120> Novel RNA-biomarker signature for diagnosis of prostate cancer
<130> B176-0002WO1
<150> EP14195709.2
   <151> 01.12.2014
<160> 42
<170> Patent In version 3.5
<210> 1
   <211> 825
   <212> DNA
   <213> Homo sapiens
<400> 1
   ttttccggct ggaaccatgg agggtgttga agagaagaag aaggttcctg ctgtgccaga      60
   aacccttaag aaaaagtgaa ggaatttcac agagctgaag atcaagcgcc tgagaaataa     120
   gtttgcccaa aagatgcttc taaaggcaag gaggaagctt atctatgaaa aagcaaagca     180
   ctatcacaag gaatatatgc agatgtacag aactgaaatt caaatatcga ggatggcaag     240
   aaaagctggc aacttctatg tatctgcaga acccaaattg gcgtttgtca tcaggatcgg     300
   aggttatcaa ttgggtgagc ccaaaggtct gaaaggtgtt gcaacttctt tgccttcatc     360
   aaatcttcaa tagaaacttt gtgaagctca gcagggcttc aataatgtgc tgaggtttgt     420
   agaaccatat attgcatggg catacccaaa tctgaagtca gtaaataaac taatctacaa     480
   atgtggttat agcaaaatga ataagaagct acttgctcca acagataata ctttgattgc     540
   tcgatctctt ggtaaatatg gcctcatctg catggaggat ctgattcatg agatctatac     600
   tgttggaaaa tgcttaaaga agcaaataac ttcctgtggc ccttcaaatt atcctctcga     660
   caagttagaa tgaagaaaaa gaccaccaat tttgtagaag gtggagatgc tgacaacagg     720
   gaagaccaga tcaacaggct tattagaaga ataaacatgc ctaccacaat tattttttgt     780
   aatctagcca gttaataaac agtacctgct ctcaaattga aaaaa                     825
<210> 2
   <211> 3120
   <212> DNA
   <213> Homo sapiens
<400> 2
   gcccgcttct gtgactccac cccttacgga aagtctatgg gactctctga aatgtatgag      60
   tgatactgtt agaaagcggc aagaaaatga aaagaaaacg tggatgtttt ccatatttga     120
   gttgatgcag gctggagaca acagctgcca gagctgcaag gcagaacacc ctgagaagtg     180
   gaaccggagg atccaggcgc ctgttgaagg gataaatgag agccgtggcg tgtggatggg 240
   actctttctg tcttccgttt ggctgaagct ccaaacgcca ggagtaaccc agtgggtagc 300
   aagcaccctt ccctccacct ctgcaggaat tgcaaaaagc aatggtaaaa aaattggttt 360
   gtgatcctta ccccagtgct caaggaggtt ggagaagctg aaagagagga ctggttttga 420
   tatggtttgg atttgtgtcc ccacccaaat ctcatgtcaa atggtaatcc ccagtgttgg 480
   aggagggact ggtgggaggt gattggatca tgggggcgaa tttccccttg ctgttctcgt 540
   gatagtgagt gagttctcat cagatctgat tgcttagaag tgtgtagtgc tggccgggtg 600
   cggtggctca cgcctgtaat cccagcactt tgggaggcca aggcgggcag atcatgagat 660
   caggagatag agaccatcct ggccaacatg gtgaaacccc gtctctacta aaaatataaa 720
   aattagctgg gcgtggtggc gtgtgcctgt agtctcagct gcttgggagg ctgaggcagg 780
   agaatagctt gaacctggga ggcggaggtt gcagtgagcc gagatcgtgc cactgcactc 840
   cagcctggcg acagagcaag actccatctc aaaaaaaaaa aaaaaaaaaa aaaaaagaag 900
   tgtgtagtgc ctcccacctc tctctcttcc tcctgcccca gccatgtgaa cacattcctt 960
   ctttcccttt gccttgctcc gtgagtgtaa gtttcctgag gcctcctcag ccatgcttcc 1020
   tgtatagcct gcagaactgt gggccagtta aacctctttt ctttgtaaat tacccagtct 1080
   caggtatttt ttttagcagt gcgagaacga agggactaat acaggtttgg agacacgtct 1140
   ttggagaaac tattgatact tggcgtcctg tggcatttcc ttgccattag cacctccaac 1200
   gttatgctgg agaaaaagcc gttccaactc ttcaccgaac gccgagtcag agagatgtgg 1260
   ggaaaccacc gaaagagctt gccctgtgtc ttctggacag actagtgcca aacccatgcc 1320
   tggaaatggg tagaagttag ggaccatggc cgctcagcct ctgcaggaaa atggattact 1380
   gcatcaggaa tgatgacttt agagaaggcc aaaggtgtaa gtttcttcca gcaggttctg 1440
   aacctccaga ggcagctggc ctggggttat ttgggattcc cagtgcagga ctgcctgaaa 1500
   atgtgaggcg cccaaatggg gctgctggac cacaggatgc ccaggacctg caggcacact 1560
   caagagtggc ctgccagagg agacacattg cctgcctccc aggaagcaaa tttggaggtt 1620
   gccagtggga gaattgccaa gaaccgccat ccctcccctg ggacacacaa gctaaggaaa 1680
   gaaggaattg gccctctaag tccagaaagc acaagatcac atcatcacaa caccagccaa 1740
   gtaaaacatc cctgctcctc acctcttcag agcccaaggg ggctggggag gagaagaaat 1800
   gagagtggag gaggaaagag gtttaatatg aatgaagttt ggagttttga catgactcga 1860
   cattctaatg acttcattta gactgttgct acactcagaa gtgacttgga gacattttat 1920
   ttttataatc atcaccatat tgttattggt catgatctaa gggtgaggag aagggctaaa 1980
   ggatcggccc aatggatcat tcggggaaag taaagaacta tcccaccagt tgtgtttgaa 2040
   tgggcagtga gagggaaaat caagttgcta cttgcacccc tctaactccc aacttccact 2100
   ctttcaacat accagacatg aaagctgagt aaatgacctc aaaggtacag cgatgacagg 2160
   gtgtggtggt ggtagttctt ggtttcctac attgcatctt caaatcgggt tttagaatgg 2220
   atgacactgg aaggctagaa atctcaggta ctgacatgtg ggctggttta taattgtctt 2280
   caaaaattca cccacaatag gatcagacaa gttagtatgg ccataggtgc tacaatggga 2340
   gaaagtttta aatgctgttc agatcggaag acatgggtca aaaagatcaa gaagagttga 2400
   catgaggcaa acattttata tctccattca agagctacct gatgaggttt cacccatcag 2460
   ggatacaatg agaatttact gggcgcctag cactgtgcag acacaattca agcaaaaggc 2520
   aaccaactct ggcatccaag tgcaggccag cagccaagtt cttcctcctt atgagctgac 2580
   atctgattga cagatggccc cagagaaagc ttcttggatc atatctcagt aaccactggc 2640
   ccagcctgtc aaatccagag atttaagatg gcatagcacc ttaaatctgg aagaacttag 2700
   caactttata atgtcaaatc cagggggaaa atacgcacac agagaaacca gaaaagcata 2760
   gaatggtggg gagaaagaaa gaaagaaaat gaaaacactg tttccatcgg cagaattagc 2820
   ctttgccagg aaaactgcaa tgttgtcttg gtaggcacat aattcatttt tctacatgtt 2880
   cagaagtaaa gaaaaagcca gacccaaaaa gccagaccct gggggaaatc gtggatactg 2940
   tttgtaactt tggcaaagac aatatatttg tgaactcagc ctcatgtgaa atgcttgata 3000
   atctcaaaaa gtaaatgcca cagcgtgtcg tatactgtgt ggtctcaatg atgtgagaat 3060
   gtctatatat cccaatacct gtcctcgttt ctaactagct tgatttattg tcatagacaa 3120
<210> 3
   <211> 1641
   <212> DNA
   <213> Homo sapiens
<400> 3
   attgcccaca gccggatcca cggtgactaa tctccgggaa ggcgtccagc gtgagccgtg 60
   aggcctgcac ctgcgccgga cttcaccact caccaggagt ctctgcccgc cgcccccagc 120
   gcggttctct tccctgctag acgtaaagtc ctggggacaa gcacagccca gggcactttc 180
   tggagagaag atttcgatgg ggaaagataa gagagcattt ggatatctac gcactgtcaa 240
   cagaaatgag gcttcaggcc aaggcttcta aaatgcagga ttcccaaagt ggcagtcaca 300
   taagacgggt ccctccatcg ccttctagaa tattgagaag atcatcacgc ctgggactgg 360
   ctcacagcac tgatcttttt cttgttagca gagactccag gaagcagatg agcagaccta 420
   tgggcctgct atgtgcaccg cggcccacac tgccgcctgc ctgggatcct gcatcacccg 480
   agtcttcctg cggaccaccc tgggtggtgc acatgcggtc atcattgtgc aaaggaagct 540
   ttagttctat aatgaaagcc cctttagtaa gtgatgctcc ttgaagactc ggtcagcgtc 600
   tcgttcacct ctttatcccc agacttagca caattccagc cccaaatcgg caacggacct 660
   ttgaccaagt gacatgtcag tgcttcaggg agcttcactg atcaggacag aagtgagtga 720
   ctggaggaag gtgacccgga tgtctgtaac ccccagaaag ggtgagatgg ctgttttatc 780
   agccagcatt gccgagttgg tggtgtgacg acatcctgtc tgactgctgg gccttaccgg 840
   tgtgttctct gctggagccg tgacagtttg agttgaggac attttacatc aatcactgaa 900
   ctgcacgaaa gatggaaatc cagcaaggga ccccgtcgga cctgattgac cgcagagcta 960
   ccttttgctc tttctggatg ccacacggcc tcctcctccc acctgtcctt ggagcttgtg 1020
   ttggtccact ttcatgctgc tgataaagcc atacctgaga ctgggtaatt tataaagaaa 1080
   aagaggttga atggactcac agttccacgt ggctggggag gcctcacaat cacggcggaa 1140
   gttgaaaggc acgtcttaca tggcggcagg caagagagaa tgagagccaa gcaaaggggg 1200
   tttcccctta tagaaccatc agatcagctg ggcgcagtgg ctcactcctg taatcccagc 1260
   actttgggat gctgaggtgg gaggatcaca aggtcaggag tttgagacca gcctgaccaa 1320
   tatggtgaaa ccccatctct actaaaaata caaaaattat ctgggcatgg tggtgggcac 1380
   ctgtagtccc agctactcgg gaggctgagg caggagaatc gcttgaacct gggaggcgga 1440
   ggttgcagtg agctgagatt gtgccactgc actctagcct gggcaacaaa gcgagacgct 1500
   gtctcaaaaa aacaaaaaca aaaacaaaaa aaaccatcag atctcatgag acttattcac 1560
   taccatgaga acagtatggg ggaaaccacc ctcatgattc aattatctct tgctgggtcc 1620
   ctccctcaac acatgggaat t 1641
<210> 4
   <211> 826
   <212> DNA
   <213> Homo sapiens
<400> 4
   caggaatggg ctggggcgcg tttgtagttg ggaatcctga gcccgggctg ttgcttggag      60
   gactcgggag cagcagtgga tttcggcgtt accaggagag ctatgtatag gaatgccgct     120
   atggaaagac atccaggaca ccttgttaag tgaaaaaagg ttcagtatct gaacactttt     180
   gctaaaggtc tctggaaagc tccctgtgtt aattctcgat gagactacgt ctgtgccggc     240
   cgcttcctga gcctccttcc atgtgcatag gcctgctgca tgctggagac ctgcggcaca     300
   gctgccaagt gccccgtgtg gttctgtgat cttgggacag tcacttcctc tctgtggttg     360
   tcagtcttct catccctaag atggaataaa aaggcctacc tcattgggct cgtgtgggtg     420
   aggagaactg aagagtctga gagcgcggca cgagccagag gctacggaaa acactgccct     480
   cctacactcc accttggaga gacccagaaa agaacaagct tcatttgtaa aaaaggaaaa     540
   caactcaggc aatgggggtg gcttaaaggc actctacagt gtgcagatgc cttccacttc     600
   ttccatctgc cctgtctctc caagaacccc tatggccccg gtctcagaac agagctgagt     660
   gcagaaatga aaatctatgg ctctgtgttc caaaacgatg aagaatttca agatggtggc     720
   agtggtaaaa tctttctcca ggaaaaatct gtccttggcc caatgtgtaa acacttgctg     780
   aggaacttgg aataacttgc agtgtcttgc agtattgtga aaccag                    826
<210> 5
   <211> 806
   <212> DNA
   <213> Homo sapiens
<400> 5
   ttcggcgtta ccaggagagc tatgtatagg aatgccgcta tggaaagaca tccaggacac     60
   cttgttaagt gaaaaaagac atgccaccat tagggcttca tttccctgca gtcttcttta    120
   aataccaaaa ctgccctggg aaggataaga catcagtttc ttcatacttc agagcaaatg    180
   ggattgattc ccaggacctc ctttctgtgg actgggtttt tgcctcctca ccaagagact    240
   gggctccctg ggcgagcaga ggctgctggg ttcagtatct gaacactttt gctaaaggtc    300
   tctggaaagc tccctgtgtt aattctcgat gagactacgt ctgtgccggc cgcttcctga    360
   gcctccttcc atgtgcatag gcctgctgca tgctggagac ctgcggcaca gctgccaagt    420
   gccccgtgtg gttctgtgat cttgggacag tcacttcctc tctgtggttg tcagtcttct    480
   catccctaag cactcaagaa gcccttcctt ctctggctgt caagtctgga ctcatctctt    540
   gtcttacaca cggacagttg ttttaaggaa aagcccctgg ttggacttgc agattgggca    600
   cagccctgga cgtatactac aggtgctcat caaggcttgc ctcatctgtt taccctccag    660
   gtcatgcccc tttctacctc tacaccctgt tgcattgcgg gcatggcctc cggaaaagtc    720
   aagcatggcg aaaggaagcg tggcccagag ctggcacgcc acaggaactt ggaataactt    780
   gcagtgtctt gcagtattgt gaaacc                                         806
<210> 6
   <211> 1358
   <212> DNA
   <213> Homo sapiens
<400> 6
   gaggcccgac attgtgctgg ggaaggagct ccagaaaggg ccatcctttc tgttttggtt     60
   cagtatctga acacttttgc taaaggtctc tggaaagctc cctgtgttaa ttctcgatga    120
   gactacgtct gtgccggccg cttcctgagc ctccttccat gtgcataggc ctgctgcatg    180
   ctggagacct gcggcacagc tgccaagtgc cccgtgtggt tctgtgatct tgggacagtc    240
   acttcctctc tgtggttgtc agtcttctca tccctaagat ggaataaaaa ggcctacctc    300
   attgggctcg tgtgggtgag gagaactgaa gagtctgaga gcgcggcacg agccagaggc    360
   tacggaaaac actgccctcc tacactccac cttggagaga cccagaaaag aacaagcttc    420
   atttgtaaaa aaggaaaaca actcaggcaa tgggggtggc ttaaaggcac tctacagtgt 480
   gcagatgcct tccacttctt ccatctgccc tgtctctcca agaaccccta tggccccggt 540
   ctcagaacag agctgagtgc agaaatgaaa atctatggct ctgtgttcca aaacgatgaa 600
   gaatttcaag atggtggcag tggtaaaatc tttctccagg aaaaatctgt ccttggccca 660
   atgtgtaaac acttgctgag gaacttggaa taacttgcag tgtcttgcag tattgtgaaa 720
   ccagcaactt gttcacaatt cttctgaatt tcttgggaaa tttgaagtgg agtacctgta 780
   ccaacatgaa atgacacgaa tttaagtgac gctcaacaac gaaaagcaaa aagaaccaaa 840
   gaggaagcaa ctgaaacaac atctggatgt atttaaaaat atacaatgcc tccaaaatca 900
   ggtgtcatta atgaaaattc tgaagaaatg ccaccggaca tagccaacgc acctacgctg 960
   ttgttattca tttcctgctt ttcacagaaa acaattttgt tgcatggaag atcgtgaact 1020
   tcaaagggca gaggggaaac tgtcccttgg cctctacccc tccaaggccc cacttttttg 1080
   tcaacactcc ttggacgcag cagaagtatg aacataatat ggtcctgaat gaggctgagt 1140
   ctttgggcgc agaagacccg ggttaataaa aataggaagg taagaaaaga aaagaaaaat 1200
   caagacacat cataggacta aattcctatt atttatccac tcaggattga ccaccccttt 1260
   gggccagata gttgtacccc catgtaccag gtgggcacat gaagacacaa gaagtgctgt 1320
   gatggttcat tttgcacgtc accttgcgtg gagtatgc 1358
<210> 7
   <211> 309
   <212> DNA
   <213> Homo sapiens
<400> 7
   gactggagag gccagcacgc acagtgactt aatccaagaa gatggaataa aaaggcctac 60
   ctcattgggc tcgtgtgggt gaggagaact gaagagtctg agagcgcggc acgagccaga 120
   ggctacggaa aacactgccc tcctacactc caccttggag agacccagaa aagaacaagc 180
   ttcatttgta aaaaaggaaa acaactcagg caatgggggt ggcttaaagg cactctacag 240
   tgtgcagatg ccttccactt cttccatctg ccctgtctct ccaagaaccc ctatggcccc 300
   ggtctcaga 309
<210> 8
   <211> 1222
   <212> DNA
   <213> Homo sapiens
<400> 8
   ctgggcttcc agcttccaag ccttctacct gtggaatgct tggtccaatg tctggggcac      60
   ccactcttac tccaaactcc tccagatctg cagagtggcc cagcatattc tcttcttgaa     120
   aggaggtctt tgcctaacag aaatggaata aaaaggccta cctcattggg ctcgtgtggg     180
   tgaggagaac tgaagagtct gagagcgcgg cacgagccag aggctacgga aaacactgcc     240
   ctcctacact ccaccttgga gagacccaga aaagaacaag cttcatttgt aaaaaaggaa     300
   aacaactcag gcaatggggg tggcttaaag gcactctaca gtgtgcagat gccttccact     360
   tcttccatct gccctgtctc tccaagaacc cctatggccc cggtctcaga acagagctga     420
   gtgcagaaat gaaaatctat ggctctgtgt tccaaaacga tgaagaattt caagatggtg     480
   gcagtggtaa aatctttctc caggaaaaat ctgtccttgg cccaatgtgt aaacacttgc     540
   tgaggaactt ggaataactt gcagtgtctt gcagtattgt gaaaccagca acttgttcac     600
   aattcttctg aatttcttgg gaaatttgaa gtggagtacc tgtaccaaca tgaaatgaca     660
   cgaatttaag tgacgctcaa caacgaaaag caaaaagaac caaagaggaa gcaactgaaa     720
   caacatctgg atgtatttaa aaatatacaa tgcctccaaa atcaggtgtc attaatgaaa     780
   attctgaaga aatgccaccg gacatagcca acgcacctac gctgttgtta ttcatttcct     840
   gcttttcaca gaaaacaatt ttgttgcatg gaagatcgtg aacttcaaag ggcagagggg     900
   aaactgtccc ttggcctcta cccctccaag gccccacttt tttgtcaaca ctccttggac     960
   gcagcagaag tatgaacata atatggtcct gaatgaggct gagtctttgg gcgcagaaga    1020
   cccgggttaa taaaaatagg aaggtaagaa aagaaaagaa aaatcaagac acatcatagg    1080
   actaaattcc tattatttat ccactcagga ttgaccaccc ctttgggcca gatagttgta    1140
   cccccatgta ccaggtgggc acatgaagac acaagaagtg ctgtgatggt tcattttgca    1200
   cgtcaccttg cgtggagtat gc                                             1222
<210> 9
   <211> 1345
   <212> DNA
   <213> Homo sapiens
<400> 9
   ggagctggtt ccaggaaaga agggcacatg agcaaacatg atggcccctt tatgagaggt 60
   aatttactga aatgcacagc gattacctgc tcacccagcc ctgatgtaat taaaaatggc 120
   aggctagggg ctctgtttcc cccggctctg gcagagaaac ctgggtttcg acttgtgaag 180
   cttgaggttg gatgtgggaa ttggcttgga gtcataggcg atgagaggga cattaggata 240
   ttatgaagcc cgtgaactca actcctgaga aggacgcagc agagcgagag aaaagatgga 300
   ataaaaaggc ctacctcatt gggctcgtgt gggtgaggag aactgaagag tctgagagcg 360
   cggcacgagc cagaggctac ggaaaacact gccctcctac actccacctt ggagagaccc 420
   agaaaagaac aagcttcatt tgtaaaaaag gaaaacaact caggcaatgg gggtggctta 480
   aaggcactct acagtgtgca gatgccttcc acttcttcca tctgccctgt ctctccaaga 540
   acccctatgg ccccggtctc agaacagagc tgagtgcaga aatgaaaatc tatggctctg 600
   tgttccaaaa cgatgaagaa tttcaagatg gtggcagtgg taaaatcttt ctccaggaaa 660
   aatctgtcct tggcccaatg tgtaaacact tgctgagtat tgtgaaacca gcaacttgtt 720
   cacaattctt ctgaatttct tgggaaattt gaagtggagt acctgtacca acatgaaatg 780
   acacgaattt aagtgacgct caacaacgaa aagcaaaaag aaccaaagag gaagcaactg 840
   aaacaacatc tggatgtatt taaaaatata caatgcctcc aaaatcaggt gtcattaatg 900
   aaaattctga agaaatgcca ccggacatag ccaacgcacc tacgctgttg ttattcattt 960
   cctgcttttc acagaaaaca attttgttgc atggaagatc gtgaacttca aagggcagag 1020
   gggaaactgt cccttggcct ctacccctcc aaggccccac ttttttgtca acactccttg 1080
   gacgcagcag aagtatgaac ataatatggt cctgaatgag gctgagtctt tgggcgcaga 1140
   agacccgggt taataaaaat aggaaggtaa gaaaagaaaa gaaaaatcaa gacacatcat 1200
   aggactaaat tcctattatt tatccactca ggattgacca cccctttggg ccagatagtt 1260
   gtacccccat gtaccaggtg ggcacatgaa gacacaagaa gtgctgtgat ggttcatttt 1320
   gcacgtcacc ttgcgtggag tatgc 1345
<210> 10
   <211> 1033
   <212> DNA
   <213> Homo sapiens
<400> 10
   aggaacttgg aataacttgc agtgtcttgc agtattgtga aaccagcaac ttgttcacaa      60
   ttcttctgaa tttcttggga aatttgaagt ggagtacctg taccaacatg aaatgacacg     120
   aatttaagtg acgctcaaca acgaaaagca aaaagaacca aagaggaagc aactgaaaca     180
   acatctggat gtatttaaaa atatacaatg cctccaaaat caggtgtcat taatgaaaat     240
   tctgaagaaa tgccaccgga catagccaac gcacctacgc tgttgttatt catttcctgc     300
   ttttcacaga aaacaatttt gttgcatgga agatcgtgaa cttcaaaggg cagaggggaa     360
   actgtccctt ggcctctacc cctccaaggc cccacttttt tgtcaacact ccttggacgc     420
   agcagaagta tgaacataat atggtcctga atgaggctga gtctttgggc gcagaagacc     480
   cgggttaata aaaataggaa ggtaagaaaa gaaaagaaaa atcaagacac atcataggac     540
   taaattccta ttatttatcc actcaggatt gaccacccct ttgggccaga tagttgtacc     600
   cccatgtacc aggtgggcac atgaagacac aagaagtgct gtgatggttc attttgcacg     660
   tcaccttgcg tggagtatgc caactcattg tttggtcaaa cactagtctg gacatggtgg     720
   taaaggtatt ttttagatga gattaacact taaatcagta aagcaggtta cccaccatac     780
   tacgggtggg ccctgtccaa tcagttgaag gcattaagaa caaagattga ggtttcctaa     840
   agaagatgga attctccttg agactacaac atagaaaccc tatctgagtt tccagcctgt     900
   tgccctgtgg aattcaaact caggactccg gtctatgaca ttaaccctca cttaactttt     960
   cagcctgcca gcctgcccta tggatttcgg acttgccagc cacacaattc cttaaaataa    1020
   atctctccgt ctc                                                       1033
<210> 11
   <211> 786
   <212> DNA
   <213> Homo sapiens
<400> 11
   cagttttcac aggcctgtgt gccgagagtg ttccttacca ttttttcatt attattctgc 60
   taaggaggat ttttagacat tatgttccta gtcaagccct cacctctgca atgaaattat 120
   aataccactg ttgacccaaa gatgagcagc aagcgctaga actgagggaa atcatggtga 180
   gatttattct gagccaagct ggaggactta ggcccaggaa cacagaatct gtgtccactg 240
   agaatgtatc tcaaagcagg ctccatgagg caccgtgcac ttacattttc taacaggcgg 300
   acgtatgtgg cccagaaggt gggagaggca agcggtgagc agccgtgacg ttcctgagat 360
   tctgatgagc gctcggtggc actgcccaca tgataaggga gacatgagac atcaatcaac 420
   acatgtagga tgaacattgg ttcggtctgg aaaggcggga caactcgaag caaaggcagg 480
   aagactcgaa gcagacgggg gcttccaggt cataggaagt catgagcgaa gagaccgtga 540
   gcctctccca aaccccgcat gtgctgacat ctccacgtgt gctgtgtcct gggtaggctt 600
   tcttcataga ccgtgcttcc gtctgtctgg agtttccgct tcatccatct gtccacaggg 660
   aggacgcaca cccctgagtc cgaggccaca ccacgcattc tgtagaaact ctcactgccc 720
   agggagcaca ctttattagg aaagctccag gttcacagag gctctgggct ctctctgggc 780
   ccgacg 786
<210> 12
   <211> 2680
   <212> DNA
   <213> Homo sapiens
<400> 12
   caagacagag gcaagcagag aaggcatagc agcagcgacc ggcgctctgt tttcattttc 60
   cactctggcc aggggataaa ctggacccca gtggactcca acaccaactc tctgcttccc 120
   agttactttc gattttcttt cccttctcct tctttctctc cctctctggc attcaaacag 180
   aagcacaaga ggacccacca gtggcaactc taagcctcct tgcaggccac attccctgct 240
   gacgtggtcc ttcctgcagg agagcccttc tgtgattatc taataatagc cagggtggct 300
   gagaatagaa tctggctcaa ggactctgca cacccggggc agagacactg aatctgtggg 360
   tcagggcctg gcaacattcc ttggagcctc cccctctgag cccactcttt gggcaccctt 420
   aggcttgcct gcccccctta taaggggctt tgtcagccct gccatccgct gtatttcatg 480
   tgctggttcc tagctaagga gaattgggca gagcaaaccc tttctcaccc tggtctgaca 540
   gttgtgatcc cgacagaatg cgaccttgtt tggagaaggc gaccttgttg ggttggctgt 600
   gttctgaaac caggcttggg agtgttatat atttattata tacaggctta aatatacaga 660
   ctcaggctta tgcatgaata tatacagact catatttcct tggtggttaa ttgtatatta 720
   ccatgcctat attcgctcaa acaccatttt atgttcatgc aaaattttgt tagtcctcac 780
   tagatgagag tgggctcatg gaggcaccgg gtgcactgag caggccctct gtgcatgctc 840
   tgctggaaat atgtgctcac tggaatcgga gtatgcgggt tccagtaacc aatggatgcc 900
   ataaaggctt tctcagacct tattgctttt gttgcccaag cagctggact ggggaaatac 960
   taaatccctt ctgctttctc tccacacctc ctggcaaact ctcccaactt agaaattatt 1020
   ttacaaggga gaccagttgt cttgaaattg agttctgcgg gattcttttc acgggcatgg 1080
   tggggctgca agcagttcgg agtagtcagg gccctggcgc tggaagcctc actggagcga 1140
   tgaagggatc agagtggtta ataaggtggc ctctggacag tgatctgggg cagctgctgg 1200
   ccagggtgct tggagaagct tgtggaaaag gcaagccaaa aggggtaggg atgcagtgaa 1260
   tatctgtttc tccccctgct tgccccagcc ggccgctcac cccgccccag cccgatttgc 1320
   ctgcatccgt ggcggcagat tgggagttct ctaaggtccc ccaaataccc agatctggtt 1380
   actggatgaa ctgcgtttta atcctcgtcg ctatctgagc caggacaaga cctggtcagg 1440
   gagacggctg gtccgggcag cacgggagag gacgctggac ccgctgcctg tggcctggag 1500
   cactgagtat gggctaccaa ggctgagaat tgatgggcct cctttcccag cctttttccc 1560
   ttccagagac acaaaaagac agagcagagg ggaaatatca atgtcagtgg aaaaccccat 1620
   aaaagaaagc agggaccaga gcggggctgg aggggttttc cgctactctc tctaggtgat 1680
   cgggttcctc ccaagactct tgtccagaca ggctgcacca agaggtgtgc agctttcttc 1740
   caccttcctc ccgcccttca aggagggcag ccttaggctt tctgggcctt ggtcacccca 1800
   ggcactgggg tgacattact ccaggattgt gcccctcgcc cagcccacgg cctctgccac 1860
   ggcgccgcag ccgctcctgt ttattgtgtc tgtgagttgt ttacttgttt tgtctgctgc 1920
   caccctgcgt cagaaacccg ccaaaccagc gaactcgccc aggagaacaa agtgctgtgc 1980
   tatccccgga ctccctcccc tttccagcaa ccccctcctg ccctcacaag ctcctgcatt 2040
   ctccctttcg gaggggagga agcagaaata gtcttaaaac agggcagctg tttatagggt 2100
   caggggaatt atggggacac cataaacctc tgggtgtccc tgtggtggac agacttctga 2160
   gcagcccttc ccttgcctag gcttttcttc cttcccctgg tgaccacttg gttaaggggg 2220
   ccacactcat tgagaaagtt gagcccaccc ccatcaaccc agtcccccac taccccactc 2280
   ctttctgcag gagtttccct gagacacagg ttgttgggtt ttaaatcagg gtaatgttgg 2340
   tggattcata aagctagata tagcatgtca ttcttctccc acactccctc tcctcagccc 2400
   cccaaaattg cagtgaattt aagcaaatcc tacaaaatta aattgcccct ttgctaaaga 2460
   tcctatatgt ctccaatcct gccaggtgta gagtcagata gttggctttt ggcctttaat 2520
   tttgctgcta gctattaaat ttaaaagagt atcattgaag taagtaatat tctcatttct 2580
   gaaattaatt ttcccctccc agtctctctc tctctctttt tgccagcaca gattaaatgg 2640
   ttctttcaga tctgcaaaca ataaaggata aaattattta 2680
<210> 13
   <211> 2422
   <212> DNA
   <213> Homo sapiens
<400> 13
   ggtaacatga aaataatgga tgagcagttc aactatatta aaaataaacg tggttaagag      60
   tgctcacctt aagtgtagga tttgaaagtg taggctctaa agtcagactg ggttcggaac     120
   ctggtatcat tactgacttg gtgtgtagct tctctatttc tcagtctatt taacctttct     180
   catccagaaa atagtgataa taatgctgtt tatctcatag agttgtcgtg agaattaagt     240
   gagttaattt gggtacagtg tttttaacag tcaggcacat ggtgtgtcca acaaatgttg     300
   gctattagaa ttgtgatggt ttctcctata gttcgagatt ttagtctctg caaagtaaaa     360
   gacttagagg ggggaagctg cccctttcag atgtggtgtg gcatttattt ccaagtgcac     420
   gtgtgagtct tctgtcttgg taactctcca gtgtggtcat tagtgttttc cagttggtta     480
   gtgtgtctct tctcttccaa tagcaagata ggtgtttcaa attcctaggg cttctatcag     540
   cacacatgag ggggcttctg tcccttggtg agcactggct tgtttataag cctacaaaca     600
   gcactgtgtg ctcaatattc tgtctgcacc tctagattta cctccattct tcaccctgat     660
   ttttgccccc agcaggctga cctctaagga tcccctggct cctgtgcctt ctatattctg     720
   gttggagtca aagggagttg cagggaggca atcagagagc aggaggagag agatgctatg     780
   ggattatctc ctccacccca acacattcct tttcttctgg gctgcagtct ggcagcggct     840
   gcattcctct actaaatgcc acaactcctt tcaagtggct cctgtctgac aacttcctgc     900
   tgggttccag tggctgctct ttctcttgcc ccttcagatc tgaaggactc ctctgttttc     960
   tagttctagg tactgccact tacctgttcc cttatctctt gccacacctc ggtagtccct    1020
   ttattgaaag ttcttcagct atcctctttg agcacactgt ttcctagctg gtacatattg    1080
   accttgagaa ggttgatgct tcctcaccta cttactgttg ggaagaaaat ggaatatttt    1140
   aactgctcct gcaatgtcaa tacagaatcg gccctttcaa agtccagtgc cataggatat    1200
   gaagagcatt gctagaacag cgggacatga atgttctacg tggctaaata caatgatctc    1260
   cagtataaaa acccaaatgg tcaaagatga actcaaagga ttggagtgaa cacaggtttt    1320
   attattactc ttagaacact ctgggttata aaagaactta cattctttaa cataattctg 1380
   ttgttttgat actaacgttt agtgcccatt ggatggcagt gccaggaata aaatgtctga 1440
   attaagctag tgggggtgat tgagtttatg gccaggagct acattggaga gagagccatg 1500
   tcagtagcct aacactggca agacagtgac ccaagtactc tagacttgga tgtttttgat 1560
   aggttctttg gaaaaacctc ttctaggtcg ttaagaaagt ttgaaaggct ttagtggttt 1620
   aattagataa aatgtagcag aaggcaatat aacataggca cccatagaaa ggagggcaat 1680
   ggtttataag agcttgactc tggctcacat ctgagagaca agaaaacata aatccttcag 1740
   tcagatcctt ttatgggacc cactttcctc atagattagt ttatgtaaaa actccagtaa 1800
   agaatagcac agagacctct gtttgtatgt ttgatgattt tactgttagg gttttattac 1860
   ttggttcatg tgcccgagga agtgaacggg tccatgaagg atatatttgt gaggttaaag 1920
   ccacatgtag tttatttaac ttttgcagtt ttccaatttg ggtctatatt tcaaccccga 1980
   attttctaat ttggggtcta catttcaatc aaaaagatta aatttctttt cctctgtgtt 2040
   tgtggatctc tctgggcgcc acccttaaaa ttagcatcat ggttcccatg gcaactaacg 2100
   tacctggaag atggcaagaa aggaaaagga ctaaataaaa tagaatgaag ctgcatctga 2160
   aaaactggaa ttaattatga acttgaacaa agggttatac tgcaagtggt tggggagatg 2220
   agtattaagc atataaggtc tttgctttaa gacctctgta attcacttac catttcatta 2280
   ctttgtttat tcagcagccc ccaaatcact gctagttttc ttttccctct caccttcaat 2340
   agagaacatc attggggtca ggactgtgca tagttcagga cgagtttacc tctacagggt 2400
   gcattttgta agatgtcccc aa 2422
<210> 14
   <211> 830
   <212> DNA
   <213> Homo sapiens
<400> 14
   tgaagcccat gagccactag aagccacatg ttctgccatg tggagaagaa tgagagagta      60
   catcctcaaa ttgaggtgtg gcatgatgat ttggctgccc agaggagaac ctgtctttcc     120
   catctaaagt gggaggtcaa gccaagtaga gggaaacagc aactggagag cagatttgca     180
   aaccagaaaa gcaactcatg aatacaaatt gcctggaaag catttattac aacagaagct     240
   ctgcaagata taccggaaat tgacagtggc atcgaattcc cagcaaggaa gttttctaag     300
   gacaccaaca gacaccagca gaagacagcc ttgcatttac aacaaaccat tgatccacaa     360
   caaaccattg atccacactt tcaaaatcat tgaggtgaag catagagaac tggagtggag     420
   acggctctgg caaatccaaa caatatcaaa agcactctca tactgaaaac ccacagtgct     480
   gaaccacagc tgagatgtaa aatatttcac aaaatcttct tcaccctcta tagtatttca     540
   cctgagagtg catgatttaa gttgcaagca ctgtcataga tgatgggatt cagagatgaa     600
   cacatggaaa tggactttat gttctctaat gtcacaaaga aagagtgatt atgaagaaga     660
   cccagattcc cacctattaa tctgagcttt gaattcttta ctttgctggg tccaccggat     720
   tttttctcca ctcagcccat cgatgagagt ggaggcggcc tgcatctttt tccggcacgt     780
   atcagcgtca ttaagcaaat cctgcagcca taatgggaga atgtctcaaa                830
<210> 15
   <211> 383
   <212> DNA
   <213> Homo sapiens
<400> 15
   ctttcaaggg cctgtgcctg tggtaactgt ctatgagcca ggtatatctg aagcatattt      60
   gacaacagaa aaagttaatg taattttcaa aggaaaaacg ccaacttttt tcaaaaaggc     120
   tggatccagc tcttcatgaa tgtcctcaat gagaaggggt cggcccaagg aaaggctgtc     180
   ctccaagtgt gtgcgaaaat atttatggtt cagagatgtc acctaaacag aaagaactca     240
   ttggtctatc tgtgttacaa agaatgtgcc cattgctaag ggaaaccgag agatttgttg     300
   gacaggtaat caagaatttt aagggcaata gaagaagact tacttaaaaa aattaaagta     360
   acatacaaaa attaatttct aaa                                             383
<210> 16
   <211> 753
   <212> DNA
   <213> Homo sapiens
<400> 16
   atatctgaag catatttgac aacagaaaaa gttaatgtaa ttttcaaagg aaaaacgcca      60
   acttttttca aaaaggaaac agcaactgga gagcagattt gcaaaccaga aaagcaactc      120
   atgaatacaa attgcctgga aagcatttat tacaacagaa gctctgcaag atataccgga      180
   aattgacagt ggcatcgaat tcccagcaag gaagttttct aaggacacca acagacacca      240
   gcagaagaca gccttgcatt tacaacaaac cattgatcca caacaaacca ttgatccaca      300
   ctttcaaaat cattgaggtg aagcatagag aactggagtg gagacggctc tggcaaatcc      360
   aaacaatatc aaaagcactc tcatactgaa aacccacagt gctgaaccac agctgagatg      420
   taaaatattt cacaaaatct tcttcaccct ctatagtatt tcacctgaga gtgcatgatt      480
   taagttgcaa gcactgtcat agatgatggg attcagagat gaacacatgg aaatggactt      540
   tatgttctct aatgtcacaa agaaagagtg attatgaaga agacccagat tcccacctat      600
   taatctgagc tttgaattct ttactttgct gggtccaccg gattttttct ccactcagcc      660
   catcgatgag agtggaggcg gcctgcatct ttttccggca cgtatcagcg tcattaagca      720
   aatcctgcag ccataatggg agaatgtctc aaa                                  753
<210> 17
   <211> 13965
   <212> DNA
   <213> Homo sapiens
<400> 17
   atcccctctg agaatttatc agaaaaacaa gcaataagtg agaccaacgt tgtgaggtat 60
   taactcggaa ccgtcatcta tccttgtgga gaaaaacccg gaggacttgg attaggaccc 120
   aatagtctac cttggcggca ggtgagaatc acctggggag cttttgaaac atgaggctgc 180
   caggctggac ctcactccag accaattgaa ccagaatctc tgaggcgggg cctgggtgtt 240
   ggtaattttc ctttgaaaca ctccccaggt gattctaatg gaatctagga gtgagaatca 300
   ctgggggaag gcatttgtag atgggaaggc tggtggcatc tgctagaaga cttgatgtgt 360
   acttggccag agataaacca caggtctggc ctgaatgggt gtggaaatgt gtcagtcatg 420
   ttttcagatt tccaaggtca ttttccaaga gacctctgtg agcttggact ggatagcgca 480
   gcacagggcc agcgatggca gctggaaggg tggctgccta ggaaggaggt aagctttaat 540
   aactgaaata gatgctgcac tttcatctac ttaattgcta tgatttgaag tctcccatag 600
   cctgaaacga ggtacccagg ggagggtgtg agagaagggg agggtactga gggatcctgg 660
   ggcccctaga ccttatctca tggaaggtgc tgggctgggt cagcaggact caattgcctg 720
   ctgcaaaggc ctcagtgctc cccagagatc cggctcccca cagaggcact gactgcacca 780
   caccctgaga tctcactgcc ggtctacagc aggaccatta agataatcaa agagtctctg 840
   gggagtttgt ggagcgtgac tgtgaccacg ggagcatatt ttattttgcc ttgaaccaaa 900
   ctgctaagga agaattctct tacattttca acagaaagag ggtatcagtc agccaaatag 960
   ccctgagcac acttctagtt agctaacagc atcccagaaa aatagacctg aagtttctat 1020
   aagcagcctg tctccttcga gaccaaagct ttctctcagt cagggatgtt gttgggcccc 1080
   atagtaaatt tggggagacc agggaaaatc caggctcagg agaggtaaat tttcactgtg 1140
   cttgaatcag gagaatctga ttatcaacat caacccttct ctaaaatgaa tttaaaccaa 1200
   catttaaaca tgcttcctgc cacctgcccc cactctatag aatttttcct cgactctccc 1260
   caccccccta ctctctttcc ttccaacaac tccatttcaa ccacacgcac ttgatagaat 1320
   atcacacaca ggcacattta ttattgacca gcttctttat ctcggatagt atgtcatgag 1380
   tagtcaagac tatagctctt gaaggtagtt tcatagattt aagatgaaga aacagtatcc 1440
   atatagttcc agagtcaagg tagtctaata gccagtgaaa aaatacaagc tgttggagag 1500
   tggaagttta ctcatgggag ctggcttgga ctttagatat gaaacatttg atttatagtt 1560
   tctatttctc tctgatagaa aactctgcta acccttattg gaaggtcgaa tgtgttgggt 1620
   tctttagctg aggtagctgt taaaatttat ttcacattca aggaaagggg ctcttgttat 1680
   taaccatgtt cccattttaa ttatgatttt cctggcatca tttttaacaa gaataaccta 1740
   aggcctgcaa ggagtgattt cccctaatca cttcttaaac cctccctccc cacccccaac 1800
   taactgctat agctacagct acacagatga aatgaaatgg agacttgagc aattacctgg 1860
   tggatctgaa cagtgtggag tcagcagtca attagaatag ttcaattaca aaaatccttt 1920
   tttctccctt cttcttttct gctatagaat tatcacctta actcttcctc ctccacccac 1980
   cctgtgttga ggcctccttt ctcatccttc tgcttccgtt ggccccagac ccctctctac 2040
   tggatggccc tggctcctct gccagcctag ccctcatatc tattcctttt tggggagagg 2100
   cctcgaattc ctcctctccc aattggatgg gcttaagatg gggaaccttg tgttacgctg 2160
   acccctgaac cctcacatca tttcccaggt gtcagtgtac cttcagaaag cctgtgcctg 2220
   aggtcctgag ccacatcccc ctggccttcc atgttgctat tctgagattc tctttgcaca 2280
   ccccgttggc tacccagatc cattcaccta cggggacact acttattctt gccagcttcc 2340
   ttcaccaggg agtgagcctg accctagtcc agtatgatga gagtgcctgc ccccagacac 2400
   agaagtcaca gatatgggtg ccagtaggcc ttctgagtat ggctgtacct cctgccaact 2460
   ggggctccaa ggacttcctc ttcaccacag tggcaaacac ttgcctggat gttgattaca 2520
   gatagatagc cagtcccatc tttggatgat tctcactgtc agccatgtct ttctccttag 2580
   cagaggtctc aagcacctct cctctggcat gtcttgctgg gaatatgatc caccatacca 2640
   gatatgtatt catctattca gccatttaac acgtgtttat taagcactta ctatgtacca 2700
   agctttctgc tagatgctgg ggaaaacaac ttacaaaccc ttatttactc ttacatacgt 2760
   attcatatgt acctttaagg aatgagtatt aatttgccag caatgcccta acaaaatacc 2820
   acaaagcatg tttaaacttg gcttaaacaa caaaaaattt atttcgtcac agttctgaag 2880
   gttagaagtc caaaatcaag gtattcgcag ggttgcttcc ttctgaggtc tgtgaggaag 2940
   aatctgtttc agactggaac actacctttc accattaaac aaaaattcac tcaagatgga 3000
   ttaaagattt aaatgtaaga cctcaaacta taaaaatcct aggaaaaaac ctaggaaata 3060
   ctattctgga cattagtctt aggaaagaat ttgtgactaa gtcttcaaca gtgatcgcaa 3120
   caaaaacaaa aattgacaaa tgcagaccta attaaagagc tgcatagcaa aataaattat 3180
   taacagagta aacagacaac ctatagaatg ggagaaaata cttgcaaact atgcatctga 3240
   caaaggtcta atatccagaa tctataagga acttaatgca acaagcaaaa aacaacccca 3300
   ttaaaaagtg ggcaaaagac atgaacagac acttctcaaa agaagacata caagcagcca 3360
   gcaaacataa aaaaatgctc agcatcacta atcatcagag aaaatacaaa tcaaaaccac 3420
   aatgagatac catctcatac cagtcaaatg gctagtatta aaaagtaaaa acaaaacaaa 3480
   acaaaacaaa cagatgctga caaggctctg gagaaaaggg aatgcttata cactgttagt 3540
   gggaatataa attagatttc caccatggaa atcagtttgg agattactca aagcacttag 3600
   aacagaacta ctattcaacc cagaaatctc attcctgggt atatatatcc taaagaaaat 3660
   aaattattct accaaaaaga cacatgcaca tatgatacag aacatatgat gcacctatgt 3720
   tctaccaaaa acaacatatg ttcatcacag cactattcac aaagcaaaga cacggaatca 3780
   acctaggtgc ctatcagtgg gggattggat aaagaaaatg tggtacatat accccataga 3840
   atactatgaa gccataaaaa agtgaaatcc tgtcctttgc agcaacgtgg attcagctgg 3900
   aggccattat cctaagcaat ttaatgcagg aacagaaaac caagtacggc atgctctcat 3960
   ttgtaagtgg gagctaaaca atgggtgcat agggacataa atatggcaac aacagacact 4020
   ggggactact agagggagga gggaggagca ggacaaggac aagggttgaa aaactacctg 4080
   ttgggtaccc acagtgtcac acaatatatg catgtaacaa acctgtacat gtactccctg 4140
   aatctaaaaa gttgaaatta tatatataaa atgaaagtat agaataaatt aataatataa 4200
   tccactgtat aaaaaaaaaa gaaaaaaatc tgtttcaggt tcctcttcta ggattgctga 4260
   tgaccatctt ctccctgtgt ctcttcacgt catctcccct ctatgcattt ctttgtctgt 4320
   gtccaaattc tcactttttt ataaggacac cagtcatact ggattaggag cccaccctac 4380
   ttgtgacctt aacttagtga attacatctg tactgatctt atttccagat aaggccacat 4440
   tctgaggtac tgggggttat atcatttttg ggggacacac attgttcaac ccataacagt 4500
   atgcatcatt ttatggggca gtaatgatag agatgtatat gtacagtgca gacctagtaa 4560
   agggagtaga cacttttttt ttttcagggc agacacatcc tcaagaaagc taatatgaag 4620
   ccacaactat gtttcaggaa ctatttttgt ttattgatca cttattatgt gctaaacacc 4680
   atttagtaat ttacgtgtgt caacacaatc ctatgaagaa gctgagccac agagaggtta 4740
   agtaaacagt cccaatgcac ataggaaggg gcagagtggc agatcaggtg tattttgcag 4800
   gaactgaatt acttatactg tcttccccta cctccctgaa acaccaggct ctgcccattg 4860
   aggatctgtc catagtttaa gaccctggcc ctgacaggga ccagcttaca tggatgatta 4920
   ggcgtgaaag ctgtctgagt caggcaaagc agaagcctca ttgtgccaaa cttcctaatc 4980
   cccttggtca gaacctctgt ccccatgcac ccttaaaata ggcactttcc agtctatttc 5040
   aggtgctgtt tattgtcttc ttggtatctt tcaaacacga ttatcatatt catttggtta 5100
   tctctagagt gtaagcccca tgaagggagg gacttgtgtt gtcttcttta gtgctgcatt 5160
   gccagtgcct agcaatggcc cagagaggca ttccagaaag acttctggta aatggattgt 5220
   gctatatatc agatttcttt ggggtcttct cagaaattag acttcattaa aaaaataagc 5280
   cctaggtgaa aaatgcctct acgaacttgt acattaaatt actgagtaaa aatgaggcac 5340
   ttaaagcatc acagctggtc tctagcccag gcatggaggc cactgaagat gtggctgtct 5400
   gcaccaaagt catattactg aattcattga taattatcct aagtggagca aatagccctc 5460
   atacaactct tgagcatagc tgtaagtttt attttcactt aaagtattga cttcccatcc 5520
   agggatccat catccttttt tttttttttt tttttttttt gtaacagtgg cccactccca 5580
   ttgctttgtg gaaacaggcc acgcgttcaa ggcaaacttg actgtacaga gctcacagtg 5640
   gggtgggtac ttcacggaca ctctctgaac tgctaaggtg gtagagcaaa gcaggtggag 5700
   ttgggagttg gaaggcctgt gtgctcaccc tggcccacag gcagctcata atttatgttg 5760
   agattgggta tcccacttgt aagaaggagt aaataatttc tgccctgttt gcctcacata 5820
   ctattgcagg gttcaattaa gttaatgcac agaaaagcac ttcggactca gaatttacat 5880
   atgtaaagaa taattacctg ggagcaacgt ggcaagaaca ggagtggtgg catctgatag 5940
   acctgggctt aaatcccagt tctactcacc ctgctgtgac tttagttatc tataaatctc 6000
   tttgattttt tgtcatttgt tttatcatct gtaaaatggg gagaacaata ccagccttgc 6060
   atgattatga gagtcaaagg gaataatatt tggaaagttc ttggcacata gtaggtgcag 6120
   tggtaacttt tttgctgctc attttagttt gtcacttaat cagagagtgt cattgtatta 6180
   gttttctgtt attgccataa actatcacac acttagccac ttaaaacaat acaaatttat 6240
   catcatgtat ttataggtca gaagtccagt gggcttggct ggtttctctg gccaggtctc 6300
   acaaagttga aatcaaggtg ttggcaggcc caggggatta tctactttca gacttattca 6360
   aggcattggt agagttgagt tccatgtgat tgtagaattg aggtctccca gctggtggct 6420
   ggggattgtt ttctgtttat agagaccacc cacactactt ggcttgtggt ctcttccccc 6480
   ttcttaaaag ccagcaatgg tgggttttgt ccttctcatt ctttgaatgt ctcctctgtt 6540
   tcccttttct gctgcagctg actcttctgt ctcctctacc cctttaaggg ccccatgatt 6600
   atgttgtgtg taatttcatc tgcaaagcca cgtaagcata ttgtgccaag gtaacacatt 6660
   cacaaattcc aggggataaa aacatgaaca tcttttgctg ggggtggggt ggagcgggat 6720
   tattttttct accacaacca tatgttgata tccgatgttc tcatattatt aataatgtct 6780
   cctcaacaag atctaatatt caaagtactt agctcaatgc ctggcgtaga gtaagcgctt 6840
   ggccaatatt agtttctatt tttgttgttg gtaagcagat gacaataagt atcttgtttt 6900
   tttctacagg ttctagcaaa tccacctggc taagggatgc atcttccttt actactcatt 6960
   gtcaattctc tccagaaagt ctttctgacc ccagtccttg cccaccaggc tggattcagt 7020
   gccagtcctc tgtgttacag catcatagaa cttcccttgt tcagctgtcc attaccctat 7080
   aactctttca ccagattgtg ctttttgagt acagaaactg acatttctgt atcatttagt 7140
   agagtgtctg gtacatagta catgctcaag aaatgcttat tgaataaatg aatgatggga 7200
   aaatttatta tatacttaaa aatttttgat caaataatta ctggttggaa agtatccctt 7260
   aaacaacaca aatattgaaa tattcagtga gaggaaaatc tatgcatatc aatactgtgc 7320
   ttcctgagaa ttatctttac acagaagact tcttgaaaaa taaataaaca ttgtgtttgt 7380
   gaattgtcca ggcctggatc ttgaggagga aggcacttct gaaatagctg caactcccgg 7440
   taagtattgt acttggaatt cactgaatga ttgtgacact ttttttttct gaatgcatta 7500
   gggaacaaac aaagagagta ttgaaataaa gccacatcat cttggcttct catttgcttc 7560
   cttactggga cttattatat ctaacagcaa aactgaaccc tagtgttatt tctcttggaa 7620
   attcacgagg agctcttcct tctgtttttt tttttttttt ttttgtctca cacattgaat 7680
   actcattttc tgtccatttt gtgaagtgtc ttaacaatac tgcaagtagt tagtgaccaa 7740
   taataatcaa ataagagcaa ttttcatagg gccattaata ttgccatgtg tgttttccat 7800
   attttagaag tttctcaatt ttggtttgtc agcctgccca tgtgattcca gatcagccaa 7860
   ggaggccagg ctcctcttgc ttgggccagg atcacttaac agctgttttg agcttggctg 7920
   gtctttttgc aataattgct cccactaagg gactttgatc ttttgatatt tatttttaga 7980
   tacaatatag tcaccattat tttaaaggaa cagttttatt atctgaaaca aacaaccaca 8040
   aatgaaaaat tggagccaaa tcacaggcct caggacccag atgaggaata aagaaaaatt 8100
   gtttatagcc ataaatgctt tctttgcagc tttccttctg tctattgggc tgggtcacat 8160
   tttctaccat cacagaatgg tgattagaca tcagattata tcttgatatt acaatggtac 8220
   tgccctctat tggaagtctg ggaatcctaa tttcctttgg attcagtgct ccattttgaa 8280
   atcacagtcc agctcaggta gcaggctcac atataacatc gaagggttgc aatgaagtaa 8340
   tttctcaatg gctcctctgg ttgaacatgc ttgcacaatt attttaaact cagcaaacat 8400
   ttgttgaaca cctatgatgt gccagacatt gtgcaaggaa ctcaatagat gcagagatgt 8460
   ctaaaacacg gttcgtgatt tcaagaatct tatgacctgg tacaggagac agacttttat 8520
   ataaataaat agaaaggaat ctgacttcat ggagttctgc aattccaagg aattttaagt 8580
   ggtatgtggg cagatgcatg gccaaggaga attaggactt actactggac cagtcattgg 8640
   ctcagtgcct ggcatacggt agatacttca taagtggggt atgcatgatt tggagaaggt 8700
   gatgtaatta ggacggaggg gtctggaagg gctgtacagg aagggatgca gccagagcct 8760
   cactgacatc ttcctctcct gctcatactc tgagcaatcc tagaaccatg gtggatgact 8820
   ttagctgcag cacagtacga gaacagagtg atcaggagtg atgcactgac agagcatcca 8880
   gcaaggcctc tttcattaat aaactattaa taaacacaac agactgatgt gccacacata 8940
   gcagtactat ttgagcctga atatgcctat agggcagcac accttgagtg gacagtgggt 9000
   gctaaagagc catttataca agtaatgtca ctctcttgcc tgtcctagag ttcttgctcc 9060
   attgtaacct ctcttgagga attcactcct ccactgagtc tctgaaaaaa gaaaagcttt 9120
   atctatcaat gagcaagctc tatagcaggg tttataggcc ttccttgacc tgggccctga 9180
   ctaccatctc tatctcctct cccagaactc tcccaccttc ctccaaccga tccaagctgt 9240
   tattagtttc ttctcacact ttcatgcctt tgcactgcct ttccctctaa ttggaatagt 9300
   ctagttttta atctaaagtt tctacttatt ctttaagact tagctcaaag gccacttttt 9360
   tttggaaagc atttctttgc ttttttagac tacaaaaaca tcttctgaat ctccataaca 9420
   ctaggtcatg tgtctgtcct actgcttact gcaattctcc aactatttgg ttatatatct 9480
   gtttccctag aggtcaaata cggtgtccta ttagtcattt tagccccagg gggttaatat 9540
   attatctgga acatagctaa cattttgtga atacttttct aataaacgaa gtggatttgg 9600
   caaaaaactc tgtgttaaga aaggtctagt gaaatatttt tcatctttca tttataagta 9660
   tctataaggt aagataggcc cattgtccac aatctgcatt aatagatctc aataactggc 9720
   aaaggaagaa tgctccccag agaccaggag agagaaaagg ccaagtgggc aaagccaggg 9780
   ccctctttaa cagacaggga ggacagaaag gatgggaacc cttgttcata actgttcctg 9840
   tgaaatatcc cacaaaaagg aatctgtgct agaattattt tgggaaatgc gatttactct 9900
   tttctatttc attatttact catcctatta tttaacaagt atattttaaa tgtctactat 9960
   ttgccataca ttgttctcag cactggaaag atggcagtga acagcatagg tcatggttcc 10020
   tgccctccca gagtttacat tctagtgtga gacacaagcc ataaacaaaa taagaacttg 10080
   aactgggcag tgttttacag aggggtatta agtgctatgg agaaaagaag tgggaaagct 10140
   tttattggaa gtggtgggga gtggagtatg ggaattttga ataagaggtc aaggaagttc 10200
   tcaccaaaag gttgcatctg agtaaagacc tgaaagaaga gaaggagtga ggtaggtgga 10260
   tgtctggcta aagagcaatc aggaaagaag gagctgtgaa agttccatgt ttagaggaac 10320
   agcacagggc ccagtgtatt ttcagcccag gaagggaggg aggtgaagat gggagatgta 10380
   ggtggagaag tcatagaaga gcaatcattt ggggttgtag gtaagaactc tggatttcat 10440
   ctgagtaaaa ggggaaagcc attgcaaggt tttgaagaaa gaggtgatat tacctgttgt 10500
   ggcaagtgag gcagtcaacc taggtgttgg gctgagagta gactatcggt gaagggcaga 10560
   ggcagggaca ccatttggaa ggcttcttaa atggtccagg tgagggaaga tggtggcttg 10620
   ggccagggta gtggagtaaa gtggtcaaat tctggatata ttttgcatgt agaatggata 10680
   ggatttgctg atggatgaaa caggaggcga agaagaaata gaggaatcaa gaatgacatt 10740
   aagatttttg gcctgagtaa cttgaagaat gaagttgaca tgaactcgtg gtgggggaag 10800
   actgagtgag gaggagttga ggggatatca agttaagttt cagacgttta ttagacatct 10860
   cgtggaagac catgaatagg cagtcaagca atcagaggac aggtatgggc aggagatgta 10920
   aattgaacag tccttatcat gaagacagta tttaaagcca ggagactagg agaggtcacc 10980
   cagagtatga aagtaggcca ggaagagaag agaagagaag agaaccaaga actgaactct 11040
   gagatggatt tagagtccca gctcttgaat tctcatctct gacagtcctt gaagaaaaga 11100
   aacaggttta acttggcttt tcccaatgta tcggatgatg gaacaccctt tcccctatag 11160
   aactgtggaa tttatgttcc actgaacact ttttggagaa ggccactcta ggcttggaag 11220
   ttatcctagg gccataaggg aaagcctttt ttatcctcta ctaagaagtt tggactttgt 11280
   ctcccaggtg ttggtagttc accagtgaat tttttttttc ttcaacacaa gaatagtggt 11340
   gttctttatt tacctttgca tatttttttt cttctctgaa tttttttatt atactttaag 11400
   ttctggggta catgtgcaga atgtgcagtt ttgttacata ggtatacacg tgccatagtg 11460
   gtttgctgca cccatcaacc catcatctac attaggtgtt tctcctaatg ctgtccctcc 11520
   cctagccccc aaccccccaa caggccccca gtgtgtgatg ttcccctccc tgtgtccata 11580
   tgttctcatt tttcaactcc cacttacgag tgagagcatg tgatgtttgg ttttctgttc 11640
   atggatggat tttaagagaa gtaacaggat ttacatattt tggtggcagt atggaaagtg 11700
   gcttgtagag gggagtctga tggaataaga caagagaata gctgactgca gtagccttgg 11760
   tgacagatga tgaagccctg aacatagcga ctaggaatgg atggaagaat cagattaaat 11820
   gggtatttag gatgtttaga atcattaaca tttgatgacc aattagatat agtggatgat 11880
   gaaaaatatt caatgataat cctgagagtt ctgtcttggg tcagattgtc tatatttacc 11940
   tgtatccctt agagagatct atcaatgcaa ggtacaaagc aacattccat gtaattagct 12000
   tttattttta actctgttca agttatattt taacagagga aattgtgaat tgatttaatg 12060
   cccaatgttg ccttctattt ttgtcattca taaagccatg ttagtttttt tattaataca 12120
   aaaatacttt aaaatggtat ttacttgagt aagcatgatg aatcgtttga atgtgcagag 12180
   gattatgatg ttatttttta aacactaata gtcatatttt gggattggtg tgttctaatc 12240
   ttttacatgc agagtacaga attaaaaaga aaacagatta tactgactag aaaagtgttt 12300
   ctagaaggtg gtttaaggac cttgtataga ttgggccttg ttctccaaca ctgggaaaag 12360
   gtgggagaaa tttccaacat tgcttcattg cttatctgga tttttgtatg tttagaatgg 12420
   gactaagaaa tgctggaatt tctctgaata ctccaaggac tctggaagga aagtacattt 12480
   ctataatgtg gctgtggtat gtgcacacct tttgcctttt aaaacagcct tttgccggca 12540
   ttaatggcat ctcctgaata tcttgggttt gaagctgtga attgagaagc agaatcaaat 12600
   tgtttagggt catgtgaata tcacatcttt actactaata aagcacagag gaaagacctt 12660
   cagttggaca tttaaataat tcctaagaca aaatcaatgc ttttttggta gtaagtattt 12720
   gtcctattca caattttttt tcttgttttc tttcctctag gcacattgta gggttgaact 12780
   tccctgtttg cattacatag gactggtcat ctgactggct gtggccagtg aaaatgtaga 12840
   tgaaaatagc ctgtatcacc tctggacgag tcattaggag tcagtgtatg atttgataca 12900
   tttccttctc tctgccatgg tgattggtga tggaggctct attagttttt gttcctgagt 12960
   aaagtaatgt gggtatgagt ccctagctga cttccaatac gagtgagaaa taaaccttaa 13020
   ttgttttaag cctccaagct ttggagtttg ttgtacagcc caccatgaca aatatacttt 13080
   tagtccttta tttacatgct ttaaaatact gcttgcattt tctgattacc atatgttcat 13140
   tgtggagaat ttcaaaaaat ataaaaaaaa acctttgaaa tcacttacat agaaatagtc 13200
   tctgttaata ttttgatgta ttgtcttcct ctcccaattt aaaaaatcag gatcctaata 13260
   tatacgatat tgattttttc acttcatata tactcttaaa atacatgaat ttgctgcata 13320
   ttattaattg atgaggaagt gttataatct atttttatat ttatgtttta tatttatact 13380
   atacaattca taagagtcac attttatttt tgtacttttt cttgttgtaa ataacattcc 13440
   aatgaaactt ttgtgattaa gtctttgctc aaattgctgc tgctgtcctt ggagtagatc 13500
   cctagaaggt gaacgactag gtcaaggagt atgcatactt ttaagatttt tgctacctat 13560
   tgccaggaga aaggttatac caattaacac gtcagctgtg tgagatgggc catctcacca 13620
   gtctgttgag tattagaatt ccttttaatc tttgttaatt tgataggtga aaacgctatc 13680
   ttgttttata tttgcatttc tttgattaca agtgttgttg aacatttatt tcgtatgttt 13740
   atgagtcatt tgcacttttt tcttgtggga attatctaaa tggattctca caccccccaa 13800
   acaacaagct aataaacgga aagccacaca ggaagccggc agtactgttg ccacggagcc 13860
   tctgcacgag cttttgaagt gtcagcgttg aacaggttcc tctaactccc tctcatctgc 13920
   cttgttttga ttattttctc tccagctgta tccctccaca gtcca 13965
<210> 18
   <211> 2218
   <212> DNA
   <213> Homo sapiens
<400> 18
   ttctttttgt ttgctgcctt ccgtagaaga tgtggcttgc tcatgcttga cttctgccat      60
   ggttgtgagg cctccccagc catgtggaac tgttttcagg tgctggttcc atggctcttc     120
   ctgagccgaa aataaggaaa ctccatagac cttgtccact ggaactcgtt cccatctacc     180
   ctccactcta tccagggtga tggatctctg cagtaagtgg aagagttctt catggccccc     240
   aaggttatat ccatctagaa cttcagcacg taatttcatc tggaaatagt gcctttgcgg     300
   atataagtta ggtaaaactg aagatgagat catactggat taggatggga tctaaatcca     360
   atgaaaatgt cttcataaaa aacaggaaag aacccataga aacacaagga agaaggtcat     420
   gtgaagatgg aggcagagat tggagggatg cagccaccgg cccaggaatg ccagcagcca     480
   cccagaagct ggaaggaaat gagggattct ctcctagaac ctttagagag aacatggtcc     540
   tgtgaacagc ttgattttgg acttgcccat agcttgtata ctcttacttt ggatacaatt     600
   ttatccaaac ttggctaaac agtttctcag cctatggaaa atttaaaatg gagaagattc     660
   aactcgattc ttacagattc aaagcaagaa aatgatggga acataggagg agaccaagaa 720
   agcctataaa aagcaaaaat atgaagtgaa cattgtggta gctttaagat gtttagtgta 780
   gctgcaggca ccctatacac atgaaaaccc ccaaggggaa tccccatatc acagtgtagt 840
   gtgatatttg acattcgtga tcatctagag atgtacagaa aaggtgaatc tgtgttctgt 900
   atattctgcc taaggcaaag aaatgtttag ctctctttaa aatagttcca taattttttc 960
   taaaaagctt tgcttgaaaa ctgtaagctt cccatatctg gagcatttca ctttaaatat 1020
   ttggataaat atgttatctt cttacttgga catttcatgt gtttagggat tgtcttctaa 1080
   attcttccta attcatatag ctgctaacac ttcccgcaga gctaaaccat tacagatatg 1140
   aaataaagac ctattgattt gacttacttt tacttgtaaa accttctgag tgttataacc 1200
   tcatttaatc tttcagcatt tacagtttca agagtttgtg tcacaattag aagaattcag 1260
   ctgcacctcc aagtgacagc agttgctctg gttggtgggt gatctcagga ggcttgagaa 1320
   ttgctttgtc tgtgagggaa gtaagacatt ttcagagccc cactttagaa ggtgtgaact 1380
   ggctagataa tgaaccccag ggctaacgct gctatagcag tgggaaggag gtgatgggtt 1440
   ttcagtttgg acctcaaaca tcaataccct cctggtacgg ggaggaacag agtccctcct 1500
   ttacttctcc attagaaaga atgagatggc aagacaatga aacaggcaaa gtgaacagag 1560
   atgcaagaca aaattcaggt gagagagcca gagcatcact cagccattcc tgacatgtaa 1620
   aacaggcaac tagaaatttg cagaaaggaa gcgaagtctc cataaagatg tttttaaagt 1680
   gagcttgaag tatttggaga caattcagtg ttacataaaa tctgcaaatc tctggataaa 1740
   gaagcagaga tcccagcatg ggacaaatgg agcctcaaaa gtgggaagaa gacagagaag 1800
   accagggcag aatgcatctc ttcctttctc ttggctttcc tggataagga ctgcatcatt 1860
   cctgtggaag gacaggccat cagctccgaa acactgtatg tattttccag tatatactgc 1920
   tagctgtgtg atgttgggaa aatttgttac cctgtctaac ccccacttcc ctcatctgta 1980
   aaatggaaat aatgatagta cctacctatc tcataggtgg caactacaag gggcagcaca 2040
   ctcagggaat taaggaagtt tcagtgaaca tcaactttat gaacacagtg ttcataaagg 2100
   caggtcagtg cagtggtttg ggagccagga gaagcacgtg ggccggagtg tgcctgcagg 2160
   agacaaggtc agagatgttg ctaataatgg agaataaagg atgcattctc attactga 2218
<210> 19
   <211> 465
   <212> DNA
   <213> Homo sapiens
<400> 19
   aggggtttcc gcttttgctt cttcctcatt ttctcttgct gctgccattt tcgcctcccg      60
   ccatgattct gaggcctccc cagctatgtg gaactgtaag tccagttaaa cctcgttttc     120
   ttcccagtct cagggtgatg gatctctgca gtaagtggaa gagttcttca tggcccccaa     180
   ggttatatcc atctagaact tcagcacgta atttcatctg gaaatagtgc ctttgcggat     240
   ataagttagg taaaactgaa gatgagatca tactggatta ggatgggatc taaatccaat     300
   gaaaatgtct tcataaaaaa caggaaagaa cccatagaaa cacaaggaag aaggtcatgt     360
   gaagatggag gcagagattg gagggatgca gccaccggcc caggaatgcc agcagccacc     420
   cagaagctgg aaggaaatga gggattctct cctagaacct ttaga                     465
<210> 20
   <211> 555
   <212> DNA
   <213> Homo sapiens
<400> 20
   aaaagactat ctcttcccat tgaattaaat tggaactttg gaatcttaat agaaaaccaa      60
   ctgacttggc ttggttttca ggtgctggtt ccatggctct tcctgagccg aaaataagga     120
   aactccatag accttgtcca ctggaactcg ttcccatcta ccctccactc tatccagccc     180
   catgaggaca aggaacatga ttggttttgc tcactgccgt atcttccgta cctagtacgt     240
   aacaggacat caataaatat tagttgaatg gaagattaaa tcaacaaaat gggtgatgga     300
   tctctgcagt aagtggaaga gttcttcatg gcccccaagg ttatatccat ctagaacttc     360
   agcacgtaat ttcatctgga aatagtgcct ttgcggatat aagttaggta aaactgaaga     420
   tgagatcata ctggattagg atgggatcta aatccaatga aaatgtcttc ataaaaaaca     480
   ggaaagaacc catagaaaca caaggaagaa ggtcatgtga agatggaggc agagattgga     540
   gggatgcagc caccg                                                      555
<210> 21
   <211> 501
   <212> DNA
   <213> Homo sapiens
<400> 21
   cttgctcatg cttgacttct gccatggttg tgaggcctcc ccagccatgt ggaactgttt 60
   tcaggtgctg gttccatggc tcttcctgag ccgaaaataa ggaaactcca tagaccttgt 120
   ccactggaac tcgttcccat ctaccctcca ctctatccag ccccatgagg acaaggaaca 180
   tgattggttt tgctcactgc cgtatcttcc gtacctagta cgtaacagga catcaataaa 240
   tattagttga atggaagatt aaatcaacaa aatgggtgat ggatctctgc agtaagtgga 300
   agagttcttc atggccccca aggttatatc catctagaac ttcagcacgt aatttcatct 360
   ggaaatagtg cctttgcgga tataagttag gtaaaactga agatgagatc atactggatt 420
   aggatgggat ctaaatccaa tgaaaatgtc ttcataaaaa acaggaaaga acccatagaa 480
   acacaaggaa gaaggtcatg t 501
<210> 22
   <211> 10817
   <212> DNA
   <213> Homo sapiens
<400> 22
   ctctttctct ccttctccct tccttcctcc ctccctccct ctcttcctct cttttctttc 60
   tttctttctc tttctttctt tctttctttc tttctttctt tctttctttc ttttcttcct 120
   ttctctctct ctctctttct tttcttttct ttgctttcct ttccttttct tttccttttt 180
   tctttccttc ccctacgtgg caaatccagg gttgggctag ccaagaaaag tagtaactta 240
   gcaggtctac cactgacccc taggagatcc aaatatgtta ttttatatat ctttaagtac 300
   agggatcact tgcaaaaact atcctgatat tattaacttt tatggagaaa tcctaacaca 360
   agtctatttt acaataaggt gttgtatatc acatgtaatt tattgactac tgcagtgaaa 420
   gtgaaaaact gaatggctgt gtgaatactc aaagtatggt ttctactgag catgtatcac 480
   tttcacatca ttgtaaagct gaaaaatcac taagttaaac catcataagc tggggactgt 540
   ctgtatattc tcattgaata gttttattgt attactattt tataaaacta atatattttt 600
   ctccaaataa gtgactttac aaagatgaat tttgcattcc aaagtatgtt atatgtttta 660
   tttattagca aaagtatgaa ctttggagtc aggtagattt gaattaaatt ccaattatac 720
   tagaaagctc tatgactttg gtcagttata gtaagaattc ccaatttata gtaattttat 780
   aagcataatt tttggcactt agaaaatgct caataaatat tgattctttg tgatatgatg 840
   ataattaatg atacctataa taacaaaggt gacgatggtg agagactaca cttctctctt 900
   ccatttacct tcagttataa tgaaaggtaa tgagtaaata tgactaagta gtgaatatat 960
   ataaagtatc agaaatattt ctatttaata caatgcatca aatggtatag tacatatatt 1020
   ttatctgaaa taaaagcctg aagtgaacaa tttggcccta accatataat ttaaactaat 1080
   cactattttg taatttttac taatttagat gttgtagaag gcaacatatt tctgagaatt 1140
   ttttcttttg atttaagaaa aatgctaaca tgagaaaaat aattcttact tgattttaat 1200
   gatttaatta taattttttg ataacttttt tatctaagat gtgagcagct atgttttact 1260
   cttagttttg aattctttgt tagtttccta tttacatagt tggctttctt atattatact 1320
   aatatttata atcatatact gcagagattg ggatggactc aagtactcca aattttacag 1380
   gaatgaagtc tctcccattt tggtaaaatg gcttttgatt gttatttaat acaattcaca 1440
   cttgctggga tttagaagtt ttgaaatcct catttcagtg acttctataa gtttttcaac 1500
   tgatgcccaa agattagtgt tcctttgcct ccattcattt aaccaaggtg tagaacaggg 1560
   taggccagag ctgaaggaaa gacagcgctg gagatgatgc tctgaatggt gcttgctgga 1620
   gccacagata atctgaggat gctgcctttc ctgcttccca ggagctgagc tagtggaaaa 1680
   aagggaataa gaatgtaatt tttcttcaga tttaaaatgt ctgataacaa aagttgatta 1740
   ccctgtttta tttggagaga aatccaggtg tagagaagat attatacttc tctttgagac 1800
   taagtacctt taaaatagtt ttagatatct ggatttaatt ccatatgttt caatttttct 1860
   atatttatat gatagataca tttaaataaa gcatgctcct ctatatttcg ctcaggattt 1920
   tggacagagc tgataaattc tttcactcat ttactcaaca tacctttatt aaatgcttac 1980
   ttctgcaggt atttatactt aaaatcataa tttacttcat aaaatttaaa atctaacttt 2040
   tggagtcctc aaattgatat ggtcttatcc atttattggt aaaatgctgc attcgataca 2100
   acatacaaag tggatgcctg tggcagagca gttacttgcc attgtctctt tttttttgtt 2160
   ttctctccca gaggaaactt caggtttggg ctgtttcatt cagacccctt atgaccaggg 2220
   tgtacatttc ccagactaca tgtatgaaac tcatggccat ggaatagaac aaacacagtt 2280
   gctccataaa gtctttttaa atacggtgtt ccaaccaact gagctaaaca atctcagact 2340
   ttgaaaagaa tatgttcttt gcttagaaga ggagcaaact caacagattt aacaaatttt 2400
   aatatctcac agatttaaaa aacaaagaat ttcaaggtta accgggaaac tacaatattt 2460
   ttcttgaggc tgtttaatct gagcctgcac ttcagaagcg attttatgtt ttaaatttta 2520
   gggttctgac ctgttttcct ttctcaaaac aaacatgcta agagtttaaa atcaaccatg 2580
   atcattgcta gtaaatatgc actcagacct gacctggctg tgcaaataaa gtccattctc 2640
   catggtgatc agaagcagca atgtctcact atggaaggtg gctagctgca tttacagact 2700
   gtacaatcta attcctcctt ctgtaaatgt tgattaagct gaaagaggat cgttggcaaa 2760
   gctaattcaa tagcaccgtc tcctctcctg aaaggctgga ggatgatctc actatcacat 2820
   gagtccatta gctatccctg cagcaaaact caccatctgg ctaactggct tagactgtgc 2880
   tgattggact gcaggctttt ctagcactct cttgtgatgg tgagtgtgag agtaagaagc 2940
   tgggaggggc agctggacaa cataagactg ctgttatctc ttagggagct aggtgaaccc 3000
   taagtgctgc ctgatagcaa caggacctgc ctggcactga ttgtctattt cttagcattg 3060
   gtaacagaag acatggtcat ttatgatcaa tatgatggag ttgacaaatt aaacaaacaa 3120
   acaagcaaac tcttccttct aaccctgcaa tctttgactt ttgatcctgt cattagataa 3180
   ctgatgtagg aaagtcactg aagcatcact ttaagtagca gtggatgaag cattttcaaa 3240
   ataacaaccg gcctaagtca aaaatcaaat cgatgtttgc tccattattg ttctcttatg 3300
   tcagttctga tggtagttcc tgttttagcc tctcttggaa ttatagagtt agaattgtga 3360
   ctgaataatt gggaataaga gggaaaattg agtattctgc tgcctttgtt gaatctcact 3420
   ccgggttaat tgctgctaat tatgcagctg cttattggtg catttctatc cacttctgcg 3480
   tctgaacaca gaaggaagac gtagggagga tccaaagtga gactccaaca aaacagctga 3540
   tcataacctg catgtaaaat tgagaatttg ttttcttttg cttttatttt acattttttt 3600
   taaaattatg gttcaataac agaatataat cggttatcaa aagcaatgac attctgttgt 3660
   cagcatctga gagttgttgg tatgatcact gttgggtgga atgtttaagt attattccta 3720
   cagcaaataa gaaaagtgac tctaattact gaagatatga aataaaagat atgaaaagca 3780
   agattgagaa agaccttctt tactggtcct tattagtgtt aaaaaaggcc acactaacaa 3840
   atcagggact gaatatcatg ttgttcagcc atataggttt tgaatgccat gtggtggtta 3900
   gcttactttg acaggagcag tacagtctta ctcgtttggc aaaataacaa atgttcccat 3960
   tattttggat tatatattta tttatgtgat tttgcatatc aagtgcacaa aaattaagct 4020
   tatggtaaaa aaagtaccat taattagtga ctctactgta attgaatcag tggtcattgt 4080
   aatgtacctt aaagtcacta tggagtttta agacattaat ttacaacaag aagagaaaag 4140
   gactaggaac ataaacatgt ctgcacatag atggggaggg gaggctaaat tgagtgaatc 4200
   cctccaattt tttcactttc tcctcactct gaccttactc caacatggcc tcaggctcta 4260
   gatttctacc agatcacatt acagaggcat gtgatgagaa ggaaccactt gagtcttttt 4320
   tttttttcca gaaccaagcc aagtaattca gtcatgcaaa agtttacgca attgacaata 4380
   ttcactcatg tccccctgtt tacaatttta aggtgtgtaa aggccagtca caagcaagga 4440
   cggtcagcat tgctttggtg tagatggaac acacggagac gatggaactg cagggcgctc 4500
   acagacagat agttgaccaa gagtgaaggg cagtagctaa aactgagtgc ctataaggtc 4560
   aaggaatgtg acataaaaga gatctgtgga tgtggcacaa ccagtcaagg ccaattttgg 4620
   ggaagatgta aattttaaat gaggtttcat aggaagattg gcgatctaag tagggaaaaa 4680
   tgttttccta aagcgaataa gcatggcgtg acaactatat gttgtgttat aagtttttct 4740
   attcacgaac ttttcttttc ttttcttttt tattcttctg ttatacagaa gttcccaacc 4800
   tccaagccat gaactagaac tagtctgtgg cttgtgggga actgggctgc acagcaggca 4860
   gcgagcaggt gagtgagcag agattatggc ctgagctctg cctcctgtca gatcagtggc 4920
   agcatgagat tctcatagga gcccaaatcg tattgtgagc tacgcatgcg agggatctag 4980
   gttgcgtgct acttttgaga atctaactaa tgcctgatga cctgaagtgg aacaatttca 5040
   tcctaaaact gccttcccac aactggccca tggaaaaatt atcttccaca aaactggtcc 5100
   ctgatgccaa aaagattggg gactgctgct gtcatataag gataattatc ctcattttac 5160
   agaggaggaa attgaaagtc agatgtacag tgactaaaat agctaaaagt gatgaaaatt 5220
   agagctgagg tgttggtcct tacagacctt ctacttctgt tgcaccccag ggctctgtcc 5280
   tggtaacata caaacagagg cagtgccaac actggagggt ggaaggctca ggccttcatt 5340
   ctgaccccaa ggcacccagt accttccata gtgcttgctg tcatccctac tactattgtc 5400
   tgctcactac ttctctactg gtcacagaga aagccataaa aagatgtttt catactcatg 5460
   agttccccca tgcaggcact gatagaactt gataagttga ttcatgctct gtagttctct 5520
   tcacagcaag cagctcgtct ccctctcagc tttcaccttc aacgccctgc acagcaagcc 5580
   tgtggcagct ctgctcctca gctgtcagac aaaaggggag aacaaagcaa gactgtgatg 5640
   caatactcat gttaggtaaa tctttcccca gctagtactt tctgtcctgg gattattttg 5700
   tgaacacaga tttcgttaga gagcaattga tctgaacatc tgggggctca ttagctcctt 5760
   ctaattgcta taatgtggtt ccattctgat ttcatgcaag gggtgcagtc ttcatgcaca 5820
   aactattgat taattacaca tttaaggcat ttgagaatat cagctgagtg catttgtggt 5880
   ttcctaggat ttctccttta ttttccctgt ctgaactttt ccttgttcct tcgcctcccc 5940
   cttacctctt gccatgccaa tcagtctcta aagcattcac tttataaaca gaaagtcaaa 6000
   atggtgcttt actatttatc tggtaactca tattggaatt tggaagagtg gctgatgtga 6060
   acaatggctc ataagaacat taatttttct tctgtactga atagtctcat tgaaacagat 6120
   ttggatatga ggatgatgct tacataataa atacccaagc tggttgggtt agcagattgt 6180
   ttacatgtgg gcacatgtgt gtggcttgtt gctgcatggt tgcccattcc accgggagaa 6240
   gctctgttgt tatgtgtgtt atgaaatttc aaataaggga agaacaacag ctgtaatggg 6300
   cccaatccaa tgatggcaaa aggtgaataa aattactccc tctgggaagt aaaatgctca 6360
   tttagtggag ggaaattatc atttccaagt agaacatttg gcttcctgag agtgaaagtg 6420
   tcggcacagc ttgataataa cacctcaggc tgtaatagta ccctttcccc tctgagagct 6480
   aggctttttc cctctaaatt tatctctaca acgtctttgc aaagtgagta ggcacagttg 6540
   ttattggcca tattttatag atagagaaac tgagagtcta aaaaactcag tgatttgtcc 6600
   ttggaagccc agaaatcaat ctagataagg gtttctcagc cttggtgcca ctgatatttt 6660
   tgaaccggta attcttggct gtggtggttg tcctgtgcac tgcagaatat tagcagcatc 6720
   cactagatgc cagtaatgcc cccagttgtg actaccaaag atgtctccag acactgtcag 6780
   atgtcccttg ggaggcaaaa tcacccacag ttcagaaaca gtgatctaga ttggtgggcc 6840
   cggacggtct tgtagccttt aggaaacctg aagcaaaatt tggaaaaata aaaatttgcc 6900
   cttgacatcc atagtttctc ccatctgtaa taacagtaca tatgtaggat tcattattat 6960
   gaagtcttta cacttgtcct gcattattca tggctttaca tcctgcattt tcagttaccg 7020
   ttggtcaacc aaagtctgaa aataataaat ggaaaattct agaaataaaa attcataaga 7080
   tttaaattgc ctgtcatcat gagtagtgtg atgaattctc aagtgtcctg cttcatctct 7140
   cctgggatgt gaattcttcc ttgtccagga tatccacact gtctaggctc cccgtttgtt 7200
   catcacttaa tcattgtgtt cgtcatcaga tcaactgtaa tggtattgca gtgctggtgc 7260
   caagtaaccc tgattttact taataatggc cccaaagctc aagatcagtg atgcttacag 7320
   ttcagatatg ccaaggagaa gctctaaagt gcttccttta agtgaaaaat tgaaagttct 7380
   tgatttaata agggaaggaa aacaatcata tattgagatt actaagatct atggtaagaa 7440
   tgaatcttct ctctatgaaa ttgtgagaaa aaaacaaaag gatgtgtata tagtatctat 7500
   aaagttcagt actatcctca gtttcaggca tccactgggg ttcttggaac atatcccatt 7560
   tggataagga gagactactg aatatacctt aacttcaatt ctattttaat cacatttttc 7620
   acataggaaa acagaaccac aaaaaggtta gctcacttgt tcaaatttat acagctagga 7680
   atcagggttg gcttcatgag cattttgacc taagcagcta cacaggatcc catgtttaga 7740
   agggtgtcat acttggttta atgatcttgc tgtccttata ttgaaactat taattatttt 7800
   tgaacaagag tttcacattt tcattttgtt ttgagctcca caaatatgta gctagtcctg 7860
   ctaggaggca aaatagccag gattccagtc cagggagttt aaagacaata tgtttaacct 7920
   tttaaccata cagcccctca ttctttcaca tgtttgttca tccatttact tttcagtaaa 7980
   ttgtgctgtg agttcattat aaaccaggca ctagacttgg cagtgatgga acaatagagg 8040
   caaaacagat gctgtccctt ctcttatgaa gctagcagcc tcaggaagga tgcaggccaa 8100
   caaacaggta tttgcaattc aacatggtat gtgttatgat aggaaaggtc tggttgctat 8160
   gggagcatac atataggagg gcacttaaca cagtatgggg ggattaggat ggtgtacaag 8220
   ctgaggcata agggctgaat aagagttaca taatcaaaac tggagaatga gtatgatcta 8280
   agctaaagga aatagaagta taaatgctta gaactgagga ggagtgtgac acaattgggg 8340
   aattaaaaat agcttagtgt gaatggaata ttgaattagt cagctttcac tgttgtggtt 8400
   cagtaacagc cccaccgtgt cagtagatta ttacaataaa ggcccacatc tcactcttgt 8460
   tgctggtgat gtgctattgg gttgtgctgc ttctccccaa gtctccattt caggactaag 8520
   ccaggaagag cagttcctaa atgaaaatgc cattcttgtg atagaagaaa aagaaagaag 8580
   tagctgaacc acacacaata gctgaagctc tttggctcat attccattgg ttaatcaagc 8640
   atgtggccat ggtcatcatg ggtagagcag ggaagtatcc tgctctccta gggggcacta 8700
   caagtcacat agcaatgggc agatatgtta tagttctctt aaagggagag cagagactaa 8760
   ctaggaacag taatatattc taccaggtaa gaatgagagg aggtgagtgg tatgagaagg 8820
   tttttggaaa taattaggaa ctaggttatg tgttttatag aatatattaa agcctttagg 8880
   tgcaattcat tactcctttc cattgtgtac tcattatttg gtcctacgcg tcctactcaa 8940
   aatctgcttt tggaaaacaa taataataac aacagtgaca agcaaaacaa aaaccaagaa 9000
   agataatgtc attacacact aaagtacaaa ttatccttta tacagtactt atatctttac 9060
   agaaacaaag gaaataatca gttgtagagt ggcagtcact aatgtactag tgaagggtga 9120
   attttgattc catctatgga aaaagatatt tcttggggtt ttggaaatca taccttgctg 9180
   acttttaagt attgccctac tattaagacc aatccataaa tatttactga ttgcattcta 9240
   cactttaaga actgtgattg ctgaattctc atcttcaaaa ttcagtcaag atgaatgaaa 9300
   ggcagcctga tagagtgaaa aaagcatgaa tcctgtcctc cggcagacct gcatttgacc 9360
   aaattaattt agttaatacc tgtatgaatt tgggcaagtt atctaacatc tttcaccttg 9420
   gtcttctcat ttgtaaaatg ggattataat accttcgtca gaaccacaga tccaacaaga 9480
   acagcaactt ttattggata ctgcttaaat tccaggcaca ggcacagggc taagaacttt 9540
   tcattatgac tcttaaactt tgtcaaacaa tgaagtgaag actataattt tacagaggag 9600
   gtaactggga attaaggtgt taagcaactt gcccaaggtc agacatttag caaatcatgg 9660
   aacctgaaag taagcccagc cacacaatga ccgaagttga actctttatt actgggtaat 9720
   aagaacagta atgaaaacaa gaataataac aaaccacaca gtattccaaa ctctttaaaa 9780
   tattagatta tttaattctc agtgataccc tttcagaaca tgcaagtgat atagaatctc 9840
   aggttcaaca ctctgtatat atataaccat atatgcatat atctattaat ctatgtatat 9900
   ctctatatta attaagcatt ttttctgttc ttggtctgtt acaataagtg aggtaaagta 9960
   ggctgttcaa tttattatac attaccatat tcaatggaaa atgctaacct ccaagttaga 10020
   gaaaacttga tagaaaactt gttaacattg tttataagat gcttaaataa gctttgagtg 10080
   aggatgtaag accctcacac tcaaatagaa ggtgaaaact ataccaaatg gactttttca 10140
   tgtattttga attcagaggc ctctttttac tccctgtgga ttctgattat atttgagata 10200
   taatcattag gttcaatgtc ccataaatat gtgaattatt aatataaatt attttcccaa 10260
   tatttttctg gctttctttt tgtgtttttc tttccagggc ccaagttccc aaatatgttt 10320
   ccttttgccc ttttattaga ttatcatcaa tagtaggtag tcattggctt tatttctttt 10380
   cattcccagg ctcatccaac aaactgagga atgtcctata ttttgcctct cattgtctga 10440
   gatgatcttt gtgtggtttc ttatttatgt atattttttt ccctccaaaa tcatatgaaa 10500
   agaaattgca agttgaaaac attacttctg gaaatgctat tcatccttca gcacaaaatt 10560
   tgactgtgat tttctaaata aaacaaggct ttttttaaaa ttatacttta agttttaggg 10620
   tacatgtgca caatgtgcag gtttgttaca tatgtataca tgtgccatgt tggtgtgctg 10680
   cacccattaa ctcgtcattt agcattaggt atatctccaa atgctatccc tccccccttc 10740
   ccccacccca caacagtccc cagagtgtga tgttcccttt cctgtgtcca tgtgttctcg 10800
   ttgttcaatt cccacct 10817
<210> 23
   <211> 18379
   <212> DNA
   <213> Homo sapiens
<400> 23
   aaacatacgt gtgcatgtgt ctttatggca gcatgattta taatcctttg gggatatact 60
   cagtaatggg atggctgggt caaatggtat ttctagttct agatccctga ggaatcacca 120
   cactgacttc cacaatggtt gaactagttt acagacacac aaacagtgta aaagtgtccc 180
   tatttctcca catcctctcc ggcacctgtt gtttcctgac tttttaatga tcgccattct 240
   aactggtgtg agatggtatc tcattgtggt tttgatttgc atttctttga tggccagtga 300
   tgatgagtat tttttaatgt gttttttggc tgcataaatg tcttcttttg agaagtgtct 360
   gttcatatcc tttgcccact ttttgatggg gttgtttgtt tttttcttgt aaatttgttt 420
   gagttcattg tagattctgg atattagccc tttgtcagat gagtaggttg cgaaaagttt 480
   ctcccatttt gcaggttgcc tgttcactct gatggtagtt tttttgctgt gcagaagctc 540
   tttagtttaa ttagattcca tttgtcaatt ttgtcttttg ttgccattgc ttttggtgtt 600
   ttagacatga agtccttgcc catgcctatg acctgaatgg tattgcctag gttttcttct 660
   agggttttta tggttttagg tctaacatgt aagtctttaa tccatctcga attaattttt 720
   gtaaaagttg taaggaaggg atccagtttc agctttctac atatggctag ccagttttcc 780
   cagcaccatt tatgaaatag ggaatccttt ccccattgct tgtttttctc aggtttgtcg 840
   aagatcagat agttgtagat atgcggcatt atttctgagg gctctgttct gttccattgg 900
   tacataacga aatgaaggca gaaataaaga tgttctttga aaccaatgag aacaaagaca 960
   caacatacca gaatctctgg gacacattca aagcagtgtg tagagggaaa tttatagcac 1020
   taaatgccca caagagaaag caggaaggat ccaaaattga cactctaatg tcacaattaa 1080
   aagaactaga aaagcaagag caaacacatt caaaagctag cagaaggcaa gaaataacta 1140
   aaatcagagc agaactgaag gaaatagaga cacaaaaaac ccttcaaaaa attaatgaat 1200
   ccaggagctg gttttttgaa aagatcaaca aagttgatag actgctagca agactaataa 1260
   agaagaaaag agaagaatca aatagatgca ataaaaaata ataaaggggc tatcaccacc 1320
   gatcccacag aaatacaaac taccatcaga gaatactaca aacacctcta tgcaaataaa 1380
   ctagaaaatc tagaagaaat ggataaattc cttgacacat acaccctccc aagactaaac 1440
   caggaagaag ttgaatctct gaatagacca ataacaggct ctgaaattgt ggcaataatc 1500
   gatagcttac caaccaaaaa gagtccagga ccagatggac tcacagccga attctaccag 1560
   aggtacaagg aggagctggt accattcctt ctgaaattat tccaatcaat agaaaaagag 1620
   ggaatcctcc ctaactcatt ttatgaggcc agcatcatcc tgataccaaa gcctggcaga 1680
   gacaaccaaa aaagagaatt ttagaccaat atccttgatg aacattcatg caaaaatcct 1740
   caataaaata ctggcaaatc aaatccagca gcagatcaaa aagtttatcc accatgatca 1800
   agtgggcttc atccctgggt tgcaaggctg gttcaatata cacaaatcaa taattgtaat 1860
   ccagcatata aacagaacca aagacaaaaa ccacatgatt atctcaatag atgcagaaaa 1920
   ggcctttgac aaaattcaac aacccttcat gctaaaaact ctcaataaat taggtattga 1980
   tgggacgtat ctcaaaataa taagagctat ctgtgacaaa cccacagcca atatcatact 2040
   gaatgggcaa aaactggaag cattcccttt gaaaactggc acaagacagg gatgccctct 2100
   ctcaccactc ctattcaaca tagtgttgga agttctggcc agggcaatta ggcaggagaa 2160
   ggaaataaag ggtattcaat taggaaaaga ggaagtcaaa ttctccctgt ttgcagatga 2220
   caagattgta tatctacaaa accccgttgt ctcagcccaa aatctcctta agctgataag 2280
   caacttcagc aaagtctcag gatacaaaat caatgtacaa aaatcacaag cattcttata 2340
   caccaataac agacaaacag agagccaaat catgagtgaa ctcccattca caattgcttc 2400
   aaagagaata aaatacctag gaatccaact tacaagggat gtgaaggacc tcttcaagga 2460
   gaactacaaa ccactgctca atgaaataaa agagcataca aacaaatgga agaacattcc 2520
   atgctcatgg gttagaagaa tcaatattgt gaaaatggcc atactgccca aggtaattta 2580
   tagattcaat atcatcccca tcaagctacc aatgactttc ttcacagagt tggaaaaaac 2640
   tactttaaag ttcatatgga accaaaaaag agcccgcatc tccaagtcaa tcctaagcca 2700
   aaagaacaaa gccggaggca tcacactacc tgacttcaaa ctatactaca aggctacagt 2760
   aaccaaaaca gcatggtact ggtaccaaaa cagagatata gatcaaacaa ggctttttgc 2820
   gccttcagca ccttggttct tttaagcttc ctttataata tcttaagaaa atctgagtcc 2880
   aggagcagtg gcttatgctt gtaatcccag cactttggga ggccaaagtg ggtggatcac 2940
   ctgaggttgg gagttcaaga ccagcctgac caacatggag aaaccctgtc tctacgaaaa 3000
   atgcaaaatt agctgggtgt ggtggcacat gccagtaatc ctagctactt gggaggctga 3060
   ggcaagagaa tcgcttgaac ctgggaggtg gagtttgcag tgagtggata tcatgccatt 3120
   gcattccagc ctgggcaaca agagcaaaac tccatctcag aaaaaaaaaa aaaaaagaaa 3180
   gaaaatctga ggttgaataa ctaacaattt taatatttag ttaagaggcc aaatgaagta 3240
   tttgtcctac cctcaagcag ccctgctgtc cttaagaaac aacattatat tttccaacgc 3300
   acaacaataa aatgcactta aatggtattt ttttaatcca tgttaataaa aggattcacc 3360
   acaactgatt aggcttaatg tgcatacaaa tcacaggaca gacaatattt cagttccaaa 3420
   tcttaaaaga taaaattaat aatgatataa cttattttca ttcaaaacta aaatgccttt 3480
   cagatggtcc aaaagtgttt catcaccata tggcagtata tctcaaagag tgatttgctc 3540
   attgcctgtg caaggtccat ttgagggctg gaaaatccag gttctcaggc tctatcccat 3600
   cttactgaat cagtgttggg gatttgggct ttgtaatttg cccctttaac aaactcctta 3660
   gaggactctt gtgcatactg atatttcaga agcagtctta tggagaacaa aataaaagag 3720
   gagataaaat atcttaagtt gtcaacagat agagatttaa caactatatg ctacttgcac 3780
   tgtatcttcg ggtttaattt agatttagca actactatgc cacttgcatg ttaggatttt 3840
   tagtttagtt tagtggcaag tgattaataa ggccaaacaa cttcatgatc ttctgttatt 3900
   gacctaattt cacaatttaa cacttagaat tggaatatta gctactgtgc aaaattcagc 3960
   cataaatatt taaggatatt tacaacttgg tttcatgctc cattaaaata tatttactat 4020
   ttgttgttta gaattaaaaa ggaaatctga ggtctaaatg ttcaatgttt agattaggct 4080
   agtctagaaa attcatcaaa ccaattccat ggggtagtgg gtatggtcat cttacaaaat 4140
   tgaatgtata ctatttgtgt tatgagaggt tgaatctcaa agtgtggtct gtggaccaac 4200
   agcatcgata ttacctagga gcttgttaga aatgcagaat ctcaggtccc acttttaatt 4260
   tgctgagtta gaatctacat tttaacaaga tgcccaggtg atttacatgc acattaagcc 4320
   aaagaagcag tgctctaaga aactgcttgt tcctagaaag tattgcttaa atataggctc 4380
   agaactgttg tttacatagg acttgtggat ttctcttgtg gttaaaaaat ttaaaactac 4440
   agttaagaga aacgagtggt ccttgcagca agctaacaag gagcccttga atattaagat 4500
   ggttgatact taaggactgc agcaattgct gtctaaacca gaaaagtatc tgagcacaat 4560
   tgacaatggt tccatagact cctggggata gtcacttgaa tgattaatta ttaattttta 4620
   aaaagaactt ttttttctgc tgtgcactat acaaaagata atgtatatta aatataaaag 4680
   aatcataaat gataaaagca tttgtatgct tagaaacttg gtaaagggga tagagtaaat 4740
   gtataatgga atcactgcat ttatatcaat aaatgagagc taaaatattt ttataacttt 4800
   tttgtcagta caataaagag gacttttgga gacagatggt aaatcaatat tgttttaata 4860
   tataatatat aatgagaagg agcaatatga tctagaatat ttttgtatca ccaaaagcaa 4920
   atatcaccat taacattcag atctgagaag aaatcaagtg tcaaagagtc cccaaaggaa 4980
   agggagactt caaattattc aaagatgaac tggaaaaatc agttactttg tataactggg 5040
   ggtatactaa atgtgttctg gtatggaaat tgtattcaat ttctaaacat ctcaggttgc 5100
   atgaactggg caaggctata attagtgact gtgcggccat ataacctgga ttagactttg 5160
   atggaaacat cacttcattg tgcatttctt gtgccagaaa ttcctatatg aagaatgaca 5220
   tatgagtttt gtaagtcagg ctcataatca ctaagcccaa ataacttcat ggtcatctat 5280
   tctcgaacca attttaagac ttaacaagta gggttggaat attggctcct gtggagaatt 5340
   cagtgatata tatttaagga tatttataat atttataatt tgacttcata ggtaatttta 5400
   tctttagtga atcccttttc ttaagggtag actactttaa cagaagctat taagatgaga 5460
   aatattttaa aacagtgaaa agtccctagc ctggtcatta atagataaaa tgtaaattaa 5520
   aaacaaaagt aaacacttag aatccattta cctaactatc gtcactcttt tgggggaagg 5580
   atagaccttg gaagaaccta tataatgacc ttacaaagta actattcatc aaatagtggt 5640
   ggactatagt cctacaaata ctggttcaat atcttcttag tcatataggc atttagcact 5700
   gcactacaat tattttgaca ctatagatgc ttgttcacat tctgtgattt gatcacagca 5760
   ggaaggacaa ctttaaatat agaagagaga agaaaaagat aatctccctc ctctctctgc 5820
   tcatttcaca ccttcttgaa tgaacaaagt tagtcttttc agaatatatg tttaatgatg 5880
   taataagtaa tgtgctacca aaatggcaaa atagatggac tagggagagc atcagtcatc 5940
   cctgggagat gctttagacc cagaaaatga cttcctatga tctcacctgt gatgagatga 6000
   gaccttttct tatctcatga aataatctct ctttttcctc tcttgtattt tttctttctg 6060
   tccaatgagg aaaacaaaaa caaagaaaaa aatgaaaaaa agaaaagaaa gcaaaacaac 6120
   acaaaactac caaaccaaca actggaaaac ccaaagcttg taaactgttt acaggaaacc 6180
   aaatagtatt tcctgggaca tgagatgaat atgagatggc gtttttattt gtcactttgg 6240
   gtacagaggc cgaaggtcat tatctaacag gcatacaaat ggaatcaatc tctccctcaa 6300
   agagcctgtc agatgcacct gagccttccc gggtgtttgc agagtggttc atggtcaaag 6360
   gcaagaatgg atattggcac ttcaggggaa actatatttt cctgtcaata gacagaagat 6420
   tgcagtgtcc tagacaggta atcagttatc aaggaccaca atcagctcac gtttaaacat 6480
   taaacaggtg gggcaggaga caagcaaaat ttatcatgtt tactagtttt gccaacaacc 6540
   aaagaactgc tttattcttt ttaatcagtt ctaatctgtg cttctttata tctattttga 6600
   aagacattca ataatttttt ccccttggca aacgcttggt ggattcatag agtcaaaagt 6660
   ctatatttct ataagtatat attttttaaa atctcccatt tttctcttgg ctttggttag 6720
   ctatatgtgt gtacaaatca gtccctatga cacccagttt ctatgccagt ttggtgaatg 6780
   gtgtggggaa agaaattgaa aaaggctaaa gcaaaccttc tcctaagatt aaacagtatg 6840
   tggtttgtta agtttttttt tttttcctat tccaccatta aaagtaaaga agaatggctt 6900
   ttcttttttt ttcctgatta gaaggcttca ctggatttgt ggcaaataaa ttgacaagtg 6960
   agggcactga tgacaagaga gtgataattg atggtggcag gtgggggcct ggtgtactgg 7020
   catgtcctgt ggagggtgag ccaaggacat ctatcaatca aattggtgac agcagccagg 7080
   gagacttaat tggagattca ttgaaccttt aatgttgtga cagagaaaaa taaggatgct 7140
   taaaaggctc ctgggctatt tcttttcctt cattccaaca attctcatag aaagaatgga 7200
   aagtggacat tgctccaggg ttgaacattc attgtttcca tcaaagttaa tcacattttt 7260
   tttttaggga agcaaagtag tgaaagtatt taccattagt ccaatgtgga atgaaatcta 7320
   tacctggagc tttagggatt cgactgttct gtggtgttag cataatcagg gatgtttagc 7380
   aagtgttcag acaccatgtg cttaacactg agtgtcaact agggtgcaaa tattgtctgc 7440
   tggagtgtgg attccaggca tcataaggaa aaaagtcctt ttcctaattt aataatcttc 7500
   tcttcattct tctaaaaagt tctctataca atccagatgt aaatattttt cttttttttg 7560
   tgtgttgttg ttgccagagg ttcaacataa ttttatttct tatttggaaa atgaaagaat 7620
   tcagtagagc tattttccct ttaggcacag tacaggaggt ttttcttaaa attagtagtg 7680
   caggaaaaat cccaacagtt caagacactg tatgtggata taatgtgtga aaagataagt 7740
   aagatggtct ctgtcatcca agattatata tttttatatt tatgagttta tacatttatt 7800
   tattcattct aaatcataag tttgggcact tactatgcat agtgatttgt taggccaagt 7860
   ctaagatcaa taggacctcg ttatttcttg gaaggaattt actgtttagt gagggaagac 7920
   agatatatgt actgagacat taaatattgc aatgttctag gtaagaattg agagttgtga 7980
   tatcacagtc taggatggta gaggctgcag atatcaggcc acttaagtga caatttttgt 8040
   cttttgaaaa ttgctagaat actgcttcaa aaagattgga aagaaagtaa agatcctaga 8100
   cttgctatgg cagagatttt atacttaatg gcatccccat ttagccaaat ttttaagtca 8160
   tagacagcag agagggatta cctacctctt ccaccacttt ctcacagttt gaaattcagc 8220
   attttgccat aacttttttc tacaaggcac tgaatacaaa catttccctt taattactaa 8280
   atggaggaat agtaatttaa attagtaatt tttaaatttt actagtagta aataaatttt 8340
   agtaactata acttttagta atagtaatta aatagtaatt ttaacttttt catcataatt 8400
   ttaaaagttt ccagaatgtc agcatgttgt tccacccagt cacaatttgc ttttccctgt 8460
   gccaaggccc cagttcagca tttattctcc gtattatgca cttcaacaag ggttgactgc 8520
   ttgaaagtcc tcctgttggc tgaactgtaa ataggctaca gattactcaa ttgtcttcct 8580
   ctagaatctt gtgtctcttt ccctgcatat ttacagatgc cttgaaaaaa ctcacagagg 8640
   caaaaatcac ggtaatcagg aaactgtaac ctggcaaaaa atctgtagag cctctcctca 8700
   ttattgagaa cagctgccac tagccaagtg catactatac ttttcaggaa aaaaaaaagt 8760
   tcatttaaaa gtaatttgag gcaggtggta tcatagaaga aaataatttt atttatttat 8820
   ttatttaaga cagagtctct cactttcacc caagctgaag tgaagtggca tgatttcagc 8880
   tcactgcgac ctccgcttcc ccgggttcaa gcaattctcc tgcctcagcc tccgaagtag 8940
   ttgggattac aggtgcctgc caccacgcct ggctaatttt tgtgttttta gtagagactg 9000
   ggttttgcca tgttggccag gctggtatca aactcctgac ctcaggtgat ccacctgcct 9060
   tggcctccca aagtgctagg attataggag tgagccacca cacccggcca agaaaagcat 9120
   tttaattaga atacttggaa gatacatttt gaaaaggaag attctagatt aaaattttgt 9180
   gctatatttc attgtgcttt tttcattgaa aatgttgata taggaagaat tctgtcatct 9240
   gtatcccgtt ttttcaggct catcaagtct tataattaac tttcctaggt cctagaaata 9300
   tattattcct aggttagaat attttctgta gaatacaaat tatttataaa ccatcagcat 9360
   gggcaatgag ccagctcaag attttgttag aaacaaaaac atgaaagtca aagttagaat 9420
   gattctttta aagtattttg gtcaaatcat caatgtggta atcaaatctc ctttagaaca 9480
   ttttctccat gtggtcttcc attccaagtt tgtacatgtg cagcaaggca gactctacct 9540
   gccatctatc tctgctgtat ttgtattcta tcttttaatg actgtctgtt aaatactttt 9600
   tgttaatgtt ggtaagaaat caatctcttt gtagcatgta gtctctggac tggattcagc 9660
   atttgtcttt ggatatttag attaaccaga aaaactccac ttgaaaattc cccaactgtt 9720
   tgaagatagt tattgtatcc cttccaaaac tctaattttt ttaaaaaaat taaacattct 9780
   tcttcccctt agccatctta cccatgttat ggttttaagt cccttcatta tcaaaagtgc 9840
   tttgctctgt gctgatgtaa ttatgatcca agtgtctcta ggcacttctt agaacatctt 9900
   gtcaagactt gaacacgaca ccaggaaagg tttgaccagg tttagagcag ggaaactttt 9960
   tcttccctta caagttgcat ctcttaaatt ttttttttct tgctgtattc aggcctttga 10020
   tggttaattt tatgtgtcaa cttcactggg tcacagtgcc cagatatgtg gttaaacatt 10080
   gtggacattc aatgaagatg tttttagatg agattaacat ttagacttaa cattagattt 10140
   tgagtaaaat agattaccct ctattagtgg atgggcttca tctaatcagt tgaaggcctc 10200
   aatagaacag acggatttcc cgagtaagaa gtaattcagc aggcagatgt tcttcagagt 10260
   tgaactgcaa catcaacttt ttcctgtctc caccctgccg gactaccctg cagattttgg 10320
   atttaccaag cctctataat tgtgtgagcc aattccttaa aagaaacatg gatgtgtata 10380
   atacacacat acacacacac aataggatat gtgtgtgtgt attatgtatt ttatatatat 10440
   atataataca taatatgtat gtaatacaca tacacataca gatatcctgt ttgttctgtt 10500
   tctctggaga atcttgatac aaattcaaaa tgacggcctg tatattttgt catcttgatt 10560
   gtgtcatcta atatatttct tttcatcatc atctttgtgt catctacaaa tttgacaaca 10620
   acaagagctg ggtgtattct ttttagtcta agaacaatgg attttcttca ttttaatgat 10680
   tataaaatta cctttgatgg aaatggttct tcatagagaa gcttctacca actattgctg 10740
   attatgctta gtgaaagatt gagaacatta acactgcatc tcagctcccc ctggtggaca 10800
   caaccctcac agacattctt caatagaatt gccgagtaga gtaatagatg ttattgatag 10860
   agtgtaaagg cacatggctt accgtaaccc tccttttaaa aaggtacttg gataaccata 10920
   aattatgaca cccctcttga gattttaact atttgtgaga ccctgaggct ctctcctcac 10980
   cataatgact taaggttgtc acagtgatga gtaagttgtt ttatgaatat tcaaaatatt 11040
   tagaaaacaa atcatacaga cctaatataa aaattatgat aacagattgt ctaacctaga 11100
   actataaaac accatttgct aacacaatct ttattaaata agcagtaata agacatctta 11160
   cattttattt ttctaattct attagaatta tcttaatgta atacttgcta tctaccatta 11220
   agttttttta attataaaag agtatagctt aaactaaaga aaaaaatctt atatatatta 11280
   agaattacaa ataaaagcta tgtattgagg gcctgttgta ttatatatac tccataaggc 11340
   agtttacata ggttaatatg catatattcg caacaactct ataagctaag ttgttttacc 11400
   ttcattctgt atttcaagtt tggcaaacta tggcttgtag gccaatatag cccttgtatg 11460
   gaacgtattt tacacccagt gagtttaaaa tggttttcaa atttttaaat ggttgggaaa 11520
   aatcaaaaga agaataacat ttaatgacat gtgaaatctt ataaaattga aatttctgta 11580
   aataaagtct tattggcaca tagctatatt cattcatttc catattatcc atggctgctt 11640
   caatgctata acgacagaat tgagtagtta cagcagagac actgtgctat ggaaagtcca 11700
   aaatatttat tgtactatct ggccccttac agaaatcatt tactgattct gctgtatctg 11760
   atgaaagtaa ggcttcacat ttttaagtga tttcttcaag agacctgagg ataatcaaag 11820
   taataatttc tttgttatta gtttcttttc tatcccaagt aattgtgtag tgaattctgt 11880
   actaaatatt aatatatgca tttagagaaa caaatttgaa acaggataaa aagtgttttt 11940
   aaacaaattt aagtcaatat ttacaaggtt tttttgtgtg tgtacacaag aagtatatat 12000
   tggtagaaag acaagtgact ggagtgtaca aataaaatgg agatggaatg gacagttata 12060
   aactcttaaa attcataacc aggcactaag atttcatggt tgttctatac agtaggtaag 12120
   ctttttgtcc catcctggat ttatgttttt ccatttctaa gcatcagtga tatggttcca 12180
   taatgtggta ttgacaatga cgtttaagag gaaacataga gatgcataga catgaatggc 12240
   tcctgtgttt atgaagtggt atctggaatt aaataggaag atgcatgtga tatgcttggc 12300
   agaaacccta gtaaaatgtc aataaacaat aatcatcatg atagttgtta gaaagttatg 12360
   gttggcatat ctataattta ttggagatat ccctggaatc tggacactgg ggcagtcttt 12420
   cagatcttcg atatgtctta tagcatggtt tctagtgcta gatgatgagg attcaagatg 12480
   atttaaagac aggagtaatt attttactgg aaaatactgg ttaaatagat tttttccctt 12540
   tagaaaggct actgaacaac tagaaggtgg acataaatat gttgtttcct tttacatttg 12600
   ctctctggag ccaggaaggg ttaagaaaga gatacttctt tcctcttagt cgggattcat 12660
   acagaaatca agagtgggaa ggggccctca ttacatcaga cacagttact ttaaggatga 12720
   ggaatctgag acccagataa ataaaataat ctgcctaaag ctgacccaga gttagtacgg 12780
   ggctctggac gatttcatta tgtcatgata ccattctctt ccaaattcta agattggaaa 12840
   atttacctca tatgaagagt gaatttaatt tatctttatt ctagtttccc ttcaaaggca 12900
   tgtcttaata tacatggata agtcatttca aaacctctaa gcctaaggta atttgcactg 12960
   caaattattt cacaccataa tatctatctc agagcttttc ctgagatgta aagtccttga 13020
   tgataaaacc gactccgtag taatagtaga ggacttattt gtattgagaa tgcagacttg 13080
   agtgcaaagg ggccagacaa gtaaagactg tatgtaaatt agtaaaggag taggagatga 13140
   aagacaatag ggagtagagg agaatgtgct caactagaaa agaaagagct aaaatatgga 13200
   cagctgttac tcagccccat ctgatggtta ccatgcatgc atgattttga atttgctaaa 13260
   tcctctcaat tcaaagaagc tgaaaatcta gacttttaaa tgtggagacg ttctgatttc 13320
   cttattactg catgggtcaa acaaaacaca tctgcatctc tactaaaaat aagaataaaa 13380
   aaagaaatta gctgggcttg gtgcggggcg cctgtaatcc cagctacttg ggaggctgag 13440
   gcaggagaat cccttgaatc ctggaggcaa ggtctcagtg agccgagatc gcactactgc 13500
   actccagcct gggcgacaga gacgaaaaaa aaaaaatctg tggactggtt tagaccttta 13560
   ggaagcatca tcagcaatga aaaatcatga tgttttcatt aaaaataaga gttttacatg 13620
   caatttgggt tgtagggttt acagtttaat attttatgat tctttttcct ctttttttaa 13680
   tcccaggagc aagagcatgc catgaatcta gcctgtagat tcagcgtggc tttctggctc 13740
   aaacttagac tgcatcaaac agctttgctt ttgttttctg cttgaaaaaa ctgacacttg 13800
   ttatggttac actcactgtg tgagccatcc cctgttgact tctggacaat cactataaat 13860
   ctctattatt tcgctgttat tgtcttgtta tactagcact gttgaggcat cttctcaagg 13920
   gaagtgagcc caggtgtgca catacagaac aagaaatact ctcccttaaa cacataaata 13980
   gctattttat ttggcacaac acacacaaat gttagtgtag ccaaaggaat cttgaacagt 14040
   tatttgcatt gcaaatactt caaaaaactg gagcaaaggt atcagagctc aatctgctag 14100
   ccatttgagt ttcattagtt cagagaacaa tccatctttt atttttttcc agtaataaga 14160
   attgaaatga aattcagtgc cttacaatca ggctccaagg cataagaatt tctcataaaa 14220
   aagaatatat tcctgtttcc ccatgaaatt cagttatata aacctctttt tatgggtact 14280
   ctttctttat ggccctgttc agagaaaatg tgcagggtta agctctcttg tgctacacat 14340
   tcattgtttt gatatacaat ccgatggaag actgcaacag tgctttctga gatatctggg 14400
   catgaaggtt tgaagattcc caaggcaaaa agttatgtca atttccttag tcgcacatgt 14460
   gattaagggg tacctttggt tctggtactt gccaattcat gacgtggcaa aaaattacat 14520
   aaagaacaat tggcagatag gaggttgtgt tttagaggcc tgagtagaat tggggaaggt 14580
   ttaataaaag ctggaagctc agtttcatat aattcacaaa aagctaaatc tatatttata 14640
   agagttaagt aagaaagaaa gagatttggg gggcaattaa ttttctatgt aagtatgtat 14700
   ttatttggtt gatgggatcc ttggtgaatt tctaattttt attattttat aaatttagta 14760
   aaacatgaaa aaggaagcaa aacttaccta tatgcataaa gctttgggga aaaagctata 14820
   acagactgaa acaatcactc tccactttac agtactatct tcagactcat atgaatatga 14880
   ataaaatatc tgaacataaa aatattttta ttttatgaaa aatgagatca tactgtttat 14940
   tcctgtttta aatttaaaaa tacatattta ataacattta aaaaataaag taagaaattg 15000
   tgacccaggc ggtttcttga catatactga tgtgcatcct ccccaaaccc caagttaaaa 15060
   gaaatgataa aagtcagtaa tcctctaatt acgaatcctc tgattttgta taagcttcca 15120
   ggacaaagaa actttgtgcc cttttccata aggaacaaag agggccctgt gggaacattc 15180
   tgcattcacg taggtttgga gaggagaacg gaaagctaca gaagcagaca cattagcaag 15240
   catattggta ggatgattgt agttgtgaga attctgaaac aaatgtaaaa agggtgatgt 15300
   tatgaagact gaagggaggc tattgttaga ttgcttggaa aggaaaggcc cggtgaaaaa 15360
   aggtacactc atgctgacac ctgattgaca agaagaagcc agacacgagg attgcagatc 15420
   cttctatgca aagggtaaag gaaatgcaaa tcctgatatg aagtaaatat cctagatatg 15480
   tattagtaaa tagcatccaa aggtatatta ttagaatcat tgtatgcaga gttattccag 15540
   caatgcaaag atttcattcg gaaaagtttt aatgtaatta attacaatca taattacata 15600
   aaaacctcca gcaggaaaac aatttgatga taaacaaaag agtgactact taaatgacag 15660
   gtgactttta ataactgcag tggaagtcag gtgatagtag aatgatatag ccaaaatatg 15720
   aaagaaaata tctattaacc tataattcta tactgagcaa aattttattt ttcagaaaca 15780
   gaaagaaatg ctttttgggg gtaaacaaga actgagattt tactgacaat agaatttaac 15840
   taaaggaatg tctaaaactt ctcctttaag agagaaaagg atcatagtgg aaaagtcaga 15900
   agtagtacca tatgaagaac ttatggtaag caaaacaatg gcaagcctgt gggaaaaatc 15960
   taaaccatca ctgactgtaa aacagtaact atcttgtcta attgggaaag gaaaaaaaaa 16020
   acttaagata gaaataaaat gtgaaaggat gcctgtaatc ccagcacttt gggaggccga 16080
   ggtgggcgga tcgcctgagg tcaggagttc gagaccagcc tgaccaacat ggagaaaccc 16140
   cgcctctact gaaaataaaa aattagacgg cttggtggca cacgcctgta atcccagcta 16200
   ctcgagagga tgaggcagga gaatcacttg aacccatgag acggaggttg tagtgagccc 16260
   aggtcgcacc actacactcc aacctgggtg acagagtgag attctgtctc aaaaaagaaa 16320
   aaaaaaagtg aaaagaaaat aaaatctcga gaccccaaac tcactatgcc aaagggaaaa 16380
   cttaagcttg gaagctgaga cacacacaca cacgtgcaca cacaccccaa aaaacccaac 16440
   tcaacccaaa ccaaaccaaa acacaagaaa acaaaccaaa aactgacttc ctcttgtttc 16500
   caaacagctg taatttcaca gctcacttta tcttatgtaa aatgtagatt tactgagtgc 16560
   tagaggaatg cataattgac cctccctttt tgcatgtaaa atgtggactc agtgagcact 16620
   aatcaaagcc tcacaagagt gtaaccactt gcctcgttgc ctaacctcct cttttatttt 16680
   tctttcctcc tctccctcct gcttgcttgg tccccttcaa atattgatgt cctcaaaact 16740
   gtctttggaa agaagcatag gccatggatt ctattataac ttgtgattct gtttcttaga 16800
   cacatcctta actttggcaa aataaacctc tattccattg agatgtgtct cagacacttt 16860
   gtggtttata aaaatcttcc ccaataatat tatataagtg aggaaggagt gtgattattg 16920
   gaattaacca ttgtaaggtc attgtatttt tcagagggaa aataaagata ttcagcttag 16980
   aactgatgag gttaaataag catgctaaaa tttctggaaa aacaatgaaa tgaaaaaagt 17040
   tagacgaggt acttaaaaat gagtagaggg aaaacatcaa atggggattg gggagaccac 17100
   gtcaattcaa aagaagaaaa aaagagaaaa aataagcaca ctaggaaaga gggggaggac 17160
   aaataagtaa tacaaagatg gtagaaatga gtccaaattt ctaatcaaga tgaatagagt 17220
   aaaccattta cttaaagaca gaggatcaaa ttgaattaaa aagcaaaata caattatatt 17280
   tgtcttatag aacttaactg taaaaaataa tactttgaaa atatatcaga gcattttttt 17340
   ggaaccctga gatgcctcct gaagccacga ggagttaatg gcaacccttt gtcttacaag 17400
   gccttacgta atttaattct tcaactttga atttcttgct cactctgctc aaccacatta 17460
   acctctttgc tgtttctgga gctcatcaga cacatgccag cctaaacgtt tgttctagtt 17520
   tttccctctg cttggaatat cctaaaccca ggtatctact tagctaactc tctcatcttt 17580
   ggttccctac tcaaatttta cattctcagt gaggcccacc tagaccacct tacttattcc 17640
   tgcaacttgc tcacttctct tcactttcta ctttctttct ttgctctggt aattattggc 17700
   acttatcaac ttctaacctg ccatatcatt taacttttaa aactgattgt ttattgtttg 17760
   tttttctccc ttagaatata aactcaagga ggaaaggaat ctttttctat cttattcaca 17820
   gattctggaa tagtgcctgg cacatagtag gcacttgata aatattcact gaaggaataa 17880
   atgagtgcta tttctacccc taatttacag cacaacatct ggcaacattt tattaagtaa 17940
   aataaccaat aagtaaaaag caaatataaa atttgatttc tgtattttct cttcgtattc 18000
   tggaaaatgc tttggagatg agggtagtag gaagaaatgg gaaaaaggca gagggagatg 18060
   agcctcctcc ctttccactg gatgagatgg ttgaactgca ggtcccttga tgtttccaca 18120
   ctagcagaga attctctcat ttctcttctc agggatgatc cttgaagcag gatctacaat 18180
   agacagcatc aaaatcaaca ggcatatgat aactgacagt ttggaaaact atgacctaat 18240
   tgatgataca agacccatta gtttgtgggg cctaagagga aatttcaaca ggaaagatta 18300
   gtcttgcagc ctgacttgtg gatgcagcag ttgcctcatg ctatggattg cattgtatca 18360
   acccctaccc ctcaaattg 18379
<210> 24
   <211> 3454
   <212> DNA
   <213> Homo sapiens
<400> 24
   agtatgtgca tttgtacctt gctttgtttt cctcaacttt gtgcttgttt ctgtaattcc        60
   ctcattcatt cctacctctg catgcttgaa agttctttgt ggataaggac ttggtccgaa       120
   ttatatttcc tttcccatcc catgcacgtg gtcctctctc aggaaatagt gcaaatgagc       180
   aactattgca ccaagttagc cattctctaa acataggtac tacctatgca ctttacaatt       240
   cttgcttcta atttatttct gggttgcatg gaccttacaa aaaggagtag cctttttgaa        300
   aagattggtt tgaaaaaaaa caatttcaag catcgtttga aaaggctgcg ggaccaaaaa 360
   gccagcacag ctcaagtcat tttaaacatg caatgattct cactctgaat actcagagtg 420
   gagactcaaa gtgtggctct ttgcaagtta atgtcactct ggccattcct tacactttga 480
   tcattcaaac agaagtcaca tgtggtttcg tctcagctct aagaaggcca aacatgaatt 540
   tctcaagcct gtccaaacaa tctctcagaa aaaaagatag aggttggatc atttgttcat 600
   acagtttgat tagctaacac acattttata gtacttgaag ggttgttgaa aaggagttcc 660
   tgtcttatca ctagaaggtg aattcttgaa cctgtaaaat aaactagtta gtttgcatgt 720
   ctttatattc cttccatgca tttagatata cctatttcca gacaggtttt cagaatgcat 780
   ttgggaagcc tgtgattaaa ggatattttc tggtatggta tgaaggctga gtctcttgac 840
   aataactttc agaaaaagaa taaaatctat tttacatcag ctgaatgtct ctaattgggt 900
   cttgaagacc aaacagttcc cttcaatgca gctggggctt tgggtagccc caaaccaatt 960
   aactcaaatt atataatttt cttcccatcc ctacccccaa agcttgactt gcttatttag 1020
   taaagaggag gattagcgca agcactaagg agctgttctc tggctgcctg gcacaatatg 1080
   tgaacttact tctgggccag ccagaagcat cctggctggg aagaaaggaa aaggggaagc 1140
   tgaagtgaag ccgtctcctt ggtgtgactc tcctctttct tgggatacaa tggaactcag 1200
   cagaaatata ttactggatt accaggcagc agaaagcatg ctggccttta aatgggaaca 1260
   agcaatcttt agccctccta aacctctaaa aatgtgcagg agctaattaa ggcaacaaca 1320
   tcatgaggtg atcagagaag agcagagaaa tgtgaacggt tagggggaat ctgaacaggt 1380
   gtgtatgtgc ggtgtgtcta aaggagcaaa aagctttact atgaatagat ttcatctttc 1440
   ttctgtttcc aaatgatttg ttaagcctgg ggtgagcatt gaacaaataa caaaattatg 1500
   taacaattcc aaatgtatat tttccaaatt aaaaaaaagc aataatttta aaaggcagaa 1560
   tgacaccctg cattgcaact gtatcagaaa tatgcttagt aacctttatt acaaggcagc 1620
   ttaatttttt ttctatttta tatagagtac tgtcttttgt gatccttgta acaagatact 1680
   aaatacctga tttgataatt gtgacatata ggtgaattta aaatagttta gttctaataa 1740
   gaaagaataa ttaatgatac cttcaggtgg gcaggttttt tcaggggagc attttttcat 1800
   atttattgga cagtgaaatc tcagcttcta agtgctgacc ttttatgtgc caacaagaga 1860
   aagcaaatag tagacatgcc tcaatgtctc ccttcttttc taagcttata aaatatggta 1920
   cttaacttgc cgtggtattt tctcacacat tcctttctga ggggaccctg ccctggatac 1980
   cagtagaatg gttttataca acaggcttcc ctgatgttct aggtcctggg agttatcttc 2040
   taagcttgag ttttttgctc atatgtctgt caaaaaggaa aatccagtga aattaaagtt 2100
   cgtcaggaag ctacaagtct gttttcttac agctcccatg ggcaaagtag cattatatct 2160
   tctgttgatg tcaccatttc tctgggagat tctagaaatt tgttcattgg tttccttcag 2220
   aatctactga ctaccaatgt aaactcacag ctggagagcc acatcccttg tccttttgga 2280
   ccatggtacc tatgaccaag gcttaggcct tttctgttga gtctctgtct ttggggaggg 2340
   ggataagttc ttaaattcag ggccagagtt ccaggaaagg tattcaagta agacacagag 2400
   caaggctacc aattaaaata aagacaatag actttcaagt tcaaattctg agaagaatat 2460
   taaaaattat ttagtctaca ctgtgatttt aaagagagaa ccatggtagc ccaggaggga 2520
   ggttgacttg ttcaaggtca catggtggct tagtggtact gcctgaccag aacttggttc 2580
   tcctgatgca attgaagagc aatctctggc tgtcaatgct gatcacatct caatcataga 2640
   aacatctcct ttatttttct gactttatag attgctaatg gcaggagaac actatgactt 2700
   acaagttgag ctttcacctt gctggccctg gctgggcaga cttggcatct gtggttgagg 2760
   ctttaaggtg gaaaaacagg gcattaccag tgcttctttt cattgaatgt acatttgaga 2820
   gatgcattaa ttaacgtaaa attgctacct ctagctctga gccttagctg gcgctccact 2880
   ttgtactgtt caccaaaatc aaagcaccgc tatggtttat tttttagata tcttgaaaag 2940
   gataatgttt gaccattcct ctccttggct ttacctccat gtgctgtgtg taatgatcca 3000
   gagagaatca attggacact gcattttaaa gataaggatt gggattttac ttctgggtaa 3060
   cctggtgacc ccaatcaatc tctgtcttac gaactctctg aagatttgat gctccagtcc 3120
   gcaattaagt cccagcttcc aagaatggca acagatgttg caatccaaac acctttatta 3180
   actatttatc ttcaaatata tatcaacgat ctcttggcca acagaaagag tggcctgccc 3240
   cttaaatgga aaatcacact ctttcatctt caagctattt tactttatag tccttttgag 3300
   aagataaaaa gagatctatt aagaaagtag ctgacaatat ataattgctt tcaaaatttg 3360
   ccttttctaa aagtagtttt tatctataca ttgaaaatat tgctattaag ttgtatacct 3420
   caactataca aaacatcaaa tggtatttag ggta 3454
<210> 25
   <211> 27712
   <212> DNA
   <213> Homo sapiens
<400> 25
   accaaaggac atgcgaaaac ttttgggtgt gatggatata gtcataatct ttattgtggt      60
   gactgtttca cacatgtgta catatatcac aactcatcaa actgtatact ttaattgcgt     120
   acagtttatt atatgtcagt tatacctcaa taaggctgga aacaaaaagt gctctgggca     180
   aagagtctgg tgacttaagt tctgctttta ttctgggccc aagttaccat gtcaccttgg     240
   ccaagtgacc caaccctaag aggctcgggt ttttcacctg tgccctggga ggtttgggtt     300
   agatcgttga gtggtaagag caaatgctct caggggccag gaaggaaata caaatgaatg     360
   acataagcca ggttcaccgc ggagaggggt agagacaggt agagactgtg gctacctgga     420
   gagtgttgcc tctgcccagg ggggcagctg ccaatcagct ttggccattt gctgcttaat 480
   ggaatgtggg cggtgatgct cacgcttcag agtctttaag agaaaccaca gctccggatt 540
   cctcaaatga aatcttcccg ttatcaaatg tagataatta acttcaaaaa gttaaaacac 600
   tgtgcaggcc aaggccacac atgtgcagac gagacccagc ctgagcccca cttgtttgtg 660
   gcctctgcac tctgtgatgc gtggggatgc tcttacccga ccaagaatct cttctctact 720
   tttagccaaa gttgcttccc taattctttt tacctatact tcactaagaa aaaatgtcca 780
   tcaatgccca tttgcaacct gcctgctttc catctaccat tcgatctata gagtgtctta 840
   actcttggta gacttgaatt cttatttctt cagtttgtgg aggagacatt ctgggcaatc 900
   ttgccgagtt aaaagaggct agtacctacc aaaaagagtc gaaagcaagg actccatcag 960
   atatttggat accagtgttc aaagtggcat tattcacaac caccaaaagg tagaaacaac 1020
   cccaatgact attgacagat aaatggataa acaaatgtgg tacagtacaa tggaatagga 1080
   ttcagacata aaaaggaatt aaattccgcc acatgttcta ccagggagga accttgacaa 1140
   cattatgtta agtgacataa gcgagacaca aaagggaaac tattgtgtgc ttccactagg 1200
   tatccacgag agacctagaa tcgtccaagt cacagagaca gaaagtcaaa ttcatggaga 1260
   ccaaagggag gaggcatgag tttcctgggc gcagagtttc agtctgggaa gatgaaagaa 1320
   ttctggagac ggatggtggt gatggttgta caacaatgtg aatgtacttc atgccactga 1380
   actctatact tcaaatggct aaatcggtat atatttatat atatttatat acatatatat 1440
   aatacatata tattatatat gtatcccaga acttaaaatt atatatatat tatatataca 1500
   ttatatatta tacattatat attatatatt atataatata tattatacat tatatattat 1560
   ataatatatt atacattata tataatatat ataatgtata atatattata taatatatat 1620
   aatatattat ataatatata ttatatatta tattatatat tatatatatt atataatata 1680
   tataatatat tatatattat acaatatatt atatattata taatatacac tatattgtat 1740
   aatataaaat ataatatatt atataatatg tattatatat aatatataat acatattata 1800
   tataataata tatataatat acaatatata ttatatatta tataatataa aatatatatt 1860
   gtatattata taatataatt atatattata tatataattt taagttctgg gatacatgtg 1920
   cagaacgtgc agttttgtca cacaggtata cgtgtgccat gatggtttgc cacacctatc 1980
   aacccatcat ctaggcttta agccctcctg cattacgtat ttgttctaat gctctccctc 2040
   cccttgcccc catcccccga caggccccag tgtgtattgt tcccctccct gtgtccatgt 2100
   gttctcattg ttcaactcta aagtggtaca ttttatccat tatttttttt tgtataaaaa 2160
   ggcaggacac gtctggaatt ttcagatccg atgatgtaat ataagaggct ttcttttcag 2220
   cacactgtgc taaggagcca cggctggagc tcccattggc acggtgtctc tgcaactccc 2280
   tcctcaggct cctcacacac ccggggaccc cctgtgagtc cctaagctgc tcttaggctt 2340
   cacccatttt gcaaagtggg tcttggcccc agctacactt tgagagcttt tgaatagtac 2400
   agatacctgg gccccaccct tggagaatct gaatcaattg gtgtggtggt ggaatccggt 2460
   ctccacattg actcagagcc tcccgggtta ttctaatgtg caggcagagt tgagaaccac 2520
   tgaattctgt tctgaaaaca tcagcaagac ctttcatgga tattataggt aagtaaatca 2580
   aaaccgcagc agacggtgct gaacagatcc atggggtgag ctgttctggg gaggcgggtt 2640
   tatcccggga agggttctgc aggagtgggt gggcctggaa gtgggaatat ctgtaccccc 2700
   attaagaggt aagaaatgag caagccatag ttaaggagcg tgtttgttgg gagggaggag 2760
   aaacagggtt aggattaagt aataaaacgt gagacaagaa ggcctgcaat tcccaggtga 2820
   agcgcttgga gtctttctat tcctgggagc aaatctaaca catgggaaat atttgcttct 2880
   gcagagaact tcttagattt cacttctaag tgaaatcagg taagtcagtg gtaagaaaaa 2940
   tcataataca agattctcta aataagaatc tacttaacct gctgcaattc tctgtcaaaa 3000
   tatcctatgc cccagtctca aggtaggatt tcattcagaa ccgtatctat ccccacaaaa 3060
   ccacttgaat gaagttaggt ttatttaatt tggtgacttg accgagtcag cttgttgagg 3120
   gattggggat gaaaatctaa agggaaaata ctggactcgt gtaaatttag actccttggc 3180
   tctgtcattc taaatgtttt atgcaacaaa gttggagaat caatccattg ctccaactgg 3240
   ttccttttcc taaacagcat cttctccaaa tcttgtcata gaagtggtgg tagtgaaatg 3300
   actgtcatgt gcaagcaaat catgaaaacc tatcagagca taatttttaa aaatcaagaa 3360
   tgagaaaggt gctctggagg gaacaaaagt ctttatttca tggaaacaat tatgtgatgc 3420
   tcagaagttg atcaaggctc tgaaagtcac agtgcatggg attatccatc ttacttacct 3480
   gtaagtcacg tctctgtcaa tcttacctgc tcacgggagt tggaaactga aaatcataac 3540
   aatatcaata acactagcca acttatattg agggcttgct atgttgcagg cgctcttgtg 3600
   cactttgcat gtatcaagat ccttaatcct cacaacaaca gcatgtgaca ggtactattt 3660
   ttgccatctc tagtctaagg atgaggaacc tgagatgtag agaataattt cgataattta 3720
   cccaaggtca catggccagt aagtggcaga ctggattcaa atccagctgt caagttattt 3780
   taggtcagag cctgggctcg tcatgtacca ctattacctt ggaagaagga agaaaaggta 3840
   gaaacaaaca cttagtaagt gtttattaag tgccagctat tttatacatt atggtgtcgt 3900
   tgaaccccta ccacatatcc ttagcctcat gttacagcca ctaggaaatg tggacagaga 3960
   gtttaatgac tagctagcaa aaggcagcct ttatttgtct gaccatacac ctgtgcattc 4020
   ttcactaaat aatgaaattt cagagtagca ttcatgcaag aaagcatgct tagggaagaa 4080
   agtctcttgc ttagagtttc atcctttact tagtgcagtt ttaaccagtt tgcctatctg 4140
   ctttcctttt caaagccagg aagtgtatgt ggggtgggag caagagggaa ggtgggtgaa 4200
   ttgctggaga aataagttta atagagaaaa taaatgtcca agagatgagg attattaaac 4260
   aagcagtatc ctagctatcc atctttgact tctttgagtg gaagttttgg ttaaaagcta 4320
   tatgctttct ttaaaggaat caaagaaaga ggttggatga gtttgttagc agcaatgaat 4380
   ctatacaggt ctgtagcaac ctcaattctt gattcctcag aagaaagaat ttgaccgggg 4440
   gacataaggc aaagtgagac caaggcaagt tttatagcag gagtggaagt ttactaaaaa 4500
   gttttagagc agggatagaa ggaagtaaag tacagttgga agagggccaa gtgggtgact 4560
   tgagagattc aaatgtgctg tttgactttt gacttagggt tgtatagttt ggcctgcttc 4620
   cagtggggtg tgtctgttct cccctgattt ttctcttggg gcggactgtc tgcatgtgca 4680
   gtgggcctgc cagcacttgg gaggggccgc gtgtgcggtg tttactgagg tcgagtgcat 4740
   gctccttgga ggcatttttc ccttaccagt ctagggttcc tagatgaagg tcacatacca 4800
   gttaaatgaa gtccatcgtt ttacctctca atgcacatgc ttgagcccac tcgcccaact 4860
   cctgagatct tatcgggaag ctgctgccta ccaggttcgg gtgttttctt ttcttttctt 4920
   ttctttgttt tttttttttg agatggagtc tcactctatt gcccaggctg gagtgcagtg 4980
   gcgtgatctt ggctcactgc aagctccacc tcccgagttt gcgccattct cttacctcag 5040
   cctcctgagt agctgggact acaggtgccc accaccacgc ctggctaatt tttttgtatt 5100
   tttagtagag acggggtttc accatgttgg ccaggatggt cttgatctcc tgacctcgtg 5160
   atctgcccgc ctgggcctcc caaagtgctg ggattacagg cgtgagccac cgtgcccggc 5220
   caggtgtttt ctatctattg ggagactgcc tttccctggt gctgggtgcg atcagttatt 5280
   attttaggga gcagtggaac aacttcctga ccatcacctg atggttgtct gacgttcctg 5340
   gggtgggggt gggggtctgc cctgcttatg tctgcctaac tacctactgt taacaagtta 5400
   atcagataaa tttagtaagc tttcaaaagt ttgtataaaa ataatcctta gttaaattta 5460
   gaacacaaaa ttaagttcaa gtgagaactt ttctaaacac agaaaacagt tttaactctt 5520
   caaagaatac attgccactt actctaaaca acataatatc aatacaattg ttattccaag 5580
   attattaaac atatatcaga aggtatgcag agctcagatg gtacttgtgg gtatgcctgg 5640
   gcttgctctt tctttctttg tctgcctggt gaacttctct gcagactcaa ggacaaggtc 5700
   ttcgggactc ttctgagaag tcagccctac tccttctcct ccacccgtca ctgccctgca 5760
   gtattctgga gctgtggttc tcacacttta gaggacatca gaatgaggag ggcttattaa 5820
   aacacagatt gccagcgcca ccccaggagg ttctgcttca gtagtctggg tggggctcca 5880
   ggatctgtat gtcccacaag ttcccagctg actctgctgc tgcttgctca gtgactacac 5940
   ttggagaacc actgccccag agaaaactcc tctctcgtct gtgctgccct ggcctattca 6000
   gtcattttat tttgacttgg gccgagcaga gaatttctgt atcttctcaa gggtctgtga 6060
   tcttctcgaa gagagagtcc tgttttcagg ccttggagaa gggtaatagc agagagttga 6120
   ggggcttcct gggagagggc tgtggctgat ccagggctga ggagtctatc cagtaacaaa 6180
   ggtgtagtca caggtctctg aaaactgcat cccaacccca gtccctgctg gactcacagg 6240
   ctcagtggaa agtagagagc tgtaaacaca gaggtagtta aaaaaaaaaa aaagtaatgg 6300
   cagaagctgc cctctctgcg accctgtaag aaagcatgaa acgaggcaga gaagcgtctt 6360
   ttcatactcc cagacaaacc tcgcttttgt catcccaagg aatttaggcc attactcaca 6420
   atgcctgaat gagatgatgg tgcccgacag agccgggcat catcaagggc tgtgctgccc 6480
   aatccagtag tcattggcta catgtggctg tatgtatgta aatataaatt acttagaact 6540
   aaagaaaacc acactagttt catttcaggt gctcagtggt cacagatggc tcacggccac 6600
   cctgctggac agttcaactc taggacatgt ctatcattgc agaaagttca gtcggctagc 6660
   actactggag ggacttggac aatgatgccg caagagcaag atggtacaca ggaaacccga 6720
   tgtaacgtgt ttgtgcccct ctccctgcat gctgttctgt ccaagaattt gcaaagactg 6780
   gcatacccca cagaatgatg cctgggagca tatgatgcct ctgcttctcc tttttcctgt 6840
   tctcatgagc cagaacttta gtgccaagtg tggttggggt acccgtgagt cagaagaaca 6900
   ctgttggagc tgagagtagg attgcccctg gtcaggggtg agtcactctc tggactccaa 6960
   aaggtccaag ggtccttaac atggggctga atgcctgccc aatgccaagg ctgtcgagtc 7020
   atactttttc ttttgttctt ttttttcttt tttggagatg gagtgttgct ctgttgcctg 7080
   ggctggagtg cagtggcaca atctcagctc actgaaacct ctgcctcctg gtttcaagca 7140
   atcctccccc tcagcctccc gagtagctgg gattacaggt gcccgccacc acgcctagct 7200
   aatttttgta tttttagtag agatggggtt ttgccatatt ggccaggctg gtcttgaact 7260
   cctgacctca ggtgatctgc ccgccttggc ctcccatggt gctgggattc caggtgcgag 7320
   ccaccacgcc cagctgagtc atgctctttc tatttcggtc agtgttactt gtgcctcacc 7380
   ttgcttcttc tgccgcagga acctcccctc ccctctcata cgcagcgtgc atgcctttct 7440
   cttgtcattg ctctttgaaa gacagcctat gcatttgagt taggatttgg gaatttaaaa 7500
   ggatggcaag tgaaaaagca gagttgggtt atctggatca atgatagagt ctccaagacc 7560
   tggtctgggg ctctatattc agtaatgagg tttcctagcc tcccctggtg acacacccag 7620
   gtctgataaa tctctcacca tagacaagtt tcccagcagt agggagctta aatgttcctt 7680
   tacctaattc ttccataact catcataaaa tgggaaaaaa aattctaatt tcctcctggc 7740
   tggctaaaac ctaagagaga tggaaggaaa aggtgttcca gggtgctttt gaggttgcac 7800
   gtggacctga gttttagcag atgggccttt gcctgcctct gccatggggg ctcgcagctc 7860
   ctcaggcctc ggtatcacag aggtcttagt caacgggtgg gtaacaaaac atggacgctg 7920
   ctttggcaca cagggcacac tgaggggagg gagaacagtg taagagaagc atgctcagcc 7980
   agatagttga aggacgtccc tcgtaagccc aagctgtgcc cacaattggg cttttaaaag 8040
   gtctagaatg ggtggtggtg ggcaaccaga agtttgtttg tatgagagcc agggagatta 8100
   attattttaa gaggacagga atgagggcac tataagcttg aattggtcag tgcaaggcct 8160
   aagctgggat atgggaggaa atcccacatg catggcgctg tctgccagtc aggtttgtct 8220
   cccaatccag attgtggagg gaaggaggag ggtgagagca gagctgctac aggcatcctg 8280
   taaaagctgt ttgactccaa actccgtgaa acatggagcc caggcagcac actgattggg 8340
   gaattctctg gccaactgtg acgaagtaag atgttgagca gaggcattct tccagagttt 8400
   cccgcagaac ccacatggct ttggcccttg gagagctcca ggccatcaga gatggtgcag 8460
   gaatggggta ggagacagag gaagccctgc aggaagccag caaaaacctc acctaccttg 8520
   agaactcagc aatgccggaa gagattgaga ttcctagaga atggaggagc cgggaacaaa 8580
   ggagaaggat gggatccagg aacaggccaa gagaaccagc tcgatgcttc ttcctctccc 8640
   ttctagaaaa ttctgttcag aagccattag gtaaggtaga gcaaggcagg cctctgtagc 8700
   tgagtgggga ggaagtagcg tgcccagagc aggagggtgg agtccaccgg ggtgagatag 8760
   aggctcacac aagggcggtg gtccagtgtg aggttgccca tgtggagcga ggagggtgcc 8820
   aacataggag ggggcagccc agcatgtgtc ggagaccaaa gcagggtccg aagggggtcc 8880
   tgtccaggga ggatgtcctg gcacggagac tcagaccctc agtggggtga ggaggggtct 8940
   acacagaagg tggcccaggg caggatggca gaactagagg ggtgaggaga gtgtctattg 9000
   caagggcacc ttgcgcttgg agtcagtgcc ctggggaagt gggaagggca tccacatgga 9060
   gggtggccca gggcaggatg ctgctgtcca ggcaatggaa ggaaggtgtc ctcaggaagg 9120
   gtggtaccgc atgagtgtcc tacctgagca gagagaggaa ggtgtttcag tgaccggcag 9180
   ggtggctgag aatcccactc tgctttgtgt cctgagcctc acagagcaca aggcatttag 9240
   tagctgccca ggtaatggta attgttaaat ggatgatgaa ataaatgaga aaaatctgga 9300
   ttcaactctc atcttgacca tatattagct atggaacttt ggatatgatc ttcatctcca 9360
   tcagtgggat actgtgtatg aaactactca gaaaagctaa aggagctgat tttactccac 9420
   ctaacaggaa agaaggctct gccattcttt gctgtgcaag cgttcctcat atcaaagaat 9480
   ggcagagccc tctttccctt tgggtggagt aaaatcagcc tagccttccc ttagcgggta 9540
   ccatgctgcc cagcctcagg tgcgtccttt gtgaggtgag aagtgtatgg agctgctgag 9600
   tgatgtgtgg ctacagtgga tgcggagaaa ctcacctgca gggagagaac aggctgtggc 9660
   cattggtgtg caggcctgtg gactttccgc atgcgttgtt cagcttattc agccttggtg 9720
   gtctctgaga tctatggcag tgagcgtctg ctccctctca cagcaaacac cacactttca 9780
   ctgaaaaatg aggctattag tcttgttcac agagcaccag gatccctccc agttctttca 9840
   tgtgtttgtt tgttgttttt tgagacagag tcttgctctg ttgcccaggc tggagtgcag 9900
   tggcgcaatc tcagctcact gcaacctacg cctcctgggt tcaagcaatt ctcttgcctc 9960
   agcctcccga gttgctggga ttacaggcgc ctgccaccat gtctggctac tttttttgta 10020
   tttttagtag agacatgttg tattttagtg gagatgttgg ccaggctggt cttgaacttc 10080
   tgacctcaag tgactatggt tctacaatcc gagttctcct gttggaccag tcgagtgacc 10140
   ttgggcaaat agcctcctgg cctgggcagc tgaggacagt gtgtgagctg tgttctgagg 10200
   cctcaggcct cagaatttcc ctcctgacac tgtgggcctg agcagacaga aaggacaaac 10260
   ttcctgccca gagcgtgtgg tctgaaggtg cctcggagac cctctgatca atctttgacc 10320
   ccttccccca cacacactac agcagttacg tgtctgttaa aatacacgtt cataaaagac 10380
   cacctactaa ataattttta agttgttatt ctaaaccaaa ctattttgaa cttttgtaaa 10440
   tgcagttttt atttttaatt aatgacatta tctttgcagc cctgaatcag actttcctgg 10500
   acacttttgg gtgttcttat ctgcatcaat ggggcctcag ttagcatttt aaaggtgaca 10560
   ttgccccaga ttttatctcc accagatctt ctcttctgaa ctgcagatcc agatatcaaa 10620
   cggcctcttg cacataatga cttggctatc tctcaggtcc tccctgtcca cagggtgggt 10680
   tctcaactta tcaactccct cctctgacac cactcctccc ctgaaagggt cctcccactc 10740
   ccctcagaga acagcagcac tcacagcagt cagcccctaa atccagctga tctttgtctc 10800
   cattgccaat atcactgttc caggcaccat cctctctcgc tggtattact gcaaactgcc 10860
   acccagctgg tctctccacc tccagccttg ccacgctgcc cccttgcccc gtccatgtca 10920
   gactctacca cgctgacctt tggaaaatgt aactctgtat gcatcacttc cctactgaaa 10980
   actctgatag gtcactatgc cctttaagat aaagtctgaa atcctcagca gggttgataa 11040
   gcagctctat gtgggactcg cttttccagg ttagcaaggg acttccctga ttttagcact 11100
   gaagtcctga attctcctta gtccttggca aactggatgg ctcaccactc ccctcttctc 11160
   ctcattcctc tgcctcaccc ctaccccaca ctcaccctct gtctgtcccc tcagcagtct 11220
   tcctcctgct actgtgaatg gtctctgccc cagccccttt gctttgtaac tttgagatcc 11280
   ccctcctagt ctcagtgggc agatggcttc ctccatgctg tctcctggac accccgggtc 11340
   aggtgactcg cccctgctgg gtggcactct tagttcccct tgtggcactt aaatgcgtta 11400
   tttcaatcac tgggttaccg gaggtctcct tccatgactg tagatgctgc aggcagaagc 11460
   tgagtcgtgt ttgcccccgt catcacctgt tgtctggcac aattgcctgc tgcggatttc 11520
   agtggtgatt tttaggtgaa tcgtgatgca cccaggcaca gcctatacac agaacgttgt 11580
   gtttatctgt tatcttggct ctgagacagg cagtctcaga aagtgaaaaa tagctggttt 11640
   ataaatacat gataaaatgt tgacaatcac tcataattaa gtagacatac attaaaataa 11700
   taacgttcac ttttttcgtt gagacagagt tttgctccgt tgccaggctg gggtgcagtg 11760
   gcatgatcat ggctcacggc agcctcaagc ttctgggctc aaggtatcct cctgcctcag 11820
   cctcctgagt ggctaggact ataggcacat gccaccaaac ccgactaatt tttttttttt 11880
   ttttgtagag atgggtttta ctgtgttgcc caggctggtc tcaaattccc agcctcaagc 11940
   aatcctccct ccttgacctc ccagagtgct gggattacag gtgtgagtca ccatgcccgg 12000
   cctaaggttc attttttacc tactagattc ataaaggtca cacaatgtga agaagcggag 12060
   gacaccagca ggcgctctcc tccctggggt agaagcataa atctgcgaga acccttgaag 12120
   agtaacttgg caatctttcc taaaagctta cctgtacata gttttttttc acagtttcac 12180
   tcctagaaat gtattctaca aatactctta gagatttgta cagaaaagag caaacaagaa 12240
   tattcaacac atcattgtta gcaatgaaaa taaaagggaa aaaaataccc tacctatcca 12300
   tcaaaaggaa actcgttaaa taaattaggc aagtccttct aacagagtat tataaaggaa 12360
   ttttttcaat gaggtagatt tacataaggt tatatgaagc aatcctcaaa gcagggggag 12420
   ggggcaggaa atgaaaaagg tctacaattt gtttattttt gttgtaattt taaaaggaaa 12480
   tatgcacatg tgttaatatg cataaaaata tttctggaag aatacacaag aaactcgtag 12540
   cagtaattac ctctggggag agagactggg atctgggata ggagagaaat ttattcattc 12600
   tatattctgt cttaagtgtt taaataattt attacaggca taaattcacc cttttttttt 12660
   ttttttttga gacggagtct cgctctgtcg cccaggctgg agtgcagtgg tgccatctcg 12720
   gctcactgca agctccgcct cctgggttca cgccattctc ctgcctcagc cttcagagta 12780
   gttgggacta caggcgcctg ccaccacgcc cagctaattt tttatatttt tagtagagac 12840
   agggtttcac catgttagcc aagttggtct tgatctcctg actttgtgat ccgcctgcct 12900
   cggcctccca aagtgctggg attacaggtg tgagccacca ttcccggcca taaattcatt 12960
   tttcaatact acaaaaagaa aactccgtgt tttttttttt ttaaagaaga aaaaagctag 13020
   ctagaaaaaa gcatctgccc gaggttctag caattttcct tccttccttc ctttttccat 13080
   cttttctttt ttttctttct ttctgcccat gctctcctcc tttctaagaa aactgtcttt 13140
   ccagaatctt ctaacaatat taagtaataa gattagacta gctcttcgtc tctgagctgg 13200
   gagtccaggc tggactgggt gagtttaccc ttgcgtgagg ctgagtgcta atgtctctca 13260
   cttactaatg gggtaccttg cagcctgcct ttcatcctcc tgcccacctt ctccccagcc 13320
   atgtcctggc atcagctgca acagatccca gcttctcact cctcccactt tctgctcagg 13380
   ggctcctctg ccaccatgat gtgagatgct gaggacactc attttgtcat tgtgatatgt 13440
   ttggaaacct ggctgtggga gagggttaac aacccccggg atgacctgtg atccatgggg 13500
   tcatccctca cagcttgcta ggggtcccca gcggtatcga gctccagttt ctcactggcc 13560
   tgaccagctc aataatttac cttggttggc ttgtcctctt tccgtttgac tcttccgagc 13620
   ctctcactcc tgtttcttgg gatcactttt caaagtaaac caccatgttg tgctaggctc 13680
   tgcttgttgc agaaacccag gctaagacac catctttcat cactcctgga gagttctgga 13740
   gtccttgtag acctgtggat taagaccaca cttcattcaa agagatccaa caattttccc 13800
   tgggagttcc tctctctcgc tttctcttcc tgagatgtaa cctttcccct ctaggtcctt 13860
   aggcctgcct ttcttcctct ctattaatgt gaggatggca ttttgctttg tgccccccca 13920
   cctcaaattc tgtattgaaa ttcaatcagc aatatgatat taggaggtgg ggcccttggg 13980
   aggtgattag atcaccacct ttgggatagg atcaatgccc ttattaagga gacaccagag 14040
   agctgcctgg tctcttccac catgtgaaga cacagctaga aggtaccacc tctgtaccag 14100
   agagtgggtc ctcaccagac accaagtttg ctggctcctt gatcttgaac ttcccagcct 14160
   ccagcactgt gagaaataaa ttcctgttgt tcataagcca cctcgcttgt agtattttgt 14220
   taaagcagcc tgaatggact aaggcacctt tataaaccta ggcattctat tctaaaattg 14280
   ccttccagtc caacagaagc atgttataga ttctccaggt agaagcattt gttgtaaaaa 14340
   ttaactgtac ccttgacatt gagatcatct tgtatcaatt ttagatccct gaactctgat 14400
   ttgatgattt aaaaagcagg agaacctctg aatctaactc attccagtcc ctgcggtgga 14460
   aggcaaacat agttcactca tttggaaacc aaggttccct gcacagaaag tgccaggcca 14520
   acccagcgag ccctgatgca aggtcactat ttattttgtc atcctgaggc gagagttcct 14580
   tcaaggggct gctgttggct ttcaaacaag cctggaaaat tgtctggtgg cctctttgat 14640
   cctgcccctt tttctgggtg gtgctctctc ttttctctgt gttccacttt caaaataaac 14700
   catctcagtt ggctgtgata aattcagtga aggaaatttc aagttgagaa tggtgagaag 14760
   acctgcagaa gggaagctat aaatagccca cttttaatgg ctatagtaac ataattccct 14820
   ttacattact gtctgccatt aagcaataag taacattgta acttgaaaat cggaccaaaa 14880
   tagattgcac catctaccca gacccaatat ggtatgtgta aatatgcaac actgccctat 14940
   tatttataga tctatgattt tccttatgaa acggattcat ttgggtgctc tgtagacctg 15000
   agttaacttg atccaacaaa atccaacttt gtcatagaaa cctacaagtt tagagcaagg 15060
   gggactttta ggaatcacgg agttgaaagc ctgtcctttg cctaagctca tgcagggaca 15120
   cagggatggg tacatgtgag aaggaagtag agactgattc caactgaaca cctggagaaa 15180
   ggcctcggat tctcagattc aatgaaaggg gccttcacat gcctttgtta tctatcacaa 15240
   catgagatct ggaggctgca atcaccattg ttcagtggag gaaataggtt cacagagtga 15300
   agtgacttga tccaagttac atagtaaatc aatggcaagg aattggactg gaacccaatt 15360
   ccaacttaag gctagtattt ttcctttcat agtggtagct ccattgtttc tggcttctag 15420
   agtcttgggc tggtcagctt tgattgacac gtccacagta agagtcccag ccctgccccg 15480
   cctcccacct atacctcagc acctcagtag gtttctgtga cccctccctt tcttagcctc 15540
   aggtttgttc acactacaga tggggtgagc caccccaagc ccctatcacc acatcgtctc 15600
   ccactcctgg ccccaaatgc tttcccctgg ccatgctcct ttcttattat tctgaacccc 15660
   ttgtcagttg ttttctgtac tttatccctg aatagtcctt tcaaaacaga acatttacaa 15720
   tttccttctt taagaaaaga gtacttaaaa aaacaaaaac gaaagccacg tttgtgattc 15780
   tcgcttcttc cattacttac cttctaattt tccaagtctt ggcttggcat tcagggcccc 15840
   ctgtaattag gcccctgcaa tgtggatcac cttctcccta tccctgtgtt gcttgtacct 15900
   ctattacaac attgcacacg ctcagccctg ctacctctat tacaacattg cacacgctca 15960
   ggcctgctac ctctattaca acattgcacc tgctcaaccc tgcaatattc caactaaaga 16020
   gcatgggaca ctgaattcgt ttttatgtcc agtagaacat ctgccacaca gtcacgcaca 16080
   tagtaggtgt ccagtgacag tgtattgaat ggaatgggct cattcctgct tcctgcagca 16140
   tggtccctat ggcctgtcta gagcacatct ttctatctgg gggcaagttt gggcctgtca 16200
   ccccctgcag tagacactaa cacacaaagg ggtgagatta gcccctgtgc atagttccaa 16260
   agaacgagct ggcctaaata atacttctga gtgtgctacc agtgcaactg agttatttct 16320
   taaaccatca catccaggtg cctagtatgt ttctaggaac tttatctgaa agcatcaatt 16380
   tctgtcactg atcccccaaa ttgtaattgc tcaagaccaa gtgaggacta aaactccgca 16440
   ggcatgacag ccctgtgttt tggtgctaca tgcgggggga ctgaatgtct tacagaggag 16500
   cttacagtct aatggggtgg ggtgggcagg gaaacaaacc tgcagagagc gacaataggc 16560
   aagacaagag catgaagaga gacactgtgt atgggtaggg gtggctggga gaatgaggat 16620
   tacatgacag agtttataga aagtgcctgg cacatagtag gtgttcagta aatggtaacg 16680
   agtcatattc tcactaccac acaagcagct gggttcacag aggatcaagg cctgtgcctc 16740
   cctctgactg ccatgcctct tcctccccta tcctgcccac tccctctgct ccggcctggt 16800
   cttctgatca tgaggaacat gccaagttct ttctttcccc agcagggaaa agctattcct 16860
   tattccagaa atctgtgttc ccagcttagg gcaagactgg ctgctgcttg ctctttaggt 16920
   ctcagaataa atgccacctc cccttgcctg accactgcac tgccctgccc cagagttatt 16980
   ctctatcaca tcacactggc tagttccttc tttgcaatta ccagaagctc taaaaatttt 17040
   gtttctttct ttgcttgttc cttgtcccac taggcttcag gctcgagaca gagaggaacc 17100
   gcctctgtgt ttttcactgt cgtgtctcag tgctgggagt gtactccatg tggtcagtgt 17160
   gtgatcaatt taactgtgcc tgagggttct gagaagacct cacaaatgaa ggtacctttg 17220
   accagagcca cggagagggg acaagagttt acccaccagg cgctgcaaag ctgaggtggc 17280
   aggtcaggac ttccagtgag aaggaggtac tggtaaagga gactgttatt atgagcagct 17340
   ccgtccagga gaagccaggg ttcctggtcc aaaacagcac cctaggttca aataggaata 17400
   ccctggggtt ggaggtgagg gaagggtggt ggacagctca ctcagctatc agaagctgag 17460
   ggataagtcc ccttcctcca atctgggctt tcttagcagc cttttgagct ggaatttgtt 17520
   ttgttttgtt ttgtttatgg tgggacctac ccactgtgca gggaggatct tatcaggtgg 17580
   ccaagcttgt tttttgtttg tttgttttga gatggagtct tgctctgttg cccaagctag 17640
   agtacagtgg tgtgatctcg gctcactgta acctctgcct cctgggttca agcaattctc 17700
   ctgccttagc ctcccacgta gctgggacta caggcgtaca ccaccatgcc cagctaattt 17760
   ttgtattttt agtagagacg gggtttcacc atgttggcca ggatggtctc gatctcctga 17820
   cctcatgatc tgcccatgta ggcctcccaa taagcctggt ttttggaaca atacaaataa 17880
   tgaaaatttt aagtactgac atttattgag ctcctattgt actattttac acacatctgg 17940
   tttaagccac agccactgca caagattggc atttttcccc atttccagag gctgaaactg 18000
   agattgagtt tcatgtccaa agagtgagta acatgtgtaa agtcatgggg ctaggaagtg 18060
   gtggatggag tttcaaaccc agctgtgttt gaccctgaag ccagggcgct aaaccacaat 18120
   cttttttctg gccagtgtat ctcacaggtg aatcaattca ctgggtttgg cgcattccct 18180
   tgtgggctga gtcaccataa gtcagactgc ctgaacctgt tcctctctgg gaggctgata 18240
   gaaacctaat ctctaaactg acaatgccag gggcaggact ctggactttg gtaaccccat 18300
   gatggggtgg ctccctactc tcccactcag agctgcagat agcttttctc caggacagca 18360
   ggactcaagg gtgctatggg ggttagttat tgcagacatc ttgctttgct ttgctgtaga 18420
   gtggggtggc taagagcttg ggtagaggca gacagactgg tttgaaatcc agcctttgca 18480
   cttcgtagct gtgtgacctt agcttgggca agtttcttta ccgctctgtg cttcaactct 18540
   catctttaaa atgaggctga gaacagtctc atggtgctac tgtgaggatt acgtgaaaaa 18600
   acgcgcttgc acatagcaaa ccctcagcaa tcattagcct ttatcattgc tgttgttgtg 18660
   actggtatat atatatatac tttttttttt tttttgagtg gagtttcact cttgtcgccc 18720
   aggctggagt gcaatgtcgc aatctcggct cactgcaacc tccgcctcct gggttcaagt 18780
   gattctcctg cctcagcctc ccgagtagct aggattacag gcatgcacac cacgcccggc 18840
   taattttgta tttttagtag agatggggtt tcaccatgtt ggccaggctg gtctcaaact 18900
   cctgacctca ggtgatccac ccgcctcggc ctcccaaagt gctgggatta caggagtgag 18960
   ccactgcacc ccgcctgttg tgaatagtct taaggggagg cgtagtggga tgtaatgggc 19020
   tggagtccta ggccaccact ttttagccat gtgacctttg agatgaacta ttaaacttca 19080
   tcccagaatc tttctaaatt ctccaaagtt aaagatgaga tttaatagct catctattaa 19140
   agatttctgg gatggcattc ttaaaattaa gattagcttc tgttaaggaa ttagaaaaca 19200
   ccagaggtag ggaatggtcc ctgtcacata tagacagcat ttgccctaaa ccatgctctg 19260
   gtcaagagga ggctgaggac ttctgagcca tttgcctctc atcagaacgt cctgggtgta 19320
   gtgtcagaga gtggctgtat tggcttgtct gaacagctag tgtgttgctt tggtttgagc 19380
   tgaaagattg ttctaacaaa ggtgcatggt gactatggtc agcataggcg ttaggagagg 19440
   aggggccgtc tgtgcacact gtcctgggtg tgcccatcct caggagctca gctgtgtagt 19500
   gtgtggcagc ctgtccccct gactcccgcc ggtgaggttc acagatggtc agagctccca 19560
   ggtgcggctc taaagctctg gctctgctgc cgtggcccca ggcaacctgg ctggctcatg 19620
   ggcttcagct tctcacatgt ggaagagcac agatcatcct tcaccccagt atcccggtag 19680
   ctgacctcag actttctgag tgtgagttga cttgagtagc cctgagatgc tgacgtcctc 19740
   tgagtacatc aacaacaggg agtttctggg ctgcaggctg agaacccgtg tttgtcacaa 19800
   ggcggcccca cagagttacc accactctaa ggccctgcca gagccagcaa cagattattc 19860
   cagaaggcac ctgtctccca atcaccttct ctatctggaa caacacctcc ctcagggttg 19920
   ggtaaagtat taaatatctg tgccttgcat acactcactc agtgctcaac aaatgctggt 19980
   tctttcccag tttctgtaat ctgtatgacc tatcttgacc aagagattgt catcatcctg 20040
   aggcccgggg cccaggctgc tgttgcgctg gcctctctcc ctcactctcc agtaagccct 20100
   gtcccaggct ggcctctccc tccggggctg tcttatccac gcttgggctc caaccacctt 20160
   gacccttctc cacctgtcag tcacgcccca ggtgttccct gagtcacgca acagctatat 20220
   agtagctccg cagaccaaca gcaggccgac agcgtagcac ctgagttcta gatctaccac 20280
   tgttcatgcg ggacctggca taaagcattc ccacgtctcg gcctccattt ccccatctgt 20340
   gaaacgagag cactggtcag ctcttcacat gcagcgtttg tgggatgaat ccttccatct 20400
   gtccatccat cccgccaaca tccatgcaca aacatttagc acctgctgca tgctgtcagg 20460
   gactatggca ggaaaagagt tgaaacagac acgtcccagg cctccagccc ctcacaccac 20520
   ggagaggaaa cagatgcccg caaaggagga ggcacccctc agggggccag cgctgcgaca 20580
   cggtggaggc agggaggccg tctctaagca gcctgcgagt ccccgcgctg caccgtggct 20640
   ttgaggagtg gcgggggccg aggggccggt ctctcttccg ggggagattc ctcccctcac 20700
   cccggcatta ctcagcgccc tgaactgtct gcctccctcc agccggccct cctcgtcttc 20760
   ctggagcgca gcacaaaggt cctgttgtgg ccgcggcccg ggccgcgggc gagcctctct 20820
   ggatggtggt gactcagtct cttgccattt cctttgtgcg gctccggggc tcctcgctgg 20880
   gagcggagtg tgtctgcagg gctgggggcg ggagccggga cccacagccc gacagcgagc 20940
   ctttcatgtg ggcagggggc ttcccgcacg caatgcgaac ctacgcctgg agcttcccaa 21000
   agacagcgtc cggcgcagga acgcgagatg ctggccgcct cgcctctggc ccccgcgagc 21060
   atcaaagcgc gtgccgtcga gggggcacgt gtcccccatc gggcgcgcct ctgctcctag 21120
   tcccctgaat gaacacatcc ttatgaaaag tggaaattag ccgccgaagg gaagggcccg 21180
   gagagcttgg gaagcgacct aggctgccag cctcccggcg ggtctttggt cccagctctg 21240
   atgacctgtc agcagccgca ggacctgcgc ttaaaggcgc ctctccaggc ctggctcagg 21300
   gtggggctcc tcgtgctcag caccctggtc aaggttaggg ctgggtgtgc gctggaggct 21360
   tggagtgcca gggcacccag tgaagagcga atgaatgaac gagtgaacac cggactgggg 21420
   gcctgcagac ggttcttcgc aggttaatta gaaggcctct agcaagctgc agcggaggca 21480
   tttcaggcat ccttcagaga ggaccctcgg cctggctcct aagggcccct ggtctggctc 21540
   tcaccccctg ctctggtggc atttctgctc actcagcctt taccctttac acgccagccg 21600
   ctctgaacag tttattcttc tgggagcaca tcatgttctt gctcacatcc aggttttgcg 21660
   taagctgttc ctgctgcaag gaaaaccctt cctgtgtcct cctcaggtga gagctcccgc 21720
   aggcagcttc tccccggagt ctggcgtaag tgttcctcct ctgcacgcct ctgaggacat 21780
   ttgcaaaggt ctgtcatagg gcttaccacc ccgttacctg tttgtttcca ccagggagtg 21840
   cgctgaagct gagagggaat gagtgagtga atgaagggct tgagaaagga aatttcttca 21900
   ccctccagtg ccctctgggt gggtattgag acaagctcta caggaagcct aagaaaacta 21960
   actgctctaa ttctgtcctt ccttttgcaa tgtttaggca actcctgtgc atctgtaggt 22020
   accattgtga ggttactgcc ctggactggg gagagatcca cacacgcaag agtgcatctg 22080
   gcatcagtat gtaagagtca aaactgacac acagttcttc atctcatgga aatgaaaaca 22140
   tgcatggccg gaatgatctt atacctgctt ctcttgggcc aggtcaccag gctggccctg 22200
   agaagtatgt gatggcatgg agaagaatcc tcacacatcc ttgcagttct ctaggatttg 22260
   caaggcattc cccccattgc tggactcact ggatttgagt aggcctgtct gcccctattt 22320
   tagagatgag catatacacc ttacaaaaac tgagtcttgc tcaatgtctg gcaaagaagt 22380
   cttcctttat ccaacaatcc tgagagtcta gcgggtgttg ggcaccataa agatgaatga 22440
   ggtgcggttc ttgccaccag gcagtgcccg ggcttgtcag ggagacccat gtggagcatg 22500
   acaattgcta ccttcttgat acgggcaaaa ggatgttgag tccagtggaa ggagaggctt 22560
   gtctggctgt gagatcagca aagtcttcac agaaaaggag aattttaaat ggggccttga 22620
   aggggaagtg tctcctggag atggagagaa catagcgagg agagtgcggt gaggtggggg 22680
   gtggcagggg aagcacatca ctacgcggtg cttagggccg agtctgagcc aacctgggag 22740
   tggcactcag ctcccccagg cccccgagat gcatgttcag cagcatccag cggcctcctg 22800
   tgaagcgact gggagtccac agagacaaca aaagcagatg tagtgatgtg taaacttgaa 22860
   gtttctagaa gttcctttgg gccccaccaa atctctgtgg caatgaacca atcccacatg 22920
   ttcctgtggg taatgctccc agagggcatg tgactttggg catcattatt ggctgaaaaa 22980
   ggaaactggg gtagtagtta gagagtgcta tgaactgaat gttgtgtgcc tcccaaatct 23040
   gtacactgaa ctccaaatcc cactgtggtg gtattaggtg agggggcctt gggaggtcat 23100
   taggtcctga gggtaaatcc ctcatgaatg ggatgagtgc cctcacacat gagaccccag 23160
   agagctctct ggcggtcttt ctaccatgta tggctgcaat gagaggtctg cagcctgcac 23220
   ctggaaggag gccctcaatg aacctgacca gactagcatc ctgttctcag acttctggcc 23280
   tcccaaactg tgagaaacaa gtttctgttg tgtataaacc atccaatcta tgatagtttg 23340
   ttatagtagc caaaccgact aggacagaga ggatgtaagg agccagtgga ggactctttt 23400
   aatggagaga ctacagcatg tctcaggctg aatggtccag tagggagagc aaaaacagtg 23460
   cccatgcatc accctgaggt ctcacccaac cctgctggaa ctgactttag tgccctcttc 23520
   cagtgttccc cgcatgacct ggtgggtgca cacctggctg cttgcattca caccgtccct 23580
   ctccacttct tccttctccc cagctctctt cctgtcaacc ctggcattgc ctttgttggc 23640
   tactgagaga agaccagaaa tatccatcta gtcagacttg cctgctcctg ggaagaaaac 23700
   aaaaatgcaa agaaaaggtt ctcagggaga ttgagccaac ctgggagtgg ccctcagctc 23760
   ctccagtccc cagagaccca tgctctgcag catccaaggg gctcctgtga aactgttggg 23820
   aggccaatga cactctgtcc cacgttgtgg atctattttg tggtctctgt atccttcact 23880
   gctttttgtg gacattattg tattagattc ctttggaaat gatgatccag ctaatggcca 23940
   gttagtagga aacatcacga ccctcccccc aaattccagc ctcttgctga aagggtgggg 24000
   cctgggaaat catatggtat tgctcctggt caccacagac cagtggtggc tttgggccaa 24060
   attctggccc agccaatccc ccccaccttt tttttttttt gagagagaga atttgaggta 24120
   agagacacag agactcaggt taggaggatg cttgaactga aaggtcctga gacagcagga 24180
   caggagctag taccatgcag aacacagcag gagccacagg caagcctgaa ctgtgggaga 24240
   gagaaaacga agggccttcc agagaggtcc agtctgccaa gaggagaaga gagttgacac 24300
   gcagaaagag ttaggggtga caagaacccc cgtagcccct tgaaggacac agagaggaga 24360
   tcttttttgt tcttttgtga ttcatccttt ctcacttcct gtccttggag gtcaacaaca 24420
   gacccctgta tccttttaat aatccctctt ttcatttgaa attgttccag agggtacctg 24480
   atccttgttg ccagcatgcc ctgaataaga aaaatgctca aaccaaacac tgagtctact 24540
   gagtctatgt gaattaattc ttggaattcc ttggctcaat tcaataaaca tttcatgcac 24600
   aatttttgtg tgccaggtgc tagaggcatt ctgagaaaaa ctgagaccca ggagctgtcc 24660
   tcagagagtc cagtaagatg tggtctgata agacatgttt ccatataccc agaatacaag 24720
   gtagtttgct aggagcactg caagagaaac gcagatggaa ggctggagtg tgtactgcca 24780
   ctgtggaagc aggaggggct tgagggaaga gggccttcaa gaatgggtgg aatctaatat 24840
   tggaaagggg aggaagagtg gaaagtataa gaatcagaag tctggttgca ggatggggca 24900
   tgatatgggt gggacactgt aaactgcaca gcgtggaagg aatatggagt ttagaggaat 24960
   aatgttagat gagtctgtaa gacgggcttg actgggatta tgaagggcct taaaggtcaa 25020
   gccaaatttg ttatcataga atcataagaa ggagaagagc cttagctgtt gtctagtcta 25080
   acttaggtaa tttggttcca gttcctctta aggtggccac catgacagac tgaatctcaa 25140
   gtactcatct gagatacagt catttcaatg gtagtgactc agaatttact tttctttttt 25200
   tttaccccag caaaatattt attttagaaa ataggattat attatttgtt gctctccaac 25260
   ttgctttttt tggacatctt ggacatcttt ctctaccagt acatatagtt ccatttcctt 25320
   ctttttattt tatttacttt attatttttg tagagatgag gtcttgccat gttgcctagg 25380
   ctggtctgga actcctgggc tcaagagatc cactcacctt ggcctcccaa agtgctagga 25440
   ttacaggcgt gagccactgc agatattttt atttttattt ttcatttatt tcaatagttt 25500
   ttggggagca cgtggttatt tgattacatg gataagttct ttagtggtga ttaccgagat 25560
   tttggtgcac ccatcaccca agcagtgtac actgtaccca atgagtagtc ttttatccct 25620
   cgtcctcctt ccccgctccc ctcacgtccc caaagtacat tgtatcttat gcctttgcat 25680
   cctcatgaga ttacttttct atctttaaca acttaagagt tctttccaga agagtcctct 25740
   tactacgagg aagagtgtta gagcacaggg aactgggcct agagagtctg cagcaaattg 25800
   tctcaaatag cagcttaaaa aggtaaggaa cagaaaagaa acactctcta tctatgtgct 25860
   ggcctcaggc acagtgcttc tagatggtag aacatctgtg aagtagaaat tacaatcact 25920
   tttcaggtga gaaaacagga gctgtaggga ggagtcggac aggtccccag gcccagcaac 25980
   tataccaggt gaggcaggaa tgtggcctcg tgttgaccag attccacagc cacgggcttc 26040
   catccctctt ctctgcttgc tttaagacta accaccctgg cttcaagtaa gtatcactca 26100
   caaaggaatt tcaaaaagac atgcacataa attaatggct gatggctgta ggtggtcgta 26160
   tgggaggaac cgattataac attgtatgaa cttagcaaag ctctaaacca tagcacagac 26220
   atttggcaaa atggttactc tttttcaatc atccaaggac tcagtttata ataagccttg 26280
   agcgagtccc taaattccaa agcccttagc atcttccagg accctcttga gaccacacag 26340
   tgcttccagc ttccggaaac agcagatgct attgcttcca tgagcaatgt cagcatttaa 26400
   tacacaaggg ggaacagaga aagaaaccta tatggatggg gagcagggag gaggggcagc 26460
   actggatcct ctcctgggcc ctgtccagct cgacattcct gaacagagcg tgctgttccc 26520
   tctcctgatg ttggcagctt agaatgcagg agccttagat gggtggcctg gaagggaggc 26580
   cagcagggag aggtgacctg gaagtgagga agctcaggag ggactgatgg taattggagg 26640
   ggcaggcaga gagaggcaga tgaagaaaca gccgaggcct gtgtgaaggc tggacccaac 26700
   ctgcgtccaa gcagaaagtc cagagaactt agcacagctt ccaaagcttc agagtacatc 26760
   agaactactt gggaagtgcg tcaaactctg gttcctaggg aaatcctcag agatcctgca 26820
   tctgcgatga ggcccagaag tggtttttgt gaccagttcc aaggcgactt catacaggca 26880
   gtccttgggg acactggtga caggatttcc ctctctggac cttggtgtcc tgctctgtaa 26940
   ataaaggggt ttgtcaaaga cggtttctgc tttctctgtc agctccaaca tacagcttct 27000
   caaaagcttc caaaaatccc cttacccaag cttgcagccc tcatcacctg tctcaagata 27060
   atggtttcct attcttttta atgtataagg cagacaggtg gtcatgtacc tgctcagctc 27120
   ttgaaaagga cctacatgat agactctagc aactccacct cgtctgccca ttttggggga 27180
   gagtaaagcc acgtcgtgtg cgttcacata ggcatccaaa tgggacagac ttcctctctc 27240
   tggaggccgt ggctccaggt caaaacaaaa ccacttccag gtctcaggtc acaggatcct 27300
   gcagaatgat ttatttaagg aagaaagctt caagggctcc agagaaatta aatgtgtata 27360
   tttccccagc aattatgcac gtcctttccc ttgaaaaata gttcccatgc cctgcctgga 27420
   agaaaagcca gattctccat gatgctgtgt tccacaggct tctagaggtg ggggggctgt 27480
   gattttctaa caggagaagc cacatgaaag tcttcagcag ctttgcgctg tttctgctgc 27540
   agcacctctc agggcagact ggagaatcag ggagtcttgt ctggaaaaac gagacaggct 27600
   gggcctgaca cattgatttc tggttcaagt ttcccagtga aatgtgaagg aggaattttc 27660
   cacttcttgt cctgattcac cttggcgaac cagtctttgg agtcccagac tg 27712
<210> 26
   <211> 1863
   <212> DNA
   <213> Homo sapiens
<400> 26
   cttcctcggg gtttgcttcc aggcctgact tttactcccc tttctaagtg tgcagatggg 60
   atgtgcttct ccacaggagg ccccacggct tccccacccc tcagaggagc gccgtgcgtg 120
   cgtctgtgtg gaggattggc agctcctgca gtcggccctt ggtcctattt ggcgacgcct 180
   ctgccttccc cttaattata cagtcatgag ccgccctgga atcacggcag ctccggatgg 240
   atcctggatg ccagaatgca gcctcagcac ggggctgcag gacaggagtg agcgaggggc 300
   tgcagagccg gcggccgcgg tgggcaccat ggagggggct gccctgggca gcacgggcat 360
   gagtctcaag gcccaggttt gagtaacagg tgttgagagc ttacttactt ttcctgagac 420
   acagtttcct catctcgaga gcacggaaaa tcattctaac ttcagaggat tgttgtgaaa 480
   gttaaatgag attaaagagg taaagcccat gacgtgctta gctcgtgctt ggctcttggt 540
   caatgccagt tagcgctgca ttttctcccc tctccctccc tccttctctc tttcttttct 600
   tctattctcc attcctgttt tctcccccac cccactcccc aaagctctgc gttgagaacc 660
   agatgctgtc tggtgggtta gggccagagg aggaaaagct gcccgccgtg ggctgcaccc 720
   ataccctctt cattccaatg acatgagggg aggggaaagg acagaggtag actgtcctcc 780
   cctacctcct cctaatacaa atggaattcc tggaactgga aaacaaagaa tacccccata 840
   aaaataagac agtacttctg gtgcggtgta ataaagggga aagtaaccct caatgtcagg 900
   aaactccgca cctcccagct catatttgtg tggaggaaaa gttaaatatt aatttggact 960
   caactgaatg tggacacaaa caatggtcac caagtcccgg aacaggttgt gtgagcctct 1020
   tcaggggttc atccagcgct gttttggaga aatctctatt tcaatttatt cctatacgtt 1080
   agttactgaa aaacaacaga caatcgcaaa agcaagttgc ccgttttgtg ttccttgagc 1140
   ccaatcatga agtgccgtcg tgactgggcc tcatgacaaa caacttgtaa caagtaacaa 1200
   cagagctcag gtcccagacc gcactgaagc tctgtgagac ctctcctcat ctgtgcatga 1260
   acgagtgtct gactctggag cccagcctgc tgcttcccag tctggtggtg aatcctccgt 1320
   agtctgatgg aggtttgctc ttgttgccca ggctggagtg caatggcaca atctcggctc 1380
   actgcagccc ctgcctccca ggctcaagca attcttacgc ctcagcctcc tgagtagatg 1440
   gaactacagg gcatggactg gaactcccaa tacccctgac atgggctgag tcaacgtggt 1500
   catgaacatg tgacaggagg cagcagaagt tgcagagaag agtgaggcac gtttgaaaaa 1560
   ggctgaaaaa tgtttctgtc caggcaaggg tgtgtgctga atgactcaag gattttttgg 1620
   gcaacacaaa ccaacacgag ccgtgtgagg atcaggtgac agctgcccaa aagctgacac 1680
   aaggaacaag cctggaggag tgaggatggg tgctgtgaag gaggttgtgc agctgggccc 1740
   gcagtcggac ctggtgagat cagaggaggg ggtgccacca gtctgtggac gaagatgaga 1800
   agctggaata gagcagaaaa caggaggctg ccactctcca tctttcccaa agtcactcca 1860
   gga 1863
<210> 27
   <211> 1402
   <212> DNA
   <213> Homo sapiens
<400> 27
   ctgtctcaag cctccaatca acagatcaga cagcttgtac tcacaggcca aggacacgtg      60
   gaaagaggct caattttcta gatgggtggc aacagccatg atcttctgtc ctctgggtcc     120
   ccacaagcct ggatgaactc aagatctgac tcagtggcac agtgaggaga cctttgaggc     180
   ctcagtgacc atccttggac ttcacctctc acggctttca ggcagagagg ccctcccatg     240
   cccacaacag gctgagccca gccttcctcg ggtttgcttt ccaggcctga cttttactcc     300
   cctttctaag tgaggcagcc atgactggcc acttcatgtg ctcctggaga agggcttgca     360
   ccagccgttt tcaggaaagt caagcagctg ttgactcctg agtctgggtg aatttgtgtg     420
   aagagcataa ggcgctgttt cttaaccaaa acgcttcctc ttgcagtgca gatgggatgt     480
   gcttctccac aggaggcccc acggcttccc cacccctcag aggagcgccg tgcgtgcgtc     540
   tgtgtggagg attggcagct cctgcagtcg gcccttggtc ctatttggcg acgcctctgc     600
   cttcccctta attatacagt catgagccgc cctggaatca cggcagctcc ggatggatcc     660
   tggatgccag aatgcagcct cagcacgggg ctgcaggaca ggagtgagcg aggggctgca     720
   gagccggcgg ccgcggtggg caccatggag gggctgcccc tgggcagcac gggcatgagt     780
   ctcaaggccc aggtttgagt aacaggtgtt gagagcttac ttacttttcc tgagacacag     840
   tttcctcatc tcgagagcac ggaaaatcat tctaacttca gaggattgtt gtgaaagtta     900
   aatgagatta aagaggtaaa gcccatgacg tgcttagctc gtgcttggct cttggtcaat     960
   gccagttagc gctgcatttt ctcccctctc cctccctcct tctctctttc ttttcttcta    1020
   ttctccattc ctgttttctc ccccacccca ctccccaaag ctctgcgttg agaaccagat    1080
   gctgtctggt gggttagggc cagaggagga aaagctgccc gccgtgggct gcacccatac    1140
   cctcttcatt ccaatgacat gaggggaggg gaaaggacag aggtagactg tcctccccta    1200
   cctcctccta atacaaatgg aattcctgga actggaaaac aaagaatacc cccataaaaa    1260
   taagacagta cttctggtgc ggtgtaataa aggggaaagt aaccctcaat gtcaggaaac    1320
   tccgcacctc ccagctcata tttgtgtgga ggaaaagtta aatattaatt tggactcaac    1380
   tgaatgtgga cacaaacaat gg                                             1402
<210> 28
   <211> 3679
   <212> DNA
   <213> Homo sapiens
<400> 28
   gaggcagcca tgactggcca cttcatgtgc tcctggagaa gggcttgcac cagccgtttt 60
   caggaaagtc aagcagctgt tgactcctga gtctgggtga atttgtgtga agagcataag 120
   gcgctgtttc ttaaccaaaa cgcttcctct tgcagtgcag atgggatgtg cttctccaca 180
   ggaggcccca cggcttcccc acccctcaga ggagcgccgt gcgtgcgtct gtgtggagga 240
   ttggcagctc ctgcagtcgg cccttggtcc tatttggcga cgcctctgcc ttccccttaa 300
   ttatacagtc atgagccgcc ctggaatcac ggcagctccg gatggatcct ggatgccaga 360
   atgcagcctc agcacggggc tgcaggacag gagtgagcga ggggctgcag agccggcggc 420
   cgcggtgggc accatggagg gggctgccct gggcagcacg ggcatgagtc tcaaggccca 480
   ggtttgagta acaggtgttg agagcttact tacttttcct gagacacagt ttcctcatct 540
   cgagagcacg gaaaatcatt ctaacttcag aggattgttg tgaaagttaa atgagattaa 600
   agaggtaaag cccatgacgt gcttagctcg tgcttggctc ttggtcaatg ccagttagcg 660
   ctgcattttc tcccctctcc ctccctcctt ctctctttct tttcttctat tctccattcc 720
   tgttttctcc cccaccccac tccccaaagc tctgcgttga gaaccagatg ctgtctggtg 780
   ggttagggcc agaggaggaa aagctgcccg ccgtgggctg cacccatacc ctcttcattc 840
   caatgacatg aggggagggg aaaggacaga ggtagactgt cctcccctac ctcctcctaa 900
   tacaaatgga attcctggaa ctggaaaaca aagaataccc ccataaaaat aagacagtac 960
   ttctggtgcg gtgtaataaa ggggaaagta accctcaatg tcaggaaact ccgcacctcc 1020
   cagctcatat ttgtgtggag gaaaagttaa atattaattt ggactcaact gaatgtggac 1080
   acaaacaatg gtcaccaagt cccggaacag gttgtgtgag cctcttcagg ggttcatcca 1140
   gcgctgtttt ggagaaatct ctatttcaat ttattcctat acgttagtta ctgaaaaaca 1200
   acagacaatc gcaaaagcaa gttgcccgtt ttgtgttcct tgagcccaat catgaagtgc 1260
   cgtcgtgact gggcctcatg acaaacaact tgtaacaagt aacaacagag ctcaggtccc 1320
   agaccgcact gaagctctgt gagacctctc ctcatctgtg catgaacgag tgtctgactc 1380
   tggagcccag cctgctgctt cccagtctgg tggtgaatcc tccgtagtct gatggaggtt 1440
   tgctcttgtt gcccaggctg gagtgcaatg gcacaatctc ggctcactgc agcccctgcc 1500
   tcccaggctc aagcaattct tacgcctcag cctcctgagt agatggaact acagggcatg 1560
   gactggaact cccaataccc ctgacatggg ctgagtcaac gtggtcatga acatgtgaca 1620
   ggaggcagca gaagttgcag agaagagtga ggcacgtttg aaaaaggctg aaaaatgttt 1680
   ctgtccaggc aagggtgtgt gctgaatgac tcaaggattt tttggagaga attggagtgt 1740
   ctcaccagag gagaccacgt ctgaagggct ttgcatccct ccttggacat gtctaatacc 1800
   taacactcag aaagcatcca gtaaatattc gtggaaagaa aggagtggag aaggggagaa 1860
   aggggaaagg gagtaggcga gagagaagaa agactctgct tcttgcccag ggcctggcat 1920
   ggggcggagg caaagcagtg gggtcctcag ctatgtccca ctgtgagtgc acagcgagtc 1980
   ctgaccttca gagggtgcag cccgaggggc cctggcctgt ctgaagggtg cgccagccga 2040
   gtggcctgct ctgaccacca ggctcaccca tgactacctg ggtggctaca gccagttcct 2100
   gacaatgagt acagcactca gttatcgggg cccttccacc cacacgctgt ccacttcctg 2160
   gggtactgct gtgggcatgt gagtgcttgc tccccggggc actgctgtgg gcatgcgagt 2220
   gcttgctccc cggggcactg ctgtccactt cctggggtac tgctgtgggc atgcgagtgc 2280
   ttgctccccg gggcactgct gtggacatgt gatagcttgc tccccagctc cactagtgac 2340
   actggcggcc cctcgctggg gccttccccg cctgctccgc tccattaccg ctgccgggct 2400
   cctcacgtct ctccttgctg cttcctgcac tggggtgagg agagtggggc tggtcccctt 2460
   gagaccggag aagctccagg cttttaagga aaactgccag ggacgaagag aagatatcac 2520
   ttccccacgt ggttggcttc cagattcaga aggaatgtct gtccttgtgg attccgtacc 2580
   agatgacccc agatgctgcc tcagtactag gtccctgtgg ctctggagcc tttgctgggt 2640
   ctgggcagtg tctcttcctc tccagttcat ccttgggtct cttcaccctt gccaggggca 2700
   ggcttcctgg tgagaggtcg acctcctgca tgaaggctct caagaggcca gttcaaagcc      2760
   aagctccggg tctgtgcctg tggggctgct cctcgatcag gagatggtca ctcccctcct      2820
   ggtctgtatc tgtgggattc tcctccatca ggagatggtc tctcccctcc tggtctatac      2880
   ccgtgggatt ctcctccatc aggagatggt cactccccat cctggtctat acccgtggga      2940
   ttctcctcca ttagatggtc actcccctcc tggtctattc ccgtggggct gctcctccat      3000
   caggaggtgg tcactccccc tcctggtcta tacccgtggg gctgctcctc catcaggaga      3060
   tagtcactcc ccctcctggt ctatacccgt gggattctcc tccatctgga gatggtcact      3120
   cccctcctgg tctataccca tgggattctc ctccatctgg agatggtcac tcccctcctg      3180
   gtctataccc atgggattct cctccatctg gagatagtca ctcccctcct ggtctatacc      3240
   cgtggggttc tcctcaatca ggaggtggtc actcccctcc tggtctatac ccgtgggatt      3300
   ctcctccatc aggagatggt cactctccct cctggtctat acccgtgggg ctgctcctcc      3360
   atcaggagat ggtcactccc ctcctggtct atacctgtgg gattctcctc cctcagaaga      3420
   tggtcactcc ccctcctggt ctatacccat gggattctcc tccctcagaa gatggtcact      3480
   ccccctcctg gtctataccc gtggggctgc tcctccatca ggagatggtc actctccctc      3540
   tcggttgctc agtccaaaaa caacctctct ggaaaactgc gtggaatttt tttttaaaga      3600
   attgaaacta gaactagcat ttgatccagc catctgccta ctgggaatac acccaaagaa      3660
   aaataaatca ttatatcag                                                   3679
<210> 29
   <211> 4435
   <212> DNA
   <213> Homo sapiens
<400> 29
   caaatgcctg gcagcgtcct cggtgcttca cctgccatag ccgacagtgg ctgacctccc        60
   atgcctgttg ccttttcttt ctgttggatc agggatacac tgccatgtgt gttaagaaaa       120
   gctggcctta cctacagggc tggccagtcc cggtcacgtt tctagtaagc cattgcctta       180
   cataagggta acggcatggg acgctatctt agccaatgtg ataaaagtgg acatgaggtg       240
   agaggcttca gagagaggtt ttaaaaaaga gacaaaagca ggacgttgcc tctcttcctc       300
   ctctccacgt gtcctacccg gatgtgaagc caaaacagat gcaggcttag tgcaaccatg       360
   gggaacccag cataagcaca gattcaacag cagaagagtg gcagagggag aaggtgaaag       420
   gaacctaggt tttcctgtcc ttgttgagtc attcagttaa aaatccctgg aattttcctc       480
   tctccggcag tgtgttttgt gggataatga gttgccttat tggggttggc ttgctagtcg       540
   ggatgtttcg ctcccatcaa catccatacg cttgctctgt gaaccaatga cctgatgagg       600
   tagtattagc accaccatca ttatgctgag gatgagattt atggcacagt ggttcagtag       660
   cttgcccaag gccatgcggc tggtaggttc tggaggaggg ctcagggcac cccctgagct 720
   acccctgctg gccattgcac caccccataa agctgctggc agtcacttct ctgaggggtt 780
   agcatgtaag aaatgtcctc ctgaatgctg gccagacaaa tggaaatctg ccagggttgg 840
   gtacccccat gacagcagcc agcctgccct cttagtccct gacagctgca gtgacagcat 900
   ctgtgattgc aaagcgtgac aatttatatc tctcatttca tcacaccatc tatcagcaga 960
   cagtcaggct ttaaaaatca atcccacact gactcagtcc ccagcagaga tggcctctga 1020
   caacagtatc cacactgcag gctggacaag ggccctatta attttgagac tcagccaaat 1080
   ttccttctga ccctaagctg gtgaatccct gctcctttgc tttggttggg gttggtgtga 1140
   gctaaggctg tgatcccatt tgctcctatg gcctccaggt ggcctgggcc tccatgaatg 1200
   ggccacatgg tcatactgaa tgcttgatta cactcagacc tagcagtcgt ctgggcgcag 1260
   ctggtttatg gatcactttg tcacaatgtt ccatccttcc aggtccccat ccccgcggtg 1320
   ggaaaacatt gctttaggca gtgctagagg acttcagcag gcattggcag cttctggatt 1380
   caggattaga acaaagaagg aggagtcaca gcaaagatag gaacagaagg cagagagaac 1440
   agacagatgg gggtgtttga gaaggagggc ctttgagacc tcagggagtg ggagacactg 1500
   gctcgagaat aataataatg gcaatttctc tcatctgtgt tttcagggca tggactggaa 1560
   ctcccaatac ccctgacatg ggctgagtca acgtggtcat gaacatgtga caggaggcag 1620
   cagaagttgc agagaagagt gaggcacgtt tgaaaaaggc tgaaaaatgt ttctgtccag 1680
   gcaagggtgt gtgctgaatg actcaaggat tttttggtgc attgaatgaa cagcgggaca 1740
   ttggacacct gctgatccat caccccgggc ccgggcaggc ccgtggatga agagagatgg 1800
   agaagaccag gcatgagact gtggagaagc cacaccacca gaaacccctg ccccatgcgc 1860
   cgtccagccc acacctgtgg atgcacgggg gattgcaggc agggctccca ccgtggactc 1920
   aggaacaggc agggaagctg ctgcctcacc aggcgaaggg gccaggaggg ggaggcggag 1980
   aggcccgtct agcccctgcg gctgtcaccg tggtgcctcc tcactggcca gtgcggtcgc 2040
   gcctcagctt cgttaatagg ggagggggcc taagagtttt cacgtccagg ctcgggcagt 2100
   ggggaggcag gcaggagtgg ccgctggttt ttcagacctc ccagggaggc cgaggaaatg 2160
   gcccgtcctg gagtgggcgt ggttctgtct tcagatggat gctggagggt tgggctgcgt 2220
   gggaccctgg gccctgctgc ttcccggagg atgcgctgtc cggggctgca caggttggct 2280
   gtgttttttg gatgcttgat attttgtttt ttcttctctt cactctgtca tgaaactggc 2340
   aatagtagtt tgtaaataaa tatgtgttat agatgaatat ttgctatgag taaattaata 2400
   aaggagtgaa taaatgagcg attgatgtag ggcctgtcct gtctcaggga gccccacgaa 2460
   ggcctgcgcg ccggccagag cctgcctgcc tgccagggta ctgggacgtc actctcaaag 2520
   cggcgggacc cagccgctga tcttgctgag gaggcccggt ctcagaaaac tgagcggctg 2580
   cttctgcaga ccctgcatcc tcccctccct ggagaaagaa gctctggctg agtcctggga 2640
   ccgaaccctt gggtgccaca gaaacgggct ttgctgcctg tcagtcaagc ggcgggagaa 2700
   acagacctgg ggaggaggag gctgggaggg ctgtgttttc tgcacagcga gtagctcctt 2760
   agcctggtgc catttctctc caaacaccct gaaggttgag tccagggtga agatgtagag 2820
   gcaagttttg gggggatgga gtgggcttgg agggatgctg gcgccttagc aggctgtgct 2880
   cctgaggtgc ccagtgtctg cgggcacagg aacatgttgc cgagggcatt tgggtgtggg 2940
   tggggtgggg aaagggagac agggctgtct cttttaatgg gtatctgcga gcatgtgatt 3000
   gtaagagagg aagaagtagg ggaggaagaa ggcctccttg ggaggtgcgt catcctgagg 3060
   aaggctgaac aatgagggtc ttggagagtc aattcagaag cacaaccttg cagagcaggc 3120
   aaaaacaata gggcttcttg aggctgcccg ggcactcatg caatcaccat ttcctgctgt 3180
   gaatgagcct acattttgtt ggggaagaga cgcaacgacg ccaaacgatg gactctgagt 3240
   caacgataag atgaaacaaa attaaaacaa agtaggaaat caagagtggc tgctgtgatg 3300
   gcgttgcgga gatgatgttt gctttgagaa ctggacaagt gagcccctga gctgcatctg 3360
   cacccagagg ctgagccggt gcacaggact tgcagaggga tgggcctggg cttgtagagc 3420
   agcacaacgg ccccaggcct ggaggagcaa gggtgggaag gggggcaggc cagctcctgc 3480
   caggctggag aaggactcgg acctcaggcc acctgtgcct gggtgattgt gaacttgtaa 3540
   caaatgtgat cttatttatg ttttgaaaaa ggcaacacaa accaacacga gccgtgtgag 3600
   gatcaggtga cagctgccca aaagctgaca caaggaacaa gcctggagga gtgaggatgg 3660
   gtgctgtgaa ggaggttgtg cagctgggcc cgcagtcgga cctggtgaga tcagaggagg 3720
   gggtgccacc agtctgtgga cgaagatgag aagctggaat agagcagaaa acaggaggct 3780
   gccactctcc atctttccca aagtcactcc aggagcaagg gtgtcattta ctgaaatgac 3840
   agactctcca tttcacattt ttcccccaag tgcagagtgc agggaagcag atgggctaaa 3900
   tttttagagt cagggttatt aatgtatact ttacatagta aactttcccc ttttaagtgt 3960
   gcaggcctga ggtttgccaa atatgtgtag gcatttaatc accaccacga tcaagatgta 4020
   gaatattccc actatcaaaa agtttgctgt gtcccttgat ggtcatgccc cattccacag 4080
   ccccagcccc agcccctgga gattgctgtc tgctttatgt tccagtggtt ttatcttttc 4140
   cagactgtat ggatgtgaat ggaatcagat gtgattccaa ggtgttttat cttttccaga 4200
   tgtgaatgga atcagatgta cgaaatccta tggtaggggg tcttctgagt ctagctcctt 4260
   ttgtttagcg tgatgcattt gaaattaatc catgtctcag gcatcaggag ttcatttctt 4320
   tttctgctga gtagtatttc attgtatgga tgtactgcaa tttgcctatc cattcacctg 4380
   ttgatgtaca tttgagattt ttggcaatta tgaataaagc tgctataaac agaca 4435
<210> 30
   <211> 4996
   <212> DNA
   <213> Homo sapiens
<400> 30
   aagatgggac aatttttttt cctcttggtt tctttataat tattgtaccc cttttctgga 60
   ataatctttt catcttgttc atctgtcaat gcctgcttgt cttctcagat ttcaactcag 120
   gtatattatc ttctcctgag ttattttttt aactgctgta aaggcgtatt tctgtgcccc 180
   atatctacgc ttcctaggat aaagcacgtt cctctaatag tgtacaacat ttggcaattg 240
   cctgtttgtc tcttctctct gctagacgtt gtgctacttg agaatatagc tttccctttt 300
   attaatttat ttcgtttaca tccttctgtc acaggattct accagcctgg atcctatgcc 360
   tgccaagggc aagtggagct ggaagggatg tgtgagcaag caagcatcag gtccggccac 420
   tgcacatagc tgctggctgc agtggggtgg gcagctccag tctccagcac aagagccagc 480
   tccttgcgag gctacagctg gaccagacat cccacaagca gcttccacag atggcactag 540
   ggaatgcagt ggcacccaga agcttgaagg caccaggaac cacagagccc caaagagggt 600
   gtcacagtcc tggcttgggg aactcctaga tctgggctcc ctgaaggact gcagctcttc 660
   tctccttctt gtcacccaca acatggtgag caaggggtgt gtttcagccc tgtttgtgtt 720
   acaaagggca tgttcagccc tgccatttgg tgggtcctga gttcttgtcc agtgcccagg 780
   aaaaatgagg aatgctgaca agtggagggt tagcaaggcg aagaggagct ttattgagca 840
   atagaacagc tcagaggaga cttgaagtgg gtagcatttg tccacagcca gggtgtcccg 900
   atgagtgttc agctctcagc agagaggaga ccctggagtg ggcagctcct cttcacagct 960
   ggtagtgtcg atgtttgctc agctctcaac agagagggga acctggtgtg ggtagctcct 1020
   ctccacagct ggttgtctta ttgtctcctc agctctcagc agagaggaga ccctgggttg 1080
   ggtaggttct ctctgcaact ggccatcctg ctgtcttcct aagtctgtct gagtccagga 1140
   cctttatggg cctcagaggg gagaaagcgt gcaccgattg attcatgggc agctatgggc 1200
   aggcatggaa aaagcaccac aagttcccgc tacagtctgc aggactagca gcctggcccc 1260
   caggcttcag gccctccctg gcttgaagat ggagcttcag tggggatctg cccgcttctg 1320
   cccaggagcc tgtctgcctc ctgctgccat tcatggcact caggctgttc atgccaaggg 1380
   gttcctgaag gtcagcaatg acccgccctc agccctgccc ttggccttcc tcttgtgcta 1440
   gttgtgccca aagtccggag gggatgaagg tggcacgggg ttggtgtgtc aatacttccc 1500
   tgattgtgcg cacacctggc caggctggga tagtgaccag actcggcccc aaccttgctc 1560
   tgagatcaaa gcagatgcta ggagtggaga gaagccaaac agtgggagca ggcacttcca 1620
   agcctgtggg ggtatggggg tccttcccag gcccctgaga gtgcagagat gcctgggtcc 1680
   acagccgtga catgaggagt tgcagctgtg cctgggaagg tggggctcct cctgcctgtt 1740
   cctggctctc accagctccc tggagcgtgc agtgctgcct gtgcctccac actgcagctg 1800
   gcatcgtggc agctgccgct ctatacaggc tgccactgcc atcacttcca gaatattacg 1860
   ttgctgctac ctagtagatg ttcagtaagc ttcttaattt taataagcat ttaactagga 1920
   acatgaagtt ctttatactt tgagacatta tatacctgct tgttattgcc atgagtgctt 1980
   tctgtctctt gactgaaaca tggctcccaa gcctgaaaca tcagggataa agtctggtga 2040
   ggaaaacaaa agcagagaag agaaccaaga gccagcatgg ttcaccaagg tcacagggct 2100
   cttggttact gactgaatcc agactgtagt ccaatgctac tagcaacaaa tctgttcttt 2160
   acactgagtc tcggctactt tgcttctctt taaatgcaag taatcaaact ttttcttcct 2220
   actactaaac attttagcac aggtattatt tatatactta ttgaatgcac agcttttcag 2280
   taacaatcct agatagctag tgtcactatt taacacctta aatctctaat tatgatgagt 2340
   ccattttgac ctttcctaga gttcctcaga ggcatcccta ttattgtctt atcattaaga 2400
   tttattagta tcatttcatt tacgaactgc cattttcatt ttattttggg gcatatgttc 2460
   aaactggcat cttccccagc tgcaatcaat gtacataatg ccacagctcc ttgattggtt 2520
   taagaacatt ttcttctcta cggataggaa cacagtttaa ggtttctgat gatggcacat 2580
   ttcaagtgat gctgcttaat tcacctgcag ttctcagtgt cttggttgtc tcatattcat 2640
   ctttaaaaat aaacttcctg atgttgcaaa ctaagaagtg gtcctaaaga aaagaagatt 2700
   aaaaccacag atggtgcaat atagatacca gacatcctat tacactaact ctgagtatta 2760
   caattaatgt aatgaagaaa aacttaaatg tgttggcctt aagaagttag ataagaccca 2820
   gtaggtgaca aagttgctta cggtgatggt tgatttccta ttccaggttg ttggtttttc 2880
   ttttttactg ttctgaacta gaaaaaaaat gtcttggaag ttcatataaa taactttcat 2940
   gatttctaag aacaatcatt ggttacagat gaatgttttc aggataattg tcatatagat 3000
   ttggttatga gaaattaata aaaattgttc caaataaatt taatgtaata tacatcattt 3060
   atatgaagtc tttatcaact tctaaaatat tgcttttaag tgtttttatt tatattaaaa 3120
   tatatagtct atcttcactg gaaatatttt cctaattttg ttttcatata attgctttta 3180
   ttattgtaat ttctgttgaa catttattta gtactcattg atcaagtcca tacaacaaag 3240
   aaaagtttct ctaaatctcc aatgaatttg gctgtgctct gattattcct gcttccctag 3300
   ctttcaagac aaacaaaact gtctaaaagt tttaattgta gttgataaat tctgaaaatg 3360
   gagtaacttt taaaggctct ttcacacaaa aatcctaaag aaaactcata aataattact 3420
   ctcagtaaac cccctcaaat ttttagagct tttttcactt tattcccttg atataaagtg 3480
   ttagttttta ggtggattaa aacaacctat tagaaaatct tgccaggtgt gttggcttac 3540
   ccctgtaatc ccagcacttt gggaggctga ggcgagcaga tcacctgaag taaggagttc 3600
   gagaccagct tggccaacat agtgaaaccc tgtctctact aaaaatacaa aaactagcca 3660
   gacatggtag taggtgcctg taatgccagc tacttaggag gttgaggcaa gagaattgct 3720
   tgaacctgag aggcgggggt tgcagtgagc caagatggtg gcactacact ccagcctgga 3780
   tgacagagca agactctcaa aaaaaaaaaa aaaaaaaaga aaagaaaaga aaatataata 3840
   gactgactct acatttattt gtactgttct atttaaaaat attttaaata atttttaaca 3900
   gactttgcac cacagaatgt ccaggatata tcaggaagga gcaatgaggt aagtgttcct 3960
   caaagtgaca gttataagtg actaagacat agggagatga atcacttggt catcatattg 4020
   atctgaattc tgggaggaca tagatcataa atttgaagga gaaaggcaat gcatttctgc 4080
   aaatggatac tgcttaaagt ccctaacatg ctccttgaaa taaatggggc acaaaagagg 4140
   ttgttttacc ctaaagcaat tttacctcca atcttggcag atttccaaat ttttgagact 4200
   tgaccatagt cttatgttag agtggcttta aggggacagg caatgggata ttcactggat 4260
   gtaaaatcag atgattttgg tttgaaagca agccttttca cttatcaact tcttgacttt 4320
   cagcaaatca tataacatct caaattatta attgtcatat tatctgtgaa tgacatatat 4380
   atacatatat aagccaagag tgttttgtaa gcagtgaatt tctttactgc catattcaat 4440
   aaagcaagca gttgttaggt gatagtcatt catttattca ttcaaccaca ctgttatgtt 4500
   gggtggagga tactgacatc catgaaaaac aatcccctaa tttcaagaca tgtatagtcc 4560
   agaaagggtt gtagactcaa ctcattgtgg ctcacagatg tattttgtct gctggcatat 4620
   ttatttaaag ttggaattgt aaggcctttt aataccctct ccagttagtc acatgctcaa 4680
   cctctccttt tcttatatta acctgcttta gtcaaacaag gtcacacact ataggtactc 4740
   tctttagtca aattattttt tccagtggat atgccttatt tatgttgcag tttgacacta 4800
   aagctacatt caagaaaatg tttctccatg caagctgttg gaataacatc cataagaatt 4860
   cagccattct tatttgtata tttgtccaaa atgctccttt tcccccattt tacttctaga 4920
   ttatgtatat ctgtaaatgt ttcttagatt tactttgcta cattatagtt ttgccaaaac     4980
   atacccctgc caaaca                                                     4996
<210> 31
   <211> 3938
   <212> DNA
   <213> Homo sapiens
<400> 31
   agctgctggc ccccctgggt ccagaggagc cttgccgccc tcacctgcgc agagcctgga      60
   gccgacgcgt cacccccagc ggaagcgcct cgctgcccgg gcgtctggcg aggtgtgcgc     120
   tccgggtccc agctccagcg cttcgcggca gtcgcccgcc tctttccgca agcagggcgg     180
   ggactgggcg gcatctaccg agatctgcga ttcgaggcgc cgaggagccg aagaccacag     240
   ctcagctcag acggcgccct agggccgcac agagggtcgg gcagtgccgg agagaggttt 300
   gaaagcgccg ccgccaactc gacagcgcgt cccagcgcct tgagctcgct tcctcctgct 360
   ctcaggaaaa actgaggaaa cgcgcctggt tccaggggtc ggcggggacc gtggagacgc 420
   tgccagcatc ctcaacagct tggaaatccc atcaccttgg ttccctgaga cttggaggac 480
   tagtaggaac tgacagctgg ctgacagaaa acttatccag ggagagaaga gacaaaagtg 540
   aagtgtgaaa ttttgaataa tgttttcaga tgctttgcat tcagaataga cctctgagtt 600
   gggacccaag ctgtcaactg atcccaaatt cctaagttcc tgacttgcag gtattgagtt 660
   ctaacctccg aagatggatg aaggtatgga aatttcaagt gaaggaaatt ccttgatcaa 720
   agcagtccat cagagccggc ttcgcctcac aagacttttg ctagaaggcg gtgcctacat 780
   taatgagagc aacgaccgtg ggaaaccccc tttaatgatc gcttgtaaga ccaaacatgt 840
   cgatcaccag agtgtcagta aagccaaaat ggtgaaatac ctgttagaga acaatgccga 900
   tcccaacata caggacaaat ctgggaaaac ggctctgatg catgcttgct tagaaaaagc 960
   tggccctgaa gttgtttcct tgctcctcaa gagtggggct gacctcagct tgcaagacca 1020
   ttctagttac tcagctcttg tttatgctat aaattcagaa gatacagaga ccctgaaagt 1080
   tcttcttagt gcttgcaagg caaaagggaa agaggtcatc atcatcacaa cagcaaagtt 1140
   gccctgtggg aagcatacta ctaaacaata cttaaatatg cctcctgtgg atatagatgg 1200
   gtgtcattcc ccagctacct gcaccactcc ttcagaaata gacatcaaaa ctgcctcatc 1260
   gccactttca cattcttctg aaacggaact gacgcttttt ggctttaaag atcttgagct 1320
   tgctggaagc aatgatgata cctgggaccc aggttcccct gtgaggaaac ctgcattggc 1380
   ccctaagggg cccaagctcc cccacgctcc accctgggtc aagagtcccc cattattaat 1440
   gcaccagaac agagtggctt cattgcaaga ggagctccag gatattacac cagaggaaga 1500
   actatcctat aaaaccaatg ggctggcact ttccaagcgg ttcatcacta ggcaccaaag 1560
   cattgatgta aaagacactg cacatttgct aagagccttt gatcaggcca gctcaaggaa 1620
   gatgtcatat gatgaaataa attgtcaatc ttatctttca gaaggaaatc agcaatgcat 1680
   tgaagtccct gttgaccagg acccagattc taaccagaca atatttgctt ccaccttaag 1740
   aagtatagtt caaaaaagaa acttgggggc aaatcactac agctctgatt cccagctctc 1800
   agctggcctt acccctccaa cttcagaaga cggcaaagca cttataggaa agaaaaagat 1860
   cctctcacca tctccttccc aattgtcaga gtccaaagaa ttgttggaga atatcccccc 1920
   aggtcccctg agcaggagaa atcatgcagt tttagaaagg cgaggttcag gagctttccc 1980
   tttagatcac agtgttaccc aaaccagaca agggtttctc ccacccttaa atgtaaattc 2040
   tcaccctccc atctcagata tcaatgtcaa caacaagatt tgcagccttc tttcttgtgg 2100
   tcaaaaagtg cttatgccaa cagttccgat tttccctaaa gaattcaaaa gtaagaaaat 2160
   gttgttaagg agacaatcat tgcaaactga acaaattaag caattagtaa acttctagga 2220
   gatgggtata aaacacatct tcttcaaatg tttatatcag agaaacaaag aactagagaa 2280
   gaaatcttaa atgttcattc ttttaaatct cgtgattggt tagtccataa tggctagatg 2340
   gattaaaatg taatgtgatt tgtactaggc gtcatgagca taaaaaataa tacatagttt 2400
   ctgttaaggg aattctctca aacaaaagga aagcgatttt tctcaatgct tctaatattt 2460
   aacaatctcc atgtagtcca aatttgtcat taaaaaaaaa tcaaagaaag aaggccacgt 2520
   atgttggggt aatttttaaa aattttcaga ttggctaaat attttattag gaaacagttt 2580
   tcaagtcaat aattttttta acatttcatg ttaataaatg agattgaaat tttttatgat 2640
   ctctaaacca taaaagaaaa caccgacagc tgtaaaatgt tctaagataa aaaaactcga 2700
   gctacaaaga aagaatgaac taattcttta caaatgttga ctgaagatag ataaaactct 2760
   aattaaaaat ttaaaaggtt aaacactgta ttagaatggg caactaaaat attcgtggtg 2820
   tcagtttaat tagtgtcaaa ctggcaagtc agagttttac aacctactag taatctatgc 2880
   cgggtcttgt tgtttgtgca ctgtgttttg ctttgctttg ttttggtttt taaacagact 2940
   tacctgtaaa gttatcttac ggaattattc tttctccata tcttttaagg aattattctt 3000
   tctccatatc atcttttaat tcctgttatt agttttggaa gcttatccat aagtttccaa 3060
   agacaagatg atataaaagt taaaaagcat tcctctttac ttatatcaaa ttattcttgt 3120
   ctttcaaaaa gaaccaaaaa aaaaaaattc atcatggata aaaactaagc gcttgccttc 3180
   ttttaaaaag aaaaactgat gattagatta ggttagctaa aattaataga ggcagtagaa 3240
   acttaatcac aaaatgtact aaatatttgg caatattaaa caatgccttt ttatttctga 3300
   tatgtagtta ttagacattt atatattaag gataacttcc ttagtgtgac attccaatca 3360
   tttagtattt taggacctgt gaataacttc caacaaaatt aatgaatacc atattagtat 3420
   tataaaatat tataaagtaa taattatatc atctatataa cttcaaagta tgatgtttat 3480
   acaaagaatc cttatttcag tctagcagct taacaacaaa ggataaatca gactatacaa 3540
   aacctaagtg tattaatttg atgattgtca cgtacaactt tctttgcctt ccaagtagag 3600
   gtgatcctaa caattggcat gatgcttttc ttggtaattt gatataagca agttgtaatg 3660
   tacatagtaa ttatttaatt actgtggctt ttattttaaa aagtaattag aggtttttcc 3720
   atttcatgtc ttgaacaaca ttaatattaa ttacactaga aatatttttt gattcagaac 3780
   ttagaattgt acctgatatt tataggtatt taaatgcttt ctgcaagtta aatatcgaga 3840
   aaagtacttg acttcaagtt gtaaggatac aatttggaag ggtatgtaaa tagattagtt 3900
   cataaacaaa atggtatcaa taaagatttt aaatatca 3938
<210> 32
   <211> 542
   <212> DNA
   <213> Homo sapiens
<400> 32
   agctcagctc agacggcgcc ctagggccgc acagagggtc gggcagtgcc ggagagaggt      60
   ttgaaagcgc cgccgccaac tcgacagcgc gtcccaggaa aaactgagga aacgcgcctg     120
   gttccagggg tcggcgggga ccgtggagac gctgccagca tcctcaacag cttggaaatc     180
   ccatcacctt ggttccctga gacttggagg actagtagga actgacagct ggctgacaga     240
   aaacttatcc agggagagaa gagacaaaag tgaagtgtga aattttgaat aatgttttca     300
   ggcacggccg tcgccagcat agtaccggct cctcttcttc tcccagaacc tcctgacacc     360
   aaccttggtg aaagaaaagc tgtgacaatg ctttgcattc agaatagacc tctgagttgg     420
   gacccaagct gtcaactgat cccaaattcc taagttcctg acttgcaggt attgagttct     480
   aacctccgaa gatggatgaa ggtatggaaa tttcaagtga aggaaattcc ttgatcaaag     540
   ca                                                                    542
<210> 33
   <211> 12351
   <212> DNA
   <213> Homo sapiens
<400> 33
   aaacaggaaa agaaattggg atttttatga aaaatgttaa aggctagctc tgttaggatt        60
   tcccatgaca ttgcagtggt gacatggtcg tggatgtgcc aaggaaagcc tgagctgttg       120
   actgctgccc cagtagcatc agagaaccac tgtgcttgct gagaccaatg actgccattc       180
   tggacagggc agcctttggc aacattcaga agcaagctgt ttgtcttcag tcccctctct       240
   tcagcctcca aatttgtcat tattgcttgg tggatggagt accctatact accccatggc       300
   tctccaaaac ccttttcctc ccagccagga ggcccaggtc tgcgcattca ctcactcgat       360
   tctgtgcatg ttgcctgtgt gcggtccatt gccctcacct cactttcctg gggaaagaaa       420
   aaatgacact gagctgagga atgagcaaaa atctaggtag agacgtggga gaggctgtgc       480
   tagttggagg gcgtgaacaa aatcgtggaa gtttacaact gtctgacttt tctagggaaa       540
   ggcaagtaat ctgatgtgac tagagaaagg ttaggtgaga tctgtaagct ggtttgggtg       600
   tagaaggaca aagaattgga gtgggaaggg taagtggcag ctagactggg aagggctgag       660
   caccaggtaa aaaagacaaa ataaaattgt ttctcctctc ccaggaaata ctgaacttta       720
   ggaggcaaca tctccatcct ggctctgtaa accaatcttt atcagaatga gaattttggg       780
   gtaagttgga agggacaggg ccaggcttac agcagcggtt ggcaaactat ggtctgtgag       840
   ccaaatccaa ctgctgcctg tttttgtgtg gcctgtgact aagaatgatg cttacatttt       900
   taaatggttg agtaaaagtg aaaagaaaaa taatattttg tgacttgcaa aactgtacaa 960
   aatccaaatt tcactattca cacataagtt ttcttggagc acagccacat acacttgtga 1020
   tgatttcact gctttggtca tacaagggta gaactagaac tgaattgttg tgacagagat 1080
   cctctgtccc gaaagggcct aaagcatttc ctgtctggct ctgcttagaa aaatattgcc 1140
   aagttctgtg ctgggcacgg tggctcacgc ctgtaatccc agcactttgg gaggctgaga 1200
   tgggcacatc acctgaggcc aggagttcga gaccagcctg gccaacatgg tgaaacccca 1260
   tctttactaa aaaaatacaa aaattagctg ggcatgatag cgggcacctg taatcccagc 1320
   tacttgggag gctgaggcag gaggatcact tgaacccggg aggcagaggt tgaagtgagc 1380
   cgagattgtg ccactgcact ccagcctggg caacagagca agactctgtc tcaaaaacaa 1440
   aaaacaaaac aaacaaaaca aaacaaaaca aaaaacaaag gagtcgagca ctagacagaa 1500
   agttcagagc aggatggagc tctacaaaga taacctaagt tttatggagc tgagttccca 1560
   aatcacttta actaaatgta ttaattccag tggccatgat ttccctagca cttattctcc 1620
   tgtagcataa ttggaaattc taacaagcct ttttttgcgg ggagggggga gggtggaggt 1680
   tgtttgtttt tagtgtgaga tgtggattaa gcatattaaa aattttaaaa taatttctgg 1740
   gaaatttgga aaactgtgtt gacagggtgg ttgccatgca ttttaaggaa cattcacatg 1800
   gttgaagagt catagagaaa acattgtctt tcagaatcac tcatccaaat caatataaat 1860
   aaattgctaa gaccttttct aaccttttaa acattcatct ttcttcatga gaacagcaat 1920
   aaatgtcctt tgtttctgga gtctgccaat cacctatttc ttctttagtg tgggtaattt 1980
   aaaaaattgt tttcataagg ctaaaagaca ttatgttata caatgtctct attttttttt 2040
   ttaatggcaa ggcagacttc attcaagggt ggcacattgt ggtaggtata ggaaccattg 2100
   caatgagtgt tgcagcaggg gagggagatt ggagtcaact cactactgtg tctttacaat 2160
   tttatttttt ctttcagaga cagggacttg ctctgttgcc taggctggag tacagtggcg 2220
   taatcatggt tcattgcagc ctcgaaatcc tgggctcaag caattctctg acctcagcct 2280
   cccaaggagc taagaatatc tttaagtttt tttaaaaagc aatccttact tcctgaatgc 2340
   accaaaattg taatcccatt ttgacccagt taagaggaaa actcaaaact gagagggcct 2400
   ggttaaagaa aagtgagtag aggatataat acaatgattg acaggaaacc ctggtgtgtt 2460
   gtgagacaca gcagtggagt tgttaagagt cgcctcccag ttgatgtatg ctatctcaga 2520
   atatccagat ttcccagctg acatcttctg cctcccctgg ggtggtgtgc aaatggagac 2580
   tgtatcagtc agggttcagt tgcagcaaag ggaagcaact ctagttattt taagcagaaa 2640
   tgtttaatac aaagaattag ctgcatggaa gtgctggtgg aagagatggt ttttgggaga 2700
   ctcccaggac acttgagagc ttaccaccta ggggaacaac tacctcttcc atgaaggtgg 2760
   ggagtcagaa agttgctata ggatcaggaa tctgccattt tttcctcaca gatgcatctt 2820
   gactcccatg aagctgggaa ttacaagtac cctgctggaa tgctgctgca gaaatatatc 2880
   tgcactacca tattttctag tgaaattcag ccaaaagcag caggaaaatt atctctgcct 2940
   cacttccact ttccaaacct tgcacaagtg catccaagtg gggataagta ggtgatcaga 3000
   accctagctg caagagattc caggaaatgt agttttttta tttctagact ctctatgtgg 3060
   ggagacacac tggaagggag tgaaagagaa tgttgaggtt aatcacctgt atttgcttcc 3120
   tattgctgct ggaataaatt ggaacaaact cagtggctta aaacaataca catttgttat 3180
   ctttcatttc tggaggtcag aagtctgatg gaggttttac aaggctaaaa tcaaagtgtc 3240
   agccaaggtg tgctcctttt ggaggctcta ggggagaatc catccatccc ctgcctttcc 3300
   cagcattaga agactttccc acattccttg ctcatggcca cattgctctg acttccgctt 3360
   ctgtcatcac atcaccttct atgactctaa ccttcacatc tccgtcttat aaggactctt 3420
   gtgattacac tgggttcatc tgataataca ggataatctc acctctcatg attcttgact 3480
   taatcacatc tgcgattccc atcttccatg caagaaacac attcacaggg attgatatgt 3540
   tgacatcttt tggggggtca ctattcagcc tgccacattg ttcatatcca aattattgag 3600
   ggaaggagct gatgtttatt aactacatgc tatgtgctgg aaactttata tatatatata 3660
   ttatctcatc aaacattcat gacaatcctt gaaggtggca ctattactcc tactacaaat 3720
   gagaaaaaga gattcaaaga attttaaata cgagtccact attcagcgat aaaaaaaatg 3780
   aactattaag ccatgaaaag acatacagga accttaaata catattgcta agtgaaagaa 3840
   gccagtctga ataggatacg tactgtatga ttctaactat gtgacattct gggaaaggta 3900
   aaattataga gtttataaaa atatcagtgg tttccagggg ttaaggtttg gggcagggcg 3960
   ttgagggtaa gtaggcagag ctacattacc ctacttatga gcaatgagtt ctccccccaa 4020
   aaaatcattt ttctatcaac caggctggag tgcagtggca tgatcttggc tcactgcagc 4080
   ctctgcctcc tgggctcaag tgatcctcct gcctctgcct cccaaatagc tgagaccaga 4140
   ggcatgtgcc accacatcca gctaattttt tatttttggt agacttggag tcttgttatg 4200
   ttgtctaggc tggtctccaa ctcctgggct caagtaatcc accagccttg gcctcccaaa 4260
   gtgctgggat tacaggtgta agccactgta tcaggcccaa atttttgata tcataaacaa 4320
   cctgtggcaa atattcttgc atatgcatct tttcacactt ctttgactat tttttgctaa 4380
   tacattccta agggtaggtt ttctgagtca gatggcaggc acattgtaaa caccttttat 4440
   atatatatat ttagaaagtg tcagtggtgt gctggaactc tgaaacaaat tagagctttc 4500
   cccctcttct atggagagct gactattaaa catttagcat actactggaa agtgtgtaca 4560
   aatctccagt cctaacagta tatgaggacc cattattttc acctcaaaat ccatactgat 4620
   gcttaaaaca gtaatatgtg ctagttgtaa tttgtatatt ctaatactga tgattcatag 4680
   tacctgttgc tatctctagt ttttcttttc tatagctcat ttagaggtta aaagaaattt 4740
   caggtatatt tgggcatatt tcatactcag ccaggtcagt atggtccatt aagggcaagc 4800
   aaattttgca gggatttagc ataattatgc aataattcca aggagtctct gcactttgga 4860
   aaattgttat ttataaaaac cttttaacta attgttttgg catacattgt tgatctgcag 4920
   ggaacttggt gacacacaga gagggaaggg gagagagtga ttagaatctc caggagtcct 4980
   ttatctaatt ttccatcatc ataccatata ctccatttaa gcccacgggt ggggatcttc 5040
   ctgaccttgt tggtactgct accctggtag ctagtaaacc actcttttaa tgtggttgtg 5100
   ttgcttctgt tgcagtggaa taaagatttg gaccactgag tcatctagtt ctaagaagct 5160
   gtgattctat cagaaatgat caatgggggg agtcagctgg cataatgaaa atagcatgaa 5220
   tttgggggtt aaggtctcca gaattctaag tcctgctttg ccacagggct atgcattttc 5280
   tgtcattttc tactccacag cttgattggc ttatctagcc aactagattc ctgcttcatt 5340
   ttccctaatc actcctggta gatcttttcg ccaaggattt acaaaggagc tactcaagaa 5400
   attcccatgt gaaagagtga tagtcttcta cttgataaaa gcataggaat agcccctttg 5460
   cctctagata actgaatgct cttagacaag cctcagtttt cacatgaata gaatgagtac 5520
   atgtgtgtta gtctattctg gcattgtaat aaagaactac tgagactggg taattgaaaa 5580
   gaggtttaat tggctcacgg ttccacaggt ggtataggaa gcatagctgg agagggctcg 5640
   ggtaacttac aatcgtggtg gaaggtgaag ggaaagttgg cacatccttc atggctggac 5700
   caggaggatg tgtgtgtggg gtgctacaca cttttagata accagatctc atgagaactc 5760
   actcactgtc atgagaacag caaggggaag tgtgccccca agaatcaatc aggccccaac 5820
   cctgacgatt acaattcgac atgagatttg ggtggggacg caaatccaaa ccatatcagt 5880
   gtgtgtgtgt gcgcgcatgc gcgcgcacgt ttagaggccg gggggaactg cactcaatgt 5940
   gtgagtcctg aagttcaatg attctatgaa accaattcac ctgtatagta aatgtggtgg 6000
   aatcacacta gtgccttcaa tagaaactaa actcccttaa aagaagcgtt tgagttagct 6060
   tccagagact gctatgtgag aacatgatgt taagttgtca tatgtatgct ataatttgat 6120
   tctcactgga agaatgcagc agtgatgtaa gaggctgttt gtgaaattag gccatcaggg 6180
   aatgcttgtg ccccatgact gtaaggacta taattctctt acacacagac atcaaatgtt 6240
   attgcaaagc cattggaaaa attctaatta ttttaaaggc atatataaaa tatgtacctt 6300
   ttattaaata atatgaatat aaagtaagat caagttctga ctctgagctg tccatgctga 6360
   tgacctacag agcattgcca gctttagtct ctgcattgcc ttcttttctt gtaagtatca 6420
   atggcagttg cccactcaat gtaagttgaa gggtgcagct gaaatcacca ccgcatcccc 6480
   aaaatatctt ttccaccaat gcttttgaaa ccaaaggttt gccacttgag atgaagaaat 6540
   cctttttcta ggtttttacc tctagcaccc ccttgttaag aatattgcaa tgtcaattgg 6600
   tcattactaa aggccagaga tagccaatgc aatcaccaag tgtatcagcg tatcaatttt 6660
   tttttttttt gagatggagt ctcgctctgt cgctcaggct ggagtgcagt ggcgccatct 6720
   cggctcactg aaagctcctc ctcccaggtt cacaccattc tcctgcctca gcctctcaag 6780
   tagctgggat tacaggcacc caccaccacg ccagttaatt tttttttgta tttttagtag 6840
   agacagggtt tcaccgtgtt agccaggatg gtctcgatct tgtgatctgt ctgcctcggc 6900
   ctcccaaagt gctgggatta caggcgtgag ccactgcatc tggccttttt tttttttttt 6960
   tgagactgag tctcactctg ttgcccaagc tagagtgcag tggcaggtat gagcttggct 7020
   cactgcaagc aacctctgcc tcccagattc aagtgatttt cgtgcctcag cctccctagt 7080
   agctgagatt acaggcacca gccacaacac ctaatttttg tagttttagt acagacgggt 7140
   tttcaccatg ttgaccaggc tggtcttgaa ctcccgacct caagtgatcc acgcaccttg 7200
   gacttccaaa gtgctgggat tacaggtgtg acccactatg gccaactagt tttctgggtc 7260
   ttctgtaaaa aaaaattacc atgaacttgg aggcttcaaa caatttaaaa aatgctgtta 7320
   tagttttgga ggccagaagt ccaaaactaa ggtgtcaaca gggttggttc cttgtggatg 7380
   ctctgagggc aatccattcc ttgcctatct cctagcttcc aatggctgct gccaatcctc 7440
   agtgttgctt ggcttgtaga cgtattgatt cagtctctgt ctccatcttc acatctcatt 7500
   ttcctctcta tccccgtgtc tccagtttct ttctgccttg ctcttatgac tcctggcatt 7560
   agattaaagg tccaccccaa atctaggata aactcatctc gagattttta catctgcaaa 7620
   gatgtaaaag aattctttgc gaagaagcaa agaattatat ctgcaaagat ctttttcaaa 7680
   ataagtttac attcacaggt tccaaaggtg aggacatgga catatctttt tttttttttt 7740
   ttgtcagggt ccaggggcac tgttcaatcc actgtagcat gggaagccct gataaaaaga 7800
   gacacaggac aagtaagtac tgaacggaag ggatggtaaa aagagagatt tgaaaggtag 7860
   gatgagaaca ctaggctgga attgggggat gggttggaga ttgaaggata aattctttct 7920
   ttcttttttt tttttttttt gtttgttttg ttttagtttt ttgaaacagg gtctcactct 7980
   ctcacccagg caggagtgca atggcgtgat ctcagctcac tgcaacctct gccttgtaga 8040
   ttcaagcaat tctcgtgcct cagcttccca agtagctggg attacaggca tgtgccacca 8100
   tgcccagctc atttttttgt atttttagaa gagatggggt ttcaccatct tgaggtcagg 8160
   tttcaaactc ctgacctcaa agtgatctgc ctgctttggc ctcccaaagt gctgggacta 8220
   taggcatgag ccaccacacc tggcctggag aaattatttc cattttactg atgagcattc 8280
   taaaagggaa attgataatc ttgaagaact ttgcctgggg ggttctcctt gcaagggatt 8340
   ctttggcctg agaaatcact ggcccttatg ctgttggtgc cataagagaa tctgggaatg 8400
   tgactaggac cgaaccattt tgtgcgtgtt tttttggctg tttattcact tgtctattca 8460
   tccatccatt cagtaaacat ttattgagca ctttctttgt tcctggaaca tagaaggcta 8520
   cctccttctc aggtgccttg ttgggatgtt ctgtccctgt ttgtatccag cgttgggctg 8580
   gcagtttagt ggagttgcac atggtcaagt cagccactaa gattacaatc aagtagagtc 8640
   aaaattgcct tagaaaactt ctgaaaacat ctataaagta cacctaaatg ggtttctcca 8700
   atctgaatac ggggttcaga cacaataaac aatcgcagga tggagataat tgaggaacac 8760
   gatgaagcac atcctaggat gataattggt tttgtcttct aacattattt taattcttct 8820
   tttcttccct ttgccgcact tacagaatta aagttgtgaa aattatatac gtacttcacg 8880
   tgattctagt ctactgccac acaaggctct ggtggagatg tagcaactgg aatctttcaa 8940
   aaccgtgtga gccagaggca gccataggga tattatggtt gcttgtttat tcagaagtca 9000
   ctgttgtgtg gttgaaagag acatgggcta ctcctctctg cttgaaacag aaaattccgt 9060
   gagagactca cgcaacctac aagaggctgg gttgcctcat ggttcatctc aatgatgtcc 9120
   tcataaagga aactccagtc aggtagccct caaagcattg aaaacaatgt aatttttcct 9180
   gaaatctcat gaagctgcac cgaagaggaa acccatacat agcatatttt cttaaaattc 9240
   cgtctcacat ttctttttta atatttcaca catcacagat ggcagttacc taaaattccc 9300
   actttttatc ccttaacatt ttgcaaatcc tccccaaatt ttttgcactc tggctcttct 9360
   ttctccctgt cctctggttt tcctgatctc aattttatcc ccttttacat gtaaatcagt 9420
   ctagatggct gtagtatttt ttcattttct tgaccactgt ttccttctta tgagatccat 9480
   aaagcgcaaa gcaaatctac accgaaaact ctggaattaa ccaggatttg aattgagcag 9540
   tttgattttg ctacttagta acaatgtgat tttgggtaaa tttcttaaac tctctgattc 9600
   tctctctttt tttctaattt gtaaaataaa gccaataata ccaaattggt agagatggtt 9660
   tgaggattaa gtgaaacaat atattttcag attcctatca caatgctggc catattaggc 9720
   ggtttaaaaa aatgctggtc gttttctttt tcttttcttt gtagaaagat ctccttgtag 9780
   agacaaatca caatcaaaga ctttttggtt aatgtggaac gagatcatgt gaattagagc 9840
   acatggcgct ctaaaggctc catgctaaga ataactgaca gatccatgct acggcaaaaa 9900
   ttgttttgca atgggaacta cggctagggg tctgcctccc atcaaggatt tatttatgga 9960
   ggaattccca aaatacagga agaatgttca aatagtagca gccaggaaat ttccaacatc 10020
   tgctacactg ggtcactctg aattggtctt tttggcttct ttctgtaatt tccattttgg 10080
   gaaatcagga gttgtgactc acagtggtaa atcttcacac agttgaaata tgccttatta 10140
   gcacaatcta ttttagtatt attaatgcca tagtataaat atgtacagca ttagaagtag 10200
   tcaattagtg gaggctgatt tttattttac ttctatgttg gctttacttt caaaatatat 10260
   gttatgttat tattgttttc cttttcttca ttttcattgc tacctttctt ggtccaagcc 10320
   atcactatct cttaggtagg tgatgagata gagcactaag aggtctggga ctcttccgcc 10380
   cctacacagc agccttgccc tccacatggc agctggagtg atctttataa aaccacacat 10440
   gagaacatgt tcctcctctg atggcttcac accacatctg gaataaaacc ctaacatcct 10500
   accctgaccg agtgggccct atgagttccc cttgcctctc tgagcacatc tactcccccg 10560
   ctcatgctgg gtcggccttc tatccgtacc tttgaccatg tattccctct gctttgatct 10620
   cttcccatct attcttacag ggctggcttc ttcttgccat tcaggtctca atatctcctc 10680
   agacagactc ctagcccctt aatctaacgt agacctgctt ctactccaat cattatcgtt 10740
   ctgctttatc tttgtagtgt gtgctactct ctgacatttt gaattatttg ttttcttgct 10800
   tattgtctgt gtaggggaga aaagatttct ttcctcactc attgtaaagt tcatggccaa 10860
   caaccctatg acaaaagagt aataagcaaa aagcttgcaa atgcatttca tgtaaatctt 10920
   acatgacatg tgagccttca gaagcaaagc cccaaagacc cagggaaaac tgtattttta 10980
   tggacagcca tacagaagta taattgaagg acaaaagggt ctgatccaaa ggtaacaaac 11040
   tgggagaaat gtagtaagac ctgtttgttc agtttcttct gtgtgggcac ctgccacatg 11100
   agtgtcttca ggggagaagg aacagaagag gtcggagagt gacgatcctg ggttttatgg 11160
   tctgcttcag gggaggaaag cctgagagaa ggtcagagac gccttcctac ttctgctgtt 11220
   ttttcagttt ccaaggtgtc agtcatattt tggggtatca cacctgaagc ctctgcgaga 11280
   ccacaggtct gtgtgcctgt ccagtgtctg ggacataaca ggagcttaaa aagcattctc 11340
   tccacacggg gatttaccgt agacatgttt gtgaaaacca aacaaatcct taattcctct 11400
   ccccctgcaa ggtgtccttc ctccatttag agctgccctt ttccagagtt cccagggtct 11460
   ctgggtcatt accaggttcc cttatcacaa agagaaagag tggaagtttc ctatgttttc 11520
   tctcctcaac ctccagtcag ttttctccat tccaggctgc ctttgctgtg tgcaaagctc 11580
   aattgtctgc ctcaatttta ataattcagt gttgaatggg cacatttgca tatgcaaata 11640
   tttgaagaat tgaaatggca attaacagat gtatatttgg gattaattta aaatattttt 11700
   tgtcttttcc tgtttacttt catggtccag cagtaattct gatcttagtg acttgctgtg 11760
   attcctccat ccaatgtgtt tccaggtcta ctctagccag agtcagattt ctaaaagatg 11820
   ctgtgctgtc tttgaatacg attgaaaaat caccatttta acccctttat agaacctaat 11880
   aaagcatttc agaagtacct gcatttcttt ttatttctac ctggttgatg agcctctctc 11940
   tttcaaggct tcagctagag gagaattgat caacacgtgt tattataatt atttgaggac 12000
   ctaccatgga aagggcactg tgctggaagc agctggggga atatagagag gagtaagacc 12060
   aaagccctgt cctctggagc cttatggttc cacaggaagg agaagatgtg aagactcgtt 12120
   acctgaagtt gacatttggt ggatgacaca gcattcaaca caaaacattt gccatccccc 12180
   aaaaagctgc caactgttac tggatggctt tggtgatttc catgacattt ttccaaaata 12240
   caatgacttt cctcttgtgg tttttgtaca tcaacaaatc ttttcaacta acaactactg 12300
   ggaaaagtga atctatagaa gtttattatt aaataaacta acatgaaaat g 12351
<210> 34
   <211> 31532
   <212> DNA
   <213> Homo sapiens
<400> 34
   tccctccctt ccttccttcc ttccttcctt tcttcccttc agtttctctt ccttctaatg 60
   ccccctgtcc ttaaaaatgt ctccattcag gcactatgca atttgttaac tccacttctc 120
   aaagataaat ttctggtcag agtgttctat tcttgctgtt ttcattttcc cacctcctat 180
   ttactcttga atccacagga atctaggttc tactcccaac aatttgcaaa agcttctttt 240
   tctaaaatca caaatggtac tgctcctaat taggaaatcc agtagacagt tttgagttct 300
   tatcttattg ttctcttaca cattgtttga acttctgaat tatttcctcc attgtagacc 360
   tctctgtttc ttcagagttc aaagcttcac attctcctta cagctcacta cccactctcc 420
   ttctgtttcc tacatggcct ctgcctgtgt caaaaatgtg aaagaataga gctgtgctac 480
   ttccatagaa acatgaggga gtatattcat aaattctgca agacatgtac gcatacaaaa 540
   ttaaacaaca acaagaggaa tgtgctcaag agaacacatg acaggaactg tggttcagaa 600
   gaagagcaga tggagagtgt gggtaagagt cgttctgtaa gcatcatgtt gatataggat 660
   gagtgtggca ttggggctgc aaatgccact gcagtgggat cccagcactg acagccatag 720
   ggccttgggc aagttacttc ggcacaatga acactctatg ggaatagtaa tagttatgtc 780
   ctgggactgt tgagaaaatt aaacaaaatc cttaagccat tatttgcagt agtacctagt 840
   atgtgcttca tgaatatatg ctcattttca taaagaaagg gagtcttgag aacacttttt 900
   tctcctctct cctgcatcca agcctgaaga ggcagaagga actccaggag ttgaatggaa 960
   gagcacagaa gggaaacagt gagcatcgtt actactgcac cctcatccca gctatgatct 1020
   gctaatcctc agatagcctg agacggggta cggaaagtgt tagatgacca gcaggttaga 1080
   caagaccttt gaccaatgtc agaaaggtgc ctggaaaagg tgtgggacct ggctgagatt 1140
   tcatctgggt acagagaata atttaaaaag cttgctggaa ggcagtggta tgatgaaacc 1200
   atgcaattct tgtttaattt tacctatatc aggtctgtct tgttcaataa atgagttata 1260
   tgtacttaca aatcattaac aggtagtact atcaaaccat acttaaaata tttctgagtt 1320
   ttcaattatt tccttttcta gataaagaaa tagcgacaga ggcaccccag tggatgtccc 1380
   tgcacacctc cactttgggg actattctaa gagcaggagc ccaatggggg ctgcaaatgc 1440
   cactggaggg caggtgatcc atgaccagtg aagacagaaa aagaggagaa tgtggtagtg 1500
   atggaggttg atattgctgt ggtagaacat gtggaaggaa aatgaagata ggcaaaaatt 1560
   gaggctcttc agattcattc catgtagggg aagagggggc aaaagtaaga gcattgttca 1620
   agaacaacca ggtagaaaca aaaggaacag agaatgtgtt gacctccaag ggaagaaagg 1680
   aaagcaaaga catttgtgtt acagtccaaa gaggacagtc cagagtaaca aggatggagt 1740
   atttgagtat ccccaagagg cttacaaaaa ggaactttac ttatagataa gcaatgatag 1800
   ggtaaaattg aaaagaacta aagagaaaga aactaagaca gttgacaccc tggagaaaaa 1860
   gtttatgagc ccctactaca gtcatgcatt ctcaaaataa ctgctaaaca aattagccat 1920
   ctcagtaaag caagactggg taagaattta agaatctctc catcctgggt tggcaagtga 1980
   tgggcagaat aattgaccag acatcctcca gctcagttcc tgggatgcag agatgctggt 2040
   ggaagcactt tgtagcacag caaggtgaga gtttgaaggg gcttcacttt ctgaaaaatc 2100
   tctcttcata atcctgtttt tccatttcag aaagaattta aatcaaaaac cttaatgttc 2160
   ttgtcatctc tgaacacatg ctgaatgcag gggaaacacc ctgaagtagg aatttctgat 2220
   ctccctcttc ctttgttgtc attcaagaat accaactcag cttttacaat ttgatgtcct 2280
   tctaacttga ggaaaccatg ccattacgaa cactcccctt gccttagaag aagatatgtg 2340
   agttgggtct tagggagact aaggaaagaa aactctcctt actggggaca aacagtatcc 2400
   tagaacactg gattctctat cagtagcttc tgaccccagg ggtgtcacac agagctttgg 2460
   acttaggaaa agtcacatag catctttgag catggagtcc caattggtaa ttatgatgta 2520
   tgttgctgcc tgatggttca ggatatttcc ttgaaagact tctttccata gcgctctgat 2580
   tatgctgttt gggattaaca gaataggcat agcggtttag ataggaaatt ttaaaaatga 2640
   caaattaaag aatcctggaa aatacactgt acattctgcc aaggtttatc aatatggatg 2700
   ctggtatagt aatgagaata aatcggtaaa ttcttttcat aaaatgaagg aaagaaatta 2760
   ctatgtgctc tttaaaatgt gttgcatcag tgcctgattt gattgtaagg gtcacatctt 2820
   caagacatgt gataccaccc tttataccac attatagcat tgtctaatgt tgtaaagttt 2880
   ataaattgct ttcactcagt cttcatctga ttctcagaat gaccctatcc aattgggaat 2940
   atgggtttca ttagtcttac attactaagg agattcagag agtctcaatt ggccaacatc 3000
   atgcagttaa aaatgacaaa tggggatttt gaacccaagt cttcctgacc ccagaacccg 3060
   tgatctttgt actacatcat gcttcctgag aaattaaatg gacctgtctt gcagaactgt 3120
   tcttcttcta actagggatc tggaggagtc tcaagtaaat aggcagatgg ccttagggag 3180
   ggatgtctgg aagcacctgt ttggagtccc agcaggttgg tggttttctg tctgtaggtc 3240
   tggcagctgg gcatggtaac ttagccatgg aaaggggctg caagaggagg tcctcagtgc 3300
   tcagacccag ggcagaggac tgtgtggctc ctgtttgttt ctccagatct actctctaca 3360
   cgccttcacc ctgctctctg tttgggatgg aatcaaagga cccctttgcc ccctggctta 3420
   tggttgcctt ttgccaatgg aagcagtgac agaggatcag attagaggta gaaggggagt 3480
   gaggaaggag agtgaagagg gataaattct ccttctgcct ctgatcctct gtcgctgctt 3540
   ccactgccac ctactgcttc cattggcaaa aggaaggatg ccgtgaggtt gccatgagca 3600
   tgctgcagcc ttccagtctc ctataagaca gcttccccat ggggtccttt tcctattcct 3660
   cctcttgccc cttctgacct agccccaggt acagtactgt attacttacc ttctcttata 3720
   ccttttccat acctttgtcg atggtctcct tgttggactc ctcacattac ccagtttgag 3780
   tgtgctttct atttcctgat gggctcctgg ctgatcagac ctgaccccag aggacctggg 3840
   taccagaatc taatgcctgg attaaggcac atgagcaaga cgcagaccct gcaaatggaa 3900
   ttgaaattac aatgacctag aacaagggag atagggccaa gcatgcccta gtgaacgagg 3960
   gacccaaatg gggtaggact gggaagaaat ctgacttgag actgatgggc taggactctg 4020
   ctagaagggc ccttctggtc ttccaggaga caagactatt cacagacgct gcaaattggt 4080
   ggtgcaagga aggcagcagg agctcaatag ggtacagagt ctaaggagag ctggaggcat 4140
   gaaccaagca gagtggctgg gtgtttgaat ccttgcttct agtggcagag actgaccggc 4200
   tattcatcga acccattttt atttcctcct gggcacatgg actatggtcc tcgtgttctt 4260
   gagttctagc caatggaatg tgggcagaag ggatgtgcta gcccttaaac cttccttact 4320
   ctctcccacc tcttgccagc tgtaaggaag agtcacagta acatctatta tttacttatt 4380
   cattctttca ttcatttatt tcattcactc cctgaaacaa gagccaaggg tacagcctct 4440
   ctaggaggtg actctcatgc ccttttatta tgctggcttg atactaatgg agatgcggtt 4500
   gaccattgca aaaacccctg ttgagacaca gcagagttgg ccagcattct tatggtactt 4560
   cggatgtgga tcctgtgatg gctacagcat aaaacactgc attagcctcc atcttctatc 4620
   ctttttccct tcaaaaatga tgatcatttc attgagtgcc caccctgcac caggtgcttc 4680
   tgatacttta tgaaatatga tgttttaaat atgaaaatgg gggcttacat aatgctgtga 4740
   ctaatttgag ccttgctcag cagttaggca gcttcaatct tcttattaat atacagggag 4800
   tggttgctga taataggcac attttcaagc ccaaaggcat cttgacagtg catgctgagg 4860
   attgggggaa ataggaaacc tccaaggaat actcctgata tctttgcatc tgtatacaca 4920
   tatgagttgt cttagtgttt ttaccataga agatccttaa aagttgacac aggcaggctc 4980
   tgggtatgtg catcgagaga tatttcccag agtctgtttt tttgacatcc tttcaccatc 5040
   attttatctc ttagcaggaa tcagttgccc aacaactcct gtgatcatct gcaaacctct 5100
   ttacagtttt cagtgtctca aggtaataca taaagaagga agaaacaaaa gagtatgagt 5160
   atcactattc atggatacta attaccaaat gtactttttt gaattaaatc cttgtttgaa 5220
   tggaggttgc acagagatga atgttttgtt agcagacttc tcgaggccat ggcaggttta 5280
   aataattttg cgccacggga gacaaatttt cctgatgttg gaatcctaat gtgggtgaac 5340
   catgtgaaat gctgctttca gcaaggagct gacttcccag gctccctttt ctgagggagt 5400
   tgggcagagt tggggtggtg acagtatggt ggaggcagaa gcttctatgt cctgattccc 5460
   ttgtactaga aatttcatta cagatacttg tgcacatcag atgcattgtt ggatcagagt 5520
   gtcgcatgtt cgttccttca cagatgttat ttcagattaa gccacagaca gcggggagtg 5580
   gacgtggcat gcattttcct cttcatttgg tagcgtctca tctcccagca gtcatttgca 5640
   gagcaacatt acttgcataa caattgagcc gatctgatat tttaggttat gatgtcaaga 5700
   tatattatac tgcagcctac tgactttata aaaaagtagc caaagaaaga aaatgattag 5760
   ggccgagttg ggattgaaag ggacctgagg gaatgtcata tgttatgaga atcttaaaag 5820
   cttctaatga cctgttattc cccaagggcg cttgctactg tgacgattga atttattttg 5880
   tactaaacaa gctttgcatt taatgccatc tttttttatg gtctgagacc cacaagaatg 5940
   tgttttacta ttttctgaat aaaatacttg gtcaacagtt tatcatgcaa tcatctttct 6000
   ttatgcttcc atgactatat ttagtaatac tatattttgt attattaaat gtatttatga 6060
   attcttctgt tataatactt ttctgtttaa ggatgtttga cagtgtatat cttaaaggcc 6120
   aagtgtcatc tttgcttttg taaatatatt ttatagtctt aaactgaaga tgttgtttgt 6180
   gagcattttc atggcataga atgtatgcac aaagcttagg attaaagtaa gccttttaaa 6240
   aacagcaacc gtgattatca ccaaatcaca gtgtttttag ggtaacatgg actatggtcc 6300
   tcgtgtgctt catatgaata atcccacctc ccttctacct ggtaagctct atgacagaag 6360
   atgctgtaca taacaggtat tcaataaata tttgttggat ggatagatgg attaatggat 6420
   gaatggatgg aggaagtaga tatgtgtgtg atttggggtt gagggccgtg ctttttctgt 6480
   gagtccagac ttaagtcagg ctgttttgca tcactgagct tcacaaacat ttactggaag 6540
   tcaatgtcct gctgttcatg tgaagaaaca aaagtgcaga gggctaatga actcttagga 6600
   ggatggcact gctttccatt aagcaaagcc agcgtatttt tggcttagaa aatgagttca 6660
   aatttgaaat tgctttctcc aaaatggttg cagtctttca aattacagaa ctctcctgga 6720
   aattggagaa taaataaaaa tgcaaggatc cttgctttgg tgagtaagct cacaaggatg 6780
   gaaataggca caaaaagtgg cagtgtctct gaattatcac tgagtgtgag tttctaagag 6840
   aacaaatcac tttcacgcag ccttgacaat cacttggtta gctcaacccc aggaaaggct 6900
   atgaacaggg ctgggaagca gaaataatgc attattttgt tgagcttatt aacatttggg 6960
   cagaaaggat agattccttt tacatagtta ggcatcattt taacatgtac attataggtg 7020
   gtggcttttt tgtaacagaa ataagtgtag ctatccctta acaggagcaa tttcccttca 7080
   tatttcttgc agctcttgtc ccatgaggcg acagatgagt atgtgatagc aatactgatt 7140
   ccagtgctga gatgccatct gatccactgg gagagctggt ggattcaaat caatgtcagc 7200
   tcatgccttc tgggcagcag tctttcagtg caggatttac tctatggatc agaaaaacag 7260
   ctggtgactt gcaatatatt atacaaaaga cctttaaaat gttttgttgt ttccagctat 7320
   gatgtagtgt gagtcacttc aatttttttt atttgtgtgc ctttctgatg gaacaaaatt 7380
   ccgttaagca actggtaaaa acataatagt aatttaaact ggtgtctgta gaatattgag 7440
   actgaagagt gattagaaat aaggttttac tttaatgtgc atttttagtg taaaaatttt 7500
   acccatggaa agcatttatt ggaaatcctc tcctgagatt ccctgtaaaa tataagcata 7560
   tgccaaagcc aataatcctt gtgggttact tggaattatt ggaaaagaat aatttttacc 7620
   gagttatttt gctaagttgt gggttacagt tatttgtttc cagggtatat tgtttgtaca 7680
   tgacagaatt ttggactttc ttctttcctt taggtcaagt tttttcccca taaaagatgt 7740
   ataataattc atattaacat taaaacattt attttgggag gaaatactca atgcttttag 7800
   tcccagaact agtcttcttt ccctcactga gtcatttttg gtttttatac tttggttttt 7860
   catgggtttt tttaaatgag gtttctttct cactttaaaa aaaaaagtat tttctgtctc 7920
   tgtagtccaa gtttctggta acaattagga tcactagtac agaaatacaa tgtatgtcta 7980
   gaaaaaggta caaaaataaa ctagcaagat tcttggatgg tctggatacc taaaagacaa 8040
   gggatttgaa attttctgag tgtttggtga gtccagttag tggctatttg gtatcaagtg 8100
   ggtatcccaa atctgatgtg attagccaaa tttccacacc aatcatccag agccaatgcc 8160
   agaatgacga cctcgcatac atcaattcaa tggctatggt ttcccagttg ctttctgtag 8220
   ttctctgttg aatactttaa tgaaataact aaatagacgc cagccatctt gggcatctct 8280
   gtggatgaag agctatgtga aatactatca tactcaatat ttgtaggggg tgagtcttaa 8340
   gtctgtggtc ctgtcaagga gttcctcact acatttgact gcaagcataa ttagcaattg 8400
   gtgttcagtg tgcaaggaac tttgattgat tgggctgtgt tttaggcctt gtcaacatga 8460
   acaaaacata tcatcaggtt cgtccatctt gcttagctac ttttatatgt acttgaatat 8520
   tccagcaata ctaatttcca ctgatcaatt aaggaagcaa tcctaaagga agatgagatt 8580
   aaaagaagga aaaagaatca caaaccaaaa gagcatttcc tgaagtgtgt ccaagaacac 8640
   taattattgg agaagttatt aagatttggg ttggggggag gtgggtaacc cagtgaaata 8700
   agtttgagaa atgctgagat aagccaagtt agcatttttt tcctatattt tagggctcat 8760
   cagagctttt aacatgccat gaacattata aatctcaaaa agggagacct agcttcccca 8820
   acttaatgga acacctcctc tacatcccac catctgattt ttttccccaa agaatatttt 8880
   gaaaaacaac cttttgggat gtttgcttaa tacatgtgga cttaaaatta aataataatt 8940
   ataaataatt aaattcagtt gtgttcttgg catcataaat ttatttgact tatcatttat 9000
   tgaaagcctg ctatgtgcca gacactgagc taagtacttt tacatatatt aaaattgtaa 9060
   tagccccatg aagtaggtgc tattttacca ttttaatgaa taaggcactg aagcttagag 9120
   aagcctggtg attttcccaa gtttgcagaa gtaggaagta gtgaaggtag aatccaaact 9180
   taggccataa ctgataacca tgatactata acaaatagtc taaataaaat aataaaaatt 9240
   gtgtgtttat gtctgtaaga gaaagctgga ccacagcttt ttagatccag agaaaggtgg 9300
   acctaaaaat tttcaggaat tattaaatcc agttctcaat gtttagagac ccagaacagt 9360
   gatatcattt tctaatgata caccactaat tgatgaacag gaaggagctg gaaccatact 9420
   ctttttgact ctttgatgat aagatgccac gtcattcttg atggcttttt aattagttga 9480
   cttcagaaaa cgaaacaaga taatattgct tttaaaagtt tagatctgtt ttgttagtag 9540
   attataggaa tttgctatta ggttcattgt ttcatgatga aacattgcaa atgttcattg 9600
   gaaatgtttc ataccaaaaa acagactata aataaaaggt tatttaaaat atttaaaatc 9660
   atgtgagtgt gaaggtgagt gagaaaatat agtgtccagg aaaagtaatg tcaaacaata 9720
   gcatttgaat aataaatgac tcaaccgaag tagaatatag gctttttttt tcttgaggat 9780
   caaagcacaa cctcagcttt atttataggg gtaatagtca tctctgtagt gtgaaatgaa 9840
   atggggcact attatgttgc tgcacagtct aaatttctac tcttacatac attatattac 9900
   attaaggcaa tatgtttcag tagccatctg tttaaatgaa aataactctg gagcagaaat 9960
   acgtaaagca gtttctcaaa tattaaaccc agtctgagct tgggcttggc atatctcttt 10020
   ttattggtgt caggaaatat aaaagataaa ttttgatggg aggaggctca tatggaaatg 10080
   gtcagaaaat gaaagaatgc ctggggcagg cagtctaagt acaggcagca atttatccca 10140
   gaagaaagga atgtaggaag tgaggtcacc cagaggaaag atgatcctag gagatgctgg 10200
   atggtgctaa agacagtatt caatatcgta actttcaacg atataatcca atcttaggga 10260
   ttgcctcttt accatagaga aacatatatt tacaagcctc tcagcttttg taaattgtta 10320
   cacttttttt aaatggttgg gtaaaggaaa gtctggagga atctaactct agcactgtcc 10380
   ttgagcagct ctcatcaagt ataacagctg ctgtgtatgt aagagaagtg ggggatgact 10440
   gggaaagttg ttccagtgtg atctcctgaa atagatagaa gacttgcaat ttcacaccaa 10500
   gatccacagc ctggccttgt tttctagttg cagatctgaa gtagtagtag tccatctgtc 10560
   agcctgacat ctttttatgc tccctttcaa tcactcccag aggaaaaaaa atattttaga 10620
   ttgattctga ttttggtaac tttcttatta cctatcagtt taaaaacatt aaaagatagt 10680
   agcagatttc attttctctc atcctgcagg tttttaaagt tggaatttta cccattctat 10740
   ttgggtccta ggaatagtga tataactgac agacagctga ctaataaatg aactggagaa 10800
   caactattct atttgtcttt acataaattc tttaaaaaat gatagaattg gtttattcat 10860
   tcactaaaca gacatgaacc aagcgcataa cagaacactg gaaagttaaa ttcctatgag 10920
   ggagatgtca ttctgggctg aacaaggctg tgcaagaaga aaagggacca agaatctgac 10980
   taagaatgaa gatgatgatg ggcacccttt gtggatgcaa agatcagtag aataggtgtc 11040
   ttgcaccaga tagattcaag tggaaagagt gatgggtgat tcaggaaaat cctcaagggc 11100
   caggagacta attaaggtca agtcctgtgg agagcaaaat ccaggagatg tatcatcatt 11160
   tgccatatga ggaagtgggg atttgctacc tcagatccca aaaaaagaaa ctctttgctt 11220
   tccatctcac tttgttttgt ctattgtgtt aggatgatcc ttatacatga aaaagctcaa 11280
   aaactgactc aagattccag gttttggaag agagccattc atgcaatgcc aaagtacaca 11340
   tcaaaatgtg caacagagag tgtggagtga caccatcaac taaagaacta gtcaaagttc 11400
   ctacgagcat ggcgccatac agccaatctt ccttcttggt tctcagtgtc ttcgagggta 11460
   gctcaaaggg tcactgtcac agtgaccatg aattgtacag gaatcctgta gacagtgaga 11520
   agatcaagcc ttaagaaaca ccacatttat ctatttggag gtagagtact gtttgttttg 11580
   gtttgatttg gtttttattt gatttggagg ttttggctat aaaagagtgt cattttgtga 11640
   ttcagaaata ctcactcttt aaaatgttag tttcattttt agcgtcctca cattcttttg 11700
   gttcctttta ttggaatccc atcttataac ctaatcctat taatatttat agggtccaca 11760
   tgaatatgaa attaactagt aggttccatt tatgaaaagg gatcaactat taagtcctat 11820
   attttgttct ttaactttgg aatttgattc atgatatctt gtaaagcttt gaagtcaact 11880
   gacagtatga cagatttaca aacatctgtt tgaatttgga ggcctttcca tctcttctga 11940
   ttgaaagtaa ttatactcct cttctgcacc tgttgcattg ttttatgttg tcatatactc 12000
   gttgagtgca tgtacacgta tctgtcacgt cacttagatt gaaagcttct tccagccaga 12060
   aatttgtgtc atttgtcttt ggatcctcag tggtaatcag agtctggcat atagtttatg 12120
   ttcagtgtat ttctaaggag ggtagattta aggagggtag agatgacatc atcatagtaa 12180
   taaataagag taacttttct taaagactgg atttctatac ttgggaaatg gatattactt 12240
   tgaaggggag aagagagtga gagcaagagt gagaaaagaa gaaaatgaat ggagttcatg 12300
   agtacctagg agttgtttgg ctagaataaa acaagtgttt cattacagga aatgaagata 12360
   atataaagag cagcttaaac ttttttttag tggactaagt tgagaatcct gtggagtgta 12420
   gagttttcat gtctgggtag tcatgccttt cttatagtta tggagggtta ggtatgaacg 12480
   gggaacattt aaccatctga attgaccata ttaaccgaaa atggcagtaa agtaaccata 12540
   aatgccaggg aagtaaccat gcctacctag gacacatcag tgataagaaa atgcttcttt 12600
   caacttttta acagatttat tgaactgtaa tctacatacc ataaagttca cctttttaaa 12660
   gtgtacaatt caaaggtttt tagaatattc gcaaagttgc atacctgtca ctgtaaccta 12720
   attttagaac aatttcatca tcctctcccc aaaataatca ctgcccattc tcccctcact 12780
   tagtcccaaa tcctaggtaa tgactaacct acttttctct gtacatttgc ctattccaga 12840
   catgtcatat aaatggggtc atataatatg tgatattttg tgactggctt ctttctcttt 12900
   gcataatgtc tttgagtttc atctgtgtca tagcatgtat cagtactgca tttctttttg 12960
   ttgtcaaaaa attccattgt gtggatatgt cacattttgt ttaataagtt gatgaacatt 13020
   tggatttttt caatttttgg ctattataac cactttgttg aaatcaataa accacaaatg 13080
   tatgggctta tttctggact cttaattgta ttctattgat ttatatgcct atccttatgt 13140
   cagtatcaca gagtcttaat tactgtagtt ttataataag ttttaaagtt tggaagtgtg 13200
   agtgcttcat ctttgttctt tttcaagatt attttggcta tttgggttcc ttgcatttct 13260
   atacaaatct taggatcagg ttattatttt atgcaaaaaa aggcagtcag tatttttata 13320
   aggattgcat tgaatctgta gaggaattta gagcatattg ccatctttgt aatgtcaagt 13380
   gttccagagg agtaatgtca gcaaaatgga ggaatatagg agctttctac cctcatctcc 13440
   ctacagtgca ccaatttaga cagccaccca tgaaaaagat taactttgtg ggagtctgag 13500
   agtccagaga agcttcagca tgttcttgga ccaaaaatct aagaagtgat gcactgaaga 13560
   aggtaagaag aacagtttca ttttacccac attactcctc ccccaatttg gcataggtca 13620
   gtgacaagag agacccacgt ggtccacaag tttctcccat gaagggaagt aaaaagcata 13680
   gtgaatgagc tcccggcctt cccagctgtg caggatgcac tcaagaggcc tatctctttc 13740
   ttgccccacc cagaatactg aaaaaaaatc agcataactg agtagctggg ggagcctggg 13800
   agcagggaag agaggaaggg attcacagca actaaccagg aaactcaaca aagggccatg 13860
   taacctacta accgtcttct cagattccat caggtagtcc acttaaaagc catttgggat 13920
   gcctcaacag cacacccact ccaaatgtcc ccatgaggac ttccaatgtt tgtcatggct 13980
   caccttcaca cacactctgt gactggatcc ctgtgcattt ccctctcatc ccctccagtc 14040
   ttcctcccaa tgcacagtga gtgtgagcct ttgcagatgg cttgggagcc tgcacaatca 14100
   gccagcttgc ctctgcaaga ctgggagaag gcacataaac ttgagcattt ttttccctag 14160
   ggcatcaccc tgaggaaaaa agcagaggca ctgagcatcc actgtgactc agtgagactg 14220
   tgagaaggca tacaatctta agatacgaaa gagaagatga gagtgggttg gatgcatcca 14280
   tagcaaaggt ctgagactat gtacagtggt tcatgcctgt aatctcggta ttttggaaag 14340
   ccaaggcagg aggatcactt gagcccaaga gtttgagacc agcctgagca acctactgag 14400
   accctgtatc tacaaaaaaa tgtttttttt aattaactgg gtgtggtggt gtgtgtctat 14460
   agttccagct actccagagg ctgaggcatg aggattgctt gagcccagga ggtcaaggct 14520
   gcagtgagcc atgattacac cactgcactc cagcctgggc aacagtgcaa ggccttttca 14580
   caagaaagag agagacagag agaggaagaa agagagagaa acgaagaaag agagagacac 14640
   agaaaggaag gaagaaagcg aaaaggaagg aagggaggga gggatccaga gaatacctag 14700
   ctgggctgac tggccaagtt atttctctcc ctaagccagt cagtaagaat tagaggaagt 14760
   gactgcttat ttaaatgcaa agacagcagt acaaaacttc aaggaacatg aaaaactaag 14820
   gaaacatgat atcatcaatg gaacacaata aatttcccat aactaacccc aaagaaagga 14880
   agatttatga attgcttgac agtagtattc aaaaatcatt gtttaaagaa ggctcagtga 14940
   gctataagag aagcacagac aactaaaaaa aaaaatcaga aaaagtacat aagcaaaatg 15000
   agaagctttg ttttgttttg ttttttcgga gatagagtct cactctgtaa cccaggctgg 15060
   aatgcagtag catgatctca gctcactgca acctcctcct cccaggttca agcgattctc 15120
   ctgcctcagc ctcccaagta gctgggatta caggtgccca ccaccacgct tggctaattt 15180
   ttgtattttt agtagagaca gggtttcacc atgttggtca gactggtcgc aaactcctga 15240
   cctcaagtga tccacccgcc ttggcctccc aaagtgctgg gattacaggc atgagccacc 15300
   acacccagcc aagaagttta acaaagaaac agaaataatt aactaaacaa actcttggac 15360
   acaatgaatg aaataaaaaa tgcaatagag aggcaacaac agacttgatc aaacaaaaga 15420
   cagaatttgt ggactcaaag acaggtcact tgaaattatg cagtcaaagg agaaaagaag 15480
   aaaaaagaat tgaacaaaga aagcttatgg gatttacaag tcaccaccaa acaaaagaat 15540
   atatgcatta taggagtctc caaatgagaa taaagagagg aaggaaaata agtttattta 15600
   aagaaatcat agctaaaatt tttccaagtg cagtgaaata tggacatcca gatacatgag 15660
   gcccaaaggt tcccaaacag gttcaagcca aaaaaaactt caccaagaca tattataatc 15720
   aaatgtcaaa aagcaaagat gatggatttt gaaagtagta tgaaaagaga cctatcacat 15780
   gcaaggagac cttcattaga ctgtcagaag atttcttagc agaaacccca gcagaccagg 15840
   aaagagtggg atgatatagt caaagtgcta aaagaaaaaa aaatgccaac cagaaatact 15900
   ttatctagca aagctgtcct tcagaaatga aggaaaaata aacatttttc cagacaaaca 15960
   aaagctgaag gagttcatca ccactagact tgacttacaa aaaatgataa agggagttct 16020
   tcaagctgaa atgagaagac actaattaat aacatcattg gtcaaggtaa gtaagagtaa 16080
   gtaagtacag tcaaattcag aatactctaa tactgtaatg atgatgtata aatcactttt 16140
   agctctagta taacagttaa aatacaaaag tattaaaaat aactatagct acaataattt 16200
   gttaatggat acacaatata aaaagaagta aagtctgaca ttaaaaacat aaaatgagaa 16260
   agtaaaagtg gagagttgtt gtatgcaatt gaagttaagt tgttataagc ttaaaataca 16320
   ctttataact ataatatgtt atatgcaagc cccatggtaa ccacgaaaca aaaacctata 16380
   gtagaccaca aaatataaag ataatcaaaa catacagtat ttttttaaaa aatcatcaaa 16440
   tcacaaagga aaatagcaag agaagaagaa aggaatataa gtaagaacta caaaacagtc 16500
   agaaaaggat tactaaaatg tcagtagtaa gtcttttcat atcaataatt actttaagtg 16560
   taaatgagct aaattctcca tttgaaagac aaagaatggc taaatggatt ttttttttta 16620
   atctgctacc tagaagagac tcactctaac ttcaagttac acctaggttt atgtgaagga 16680
   ataggaaaag atactccatg caaatggaaa ccaaaagaga acagggtaac tatactaata 16740
   tcagacaaaa aagagcttaa gtcaaaaatt gaaacaagaa accaagaagg tcgatatata 16800
   ataataaagg gataatttca tcaagaggat ataataagtt tatatacatc taacatcaga 16860
   gcatctaaat ataaaagcag ttttaatgta tctgaaggga gaaatagaaa gcaatacaat 16920
   aatagtagag gacttcaata ccccactttc aacaatggga agatcatgca gtcaaaaaaa 16980
   ttaataagga aacattggac ttcaactata ctttagacca aatggactga acatacatat 17040
   acagaacatt tcatctaaca gcatcagagt acacatgcat gtcaagcaca catagaacat 17100
   tctccaggat ccttatatgt gtcttagccc ttttgggctg ctataacaaa atgccataaa 17160
   ctaaatggct tttaaacaac agaaatgtat ttctcacagt tctggaggct gggaagttca 17220
   agataaaggt ggcagaagat ttactgtcta gtgagggtcc gcttattggc tcatggacag 17280
   ctgtcctcac taatggaagg ggcaagagct tcttatataa ggacactaat cccattcatg 17340
   agggctccac ctcatgacct agtcacctcc aaaaggccct ggctcctaat tatcatatca 17400
   tatcataaaa tgtattgatt attacatttt aacatgtgaa atgtagggag gcataaactt 17460
   tcagtttata gcaatatatt agtctacaaa agaagcctta acaaactcaa aaatattgaa 17520
   atcataacaa ttatctcttc caaccacaaa agtaagaaac tagaaatcaa taacaggagg 17580
   aaaactggaa aattcacaaa tataaggaaa gtaaacaaca tatttctgaa caaccaatga 17640
   gtaaaagaag aaattgaaag aaaatttttt aaaaattgaa ccaaatgaaa atagaaactt 17700
   aacataccaa aacttatgag atgaggcaaa agcagttcta aaagggaaga ttattgtaat 17760
   aaacacctac attaagcaaa aagaaagatc tcaaataaca acctaacttt aagaaaccag 17820
   agaaagaaga acaaactaag cccaaagtta gcagaaagaa gaaaataaca aagattggag 17880
   cagaaataaa taaagactag aaaaacaata gaaaaggtca agaaaactaa caattggtgt 17940
   tttgaaaaac agaattgaca tgttttgaaa aacagaattg acaagccgtt agctagacta 18000
   agaaaaaaat aagaaaagtc tcaaatacat aaagtaagaa aagaggagag attacaactg 18060
   atatcacaga aatacaaagg ataagagact actatgaaca actatatata agcaaattgg 18120
   ataacctaga agaaatgaat gaattcctag atacatacat cctatcgaga atgaatcatg 18180
   aagaaataga aaatctgaag gtaccactaa taaggaggtt aaatcaccaa tcaaaaatct 18240
   ctcaaaaaag aaaaagtgag gaccaggtgg cttcactact gaattctttc aaacatttaa 18300
   agaataagta atagcaagtc ttctcaaact cttcaaaaaa attgaggaga atgggaacac 18360
   ttccaaacgc attttacaag gtcagcatta ccaaagacag acaagaacac tacaaaaaaa 18420
   gaaaactgca ggccaatatc cctgatgaac atggatgcaa aaatccttaa caaaataata 18480
   gcaagccaaa ttcaacagta tatgaaagaa tcatacacca taatcaattg aggtttgtcc 18540
   ctgggatgca agcatgattc aacatagaca tgtcaataaa tgtgatacac aacataagca 18600
   gtgtgaagaa taaaaatcgt atgtccatct taatagtcac agaaaaatca tttgacaaaa 18660
   tttaacactc tttcctgata aaaactctca acaaactagg tataaaaagg aatagacctc 18720
   aacataataa aggccatgtg tgttaaaccc acagctacca tcatcctcaa tggtgaaaag 18780
   ttgaaaccgt ttcttctaac atcaggaaca agacgaggat agccactctc atgtctattc 18840
   aacagagcac tgaaagtcct agccagagca attagatagg aaagaaataa aaggcattca 18900
   gattagaaat gaagaagtaa aattatctgt ttgcagatga catgatctta tatatagaaa 18960
   accctaaaga tgccccaaaa aactgttaga actaacaaat cagggctagg ccctggaaac 19020
   ataaggagga agagaacact agtccctgct ctctaggtgc tcatggccta gtggggaaag 19080
   catggatcca aatgaaaatc acatcatgca agaaatgcca tcaatggcat gactaggtgt 19140
   gataaaatta ctgttaagag agtgattggt ctgactcaga tagttagata acgtttgcat 19200
   tgatcagagt ttagttcaat ttttaagaat gtgagaaata ggcatgggca tactgtaatt 19260
   ggggaccagt ctgttgccaa ggcacaggca ctatgggggt agggaagttg gaaaggtgga 19320
   gaaagagtgg caagaaatga gtctaaaatg atagcttccg gctagaatgc catcccaagg 19380
   aatttggagc caatccaggg ggcaataaag agcattgcag gtctataaac cagaagacta 19440
   gtaagttcgt attttgtttt agggaactga ctctagcagc cccatgtcgg aaagattgga 19500
   ggaaagagat gaaaatggga gatggtgaag gcctgagtta agacaatggc agcagggctc 19560
   atagaggagg gggtaaaact gagacaatat tgttgatgta gctgggatga aaactgaaat 19620
   gtgtcaaaga tggagtcgga aattagaaga taacatagtc tgatgggaag aacagtacat 19680
   gaagtctgca aatctggact caagttccag cttagctgtt aactagctgc atgtccttaa 19740
   ttaaattttt attttatttt attttatttt attttatttt gattttgatt tttttgagat 19800
   ggagtctcgc tctgtcaccc aggctggagt gcagtggcgc gatctcggtt tgctacaaac 19860
   tctgcctccc aggttcaagt gattctcctg cctcagcctc ccgagtagct gggactatag 19920
   acacacaaca ccacacctgg ctaatttttt tgtattttta gtagaaacag ggtttcatcg 19980
   tgttggccag gctggttttg aactcctgac ctcaggtgat ccacctgccc tcagcctccc 20040
   aaagtgctga gattacaggc taattaagta tttaaccact ctgttcctgt ttcctcagct 20100
   acagagagac tgagtagatt atctcacaga ttggcttcca gaactaaaat acaaaactaa 20160
   aaggaagagg aggaggagaa ggaggagaga aattgagaca atttatgtag atatcatcca 20220
   agataaagat gactaaagaa acagagcaat tggaggatta taatttaaga gagttttgtt 20280
   tttaggagat aaaatgtctt gtagaaaaag tttacataac acatttttag gtgttcagga 20340
   tatccaaatc ctggcacttg ttcttcttac ctacagggtt ctctgccaaa gaacacctac 20400
   aaaaggatca ataatcgaat aagaaatcca gtgtaaattt tatcagtatc tgaatagttc 20460
   cctgaaaatc caagggaaag ctggatggca ggcataattt gggacaaaga aggaatggag 20520
   tctcttatgt cccatccaaa atctagacct tgggatccta tgctcatgtg tgggagaagg 20580
   tgtataggca catacactct catgttcaga gagttggtgg gtggagggag agggaaaagg 20640
   tgagcccaag gacagcagag agaaagaaaa agaacctaaa tatatgggca tgctcacctg 20700
   tccaatatca ggctgaactg agcccagaga tacccattat atttctgtgc aggattactg 20760
   atcactaggg gaaacatggc cccataaata acagatttaa tcagataagc agctcctctc 20820
   ccatgagaac tagttcagaa ctaaagtaaa gagctggctg tgagggattc atccaagaat 20880
   ccactgagcc atgcaaggat ggatgttttc tcttatgagc tgagatagaa agctgggaag 20940
   agaagcgaag tgtctcatcc ttacccaaca gtattatttt gattcttcaa tttttataat 21000
   atgatatcct ttgatggtgg gaactatgtc ttacccatca ctgctcctag cttactgaac 21060
   tacagtgaat gttaattgaa tcagtaaata tattaataat caggattgac tggatttaaa 21120
   taagattatt gcaactcaaa gaatcctcgg gagggcatca tccatcttca ccatttgtta 21180
   tgaactcttc atgtgtatta attcatttaa tgcaataaca ctataaggtg ggaactatca 21240
   tcattccccc attttacaaa tgaagaagct ggggtctgga ggacccatct cgccccatca 21300
   cacagttagc aagtggcaga acaggaatgg gaagccagat attagataca gaatttatgc 21360
   atttgcaacc acacacactg ccagccaaaa acatgctttt cctctccaag agcacaaccg 21420
   ctagctttcc ttattggctt atgaaaactg gaaacccagt ttttaaaaga ctgtgaaaaa 21480
   aaattaagcc aatatgctta tcattttaaa aggtactcta tttaattgag gatatatgtt 21540
   ttcatacttt tattcccaaa tggttatcac ttccttaatt aaataataca tcaattatat 21600
   ttgtctataa atatgtatgt gtatatatac atgtgtgtgt gtgtgtgtgt gtgtaccagg 21660
   ccctcaataa gtatttgctg aatgtttctt ttctgcaaaa tctatgtata ctaatgtttg 21720
   ctaaattagt atacacagtc tttctactgc tgtttagagg gaaggtgcga tgatttccca 21780
   tgtttttaaa atcagatggt tcaatatatt catgccacat ttagagaaga aagcaattca 21840
   cggtagagta tgaactctca aagactttaa gtctcaatgt aatttatcaa ctttatatat 21900
   caacaaactt ttatttagtc ctctctgtat ataaaaagct tattagcgat cacatgctaa 21960
   agcagatttt taaattgcta gagccttaaa atgcccacaa tatagtccag aaagaagaca 22020
   tgaataaaat tggattatgt agcatagcat ttaagggctt attcacactt tatgccctcc 22080
   ctcttctatt cctacctatt cttctagcac acagggtcag gtcctttcca gctgcctgtc 22140
   tttcctcatg ctacgctggc acacttggcc aatgttggtg ccatgaagag gttactcatg 22200
   cagataggaa gctgaggagg aggttttaga aagaagctga tgagttgagt gatggacacg 22260
   ttgagtttag gtgccaatgg gaaagtacaa gtaaatgtat tgaagcacaa ttgaataata 22320
   atgctttaac aatttgataa ttatgtgtgg attaagggag agggaaagtt ttaagcctag 22380
   gatattggaa gaaggatgat aaaaaagtag gaggtcagtg ggaaagttga tttgcagaga 22440
   aagatgctga tattagtttt ggatttgttg cacttcagaa gatgatgaga cattaaaact 22500
   gatattgata atgtaatctt cacgatgaaa ataaagtaga gattgaggaa ctgatccaga 22560
   ctgaaggaga ctagagagac ccggaattgg gtgcaaccca ggatcctgga gcagtgcttt 22620
   tgctatgaag gacgggattg agccaattgg caaaacttga ttgcatcttt gggcaaaggg 22680
   tatccaggtt ttttaaatca gatgattaaa tagtatccat tgtatgattc ttataatttt 22740
   tctataagtt tgaaatcatt taaaaataaa aagcagaaaa aaacaaacat caccactttg 22800
   gcaacaaaaa gttggcattg tcaatatatc ttaagatcta cattagatat gtaagatgtc 22860
   tgtatcttaa tctagatgtg tgtgttcaca aactttaaac tagtaattct actttgagac 22920
   actatcataa agaactaata atttataggc atacttcatt ttattgtgct tcactttacc 22980
   gtgcttagca gatattgagc gttttttttt tttttttttt tttttacaaa ttgatggttt 23040
   gtggcaaccc tgcattgggc aagtcttttg gtaccatttt tccaacagta tgtgctcact 23100
   tcacgtctct gtcacatttt gggaattctt ccaatattta aaactttttc attgtatctg 23160
   tgatctatga tcagtgatct ttgatgttac tattgtaatt gttttggggt accacaaacc 23220
   atgcccatat aagacagcaa acttaatcca tgaatgctgt gtgttctcct acttctccag 23280
   tgactagtca ttctcatgcc tctctccctt tctccaggcc tcctttctcc aggccttctt 23340
   attccctgac acacaacaac attgaaatta ggccaattaa taaccctaca atggcctcta 23400
   agtgttcaag tgaaagaaag agtagcatgt ctctcacttt aaatctgaaa gctagagatg 23460
   attaagttta gtgaggaagg catctcaaaa gccaagatag gctaaaagct aggcctcttg 23520
   tacccaacaa caaaattgtg aatgcaaaag gaagagtttt tgaagaaagt taaaagtact 23580
   actccagtga acacacaaat gattaaaagt ggatcagcct ttttgtggat atagaagaag 23640
   ttttagttat ctgggtagaa gatcaaacca gccacaacat tcccttaagc caaagcctaa 23700
   tccagagcaa gaccttaact ctctttagct ctgtaaaggc tgagagaggt aaggaagctg 23760
   tagaagaaca gtttgaggct aaaagaggtt ggttcatgag gtttaaggaa agaagccatc 23820
   ttcataacat aaaaatgcaa ggtgaagcag caagtggtga tgtagaagct gcagcaagtt 23880
   ctccagaaaa tctagctaag atcattgatg aaggtggcta tgcaaaacaa cagatttacc 23940
   ctataattaa ccaggcttct tcttcttctt cttctttttt tttttttttt tttttttttt 24000
   tttttttttt tttttttttt tgagaggcag tctccctctg tcgcccaggc tggagtgcag 24060
   tggcacaatc tcagctcact gcaagcccca tctcccgggt tcacgccatt tctcctgcct 24120
   cagcctccca agtagctggg gctacaggtg cctgcaatga aacaggcttc tcttgggaaa 24180
   agataccatg taagacttgc atagctagag agaagaagtc aatgcctggc ttcaaagctt 24240
   cgaaggacag gctgcctgtc ttgttagggg ctaacgcagt tggtgtcttg aagttgaagc 24300
   caatgcccat tgattattcc aaaaatccta gaacctttca ggatgatgcc aaatctactc 24360
   tgcctgtgct ccacaaaaag aacaacaaag cctagatgac agcacatctg tttacagcat 24420
   ggcttactga atattttaag cccactattg agaccatttc tcagaaaaaa aaaaaaaatg 24480
   attcctttta aaggttactc cttattgaca atgcacctgg tcacccaaga gctctgatgg 24540
   agatgtacaa ggagatgaat gttgttttca tgcctgctaa cacaacatcc attctacagc 24600
   ccatggatcg aggaataatt ttaactttca agctctatta tttaagaaat acattttgta 24660
   aggctaacct accatagata gtaattcctc tgatggatca gggcaaagta cattgaaaac 24720
   cttctggaaa ggatttatca ttccagatgc cattaagaac atttgtgatt tatgaaagaa 24780
   ggtcaaaata tcaacattga caggaatttg gaagaagttg ataccaaccc tcctggatga 24840
   ctttgagggg tcaagacttc actggaggaa gtaactgcag atgtggtgga aacagcaaga 24900
   gaactagaat tagaagtgga gtctaaagac aagactgagt tgctgcaatc tcacgatcga 24960
   acttgaatgg ataaattgtt tcttatggaa gagcaaagaa agtgtttttt ggttttgttt 25020
   tgttttttta agacagtgtc tcactctgtc acccaggctg gagtgcaatg gtgtgatcac 25080
   aactcactgc agcctcaact tccccaggct caggtgatcc ttccacctca gcctcctgag 25140
   taactggtac aggcacgtgc caccacaccc agctaatttt tgtatttttt tgtagagacg 25200
   ggggtttcac catgttgccc agtctggacg tgaactcctg ggctcaagtg atcctcctgc 25260
   ctcagcctcc caaagtgctg ggataacagc tgtgagctac catgcctggc cagaaagtgt 25320
   tttcttgaga tggaatctac tcttggtgaa gatgctgtga acattgttga gatgacaaca 25380
   aagtatttag aacattccat aaacttagtt taaaagcagg agcaaggttt cagaagattg 25440
   actccaattt caagagaagt tctatcgtga gtaaaatgct atcaaacagc atcagatgct 25500
   acagagagat cttctgtgaa aggaagagtc catcaatgca gcaacttcat tgctgtctta 25560
   ctttaagaaa ttgtcggccg ggcgcagtgg ctcatgccta taatcccagc actttgggag 25620
   gctgaggcgt atggatcact tgaagtcagg agttcaagac tagcctggcc aacatggtga 25680
   aacctcatct ctacaaaaat agaaaaatta gctgggcgtg gtggcgcatg cctgtagtcc 25740
   cagctactca ggagaagaat cacttgagcc caggaggagg aggttgcagt gagccgagat 25800
   cgcgccactg cactccagcc tgggtgacag ggactcatcg aagcaagatc tccaccagct 25860
   aaaagattgt gacttactga aggcacagat gatcattggc atttttagca ataatgcatt 25920
   ttcattaagg tctatatatt gcttttttag atataatgct attgcacact tttagactat 25980
   agtatagtgt aaacataact tttttttttt ttgagacaga gtctagctct gtcacccagg 26040
   ctggagtgca gtggcatgat ctcatatcac tgcaacctcc gcctcccagg ttcaagtgat 26100
   tctcctgtct cagcctcctg agtcgctggg atcacaggtg cacaccacca cacctggctt 26160
   tttttttttt tttttttttt tttgtatttt tagtagagat ggggtttcac cacgttggtc 26220
   aggctggtct caaactgctg acctcaagtg atctgcccac ctcggcctcc caaggtgctg 26280
   ggattatagg cgtgagccac cacgcccagc cttgctttca tgattttaaa ggtattttaa 26340
   atcaaaaggt caagaagtgc acaaattcag gattaattat ttgtatttag gattatttgc 26400
   cacagcgttg accaaataat catttgtggc caaagttttt gaagaataga ctatagagag 26460
   aggagatcag agtgggccaa tagggagcca tgcagtggcc cacatttgga aaaggagagg 26520
   aatgtgctga gcggagttga cggataatta actagaaatg actgcactga aaagtgtgtg 26580
   tctgctggtg ttgctgaaaa aggttggagt gtgggtttat cacatcccga aaaaggctaa 26640
   ggagaagagg aactgaagaa aaaacctacc gattcactaa ggatttccca ccaagaggcc 26700
   tcaataaaga caaactggct tttataattc aattcaaaca caaattattt tttaaattaa 26760
   ttcctacggt ttgcattgat attttaacat gagaagtgga gttcagaaac tgcaacaaaa 26820
   gggatttgag cagtgataaa atatttgtaa gcctctcctg atgaccttgg tactgaaaga 26880
   gactgttctc ctgtgtcggg gaaggttctg gaccgccttt ccagcagcat tttatcatca 26940
   gaaaacggct cacttcataa ggtttataac cttccataaa actgcagaac attttattct 27000
   tttttcatta agccaccttt ggaatcattt tagtcccttt atagtctctt atttaatctg 27060
   ttttttcgta gactaaaggg agaaaaaatt ctatcagtgg tcaaaatagt aattagatag 27120
   tgtaagtcac agcctgatgg cttcaaggca tacatactta ctacttatct cctccatctg 27180
   ccttcatcag tgaattcttt tactcccaag cacatcttta cctttttctt aaacaatgga 27240
   aaaaagaaac taccaaaccg tgcattttcc caattataag agttactaat gtttatattc 27300
   ttttctatta aagcatcatt cccatggtgt aaaagctctt ttaccaaacc ctaaggaaag 27360
   catgaattaa taaaacatca agagatttta tttcctatat tgaaaactcc tatagttgca 27420
   taaaaacaac tgaatctttt ggaaatacgt gtaatcacaa ggagaggaac attaaaagag 27480
   agaaactcac tacaaaataa atcttaaaac cactttcatg tttaaaaaga agaaaatatc 27540
   agttcttctt attcctgaag gatttttttt taaaaaaatc ttttaattaa tgacagccct 27600
   cagggcactg agcaattgtt aattaaaaca aggagaaaag gaagaggaag taggcaaaaa 27660
   tgctaagatg aacacgtttc cttttctctg caggggaata acattaccaa atgaacatta 27720
   ataaatatga atactattga gaagaaatac atttaaaaaa attttaactc ccacctcagc 27780
   ttgacacaca ctagcagcta cataaattaa ttggggcgcc tcgagaatta taaacccctg 27840
   ggtgtcacct tttgtctaat tgagtattgg tagccatcac cctaccctca tcaggatgga 27900
   ttgtttcttt ctcggtgcaa gctgacttct gtcagggaat agcgcctgat caaaggtctt 27960
   ctaagggatt tcattcactt tcattcactt tcattcttgc agtgggcggg cattcacgta 28020
   ggcaaccgcc atcattataa aacatttctt aaatgcgagt taggagctag actcactgcc 28080
   cattcaattc attggtcttt ccctttgcag ccattaaatt tttacttaga aaaaaataat 28140
   tatcagatct tttcgttcac cccctgtgct tacctaattt gggcaggtta ttagtaataa 28200
   cattttcctg gtcgttccct accactttcc cctctacctc acatgaaaaa ggcttttatc 28260
   tttctcataa gctcagaagt tcacttaaca agcagagctt tagaagccaa gaacaagaaa 28320
   aaaaaaaaaa aggtaagaga atctgttttc tactttattt ggagggggac agaaacccag 28380
   aatcccatag gctgtatgtt tagcatgaaa aaaaaaagtc attatttgaa attaggacaa 28440
   ccagggatag tttttcccaa cttttaaatg atcccagttt tacgtaattt tatgggcttt 28500
   tcatcaaagt tgtttttcaa aaatagaact caacccctta gggtttaaga gttttttccc 28560
   ctttagtgtg cgtgcacaca cccctttgat gttccgcatg ctagcatatc agggtcagtg 28620
   catgggggcc agtgtcattt acagaatttc tcagatgcca gctaagatca gtagctagga 28680
   gacaacttag aatttcattt tgctctttgt tcagatacat tctttaccac agataagatc 28740
   ttacctcccc agcagtctat ctctaaaaga acaaactcta ggtaatcaaa aagctatatt 28800
   ctacccccca accaaaaaca aattgcttat tttagctaat acctagtcaa acataaatgc 28860
   cagggtagaa aaaataatgt attttctctt tccttgctat aataatatat accagttgaa 28920
   aactcctgat tttcctcaga ccacctaaaa agctttgtcc attgcttgtg ctcatgtagt 28980
   agctaaagga ggttatttac aaggcacaaa ggagaaatac atagcccgtc tatactgcat 29040
   cacttttttg ttctcttcac ttgtgcatag tatctactaa tgactagctg aggatagtaa 29100
   tttacataac attgaaatgg aaaaattaaa ggaacacttt ttgcttcgtt tttcaaagtg 29160
   gacttaccta agaagcacta ccatgctcct taaaaatgct ttcttggttc tccccactga 29220
   tgtcgccaat ctgggggtgg ggctcagaaa tctatttttt gtttttttag agcaaggcag 29280
   ggtcttgctc tataacccag gctggagtgc aatgttgcaa tcatgactta ctgcagcctt 29340
   gaactcctgg cctcaagcaa tcctctcacc tcagcctccc aaagtgctgg gattacaggc 29400
   acaggaatct atttttttta acaagcatga gagccattca taggccaaat ttatttattt 29460
   atttatttat ttatttattt atttatttat ttgagatagg gtctcattct gttgcccagg 29520
   ctggaatgta gtggtacaat cacagctcac tgcagcctcg acctcctggg ctcaagtgat 29580
   cctccaacct cagtctccca agtaaccggg actacaggca tgcaccacca cacctggcta 29640
   atttttattt ttttgtagag atgggatttc actatgttac ccaggttggt ctcaaactcc 29700
   tgggcttgag cgatcctgcc acctttgcct cccaaagtgc tgggattaca agagtgagcc 29760
   acctcagcca gcatgttttt tgtgggatct cattattttt aactgtctca tgcaatatct 29820
   aataataaaa tgtacatgtc ttcttcattt tatttctata tagagcgtgt tttattaata 29880
   catgttgtta caacatctaa ttgcaaatac atatgaaact ctatccaaac tcctacccaa 29940
   tctcttggta gtggaaagta catcagtcac aggaatagaa cccagagtca aggtcctgaa 30000
   gtttgggcct tggctggaga gcagagtaga ctcaggtcac aacaccagga tgggatacat 30060
   ggaggagaag gaggaagatg gggggaaagg aagattctag gtctgccgaa aaagatcagg 30120
   caggtaaaac tcagagacct tccagcaagg cccaaaacac cactagccac atgagaggtt 30180
   tcaagcaatt gactcatctg cccctagtgg gatttttaaa ctacagactg gtataggagg 30240
   tgacctgttt agatttactc tcaaacctgc tgtgttgggc cactccaatt tagactacac 30300
   tttgctgaag ttctgaaatt caagaattgc ttctcacctc cttgtgacct atgaagagat 30360
   gtcattatct gggtccaccc acattcactt gggagttcat tcaataaaac tttgcaatat 30420
   aatgtaatgg gtgtcccata gggattcggg gcagaaatat ttttaactct cattttgttt 30480
   caagtacaat agacagtaac taaattttcc agaaaacttt aaagatgctt ttatctctga 30540
   aaggtttttc ttttttaacc cacttcagtc ccactgatgt tttcactagc ttgcctccag 30600
   gataatgcat ttgatatcag tgaatgtgaa tacacacttc caaatgcttt gtgcaggctg 30660
   atccactagc tctctttaga cttttgctaa tcagaaattg ctcaagaagg aaatttgtga 30720
   gctgtttcca aattgcctac tgccttagac ttatgtaact atgacaggtt tattgatgca 30780
   gctgggagca agtttttaat cttagaagaa aatgtttatt tctaaataga ggtgcatata 30840
   cttttcataa aagtatttct agaggctttt taaaatgctt tgatggttta aactaataga 30900
   aataataatc tttaaatatg cttatcagta gcatccccag aattcacgtc tttagtcttt 30960
   gtacgaatta gatttcctgg ctaaacctgt gaaaaatctc atggtattag gaaatctgaa 31020
   tgttgtttct ttgtattatt caggaattat atatttttaa tttttaaaaa gttaaataga 31080
   tggagataat gttgcagtct gaaagggcag ccctgagaac ttataatcag tgctatggtg 31140
   gctgtcctgc attctttggg gcagagcttc ctgagttagt ccccacagtt actacctctc 31200
   cttcctcgcc tttcctctct tgctgctctc tccttcctat ctttactaat ttatcttccc 31260
   atgtcaaccc cttaaattct gctgctcttt catagaaatc acctcgttgt cccgcttctc 31320
   caccactcat attctagatt ggtagatatg gaccatacag atagtctacc atggctacca 31380
   tcaaatattt ttgttcactt cattgtgcat agttacctac tagttattca ttgggagtag 31440
   taatttgcaa attatttaaa aggaaaaatt aaaagaaaaa actttttgct gtagtttccc 31500
   tttttttttt tttttttttt tttttttttt tt 31532
<210> 35
   <211> 1390
   <212> DNA
   <213> Homo sapiens
<400> 35
   ccaagatttc tcatccatgg tttcaactaa gaatatttta ttctctccag tgaaattttt 60
   tacaattagg attgcaaaac tacatacatt caggtagatc tgtgcacatc attatctatt 120
   tggaaaactg tctgtagaat tgtgccgtgt accatccatc cagccatacg cagcagccca 180
   cagtcatgct gttctccatt catttataca ttctctacat catgttactt gcacacaaaa 240
   gtccttacac atggctctaa gccattttta accccctgga aagtgtaatt ccttgacttg 300
   gcaccctttc tcttaagaat tgatgtccct tgcaattctg atgaatacga gtctctacct 360
   aattagacaa aaatcaattg cgatcaatac tcaagaaatg aaccccctac acaacactct 420
   aaatttgctt tctgatccct agcaggaaca actgtctatt cattcatata ctcaatactt 480
   tagaacatac tgggtacaag gaatcatact atatgctgga aataaaataa tcagtgatgc 540
   tgtccttgat tttaaggaat ttaaaaccca agtatggaag ccaaacaagt aaattgacaa 600
   tcataaaagt actataagaa gcataagtaa ggactgctat agaaatacac aagtgaactt 660
   ttcaaggcat gcatgaaaga gaaaacattc tatagaagtt tcattcattt acttaataag 720
   ttaatattca gccggatctg gtactaggtg ccatgttaac aagtctgtat tttatccaga 780
   aggaaatgtg gaactattaa tcactgttaa gcttgcatgt ggcataatca gtttggtatt 840
   caagaaaatt ctgaattact ccacaggtaa agttttattg ggataacaaa gggaaagaaa 900
   aggagtaaaa agttattaga ggttactgat cataaggaat aggatgaaca aggaaagaaa 960
   attcagtgac tgaaaagcaa ataaatagga acatagcaaa aattcaggac tggctaaaga 1020
   acttgtatag tgtaaataac agagtgagaa aaatcgagag atcatgattc agaaatgttt 1080
   taggattttt agatgtagag gaattttagg ggacagtaga tctagggtgt acccaaaagg 1140
   gaagacagct gaaatgttag atagttgaga tgaagaccag tggaattgag gaggccataa 1200
   acttggatgg gtactcccct tggaagctga gtttagccac ttcgatgttt ggactcaaag 1260
   taaagtataa aaaagacagt agtcttatga gtttatcata gtggataaga cactatttca 1320
   ggaggcagat tgcagcatac taattgtatg ctcttcggca agttacttag ctcttagatt 1380
   ttcctctggg 1390
<210> 36
   <211> 38342
   <212> DNA
   <213> Homo sapiens
<400> 36
   cactgcagtc tctccctccc tggttcaagc aattctcttg cctcagtctc ctgagtagct      60
   gggaccacag gcgctcacca ccacgcatgg ctcatttttg tatttttagt agagacgggg     120
   tttcaccata ttggtcaggc tggtcttgaa ctcctgatct caggtgatcc acccgccccg     180
   gcctcccaaa gtgctgggat tacagaggtg agacaccgcg cccagccata tttagattta     240
   ttgtcgtttt cttttttgtt gttgttttct tgtttttttt ttttttttga gacagagtct     300
   cactctgttg cccaggtggg agtgcagtgg cgtgatctcg gctcactgcg agctccgcct 360
   cccaggttca cgccattctc ctgcctcagc ctcccgagta gctgggacta caggcgccca 420
   cccccacgcc aggctagttt tttgtatttt tagtagagac ggggtttcac catgttagcc 480
   aggatggtct cgatctcctg acctcgtgat ccgcccgcct cggccttcca aagtgctggg 540
   attacaggca tgagtgacat cttacagata acgataagtg caggtggtaa gttgttaata 600
   ccaaaggcaa ccatattctt tggacctaac gttttcttgt aggaataatt tgattcattt 660
   taccgccaag gagcaagtgt tcaagaacct cctccctata agggcagaaa atttagatgt 720
   cagtcttgct gaaatgcccc agtgccctcc ttctcacgca tccaagagtt tcctcatagc 780
   ttggttgggt ccagatgggc ttggtcccat gtctacatat aattagggtg gagaggagtt 840
   gaggcataca gcctggtgtc agcaagtcat gtcctgagcc tggagctgga caaagcccag 900
   aactcaccta gtggccaagg gcattcagga gaggcagtga ctcctacgga agttggtctc 960
   agcatttgtg ctgccatctc ttccggtcta actggccaga gacatttagg tgaagacctt 1020
   tcacattgcc tttctctcag tcaaggaaga ggtcagctaa attggtccag gaactttgag 1080
   tcaaactcct tcaattcctt gtcagctcag gggtgtagcg tggcagagag tcacggtggt 1140
   aggtggataa ggagttggga gtgtttgggt cttctcagta gaaggggcta gggtaggggt 1200
   tgcatctgcc tctggatttt gttgttgtgt tggtggtggt tttttatttg tttgttttat 1260
   agagacaggc tcccactatg ttgcctaggc tggtctcaaa ctcctgggct caagagatcc 1320
   tcccgccttg gcctcccaaa gtgctgggat tacaggcatg agccaccatg ccggcctgcc 1380
   tctgaatttt gtgtcttctt tccctgctgt agcatttagg ctagaaccaa gcccttttgc 1440
   agcagttctg gacacagtga ctgcctacaa ccgggcttgg ctgctgggac ctcactccct 1500
   actaggccca agctcagagt gttctatctc ttagggacca actctttcat cataagtaca 1560
   ttcaccccag gggcataacc caccccagga aataggactc aactctgtac caccgcagag 1620
   aacaccgttc cttctggctt ctgagggaat gatacctcct gaacctgtgg gatgcagcag 1680
   cgccgccagg accccggctt gggatgatct cccgctctga ttatcaccac cctgtggacc 1740
   aagaccatcc cccacacccg ctgtccaggg atctactctc ctgggaactg agcaggtgtc 1800
   ttcttaaacc ctgaaagccc aaagtgtctt gtgagtttgc cccttttctc cagacaatgt 1860
   ggatccttga cctactcctg caagggaggg tcccctcttc cactcttacc aagaaggaga 1920
   tgacacaaag tccatttctg gtagggtgct actttaaaag gcacccgtca cacacatgcc 1980
   tcaagcccat ctgaaattat ccagggtgct ggcctttctc tacctatgca gctgccctcc 2040
   tggaagcctg ggttgagaga gatgcgactc gttcagcagc ccgtctatgc tcagggtaag 2100
   cccgagcacg tcagcgaagt ctccctggca tgggatccgg catccgggaa gctaagaggc 2160
   atcagtgggg caagctggga tggttccaat ggcccatggc ctcctctcag ctttacccca     2220
   cctcacccca accagagaga tcagtaggct ccttctgagg gtgcctcctt tctcaaaggc     2280
   aggattcttc cctccagtgc aatggatcat gacctggctg gtccttcttt ctctctgatc     2340
   ttggcctcaa tctaagtttc agtcaagtga atgacattat cccttaccta ctaggaatta     2400
   aataggtttt aaagtacaca cagacacaca cacactttgg aatatttata tcaaaatgta     2460
   tgttttttag gccaggtgtg gtggctcacg cctgtaatct cagcactttg ggatgccaag     2520
   gtgggcagat tgcttgagcc caggcattca agaccaggtt gggcaacatg gtgaaaacct     2580
   gtctctgcaa aaaatacaaa atttagcctg gcgtggtggc acacacttat agtcctagct     2640
   acttgggagg ctgaggcagg aggatcactt gagccctgga ggttgaggct gcagtgaacc     2700
   gtgatcgcag tactgtactc cagcctgggc aacagagtga gaccccatct caaaatatat     2760
   atgtatatat ggttttttaa accaggtttc ttgagatcat ttcattggga atttagaaga     2820
   aaaagaggca ttctgggcgt gatgtatgga gaattcttct ggagcgcttc atggaaccca     2880
   gcctctgaag cagaagaaac aactgagttc caccaaggag ccctggtcac ttcacggtgt     2940
   cttaacttct ccacatcttg gagtcactta acacttccag gacaccaaac agtctaacaa     3000
   cttagttctc atgaaagaat gctgctgggt tgaattgaaa agcaggcaga gttagagaat     3060
   gtgccttcta caaagtagcc atctcagaga gacttgtttg agtgaataga aaaatgaaac     3120
   ctttagttaa agcaactgag tattgaacat acgcatgctt tagaactaaa aaataggccg     3180
   ggcacagtgg ctcatgccta taattctagc actttgggag gccaaggctg gcagatcact     3240
   tgagctcagg agttcgagac cagcctggcc acatggtgag gccctgtttc tactaaaagt     3300
   acaaaaatta gctgggcgtg gtggtgcatg cctgtaatcc cagctactca ggaggctgag     3360
   gcagaagaat cgcttgaacc ctggagctag aggttgcagt gagctgagat cgcgccatta     3420
   cactccagcc tgttatccca gaactttggg agttatccca gaactttggg ggacagagca     3480
   agattccgtt tcaaaaaaaa taattatgat tttaagactt gtgcttttta aaaaacaatt     3540
   tggaataata ttgcaatgct tttttgtttg tttgtttgtt tgtttttgag atggagtttc     3600
   gctcttgttg cccaggctgg agtgcaatgg cgcgatcttg gctcaccgca aactccgcct     3660
   ccaggttcaa gtgattctcc tgcttcagcc tcctgagtaa ctgggattac aggcatgtgc     3720
   caccatgccc gtctaatttt gtatttttag tagagacagg gtttctccat gttggtcaga     3780
   ctggtctcaa actcctgacc tcaggtgatt tgcccacctc agcctcccaa agggctggga     3840
   ttacaggcgt gagccactgc gcccggcctg caatccttat ttaattttaa ttttttgtgg     3900
   agacaaggtc tcactatgtg gctcaggctg gtcttgaaat cctggggctc aagcgatccg     3960
   cctgttttga cctcccaaag ttctagcata acatgtgtga gccaccacat ctggcctgca     4020
   atgctttttt tggccaatta taagttagct atattaaatg agactaacag tgatgtttaa     4080
   tgatactaaa taagacctgg ataatataaa ccacctttta tgtgatcatt aatatttttt 4140
   atggtaggct gtaagtgtac agtgacagaa atgaggttca aaagccaagc aggtatgcct 4200
   gggataagtt atagcctttt gttagcttta tgccactagc attcataagg cacatactat 4260
   attcaaggac agcgctatgg tgtgagtgtt tgcatccctc caaaattcac atgttgaaat 4320
   cttctttttt ttttcttttt ctttccttgt tttttttttt tttttttggc aggtaacctt 4380
   ctgagctaga gtaggctcag agactccctc atctgttgaa atcgaatctc caatgttttg 4440
   cctttaagag gtggggctgt tgggtcatga gggcagaacc ctcgtgaatg ggaataatcc 4500
   cgccataaaa gaggcctgag ggagtttgct tgcccctttc ctccttccac catgtgagaa 4560
   tgcagcaaga aggtgccctg tatgaagcag agggccctca ccaggcaccg aatccggtgg 4620
   caccttgatg ttgaacttct cggccttcag aactgtgagc aataaaattc cgcacttttt 4680
   ttctttttga gattgagtct cgctctgtca tccaggctgg agtgcagtgg tgcagtctcc 4740
   gactcactgc aacctccgcc tcccaggttc aaactattct cctgcttcag cctccagatt 4800
   agctgggatt gcaaactcca cctcccaggt tcaaactatt ctccagcttc agcctccaga 4860
   gtagctggga ttacaggcac ctgccaccac acccagctaa tttctgtatt tttagtagag 4920
   atggggtttc accatgttgg ccaggctgat cttgaactgc tgacctcaag tgatctgccc 4980
   acctcaggct cccaaagtgc tgggattata ggggtgagcc accgcgcccg gccacattct 5040
   gctttttata aattacccag tctaaggtat tttgttacag cagcccaaat ggactaagac 5100
   agtgtattta gactggaggg catggggttt gggtttagat atttgaaatt gcagagagat 5160
   gggctatgcc tcgcctttgt ctgcaggtct aggacaggtt ctgaggaatg tctgtagtgt 5220
   caactggaca atgcaatatt caaacataac acagaaaatg gaattcctta actcttgctc 5280
   tgcttctgtc ttctcaaaaa agaacatttt tcaaatcaaa aagaacaaat caaacctatg 5340
   aggagatggt atgagaatat tttcatagta aagaaaatgg aaagaatttg tggaggtgat 5400
   tgggaaatct tttggatgat ctctaggaat tatgaaaaat aaccgagcca cagagatggg 5460
   aaatacgtaa ttctaatttt caaaaagaaa aagaaggtgg gttctatgac ctatagatca 5520
   gtaagcttga gattgattct tggtgagagt gttgaagaga atgctagaag gtggtttctg 5580
   agctcttaga aaccagcagg cagtgattat agtcagcccc atgtcactaa aagcaaattg 5640
   tgttagcctg attccatctc cttttctgat aggattactc cactggcaaa cttagagata 5700
   caagatggat atggtccctc tacacttcag aaaggaaact gacatagttg cttgggtgcc 5760
   ctggggataa ggtacagagg cagagtatca ctcgcagctt ttaggtcaca aagaacagaa 5820
   acaaattcta gtgagtttaa acagaaaagg gattttattc aaggatatag ggaagtttac 5880
   cagctgtcca gaagggccag acaatcaggc tcagatatcc tacatctgat gtgatttatg 5940
   gatttttgga tgcagagccg catccacaat cacagccctg gtctttggag ggcatgacag 6000
   cttgtcaaca tcggcatgaa caccaaggtt tgccctgctg acacggctaa cactaaccct 6060
   ggtgtcctga aaagggtata aagctgtccc catcctctgc caaaacagag tccttaaagt 6120
   cccaacgttt ttgcctctcc agctcctgag tcaaagttca gcccaatgcc tctgtttggt 6180
   ggagcccaga tcatatgccc acaccttagc tatggaggga gctgggaaag ccagatgctg 6240
   acctgtttca cttctcatgg gaggaggtgg tctctgcctt ctgacagact cgcaaagtag 6300
   ggaattctcc cacgcagaaa gtggggtcag atgatgagct gccgtaaaga atggctaatg 6360
   ttgtcctcac aggggttgag ggatagggta attgtgctgg tttttaacgg gttgaaggat 6420
   catagccaaa gcttgctaat taattgattc tttttaatcc agaggactgg ttttattgga 6480
   ataccacaga actctgcatt atcgtgccac atgagggttt tttgttgttg ttgtgttttt 6540
   ttgtttttgt ttttgtttta tcagtggctt ggccaaagac caaaaataaa cttgttgtat 6600
   ttgcaggtga cacagagaat gagtggaata ctaatacatt gaagggcaaa atcaaaaccc 6660
   cagaagatct ctaaaagtct gaatgaatca gcccatatta caagataaag tccataatgg 6720
   taaacggaag gctctggtct tggtttacaa aatccaacca ctcaaatatg tagttgctgg 6780
   taagaaaaaa agccataatt tcatgtggtt gccaagaaag ctcattaaat ccttggctgc 6840
   attaatagat gtatggtatt tccaacaaag ccttctgcct cattctgcac tggagagacc 6900
   acagctgagc cctgtgccaa gctatgtgca ccacagtttt caaagggctc gacgagttgg 6960
   aggctgttca gaggggagca gcggggtggt agacactctc agccccttcg tagaggaaga 7020
   atgataccac cgcaggagct cggccatgaa gaagggccat ggggggagtg atgacatagt 7080
   ggtcaccaat tttctacaga gctttcacat ggaagatggg tacgatttat cccacggggt 7140
   gtcaaggcat gaaattaggt acaattgatg taaattatag ggaatcttca gtctaagaaa 7200
   gggtttccac tggagcaacc caaaagtgga tgagtctatg ttgggaggga gcaagttacc 7260
   tccctgtcac tgaagacgtt cagattcgag actggatggc cagcatccag gggtgctgtc 7320
   aaggggcctc aagctatgtt tagtctaaac gaactttcag ccttaagagt atattttttt 7380
   taatgaaaac ataaggcatt acaaaggaga gatgagccag gtgtggtggc atatgcctat 7440
   ggtctcagct actcgggagg tgaggtggga ggatcttttg agcctagaag atagaggttg 7500
   cagtgagcca tgattgcacc actgcctggg tgacagagtg agaccctgtc tctaatgtta 7560
   aaagaaaaac cacagagacg tttaacttaa cagagtttaa ttgagcaaaa aattatttgt 7620
   gaatagggca gcccctggac cagaatgggt tcagagaagc tccagcttag gcacatggtg 7680
   gaggaagatt tacggacaga aaaaggaaag ggatgtacag gaaacagaag cgaggcacag 7740
   aaagagccag gctggttact gctggatgat tgccttgttt gttcatggtt tgaagaactg 7800
   cccgcctttg gccggaactg ggtgattgac acaagagtag gcagccgtgt gtttacacac 7860
   cctgtcaggt gacagtgcac tatgcatgag cacagcttta ggcagaatgt aaactataag 7920
   gaggcagcat tgggctaaac ttaattttac actaaataaa gtctaaaaaa tttaaaagca 7980
   tttttaaagg taatagaagt tagctgtgat gtgcatgtga acatttaaac tacatttttc 8040
   gttttttttt taaaaaggaa gttttgtggc catggggatg ctgacaaaga ttgcttggga 8100
   tgcacacaag gcagcggcga agcctgaggg gtgctgccag gccgggctct ccactggcca 8160
   cggtaagaat cttcctgggc ctgtttgttt tggcctctgt gagcatgggc cacaatcacg 8220
   gggaaatgaa ttgctaggaa gtcaattcct ctctgaagga aggactccaa agagagctga 8280
   ggctgccgtg ttttctggcc gcccacacaa gccaacaagc tattccctgg gggcaaccac 8340
   taatctctaa ccagacaacc ggtaatctct tattcattct ccgttgaatt cttgggctac 8400
   actcaattta taatagaact taaagaatgt cctcatttta tctagagggt ttgtaatgcc 8460
   tttatttcga ttcctcatga atcattatag tcatggatac ctgtgaacca cagcttcctt 8520
   gtagagaggg gagaggggaa taagtgaaat tactccggag aaaacaaaca aggacgttta 8580
   gctccagggc atgtggagag ccacagtctg agtctctcac tcgcatacca ggagataaat 8640
   aattcagagg cctaacaaag ggaggcggag tagtcaaact ggttgaacac ctggtcggct 8700
   gcacccagct aaacagggct ggtgtctgtg agggcttcct aatcattgtg gatgacattt 8760
   aaagacaaca ctcatagcaa atattgcatt agaattttct gcttccaaga taccagtcag 8820
   agtaagggtg ggagagaaat gagggaaaga acagataccc tactacttat cagattgcct 8880
   tagagtacac atcatgaaag taattatgat tttctttttg agatggagtc ttgctctgtc 8940
   tcccaggctg gagtgcaatg gcatgatctc agctcactgc aacctccatc tcccaggttc 9000
   aagtgattct cctgcctcag cctcctgagt agctgggact acaggcaccc gccaccacgc 9060
   ttgcctaatt tttgtatttt tagtagagaa ggggtttcat cacgttggcc aggctagtct 9120
   caaactcctg acctcaggtg atccacccac ctcagcctcc caaagtgttg ggattaaagg 9180
   tgtgagccac cacacctggc ctaattatga tttttaagat gacaacaggg ctatcaatgt 9240
   tcatgtcttt gtgtcctcta taagagatca atgtatctct tgcctgaata cttccccaga 9300
   ttagatttct ggaagctcaa agtagtgctg attgctgtct ggtcctggtc ttgcaatcac 9360
   accccaccaa atgtaggcaa attgtcagtg tacaaggctg cctctgtggt gggaacattt 9420
   tgggaacata aaagggaggc agggatttct cttttttttc gagacagagt ctcactttgt 9480
   cacccaggcc ggagtgcagt ggcatgatct cagctcactg caacctccac ctcccgggtt 9540
   cgagtgattc tcctgcctta gcctcctgag tagctgggac tataggcgcc caccaccacg 9600
   cccagctaat ttttgtattt ttaatagaga cgggggtttc accatgttgg ccaggatggt 9660
   cttgatctct tgacctcgtg atctgcccac tttggcctcc caaagtgttg ggattacagg 9720
   catgagccac cgcacccggc ctgagggcag gtatttctaa tgtgtagtgg gtttcaagtt 9780
   tacagtgaca tctcagagct gcaaaaatga aacattattt ctctataact gtactcaatt 9840
   tacttatgaa gtagtggaaa actcttcatc ttggcacttt aggataactg acacccttct 9900
   ggtttcaaag cttctttagg gtaattcctc agtcaatcca ggattttaat tccatcaggg 9960
   ccacactccc tcatctcctc ctctggatga gccccaggtc tgtcccatgc ttcctgttct 10020
   ccggcccccc ttggccttgg agacatcttg ttgccttggt ggtgcgtgtc agtcttggtc 10080
   actgttgcct tcaccattga cccagtttgt cagctgctct taggcaacaa aaggagaggt 10140
   ggctgttccc tgccggccag gccccgtcac tgcagtggca gtgctgctct ggccaaggct 10200
   tcattctctg ccaccttcct caccctcacc aggcgctacg ggctcttccc ctgatttccc 10260
   cacacaggaa ggaagtaggg gagactcagg tggggcggtt cttggaggcc cattttcagt 10320
   ccaggtctgc cctgcctgcc tttccgctct gcttctctcc cctctcatgt tcttgtggtg 10380
   ctggaagcaa gggttttaga gcaaacaagg gccacgtccc ttctgccctt acccctgtac 10440
   tctctgtccc ctgccagtga aatgtcggct aaggaggcag agccaccgcc cccttgggaa 10500
   caagcacacc tctgtctgta tctcttacaa ctcttctctc ctcccccatt tctgctcccc 10560
   agttccctgg gtgctgagtc aggaaagcct ttatttcccc cccctccccc agtaagcttt 10620
   tgcagtgcca ggaaagctat ttttttaatg cttattaaaa aaattttttt ttcgagacag 10680
   agtctcattc tgtcgcacag gctggagtgc agtgacgcaa tctcggctca ctgcaacctc 10740
   catctcccag gttcacgcca ttctcctgcc tcagccttct gagtagctgg gactacaggc 10800
   gcccgccacc acgcccggct aatttttttt tgtattttta gtagagacgg ggttttacca 10860
   tgttagccag gatggtttcg atctcctgac ctcatgatcc tcccacctcg gcctcccaaa 10920
   gtgctgggat tacaggcgtg agccaccgtg cccggccccg gctaattttt ttgtattttt 10980
   cgtagagacg gggtttcact gtgttagaca ggatggtctc aatctcctga cctcgtgatc 11040
   cgcctacctt ggcctcccaa agtgctggga ttacaggcat gagccactgc gcccggccaa 11100
   aaaatttttt ttgtttaagg aatagggtct tgctatgctc aggctggcct cgatctcctg 11160
   ggctcaagca atcctcctgc ctcagcctcc taagtagctg gcaatacaga tgtgtgccac 11220
   catgcctggg cagcaatttg tattgctttc ttttatggtg gagaaatgac cttgtgccat 11280
   gagctgcatc ctttagagca gggtgtccaa tcttttggct ttcctgggcc acattggaag 11340
   aagaatcgtc ttgggccaca tataaaatat gctaacacta atgatagcta atgagctaaa 11400
   aaaaaaaaaa attcaaaaag tctcataatg ttttaagaaa gtttatgaat ttgtgttggg 11460
   ctgcattcaa agccatcctg gccacagatt ggacaagttg ctttatcgta acacctataa 11520
   atgctatgct ctgtccccca agcttttact atcatcttgc ttatttatgc ctagtgttcc 11580
   attattggaa cggtaagcat gtgggagtta tttatatcct actgctcaag gcatttccaa 11640
   ggtctgattt ttcaaattaa aaaaattgcc acctcagcca taaatgggtt aaaactaagc 11700
   cattttgaaa ttcagaacaa aactgcctct ctctcacatg actagctctt tcaagcgact 11760
   gtggcttcag atattgtgaa ttatccatcc gctcagcacc tcctggctgc tttgttctcg 11820
   ctgttttcac tttctcctga agtaatgaat gccattgagc caatgggggc agagtcaata 11880
   actttgaaga aaaagaacct cagtttaaaa tttcaaaata ttatttttaa tttttgataa 11940
   cttatatgcc ttcactccaa ataaatgaag atcaaaatat gtgaataaaa ttcaagatga 12000
   caggccgggt gtgatggctc acatctgtaa tcccagcact ttgggaggct gaggcaggca 12060
   gatcatgagg tcaggagttc gagactagcc tggccaacat agtaaaaccc catttctact 12120
   aaaaatacaa aaattagcca gagtgatggc acatgcctgt agtcccagct actcaggagg 12180
   ctgaggtggg agaatcgctt gaactcggga ggcagaggtt gcagtgagcc gagaccatgc 12240
   cattgcactc cagcctgggt gacaaaaaaa aaaaaatcaa gatgacatca ctctagtttg 12300
   tcccaatcct ggtgtagctt gagtaaggaa aagcagatgc cctcgttata ggtctaagtc 12360
   tcgcaccaag gttgctcatg tttctgtttt taaaccttcc acaaagaaaa tcacatgtag 12420
   gtcacactgt cctcactaat atctggtgga gcctcatatc atctggattc ctgttttcac 12480
   ctataagaac tgatagtttt gtttgtttgt ttttgttttt gagacggggt cttgctctgt 12540
   tgcctaggca ggagaagtgc agtggtgtga tcatagttta ctgcagcctt gacctcctgg 12600
   gctcaggcga acctcccacc tcagcatctt gagtagctgg gactacaggc atgcgccatc 12660
   atgcccagct aattttttgt attttcagta gagacagggt tttgccatgt tgcccagcct 12720
   ggtcttgagc cccagggctc aagggatcca cccacctcag cctcctaaag tgcggggatt 12780
   acaggcatga gccacctcat ctggcagaac tacttttttt ttttttttga gatgaagttt 12840
   tgctcttgtc gcccaggctg gagtgcggtg gcacgatctc agttcattgc aacctccgcc 12900
   tcccaggttc aagtgattct cctgtctcag cctctcgagt aactaggatt acaggtgtgc 12960
   accaccacgc gcctggctaa tttttgtatt tttagtagag acggggtttt tgccattttg 13020
   gccaggctgc tctcaaactc ctgacctcag atgatctgca agcctcggcc tcccaaagtg 13080
   ctgggattac cgtgagccac tgtgcctggc ccagtggaaa tgatttttaa tgaggcagag 13140
   ggagcagcgt ttgattcaca cacatttagt gtatactgtt ttcaacagtt taccacatgc 13200
   tgcagggggc agaggtggat ccaggttttg ggaggcctga agcttacaga atctgggcgc 13260
   cctttttaag tagaagaata caaagttatg tagataaata gttatcacat aatgagcaaa 13320
   gaatgtacag caaatcacat tatcaaaagc taacaaatca cacaaaatca taaaactcca 13380
   aaaagtacat aatattttat taaccacttg ctatactttt tttttttttt tttttttttg 13440
   agacagagtc tcactctgtt gcccaggctg gagtgcagtg gctcgatctc ggctcactgc 13500
   aacctctgcc tcccaggttc aagagatact ccagcctcag cctcctgtag ctgggattac 13560
   aggcatgagc cactgcgcct ggcccacttg ctatacttct atcgtactcc cctacccctt 13620
   gtattttttg gctgtgtatt cttaggtgac cttttgagag aaaacaattt tataatttca 13680
   ttttctctaa agagaataga atcattcagt ttttcttcta gcatggtgga ttgcaatgta 13740
   cttatacata gtttgaaagt tttgtttggt aatgtcattt aaaactttct tttttttttc 13800
   tttttttttt tgagaccgag tctcactcta ttgcccaggc tggagtgcag tggtatgatc 13860
   tcggctcact gcaccctccg cctcctgggt tccagtgatt ctcctgcgtt agcctcccaa 13920
   gtagctggga taatatatgt gtgccacaac acccggctac tttttgtact tttagtagag 13980
   ataggcgttt caccatgttg gccaagctgg tctcgaactc ctgagctcag gtgattgccc 14040
   acctcagcct cccaaagtgc tgggattaca ggtgtgaacc actgcgcctg gcctgtcatt 14100
   taaaattcta tccaaggttg ttgttaaatt tggtataacc tctatcaagt tctcttgata 14160
   tacatacaag ctataaaatt ggaaagaata ttcccccaga ctggcttctg gctccaaact 14220
   gtgttttatg ttcatgagac acatacttgt ggtgccagaa gccacagcac acatttatat 14280
   catgatgcaa attttggcct gcaccttcat gtcctgatgc ccagtgagtt ggcacaggga 14340
   gagggcgttt tcctggaagc cattcctaca tcaggatgag tagcaataac ttcaactatg 14400
   cacagacaca tctgcaaatc agatgactgt gttttaccaa gtccaactaa atgtacccct 14460
   aactcaactt gccgttagcc aattccccaa aatgctcaca gccacttcta ggacccctaa 14520
   cacaagtggc gttggagcag aatgcaaatg atcttaatcc cctaaaattg cagtttctta 14580
   cttatagaaa ttttatatat aaacacacac acacacggct aagggtcctt gtagggcctt 14640
   ggaaggagcc cgtgcaaatg agggattctg aagcttcaac cccattagct tcactgcaca 14700
   gccatagctg atggggtggg aggatggatg ctgagcaaag ccagaaatca ggttcttgct 14760
   gagaagacca acacaaaaga ccacgatgcc acagtccgca gtccagtgac ttgctgactg 14820
   tgtcactctg aaggagagat acgggtggga taaataaagc cctgagtggc agctcagaag 14880
   gagccagggg ggattttctg caggccccgg tacccacagt tacatcgccc tcttggtaca 14940
   gcctcatcat gatgagtttc aagagagaat tcaacaacgg ctctcctccc ttaactgttt 15000
   ggatgctaaa gtgttgtgat tttgttattt actgtttcta attcagatgg ctgatcaaac 15060
   tcggtgtttt ctgatgcatc tgtgaacaaa gattacactt agataatgtc taaaatgcaa 15120
   tgccagctga gtgcagtgac tcacgcctgt aatctcagca cttagggagg ccaaggcagg 15180
   aggattgctt gagccaagga atttgagacc agcctgagcc atattgtgag gccccatctc 15240
   cacaaaaaaa tttaaaaatt agcctgcagt cccagctatt tgggaggcag aggtgggagg 15300
   atcctttgag cccaggaggt caagactgca gtgagccatg atcatgccac tgcactccag 15360
   cctgggtgat agagtgagat cctgtctcaa aaaaataata aaataataaa acaaaataaa 15420
   ataaaataaa atgcagtgac acttctaccc tcatatgctg gagtttgatg ggggagcaca 15480
   ttgatttaga tgccgcaaaa gcagcattta gctgtggaat tacagtcgtt acccatttgg 15540
   gtatatttta ctctgcctgg aagagttcat gtgtttcacg gttgctacta ggtaaggacg 15600
   caggccctgg gagatcctgg ttcaacagtg actgtcttat gtaacagagt cattctcttt 15660
   atgaccacct taggaaccgc taaggaacat aataagaaat acacagaact gattgtggaa 15720
   ctgctctttc tccctgcgat tcagagggga aaagataaag ccacacagcc ctggggcctc 15780
   ttgcttaaga acacatctca gtttaaccac caaggccgtt atgataacaa atgtcagtat 15840
   ggaggtgtga acatcagggg ctattcaagg acgcttctct gatcactgcc atgtctatct 15900
   gctgtgatca cgactatttt ataaacaccc ttcctaccca atgccatcag aattttaggg 15960
   acgatgcttc agtaattatc tttttttccc tccttcctcc ctattttctt tttcctctga 16020
   atagctgcca aagtaactag agtcatataa ttttagagct ggaagctcag ggagaggctt 16080
   ggatcctgat gacccaagca tgggaggggc ctgaaaaaat gtcaacagcg ctccgttgtt 16140
   gatcccacac atttacttaa tatgagctgg tgctggcaga ggtttatgtt ggaaaggctg 16200
   caactttctg tgccccacat aaatgagata aaacctgggg aaaggagtgt tggtttaaat 16260
   cagaaaggct ctggttttta tttgatacta aacaaatcat gcagtttaca agtctgtgac 16320
   actctaagag ttaactagca gttttgtttt ttttcttttt tttgagacgg agtttcactc 16380
   ttgttgccta gtctagagtg caatggcgca atctcggctc accgcaacct ctgcctccca 16440
   ggttcaagca attctcctac ctcagcctcc cgactagctg ggattacagg catgcgccac 16500
   catgcgcagc tgatttttgt atttttaata gagacagggt ttctccatat tggtcaggct 16560
   ggtctcgaac tcccgacctc aggtgatccg cctgcctcgg cctcccaaag tgctgggatt 16620
   acaggcgtga gccactgtgc ccggcctaat cagcagtttt taacatgcta cacagggtct 16680
   gggaaagaca gctgccaagt tgggggcttt gaaaatatga gacatctgta aataataacc 16740
   tttgggtatt tcacctgtgt ttgcttcttt atattacttt tgtgtgtgtg tgtgtgtgtt 16800
   ttgagacaga gtgtcttgtc acccaggctg gggtgcagtg gcgtgatctc ggctcactgc 16860
   aacctccacc tcctgggttc aagcaattct cctgcctcag cctccctcag gcacttgcca 16920
   caatgcatgg ctaatttttg tatttttagt agagacggag ttttgccatg ttggccaggc 16980
   tggtcttgaa ctcctgacct caggtgatcc acccgcctcg gcttcccaaa gtgctgggat 17040
   tataggcgtg agccaccatg cctggcatat attactttgt tctaaaatta atttagatct 17100
   ggcatcctct ttttgtccct caaatgttat tttaccaaaa catatattat tgggacaaga 17160
   ggcaaattgc atggccaagt tgaatgacgt tattccccac tctgacagct tttgttttta 17220
   actctagaat ttgtacatat ggctttggaa acctcatcca acactaagta atgaacatac 17280
   atgaggatac tatgtatttt tcaacctgca agcccaagat ttcaaattct ttggtttatc 17340
   gcagagaaca catctctcaa attgtcttca aggagcactg caggaggtac aatccattcc 17400
   ccactaaccc aggaagactg tctgaatttt ttcagaggga agatgggtgg gggagaagtg 17460
   gggatggtta atgggtacaa aaaaaaaaaa tagaaagaat gaataagacc taatatttga 17520
   tagcacaaca gggtgactat agtcaataat aatttaactg cacttttaaa aataatgaaa 17580
   agagtataac tggattgttt ataacataaa aaataaatac ttaaggggac agatacctcg 17640
   ttttccatga ggtgattatt atgcattgca tacctgtatc cagagtatct catgcaaccc 17700
   ataaatatat acacctacta tgtacccaca aaaattaaac attaaaaagt taatcttttt 17760
   aatgtttaat tttttttttt gagacaggcg tggtcttggc tcactgcaac ctctgcctcc 17820
   tgggttcaag caatcctagt gcctcagcct ccggaatagc tgggattaca ggcctgtggc 17880
   accacgccca gctaatttct gtatttttag tggagacagg gttttgctat gttggccagg 17940
   ctgaactcct gacctcaggt gatctgcctg ccttggcctc ccaaagtgct gagattacag 18000
   gcatgaggca ctgtgcctgg acagaaagtt tttaaaaaaa atttgttcag agggaaccca 18060
   tttcctcttc tgctacacag acagccaggc ctgacttgca ctgtttctat ccccccacaa 18120
   aaaaagcaac aacatcaccg agaagatagt tctgtttgtc ttgatgatca acatagatct 18180
   gtatagttga ttttgtgcca gcaacaggct ccagtcacaa caacatgaga ttccaggcct 18240
   ttttgcctgc catgctggac acagactggg ccatagagaa taggagaacc cgtgtgttct 18300
   tctgagttgt ctgtgaagaa aaatacacgg ggagaggggt agacgtgagt tcctggaaga 18360
   gtggagatgt gtggggaatt ctccagcgtt ccttaattag aaacacgact cagtgatgtt 18420
   ctttggagcc caagacccca ggaggcactg agtccagtgg tcccaggaag ctaaaaatga 18480
   gggcaagagt cagcgtggct ttaaaaatct ctgatcactc atcccccaga atgaaatctg 18540
   ggactcaaaa gctctgatgg aaatgaagca gactttagga ggtctggttt cgggggtgct 18600
   ccaggagctg aatgtgaaat gttatttgtt ccagaaagtc ctggcaactt ggcgcagcga 18660
   atttgatcag caccatacgt acgacctgtc tcaggaacgg tgccatgtgt gtgactagga 18720
   ctgcaaagca gcttgtgttc gtgggggaaa tgacaggcac aggaaggaaa agctgagaac 18780
   actgaaacgg ttcctaatcg tgcagtgggg aaaggagggc gactgccgcg cagagtggca 18840
   cgttggctgg aagaggggat tgatcagcag tgctggcagg aacagcatag gcttgtcgtg 18900
   tgatctaaca gaagaggcag caatctggag gcgccccaga tcagagaaga gacatcaagc 18960
   cccaggagaa atggatttcc tttctcccca cccatcagct gggtaggaag cccgaaggag 19020
   cctttcatgg aactatggga agggctcctc tgacgcagag gggatgaact ggaagcaaga 19080
   tgctgactgt ttccaaaggc ttgacagtca gaagcaggca ggcttcagcc tattgagata 19140
   ctttacccaa gaggatttcc gcttaggttc tgtcttgaaa gaaatattcg ctcccaaatt 19200
   tcctcccatc aagagtgctt ctccctacag aaggctaagg ctcacaggtt gtccccaggg 19260
   acacatttct ttcaagtagg atgactggta tgtttagact gaaaagctga tttactggaa 19320
   gattgctatc accccctggc ctaccatgtc gttggtgtgg ccagtgttta tcagacagtg 19380
   gtgtttccag cttaggcctg aatggaagaa gaaactgaaa gaagaaataa aaagagagat 19440
   gcgccgggca tggtggctca tgcctgtaat cccagtgctt tgggaggccg aggtggcaga 19500
   ccacttgagg tcaggagttc aagaccagcc tggccaacat ggtgaaaccc cgtccctact 19560
   aaaaatacaa aaaattagct gggtgtggtg gcgtgtgcct gtggtcccag ctactccgga 19620
   ggctgaggca ggagaattgc ttgaacccgg gaggcggagg ttgcagtgag ccaagattgt 19680
   accactgcac tccagcctgg gcaacagagt gatactctct caaacaagac aaaacaaaca 19740
   aaaaagagga ggctcggcac ggtggctcac gcctgtaatc ccaccacttt ggtaggctga 19800
   ggctggcata tcacttgagg ttgggagttc gagaccagcc tgaccaatat ggagaaaccc 19860
   catctctact aaaaatgcaa aatcagccag gtgtggtggc gcatgcctgt aatcccagct 19920
   actcagaagg ttgaggcagg agaatcgctt gaacccggga ggcagaggtt gcggtgagcc 19980
   aagatcacgc cattttactc cagcctaggc aacaagagca aaactccatc tcaaaaaaaa 20040
   aaaaaaaaaa atggagagag agatgtttct cagctttggg aaatgctgac ttttccagca 20100
   cacctttgct cagaaaagcc ctatgatata aagtgtcata ggaaccttgt ccttggaacc 20160
   aagcctagcc taggctcagg gacatcatgt gggagtgaca taaaggaggc tcctctgtca 20220
   ctgtgcttgg gttacaagta attctcgaca tgggagcatg ttgatttcta accatcggtg 20280
   gcggagctgt gacttcccaa gctggtggct gacctcaaga ggactattat agacactgtg 20340
   ggaccacggc ctgagaaaag aatggctacc tccctctcta tcccatgacc tctaacggcc 20400
   cggtcaccac catggccttt tgttcctgga gagggacagg aactttggaa agtggggtcg 20460
   ctctattgct tctgcaggtc atcttcccag acttgcctaa gcctcagaat ctcagtcttc 20520
   tgctctgttt tgcacgtctt ttttgaaagg tttttaaaaa ttacaatcta ctgtacagaa 20580
   gaaaataata cacagaaaat aattttagaa aataatacaa cagatatcta agtatccacc 20640
   acccagattg gacaacatat taacattttg ccccatctat atcagatatt atttattttc 20700
   ttttttactt atttttgaga cagaatctcg ctctgtcgcc caggctggag cgcagtggtg 20760
   tgatctcagc tcactgcaac ctctgcctcc caggttcaag cgattctcct gcctcagcct 20820
   cccgagtagc tgggactagt ggtgtgcacc accatgccca gctaactttt gtatttttag 20880
   tagagacagg gtttcaccat gttggccagg atggtctcga tctcttgatc tcgtgatccg 20940
   cccgcctcag ccttctacag tgctgggatt acaggcatga gccacctcgc ccagcctatt 21000
   tttatttttt ttaagacatg gtcttgctct gtcacccagg ctggagtgca gtggcacgat 21060
   cttggttcac tgtggccttg accttccagg tgcaagcaat cctcccatct cagcctcctg 21120
   tgtagctggg actacaggag catactgcca cacttggctg attaaaacat tttttctttc 21180
   tgcagagaca gggtcttacc atgttgccta ggctggtctt gaactcctgg gctcaagtga 21240
   ttcccctgcc tccggctccc aaagtaatgg gattacaggc atgagccagc atgcccagcc 21300
   tatatcagat ttttttaaaa ttgcagatag aattaaaaac ttgcttatct atttttagta 21360
   gagggaaaaa cctaaataag tttaattcta gtaagttgct gactctacac aacaacttcc 21420
   tttcagtctt cagataatgg aaactttgtg gatttatgca cagcacccaa accccagaga 21480
   tctgccccaa cagaaagatt gccaacaaaa tgtttgtctt aatacttgaa caaatttttc 21540
   tcaaacttac attgtatatt tttctccacc agtttgctaa atgtccacaa ggttaatacc 21600
   cgcccagcca aatgaaaaga ccactgggat caggtgcggt ggctcacacc tgttatctgt 21660
   tccagctact tggggaggct gaggtgggag gatcgctgta gcctgggaaa tcgaggccgc 21720
   agtgagctat gatcaagcca ctgcactcca gcctggccaa cagcgcaaga gcctgctcta 21780
   aaaaatcaaa ataaaaaaaa aaagagcatt gggtctccaa aggtaacagg tgtgaaatac 21840
   accttagcct actcagaatt ttgccccatg aaataagtaa ggattattaa atatttctgg 21900
   aaactcaaac aactctttaa acaatgttca caatttttca atgtccacat cagccaaacc 21960
   cacggaaagc taagcattaa ttcacctact gttggagagg ctagactatt taaggaatct 22020
   ctcctgaatt ctagtcctga atttccaatg caactcgtgt ccactagaat gttctgtgaa 22080
   tgtttcaagc ttgaaacgcc tgcagccaaa cttgttttct ttccatccaa ttatatcctc 22140
   tttccaatgt tccccttttt tcattaaagg caccactatt cttccagcca tcctgattca 22200
   cagcctcaga gctggcctca atttccttgt ctccttcgtc ttccacatcc aattattcac 22260
   taaatcctgt tgaatttttc tcaaaatatt tcttgcacct gtttactcca ttctgctccc 22320
   actgccacct ttctaatgaa gtattttcca cctgtgtgtt tcggctcctg cagcagattc 22380
   tgcatctccc ctttctcctt tcaatgaatt atcagtatca gattaatctg ctgaaagaag 22440
   cactgtgtgc catcctgctc aacataattc agtgattccc agatggctta ccaaataaca 22500
   gcttttaaaa aaatccagaa ccttcagtga tcttcacgat ctttatatgt ccaccttcct 22560
   agacacatcc cacattagga acccattgaa acctctgccc caggcgcatg gcactactgg 22620
   cttgctaatt ctgattcctg ttcaagtatt tctttggcct aagtattcct tcccctcctt 22680
   cactacatga accaatcttc atggtgctta atatgaacca gatcatctgg tgggcctcta 22740
   atacaacagt gaacaagaca gaaacaaccc ctgcctccat atactcctta cagcctagtg 22800
   gagaaaacaa aagatgaact ggcagttggc atgccaggct gtgcaggaag aactgtgaca 22860
   aggaaagaca aggctgaggt gggcggatca cttgaggtca ggagctcaag accagcctgg 22920
   ccaacatggt gaaaccctgt ctctacaaac aaaaaaaaaa attagctggt gtggtggtgg 22980
   gcgcctgtag tcacagctac tcagtaggct gaggtgggag aatctcttga acccaggagg 23040
   tggagattgc agtgagctga gattgtgcca ttgcactcca gcctaggtga cagaatgaga 23100
   cgctgtctca aaaaaaaaaa aaaaaaaggc caggacccca gagccaggca tggggattca 23160
   gagcaggctt ttcagaggaa gggataccca accaagtttt gatctgttct tcatgatggc 23220
   tagtgaggtg aaggagaggg agaagggtca gaaaagaact ctacagcaga gagaatcatc 23280
   ctgctcagcc cctatccaac ctttaatgcc cacctcaaat gccttccttt tttttttttt 23340
   ttttttttga gatggagtct cgctctgtca cccaggctgg agtacagtgg caccatcttg 23400
   gctcactgca acctctgcct cctgggttca agtgattctc ctgcctcagc ctcccgagta 23460
   gctgggatta caggtgccta ccaccatgcc cggctaattt ttgtattttt tagcacagac 23520
   agggtttcac catattggcc aggcttgtct tgaactcctg acctcaggtg atccggctgc 23580
   cttagcctcc caaagtattg ggattacagg catgagccac tgcgcccagc ctcaaatgcc 23640
   ttcttgacca ggccggtggt gtactgtgag aacacagact taaagtcatc cagaaacaaa 23700
   tcctaaccct gcctctgctg catgctagtt gtgtacatga ccacgcaaaa tttgctcatt 23760
   ctcaatatga gtcaatctct gaatctgtaa aaatactctt tcttttgaca gggtctccct 23820
   cggttgctca ggctggtgtg cagtggcatg atcacaactc actgcagcct cgacttcctg 23880
   ggcccaagct atccttccac ctcagcctcc tgagtagcta ggactacagg cgtgcaccac 23940
   tataaccacc taactaaaag acaatttttt tgtaagggtg gggtctcact atgttgccca 24000
   ggctggtctc aaacctctgg gctcaagcga tcttcctacc tcagcctccc aaagtgctgg 24060
   gattacaggc atgagccact gtgcccagca gaaatactct tacattacag agtttattca 24120
   ttaaataaat attaagcaca agaaccacat actcttctcc gtgataagga tacacttaag 24180
   aataggatag aaatcttggc tcttatggtg cttacattct agtaaggaaa aggtgacagt 24240
   aaataagata aatatgtaca ttatggagta tattggaggg tgatggattc tatggggaaa 24300
   atacgtcaga gaagagagag agtagggggg aattgcaact ttattcattt gtttattatt 24360
   taactcattt attcaaacaa atagtattga aggtctgcta catgccacac cctgttctag 24420
   gccctgggtg tatagcaggg aacaatggac aataaccttt gcctttatgg agcttacatt 24480
   ctgatcagaa agagagataa gcaaaataaa aatagaaaaa tatggagatg tagccgggcg 24540
   cagtggctta cgcctgtaat cccagcactt tgggaggccg aggcaggcgg atcacctgag 24600
   gtcaggagtt tgagaccagc ctggccaaca tggtgaaacc ccatctctac taaaaataca 24660
   aaaattagtc aggtgtggtg gcacatgcgt ataatcccag ctactcggga ggctgaggca 24720
   ggagaatcac ttgaacccag caggtggaga ttgcagtggg ctgagattgt gccattgcac 24780
   tccagcctgg gcaacagagc gagactccat ctcaaaaaaa aaaaaaaaag ccaggtgtgg 24840
   tggctcacat ctgtaatccc agcactttgg gaggctgagg tgggtggatc acgaggtcag 24900
   gagttcaaga tcagcctggt caacgtggtg aaccctgtct ctactaaaaa tacaaaaatt 24960
   agctggacgt ggtggcacgc acctgtagtt ccagctactc aggaggccga ggcaggagaa 25020
   ttgtttgaac ctgggaggcg gaggttgcag tgagccaaga aggcaccatt gcactccatc 25080
   ctgggtgaca agggtgaaac tccgtctcaa aaaaaaaaaa aagaaaagaa aaaaacatat 25140
   ggagatgtta gtgcaaaata gaaagatgga taggaaaaat agtagatgag ttccaacatt 25200
   caacagggag gccagctaac ccgactgaga aagcagtagt tatgaaaagg tgtggcggga 25260
   tctaggccag ccgtgtgaac aaatggagga agagaattcc aggaacagca agtgcaaaga 25320
   tgccacattg ggactgcatg gtgtgggcag gaggagcaag tgaggggaga gcagaaagag 25380
   gcaaacaggg gagagcaagg caggtcaggg ccctgtgggt tattgtaagg actttggctt 25440
   ttactcggag tgacaaggaa acctagtttg gagcagagga gttacccgat cagatttgta 25500
   ttttaacatg gtggcgctgg gtcaagaata gtcaagaaga gggaccaggg cagacgctgg 25560
   cagggcagct gggagaccac cgcaatcacc cagacaaggg gtaaggaggg tgtggacaag 25620
   cgtgatggca acagaagacg ggcagatggc agggttctgg atgtatctta aaagcagggc 25680
   taacaggact tgtgatcaga tacgggatgc agaagaacta aattgattcc atttttggct 25740
   tgagcaactg ggagaatgaa atagctcttt actgaggcag ggaagactct gggaggaaaa 25800
   ggttgggggg aactatcagg agtccagctt ttgatgcgtt gagtttgctt tagggtgatc 25860
   ctgcaaagtt tcactgcaaa ggggatagtt cagtaaagac ttgaaggaaa tgaggagcag 25920
   aacttgggaa gattaaatga aagggtgttt gagagggcac ttcgcatagt gcagtgccgg 25980
   ccagctcagg ctccgttaat tccattccct catccataac aaacttcttc cgctccaggg 26040
   ggcaggggct gggaagcatc agttcattat tggaaaatcc tcgctctagt gggtaggctg 26100
   caccttgtgc attgtagttg ccaaactcta tttgctgaat tcacgagaga aaggaataag 26160
   ctataatcag taaaggaaac tgttccaacc agcttccctt tctccttccc ttccaaagaa 26220
   gaggtttgag ccaagatgct cttgtgtccg ggtgagggga attagaggtc tgaatcctct 26280
   gtcttatggt gaacacagcg tgggggttct ctgagtgtag aaccacctgc agcccaatga 26340
   gcagggatag tggtaaaata tgaattcttg gaccctttcc cagaccttct cattttgagt 26400
   tgttagggga gaaacgggta tctttctgtt acagacacat gcagtgcttc cttggaaact 26460
   aaagtttaag aactactgac actagggaag atgacccttc attgacattg ctcctcggag 26520
   gcagatcatt tccagccatg atgtggtaaa ttacagccgc tctgcaattt tatgaataat 26580
   gtgtgctcat cacctaatca aacctctcca gtggaggtag aacctggctg gctcagatcc 26640
   ccactcacat tgtcaggtga ttgctttagc aaaaggcctt ctgccaaata cctcttggtt 26700
   gaactttgca gcttgaggag agaagtatgc acgaacacat aaatgccaaa tctcttatta 26760
   ttctgagttt tcaaacaatg aatacgattc caccccacat tcttgagaaa gggcagcaca 26820
   cctatctcac cctcagctgt gatgaagcaa gaaactcagt ttcttcacat tgctcggtga 26880
   gtctatacca caaacaggac taaaagtcac atgttggctt tgggcaccat tttatcgtga 26940
   gcctttcata gcccatcttc caaggctatt aaaatatcag ctccatgagg gcaacattcg 27000
   tgttgtttat tgctctcttc ccagtgccta gaagagtgtg tggcacacag tagtcattca 27060
   gtaaacggca gaatgaatcc attccacttc ctaaagtgaa gagccaaatc gacacacatt 27120
   cccatctgtg gagtatcaca gtttcctgca gtttacttcc acatacctca ctccaccagt 27180
   taactacccc ctatataaat ctgtgcagtt ttcacttaag atataacttt ctggtgaccc 27240
   cataaaaaat tggcagaaat tctcagaaag gatgataatg taattggtct tgtggggaat 27300
   ccatggaggg atatatttca cattattcat gtttccagat gggcttcagt ccccagtgca 27360
   gtcattccag aaccctttct cagcgttaag tttactccga aaaatataaa tagtgattta 27420
   gggcttgatt gctcccacaa aaggcacccc tgttttggtg cagggtggga aggggggcag 27480
   aggaagaggc agctgggaag ctgagtgagg ggccaggcgc ccgctgggtg tgagccagca 27540
   gctggtcaga gtatctctgg aggatgaggg tttcgcaggt gggggctggg gactagctag 27600
   agatgggatt gtccgtggtc aggcagttca ggaagctttc agcagcaata aagcttggat 27660
   gtgggcatgc caagcttgga ggagatatgg gtccattccc atccttctga aaaaacagtc 27720
   ttcaaacacc acggctagtg tccaaggcta cttagctcag ctggttgggg tgctgggcta 27780
   ctaaaatttc actgtagcat ttcctgaatt cttactaggc tcatatatta acaccttcaa 27840
   caaatatttg tgtgtcagcc tctatgctga gggctgggga tacagctatg aacaaggctg 27900
   tgaactaagt actgtttctt taccagtgaa ttcttccatt tcatgggcct ctgattctgg 27960
   ccctagtcag atatttctca aatgcctgac gttgtttcca aaggggtaca ttcggagtag 28020
   ctagatcaca gcaaacgctt caagagttct caacaatagg tcagtggaac cttgatattc 28080
   taaggacata atgatctaat ttagactgaa aatcatctag ccaaacacac actaggcatt 28140
   taattcatgt aaggctcaat cagaatgtca gtatgaacca gataggacag agtgttcatt 28200
   tgcagagaac aatatcccgg cttgcctggg cggctgcaaa gcctttaaac tgaaatcctt 28260
   gcttccactc ttgctctgca cagcagccaa agaattttat caaccctcca ccggcttcct 28320
   gttgcaattc gaataaaatt ccatctcctt accaggatct acgcagccct ggagtctggc 28380
   ccctggttgt atctctggaa gctggtcctg gccttggagt tgacctgaaa ctctttcccg 28440
   cctcatcttt tctcgcctgg ctcctcatta tgcagagctt tgttcaaata caaagctctc 28500
   cttcccaggg aggtccttcc tggccagtct ttctgtagca gttctggacc ttccccacgg 28560
   catctcttta tgtccttagc gtatttctta ttcttcatag tgggaatatt atagcaattt 28620
   atagcaataa atgtttgttg agtgaattaa tatgcatcac acttactaaa cacccaaagg 28680
   ttttctgggg atgtctatga ctattaccag atggccaatg gtttgcggca ctttattttt 28740
   atattttttt acagcccacc cataggagat gacagtactt tattttattt tatttttatt 28800
   ttaagagaca gggtctcact gtgttatcca ggctagtctc gaactcctgg gctcaagcag 28860
   tcttacctca gctgcccaaa gtgtcaccag gctggagtgc agtggcacga tctcggctca 28920
   ctgcaacctc agtgaggtgt gagccaccac ttttaaaacg ggcctgacag cactttttaa 28980
   aatcatattt gaaaacctgg tacttgacac agagccctag aacctactat taacaaagtg 29040
   cttaatattg ttgttgttgt tgttaaattg attctaacta aattaacacc agaccagtga 29100
   ctggaaaaat cacatgtgac caaagaatcc cattgatggt gtcagacaat gacccaggaa 29160
   gaggagttca tagcggctgc ttgtttcctt actggaaatg cagggtgaca gcagaactca 29220
   gggaacatgg tgctacccag taaaagaaac agtgcatagg ccgggtgcgg tggctcatac 29280
   ctgtaatccc agctctttgg gagcccgagg cgggcggatc acctgaggtc aggagttcaa 29340
   gaccagcctg gccaatgtgg tgaaacccca tctctactaa aatacaaaaa ttagccgggc 29400
   atgatggcga gtgcctgtaa tcccagctat tcgggaggct ggagactcgc ttgaatccgg 29460
   gaggcggagg ctgcagtgag ctgagatcac gccactgcac tccagcctgg tgacagagcg 29520
   agactccgtc tcaaaaaaaa aagaaagaaa gaaagaaaga aacagtgcat aacaggggca 29580
   gggctgtttg caatgtgatg aaagcataat ggtgctgcag tcctgggcct gatgtcatct 29640
   tgagactgga tgttggtagg atggtgccct tctgtaatat gtttagtagc tgcacttgct 29700
   gaattgacag cctctctgtt taaaactgac aaggagaaaa ttaaagtttc ccctgctttt 29760
   catgtcatgc atgaataacc aattgcttgg cagctagtta aaattcatat ttctcttgtc 29820
   aggtttttag agactgtgaa gatcaggtta atgtaagcct tccctccttt tgccctcttg 29880
   gctaactagc tagttatatt tgcatttctt tttatataaa tctttttttt ttcctgtccc 29940
   tttgacagca cagtatcaga gatggcatat tatcttcaga tgtgccatgc tggaaagcgt 30000
   ttgctcttat ataaatatag aggcagaaag agactgtctt tcttcgcacg tatccaatcc 30060
   ttttaggtat ccaatccttt taatgactcc aagtcaaagg ttgttttttc tcaccaacaa 30120
   aaaggatttg tcaactacca ggaatctgag agttttctaa tcctactggg gtgggaagtt 30180
   ctaaacattg tgacatagac ttttctattt gtatagagtc actcacagat cctacaccat 30240
   gcaattgtat cattgcctca tggaaaagaa gacgctcttc aactttaaca tgtttttgac 30300
   acctgctaaa cacctggtaa tgctagcttt ctactcaggt ctcttctcag ctctcttctc 30360
   agctctacag attataaatc tttaatgctg acaaaggagt tatcctgctg ctgccagaaa 30420
   gaagctacac aaattttgcg atgctgtgag gcaaggcaca aatctcagaa ctgtggaaaa 30480
   cagagaatct ttcccttctt cagttgtgtc ttcttgccac aacttccata gaatcgcagc 30540
   tgagagtcac tggcagctgt agccacatct tctctccaaa tcacagagaa ataactgagc 30600
   ggggaattaa gagttgtctc tgggttgcac aaacaatgtc tcaggaacac gctggccacc 30660
   actctgtatg aggaatgaga actggagcgc aggatatgaa tttttctcac cctcaaaaaa 30720
   cccaggagag ataccctcct aaggtttata ggaaatttgt tcctgaccca agggtcagag 30780
   aatagcttac tatgaatttt aattggctcc caagtaattg ctaattcatc tctgactttg 30840
   gcaggaaaaa aaaattaaat gttaaagaaa tgaatttagc aggaagactt ttttcccttc 30900
   ttttcttttc ttttttatat gagcacggtc ctacagctgt tgaagcctaa cagagaagat 30960
   ggtaaataaa catgcagggc taggggcgtt catttgcaga actttctgct tgaagaggag 31020
   cctggaaaac tgcaggactc atttgggatt acaaatcttt tttttttttt tttttttttt 31080
   tttttttgga gacagggcct tactctgtca cccaggctgg agtgcaatgg tgctatctca 31140
   gctcactaca acctctgcct cccaagttca agtgattctc ccacctcagc ctcctgagta 31200
   gctgggatta caggcacatg ccaccatgcc cagctagttt tagtattttt agtagagatg 31260
   gggttctgcc atgttggcca ggctggtctc gaactcctga cttcaggtga tccatccgcc 31320
   tcggtatccc aaagtgctga gattacaggc gtgagccact gcgccgggcc tggattataa 31380
   atctttaatg ctgacaaagg agttatcctg ctactgccag aaagaagcta cacaaatttt 31440
   gtgatgctgt gaggcaaggc acagtgggtg cgagatgcag aagggacagg acacaggagg 31500
   gcctgtcccc accatgcaag tcacatgaaa ccaaaaccaa cctaacttgg gtttgagaaa 31560
   ctaaagtccc aggtgttgga ctcacctctt gtccttgtgg acttcagagg atccgtcttt 31620
   gtccctaccc tttcctctca tttcctccct ccccaatttt tttccctgac atttctctgc 31680
   tcaaactcac attttctctt atttgatgtc tgctacatat ataacataca acatgtttta 31740
   aaatgtatag ttgaatcatc aaacattgag cattctttct gggaatttat gctgcctggc 31800
   agataacgtc catctgttca ctcgccattc attgccagtc acctgtgccc ttgtctttct 31860
   ctgttgtgga ggctaaagag agaaattttc aacttcccca attgcccatg gctaggtgga 31920
   acccgaggag tctggttcct gcttaaatcc catgtgtggc tgggcacggt ggctcacgac 31980
   cgtaatccca gcactttagg aggccgaggc gggaggatca cctgaggtca ggagtttgag 32040
   accagcctgg ccaatatggt gaaaccctgt ctctactaaa aatacaaaaa tcagcttggg 32100
   cgtggtacgg gcgcctgtaa tcccagctac tcgggaggtt gaggcagaag gatcgcttga 32160
   acccgggagg cggagctttc agtaagccaa gatcgcgcca ctgcactcca gcctgggtga 32220
   cagaacgaga ctccgtctcc aacaaacaaa aacaaaaaca aaacaacaac aacaacaaaa 32280
   ccaaacccat gtgtgatttt tttcaggcct gaccgactgc tattccagag gcatcacccg 32340
   tcaggcctca ccggaaggct gcacagccgg tgccacctca gagagtctct ggcaagaggt 32400
   cagcctcctc tggctgctcc tgcccttttg tggatctgct ttcctagtcc aggtgctctg 32460
   ttcggagggc tcagggaccc ttaagggaag acacctttct tcactctgac tcagtacttt 32520
   tacactctca gctttttccc ctctcactac ccaactcccc agcccctggt ccacaaccct 32580
   tcaggaagcc tttggttagg ggccccctca agagtgagat gacccccaca tctgtgctga 32640
   tcctcctgac cctgaaccgt atgggtgcat tgttccaagg ggaaaatgaa gcaggagagc 32700
   tcagcactcc ctccagtgtt agcctcttgc ttgtactact gcagtgcggg attaaagtct 32760
   tcgctattac ttccatttgt gcttgttgta gcttcaagtg gctactctaa cacctgacag 32820
   ctcagctccc ttccagctct actgagctcc tgacagcgcc atgcgatgca atttaatcta 32880
   agaggaattc agccgcatgg ttataccggg gcacggtgga gaataaatga cattctgcct 32940
   ttgttctgcc taaaatacag gtgtgatgac tggagatgca gaagccattc cattatatca 33000
   acataacatg ataagccaag accctaaaga tgctgagaaa aatggtcaaa agagcttggg 33060
   gtttttttgg tttttttgtt tgtttgtttt gttttttttg aggcagtggt ctcactgtgt 33120
   tgcccaagct ggtctcaaac tcctggcctc aggcttcagc ctctcaaaac tgctaggatt 33180
   acaagtgtga gccactgcat gagactgggc tgttttttgt ttggtttggt ttttttagag 33240
   acagggtctt gctctgtcac ccaggctaaa atgtagggat gtgatcatag ctcactgcag 33300
   cctcaacctc cttggctcaa gtgatcctcc cacctcagcc tcccaagtag ctgggaatac 33360
   agacatgcac taccacaccc agctaattaa aaaaacaaag tttagtagag acggaggtct 33420
   ccctatgttg cccaggctgg tctcgaactc ctcacctcaa gtaatcctcc taccttggcc 33480
   tcccaaattg ttggaattac aagcgtgagc caccatgccc agtactgtct ggtttcttaa 33540
   taacattgtt gagccattgc aatagccttg ggatattgct ctttgtactt tttcttatgc 33600
   aagaaaaaca gattcacaag ttatttgatg actctcttcc catcaaaagg aaaagtctaa 33660
   ttctttttcc cttgattatg gatcagcttg agcttgttgc ttctaacaaa tggaagagag 33720
   caacatgctg gagtgatcac atggaggaag cacaaaggga tacatgccca aggggcccca 33780
   gctgttccag cccctagaca tttgaatcta ctcaggcacc aagtaagcag gtgaatagcc 33840
   ttcaggtgat tgcggcccac tcccagctga agccaagtgg agcgcagatg agcttcccct 33900
   gctgagccct gcccaaattg cagatttgga agcaaaatga atattgtttt aagccacaaa 33960
   tttgtgagtg ttttgttaca tagtataagg agccagaaca gctctacaga tgtaaattat 34020
   agtcagatat gttgctattt ataaccaaag cattcctaac ttataaatgt ttcaacatta 34080
   ataagcagaa gaaagtgtaa taatcttatc aaggtaatac aagttacttg tttaatataa 34140
   ccaccttcat ggcaagcctc aatttcataa cctaatcact ttgagtacaa cttggaccta 34200
   atgggtagct tttttttttt tttttttttt gagacagggt cttgctctgt cacccaggat 34260
   ggagtacagt gacacaatca tggctcagca cagtctcaaa ctcctgggct gaaatgatcc 34320
   tcctacctca gcatcctgag tagctgggac taaaggcagg ccccactatg cccagctaat 34380
   ttataatttt tttttttttc tgtagagatg gagccttgct atgttgccca ggctgatctt 34440
   gagctcctgg gctcaagcag tcctccagct caacatcccc aagtattggg attataggca 34500
   tgaaccacca ctcttggcct ctgtgtagca ttttatagaa aatataaagt tgatattata 34560
   aattttatat taacagattt ctataattaa aaatcttggc tagattgttt ttcctttact 34620
   cttttttttt gagatggagt ttcactcttg ttgcccaggc tggagtgcaa tggcaccatc 34680
   tcggctcact tcaacctctg cctcccgggt tcaagtgatt ttcttgcctc agcctcctga 34740
   gtagctggga ttacaggcat gcaccaccac gcctggctaa ttttgtattt ttagtagaga 34800
   tggggtttct ccatgttggt caggctggtc tcgaactccc aacctcaggt gatccgcccg 34860
   cctcagcctc ccaaagtgct gggattacag gcatgagcca ccatgcctgg cctcctctac 34920
   tctttcatta gtagagttaa tatccattca ttgcgcactt gctatgtgct aggtgctaag 34980
   gacacaatag tgaacaagat taatcacctg agatccttgt cctaatgaag cttaccttct 35040
   gggggggaaa acatgataaa aatgcagaca agtatgttaa tataataatt tcagatcata 35100
   atgtgaagaa attagtgtgg gatatgtggg ggagatgggg aatagatagt tttgctttag 35160
   ttaaaatgtc agggaaggca tctctaaaga agaaacatca gagatgagac ctggatgatg 35220
   aagggagcca gccatgggaa gttctggggt agaagtgctt cagactaagg gaatagccag 35280
   tgcaaagagc ctgtagccag aataaggttg gtagagtcca gaacagaaat taaaaaaaag 35340
   tggggacaga gggtggggtg ggggagatgg tactaagtga gagaagggga gcagacagga 35400
   cccatatccc ttaggtcctt gtaggccata gtaaagaatc tggaagttat ttctgtaagt 35460
   gcaatgttaa aggtactgac aggatatgct ttgcactttt aaaagatcac actatggaga 35520
   atggagtgca gaggggcaag agttgaagca aggagaccca ttagaaggct gtggcagtaa 35580
   tcaagaagag atgattacgg cttgattaca cattggcaat ggaggtggtg aggagctctg 35640
   agatctggga taaattgaga cagagcttga aagagagctg atagaaattg ccaatagagt 35700
   gctgacggat ttccacgtcg actggaaagg caaattatta atttatgtga atctcaatgg 35760
   ttttgtcatc atttgagtgt accatccggt tgtcccctga gcctacgtgt atggtatttg 35820
   gaggataaat agaaggagca caaggatgca gacactaaga gtttaaagct gaagccaggc 35880
   atgtcctgtg cattatttca agcaggtgtt gttaattaat gcccctctct ggctgtatgg 35940
   caacaagctg tagagcctcc ttctggggcc atccctctag ctctcagttc cttttgttcc 36000
   aaatcagagc actagggttt gagttactta ggatctcaca tgaatcattc ttggggaact 36060
   aggggcaggg taatgaatgc tactaattag cctgctaagt atcctctgta actagaggat 36120
   atttggttgc aactagaaag ctgaggtaat tatctcttta ttatgtacga atcttattta 36180
   catgagccac atgggcattt atgaccgatt agctcagata aatggacatc caggctaatg 36240
   acaacatcac aaattcattc cctgtagaat ccttctgttt tagggctgta aactctattc 36300
   cttgtagtat gaggtggcca gcaagtctgg aaacatagat aatactattt ttattatgta 36360
   cgatttataa cccattcatt atgcttttcc ctgtgtttca agaatttggt ggccaccctg 36420
   gagacacttc ttggggtatc tttaccaaaa ctcaacatac cagctctcaa accattctac 36480
   ctttctgaga cgcaccccat ccacagaagt gggcttccag ggactctgcc catgtgagtg 36540
   actcacctag ctggcctcag gtgattggac agagctgaac acttgtttca agcagggcca 36600
   atcagattcc ttctcccaga aggaagaaat ggagagacag gcacacggtc tctgtgaatg 36660
   agagaacaat cacctctaaa cttgttgctg tggttcagcc atattcagcc ttgtggataa 36720
   agaagcagag aaagcaggac tctagtgtca gagaatgagc tagatgtgca aaaaaagtgg 36780
   aggaaagatg aaccaagggt gcatcttggg aatgcggatg ctttttcgtt ctcaactttg 36840
   cccttcctaa ggtctggctg tactcctgcg tttggttcct caacacactt ttgtatatgt 36900
   ataccaaatt ccccctttgt gcctaagtgg agtccttttg gtttctgtca cttgccgccc 36960
   aagagtctta actaatatga ctttagatct tgtccacact ctccccaggg aattccattg 37020
   gtcataaggg tcaggaagga atacatagat aagtatgaat ccattgtcac tgaagtggaa 37080
   acaatttaag agcagtctct tctagaatga taccaatact atcatctgta aacacaaatg 37140
   ccagttgcat cccgtcctgc agagcaagga aaaatcctct ttctattcca gaattgactt 37200
   ttttctatta gaaaagagtt tccctaaggt actgacagaa gaatctgctc tttgaaaaac 37260
   aatttcaaat ccttatacgc cagcagatct gcactaaatg aaaagttgcc ctcagatatc 37320
   catggctgca atgtcctcag aaattgtttg caccgtcctt taccaaaacg aactctatca 37380
   agtataacca aatagacttg ctttcccggt cttggttcta tgtacaatgc caccaaactg 37440
   cattttattt gatgaaaaag gtgatgtttc cagtttcaaa tggttagcaa aaagtagggg 37500
   aattaaggct tcttatttta gtgtaatgtg ccttcttgtg tttagatggt aacttcataa 37560
   gggcgagcaa agagtatccc cccaaaatgg taaaatactt taaatataac aagaaacaca 37620
   agacataagg aaagtaaaag tctaggctgg gcacagtgac tcacacctat aatcccagca 37680
   ctttgggagg ctgaggcagg aggatcactt gaagtcagaa gttggagtcc atccggggca 37740
   acatagcgag accccatctc tacaaaaata aaaaattagc tgggtgtggt cacatgcaac 37800
   ttttgtccta gctggttggg accctaaggc aggaggatct ctcgagccca ggagttcaag 37860
   tttgcagtga gctatgatcg caccactgca ctccagcctg ggcaacagag ctaaactctg 37920
   tctccaaaaa taaataaata agctatattt tttaaacaag aaaaaaatta aaaaggctaa 37980
   caacccaaat agcacaacca gaggcagtgg gtgtggggca gatcagtagg caaattcctt 38040
   gagggcaagg gccctttgcc ccagagccaa gcactttgcc tggaatagaa caggaaataa 38100
   atgtttacag agtaaaccga ttgtatgaac acatgattga aagtattgat aaaatgaaag 38160
   ccagaaaact atattaccca ttgctaatag gacacaaatt ggttgctgtc aaatttaaag 38220
   tattaaatac tcttgaattt gtccacgcaa ttgaatctga tatagcctct ggatataaaa 38280
   agtaagattc taaaagctca tcaggctccc ctagttactg cttttttttt tttttttttt 38340
   tt 38342
<210> 37
   <211> 13854
   <212> DNA
   <213> Homo sapiens
<400> 37
   agtgatccgc ccgcctccgc ctcccaaagt gctgggatta caggtgtgag ccactgcgcc 60
   tggccgctgc tcttatacta ttttgaatgt aggccggccg tagtggctca cacctgtaat 120
   ctcagcactt tgggaggccg aggcaggtgg atcacctgag gtcagaagtt tgaggctggt 180
   tttagtagag acgaaacccc gtctctacta aaaatacaaa aattagctgg gtgtggtggt 240
   gggcacctgt aatctcagct actcaggagg ccaaggcagg agaatctctt gaacctggga 300
   ggcagaggtt gcagtgagct ttgatcatgc cattgcactc cagcctgggt gacagagcga 360
   aactccatct caaaaaaaat tttttttaaa gaatatagat ataagccaca acccgaggac 420
   cccaacctcc ccactgcata tgtgaatagg gagttcacta tgaactcttt ctacgtgacc 480
   actcttgaga ccttaatggc tgctgtactt gggctgccga actccataaa ggacaagact 540
   gaaagattca cctccgacct ggtgtttgca gcctgaggcc agtcctgtca caggtcacag 600
   ggaatcactg ccacccgttt taattacagt ctctggaaga agaggccaaa ccccttctga 660
   tttgcagaaa gcctggtttc tttatcttat taacaacaac atatcccagc gctccagtga 720
   ttacagaccc attcctgaag caatcaaatt gcctatttga aagaaagact atttcctttc 780
   cctaatgaat gtgaacagtc ccccagagcc tgaaggtatg gctatgaatg ccccactgta 840
   acaatgaaaa actattataa ataaatgaca cagagctgct ctgttcttaa aaatcaccac 900
   atgatcaatt tgagagacat gcatgaagat gggactcagg agattttgtt ttaattattc 960
   tgcccgaagg aaaacaatac tttgctcttt gctggcaccg ctgtacagag aagcttaaag 1020
   agactttgaa aacatgatcc tgttcatcct tctcaaatca caggcagaca cagttgccct 1080
   cattttttga aaggggaaac tgaggtgtag acaaatgagt gttttctgtg tttagcaggc 1140
   tatttaaaga ctcatatatc atcatatcca acatgttttt atgtctctcc cctactcagc 1200
   tgttctgatc attgtatttg aaaactcacc aaaaagggga gaaattaaca aaaggctata 1260
   gagaaggtga gcttttactt ttataagctg ccaacttctt aaaataattt taaaatgagg 1320
   tgtgtgcacg tgtgcctgtg tgtgcatgtg tgcgcacaca catacataca catgtttgag 1380
   tatgccttgg tattggggtc aggaaatagt aaggatagaa agataggaag aagtgagggc 1440
   acttcattgc agccaccaaa cccaggagta cctgcccctc cccgaaggct gcagcctatt 1500
   agccagaggg cctgggatat agccagcgac tggggctgga ttagcgaggt tgatgaaaaa 1560
   ttatgtactt tatagttctg tcagaccagg gtacccttgg aataaaatct tttagaattt 1620
   gaaaaaatat ccacttacta gtgaatacag gacccccatc tgagacctac tgctgcccag 1680
   gatggcagtt tataattaat atttcatcag gggtcaaact gcagaccacc aagaagaccc 1740
   tgtgagactg tattgaaaaa tcaacttgtt accctttgcc attttttaaa aaagaaaaaa 1800
   gtgaatccat ttgaaattca tagataggag tctgcgactg caaaatgtcc aataagatta 1860
   actaaaaatt cctcaggagc ctgaatattt atatttccag ggattcttta ctgtagtatc 1920
   agtacttctt agaagggttt gttgtttgtg gatggcaagt gcctttgcac tgcttctttg 1980
   tacaattctt agccacatga ctttcctcag gagttgacag aatcccccac ccccccaaaa 2040
   aaattcagag gagccttatt tttgcttact tttaaaggaa gagaacacaa gttattttaa 2100
   aaggtttctg gaatttggct atctagatgg tttgacagtg ttaagtgaat caactaatca 2160
   cattgaccaa atcactggct aattcagtaa agcaatcagc ttaactaatt gtccaactga 2220
   tgatcaaatc aatgaaccgg tcttgttttg gatcttttgt ttttcgtttg ctttgttctt 2280
   gacattgggt aggcacagct caagttgtgc aagccagaca ttctcaccaa tacttatagg 2340
   aaagaagtgg acaatatact taaaaatgtg agaatccgta tggaaaacag aacttcatta 2400
   aagaattaga tggaattaaa tgggttaagc tcatcagaaa aaaaaaaaaa attaaaggaa 2460
   aaggctattc ttaccaggga atgaagtctc tgcagaaatt gattatgtag agagtccctt 2520
   tgggtcttgg aacaattagc tctcagaaaa ttagaggata gcctttggct tctggggatt 2580
   cgaatgtaac aggtgtctgc tgtcccttag gaaattcttc actccttcta gctgctcatt 2640
   ttttggctgc ctgcccctgc agggtaaacc caaacccctg agagttaaag attttgagtc 2700
   atagagaacc tgaataattg gattctcgga gggcattact tttgcaagat tccgccacag 2760
   atgtacttct ggggtgtttt ctgtgataga aaaaagaaat cccttctcct actgagaaaa 2820
   gacaacaact aagaggccgt atttcctaca gtgggtaacg gagaggagac aatatttgtt 2880
   tctgtccctg tactaacctc attcttagga cagcatgaac actgggggag gtgactaggc 2940
   ctccgtcttt cagatacctg agaaaagtga aacgtgatct ctgtggtgat tcaggtagag 3000
   ttattaggca tggacagaaa gcagggagtg cacagatgcc tttgtgcaaa ggagagtgat 3060
   tttctcattt tacctgctga gaaagagtag aggtatttat gccaggatgg gtgttattaa 3120
   acagatgaag gacaatctga cagagttaat taaggatctt gagtgtcagg atccagagaa 3180
   agagatatct cgtgtaggag gcaggtatgg gaaaggtaag tccgcaaggg agggtgagtg 3240
   cttttcccac aaaggggagg cagcacattc ttgcttcagg gtgaaggata caacacattc 3300
   tgaaatgagg tttggaatat tgtgtatttt tagctgctgc tgtgaaaatt cctcctaagg 3360
   agagagccat ctttggggaa attgcctctc caagtctccc tgggtagggg ctgagcctcc 3420
   agggtttggc taggccaagg cccagctctc agaggccagg gtgaggtaag gctgcctgta 3480
   gaggatggaa cctgggaaga aggggaagaa agagatagaa aaaggcttcc agagggacct 3540
   gggtatgaaa tgactgggtc tacgcgggat gtgactcctc tctctggctc ttcaaatctt 3600
   cataataatc aaactgacaa catgtttggt ggaaatagat tttttttttt taaaccagtg 3660
   aagttgggcc cctagagtag aacagagata caatgtgtgg aagtagggtc tggagcagag 3720
   gacctctaaa accactatgt ctgtgctagg gacacaggaa ccatagtggg ccccacccaa 3780
   tatctgagtt tcagagtaaa acaaaatgag gctttgagtg tgctgtattt taatatgtgg 3840
   ttgcagagat caacacgcag caggagaacc actttgcaat ataaaaatct gtctccaggc 3900
   tgggcgtggc ggctcacgcc tgtaatccca gtgagaggtg acagcgtgct ggcagtcctc 3960
   aaaaccctcg cttgctctcg gcgcctcctc tgcctgggtt cccactttgg ccgcacttga 4020
   ggagcccttc ggcctgccgc tgcactgtgg gagccccttt ctgggctggc caaggccaga 4080
   gccggctccc tcagcttgca gggaggtgtg gagggagagg cacgagcggg aaccagggct 4140
   gtgcgcagcg cttgcgggcc agctggagtt ccgggtgggc gtgggcttgg ctggccctgc 4200
   actctgagcg gccagctggc cctgccggcc ccgggcagtg aggggcttag cacccgggcc 4260
   agcggctgcg gagggtgtac tgggtccccc agcagtgcca gccgaccggc gctgtgctcg 4320
   atttctcacc cagccttagc tgccttcccg cggggcaggg ctcgggacct gcagcccacc 4380
   atgcctgagc ctcccaccca ctccatgggc tcctgtgcgg cccgagcctc cctgacgagc 4440
   accaccccct gctccatggc gcccagtccc atcgaccacc caagggctga ggaatgcgag 4500
   cgcacgcgca ggactggcag gcagctccac ctgcagcccg gatgtgggat ccactaggtg 4560
   aagccagctg ggctcctgag tctggtgggg atgtggacag tctttatgtc tagctcaggg 4620
   attgtaaata caccaatcag caccctgtgt ttagctcaag gtttgtgagt gcaccaatgg 4680
   acactctgtt atctagctgc tctggtgggg ccttggagaa cctttatgtc tagctcaggg 4740
   attgtaaaca caccaatcag caccctgtgt ctagctcagg gtttgtgagt gcaccaatcg 4800
   acactctgta tctagctgct ctggtggggc cttggagaac ctttatgtct agctcaggga 4860
   ttgtaaatac accaatcagc actctgtatc tagctcaagg tttgtaaaca caccaatcag 4920
   caccctgtgt ttagctcaag gtttgtgaat gcaccaatcg acactctgta tctagctgct 4980
   ctgctggggc cttggagaac ctttgtgtcc atactctgta tctaactaat ctgatgggga 5040
   cgtggagaac ctttgtatct agcccaggga ttgtaaatgc accaatcagc accctgtcaa 5100
   aacaggccac tcggctctac caatcagcag gatgtgggtg gggccagata agagactaaa 5160
   agcaggctgc ccgagccagc attggtaacc cgcttggatc cccttccaca ctgtggaagc 5220
   attgtttttt cactctgcaa tgaatcttgc tactgctcac tctttgggtc cacgctgctt 5280
   ttatgagctg taacactcac cgcgaagatc tgcagcttca ctcctgagcc cagggagacc 5340
   atgagcccac cgggatgaat gaacaacccc agacgtgctg ccttaagagc tgtaacactc 5400
   accgtgaagg tctgcagctt cactcctgag ccagcgagac cacgaaccca ccagaaggaa 5460
   gaaactctga acacatctga acatcagaag ggacagactc cagacgcgcc accttaagag 5520
   ctgtaacact caccacgagg gtccgcggct tcattcttga agtcagtgag accaagaacc 5580
   caccaattcc ggacacacca gcactctggg aggccaaggt gggaggatca cctgaggtca 5640
   ggagtgcgag accagcctgg ccaacatggt aaaccccgtt tctactacta aaaataccaa 5700
   aattagtggg gcttggtggc gggtgcctgt aatcccagct acttcggagg ctgaggcagg 5760
   agaatcactt gaacctggga ggcagaggct gcagtgagcc actgcattcc agcctgggca 5820
   acaagagcaa aactctgtct caaaaaaaaa aaaaaaatct tgagtggtga ttcttactta 5880
   agtgcacaca ttggcacttc tttattttga gggaactgtg gtagtatcta tcaaaactta 5940
   aactaggaac aacctccaac tcaataactg ccttatagaa acactcaaat atgtgtatac 6000
   ttcaaggaaa ctgccttata gaaatgctca aatatgtgta caaacacata agtgcaagga 6060
   agttgttttg aagcataaat tgtaagagca aaaccttgga aataacctga ttgtccataa 6120
   atagggaaat agtcaaactg attattatac gattatgtaa tcttcagtat actaattttt 6180
   ttttaattgg ggcaaaattc acataacata aaattaacca caaaccattt ttaagtgtac 6240
   aatttagtgg catttaatac attcatagtg tgcaactgag acagccaaat gcttaggcag 6300
   ataagaaggg gtccccagag aatctccaac ctgccccaca agtgtttaca tcggatgctt 6360
   tcgtgcagat gagggactct gcccaaagct ttgtctgggc atgcccacag tggactggag 6420
   ctccacatgc gcactggggg aagtgggtgg agccatgggg aattggcacc ctacacaggg 6480
   gaggagcctg ctctcccagg ttcctgtgtg gtggccaggg attcagtctg tgaggtggac 6540
   ggcatgttac aggtctccat ctcactttgc tgagtgtttt tttttctttt ttttcctttt 6600
   tgcccaataa agtcctgctc tactcaccct tcaatgtgtc cgtgtgccta aattttcctg 6660
   gtcgtgtgac aagaacctga gatttagctg aactaaggag taaggttcta caatacaacc 6720
   atcacctctc tttaattcca ggacggtgtt atcactccaa aagggagcct catacacatt 6780
   aagcagtcac tcctcatcac ccttgagaaa tgatttcttg aatttttgag acagggtctt 6840
   gctttgttgc ccaggctgga gtgcagtggt gcaatgatgg cttactgcag cctcaatcgt 6900
   tcaggctgaa gcgatcctcc cacttcagcc tcccagagta gctggtatta caggtgtgta 6960
   tcaccatgcc cagctaattt atcaattttt tgtacagata gggtcttact atgttgtcca 7020
   agctggtctt gaactcctgg cctcaagcca ggagtttccc aaagtgctag gactacaggt 7080
   gtgagccacc atacctggct gagaaaagat tttaaatgtc agaatagtat gaacagtgtc 7140
   accctatata tgtatgaaaa ccctctaaac tatgtcctat gtttccgtct actgacagag 7200
   gcattgcaga atagtggttc taggcagagc tccagaacca ggccgcctgg ctttcaaatc 7260
   ctggccctga cacagaccag ctgtgcatca ctgggggtgt gtttagccta tctgtgcctc 7320
   tgtttccaca ggtgtaaatt gggcatagtt gtgaagagga aatcagtaag gacaagccta 7380
   atacagtcac tgtttagtat acttacttct gtgtttgaag ttttcttttt aaagtcaaaa 7440
   tcatgggcaa ataaacctat tgccagtgta gaacacacat tcctagacta ctccctttca 7500
   ggacaccagg tgagggctac cccccatcag tgcttgcctc ttgcttaaag ctaaggctgc 7560
   agggaggatg catattttca cgccagcaca gagcacactt taaatctcct caacagcgtg 7620
   gcagaaatgc aggctttaaa gagcaacaaa atcctgactg acaagattca agggaagtac 7680
   ctgatcaggg aagtacaagt acaagattca gggaagtaac tgaaaaatgt aagttttaag 7740
   acttacattt gactcaaggg ccagcaggca tttcagacag caagagggag ggtctctgtg 7800
   aaggtgagat acccaaagaa gaaggaacag agggaagaag gaaggaaaag gaagaaggaa 7860
   gaggggaagg aatggagaaa agaagaaaag gaagaaggga gagaaggagg aaggaaggag 7920
   aaaggaaggg aggaaggaag aaggaaagaa gggaaggagg aaagggagaa gaaagggagg 7980
   aacaaaggaa gagggtaaga aaaaggaaga aaggaaagag gagagagagg gaataaggaa 8040
   ggaagaggaa aagaaaaaag ggttaaaaaa taagttagga tgaaggaaag gaagaaaaga 8100
   gaaggtgagg aagtacaagg aaacaggtga gccccagccc tgctgtgatt tcagttctca 8160
   gatctaaagt gttcaagtaa aaacaaagca caggcatagc cttctttttt tttttttttc 8220
   tagatggagt ctcgctctgt cacccaggct ggagtgcagt ggtgcgatct cagctcactg 8280
   caacctctgc ctcccgggtt caagcgattc ttctgcctca gtctccagag tagctgggac 8340
   tacaggtgtg cgccaccaca cctggctaat ttttgtattt tttagtattt ttagtagaga 8400
   tggggtttca ccatataggc caggctggtc tcgaactcct gacctcgtga tctgcctgcc 8460
   tcagcttcct caagtgctgg gattacaggc atgagccacc gcgcctggcc agcacaggca 8520
   aagcctttca accaaaaaat caagggagaa ctaagagaag tatttgcgat ttcaattttt 8580
   ttctactgag tcctaatcta aatggaggtc agaaactgga caatacaaag ctgccatgcg 8640
   tcaaggatgg aacaagcaca gccgcggcca agctcgcaca ttcctaggtg ctttccagga 8700
   tgcccctcca gggtgcgtgc ggtggcctcc aggatgcccc tccagggtgc gtgcggtggc 8760
   ctccaggatg cccctccagg gtgcgtgtgg tggcctccag gatgcccctc cagggtgcgt 8820
   gcggtgtccc agcccccaga ggtgcctgcg gaaactgttg caagggtgat cccctgataa 8880
   acctttcact ttgtttctga atcggaacac agccccaagc ggacttgggg ctgctgggct 8940
   gccaggggtt tttaaatatt taacaaggtt tggaggcagt gagcacaaag ctcaggtcca 9000
   cccaatttct tagcagaagg tggaaatgca agtatggaat tgtcttgcag aaaaacgcag 9060
   ttttgaactt ctgtcttttc aaagacaata ttttaaaact caaatgctac gaaactacca 9120
   gataatgagg tagttagatg gaattttgct agcatatatt tgcaaatatt ttaagaatgc 9180
   tttgtatgct tctgttgact taatgaagtg attgaaaagg gttaagccgt gtttggtatc 9240
   ttcaaaatgg cgattttgct tctgataact ttccggaaaa attaaaggtg ttgtgaagga 9300
   taaattatag tttgatttga gtcgtcttgt taaaacgtca ttcctctttc tcagcaacct 9360
   acttcccgtc ttattaagcc agttgactag ataggtctag gcaattatga cgatgattta 9420
   ctatcttcat gctccattgt cactgggagc caagggatgg gggaagggaa ttcacattcc 9480
   catcttacgt gtctcttgga gggacaatca agtcctcagc tgtagcgtta aacacagcaa 9540
   tagaaagcag agacacagta aagcattttc actgctctta tgagctgaga tataaaatga 9600
   gatttatttg caacaatctg atagagaaaa ttgagtcctg agaattgatg tcctagaaag 9660
   gctttcaagt cagccaggag ttactacagg acgtgagtca cacagcaaaa tatcaaagag 9720
   ttggaggaga aaaaaaattg agctttgaca ttttctgcag ccgctgttaa agaaattgac 9780
   caatgtgtac aatacgaatt atggtcacaa ttacaatctt ctccgatcca gtctttccta 9840
   acagttggat accttggatc cttagattga tgtcatcatt cagctttttg cttttttaaa 9900
   atattcagtt ctactgcaga tgggatgcta aataaacaaa ccccctaaat ggctctctcc 9960
   tttcccccca gaggtttaat ccttataaat aactccgaat aattgtgatg tatcttctgg 10020
   catttcacat attctgaaaa ttaccattga aatttgaatt taatcatata ataaccccca 10080
   aatatatttt aaactgagag tataaactag aagttttggt gtaatctcat ttactcagga 10140
   attcatttag tggccactat ttgtcatgat tttaatgtgg gtctgacatt ggtaggtcat 10200
   gttcggagtg aagatatctg cagtttttca atttgcataa tccagttagt gcttccagaa 10260
   tgtcttccat gtctcttcat gtttgcaaat taccagaggt cacaggtaaa attttaaaac 10320
   gtgagagttg ttcatgaaca tactcgggct ttgtcaaaat caggatatct cccttccctt 10380
   tgtagtttac tttttttttt tttttttttt tttgataagt aatgggtctg aagcttcagg 10440
   acacaagatg accaaaacca gtttttctgc tgggccctct gaattctacc caagataatg 10500
   ctacacattt atataacgcc cacacctgct tatgctgcag taattctttc aaaagttcta 10560
   gcaatttgga ctcctctttt tatttaattt ggggatttag tcgctaatct acagttactt 10620
   ctagaaataa gcagatgact gcttcaaaaa gcaaaaactt tctgacacct tttattaccc 10680
   tctagatgaa aatgaggacg gtaataactt tcaaaggtaa ctgatacact caatggtggc 10740
   tcaccacctc atttcagaca accagggggg ctcagtgcac agctgctctt ttattctctt 10800
   ccaagagagt ttgtgtaaga actttgcaag catttccttt acaatttgtc tttctgggaa 10860
   aattgcatat ggcgttggta taacttgctt aatagtcaca atttattaat ttcatcattt 10920
   tttctcaaga gaggtagaaa aaaatgacta atcgtgtggt gggtgacaga gagtctttct 10980
   ttggaccata aaaggtaact ggatcaagtc cccaaacaat gttactgact taaaaattca 11040
   catctgcact gtatttatag ttaccttatt ttttgaccca ttgcttatgc ttgaacaaga 11100
   ctcttttttt tttctttgag acagagtctt gctctgtcac ccaggctgta atacagtggc 11160
   acaatcacgg ctcactgcag cctccatctg cagcctccat ctcctggtct caagcaatcc 11220
   tcccacctca gcctccctag ttgctgggac cacaggctca tgccaccatg cccagctaaa 11280
   aaacaaaact ccccgcgccg cctcaccaaa aaaacaaaac aaaacaaggc tcttaagttc 11340
   aattttctaa gtaaaaattt gtgaaactat tttgttgttt tcattaccat tcgtttctaa 11400
   gaccaaagga tacatattgt tctctgcttt cttctcccta aagccaaact ttttttttct 11460
   ttctttctat ggttacgtca cgttaatttt ttttttttga gacggagtct cgctctgtca 11520
   cccaagccgg agtgcagtca cacagtctca gctcactaaa agctccgcct cctgggttca 11580
   agcgactttc ctgcctcagc ctcctgagta gctggggctt caggtgtgtg ccactacacc 11640
   cagctaattt ttgtattttt agtagagaca gggttttact atgttagcca ggctgctctt 11700
   gaactcctga cctcatgatc cgcccccctc cacctcccaa agtgctggga ttacaggcat 11760
   gagccaccac gcccggccat gtcacgtaaa ttttaatgag acgaaatatc tatcaagctt 11820
   aaaatcatat gagaaatttg aaggaaaatg attgtagatt ttattcggaa aagatactct 11880
   gagtcatcag ttattccttt ttttctgatt attaaggctg ataaaaaaaa acctctttat 11940
   tttgaaatat gtatgggctc acagaaagct gcaacactta acacatagag ttccatgtgg 12000
   tgacatctta tataaccata gtaccatatt aaaaccagta aactggcgtg gaataacact 12060
   agtaactaga gtatagacct tactctgcat tctccagttt ttacatgcac tcatgtgtgt 12120
   gtgtatttat agttctatgc aatttgaccc cagttataga tcagtacata attactgttg 12180
   tagctaacac aacaattagg atacagaatt gttccatcag cacaaaggaa ctccctcatt 12240
   ctaccccttt gcatcctccc tctgtccgct gttcataatc cctggccacc actaatctgt 12300
   tctccatctc tacacttttg tcatttccag gatgttctat aaatggagtc atacagtatg 12360
   taaccttttg acattgactt tttcacccag cataatgtcc ttaagaccca cccagtttgt 12420
   cgcatgtatc catagtttat tcctttgcgt ggctgaatag tattccaccg tatggatatg 12480
   ccagagtttg cttaaccatt tgccctttga aagacatttg gattgttcac agtttttttg 12540
   ctattacaaa taaagctact atcagcattc atatacagac ttattctctg tgtatatgag 12600
   cataagtttt catttctctg ggataaatgc ccaagaggat atgattgcta ggttatgtgg 12660
   caaatgcaga tttagtttta tgaaaaactg tcaagaaatt ttccagagta gatgtacatt 12720
   ttaaacttcc accagcagta taggaggggt ccagttttcc cacatcctcg ccagtaacaa 12780
   actttttaga tgagtaagtg aacatgaaaa cattgtgaga tgacaaaggc cacatataga 12840
   ctgggcaaag tatgtgaaag gagatatttg gcttgtatat gatcctcaga ccactttttc 12900
   aggagagatg gcagcaacat ttgctgtaat ataaatttcc cctgctttta tcagcctgta 12960
   gccatgaatc agcagtggct tcaagtgtta gaaaactcaa ctacagccat ttaagcacat 13020
   ggggctattt tcctcacaga acaagagatg cagaaatcag cgactgcgga ggcttaaaga 13080
   tgatggctgc tgctgctgtt gcttcaggaa tcatgttcac attcctccag gaagaagaca 13140
   gaaggagaaa aggcagtgtt tctatcacag aaaaaaaaat tgtttttttt tttttttcag 13200
   gaacaccact agtgttctgt tttggtcatg ttgcccagaa cactgcctca gatcccctca 13260
   tggctggaac aacatggtag ggagaagggc cttggaatgc tgactgggtc aggaattcac 13320
   tattatctgc cacaggggac cgtcaacatt aatggtcaac ctttgccagc ttaaggcttc 13380
   ccccagtgat gcatggcctc atcttattgt aatgtcctcc atcgtaaagc atttctgggt 13440
   atttctatgg ggtatctcac ttgtgcttat aagcaaagtc cggtcccact gctcaacacc 13500
   attgttctct gcaggtcctt gtgaggtaca atgttatctg cagggccctc ccctccccta 13560
   agcttccgcc ctggtcctct ggttgtcacc atggaggaac tagaaagttc acgcagttgg 13620
   ggctgcattc cttgccaaag tggggagaga gctctccccc ctgctttggg cactctttgt 13680
   catgctctgt gccatgcgtt tcatgactac ctttctgaac ccatttcctc acttgagctt 13740
   atgaacgttc actcttcatt ggttcataca ttgtccatgg ggctggacag gtgaccagac 13800
   acaaggccat ccgttccact gataatgtct ttctcattct tacattccca caaa 13854
<210> 38
   <211> 346832
   <212> DNA
   <213> Homo sapiens
<400> 38
   agcagatggc atttgagcaa acacttgcaa aaggtgagga agatagccat catagctgat      60
   ggaacaagca aaacaaaagt cataaggaag aattgtactc gtgtgtttga agacatcaag     120
   gaatccgggg cgatagacat agcaagtgag gggagggtat gagaggaatt gggtgggagg     180
   cacagtggga ggccagggca tttaagatgt tgaggacttt cggcttcatg cagcataact     240
   gagtatattt aagaaagaat gctcattgaa tgttcccgtt acaccatgca ctgaggtaag     300
   cgctttattt atatttctct gtgatttttt tggttagtct tcataagaac cctacgtaca     360
   aaatagtatt attaatatct atattttatt aacaagcatg tttagtccta aagaagtcga     420
   ttaactcacc caaggtcaca tagatggtaa atgatgagaa ctgtatctag gctttccgac     480
   acctgtctgt ttgatcatag cattatcctg agtccaagtt gaatggcttc tatttgattt     540
   tgcaatgtca aattacctga aatgatgtag acatgcaatt caaagttcca ctgctgtgca      600
   aagacttaat tcttgctatg ggctgaaagt ttccattccc ctcaaattaa tgttatactg      660
   ctaaccccca aagttgtggt attaggagct ggggaatttg gggaggtgat gaagtcattg      720
   ggcagagtcc tcatgagtaa gattagtgca cttttaaaag aggcctaaaa aagactcctg      780
   ctcacctctt ccaccatgtg agggcacagc aagaaggtgc catcagtgaa ccagaaagtg      840
   agcactcatg aggcatggaa tctgccagtg ccttgatctg ggacttccca gcatccaggg      900
   ctgtacgaaa gttctgtagt ctgtgagccg tccagtttat ggtattttgt tatagcagcc      960
   caaatgctat atgtacttag cccatgtgga ctaagataat tggtaagagg tcttcacact     1020
   ttctcagcta ccagaaagta catttgaatt caactgagga aatctattta gtcattgttt     1080
   tgccatgtta cccccaccca acaagcattt gttatgtttt catgttgcaa gttggattct     1140
   tggagaggcc tttgggatca acccttgtga aaaggagaga agagaaaggg gaataggcag     1200
   aagaggaatg agctgtgatg gaggcccact gatggcctca gctgatctca ccagaagctt     1260
   ggcactctgg tggctctcag caagacttta ttcccttaca tcaatccatc attggttgtg     1320
   ggcctctgaa ggagtattga ggcacaacac cttacagcat ccaccacaat ctgtagcatc     1380
   aaaatgcttt ctgattactt gagttgccct gggtgctaca agggaaatac aagcagactc     1440
   taagtgtggt tttattgatg ttgaagtagc ttcctttcat ggaaaaagag gcaaaaacct     1500
   cgacatttcc cctatttccc cttctggtgt acagaaatgc atttggtgtg gatctataaa     1560
   tttaaagcta ataagatttc tattctactg atgctataac tgtttgataa tctaaaaaga     1620
   ttctcaaatg atttttgtgg tggggataac ctagatttgt gaaggtgcac atatgggttt     1680
   gtgagtgtgt gttttaaaca tataatagaa atagaagaga gataacataa ggaaatgtgt     1740
   cacttttccc aatttttcat tctcatgaat gctcacttct tcctttcgct ttcatggttg     1800
   ctgagggtta tttgctggcg ggacaatagt ccctgtgtca aagggctggt gggtcaactc     1860
   accaagctaa gctgctgctg acagatgagt gatggaaaga tgtactgttg tatctgtcag     1920
   ctctgatgag aaaaagtgga gcttagctta gaatcattct catttccaga tttttatttg     1980
   tattgttatt ttattcctga aaagaggatt gatttctttc tgcattatgt aggtctcttt     2040
   ttcaatgctc ccgggtaaac caatctcaaa gcacaaagac tgaaattcct ctactttcca     2100
   cagtttgtag gcaacgaatt tagaatgtgt ctcatcacca ctttattctt ctcatcacct     2160
   cgaaaagtga ctagcagaat tcccacttgg tttataataa aagaaagcaa caatttcatc     2220
   tacgaattag catactagta tgaatgcgca ctcttgaaaa taaatgtgct attttgtata     2280
   gtcttgtttc ctgagagagc agtgacttgc atttttatat aaacatgctg aattttacgt     2340
   gtgtttagaa aaatttggca tacatatttc cagaatactg aaaatctgac aaacccttgt     2400
   gccctactgt tgttacccaa cgttccttct ctgggaactg cctattaaaa aatcctgcag 2460
   attgtctccc atcccttatt aaaatgtgca ggaaataagc ctaactgtta agtgtatgaa 2520
   ggtcttgaaa accatactct gctggctatt gtcattgaca ttaaactccc taaaatattc 2580
   taaaacaagg acattcaagt taaccttact tcattattta acactaaaga aggcaacatg 2640
   gattctccaa tctctgagat tgttaggaga taagatcttc cagaacctta tcactcaatc 2700
   acagaatcaa agaatgtgct cagctaagac acattgattg acaaatcctt tatgcatatg 2760
   agtgacaatg tcagctttca ggaagcagag aaaagatgaa gaaaaataaa atctctagat 2820
   gactcacaat taactaggac aaattttctg gacatacaga gttcataggt accttgggag 2880
   gcgtttataa tgtttgcccg ttaattgaag catttattta tttaataatc aaacatttat 2940
   tgaatgccta tggggatcca ggatttaaag atacagagct taaaaactga gaccttgcct 3000
   ctgctatagg gatttacaga aaaagaaatg aatttataat gcaatagaac gaaggcatct 3060
   ctggaagcac gctcaggaat atggaaggat atgggctgga aaccaaaccc aaatagcaga 3120
   catggaggaa gatgttttgt tttttcttca ggaattccac gtgacttcat ataagcttga 3180
   aatatgagta tggataggcc aggaggccca ggaacaggga ttggtgaagt ggtagtgatg 3240
   gtgggttggg gttacataaa ggagagaaag gacagcacat tcggagtcac caaggggcag 3300
   gattgagaaa ctgcaagcaa ttcaagatgg cgggaataga actgatgagg ctagaaataa 3360
   agaggttgat ctataagagg aggacaaggg ccagatcgag gccttataag ccctgctaag 3420
   gaatttgaat tttatcatga aggcaatagg gacccagtga aggctaagta gggagatcct 3480
   atggacattt ctgctttaga gctgggagtt acacacaggg gttttaaatc cattagggcc 3540
   atgaaattag atattcagac cggtatattt tcttaatgaa atagaatcaa ttataataaa 3600
   aaggaaatct taaaatacat tgcaagtagt aatagtaatt aatttgtttt atacaatttg 3660
   tgtagtaggt cacaaagtaa gtttttgtta ctgagggtgt ggccaaaata atttgagaag 3720
   cagtaattta gaaagatctt tcaggcagct gaaagcaggg agagcagtta aaagattatt 3780
   cctagaacca tattatacaa gaggagagaa gtgggtcctg cagaatcatg ggaagatggg 3840
   aggacacgtc atttcattcc tgtgatgtct ctgaggtgac taaagcctcc agactctgca 3900
   tttcatagat tgcaacatga gacatggatg cctatgacct ctcaaaggaa gggcttccac 3960
   catgtttgca gtgctgcttt gctgttccct ccacctcaga tgatagtgct tcatttctac 4020
   atgaaaggtc cattattcga aagacagctt aaagtgttgc attttccatg aaatttcctc 4080
   agatctccct atctagaaat aacctagcat caatcctttc ttccactgta ttagtcattt 4140
   gtgtagcatg ccccataaat cccttcttgc tgcatccacc tgttgaatgg tcttcagaat 4200
   aatcaacact atttgaaaag tgcttacaag tgtctttgca tttcttcctt ctcttccctc 4260
   ttctagaatg tgagacactt gagggcagga gtggaactgt cttcttcact atttcaaggg 4320
   ccaggaaagg gagtgacatt gagaagagac tgaataaaaa gttattaaat gaaggaatgg 4380
   atttaaaagc accctgatct gcagtgtacc tgaaacagca tatacttcat atcacaactg 4440
   tagtatcttc atccttgctc atgttttttc cactgcacta aagtgcttct ttaaatagaa 4500
   gaatcaaaat agtgggattc aatgataagt agatgctatt ttgaaatttt attttgatta 4560
   gaaatggatt gcagtaatat ttttactttc tgtggaatgg ttatgtacat aataaatact 4620
   ctgcctgcat tgtatttgca aaacatcgat gtctttgatt ttgtggcttt agaaagtttt 4680
   tcaaatacaa attcaatctg ccattgtttg tgtgagttgg ggaaagaagg gcccacacat 4740
   tttggagctt cccaaatgga catgttggca atgattccat gagtcctgtc tctgaagtct 4800
   ttgggaaggt ctccaacctc ctgcccactt atttttttcc ttgtttattt ttatggagga 4860
   agccaaaatc aagttaagga tatttaatta taggctgtgg ctttctttag aattaaaata 4920
   atcagtgata tattaccatt ttgccaaaca tctcatgatg ctcttgtttt taaggatgac 4980
   aataattttt aatttaaaat gcaaacaaac ctgctgctta gccctttgtg ctttacctgt 5040
   tgagagtatt tggaaatctg acattggaag gcttgtgtta cctttcatca ggttcaacaa 5100
   ggcagtagca acataaagaa aaccctgggg agtcagactc atgatacact cataggctta 5160
   tttagcgtta agatacaaat atctaggatg tgaagtccac gctaatggtt gatggaagaa 5220
   tttaaattat tgctataacc tgaccatgca caattgtcta actcttactt tggattgatt 5280
   gcttatatgt gatgtcataa tgtggttaag aaaggcaaaa atttactgaa atcaaaatac 5340
   ctggattgta attgccgttc tgctataagt tagttgtgca ataattagag gattctaaaa 5400
   caaagctata acagcaacaa caaatataga caaatgtagt tgaattcaag gaattataga 5460
   caaatttaaa atcaaattct actttcagca cttaccattt ttgttagatt gagcaaggcc 5520
   ctgcacctct cagagcctca gttttctcat ttgtgaaaaa tggtgcatca cgagcttatc 5580
   agaattcact gagttgtgtt attgaaatgc ctaggaaaaa tgctggggat attatttttt 5640
   tgtataacta aactcaggtg aactgaagca ctgctggagc tgttttggga gaaagtgacc 5700
   aagatctaga agaggtggga aaagagtctt gaacaaagag tacttggagg atgaaattca 5760
   gtaattcaaa cttaaaataa ttcagacgta aagctgttgg aactttattc tgaggaatgt 5820
   ggctgtgaag cctgagtcgc atggcatgcg gatgcaactt ccgtcttttt tctgtaaaca 5880
   ttcaggacca aatagtgcca aagaaaagac acctccccca cctcagatta tcctcctcat 5940
   ggagtaataa agtcctcttc cttgtagcta atcaaatcac tggggtgttt gcactggtct 6000
   tgtataaaaa atataatcct gctaaaattt ctatgtaaag gaaaccatac cttctctact 6060
   ttggaatgct ggccccattc ctttggagtc gatgtttctg ggtggcccgc ctcaaacttt 6120
   gcctcaaaga aactgtatac ttaatcatat tttctgaatc tcattattta aagttgacag 6180
   gaggaactgg gaagttcaca gtggagaaga gaagggagca tattaaaaag agaagtgggt 6240
   tgagagttgg aaatatgatt tatccactca gcactgccac taaatatgta tgccatggga 6300
   ggctggtgac tgtcctattc tggaccaatt tttcttggct taaaaaatga gaaagttgaa 6360
   gtggatgacc tttgaagtct ctcactgctt tcacaatcac ttgagtctga ggataaaccg 6420
   agcagctact tgcacatttt taaaagtatt aatatatggt aggaggaata gacttattca 6480
   gtgctgctat ggaaggcaga acaagtcgtg aaagttatat tgagaaagaa tatttgctta 6540
   acataaaaaa gggcctctca aaaaattaga attatttaat gatagaaaag ctgccttaga 6600
   aggtggtaag ctccaatgtt gagattttaa gtctctgtat ttcctctaaa tatttagaat 6660
   attcttcctt tcactttttc actaagccgg ttcatattaa gatacaacca acttatcacc 6720
   tttttggggg aaacttccta gggtttctct gtctggtaaa atcatcccct ttcttttccc 6780
   tcagactttt gtgcttatct ctattttggg ggcctatttg ggggcaaaaa tttgtcatga 6840
   tgagaaattg aaacctctcc aaaatggaag aatacttgaa aaattatggg atagccatgt 6900
   gataaaatac ttcacaatca tttaaaattt tgttcatgaa aagattgttt tgaaatacaa 6960
   aattatgtta ttttataatg ctacctgaga aaagaaaggt acaaattgta tatttaagta 7020
   tagtcttctt tttttttttt ttaaacgttg tttcactctt attgcccagg ctggcatgca 7080
   atggtgcaat ctcggctcac tgcaacctcc gtctcccagg ttcaatcagt tctccggcct 7140
   cagcctcctg agcagctggg attacaggca tgcgccacca tgcctggcta attttttgta 7200
   tttttagtat agacggggtt tctccacgtt ggtcaggctg gtctcaaact cccaacctca 7260
   ggtgatccac ctgccttggc ctcccaaagt gctgggatta taggcgtgaa ccactgcacc 7320
   tggcctaact ccaactgttt taaatgtgct tataaacact tgaatagaag ggtatatata 7380
   ttaaaatact cattttgaaa gtgggcaaat ctttacaggc aaatatttat tattttatct 7440
   acatttttct ttattttctt atgttctcaa gtgagcatgt ctgcttttat aatataaaaa 7500
   ttgttaaaat agaaaaacaa taacttgtat tttgaaaacc aagaaaatag ttgatattca 7560
   attacctgga tttctggatt gggttttgtg ctgtcacaaa ttgtgccgtc acaaatgaca 7620
   cacatgacac acaaataaca caaatgacac aaatgacaca aaactgtcat gcacaattcc 7680
   tgatgaataa tctttttatt ttgcaagcat cttttgtcct tcatcctcat ccagtatcca 7740
   gcatcacagg ccaccgcagg aaagctgagg ccccgtggac aatgaaacca cataaactga 7800
   gctcagagaa atgcagcttg atgaaataaa gtagttgaaa tggaaaaata gttccctata 7860
   ttcttcctga ttccttcagg ttacctgctc caactatggc taaagtgctg gagagttttg 7920
   attgccagcc acttcttgat catttctttg agtctctatt ctttcctggt ttaagctttt 7980
   aaccctatcc ttccagagct cctggcatga aaggggctaa ggtcctagtg atcgtaatac 8040
   aacaaaactc tgatgttgac tccagggagg gaaagaattc aacatcaatg tgctatataa 8100
   tccaaagaaa ggatttggtt ttattgtgag caggtgactc aagtcaggtg cctgcaataa 8160
   gctagaaaaa ggcaactttg tgaaagttat caagctgtta atctagttta atctcctcaa 8220
   ccagcaaaca ttgggaaaga gcatacaata ttccaggctc agtgctaggc atttagggta 8280
   acataaaggt aaagaaatct caaggtactc atcagggatg gcagcaagca gagatgtgaa 8340
   gaaacaatta cgatgctgag ccataggcac aactctacac attatccaca gtgtatacac 8400
   attatacaca tacatgtgca tgtgactgtg tcttccaact taggcggaca tgaacaatga 8460
   gaaaaagagt tttaagactg aatgagaatt taccgaaaaa tatgagcaaa ggaaaaaaga 8520
   ggtttttagg tggatgaaag tgcccaggta gatccttaga catatgaata ggtatatgtg 8580
   atgggagatt aattgtttac tttcattaat atttagtggg agcctatgca tattaggaac 8640
   tatatcagga actgttttag gtaatgagaa tcccaagctg agcttatatt gtgtcaggaa 8700
   gggagaaaat aaacctatta acaaataaaa gtataattcc tactctgccc tcaaggaggt 8760
   ggagcttgtc ttcctacccc ataagtgtgg gtagcatata gtgcctttct ttcaaagaac 8820
   acagcgtgga aaagagtagg aggagaaaca acttcccagt ggagaaacct gacaaacttt 8880
   tcctccacca ggtcatgaag gtcaacatca acagtcataa atcatgtgta taacatttac 8940
   ctttgataca ctgcgatgaa aagggcactt cacctctgca gtctttctcc ccagaacaca 9000
   taaccctagt ctaatcatga gaaaaaatca gacaaatttc aaaagggaaa aattttatag 9060
   aatacatggc caatgctcct gaaaacatta aaagtcatca aaaagcagta acgtgtgagg 9120
   aattatccca gcagagagga gaccaaggag acatgacagc tgaatgtaat gtgttgtcct 9180
   ggatgggatc ctggaaggga aaaaggacac taagtaaaaa ctaaggaaat atgaataaag 9240
   tatggacgtt agttaatcat aatgcattga tattgtttta ttcactctga catatgtacc 9300
   cctaatgtaa gttgttaata acaggggaaa ctgagtgtat gcaatatgga ctctataaca 9360
   tcttcataat ttttctgtga atctaaacca ttttttaaaa atatataata ccaagtcggt 9420
   tgatgataag tgctatgaaa catagtaagg caaggaatgg ggtcaggaag gggctgggga 9480
   gtgaggagtc ttttggcaaa ggtcctaggg gaggcctcca tgagcaggtg tatttgaaag 9540
   agtcctgagt gaagagggag agatcatgcc agcacatggg aatgcagatc ctaaggccaa 9600
   aaggctgaga caggcttgtt gtgtttgaag gacagcacgc atgcaagcat gcatagagca 9660
   cagtgagcta gcgggagtac gatgaggtta gagaggaagc tgaggctaga ttatgtaaaa 9720
   ctttgggggt catagtaaag gatttgaatt ttacttggag taagattgaa cagtgttgca 9780
   tgatttgcct tgtatattcc tgagatttac ctggttgttg cctgaactat agatagtaga 9840
   gggcagagtg gaaacatgtg aacacttagt aacctattgc agtggactag ttaaatgtcg 9900
   atggctacct gggccaggat ggcagtggtg gcattggtaa gtagtagtca ggtcttggat 9960
   gtgttttgaa gaaacatggc atgctgatgc atgagaatca gggaaaagga aaaaattaag 10020
   gatgttttaa aattctttgg gaaattgggt ggatggtaga taggtggtct gagtccataa 10080
   actgaatgag aagaatggtg gagtaaaagg gtgcagatgg ggttaataga tttgatggtg 10140
   taaggctgaa gaccttctct gattaaatct attttttttt tttttggaaa atgagaagca 10200
   ggtcagtaag ctgggaggaa cttagggagc aacagatttc agtgttaaaa caagaattca 10260
   aattgagaga tgtgaagttt gagattcatg ttaggcttcc aattagagaa tcagatggtt 10320
   agttatgtag atgactttgg aaagcaaggt agtgatagga gctgaagtat acacttagtt 10380
   accagtttat atgagtgttt aatcaagcat aaattgtgag tttacatagg gaatgaccgt 10440
   aagttagagg tgtctgaaga ctgaggcctc tggaactgca agagtgagtg gtcagaaaga 10500
   aaataattta acaaatggac attccattga tgtggaagta aaagcaaaca gtgattgtat 10560
   tgcaggagcc aaatgcaaaa gttgttttaa gatagaaatc agttggaaat gggttccaag 10620
   atggccgaat aggaacagct ccagtctaca gctctcagca cgagcgatgc agaagacagg 10680
   tgatttctgc atttccaact gaggtaccgg gttcatctca ctggggctca tcggagagtg 10740
   ggggaaggac agtgggtgca gctcaccaag cgtgagctga agcagggcga ggcatcgcct 10800
   cacctgggaa gtgcaagggg tcagggaatt ccctttccta gccaaggaaa ggggtgacag 10860
   atggcacctg gaaaatcggg tcactcccac cctaatactg tgcttttctg atggtcttag 10920
   caaacggcac accaggagat tatatcctgc acttggcttg gagggtccta cacccacgga 10980
   gcctcgctca ttgctagcac agcagcctgg gggaggggca cccgccattg ctgaggcttg 11040
   agtaggtaaa caaagcggct gggaagctag aactgggtgg agcccaccgc agctcaagga 11100
   ggcctgcctg cctctgtaga ctccacctct gggggcaggg catagccaaa caaaaggcag 11160
   cagaaaccgc tgcagactta aatgaaccct acagcttgga agacagtagt ggttctccca 11220
   gcatggagct tgagatcaga gaaaggacag actgcctcct caagtgggtc cctgacccac 11280
   gagtagccta actgggaggc acaccccaat aggggcagac tgacacccat acggctgggt 11340
   acccctcttg gacaaaactt ccagaggaac gatcaggcag caacatttgc tgttcaccaa 11400
   tattcgctgt tctttagcct ccactgctga tacccaggca aacaggttct ggagtggacc 11460
   tccagcaaac tccaacagac ctgcagctga gggtcctgac tgttagaagg aaaactaaca 11520
   aacagaaagg acatccacac caaaacccca tctgtacatc accatcatca aagaccaaag 11580
   gtagataaaa ccacaaagat ggggagaaaa aagagcagaa aaactggaaa ctctaaaaat 11640
   cagagcacct ctcctcttcc aaaggaatgc agctcctcac cagcaatgga acaaagctgg 11700
   atggagaatg actttgacga attgagagaa gaaggcttca gacgatcaaa cttctcccag 11760
   ctaaaggaag aagttcgaac ccatggcaaa gaagctgaaa accttgaaaa aacattagac 11820
   gaatggctaa ctagaatagc caatgcagag gagtccttaa aggacctgat ggagctgaac 11880
   tacgtgacca atgcacaagc ttcagtagcc gattcgatca actggaagaa agggtatcag 11940
   tgatagaaga tcaaatgaat gcaataaagt gagaaaagtt tagagaaaaa agaataaaaa 12000
   gaaatgaaca aaccctccga gaaatatggg actatgtgaa aagaccaaat ctacatctga 12060
   ttggtgtacc tgaaggtgac agggagaatg gaaccaagtt ggaaaacact ctgcaggata 12120
   ttatccagga gaacttcccc aatctagcaa ggcaggccag cattcaaatt caggaaatac 12180
   agagaacgcc acaaagatac tcctcgagaa gagcaactcc aagacacata attgtcagat 12240
   tcaccaaagt tgaaatgaag gaaaaaatgt taagggcagc cagaaataaa ggtcaggtta 12300
   cccacaaagg gaagctcatc agactaacag ctcatttctc aaaagaaact ctacaagcca 12360
   gaagagagtg gtggccaata ttcaacattc ttaaagacaa gaattttcaa cccagagtca 12420
   tatccagcca acctaagctt cataagtgaa ggagaaatga aatactttac agacaagcaa 12480
   atgctgagag attttgtcac caccaggcct gccctacaag aacccctgaa ggaagcacta 12540
   aacatggaaa ggaacaacca gtaccagcca ctgcaaaaac atgccaaatt gtgaagacca 12600
   tcagtgctag gaagaaactg catcaactaa cgagcaaaat aaccagctaa catcataatg 12660
   acaggatcaa attcacacat aacgatattt accttaaata taaatgggct aaatgctcca 12720
   attaaaagac acagactggc aaattggata aagagtcaag acccatcagt gtgctgtatt 12780
   caggaaaccc atctcacgtg cagagacaca cataggctca aaataaaggg atggaggaat 12840
   atccaccaag caaatggaaa acagaaaaag gcaggggttg caatcctagt ctctggcaaa 12900
   acagacttta aaccaacaaa gatcaaaaga gacaaagagg ccattacata atggtaaagg 12960
   gatcaattca acaagaagag ataactatcc taaatatata tgcacccaat acaggagcac 13020
   ccaggttcat aaagcaagtc cttagaaacc tacaaagaga gttagagtcc caaacaataa 13080
   taatgggaga ctttaacacc ccactgtcaa cattagacag atcaacgaga cagaaagcta 13140
   acaaggatat ccaggaattg aactcggctc tgcaccaagc agacctaata gacatctaca 13200
   gaactctcca ccccaaatca aaagaatata cattcttctc agcaccacac tgcacctact 13260
   gcaaaattga ccacatagtt ggaagtaaag cactcctcag caaatataaa agaacagaaa 13320
   ttttaacaaa ctttctctca gaccacagtg caatcaaact agaactcagg attaagaaac 13380
   tcactcaaaa ccactcaact acatggaaac tgaacaacct gctcctgaat gactactggg 13440
   gacataacga aatgaacgca gaaataaaga tgttctttga aaccaacgag aacaaagaca 13500
   caacatacca gaatctctgg aacacattta aagcagtgtg tagagggaaa tttatagcac 13560
   taaatgccca caagagaaag caggaaatat ctaaaattga caccctaaca tcacaattaa 13620
   aagaactaga gaagcaagag caaacacatt caaaagctag cagaaggcaa gaaataacta 13680
   agatcagagc agaactgaag gaaatagaga cataaaaaac ccttcaaaaa atcaatgaat 13740
   ccaggagctg gttttttgaa aagatcaaca aaattgataa accgctatca agactaataa 13800
   agaagaaaag agagaagaat caaatagatg caagaaaaaa tgataaaggg gatatcacca 13860
   ctgatcccac agaaatacaa aataccatca gagaatacta taaacacctc tatgcaaata 13920
   aactagaaaa tctagaagaa atggataaat tccttgacac atataacctc ccaagactaa 13980
   atcaggaaga agttgaatct ctgaatagac caatagcagg ctctgaaatt gaggcaataa 14040
   ttaatagctt accaaccaaa aaaagtctag aaccagacag attcacagcc gaattctacc 14100
   agaggtacaa ggaggagctg gtaccattcc ttctgaaact attccaatca atagaaaaag 14160
   agggaatcct ccctaactca ttttatgagg ccagcatcat cctgatacca aagcctggca 14220
   gagacacaac aaaaaaagag aattttagac caatatccct gatgaacatt gctgaaaaag 14280
   tcctcaataa aatactggca aaccgaatcc agcagcacat taaaaagctt atccaccatg 14340
   atcaagtggg cttcatccct gggatgcaag gctggttcaa catacgtgaa tcaataaacg 14400
   taatccagca tataaacaga accaaagaca aaaaccacat gattatctcc atagatgcag 14460
   aaaaggcctt tgacacaatt caacaaccct tcatgctaaa aactctcaat aaattaggta 14520
   ttgatgggac gtatcttaaa ataataagag ctatttatga caaacccaca gccaatatca 14580
   taatgaatgg gcaaaaactg gaagcattcc ctttgaaaac tggcacaaga cagggatgcc 14640
   ctctctcacc actcctattc aacatagtgt tggaagttct ggccagggca atcaggcagg 14700
   agaaagaaat aaacggtttt caattaggaa aagaggaaat caaattgtcc ctgtttgcag 14760
   ataacatgat tgtatatcta gaaaacccca ttgtctcagc ccaaaatctc cttaagctga 14820
   taagcaactt cagcaaactc tcaggatacc aaatcaatgt gcaaatatca caagcattct 14880
   tatacaccaa taacagacaa acagccaaat catgagtgaa ctcccattca caattgcttc 14940
   aaagagaata aaatacctag gaatccaact tacaagggat gtgaaggacc tcttcaagga 15000
   gaactacttt cactgctcga tgaaataaaa gaggatacaa acaaatggaa gaacattcca 15060
   tgctcatgga taggaagaat caatatcatg aaaatggcca tactgcccaa ggtaatttgt 15120
   agattcattg ccatcccatc aagcaaccaa tgactttctt cacagaattg gaaaaaaact 15180
   actttaaagt tcatatggaa ccaaaaaaga gcccacattg ccaagtcaat cctaagccaa 15240
   aagaacaaag ctggaggcat catgctacct gatttcaaac tatactacaa tgctacagta 15300
   acccaaacag catggtagtg gtatcaaaac agagatatag acaaatggaa cagaacagag 15360
   ccctcagaaa taatgccaca catctacaac tatctgatct ttgacaaacc tgacaaaaac 15420
   aagcaatggg gaaatgattc cctatttaac aaatggtgct gggaaaactg gctagccgta 15480
   tgtagaaagc tgaaactgga tcccttcctt acaccttata caaaaattaa ttcaagatgg 15540
   attaaagact taaacgttat acctaaaacc ataaaaaccc tagaagaaaa cctaggcaat 15600
   acccttcagg acataggcat gggcaaggac ttcatgtcta aaacaccaaa agcaatggca 15660
   acaaaagcca aaattgacaa atgggatctc attaaactaa agagcttctg cacagcaaaa 15720
   gaaactacca tcagagtgaa caggcaacct acagaatggg agaaaatttt tgcaatctac 15780
   tcatctgaca aagggctaat atccagaatc tacaaagaac tcaaacaaat ttacaagaaa 15840
   aaaacaaccc cctcaacaag tgggcgaagg atatgaatag acacttctta aaagaagaca 15900
   tctatgcagc caacagacac gtgaaaaaat gcttatcatc actggccatc agagaaatgc 15960
   aaatgaaaac cacaatgaga tgccatctca caccagttag aatggcgatt attaaaaagt 16020
   caggaacaac aggtgctaga gaggatgtga cgaaatagga acacttttac actgttggtg 16080
   ggactgtaag ctagttcaac cattgtggaa gtcggtgtgg tgattcctca gggatctaga 16140
   actagaaata ccatttgacc cagccattcc attactgggt atatacccaa aggattataa 16200
   atcatgctgc tataaagaca catgcacacg tgtgtttatt gtggcactat tcacaatagc 16260
   aaagacttgg aaccaaccca aatgtccatc aatgatagac tggaataaga aaatgtggta 16320
   catatacagc atggaatcct ctgcagccat aaaaaaggat gagttcatgt cctttgtagg 16380
   gacatggctg aagctggaaa ccatcattct cagcaaacta tcataaggac aaaaaaacaa 16440
   acaccgcatg ttctcactca cagttgggaa ttgaacaatg agtacacttg gacacaggaa 16500
   ggggaacatc acacaccggg gcctgttgtg gggtgggggc agggggaggg atagcattag 16560
   gagatatact taatactaaa tgacgagtca atgggtgcag cacaccaaca tggcacatgt 16620
   atacatatgt aacaaacctg cacattgtgc acatgtaccc tagaacttaa agtatattta 16680
   aaaaaatgct gggtgcggtg gctcgcgcct gtaatcccag cactttggga ggccaagggg 16740
   ggcggatcac gaggtcaaga gatgagacca tcctggctaa catggtgaaa caccgtctct 16800
   actaaaaata caaaaaatta gctgggcaag gtggcagggg cctgtagtcc cagctactcc 16860
   tgaggctgag gcaggagaat ggcatgaacc ccggtggggg agcctgcagt gagctgtgat 16920
   cctgccactg cactccagcc tgggcgagag tctgactcca tctcaaaaaa acaaacaaac 16980
   aaacaaaaaa caagaacaaa acaaaacaaa acaaaagagc acctacctcg tagactgctg 17040
   tgaatcttaa aggtgccaat gcacactaaa actttgcata tttccaggca catagcaaac 17100
   ccttagcatt acctatctca atatttttct tggttttgtt ttgaataaaa gcatttggac 17160
   ctaattttaa ttgttaatta taataaataa tttatttgtg aaaaaaagat agaaatctat 17220
   tgtgtcaaat gctacaaaga gtcaattaag taagttcagg tgtgggaact gaccagtgaa 17280
   ttaactccct tgacaaatgc gtgttagggt aatgatgagg ataaaggcat gactcgatgg 17340
   aattcaagag agattaaagg gtgagagagt gaaaacagtg taagcaacac ttgctgtaac 17400
   ttttgtcaaa acgaggaagg aaatgttaat ggtatctgtt gaaggaaata aaaaaaattt 17460
   tcctattggg tatttattga gcacaatttt tatttggtga aaatgatcca atagggaaag 17520
   aatagttgat ggcatgcaaa gagatgggaa aaattgtagg agcaaagcac tttagggaca 17580
   agaataaata ggacccaggt cacaaataaa gggatttgct tagatagggg gtctgagtcc 17640
   attcactgaa tgagaagaat ggtagagtaa aagggtgcag atggggttaa tagatttggt 17700
   gtatggctga agaccttctc tgattaaatc tatttttttc agaaaatgag aagcaggtca 17760
   ccagctgaaa atgagggggg tggagtgatg atttagtatt ttaaaagcga agaaaaagtg 17820
   agaaatagtt gttttggtga gtaagagagt gaattggatg agcaaatata atagatttcc 17880
   tggacagagt ttggatcata catttgaagt taagcacagt tagcatgtac tgtgatttct 17940
   ccagcttgaa tcatcttgca aggatgatag agacaaaagc aaaagtcgaa tttaagccaa 18000
   aattcggatt tcgtagatga ttatgatgaa ggaaacaggc aagaaaaaga atgacataga 18060
   caagtaatga gatgataaca atagatgaat catacagact ttcgactgaa tacaaaggta 18120
   gtgacaaact gatggaaggt ggagaagaaa aggggaaaat gttaatggaa tcacagacct 18180
   atctgatgag ttgttggagt gttggagtga gagatctaga gaaagtagaa atggaaaaat 18240
   gagtgttgct acacagaaga ctagatgctg aggtgaaata tttaattatg tttgagttat 18300
   tggtaaggac agtctaagaa atggcctggg aagtggggac agttagacaa aggaaggtgg 18360
   aaatgtaaga tggggtgttt ggaagataag aagacaagct attgagaagc ttgagtagta 18420
   gatggttttg agtgatcaag gatgatgcta agagtagtaa gcagtggaga aaccgagcta 18480
   aattaggtgc taaaaatctt gcaaaaatta ggaggattgc tcagaaggat agcaatgtga 18540
   caaaggaaaa tagtggctgg tataactttg cagtgtattt gtcaaagaag ctggaaggtt 18600
   tagggaggat gaaggagcaa tattctggaa ctgacaatgg agatcaaaga ggatacttat 18660
   cctaagccca gtgaaataag aaagatatca cgccatggga gggctgtagg gaaggataac 18720
   ctctggggag agccaggttt gtagaatgga aggtaaaata tttagagaag tggttgaata 18780
   tataatatgg agattttact tttcctagag tttaaattct ggagaaagta ttttgaggag 18840
   tctggaaagg attacagctt gagtcccatg agggaactta cagaacagtg gatggaagaa 18900
   caaattttga cttattgtag taatagagac aaaaagggca tcatgctagt gttctcttat 18960
   ggagaggggg aaactaccag agtatacaca aagactaaag aacagagtat gaatatcttg 19020
   attaagaaag gtcacttgag caattcctag tgatgtagca gacagtgttg gatggagaga 19080
   aagtggagtt ctataagcct ccaggaaatc caactgtggg tattgttgat accaaaactg 19140
   aagctaagac agcaagctaa agtcatggga agaagattgg acttagtctt taggcattag 19200
   gatgctgtat taggttgaat aatgacactc tctgatatgg ttaggcttgg tgtccccacc 19260
   caaatcttga attgtaatct ccataatccc catagtccct tcatatcaag ggagagacca 19320
   ggtggaagta atggaatcat gggtgcagtt tcccccatgc tgttctggtg atagcgagtg 19380
   aatctcacaa gatctgatgg tattataagt gtctggcatt tccccttctg gcacttctcc 19440
   ttcctgatgc cttatgaaga aggtgccttg cttcgtcttc accttccacc ttacaccatg 19500
   attgtaagtt ttctgaggcc ttcccagtca tgcagactgt gagtcaatca aacctctttc 19560
   ctttataaat tgccccatct caggcagttc tttatagcag tatgaaaaca gactaacaca 19620
   ctccctcccc aataatatgt tcataaccac caaacctgta aatctaacct tatttggaat 19680
   aagtgacttt gcttttgtaa tgaagttaag gatcttgaga tataatcatc atggatgagt 19740
   ataagcctta aatctaatga catctttata agagaagaga agataaaaga agataaaaga 19800
   agagaagaga agagaacaaa gacacagaca cagagaagaa tgcgacatga agacagaggc 19860
   agagattgga gtgaggcaac aagccaaagg gcaccaagga ttgccagcag ccatcaggag 19920
   ccaggataga ggcatagaac agagtctacc tcagcgtcca gaaggaacca accaaatacc 19980
   ttggcttcat actctagcgt ctagatctgt gagtaaatgg gcttctgtgg ttttaagcca 20040
   ttaaatttgt ggtaatttgt tatgacagtc ctagaacact aatccatatg cctaagtaag 20100
   aataacttag tcacatatat gaacacatat gcatgacttc tcgcactttt gaagcagcta 20160
   attttcagag acctgtggcc agcatggtct gagatgtcct cagcccagag tgcttattga 20220
   attgatagaa acacatagta attgcattac tgaatgctat tctaatattg tgttagaaat 20280
   aactttatgt taacagaatg tgcaaagaga ttggaataaa ttaggtgcat gagaaaaaaa 20340
   tattgtgtgt aaatgaaagc aggtcaaata tttcagtaga tgagttaacc caaagacagt 20400
   tttctgaagc tggcaggact ccttcttcat aagatcagca agaactgatt gaccaacatg 20460
   ccttggggta tgaagggaca agaagtgtca tctgacctga ctttgtgcct tgacttagga 20520
   ctggccccca aaatacacca cagaaaacaa aataacacat ctctgcagaa attctcttgg 20580
   actctttctg tttgttcttt agtcttcatg gattctctgg tagtttctac tgtcttcccc 20640
   catgatgtgg agaagtttcc tccagtctgc ttcaccctgt aaagtagatt gtccgtgatc 20700
   atggctatgc catgaggaat cgctgtagtt tcccaatgac atcctggtat tggctcacct 20760
   ggtagatgct tcctctggct tctctgtgat aggtgataga cttttgtgtt attcatttgt 20820
   acatcaattt gaccaatatt ttacctctta ctctttgcca agcattgttt tagatatagg 20880
   agataaaaac atctcataag gcatatattt tagtgaatga gcaagaaaat taccatgtga 20940
   aaacataaat gccacttcag aaaagaaaca aaataaggaa atatacaaag aggaatgtta 21000
   ttttggaaag agtaatgaag acaaactcta aggggtgaca gtagagcaca gatatacatt 21060
   atcatgcatg tggggaagaa tattttaggt agagagaaga gcaagtgaaa agcccttaga 21120
   acttgctata tgtgagtaac agcaggatgc tgtggactga gttgggggaa gagatgaggc 21180
   tagagagact ggattttaag tgtcattgtt ttaaatgatg gtggaggagt aaagggagcc 21240
   acataaactg atttatattt ccaaaatatc acaatggctt cagggtgaag gagatagtgt 21300
   agaagccaag agtggacttg gagaaccatt tgagagatgt ccccagtagt tgagatgaga 21360
   gatgatagtg actgggatga tggtgacaat ggagaaagtg ccaaaaagca aactgattcc 21420
   aggaatattt aagggtagag tcaacagaat gtgccggtgg acttaaaaat ggatgtgaaa 21480
   gaaagggaga aatcaagact cctgggcttt ggactaacaa ttggatgaat attgattata 21540
   tttattgaga tgaggaaggc tagtgaagtg tcatgttgag ataggagatg ggaaacaaaa 21600
   gaggtctgtt tttagatatg taagtctgca atgtatttct gatgtttaga tgagacattg 21660
   tataggcaga tagaaatatg agttgatgct caggacagaa attgaagcta gagttacaat 21720
   tatggtgtgc aaagccatga ataaggatga gctcatccag ggagtgagcg gaggacgaaa 21780
   tttccatgct ggcatggaga gataacaaag aggcctggaa agtcttcaaa tctgcaaaac 21840
   agtattgctg ccacccaaac tatcctcttg tgtaagagag atggacacag cactgatgga 21900
   atggtgtcaa aggcatcatc acagacttca gtattaaatt gaagttgtca tttgcttgtc 21960
   ttccaattat atcgggacac tgggtaaatg tactaatctt tggtgatttg gtgtgtgtgt 22020
   gtgtgtgtgg ctgggtgtgc tctgtgtgta tttatgtgaa tacatggact tttaaaatat 22080
   ggatgattat gaaatatatt aaaaatgggt aacccatatc cacttgcttt gcattataaa 22140
   gctttcttcc tgagaagcta tgctacgaaa cattggggac aacttttttt tttttaaaga 22200
   gaaattgaga ctattgtgtc ctggcatcag gccaccatca aaatgagttt gagtttggta 22260
   ttacagagga tgagaaattt aatggaaaaa tttaagagga actttgagat gtggaattaa 22320
   atcttaataa tgtgatcaag gctggagata tgaatttagg tgtcttccat ttataagtga 22380
   gttttgaaac catagaaatg gacatgtttt ccaagagaaa agcatgccca gagacgagag 22440
   ggcagggagt aaagctgtgt ccttgtgtaa gggcatggat tcttatttga cagctaaact 22500
   tatacaacac agagaaatgt cattccaaga agcttaaagc ttagcatgtg gaagcgtcac 22560
   attaaatatt tttccagttc atgaaaaaag ggagtaaatg aagacgtagt tgaaaggaaa 22620
   agaactttgc acagaaatct gagaagaaat agttggagat atagcagcag aaccaagtta 22680
   gaatgttgtc aaaaaaggca acatggtaga aaactaagaa ttctgaagtt gtttaaaata 22740
   ttaaaagaga ggaattcagt tagcaatgag tcaatactca tagtttattg ttgaccttga 22800
   tgacagcagt tttaacatga tgcagtagaa tgctaggaaa tgcagaagag tcataaatcc 22860
   aaggaacaaa gtgagggtga gtctctgaag gctttctgga agaagtgtta ctaggctaga 22920
   gtcttaaatg gctaggagga gtttcccaga aaaagtcgga aggatgaaaa atattaatta 22980
   atattttcca atcactgtgt gtcctgttcc ttttgaatta acccctgaca gctctatgga 23040
   atgtgattct tattcacaaa tcctatttta tactaactga aaagaatctc acatcaatca 23100
   agttctaata atgactatat aattttttga gtaccttcta tgtacctgaa tctgtgcaaa 23160
   gtactatggt agatccaaag gtgccagagc actgttctgt tctcaaagtg caaatgctac 23220
   atttgcagat caaaaaaaat taaggtccat ttataaagca caggttacat gtctggtcct 23280
   atgctctgga ttgcgggcat taagataaaa acataaggca tgtttttgga gaattcacag 23340
   tctagtaata tttgatatac atgcaaacag acacagtttc tcatgatcta tgatatgaca 23400
   gggtactgtg agaacaaaga ggagggatgc ctaacctagg tcacatgagg ggtcatggaa 23460
   agtatagaat gggtaaacct tgagtgcagt cttgcagggg actctatcta ggaagttatc 23520
   taggtgaagt tatctaggag aagcagaata tcctgcaagg gttaatgcag agatctgaga 23580
   gagtaagttg catttgagca ttcagaataa acacctgaaa agtaaaatat gaaggtgagg 23640
   gtgatagaga aaagacttga aagcatgaat tttggctaca aaatgataac ctcagattat 23700
   ccagaaaagt gtgactttct aaccttttaa caattccata cttcttagta aaccttccaa 23760
   taacatcatc tttattaaca gcttctacaa acccactagc atgatggagt tctgcttcag 23820
   cacatctgga tcccaaatgg ttgtcattag tcatttcaaa ggcttagctt attttattta 23880
   tgacttgctt caataaacac actattccta catggaattt aatgtgaact tttgacagtg 23940
   attactattc aacatttggc ctctaatctc ttctgtgcct cgaagtagtt aacgagcact 24000
   cattcaacca gaatttattg agcacttgct acatgtctgg aaccaggaca ggtgccttac 24060
   agtggtgctt ctcaacttta ctcttactaa acattaaata tcactcttta aaaaatatca 24120
   attgcaattt caaaattagc ttcaatttag aacaacagat atttacagaa gacaatttcg 24180
   atatggagat gcttaaaggc ttttcttggc tcatacgacg acaatagaaa gacaaaggtg 24240
   cacttgattg cttgttaaaa acttctctat cctaaattta taatgttcat ttaaccaata 24300
   tttattgaaa atcagctctg cactgagcac tgatcatttt tgggatactt caatgaatac 24360
   aacaaattaa aaaaaaacaa aacctgaaat ggagcttatg ttctaggaag tccagacagt 24420
   aatatacaca aacaaaatga ataagctaaa caaagtttac ctttccgttc aagtctaagg 24480
   tgctgctgct gctgctgctg aaattcatca tcagtgcaaa taatggtctc aaaacccacg 24540
   tgaaagattg caggacaagg gaataaaaaa acttcccttg gaatctttca gtcaaagact 24600
   aagcattggt tacttaagct cacaggcaga tatagaggtg gccgtagagg tccctggaat 24660
   cagactagct tggttcaagt cttggtcccc ccacttccag gccaggtgac ttcagcaagt 24720
   tacctggcct gtctttgtct catctcctca catgaaaaaa gtggcattaa tcttcccatc 24780
   atagcattct gtgagaatta agtgagataa tacacataag atactcagag gtgttgagga 24840
   tcaagaccgc tgatggcagg tacccgtgtc tgacatagac taaattctca gtagatatca 24900
   cttattatta ttgctgagaa gacagtttca ttcatgtgtt taacagaaac tttcagctcc 24960
   aatgtgaaga aaatatcttc tgcacacaga gtagatgaga tttgatactt tccctcaaat 25020
   cgctttcaag atctttgtcc ttgagagtct catcaatctg agaacacgaa gagatgtttt 25080
   agtaccaatt cacagtgttc atagatatat tttggctata aatggtgaag tatgtcataa 25140
   taagtaaagc tattggtatt tgtccctaag ctttttgtgt catgtattca aagcttcaaa 25200
   atgtatgttt ttatatgctg aacagaactg taaagcataa ttgtgggaag tttggagcaa 25260
   atgattgttt taatggcttt cccaaagcag cttatttccc cttgaccact aacaaccata 25320
   caattgaaga actttgtgat tggtcttcag atttgcacag agacaggcag tggccatgtt 25380
   tttatcttta ctgtgcttgt cttgaatgtc atcagctgac aggaagcata gcaagactga 25440
   aggcctcttg tgagttaatg ggcaatttta gttgtcaaag ccaggtagag aataatggaa 25500
   ggagttctta aaattaaagt gcgtctgtga gtgtgtgttt aaatgaattg tatttgtttt 25560
   gtttttcttg tctttatttc tttttctttt ttttctaaca gaagaaatcc tgacactcct 25620
   gcagaaagct tggctgactc acagtcagaa caggccctct ttattaattt caaattcacc 25680
   tcattcactc caggacatag ctttaaagaa aaaaaaaatt agcatttttt tttggtaata 25740
   tcctaggtta cagaaagcat caattcttct gcacatagtt aaacacaatt cctcttatca 25800
   ctgaaggctt taaaactaaa agctaaagag agtgacaacc atatatttga aaaatgttgt 25860
   tgatctcctt tgttacatta ccaatttggc acttaactga aatttctttt aaaatgtata 25920
   atccaagcaa tgttttccaa agtagctatt ctatacattt ttataagttt ttctatgttt 25980
   tttaaaattt tctatttcat aaaatgtttt cttaagctgt gaattcctgg tactctttag 26040
   tccaatattg cctagcttca tgagtaggaa gagccaaatt aatcaaccaa tcacagaaac 26100
   aaacaagcaa acaaataata aaaaacattt gtttaaatca tgtacaatat aaatattttt 26160
   tacttaacta atttttaagt caagagagct ctgctcaggt ttaaaacata aaactcacag 26220
   acaaatttct acactcttct ttacaatgta tacaaaaaac accgactagg aatgcactgg 26280
   aggttggcat tggccttgat atttgaccaa aatctgatat ttggcttcta tattagtatt 26340
   caaagggctg agtgtctact tcagtataat tttaattttg tgatcagata gattaccatc 26400
   gatctagtca caataaaggc atgagtacca ggagcacaga caattttgat tctttaatgc 26460
   ctaaatatta tttttcagat ataaaaatat ttacctgtaa cttgaagtga acatttctcg 26520
   ccctactttt atacatgaaa aaattgtcta cttggttgaa gagtaaatct atatttacta 26580
   ttacaagtag aaaatgctgc aaaaataaat aatgttgatt atgaaaaatg ttaattttat 26640
   tttaaaaacc aataaagaat tcaccagtaa agccatctgg tcctgagttt ttctttctga 26700
   gaagtttttg gttgttgatt caatctcctt attctctatt ggtctgttca aacattctat 26760
   ttattcataa tacaggtatg gtaggttgta agtttctaaa aaatgtattt atttcttcta 26820
   gattatgcaa tatgttggca aataattgat cagaataatc acatgatttt tttatttctg 26880
   tggaatcagt tgtaatatgt tcttttacag ttttgttttt atttccatct tctggttttt 26940
   ttttgtttgt tttctgaaag ctaaaagttt aacagttttg tttatctttg caaaaagcca 27000
   acttttagtt ctgttgattt ttttctattg ttttcctatt ctatatttag cttatttctg 27060
   ctttaatatt tattatttcc tacatcctac taactttagg cttgctttgt cctccttttt 27120
   ccagctcatt gatttgtaaa gttaagttgt ttatttgaaa tttttcttct ttaatataga 27180
   tttttatcac tataagcttt tctcttagaa ctgcttttgc tgcacctcat aagttttggt 27240
   atgttttcta aatttttata tattatctaa atccccttac aatatattcc ttggcccatt 27300
   ggttgtccaa attccacata tttgtgattt tttccttctt tttgtcattt ttagtttggt 27360
   atttttagtt cttagtattt tttagtaatt tttttagtat ttttagtttt ttttctgtta 27420
   ttcattttta gtttcattca tctctgttgg aaaagatact tggtataatt tcgatcttct 27480
   taaatttatt aagacttctg ttgtgacctt atttgtgatc tgccttggag aatgtttcat 27540
   gtacatttga aaagaatgta ttctgctctt gtaggcagaa tgttctgttt atgtatgttg 27600
   atccatatgg tgtatagtgt tgttctaggg agaattaaca ataatccttc aaaaactctt 27660
   ccaaaaaaat taagaagtat aatttccaaa ctaattttat gaggtcagca ttatcctgat 27720
   accaaagcaa gacaaagaca ctataagaaa agagaactat aggccaatat ccttgataaa 27780
   catagatgca gaaatcttta aaatattagc aaactgggga aaactggcta actatatgca 27840
   gaagaatgaa gctggactcc tacctctcaa tatatccaaa aattaactca agatggatta 27900
   aagattttag tgttaagacc ttgaactata aacctcctag aagaaaacct aggaaatatt 27960
   cttctgtaca ttggcctaag cagggaattt atgaccaagt ccttaaaagc aaatgcaata 28020
   aaaacaaaaa ttgacaagtg ggacatagtt aaattaaaaa tcttcttcat aacaaaagaa 28080
   agtatcaaca gacaacctac agaatcagag aaaatatttg ccatctgtgt atctgagaag 28140
   ggactaatat ccagaatcta taaggacttt aaatcaagac aaaacaaatt acttcactaa 28200
   aaagtggtca gagaacatga atacacactt tctaaagaag atatacaagc aaccaatgaa 28260
   catatgaaaa attccctgca tcactattgg agacatgcaa atcgaaacca caatgagata 28320
   ccatctcaca ccagtcagaa tggctaatac aataaagtaa aaaataacgt gttggtgagg 28380
   ttgtggcaaa aagagaacac ttatacactg ttggtgggaa tgtaaactga ttcagccctt 28440
   aggaaagcag tttgaagatt tatcaaagaa ctaaaaatac aactgccatt caacccagca 28500
   gtcccattac tgggtatata cccaaaagaa aatgaattgc tctaccaaaa agacacatgc 28560
   acttatatgt tcattgcagc actatttaca acagcaaaga cataaaatca aactaagtac 28620
   ccatcaatgg tggatgggat aaaagaaatg tagtacagac accatggaat attatgcagg 28680
   cataaaaaag aatgaaatta tttgctttgc agtaacatgg atgcatctgg tggccattat 28740
   cctaagtgaa ttaatgcaga aactgaatac aaaatactgc atattctcac ttataagtga 28800
   gggctaaaca ttaggcacac atacacataa aaatgggaaa aatagacact agagacccca 28860
   aaaagtaaga gaaagaggga caaggggcaa gggttgaaaa actacctatt agtactatgt 28920
   tctctatatg ggtgacggaa tcaatataaa cccaaacctc agcatcatgc aatataccta 28980
   tgtaatgaac ctgcacgtat accctgaatc taaaataaaa aatatatatt ttaaaaactg 29040
   gcaagataaa tttgataaca cattttttag ttcttcttca aatatttgtg agaattcacc 29100
   agtgaagcca tctagtccag ggcttttctt tgttgggaag tttttgattt ctgattaaat 29160
   cctgcttgtt ataggtatat ttagattttc tcattttctt gcattagttt tggtagtttg 29220
   tgtctttcta ggaatttgtt aatttgatct gcagagtatt ctttcataat cctttcaatt 29280
   tctgtaaagt ttgtagtgat gtttctactt tcacatctga ctaagtattt ttgcttcgtg 29340
   attaattttt tgttagtcaa tttacttaat gattgactaa ttttattgat cttatcaaag 29400
   atccaacttt tggttgtact gattctctct attgtttttc tatgaactat tttgaatgtg 29460
   gctttctaag attaactgta ttcagtgctt ttggatgttg taatttccta cagaaactct 29520
   ttcccagctt tgcttcccag gctttaggga gtcttttgta catcacaact acaatctttt 29580
   gccccagata actgggcttt tgattcatca tcaaatgttt ttgagcaatg ccttctcctt 29640
   tttctccttg agttctgagt taggtgaaag agagataagc atcttccgca gtccttcatg 29700
   tagcctctag actagttaga acagacaaac acacgaattt tcaaataaag tctgctcagc 29760
   tccattaaga accagggaca aggactgcac tggaaatgca gggtgtcact ttcaagtctg 29820
   cagcagaact aggaagggta gccaagggca actgaaaatg ccacaaactt tttctactat 29880
   ttttatgttg cttttttctt gattcaacat tcacttactt cctgtatgct tttgaccatt 29940
   ttctggggct gtgacaaagc ggtttcttat aattctgctt gctttttgat gtttttgtgg 30000
   aggaacaggt gtttcgagcc acttgctcca tcagtttgct tacctcacct atccaaattt 30060
   atttttgatc ccagaattta attctatgat agtgattatc agttttggcc tcaactttca 30120
   gccactttat aaaattgggc cttttgacat ttggccaaaa tgtgtattca tttcacctta 30180
   ttattgaaat gggaggaatt ttcccttatc cccctagcat ggccagcgac agggacgggg 30240
   gtgtggctga gttcttcagt gccccacagc tcgaaaccct agggggacca ggcagacggg 30300
   caggccatgg ggagcatttt tgggcttcga ctctatggcg atgtctaggg ttgagagttt 30360
   acagctccca aagccccagt gggtgtgtat tacagtgtgc tctttcagtt ttgccgtcca 30420
   caggtggctt gtgttaaaca gctcagttag accctcgctt tatcgcaagg acagagggat 30480
   ttctgtatct tgggtgcttg ccttagtgta ccgaaaaaat cagatcacgc ttgggcttgg 30540
   aggatgggtg caaggtttta ttgagtgctg gaagtagctc tcagcagatg gatggggagc 30600
   cagaaggggg atggcgtggg aagatggtcc ttccgtggag tctggcctct cagcagccag 30660
   gctcttctcc aaccgcctgc ccttggccga attcccctct gcattcgcgt cgttctgccc 30720
   tccatggcct gcgagcgtct gttggtgggt gtgtttctct ggaaatccag ccacttgtac 30780
   gtgcccgcta gggcctcggg gtttttatag gcacaggatg agggggtgtg gcaggccaaa 30840
   gtggtcttgg caaatgcaac atttgggaac gaaaacagga gtgcctgtcc tcacttaggt 30900
   ctgtgggcac aggcccaagg atggatcccg tggcagggac cccattcttg tctccccagc 30960
   actttcctgc agccctccca tatctttagg aactttggaa aatgttcagt cttgtcacaa 31020
   tcttgattta ttccttcttc ttccaaaaca taaatatgag gagtattaat gaggttgtct 31080
   aatttacgtt ttggagaaat taatggattc ttggcttggc tatctgcagt atttactcat 31140
   gtctgaacct taagcaaacc tgctttcaca tttatgtctt gatctgtagt ctaacacttg 31200
   actcaattta ataatgccca aaagaagatt aaaatgctct ttatttgata aaatagtaat 31260
   ttctagcata atgatttcaa gacaaaattt ttaaaaatgg aagtggtaac aaaaataacc 31320
   ttgttgagtt caaacccgat atagtttcta agaccaacaa acaaaaaata gtttttaaac 31380
   ttcttcattt ttaaaaatag aaagcttatt tacatttata atataagaaa cattcttgaa 31440
   ctgaagtgta atgcaaatag gaacagtggt tatgttttag aggatgctgt cacttgagta 31500
   gtttcaccca tcagacaaag aaacaaggat ggcagaaaac acagcaccct tttcatgtgt 31560
   aaaatgtgac ttgtcacctg tcaaactctg aatgataatc tggaactcaa ctagttggca 31620
   atttgaaaag tacacacaaa tatttatcca atatggcaca caaaacaact accattttgg 31680
   agagccttga ggcagtatct cattaatttt aagatgtgca tacattacta tctagaaatg 31740
   cccacagtta ggtagctacc ttagaaaaat tttcaaatac ttgtcacaag gacacatgta 31800
   acacaatgtt cttttcagaa ttctataaca caaaataatc atagaaaatc aaaattatta 31860
   caattagaga aaaaaaatta aatggttgct cttctttcca tccatgagtt taaaataata 31920
   tatcaaatta ataagcatag ataaatgaat gagatctaca tgtattaaca ttgatataaa 31980
   cattacactg agtgaaaagt tgtagaaaga tacatttcat cagttacata tatttgagaa 32040
   acacaggaaa tactatatat tccctcaaat gtttttgagc aatgccttct cctttttctc 32100
   cttgattgag ttctgagtta ggtgaaacag agataagcca tcacttcttt tttcatccta 32160
   gggtgaaatt tgatatcacc ctatgatatt tgttatttga acaataagaa tattttttca 32220
   taagtatttt ctttagttgt ccccaaataa gttaatatta caaatataga ataggcttgt 32280
   ttacattaaa aacacccttc tccatcaaag aaattgtgat ggttgtactg ccctttcacc 32340
   ccttaggtgg ttgcactgcc ctttcacccc tccttcctac tcccaccctg gataagccac 32400
   agctccgggg cacgtgagag taatttctta taagatttct ctgtgataga cttcagaaga 32460
   caagttatat actcatgaga ttctgaaatt gtaaaatatt agtgccaaaa atccctcaag 32520
   aactgaatta tcttttacaa cacctcaatg aaaagactaa gaactgaagg ttagtggggt 32580
   gtattcacct ttctagggtc acataatgaa gcagggcatg taatacacag caataaagga 32640
   gaagaaaggg attaaatgct gaagtaagtg gtgaggttaa caattctaaa aaaggcagaa 32700
   acaaaattga tttgagttgt ttaaaggtta cttaacattt catgtaaact ttcatagtac 32760
   agaaaactat aggaaagcat tccaaatttg cggaccacag tctgataaaa gaaatgaata 32820
   gcttattctt aaacaagatc tgtttggccc atctaggaag ttatgctggg aaagacatga 32880
   aattaacacg cattaagaga tagaccctct atctgtgcaa tgtttcttct ctcataatta 32940
   agaaaaatcc actgaattca tgccaggaag tctgtctgac cctgatgcct accttactgt 33000
   caccataaaa cactgtcttc caggatgttg gggaatggag agattgaatg ggtgtacatt 33060
   ttgggaaaaa aaacttcaaa ggcaaatatt ttgtgtgcgg ctttaatgct aagaccagca 33120
   attagctatt gaggataatt ataccatggg gctaacttct atgacattta aattcgtttt 33180
   cattgtgaat catttcagcc atataaaaaa gaatagcaat ataaaaaata atccatgtgt 33240
   ttactaatat gatttatgaa atgttgacat tttgctgtat ttgtttcatg ttgtttttgt 33300
   ttatcttaga agttttagat aagataaaag ctcctcctct ttttgttccc catctttcag 33360
   tcccagaagt aaccatttcc tgaagcaact attttggtgt gtggcctcct gaagctactt 33420
   cattctttac aacatgtcta ccctatatat tcttatgtgt ataaagccta tataatattt 33480
   cctgtgtttc tcaaatatat gtaactgata aaatatatcc ttttgcaact tttcaatgta 33540
   atggttatat caatgttggt acatgtagct ctcattcatt tatctatgct tattaatttg 33600
   atgtattatt ttaaactagt tgattgaagg aatagcaagc atttaatttt tttctctaat 33660
   tgtaataatt ttgtttttct atgatttttt tattatagaa ttctgaaaag aacattgtgt 33720
   tacatgtgtc cttgtgacaa atttttctaa ggtagctacc taattgtgga catttctaga 33780
   tagtaatgta tgaacatctt aaaattaatg agatactgcc tcaaggctct ccaaaatggt 33840
   agttgttttg tgtgccatat tggataaata tttgtgtgta cttttcaaat tgccaactag 33900
   ttgagttcca gattatcatt cagagtctga caggtaacaa gtcacatttt acacatgaaa 33960
   agggtgctgt gttctctgcc atatttgttt tttttctttg tctgatgggt gaaactactc 34020
   aagtcacagc atcttctaaa gtatacccac tgttcctatt tgctattttt tgctttttgt 34080
   gatacaaaaa gaaggggtag gaatttgcta catgaccttg ttccatgcat atttgctaca 34140
   tgagtcacat gtacacctgt gacctcctgc aagaatgcaa tcagtaccag agaaagaatt 34200
   tgccctcaaa cctcctccat gagctgataa agattcctga gtcctactga ctgcttgaat 34260
   tgtctaatgg ataagatgta gagtcttttt agtggactaa ttggtatcct gtcacatgtt 34320
   aaagataccc aaaaatatga attgtccaaa tatactagaa gtcatttagt cattgaaggt 34380
   ttaccaccca gaggccaaaa tccattcaag cgtcaaacaa ctgtgtctca ctccttcaat 34440
   tttaccctga tggaaaatgg atcctgctga tggttatctg aggcaaactc tatcatgact 34500
   ggttaataat aatcatattt cttcttggta tacctttacc aaccaattaa gcccttttct 34560
   gtcagcgaag aaagctgaac tgtgtctttc atttgaacca catagcccag gaggtgcaaa 34620
   tcttctcctc tgtcactaga ctctgagatc tctaagtgat atggacaatg aagacctatg 34680
   catgtgatct ttccagtcaa tctcaagagc tagaatcaag ttctgccata cctagccact 34740
   ttgtgtcagt atgaaaaagg tgtttcttcc atgtgaaagg agcgctgttc agggtgtatt 34800
   agttagaggg aagagcccag gctgggattg ggcccactaa gccccttatc agggcagccc 34860
   attgcagtca acacccgata tggccataag ctgcagctgt ctgctaacag ttggctccct 34920
   ttgttattgt gctgttccag agagggattt gggaaagggc agtaagagac acaaactcag 34980
   attttcatct tctcacaatc ctctccaaca tgtttacatt gtttatagat aattttaatt 35040
   aaaggggatt acaatgcaga taaagtaatt ttatatccta ttttgtaggg ttgtattgta 35100
   ttggttccat tttctgatgt cattaaattt caataaatgc aaatcttgca tattttattg 35160
   tatgaataga tattgatcat aagacttatg tgactagagc tctgttttaa agacttttat 35220
   ttccagtaat ttttagctta gtacataatg ctctgataaa cttttttatg catacatttt 35280
   ggtctgcttt tcagactact ctctcattct ttcattttgc acatatatat tttgtgtgtt 35340
   ttatgggaca gaaactgttt ctagcacttt tgatacacca ataatgaaga attaaaagga 35400
   aatatgggac tctagaaatc taaataattt aatgttcttt acaaatattg gcagatttgc 35460
   acctatgatt ttaaagttag agatggtctt caagcactac acattttgaa gccttgagat 35520
   taatcactga ttgttggagg tgggatccca agacctgtct cttctttgct ctacaataca 35580
   aaatattaga agttactttc aaaaaactat tcgtcaaagg aaggtagcaa tatcagcttt 35640
   attgagatca aatgactgca gagcacgatt tccacctgag atccacactg ctggttctag 35700
   tttctcccca ctgacaagaa agacctttga agaagcagct gaagaagcta ccagaaggca 35760
   cagatcctgg taatattcct gtgctagcca gaagctcaga aggcccaagc ttcactcatc 35820
   cataaaacag gcaacataac agaagacgca gcttgttcct tacacagaaa tcctatgcac 35880
   cctcaccagg ttccaggatt tatctcgctc tttccagtca aattatctat agctccttcc 35940
   agcttcctcc attcagagaa agaataggcg gcctatcttc agaaggaatg tgaggaggag 36000
   tatttaagaa aagttagtcc tttggagaat attgaatgaa agttataggg agcaatatgg 36060
   cttcccagaa ggggtaaggc aatagggctc tgattcctgg acacttacta tctatctaac 36120
   cttgaaaaaa ttactcaaat tatcagaact tagagagtga gaaataagac actcatttca 36180
   tagtgttgca tgaattaaaa gagataatat tgttgagtaa ttttacctcc ctacgaagtt 36240
   gccaattcat tgacagagag aaaactaaac acacacacac acaaaacaac aacaaaaata 36300
   gttgtttgat acgttacctc attgaatgtt atagccatat aggtcaatgg ttaaattaga 36360
   gatgctttgt atatattctc attactaaga cagtttacta gatcttatgg ctcccatagt 36420
   cccaatatac ataaatcagt cacacataat tgtccacata tgctccacat tattacacta 36480
   acatgagctt gcttatttta tttgtatgtg atattaacag atttgtttct tacttgatag 36540
   gccttgaaga tgacttagtt gttgagttca gtacctgtgt ctgttctacc gtgcactgaa 36600
   tgttactgac ttgtacagaa atcatgctca aggtttggtg tccacactga ttgtctctag 36660
   aagattaaaa tatatataaa tgacttatct atatttacta cttttattta tggaaaaatt 36720
   aaattcttta tagagtctta tggaagcttg ctctgataaa atttttccat aactgtcaaa 36780
   gtggaaagta tgacgagttc aggattagat tagaatccta atggtattat ggccaggaac 36840
   ccaaatattt ctgaatcctt cctgggatat cacctttttt aaccatagga taccagagag 36900
   agttgcaaaa cagaaaaaaa ataataatgg cataagtagg aatttggaat gtagtttatg 36960
   tgcagatttc tgtcaaaagg ctaagtggac gctcatgtct tagcctagaa gcttcattga 37020
   tgcctccaaa tctcctatcc caggatccta aaatctggca tgctgcaaga aacaagacac 37080
   tgaaaaaggc ttggagtcta cagaaaaacc ttatgaatcc acacaactac ttctgatgga 37140
   aaataagaca agacagagat tcactaagcc cacctgagtt gtcaagcatc acataccaaa 37200
   gaaaggcaga aactggaagg caccaaaaag actgtaaaaa gcatttggtt tctgactgta 37260
   agacattgag acactcattt gttaggagca actcctcagc tcttggcgag cagtggggat 37320
   gaaagcccag aaaggtagaa gctcgctggt ttgtggatag ggttagaagt gttaaccttt 37380
   ccctatattt ccctctcctt aggcaccttt taagcgatta gacttaaaaa aaaaaagttc 37440
   agtgaaaggg caagcagaga ggtgggaccc accttaggat aggaaagaaa tagtcaaagc 37500
   tgctctctcc ctcctgctgt aagctacttt ctggggccct ctttggcatc ttggaagact 37560
   aagatgggta agttgtttct ctaagaatcc acgaaataga tcagaacttg aagttaattc 37620
   caaagaggca atgcacatta ggatgaacaa ctattcctct tgaatttctc aactcatgtt 37680
   ataccttgtt atgtccttta ccctagttta ccagaagggc acattaggag tacatatatt 37740
   ataaaaccat gatacaatta aaatgacatg gagtcaaaca aggaaactgt ctttcttcca 37800
   gagaaattac atttcaaatt gaaatttcag aggtgacatg aatggaaata ttgtaaaagt 37860
   gatattgcta tcgtcttttg gccatcagtt ttctgaatat gccttctaat atttagatcc 37920
   ctggagtgaa agaggggaag tcttgggaac tcaaactcca ataaattaga ggtcaattat 37980
   aaggccatat aaatgtttgg tgcttaaaga gcacctcact aaaaaatgat aggagcaggc 38040
   cagatgtagt gctcacgcct gtaatcccag cactttggga gaccaaggca ggcggatcac 38100
   ttgaggtcag gagttcgaga ccatcctggc caatatggtg aaactctgtc tttactaaaa 38160
   atacaaaaat tagctgagcc tgttgatggg tacctgtaat cccagctact tggggggctg 38220
   aggcaggaga actgcttgaa tctaggaggc agaggttaca gtgagccgag atcgcatcac 38280
   tacactcctg cccaacaacg aagcaagact ccatctcaaa aacaaaacaa aacaaaacaa 38340
   aacaaaacaa aacaaaaaat ctggtgtaaa atacctccaa aaagctgctc ctttataaag 38400
   gcaatgagta cactggaaaa gtagtaaata aactttttca gaactcaaac taagcaaaag 38460
   cttgcaaaca aaaaaatgaa gttcttattt aaaaaaatag actgaatctc agtaaaaggg 38520
   aactttgtga tgttttacct tatactattc tcagttccct ccctttcaga cccagaatag 38580
   ctgttacaga ttgaattaag tggttttccc caccacaatt catatgttaa accctaattc 38640
   ttaatatgat tgtatttgaa gatactgcct ttgaggaggt gactaaggct aaatgatgtc 38700
   ttaagtatag ggcaccaatc caatagaact agtggtcttt taagaagaga aagaggtacc 38760
   agagctctct cttcctctgt gtgtacagag aggaaaggcc atgcaaggac atagcaaaaa 38820
   ggcagccatc tacaagccag gaagagaggt cttaccagaa accatccctg atgataactt 38880
   gatcttggac tttttgtctc cagaaccttg agaaaataaa tgcatgttgt ttaagctatt 38940
   cagcctatgg tactttttaa tggaagcctg aataaatgaa tacagaagcc tttaaaacca 39000
   acagacttat aaggcagagt tgtaagctgc ctgcctcagt attgaagtca taccccaaca 39060
   tggacacaaa cactaccgtc aaaggcagca atgcttatta atcctaaaca tttaagaaaa 39120
   tctttggctg gtcagcagtt gaccactaag ataactgggc aaaaactttg gtgaccatac 39180
   aaagaaaata aaaaatttac agaattcttt cctgacaagc aaacaataac agcaacaagc 39240
   agcaacatca aactcagtgt ggggaatgga aactgacttt cagagtttcc atattatatt 39300
   attttaaatg tccagttttc aacacaaatt atgaaatgta cacattaaca agaaagtgtg 39360
   ccttatatga ggaaaaaaaa atcagtcaat agtgtccctg aagatcccag acactggact 39420
   tgttagaaag catttaaatc aactgtttta aatatgttct aagaactgaa taaaaacaat 39480
   catttctaag aaaatctagg aaaatatgag aacaatattg caccaaataa agaatatcag 39540
   taaattaata taaataataa aaaagaaacc aatagaaatt ctgtaatgtg cagtaattga 39600
   aataaaaact tccccagagg agctcaacac agattagact tagcagaaga acaaataact 39660
   aaatatgaag atagatcagt tgagattgtc catgtgagga acagaaagaa aaacaagcaa 39720
   ataaacataa acagagctag acatctgtag tacgccccca atcacacaaa catatgcatc 39780
   atgagagttt cagaagagaa aaaagaaaga ggtagagaga atatttgaaa ataactgatg 39840
   tccctaattt ttccaaacat taatcttcac agccaagaag ctcaacaaac tctaagttgg 39900
   ataagttcaa agagagactc ttcaaagcag caagggaaag caattgataa gataccatta 39960
   ataatattaa tagacattct cagaaaccat agaggcaaga atagagtaga atgacatgtt 40020
   caaagagcta aacaaaatta atctcaatca gtatttccat atttcagcaa aagtatccat 40080
   caaaaatgaa aaaaaaaatt attatcagta aacaaaaatt gagataaact gttgccagaa 40140
   aatgtattct aaaggaaaca ttaaagagaa tccttcaggc tgaaatgaaa cactaaacag 40200
   taagttgaat ccatgtgaca aaataaataa caccaatata aataagtgca caggtaaatg 40260
   taaaatacag tatcaatgta ttttacattt acaagtcatt tttcccttct attttatttt 40320
   caaaaaattg tataaagcaa taattctaca ttgtgtttat aggcatatga tgaataaaga 40380
   cacaacttgt atgataatta cagcacagag aaggaggaag aaaatgaagt tatataagag 40440
   ccaggttttt gtatatgaat gaagtcagtg ttcatctgaa ctaaattgtt gcaaattaac 40500
   gtgttaattg taatccccag ggaaaccact gaaaaaataa caaaaataaa cagtaaaata 40560
   aattacaatt tattggttag agctacagag aaaaaaaaga aatgatagtg atattaaaat 40620
   actacagaga caaatatcta tttaacagaa aagacagtaa taaagaaaag gaagaagaaa 40680
   aaataagaca tttgacaaat aaatatcaaa ttggtatata aaggagaatt ttcagtaact 40740
   gtattaaata aaaatgggtt aaatactcca accaaaaggg attgacaaaa tattaaaaca 40800
   aaaacaaacc atagtccaca acatgctgca tacaaaatac tttagattaa aagatacaaa 40860
   taggttaata gtaaaaatat ggagaaagat atactaaacg aacagtaacc acagagctgg 40920
   aatggctatg ttactcacag acaaaacaga ttttgggata aacattacca aatacaaaga 40980
   acattatata ttaaaaaagc atcaatccat cagaaatata taacaattat aaacatacat 41040
   gcccataaca aagggtccga aaatacactt agcaaagcat gacagaattg aaggaataaa 41100
   tagacattca acaataatac ttggagagtt gaattctcac tttcagtaat ggctagacag 41160
   caggttagta aatcaacaag ggagaagaag gtttgaaaaa cattattaag caattggacc 41220
   taacatacgt ctacagaagc ctccaactaa caacagcaaa tgattttttc tcagttgcac 41280
   attaatattc tccatgatag accttatgtt agaacacaaa acaagtctta ataaatttta 41340
   aataatttat actacacaaa tatattctcc caccgtagtg gaatataatt actaattatt 41400
   aataaatgga aaattggtaa attgccaaac atgcagaaat ttaaaaatat atttctaaat 41460
   aaccattgtg tcaaggaaga aatcacaagg ggaattagga aattattgga gatgaaaaac 41520
   acaacatata atttatggta ggcagctaaa gctgtacata gagaaacact tataactaag 41580
   tatctataat taatagtaac tgaaatcaat aacataattg ttcacctttc aaaactaaaa 41640
   aactagaaac aacttaacaa cccttgctaa caaccttaag caagcagaaa gaagaaaata 41700
   ataagcgtat gagagaaaaa ggaatgaaat agagaataga aaaacaatgg aaaaataaaa 41760
   aaaccagaat ttagttcttc aaaaatataa acaaaattga taaatgttta gctgggttag 41820
   attaagaaca aaaagagatt aaccatgtag ctggatcaga ttaagaacaa gaagagatta 41880
   aagttaataa aacttggact aaaagaagga atattactgc tgattaggaa aacaaaaatg 41940
   ataatatgac atactataaa taattatatg cctactgatt agataatgtg tatgcactga 42000
   aaagattcct agaaaaacaa accactaaaa ttaaattatg gaagaaaaac atctgtatag 42060
   acttttaaca agaaaatatg tagaattagt attaaaaaaa aacctttcct cgaagaaaag 42120
   cctggtccac atgattttac tgatgaattc tatcaaattc ttaaagaata agcatcaata 42180
   atcttcaaac tacttcaaaa tatagaaaat cttcttattt tattctatga ggctagtatt 42240
   atcctgacat aaaaaccata caaagtcatc acaataaaat taaactatac attagtatcc 42300
   cttttgaata ctgacacaaa cattctgaca gaatacttgc aaactgaatc cagcaacata 42360
   gaaaaaaagg attatacaat attaccaagt tgggcttatg ccaggaatgc agggttggct 42420
   cattctatga aaatcaatgc aggtaatata acatacaacc aggctaagga acaaaaacca 42480
   tatgattatt tcaactgatg cagaaaaagt actcaacaaa atctaacact cttatatgat 42540
   taaaatataa aaataaaaaa cactaaacaa tctagagata caagtaatgt ctttaacatg 42600
   ttaaagggct tttgtgagaa gcccataatt aatgtcaggc ttcttgaaga ctgagtcctt 42660
   ttcttcctaa gatcaagaat cagacaaaga tgcctgcaat gtctgctcct gctatttcca 42720
   tttaatgttg aagtgtatgc tcaggccatg acaattaggc caaaaaaata aaaaaagaaa     42780
   aagaaaaaaa agagaggaga ggagaggagg ggggagggga aaggaggacc agggagggga     42840
   ggggaggtgg ggagagggga gggcagggga ggagagaggg gaaaggaaga gaggggaaag 42900
   gaaggaagaa aggaaggaag gaaaaggaag gaaggaggga aggaaggaag gggaaggaaa 42960
   ggaaaaggaa ggaaggggaa ggaaggaaga ggaaggaagg aagaaagaag gaaagaaaga 43020
   agaaaaagga aggaggaagg aagaaaaagg aaggaagaaa gaaggaaaga aagaacgaag 43080
   aggaaggaaa agaaaggaag gaaggaagag gaagttaaag aaaaagaaag aaaggaagga 43140
   agaaagaaag taagagaaag taaaagtaag aaagagaaag gaaggaagaa agaaagtaag 43200
   agaaagtaaa agtaagaaag agaaaggaag gaagaaaggg aaaggaagaa agaaaggaaa 43260
   gaagaaagga aggaagaagg aaggaaggaa agaaagaaag aaagagaaag aaagaggaag 43320
   gaagggaagg agggagggaa aggaagaaag gaaagaaaaa aagaaagaaa aaaaggaagg 43380
   aagggaaagg gagaaagaaa ggaatgaaga aggaacaaag gaaagaaaga aagagaaaga 43440
   aagaaaggga aggaaagaag ggagggaaag gaagaaagaa agaaagaaag aaaagaaaga 43500
   aaggaaaagg aaggaaggga tggatggagg gagggaggga ggaaggaagg aaggaaagga 43560
   gggagggagg gaaaggaaga aaggaaggag gaaaggaaga aagaagaaag gaaggaaaga 43620
   aagaaagaga agaaaagaaa agaaaaaaga aaggaagaaa ggaagagcgt ccaaattaaa 43680
   aataacattt aaaataatat ctatttgtag ggtacatgat ttgcataaag aaaatccaaa 43740
   ataacttaca agagagctaa caaattaatt cagccaggtt gcaggataca atatcaacgt 43800
   aaaaaaactt aattgtgttt ctacactatc atgaaacatt tgagaaatta aggaaatgat 43860
   tacatttgca gtagcagcca aatagatccc ttagtaaatt gtgcttaggc agctggatat 43920
   tcacttgcaa aagaattcag ttggattttc atctcacatt atgtacaaaa ataaacttaa 43980
   aattgactaa aatatctaaa cataaaagct aaaactataa acatctttaa aaaacagaac 44040
   cgacccaaat gtccatcaat gatagactat attaagaaaa tgtggcacat atacaccatg 44100
   gaatactatg cagccataaa aaatgatgag tgcatgtcct ttgtagggac atggatgaag 44160
   ctggaaacca tcattctgag caaactatca caaagacaga aaaccaaaca ccacatgttc 44220
   tcactcacag gtgggaattg aacaatgaga acacttggac gaaggttggg gaacatcaca 44280
   caccaggacc tgtcatgggg tgggggaagg gggagggcta atattaggag aaatacctaa 44340
   tataaatgac gagttaatgg gtgcagcaca ccaacatggc acatgtatac atatgtaaca 44400
   aatctgcatg ttgtgcacat gtaccctaga gcttaaagta ttaaaaaaag aaggggggaa 44460
   aaattcatga ccctgacttt gacaaagttt tcatagatat gataccagaa atacaaggaa 44520
   caaaagaaaa aaaatagata tgttgtgctt catcaaaatt taaaactttt gtaaataaaa 44580
   agtcattaac aagagaataa aaaggcaatt caaaacatgg gaaaatatat ttgcaaatta 44640
   tatatctaat aatggactag catccagaat ctataaagaa atcttaggac tcaacaaaaa 44700
   gagataaaca accaaattaa aaatggacaa aggctagaat gaatgttttt caaaataaga 44760
   ggtaatgaat gtcccacaaa caaattaaat gatgttcaac atccttagtc attagggaaa 44820
   tgcaaatagg gaccacaata aaatgccact tcacacttgc taggttgctc agaatcaaaa 44880
   caatgaaaat ataatgcata tgatcaagca tgttgaaaag tcaaaaccct tgttccttgc 44940
   tggtaagaat gtaaaactgt gcagccactc tctctggata acagtttggt agttcctcaa 45000
   aagttcaaac ataattttcg tattacccag caattccact tctaggtaca taccaaaaag 45060
   aatttaaaat agttacggaa atgcaattgc atatattttc atagcagcat tattcacaat 45120
   ggataaacga tgaaaatgcc caaatgccca tcaactgatg aatgaataaa atgtggtata 45180
   ttcacacagt ggaaagttat ttagccataa aaatgaaggg agtactgata acgtgctata 45240
   acataaacaa gccttgcaaa aattgtgtta actgaaagca gacctacaca aaatatgatt 45300
   ccacttgtat gaaatgacca gaataggcaa atgtatagag ataaagagca agtagctaat 45360
   gtctgtggat cggcagagtg gcgagtgatt gtttaataag tgtgggtttc gtttttggag 45420
   tgatgaaaga gttttgaaac aatgctgttg agggtcacaa aacattttga atatactaaa 45480
   aaaccctgaa ttttacacct tgaaatgtat aatatataat ttttttccca atgagaagaa 45540
   ataaaaattt gaaaacatca acaaaaaatt cctgactatt gctaccaaca atagcaataa 45600
   attttcagct ataaatttac caaaagaaaa tagaaacact aaattagtag aaagaaaaat 45660
   taaaatgagg aaaacagtca accaagttct cttagtgctt tggaattacc taggaaagta 45720
   aaggaaagag atgcctaaat tttaaccttg tatgtggtta cacttttctg tcattggagt 45780
   ttagatggca ctggtaagta aatcaattgg gcatattgat atgaactcca aaatatttaa 45840
   agccaacttt cgataaaagg gtaaaatcag agcattaata cagatatgga cattttaagg 45900
   gaacatgaat attgatcaaa atattaactg aatatataag taggggaaat tgattttgtt 45960
   acactgtgag gagggtggac aattgaatct ccactggaca aaacattgtc tgtcagtgac 46020
   ctatgactta tagcatataa aggaactcaa agacaatgaa agtctagtat caggtaacct 46080
   gaagtggtgt cctctagacc agataaattt ttctattggg acttcttttt ttctacacta 46140
   ttctacaaca tcaaatagaa agttagactt ctttctaaaa gagggttcag ccattagagt 46200
   gacaaagtgc ctatcacaaa catttctagc cccaggcctg atcaggggcc accaatgaca 46260
   ttcctaaatc taggtgtcag aaagcctact ccctaaccaa tgaggggctg acacatggag 46320
   atacaattgt taagacagaa ctggcatatt gagaagcccg gggcaactga aaaatcaaag 46380
   tgggacaatt ggagcaaaga ccatattaga gagaaggagc catcagagtt tctagaggag 46440
   tggaaattaa atctgcttct acttcataag agatgatctg taagttccta gtgagccaca 46500
   tggcctgcct cctgaagaag aatcattatg cctagcactt gagctcccag gagggttttt 46560
   cttgctgcca atctcaagca aatgaagcca ctatcctgga tttggcaaga atggaagctc 46620
   acaacccctg cagcaggatg tgtagacatg acagtgcaaa ataatccgaa gattggctgc 46680
   ctcttgaggg atataaccat ttctctggtt aatgaacttc tgatccagga aaagtttctg 46740
   atttctgggt cctggagctt tgggggaggc ctcacagact catccagtaa gtactgtcaa 46800
   agagtttaaa tatatgggct tgcttctgac tcctcacaat tctagtcttc tttaattgga 46860
   agtatctatg agacatctgt attagtccgt tctcacactg ctgtgaaaaa atacaagaga 46920
   ctgggtaatt tataaagaaa agaggtttaa ttgactcaca gtttcacatg tctagggagg 46980
   cctcacaaaa ctttcaatca tggtggaagg cacctcttca cagagtggca ggagagagaa 47040
   ttgcagagca aagggggaaa agccccttat aaaaccatca gatcttgtaa gaactcactc 47100
   actttcatga gaacagcata agggaaccac ctccatgatc taatcacctc ccacgaggtc 47160
   actcccgcaa ctcttgggga ttacaattca agatgagatt tgggtgggga cacagccaaa 47220
   ccaacatcca agcaaagata tcccaaagag atctggaggg ttagaaggtg gtggtcctgt 47280
   gaggtgttcg aatcagtaat gtcagaaagg aacagtttct tatggtgaca cttgaggaag 47340
   tggccttagg taggaatgtc tggctgaggt gtcaggataa agaagaccac ctcattggcc 47400
   ctgaggaagc caaggtcttg tgaggccagg atgttggctg gaggcctctg gggtgtgttg 47460
   aatttcctag agtgatggta gtaactgagg tagaactgga gatcttggtg gacttgctaa 47520
   caactttatt tagtatagag aatatctaca ggtaagaagg tgttggcaat ggggatgagg 47580
   tggagaaggc agcatcgcag ttggcatgga cttaaatggg gcaggggttt ttacatgaag 47640
   attgaagggt gataagtgaa acaaaaaaga aatacataga aaagaaagaa aaggaattga 47700
   ttccaaatac atattataac atacgtgtta cggactgaac tgaactgggt atactttgtt 47760
   atggcaacct tgctaactca cacagattct ggtatcaaaa gggaggattc tgctgtaaca 47820
   aataaccaaa aatctagaag tggctttgga actgggtaga gaaatccatc tcatttggat 47880
   gtatgttaca caaagcctaa attgattgga agagagtatt gttaaaaata tagatgctga 47940
   atgtgattcc cgtgagtcct cagaaaatta gagagctgta gagaaatctt ctagcatctc 48000
   ataaaaatac atgtacagtc attaacaaaa tgttgctaga aatatgtatg ctgaaggtgc 48060
   ttctggtgag gtctcagagc ttaggaatat gtaattgaaa actggaggaa aggagattct 48120
   tcttacaagg tggcaaggac ttggctgaat tgtattctag tgttttgtgg aaaatagatc 48180
   ttgtaggtga tgatcttgaa taattcactg aggggatttc taggcaaaat gttgaaggca 48240
   taacttgatt tctcttcgct tgtatggtaa aatgtaaaag gagagataga ttgaagaatt 48300
   gtcagcaaaa agataccaga gcttgaagat ttgagaaaat ctcagcctat caatattgca 48360
   gaaaataaga aattgtgtcc tgaaaaatat caaggatagg gctggataat gatttgccaa 48420
   aaaaataatt ataggtgtgt gacttatgat tccaatcagc tgtctcagct gaaaccatga 48480
   atggagatgg ggatttatac cagtagaagc actgtcaggt tgactgaagg agagaaagat 48540
   gaaatgaaat gaagcaaggc tgttggactc ctgggatttt atagcatggg ctaatacaga 48600
   cacgtggttg tgaacatatg ctatctttca tgaaaaagga atggctgtga aggcagttca 48660
   gaggttgttg gggctgccac tgtcaacgtg gatcctcagg tctgagagtt ttggggtagt 48720
   gccaccacac tggtgggccc aaaaggcagc gttaggacta ggatgggctc tgaggacaca 48780
   gcatggagtc aaagaggatt attctcaagc ttcaaaatcc tatggaattt tccttgctag 48840
   gtttcagact tgcttgcaac ctgttacccc tttattcttc ccaattgctc ccttttgcaa 48900
   tgggaatgtt tacctcatgc ctattgctct aagatgaatt ttgtctcccc aaaatccata 48960
   catcaaaacc ctaaccccca ggatctcgga atgtgactgt atttggagat aggtacttta 49020
   aagaggttat taagttaaaa agacagttaa gatgagccct catccaatct tactagtgtc 49080
   cctgtaagaa caagaagcga gccaggtgca gtggctcatg catgtaatcc cagcactttg 49140
   ggaggccgag gcaggcggat cacaaggtca ggagattgag accaccctgg ccaacagggt 49200
   gaaaccccat ctctaccgaa aatgcaaaaa ttagctgggt gtggtggtgc gtgcctgtaa 49260
   tcccagctaa tcaggaggct aaggcaggag aattgcttga accagggagt cagaggttgc 49320
   agtgagccga gatggtgcca ctgcactcta gcctggtaac acagcaagac tctggcaaaa 49380
   aagaaaagaa caagaagctt ggagacacag gaaggtacca gagatgtgca agctcagaga 49440
   aaagaccata taagaacaca acaagaaggc ggccacctgc aagccaaggc aagaggcctc 49500
   agaagaaacc aaatctgcaa aaacctcgat cttggacttc tagcctcaag aactaaaaaa 49560
   gaaaataaat ttctgttatt taatgttatt taagtcaccc agtctgtggt attttgttat 49620
   ggtaccccta gcaaactaat acacctacca ttctatttta gaagtagata actcatcaat 49680
   ttcacaggat cacagctgga gagcaatttt gccccaggaa tcatgactca agtctcattc 49740
   atacctgatt tagacagtat ttatatgaga ttttggactg agatttgatg ttgcaatgag 49800
   ttgacttttg gggctgttgg gatggtgtga aagtattttg tatatgagaa gacaatgaat 49860
   ttgcagagtg gcagggggtg aagtattatg gacagaattg tgttctcctc aaattcgtat 49920
   gctaaaattc taaccccagt accttagaat gtgactgaat ttggaaatag ggattttaaa 49980
   gaggtaatta agttaaaatg gggctgtgtg gacactaatc caatattact tgaatcctca 50040
   taagaagaag agaatagaaa cacacagaga gaagaccatg tatggagacc agaaaaagat 50100
   ggctatctcc aagccaagga gagaggcctc aggagaaaca accctgctga caccttcttc 50160
   ttagactacc agcctcctaa atggtgagaa aacaaatttc tgttgtttag gtcatccagt 50220
   cttcagtact ctgttgtggc agtttaagca aatgagtaca atgggtgaat cttgaaaaca 50280
   ttatgctaaa taaaagaagt catatgcaaa agaccacata ctatatgatt ctgtttatat 50340
   aaaatgtcca gaaaaggcaa atccatagac ttataaggca aatcagtggg tgtcagaggc 50400
   aagggagtga gcacttagga gtgagtgcta atgggtatgg ggttcctttc caggatatga 50460
   aaatactctg gaattatata ggggtgatgg ttgcacaact ctgtgaatgt actaaacact 50520
   actgaattac acacttacag aaacaaattt tatgatacaa tgtatctcac ataaaaaaat 50580
   gaaatcacag aatttatagc ttcaaagttc acagagctta aatacttcct ctgacgactg 50640
   ctgcttggtt aagcaagaag cgttgcaccc ggtggcttga tggggtctga gccattcccc 50700
   tttgcaataa gaatagctcc ttccatatct ccaaagtaga tctgaggcct tgccacacag 50760
   gtgttccact tttctaaaat tctagtgaat caaaaagata tataaacata tcttaaaatt 50820
   ctcagcaagg aaagtatttt atccatagca atatgtttcc agaatccagt ttacattact 50880
   tacatccata ctgctgttac aaacaaaaat cagtaacaga ttcttacaca tttacataaa 50940
   gaaattagaa ttgagagaat tagaatggag taagcaagtt gagtctcttc tgttcagcat 51000
   tttctcccac aaggatgtct ggcattccag acaaattgtc tggagaaact gttaccttta 51060
   actgagttgg gataattctc ccatccagtg tcttttgttt agctgaagga ttttcctaac 51120
   cctatcagag gcccaagagc aacttagccc cttcctgctg ctgcagacct tccctagact 51180
   gggcattgat ctttgccata tgattgtccc ctaaattgtg aagaagggta aggcctttag 51240
   ccttgtgctg tctctgtttt tggaacatct caatctacat ctttagagga ccagagagaa 51300
   gctgtgacca ccctctcctc catcgttttt tttcttttgt taattgttct tattcctctg 51360
   gttttatttt atcttatcta catcaccaat gacataaaaa gattctttca atctcaaatg 51420
   ttcctgctac aacaggattt tgatttgatg ctcctcagca ctggctgaga aaacacaatc 51480
   tgcagcggcc acatatattc cccaaagctc tgcccttttg agttgaaccc caactttggc 51540
   ctctccatgt gggactctgt ctaaggaatg aaaggttgct ttgggagttc aaggtctcca 51600
   aaagcatctc tcctatcttc ccccaaattt tctattcaag ccaagaccaa gtcataattt 51660
   gctttgtcta agattatgaa cggaccttgt cttacccaca ggtcgattgt ctttgataat 51720
   aacactagtt atagccacac tggataactc aaactatcaa cataatatct gccaaaacaa 51780
   tcatggtact ttattggtat ccatagagtt tctttcctca tgagatattc agaacccgct 51840
   ggctagtacc tgtgcaaaac ttactctatt cctgcttcta cattctatga acactccaaa 51900
   gtggacattg ctaatcagat tggtcctaga tatggagacg agataattag aagagggatc 51960
   ccttcacttg aggacagaag ttcgagacta gcctggccaa catggtgaaa ccctgtttct 52020
   actaaaaata caaaagttag ctgggtgtgg tggtgcacac ttgtaatccc gctacttggg 52080
   aggctgaggc aggagtatca cttgagctta ggaacagcga gccgagatcg caccaccgca 52140
   ctccaacctg gatgacagag tgagacccca tatcaaaatt aaaaaaaaaa aaaaaaaaaa 52200
   aaaaaagaag aagaagaggg atctctgcat tttaattgca ttttaactta ggctccctgg 52260
   ctcataattt taaacttttc atggaagact taacaagttg aaggtagcct ttcttggaat 52320
   tgtcacctaa ctttctcttt ttctttatga ttcctgtggc ttggttgtaa acggacttga 52380
   catccatatt ggcactagct gggcctctct acagacacac aggcagagtg gcaggccctt 52440
   cttaggcgtg gaatatgagc cacagcaatt cagcatttga ccagagtact gcatagagtt 52500
   ctgcagaatt attaattatt tagtgaacat tttgctgtta tttcttctga taactttttt 52560
   taaaattttg gaagaatgta tggataatca taggttaaat ttgtggaaat tattggcttt 52620
   cttactataa catcattgta atattccaca aattaaggga tttcctactt gtagttgcaa 52680
   tattctataa gctattatta gcaattttgg cctggacaat tttgaaaaac aaaatgaaac 52740
   aaaaaaacaa acttgaccaa caatgagaat tatagtaata aagaactatg tgaatatgta 52800
   tatatatgag tgtgtctcta tgaattttaa attaaatttg gctaaagaaa ccaacctgtg 52860
   caaaacttaa gctctagcaa tagcctcagc cttaatattt aaaagacact taacggaaat 52920
   ttagcaaaaa gctccagcca ttgaggacaa ttttatcaga tgcccaaaat gtggccttga 52980
   agcttatcta aaacctgctc aaaaaaatct gatgaataag gaatatggag aactttcaac 53040
   tgggaagata agacatgaag ataaacatct gtgacttaag aaagacgcaa tgtcagctac 53100
   tatggaagga ccaaggaaag tgacacccat gtaaagaaaa ggcaatttga atgtgaagac 53160
   aatggggttg tcatcacctc atgtgaaagt tagccaccta ctagcaatga tctggaagga 53220
   acaaaacaaa tcatagtcaa taaatgtgca aagataaata ttgtgttagt aactatttgt 53280
   gctttttctt cttgtcggtt gtccaaggtg tcttagcttc aaattaggat agcttatgac 53340
   aacatggtgg cagattggag taggggagga tatgcccata ccaggaggtc tggagctgag 53400
   gattctgact ttatcgaagg gctaggcttc aagatggaag atcagtagtt tttgtttgtt 53460
   tgttttgttt gttttgtttt gtttttttgt tttgttttgt tttgagacaa agtctcgctc 53520
   tgtaacccag gttgcagtgc agtggcacga tctctgctca ccgcaacctc cacctccctg 53580
   gttcaagtga ttctcctgcc tcaaccttct gagtagctgg gattaccagc acacaccact 53640
   ccacctggct aatttttttt tttttcattt ttagtagaga agagtttcca tcatgttggc 53700
   caggctggtc ttggactccc aacctcaggt aatctgcctg cctcagcctc ccaaaatgct 53760
   aggattacgg gtgtgagcca ccacacccag ccgatggtca gtggttttgt aaacagtatt 53820
   attcaaatgc agatagtaag tttagttgtg atttacaacc atatgatata taaggaggtt 53880
   caactctgta ggatacccaa aacaagagta aactgaatgt aacagtgttt tgtataagtc 53940
   agggataggg gtctctagtg aaaagtacat tccaagagca tatagaagca agtttcactc 54000
   atttgaaaag atgctttatt aaaattaggt aaagtgatgg gtttccagtt ataaagggta 54060
   gggttggcca aatatccatt ttcagtaaga atctgaaacc agtgggtatg gagcaacgaa 54120
   aatctaaagg tcaaaccaag atacagagat ttagccaaca tggcaggggg taatgaaaag 54180
   acctacaatt catggccagg tgtgatggtt catgcctgta gtcccagcat cttgggagac 54240
   caagacgggc agatcactag aggctaggaa ttcgagaaca gcctgatcaa catggtgaaa 54300
   cccatctcta ctaaaaatac aaaaattggc tgggagtggt ggcgcatgcc cgtggttcca 54360
   gcaactggag gctgaggtgg gagaattggc tgaacctggg acctgggagg cagaggttgc 54420
   agcaagctga tgtggcactg ctgtactcca gcctgggctg cagagagaga cactgtcaaa 54480
   aaaaaaaaaa gacgaaaaag acgtacaatt attggatata tgtgtatcca gagtttaccc 54540
   taactactgc agatactaag cctgctccat agccacccag caaattacaa aactaatgag 54600
   tggagcaaga cttgggagtt gtagctgatc ttacacagtg cctttagtta agtgcttcat 54660
   gttttctaga tttcctggtt tcccaagtta cttgtcaaat aagcttaaaa atcatttacg 54720
   taatatttcc atttggatgg atttataaag catattatcc tatagtaaat aacaataaaa 54780
   aaaacacttt gcttctccca attctattat gatctgtttt tgcccacaaa acacttctga 54840
   caccaaatgt gtgggttttg tctacaccaa caaccagttc ttcaactaac tctccagaca 54900
   tcaagtgggt gttcaacaat tcagttcaat tctgatgcta actttagaac ccagaggtga 54960
   agggtgcagt cccacaagac tgctcccact tgacatgacc actgcaggtc ctggatttct 55020
   catacttctg actgactggc tataaattgg gagttcccac aaccccctcc ttgggttcta 55080
   taatttgtta caacagttta cagaactcag cagaccagtt tccttactac tggtagttta 55140
   tttttataaa ggatataact cataaaacgc cggaagagaa acatagggtg aggtatgagg 55200
   gttatgtgtg aagcttccag gccctctcca gctgtgccac ccacccacca tctccatatg 55260
   gtcaccaaat tggaagctct gtgaactctg ttttgggatt ttattacagt ttcattacat 55320
   aggcatgatt gattaaatca ttggctgttg gtgattgatt caatctctcc tgcccctttt 55380
   ctgaccccag aggttagggt gaggttgaaa gttccaaccc tctactcatg tgcttggttc 55440
   ttttggtcac cagcccccaa cttaaagcta tgtagggccc atggaaattc actgccttag 55500
   cataaactca ggtacagttg aaagggctga ctatggatat caaaagatgt ttctctcacc 55560
   gccaccactc aggaggttac aagaatttta gaagcctttg ccaggaacct gggacaagga 55620
   ctaaatatgt ttcttattat attacaatat cacatactta atatattata ccaaagcaat 55680
   gtattaatat attgcaatgt caatgcattt tcattactta agaaatgaat ccttggaaaa 55740
   gtactttgaa agtcttgctt taatccaata catttgtctc aggaccagag atccagcaat 55800
   ctgatccaga atatatccaa ctctgaacat ccttttccaa aaagtattgt tcactctatt 55860
   ttctgcgttg ctgtctagtg tctgtccttg atccctttag cctctactct aagcattttc 55920
   tttcctagag aaggagctgc ccagactcac ttatttctta ctagcctttg cacatactat 55980
   tcctcaccac aagaatgcct catacttcac acactctccc cacttaattt gtgtgaagtc 56040
   ctcctcctag atattcctag ctctcttttg tttgtatttg ttgagcacct attttgtgcc 56100
   aggttctagg tgtttgggtt acatctgtga acaaaagttc tggcgttaac atctgtctca 56160
   ctagatcctg atgttatacc cgtctcatta gattataaag tatgcccttt gcttacctag 56220
   ttctcatgtt taactctagc tctgtaagaa aagaatccat ccaattaggt gaaagtatta 56280
   ttcctgaagc ccagcatagt ttttggcatg tcttaatcat tcagtaagtg cttgatggaa 56340
   tttaaaaaca atcacattat ttcttattca tttttttctt tgtttgctac agttgcatct 56400
   atcaaaatcc tatctgggaa gctgtgtcta acatatccaa ataatttata gtgcaaacct 56460
   caacagaagc ttctacagtt ttgccaagtt ccccttggcc atctctcagt tttctaccaa 56520
   aaatgttttc tgttatctca gaaatatttt ctagagttag gaagagtcca gactgactca 56580
   tttgaattaa gtcctcccct tgtcctggtt ggcttctatg actttgagaa acttacttaa 56640
   catttttgag ccttgctctc tttatttgca aaacaggaat gactatggta acctccagaa 56700
   ttatgctaac agccaaagaa ataggagtgg cgcatacctc gcacagtcgt gtgtctgaca 56760
   tatagtagaa gctctataaa tgttagtttc ttccttctcc caagagaggg acttgccttt 56820
   tatggaaagg attggggaaa gctatatggg aatgtcaaat tttggtaaga atgttaaatg 56880
   aagacatttt aaggtgcaaa ttttatgtct cccttagctg agctttcatt attcaaagga 56940
   ctgaacagga gtaacttgag gtaaaggcat catttaaaaa aattactgac tttatctaca 57000
   gaggctcttt cacttgcttg aggtctgtaa ttaagacttg tctgaataag cactagtagt 57060
   ttaatcgagg tataagagga agaagatgaa atgcagggaa gatggagctt ccagctttct 57120
   tcagaatgaa agattactca ctttagagaa cagagtgagc cttcaaaaac tgcttacaat 57180
   gggaaaattt taacattttc ttttaactac acaagtaaca tcaatgtgct aattattcta 57240
   aaatgagctc agaatttgct gtatggttac actttatgaa atacttgagc tccattacac 57300
   caagaaataa aacgctgcaa attcatttct gttaattcat gcataattga agaaagggtt 57360
   ggtgtgaaga ctctagaaca tgcatatttt tgtttctttg ttctcactgc cattatagtt 57420
   gaaggtttac aataacaaaa aggggtaaat acattggact cataactttt atgaactgtt 57480
   taaatgcaca atgaaggtga ttcataaaag tctctaattg tataagcaca tcactaaata 57540
   tggattgtaa aatatcttgt ttacttttta tataaaccta ctatagatga aggaatattg 57600
   caaacatagc atgtattttg tgcagaatgg agattggatc tacttttaag aaaagtggtc 57660
   ccacatcatt gaggccaatt tttcttcaat tcctttttaa ccaaaatcag aaaaatcatt 57720
   ctggtaacat cttggtaaat ataaatgagg gccatgttta gctgaacttc ttaattaaag 57780
   ctttaagcta aaccacattt ataataatag ctaaaagaga ggttttgaat gtcttcatca 57840
   caaagaaaag ataaatgttc aaggcaatgg atatgctgat taccctgatt tgagcattat 57900
   ataatttata tatgtattga aacatcactt tatactccat aaatacgtac aatgattaca 57960
   tatcaattat aaattttaga catttctgct ttgaaaatct tgttaaaatg tttgaatcaa 58020
   gcccctgctg cccctaggcc ccaccttcta gataaatata acatactatt atgattagat 58080
   cattatttgt cttgctaggg tttttgtgtg acattaagaa agtcacaata gctattatat 58140
   aatgatcagg tagtttaggc caaacatatt tgtaattacc tgttctcctg tatgcctctc 58200
   caaccccacc ccctaccttc tagacagtga tccaggaatc aacttcttca ggcatatatc 58260
   aggtactgga ggcactcagg actgttcatg aatgattttt tttttagttc atttgtgaat 58320
   aaatacatgg tgggagaaat ttacagtaaa catctccaat cccctgcaaa ctctttgtgg 58380
   cagcattaac taaaaaagtc tgagtcatct agccattgta acactgagga gaaataagag 58440
   gaccgagttc cacaccaggt cttcgagatc taacctgagt tgtttccagt gctcaaactg 58500
   tgctacaacc ccacctgccc aaacaggctc actggcttgg attgcaggaa actaaaaaat 58560
   atacagggga tgaggggttc catggacttg caccaacctc tgaaaggtgg gtttctgggt 58620
   ccagataaag gtaatttttg gtttgcttcc aaattgtttt ttagcaagta tctttggtta 58680
   gtggaggttg tggttagttt ctctttaatt ggtgttttct ccacctcaat gcctgatact 58740
   tgggattcta tagaccaggg gagactctgg ggcttatagg ataacatcaa ttttgagcat 58800
   cagcctagcc taatataatt gggatgggtg gggttcttcc caattagcaa ccacacagat 58860
   gactgaaatt aaagttggtg gttagaaaag tcatgcctag agagtaaaaa ggaaagttca 58920
   ttagtttttg tagaggcaaa gggagaactg cccattggtc ctctgaaggt tcactgaaaa 58980
   tcaactgaca aaaggcagat tcataggagg aaaggtatag aaatgtacta acacgcacaa 59040
   gggggaaatc ccacagtgat tacctcacta cccaatgagg tagaggtcct tatataccct 59100
   cttcctagga gaaagggaga tggggaagta tggatgattt tgaggggtta gtaaattatc 59160
   tttaggatta tcaatggatt tgaaaaacat acaatggcct gggacaaagt ccggcgggcc 59220
   cacagaatag tttgtgacaa aagtctgccc agctgcgttg acaagcttca gtttttcttc 59280
   ctgcgatatg agattcagtt aatgaaaact cagggaaggg accagaagtc actgtcacca 59340
   ccttttggtg gtctggacct taggcagatt aggacacttt agagaacgat tttatcctgt 59400
   gctttggaag agagagggtg gagctgggga gggtcacaga gaccttgagc ggcttcttca 59460
   gttcagcaag ttgaagcgcc atattttggg gtataggttt ctgatcccca atgccttctt 59520
   cactaaagct gaatgctttg gaggggaaca tgttccagaa gaaactgtgt aagtttggga 59580
   gtcagacaaa actagatcct aatcctggta tacccattta tcgactatat aactttgaga 59640
   cagttactta atcctgctca acctcagttt ctgttctgta taatggggac tcacacctca 59700
   cttgtgaagt tgggataagg aatattacac atatatatga tatatttgaa gtacctaaac 59760
   cataagtttt gtctttaaac tctggctttt tgcacatata ggttgtctga attcttggtg 59820
   gaacttacag aattatcatc atttgaggtt acagccatca gaacttcaca ataaactaaa 59880
   tatcattttt ggaggaatta tacataatta aaatatttgt agcctgcatc atcagtggta 59940
   gttgccaaga ctgtgagaga tgaagatata tatatatata tcttcatctt gtggcaccta 60000
   gaaccaacta ataagcccct agggagaatg ctaaatggct atgatttcct catgccctgg 60060
   agttatagct gaggctaaat tacagctgta gaaaccgaaa agctggaaac agaagccatc 60120
   agcccagcat gctcttccca aggcactcgg ttctggaagt ctgaaggccc tggagtggag 60180
   ctttaagcca cacttggact aagaaaaggg atataatttg tgaatgccct aaacattgaa 60240
   ggcttttatt atttgaacgc tttgattatt tgaaggcttt tattaaaatt taaaaaacac 60300
   aattttcaac taattaatgg attagtttat tttatcattc agaaagcagt ataaacctgt 60360
   gtatataaaa agtctcctta aatttatcct ccatgttttt attggtttta agcacataaa 60420
   tacataaaag tgcctacacg gcatctctcc ccttcaaaca tagattgcaa tacttataca 60480
   atggtaaaag ttggatgtat acctttccag aaatgtatgt aatggaatac acagagagag 60540
   agagagagaa agagagaaag acagagagag agagagagag agagagagag agatcattct 60600
   acatatattg ttctgtcatg agttttgttt cataatacat gtggacaaat taatacataa 60660
   tggccttttc attctgttta ttgcttatat aatattcttt gcacagatgc attatgaata 60720
   atttaaacat tcttttcctg atatacacgc agacttgaca tcgtttgtta ttttggctaa 60780
   gtccaactct cctgcagtgg atattctttt atgcctttgc atgcattctg gggaaagact 60840
   gtttgaagta gttactaaag ggtgtgtaca aattgccgtc caaaaatgca tttttatcca 60900
   caatacatgt cttctcattc ttaccagctc tagatgtttt gttttgtttt tgaacttgtc 60960
   agtctgaaaa ttaaaaatgg catttaattt tactaatatt tgtgtttttc taattactag 61020
   tgagcttgag tatatttaat atctttatgt cgtggaaatg aatttactgt cgagtgagct 61080
   ttcctctgat ccatgcccac aatatgaaaa ccagtttctc tgattacaag aaaacagagg 61140
   agtatgtgtg tggcctggtg tgtgtgagtg tgtgtgtgtg tgtgcgtgtg tgtaacacct 61200
   actgcagata agcaaggcct cgttggcatt ttcattgtgt ttgctctggg tattcatttc 61260
   tgcaaaagca cacctttttc taaataaaac taaattcatc ataggtcttt tatttctaat 61320
   ttggaaatat tttagaattt ctcaaagtat cataaaataa tttagaaatt attttagaat 61380
   ttctcataaa taccattcaa actcctaaag aatatggaga aagatatatt ataaaacaag 61440
   aacttagaag ctctacttta gaggaatttt taagcatgaa atcatgtcta gttcccaatt 61500
   ctaaaaaata gcccttgact cagtcagccc acagacatta agcatttgtg acatgccaca 61560
   agtaggctgg tatggagatt cagaaacgaa ggggatattc tctaccttcc tggagccctc 61620
   caggggagga ggaagtgaaa agtagagata agcataatac agtgcctgaa tgctataaat 61680
   attgagtgta gtggcaaccc gaggaagggg agtgtttggg acctgctgcc tgctttattg 61740
   tctcttgtca caggaggaaa aataattttc tcctgtgaca agagaataaa acctcaggac 61800
   cttccaaatt tactatgcca agggggaaag ttaaaccttg aaaacgggct tgcataacac 61860
   aactgttttt cttctctggt tcctgactac tgcttactga cctttgcgtt gagactttat 61920
   accttgacca gactccctgt tcttcattca aacctagact agatgacatg aagatagaga 61980
   cccttgtgac tattaccctt tatgacagaa tgttaagcaa gccttttaga gtgtaatcaa 62040
   tagtagccaa tcaaatctta tatctgtatg ttagcctttg tacggaaaat gttatagttc 62100
   agcccctcca tttttgctta tagaaacaat cctcattttt cccccacact aggagcactg 62160
   atcactattc tttggtgtaa ttctgctccc ttgtcagctg ccctaccttt gtgcttgagt 62220
   aaactctttt aaccggatgc tgagcctttt gactgtttta tgttggcatt cgctaccctt 62280
   catagttctt agctgggaca aatccctgta acaaaagaaa gattcacaag agaaaaacaa 62340
   acagaagttt aataacatgt atacttcatg tgtacataga agatatccag ggaaaatgag 62400
   taaatctcaa agaagtggct taaatttagg cttaaatgcc atcttcagct gaaatgataa 62460
   gagtatttgg gtaaggagca gttaatggga agatgaccac agaagcacag gaagcaggat 62520
   aagttttgtt atgcaggttg gtccatgtct tctctataga tgagcgtttc tagtgactga 62580
   gagtcatcct tcccttcctg ctacggagaa gaagacattc ttacaaatgg atgtttcctt 62640
   tatagatata aattgctctt gtgaaaggac aacttctact gttttcagag cttcttttat 62700
   ctttgctgtt gcccaaatta atcagttcaa aataatcctt atgctaaaga ggcatatttt 62760
   ggggtggcat attctggtct cctacagtca tattttgttg tggtacatac taaacaccac 62820
   tactccgtaa cagctcatat gctgaacacc actatgcagc aacagctcat atattaaata 62880
   ccactactcc ataacagctg taaaattctg tctaaaaaga aatctgcttt ttgtgggtgc 62940
   atgtgtgcat gcttgtatgt gtgtgtttgc atgtactaaa catttattga attcgctgat 63000
   ctacaaaatg tatgaccaac ttccatccag aaattttaat aagattgcac cataccaaaa 63060
   gccagagaat caggagatag attacagact atgctgtaat tcaactacag aatacagaag 63120
   tagaggggat taataaaggc taaagttgga tgggtgtggt ggctcatgac tgtaatccca 63180
   gcactttggg aggccaaggc tggtggatca cctgaggtca ggagttcaag accagcctgg 63240
   ccaacatggc gaaaatccat ctctactaaa aatacaaaaa aattagccgg gcctggtggt 63300
   gggtgcctgt aatcccagct acttgggagg ctgaggcagg agaatagctt gaacctggga 63360
   ggcggaggtt gcagtgagcc gagatcatgc cattgcactc cagcctggat gacaagagat 63420
   aaacttcatc tcaaaaaata aataaataaa taaataaagg ctaaagttcc acatttgcaa 63480
   tgagttgcta caaccagcaa aaaaattaag gtttttttca gatgctagca gtcacagagg 63540
   tgaaggtgca ggaatcagaa tggagagatc attaggagca aagaaagcag gcattaagat 63600
   agaatgaatc accaaagctg ataacacttg aagcccctga tttcaatttt aatgattcct 63660
   ggcatggttg aaaatagttg acatactgtt cctctattat atttgtgttt tcctgctgcc 63720
   ttcactcttt cagacagagg gagtagagct acaaatatta aaagaaaaaa atttttttgc 63780
   agtattttaa aaaaagctaa gttaaaagtg ctgaacattt tgcaaaaact tgtccttgtc 63840
   agttctggaa actcaagggt tccatgtaat tcgagtaaac aaagaacacc tttcactgac 63900
   actttctttc tttttttttt tttttttcct gtctcactca ttccagtaac attttggtcc 63960
   ttgcattcct gaacatcttc ctgggaaccc accgtttctt cttattactg gtctattgct 64020
   gtgctttata aaccccttaa agaagctttg aacactctga agaactgaat aatatatctc 64080
   agaggaaagg tatagactaa attgactact gcaaatagtt tgcacagaaa agtaatgttt 64140
   ggaaaaatag attggggtct cagtattggg gcatggaatg tttggttgtg gagtttagag 64200
   cttattgaat aggcctcagg gatacacact taaaaagtaa tgctttggtg agattaatca 64260
   gatgtaaacc tagagaatga ctgtaggatg gggataggag aagatgctga agacagacta 64320
   ttacaacaat atgtgttata gaaatattat ataatactac agaaatattg cagaaatact 64380
   attgcagaaa tatagaaatg tgagttgcaa attcatattt gaaatattgt cgaaagggaa 64440
   gattaagaac acatgtagag tcataattca atcattaagc tttttatttg acatttgtgt 64500
   tgcatgattt gagaagattc aaagatgtat caagcctgtt ttaccctgta acagcttcta 64560
   taactaattg aaacttttgt ataaaattta ttcaagcaca acctgctgaa gaaagctttc 64620
   tctgatacca tgcaacattg agagtgatgc tcagcttgat gctttcagaa tcttggtggt 64680
   ttctatcatc aagtgctctt cacactgtaa tcatgatctc tttatatgta tactatccac 64740
   tttacacggt gaggcccttg aaggaaggaa ctatgtctca tttatgtagg tacctcagag 64800
   aaagtagtgc agtgcctgca acagaggacg aatcagcatt agccaaagga atgatggctg 64860
   caagtatggg aagaaacaca attctggtac aaagtgttgt caataccgtt gggtaaaaaa 64920
   agtattattg cttgtataag agctgggcaa gtgttggaaa accagaagaa tggtaatttt 64980
   tctgtgaaga aattgagttc atttaattac atgttgcatg tgaagagata tttgctgtct 65040
   ggcaaaaatg taaagaagtc aagtcaaaag cacagcacta tcaaggacgg aaagctctac 65100
   acagatttgg agaaggaaaa ttaagtgtta gatttaaaga aagaagaaag ggcctaagga 65160
   atgagggaat aaaggagcag cattaaaaat caaagagatt aaaaatatgt aggaagttga 65220
   atacaagata aattggactg taaaggtcag tttggagaca gagcaatcag aggattctgg 65280
   agaccaattt cttttctttt cttttttttt tttttgcgat ggagtttcac tcttgtcacc 65340
   cacactggag tgcagtggtg caatctcagc tcactgcaac ctccgcctcc aggttcaagc 65400
   gtttctcctg cctcagcctc ctgagtagct gggactacag gtgcccgcca ccacgcccag 65460
   ctaatgtttg tgtttttagt agaaatgggg ttttaccctg ttggcaagcc tggtcttgaa 65520
   ctcctgacct caggagatct acccatctcg gcttatcaaa gtacagggat tacaggcatg 65580
   agtcactgcc cccagcccca gaggccaatt tttacgcagt gttgatctta tggcattggg 65640
   cacacctgct gctcaaaaag atatagcaac cagggcatgg agcgtcaatg tagcagtcta 65700
   gagagcagct agggttgtag aagttgcttt tcaaaggaca gtcccagatg gatttataga 65760
   tgccattcag ttattcatta ttgcaacatt ttttaatgtt tattggctaa caccgtttga 65820
   gaaactgaat tggctttaac acatttttgc taaataaatg ctagatgctt tgaacattac 65880
   acaagtaaga tacatgctct ataactctaa ttctaagtaa aacatgaaga ttttaaggga 65940
   cagagaggac cagttagaac accatgaaaa ggcatcagtt gatttgatgc tgaaagataa 66000
   ataacatcct agcaaataga atttgggaaa aggaattgtg aaagagaaat ctagacatca 66060
   ggaaatgcag gaatagacat tttataccag aaaatagtac atgtgtctaa gaaaatggta 66120
   ggagttctgt ttagtaaaat aataggacat gtgcgtaacc tccattaaaa tgaagattct 66180
   attggtgaga tactcacttc ataccaagtg aaaggccgat gcagttcaag gttgataaat 66240
   gtacataata agtaagcagt cattctgcac acatttatga aaatgccctt agagaattta 66300
   tccctctccc atagctcgtc ctgtgctttt cacactgagc ctcaatttta ttcacaaaga 66360
   tcgtgataaa aagcaactag tggaactgaa agtcattaaa tctgaaaaca gccttggacc 66420
   aggtagagaa accaggagcc agccaggctt tccttctgct taacaatgtg actttggaga 66480
   tacttgataa gagaattctg agtatcaatt tccccatatg ttaaaacagt attgagctaa 66540
   atgatagctt caattctttc tggccctgaa tagttgtaag tttatttctg ttttttgttc 66600
   acatttgttt aaagacacag aaagaaagtg catgatctgg acaaagcagt accttctgat 66660
   gatgttttct ttcccccaga tgattcatat aactacataa tgggatattt aaaaacaaat 66720
   agaccgggca cggtggctca cacctgtaat tcctgaactt tgggaggcca aggtgtgtgg 66780
   atcacttgag gtcaggagtt tgagaccagc ctggccaaca tggtgaaacc ccatctctac 66840
   taaaaataca aaaaaattgg ccaagtgtga tggcaggcac ctgtaatctc agctacttgg 66900
   gagggtgaag caggggaatc tcttgaaccc aggaggcgga ggttgcagtg agctgagatc 66960
   acaccactgc actccagcct gggtgacaga gtgagactgc gtctcaaaac aaaacaaaac 67020
   aaaataaaac aaataaacca tgataaaaac aagtgacact agcatcctat aattctatag 67080
   attgagtaat gacatagtaa caaattcact attgcagaac tggggtggga gttccactcc 67140
   ctgggcaaat taaatccacc caagtcatca gacctttaat tttcatttta aagtgagaaa 67200
   atacacaaga aatcatctaa aaatataatt acagggtgaa ataaataagg cagagtatgt 67260
   gtgtgtatgt gtgtatttta agttactctt ttcagaataa atggctttag gaaattattt 67320
   ttgtggaatg agtttctcta ctatcaagga gacttccctc tactgtattc aaatttaggt 67380
   aaaataatag gtaaaagtgc atattccagt actaaactaa taagcaatca gaaggaaaaa 67440
   tagatttcag ccgttatgcc catagaatga atttgattgc tgaaggtggt atcttcagga 67500
   tcctgttatt gttacttttg ctgaaataat aggccagatt ttattttata aaagaaaact 67560
   atagattttt cttctaagtt cagacttgcc agctagttct actgttatta ttctgaagtg 67620
   cataaagtcc tcattaactg atgccagctg accgggaagc acgttgatag atgccttctt 67680
   actggtgggg aagttcactt aacatctgta cttggacgtg caggcgggtg cttggcaggt 67740
   gacagcctgc aatgggtgct gtcatgctat aagcaagcag gctgcagagc cagagtttag 67800
   cttctgttta gctctctttc ccagcataat gtgtgcaaaa cacaagtgat gaaattgatg 67860
   cacagcgctg tgtgttccta caccactgca ttatatgaat aaggaacatg gaacaaaatg 67920
   gcaacattaa gggtatgtca gattatttcg cagaaatctg ttcctttttc ccctgcagtc 67980
   aaacccacag cattctatct cagaaatgaa tatagaggat tctgttagat tttgccttga 68040
   ggtttgtatt tgtcccaaag agaaaaggag acttatttag aggactggtt gtgcagagtt 68100
   gaggacatta gcctttctct caggcattat gagatgtcag catgattctg caactcggac 68160
   ttcacaatat caagtgttcg catatttctg gaaaatgtga aagtaggcag ccagaaagtt 68220
   atttaaaata tgaagaattt acgtgaaaat tttcttgggg tccaaatcca ccaattggtt 68280
   aaaaggttta cccaaactgt gtgcatccag atattccact gacaaatatc cattcattca 68340
   ctcatttata cattcactgc acaaatatct attaaatacc tggtacaaca gaaaaattaa 68400
   gagaaagtcc tgcttttatg aagtttatat tctagaagat ccttgtgatt ttagcatttt 68460
   tataatttaa acatttttca tagatttgct gatatgtttg gctttgtgtt cccacccaaa 68520
   tctcatcttg aattgtactc ccatcattcc catgtgttgt ggaagggatc tggtgggaga 68580
   taattgaatc gtgggggtag tttcccccat actgttcatg tgctagtgaa taagtctcag 68640
   gagatctgat ggttttaaca gggggttctg cttttgcatc ttcctcttct ttctcttgct 68700
   gttgccatga ttctgagaat tccccagcca tgtagaactg taagttcaat tgaacttctt 68760
   tttctcccca gtcttgagta tgtctttatc agcaatgtga aaacagacta atacagttgt 68820
   atacctacac acacacatgc acacacatgc acacacgcaa aggcacacct acacgtagtg 68880
   ttttcatcag ctagggtctt aataggaaga aataacagaa ataggaagca ataaaaaaac 68940
   tgactgcagt ttgtttaaac taaaaacaaa ttgttgaaag ggtattgagt agttcataga 69000
   attgtagaga atgctaggta accaggttta gatatgaggc aggaatatag aaccacagcc 69060
   agtaaatact acagagccag caagggaaga ctctgccatc actgctgatg aacaaacaat 69120
   attgtagctt caaccctgga gactggatgt tgccactgaa aaccataagc tcttgcatct 69180
   gggctttcaa agttattgct tccaatctgc ctgtttctta ggatcacaga ctatgatgca 69240
   agtctcaggg aggcaagttt gattggttgg gcctggtctg agtgaaccaa aagttgagtc 69300
   ttagtgaaat acacactgca tttggtcaaa tgtcagcggt tgtgaaaaag gtagctacct 69360
   gcaccctcat ccccatttta ttgtatcttc atgagacagg acatgtttga ttttacctct 69420
   gtcatctcag tttctagcat aacacaggcc acatggaaca tatttattaa atgtaggaat 69480
   ggaatcattt attaggtgtg gtggtgaagg tagctttata ctcatgccac acatttgcat 69540
   ttttttattt ttctctgagg tttgagttag ctcctaatgc tttacctagg tttctaaagg 69600
   cttagactag gatgagtctt tggtctcttc cagatcatat caggattcag gggcctttga 69660
   gctcctttga gagataactg agtgttgagt ggactctgaa aggaattttt catccaggga 69720
   aagaagtcca cagaggaaac aaggcagaga cagcagagct gctgctgccg gaggcctgaa 69780
   aactatcaac tctttctgac tctcctctga gactgggcaa caagtgtggg tccacaccat 69840
   tcttgtaact gtttaggatg ggttatgctg ccctgaaaaa ttcaccttga gattcagggt 69900
   ggtctgcatg ggaagaaaag aatggtgcaa tattagtgtc ccatattcaa ggaggcttag 69960
   ttcttgaaag gctcattatt cagagcagac atttaatcag agggagttga gtgttaaggt 70020
   caaactcctt gagatgagcc actgtgagga gactcttcta cttttcaagc taatttcatg 70080
   tgtgccataa atcttggaaa gttaaaaaga gggcgggatt tgctaggtag tcaatttagc 70140
   aacattttct tacaacttta agacattttt ttctttacta ttagagaact atttagaacc 70200
   tttaggactc taattatcat gcacacagct tggaagaaat taaccaacaa agttcagaaa 70260
   gcaaaaagtt tataagacat aatttaatgc actttatata tttaaacagc aacaatgaaa 70320
   tgctatcctc ctgtgatgta aagttctctc tttccacctt ctaaatataa aaaaacgtgg 70380
   ctctcatata caaatacaaa cacacacatg tgcacacgtg aacacacatg catacacatg 70440
   gacacacatg tacctcacaa actatattaa gaagaacaag agaaaaaata atccaattta 70500
   tgtggtgctt acaatgccag actaaggagg tgcttttact tgccttctaa tttattaatc 70560
   acatagcaaa gttgacggta atgtactaaa attactgttt ttttttttgt agaatatagc 70620
   ttagaatcaa agaggttgca tatgcttttc aagccaatgc agctaataaa tgacagaatc 70680
   aatatttgat aatgaagggg tggagcaaga gagttgaata aaaggcttta ccaattgtcc 70740
   cttctgcagg tacaccaaat ttaacaacta tctacattaa aaaaaaatcc accttctcaa 70800
   gaccaaaaat cagatcagtg ttcacagtac ctgattttaa cttcatatca ctgaaagagg 70860
   cattgaagag actaggaaaa gcagtcttga actgccaatg tcatccctgc cccatcccct 70920
   ggcagaagtt gtataacatc aaaaactaat aatatttgtg cttgggagag ggagagtgta 70980
   gtgattgtga aactctgcat tgacctcagt gttgccctgt catagtgaaa agcaaaattg 71040
   ggctgaattc gccaatgcct gtgtacagag ggagcattta gatcagccat aacgagatga 71100
   aaattgccca ttctagaggt cagaatttga gttctggaaa gcctcaccat aggaggctaa 71160
   agtgctctgg gtccctaaat aaacttgaaa ggcagtgtag gccacaagaa ctgcaactca 71220
   taggcaagtt ctagtcctga gctgtgctct aggccagtgg gcttggggga ctcgtgtcct 71280
   actgagatgc caactggggc agctaaggga gtacttgttc catccctccc ccagcctcag 71340
   gcagcacagc tcacagctcc aaaaagaccc cttccttcag gagaggagag ggaagagtaa 71400
   agagaacttc atcttgcatg ttggatgcca gcttagtcac agtaggatag gacataggga 71460
   agagttaata ggcctccatt ccaggcccta actccttaat gacatttcta gacacaccct 71520
   gggccagaag ggaaactgct accttaaagg gaaggaccca gtactagaaa gatccatcac 71580
   ttgatgattg aagagccctt gtgccctgaa taaccagcaa caacacccag ttagtgtgct 71640
   atgggccttg ggtaagactc tgagacatgc tggcttcaga tgagacatag catattctca 71700
   tctgtgatga ctatggtgag aaactccttg tccttaagaa aagcagaggg aaaagtaaag 71760
   aggactttat cttgcacctt aggtacaagc tcagccacag tggggtaatg caacaaatgg 71820
   gctcctaggg tttccaactc cagattttga cttttgtaca gcattactgg acctggctgg 71880
   gtgagagggg agcccacttc cctaaagggt gagttccagg gctggcagca ttcactacaa 71940
   tctgacggaa gagccattaa gccttaagtg aacgtcagcg atagcctggc aatactctgc 72000
   atgggcctgt ggtggtggtg gccatgggat gagccccctc tgcctgtgga aagataaagg 72060
   aatagtgaaa agagctgtgt ctcatggttt cagtgcagct cagttgcagt agaatagaac 72120
   accaggtagg tttctaaagt tcttaactct agtctctggc tgccagatga aatctctgga 72180
   tctcactcag tgcctggggg aacttgtcag cccaaaggga aagacagaac cctggctggc 72240
   tttgtcacct gctgactata gagccctagg gccatgagtg aacattggca atagcgaggt 72300
   agtggtaaca gtgggctttg ggcaagaccc agttctgtgc tggcctctgg tctgacccag 72360
   cacagtccca ctggtggtag ccacaggggt gcttctgtca ctttatcctc agctccaggc 72420
   agcccagcag agagaaggag attccatttg tttgggaaaa agtaagagaa ccagtgtctc 72480
   tgccaggtaa tctagagaat ttttccagat tttatccaag accaccaagg tcttgggcaa 72540
   gaaccacagc attactgggc ttggagtgct ccctaatgca gaaataactt agatcacaac 72600
   atccaagact tttttttttt tttttttttg ctatctggaa agccttccca agaagaacag 72660
   gtataataaa gcccagacat tgaagactac aatacctaac tcttcagtga tgagacacca 72720
   ataaacatac ataagcataa agaccatcta ggaaaacaag accttaccaa atgaactaaa 72780
   tgaggcatca gcaaccaata atggagaaag acagatatgt gacctttcag atagaggatt 72840
   caaaatagtg ttgacaaaac taaaaaataa tcaggttaac tcagagaaaa aaattcagaa 72900
   ttccatcaga tatatttagc aaagagatta aagttaatta agaagaatta agcagaaatt 72960
   ctggagctga aaaattccaa ttggcatact gaagaatgca tcagaatctc ttaccagcag 73020
   aactaatcaa gaaagaaaga aagagagaaa tggcgagcct gaatgcaggt aatttaaaca 73080
   tacatacagg agacaaaaga aaaaagaata aaacatgcta caggatctag aaaatagcct 73140
   caaattggca aatctaagag ttattggcct tatagagaag gtagagaaag agagagaggt 73200
   agaaaattta ttcaaagata atatcagaca atttcccaaa cctaaagacg atatcaatat 73260
   ctaagtataa gaaggttaca gaacaccaag cagacttact ccaaagaaga ctatctcaag 73320
   tcatttaata atcaaactcc caaaggtcaa gggtaaagaa ataatcctaa aagcatcaag 73380
   aaaaaagaaa caaataacat acaatggagc accaataaat ctggcactag acttttcagt 73440
   ttacacttta caggccagaa gagtgatgtg acatatttaa agtgctgaag gaaaaaaaac 73500
   tacacaaaac ttttatccta gagtagtata tctggtaaaa atattcttca gatgtaaagg 73560
   agaaataaag actttcccag acaaacaaaa gctgagggat ttcatcagca ctagacatgt 73620
   cctacaagaa atgctgaagg gagtacttca atcagaaaaa aaaggacatt attgagcaat 73680
   aagaaaccat ctgaaggtac aaaaaaacac accaaaactt gacttgtact agtaagtgca 73740
   aagaagaaca gaatgttata actgttaact atggtgtgta aactaaattt tcttaagtaa 73800
   aaagactaaa agatgaacga gtcaagaata acaacctttc aagacacaga cagtacaata 73860
   ggatataaat cgaagcaaga aaaagttaat aagccaagca acacagttaa agtgtagagt 73920
   ttcaatattt ttgtttgtgc ttgttagttt gtttatataa tcagggttaa gttgtcagct 73980
   taaaatgatt agttatagaa taatatttgc aagccttttg ttaacttcaa atcaaataac 74040
   atacaacaaa tacacaaaaa taaaaaccaa taaatgaaat cataccacca gagaaaatta 74100
   tctttactaa aaggaaggca ggaaagaagg aaaaaagaag gagaagacca caaaacaacc 74160
   agaaaacaaa taacaaaatg gcaggaataa gcccttactt ttcagtaata actttgaatg 74220
   taaatggact aaactatcca ttcaaaagtt atagagtccc tgaatggatt aaaacaaaca 74280
   aacaacaaca aaaaataaga aaaaacaaga ctgtatgatc tgttgcctac aagaaacaca 74340
   tttcacctga agacatacat agattacata taaaagaata gcgaaagata ttccatgcca 74400
   atagaagcaa aaaaaaaaaa gtggaagtat ctatactgat atcagacaaa atagatttca 74460
   agataaaaac tgtaagaaga gataaagaag tgttcattgt tagtatatgg aaatacaagt 74520
   agattttgtg tgatgttttt ataccctgaa attttgctga gttcatttat tcattctaac 74580
   acatgtatgt gtttgtgtgt gtatgtgtgt gtgcctgtgt atgtgtgtct gtacatgtaa 74640
   ttttaggatt gactgcgtat aagatcatgt catctctgaa cacagggtga atttttcttc 74700
   cctttttttc tatttgaatg cctttatttc attttcttgc ctaattttgc aacaaggatt 74760
   cccagtactg tgttgaatag aagtagcaaa aacaggcatc cttgtcttgt tcttgatctt 74820
   agaggaaagg ctttcagtca ttaattatat tagctatgga tgtttcatat atggccttta 74880
   ttatgtggaa atatattttt ctattcctag ctttttgaat ttttattcat gtaagaacat 74940
   ttaattttgt aaattttttt ctgcatctat tcagttgttc acatagtatt ttttcttcat 75000
   tctgttaatg tgtataatat taattgattt gcatatattg aaacatcctt gcatccaata 75060
   aataattccc cttgattttg agtgtatttt aattttaata tattgttgaa attagtttgg 75120
   tagtattttg ttgagaattt tacatccata tttatacagc atatgagtct gtagcttttt 75180
   tatgttacag tggttttatt tgtttgtttt ttgaatcagg ctgatactgg acccagaata 75240
   agttaggagg tgttcctgct tcaatatttt ggaggaattt cagaagaatt gatgttaact 75300
   ctaaatgatt ggtagaattt atcagtaaag tcatcttgct caagatgttt ctttattaag 75360
   aggtattgat tactgactaa atctgcttac tagctatagg tttatttaga ttttctattt 75420
   ctttataatt cagccttgga aggttgatta tttattggaa attgcctggt tcatctaggt 75480
   tatccaattt gtttgtgtgt taccattcat agcattttct tacaatcctt tttatttgta 75540
   taaaatcagt ataatgtttc cactttcgta tctgatttta gttatattaa tctttttcat 75600
   agtcaattta gataacttta ttaattttgt tgatattttt gaagaatcag ttatttgttt 75660
   tgttaacttt ttaattgttt ttctattttg tttacctctg ttctaatctt tattatttcc 75720
   tttcttctgc taattttaca tttagatttg tcttcttctt aaagtgcaaa gttaagttgc 75780
   tgatttgata tctttctctt tcaattaaag tgtttgtagg tatgatttgt tatattagca 75840
   ttactttcac tgcaactcgg aagttttagt aaattgcgtt gtaattttta tgaagatatt 75900
   ttctaatttt tctgtaattt tttttattta ttgcatgtct aagggtgtga tgcttaattt 75960
   cctcattgtt gtgaattttc cagttttcct tttgatattg attttgagtt tcactccatt 76020
   gtgattgaaa aatatacttt atatgatttt aatcttttaa aatttattga cttgtttttt 76080
   tgcctaacat gatctactct ggcaaatgtt gcatgtgccc taaagaaaaa tatgtattcc 76140
   attgttgttg ggttgaaaat tcagtgtgtg tcttttgggt ataattggtc tatacttatt 76200
   gttcaataga aatttcttat tgatcttctg ttaggttgct atatccgtta ttgatagtga 76260
   ggtattgcaa tctctatttt tgtagtgcta tttccctctt caattctgtc agtgttgttt 76320
   tatatatttt ggagctctga tatttggtgc atatatgttt ataattatta tatctccttg 76380
   gtgaattgac catttatcat tatatgatgt tttctttaac tcttgccaca gtttttgact 76440
   taaaatctat tttttatatt attagtgtag tcatcttgcc cttttttggt tactgtttgc 76500
   aagtaatatc tttttccatt ctttcacctt caaattttgt atgtccttcc atgtaaagtt 76560
   attctatagt tggatcatgt tttattatac attttctcaa actatgtttt ttgatcagag 76620
   aagttaatct atgtaaaact aaaatgacat ttatagggaa ggagttactc ttccattttg 76680
   tttttgtttt tctccttgtc ttgaatggtt tataagaatc cattgtcttc ttcttaatca 76740
   tcttctattg tagttaatta attttttagt ggactgtttt gattcccttt tcatttcctt 76800
   ttacaaatat tctacagatt ttattttttg ttaccatgga gattacttat aacattagga 76860
   agttataaca atctaaggtg ataccaagtt aaatttagtt acatacaaaa actccccacc 76920
   cttacagccc cccctatttg aggttattga tgtcacaaat tacatcatta gttattatgt 76980
   actcaattaa tatagattta taataatatt tattcattag ccttaaaaat gctgtaagaa 77040
   ataaaacctg aagttaccaa ccaaaatgaa aaaaataaac cttgttttta tatttgccca 77100
   tatctttatc aaagatttat atgtcatcat acatttttga gttactgcct atcatctttt 77160
   cattttgacc taatttattt catttaacat ttcacatagg gttggtctag tgataattaa 77220
   ctctctcaga tcttatctgg gaatgtctaa tttctcccac aaacctgtct gaaggacagt 77280
   tttgttgcat acaagtcttt cttgacaatt ttaatacaag atccctctgc cttctgacct 77340
   gcatgatttc tgctgagaaa tctgcttatt atctcattat ggatttcttg tatataatga 77400
   cacttcattt ttattttctt tttgtgtgcc tgtgtgtgtg tggacttttt ttttgtctgt 77460
   tgacaaaatt ctctttgtct tcagattttt aactgtttga ttataatttt tttaatgtga 77520
   gtcttcttac atttattcta tatgatgtct gttgagcctc ttaaatttgt agattcatgt 77580
   attccatcaa attttgtaag ctttccataa ttatttcttt agataatctg tcttctcttt 77640
   ctctctccct tcttctgtga ttttcataat acagacatgt ttgtcccaca agttccttaa 77700
   gttcctctgt tcacttttct tcacttgtcc cataacactt gatggtgtcc cattagttcc 77760
   ttaggatctg ttcacttttc ttcattcctt tttttttctg tttgctcctt aaatttgatt 77820
   atttcaaatg tcttttcttc aagtttgctg attctttttt atgcctgttc aagtatgctg 77880
   ttggactcct ttagtgagtt tttcaattgt cattctattt ttcagctcca gaattttcag 77940
   tcagttcctt tttacaatat tatctttgtt gatacaatca ttttgttcat gtatcatttt 78000
   tctatcttcc tttatttatt tttctgtgtt ttcttttagc ttttagaaca tatgtaaaca 78060
   gttgctttaa aatatttttc tagtacacct gatgcctatg tttcttcagg aaatacttgt 78120
   actagttttt ttcatttgaa taggccatga tttttatttt ttctttgtat tccttcttcc 78180
   tttttttgaa aattcatcat ttgaataaaa agttacttct tctggtcttg gcagagtgat 78240
   attgtgtgga aaatacctcg ctaattagta gggcatgccc aaacaattca ttttgcctgg 78300
   actatatgtg tgcttttttc aattccttta tatacatggc tatttttaaa tgtctttatt 78360
   tgacagagag tttcatctca gcctatccat ggggatttag gtgatctatt atgtgtcttc 78420
   atctgtattt tcttatgcca gagtctgcag gtcagtagtc cctggctgtt ttcatgcgca 78480
   atgcctgtta ctgttttcca tagcttttcc caggctgaga tctgagctat gtcacttttc 78540
   cccatacagc cttttctcca aacaagtata tatatagaaa aacttctggt ctttatttct 78600
   tttcttataa ggacaacaat tctgttgaat tagggtccac tcttatgact caattaactt 78660
   caattacctc cttacatgtc ctatttccaa atacagtcac acagggtttt agggcttcaa 78720
   catatgaatt tgggtgaggg ggttccaatt cagtccatag cagacaaaac aattgcatgt 78780
   tggtgattaa tatttgtttg ctttttattt atttattttt attttttgtg gttgctggtt 78840
   ttactttttc ttaatatgtc attattttcc tttcattctt gaaaggctat tttaccaggc 78900
   ataccattct aagttggcaa ttattttctc ctctccctct gataatattt gttccactct 78960
   ctcctgaccc ttttggtgct attgataagt ttactgtttc tctaaataat gcttcttata 79020
   ttcttactta aaaaaatcaa tttgcttttg gtgttctgta ttttcaccga aatgtgtcag 79080
   gataattttt tgtctattca tagtatttag agtatgctat gctttctgaa tcataagagt 79140
   tctttccttt ataatttctg gaaaattctc agtgatcatt ttattagttt ggttccattc 79200
   tctctaagtt cctatgttct ctttctcttt atgttctgca tcaggaaacc tgagtcaatg 79260
   cacgttagac cttctgaatt tattttccat ttatctcatt tttactttta ctcctctttc 79320
   tctgagttct gcatttcatt aactttttcc tatattttta gtttactaat tctccgttca 79380
   actatgttta atccgttctt taatatattt attgggtttt tgatataaag ggagatattt 79440
   ttaatgtctg taaactctgt ttgattcttt aaaaaataaa catggtcatt gtatagctgc 79500
   tttcccttct tatgctttca attctctgtt ttgttccttt acccttttcc catatgcccc 79560
   aagaatactt tctagcagta cttgcggctg cagcatttac cctgagataa ctttgccaca 79620
   aaatatcttg cttttattat taatttttgc accactctag tatacccact ttggaaacaa 79680
   atgatatcat tctatttata gcattctatt tttagtagtg gttacttgta tttatgaaat 79740
   atagtaattc tccatcgctg aattaataaa tccattttct tctaaatata tgattgacga 79800
   ttcagatgat tctgatgtta gttctgttta gaaataactc aaacaatagc tttatatttt 79860
   attttcacat taaaaatcag atttgcttca gcctcaaaga gtgtgtttat ataaaattaa 79920
   atgagcattg acagtgagct gccttttttt ttctaaataa gaaaagggtt aaacacataa 79980
   agtgtatttt ctgtgtgtgc tattttcaat acctgaagtc tttggaagtc taattctgtt 80040
   gttttttaat ttctgctgac tctccctaat aatggcttat tttcatgcaa attttgaaag 80100
   attggggaaa acccaattaa tgtgaagctc tgtttaaaaa atgtgaagga ctgaggttat 80160
   ggattttttg aggagaatat tttttcctct ctaccacttt gggtgctgta atttactctc 80220
   tcatatctgt tctaacttac ctcccacctt ggagatgcct gatgccttct ctcctgtact 80280
   gttaaatctc aatactctgg atcaccgtga atcattggtt ctcagtgcat ctgctagctc 80340
   cagagtgtac tgttcaacct tcaggcttct gctagtgaac tgaattcttt atttaaaaaa 80400
   tcatatttta tctttttttt tttttttttt tttttgagaa ggggtcttgc tctgtcaccc 80460
   aggctggagt gtagtggtgt gctctcggct cactgcaacc tctgcctcct gggttaaagc 80520
   aattcccctg cctcagcctc ccgagtagct gggattacag gcgtgcgcca accatgcctg 80580
   gctaattttt ttgtattttt agtagcggca gggttttacc gtgttagcca ggatggtgtc 80640
   gatctcctga ccttgtgatc cacccgcatt ggcctcccaa agtgctggga ttacaggcgt 80700
   gaaccaccgc gcccggccat cacatattat cttgtatttt gtagttttag ctactttttt 80760
   ttttcttttt tcaagcttcc tgtttcttta tgagaaagga aatggtaaat aatcacttaa 80820
   ttttgaagct ctttatgtga gtacgtgact gtgtgggaaa ataaaagaga cattaagcca 80880
   agtgaattca ggaaaagctt ccaaaccaat agaagggaaa atatatggga gaaaaatata 80940
   tatttttttc tttttttttt tttttttttt ttttttgaga cggagtctcg ctctgtcgcc 81000
   caggctggag tgcagtggcg ggatctcggc tcactgcaag ctccgcctcc cgggttcacg 81060
   ccattctcct gcctcagcct cccaagtagc tgggactaca ggcgcctgcc actacgcccg 81120
   gctaattttt tgtattttta gtagagacgg ggtttcacca ttttagccgg gatggtctcg 81180
   atctcctgac ctcgtgatcc gcccgcctcg gcctcccaaa gtgctgggat tacaggcgtg 81240
   agccaccgcg cctggcctat atttttcaat ccaacagata acagaaaaga aataaaaaga 81300
   agaagtctgg caactaggaa aaaaaatgat atggtttcat caaacctact atatcagtac 81360
   tctaattcag aaggtttaaa gtaatctatt aaacgatttt tcaaaaatgg attacatatt 81420
   tttgaaatcc aactttattc tccttataag acatgagtat tgaccctatg ttttacaata 81480
   tctaaaactg tatagtaagt accgttctct accttagcat atatcaactc tcattgaaac 81540
   tttaccatgt caccattatg catagtctct ttatgtgtaa acttttttag tctagaaaaa 81600
   ttatttttaa aaatagatta tctctatctt ctttcttacc aacccatcct ctgggttaaa 81660
   tttgcttctt tctaattttt tttcatactc atacttttag ttataaagtc tgtaagttca 81720
   acttagtggt ttatatctaa agtttttatt ttggctttac aattgcatga ttgtttgcct 81780
   tgatgtataa ttccagattg acaattatct tccctaagca ctttcattct attattccat 81840
   ttttgtctgg catttattat tggtgatgaa acatctgctt gtcttgataa taagacattc 81900
   taattgccat ttgctagtaa gtaacctgtt tttttgcttt agaacgttac attttttatg 81960
   ttcttcagtt ttattactat atgtttactg tagatttatt tggatttatc ctttttaata 82020
   tttaatatct tttcaatctg aaaaccatgg ttcttataaa ttatagaaag ttcgcatcaa 82080
   aaaccttttc gaatgttttt tttttttttt tttattctct tctttatcta ctttgggaat 82140
   tcttatgaag tgaataccaa agcctcttaa tctatctttc atgttttaaa atttctttca 82200
   cattttcctc atccttttag ctttctgtat agtggtcagc agtgtattct aaaactgtat 82260
   taatatactg tagatgttac atttttgaag acttttgttt caatggtcat gtttccctac 82320
   tccagatttt tattggaatg gctctctttc tatattctgg ttacttctca tgcagcaaaa 82380
   agctatcttt tcttaccacc ttttctgata aatatgtttt acttatctct catttctttt 82440
   ttattttttg cttcatttct tctcataact catattactt cctgaaatgt ttgttcttaa 82500
   tttctttctt tccccactcc ttctccctct ctttttcttc ccttcactct tgattttctc 82560
   cctcactaca agtcagtagt gacttctgtg ttttgttcat gtttctttct agcacctata 82620
   atagcgcctg ctacatagta atgttcaata aatacttgac gaatggatga ttctctttaa 82680
   ttttccttct cttgatacaa atctattttt gtttttaaat tcgtttggtt aaaatattgt 82740
   taatttcttt aacttttgag agtctttaaa catgcagtta ttcctttctg attcttttgg 82800
   tagttaaatt aattttttga ctgtttacat tagtttgctc ttttacttag cctactttaa 82860
   tctaggggat ttgggatttc agcctgcatg ttccctttga ggaccgtaat tcttttctcc 82920
   catgttccct ttgtgttctg ctccctttgt gcgtattggt ttaatgattg catctaccca 82980
   gacacattcc aaaatcaggt ttccaagtgg cacagaatgg acagaatagc ttttgtcctc 83040
   agtgctatca gagcttactc actgatcctg ggggtgcttt ggcaaggtcc tgattatcag 83100
   gctgtctctg gtcactccca ccatcctagt cgtataggct gacataaagc aaaaccctga 83160
   cagtggtcac tattttcttg accactttta cataggtgag aaatctattc tagcccctct 83220
   ttaggcctgg ttgtattctc ctattctcct ttattcatgg gaagactttt tttctttttt 83280
   cttttctttc tttctttttt tttctttttt tcttttgcct ccgttgtact aagataaact 83340
   cccagttacc tctacctatt ttgggacctg gaatgcagaa ttgtcagttt gtgtcttcag 83400
   cacacttact gatttgcatt ttaaaagtag ctaagtctta caattcaaaa aaaatgatta 83460
   tcaaagtcgt gcttagaaca gaagggttgg gaggaaattt aatgcaagaa cttgactaag 83520
   tttttaaaat gttaaatata atacaaatat agaagagtgc ataaaataag tacacaaatt 83580
   agcaagttgt tataatgtaa acaccatgtt aaaaataatc caaatcaaga gatttaccag 83640
   cacccaaagt tcctctcatg ctccttctgt ttttataact acggtttgtc tctattgctg 83700
   tgtaatagtc cattctacca gagatcagca aaccatggcc tgggagtcaa gttcagctca 83760
   ccacatgttt ttgaaaacaa agtcttgtta ggacacagac ataatttttt atttaaaaat 83820
   tatttatagc tttttttttt ttttgctaaa atgaccaagt tgagtacttg tgacagaaaa 83880
   catacggctc agaaagccaa aagtatttat tatctagtcc tttcagaaaa aaaattgtca 83940
   ctctcctctt tatatgatta tatggtaata ttttatccat tctaatgttg ttaaagatgc 84000
   agattattat taacttctga tagttcatta aatgctacta aaataattca tcttcacttc 84060
   tcttggatca catatctgtg tctctgtagc ggtatattta ggaaaggaat gactgggttt 84120
   ttggtacact taccatcaat attatgagac aacgcaaaca gttttccaaa gtcatcatat 84180
   catatataca tatatatatg tattgcattt ccactatgag tgtgttagag tgaactttat 84240
   tctacatcct tggtaacact tgatattatt gaatctttta attttagcca ttttgatgag 84300
   tgtgtagttt taatttcatt ttccggttgg ctaatgagat tggttatttt tttatatgtt 84360
   tattggccat ttagacattc tgttttgtaa gtccatattc aaattgtgtg tttgcttttc 84420
   atctaggttc cccttttact tctaaatttt gagagtacaa attacctgat agtgtgtcaa 84480
   atttttcccc actgctatat aacgtcatat ttgtaaacaa acaaacaaac acctcaacaa 84540
   atacaaattc atgggtccat ttctagggct ctgtagcttg tttcaatagt attctgtcct 84600
   aagtcctgat acccagagaa gcaagtcctc ctgatttgtt cttcaaaaat atcttggatg 84660
   ttcttgaacc tttatctttc catattaatt ttacaattag cttttcagtt tacacatgca 84720
   tacacacaca tgcacacacc ctgttatgat ttttaatgtg gattaacttt ataggtcaat 84780
   ttgaaagttt tcaaaaaata tttttgcttt ggcttccagg aaactctgaa aaagattttg 84840
   attaatctgc atatttgttg gaactttcta tgtccttgac cttttattcc actgtttcat 84900
   gtttcctaag aattgcctga aattatgtgt ggagggaagc aaggcatatt aacaatttac 84960
   aattggagca ggagcaggag gatgtggtgt aactcagtac catcaatgtc tatctgatta 85020
   ggactatagt cttaaaacct gtgttacttt aaaaatacag atataatttt tagatccaaa 85080
   gcatgaagat attaatgctg gcaggcctga aatttgactg acttatcaat atttttaaaa 85140
   atcttagcag gtgtttctaa tagataagtc taggttgagc attgctgggt taggatgttg 85200
   tatagagagc agggcctctt gaaatgctga tagcagcatc ctctctggcc ttgaacaata 85260
   catcaagact tgtgtttgga gtcaaagtta cttaattttc aaataaatga attatgagta 85320
   tatacgccta aatctttgtg tactatcata aaattctgcc accctatgaa gatggattga 85380
   tgtgaacatg gcagttggga cttgtctcct cttattttta tttttttgtg catgaatata 85440
   gtcctaacaa ataaatccca tccttggtga acaatttaat ggttatgttt ctgttctcac 85500
   tgaacaaaca cagaagaaat atgccagact aaattaatga agtgaaaacg atatattcag 85560
   taatcccata cattttctaa tatagactaa aaacctattt tcttccatct tatcttcaga 85620
   cataatggta tgaggaaaaa aacaattcaa agaggaagtc tttaaggcaa tttctacatg 85680
   aagtaacatt tgttgggttc atgtgtcaca taacaggcat tccacctttt acaattcaat 85740
   ccctttaagc ggcttatgaa ttaggcagct atttctattg aaaaaatgaa gataataaaa 85800
   tcagaaaact taaataaata aaaagttaca ttcagtgaat gaataggagt agagtctgga 85860
   tttgaatctt gcttttggct tttgtgtgtg accacatgtc acattctttc caccacccac 85920
   agagacaaag cgacattcac attgaaggtg taagagatgg caatgaatcc atgacctcag 85980
   agaatcactt cactgctgca tgaaatggat aataattatt tcttctcctt tcgctggatg 86040
   ttgtggctta attcacattt gtaagatgtt atggataaaa tcactgcaat cgctaacagg 86100
   catttatgtc ccacagtctc tttgggcttc ttgcctttta tcttccagca tgccatagcg 86160
   atggtgctcg ctggatttat gaacaccgtg actgtctgtt tggaacactg agatgtgaaa 86220
   ttgcctccac attgaacaag atttggctct tggtggtagg agggttgtga ataaagtgag 86280
   cactggtggt acagaggagg acatgctgca acattatttt cagtaataat cctttagtta 86340
   ccaggaatga tcattttttc tgattcattg tttagtttga aaacaattaa caactaaaaa 86400
   tatcatacat ttaaagataa tatttaagta aaatatttgc atgtagaata aaggttttta 86460
   cttaccaaat aaaaatacta caccttgaag tttgcattct gctttactgt tttacatttt 86520
   agacagaaat taacatttga agtggtcaca aagaatgata gaatgaaagt cactcatatt 86580
   ctgtttgttc ttctttacag ttaagacatt atctggttta ttttgactgc tgtttagaca 86640
   tgagaataat gtggtcctac aggctctgaa gacatgaact ataatgcact gtcctctaag 86700
   tgagctcaaa tgattctaac tccttaccaa ctgaccctcc aaacaaacac ctgtagctga 86760
   gtctgtattt gtcaggggcc aacagtatga ctgggagttc ttaaagtaat ttgcatgtgg 86820
   aacataatgt ttattatagg ataggtcata aaaatgtgct catttgacac agagaaatgt 86880
   aatatcagat ctcaatagca gttaaaacac tccctagaag ctagaccaaa aaaagatttt 86940
   ttgaggagaa atatttttct ttgacattgt ttataattgg tgaagcacag caatgacaag 87000
   tagctgaatt tcagttcagt gtttcctttt ttaattatct acagacaatg aacatccttg 87060
   ctgtctgcat gtgggatgga tcacttcacc ctgtagccta acagcacttt ttctgtgact 87120
   gttatcacaa tccaagagac cttggagaaa agctgctttc ttacataaaa cactttcaaa 87180
   gaaactgagc aaagatgcta agtgtgcatg gcacatggca agggtgagca caaatggatt 87240
   caggcttcct tggaaaatca agaggataga gtgtgttgac caaaatatct gtgaatggat 87300
   atagggctag aggagtctgg tggggtcatg tgttgcaaaa gaaggggagg tgcccattct 87360
   gaaatgaaaa aagttaagtg aaaagtctac ttatatgtga ttcagggaca gagggaataa 87420
   ctgaaaaccc tccctctcca cggaggctct gggctaagtc agctgagtgt tgtgagtcat 87480
   gaaagccgag atttggagag tccagtgtaa gaaagcacag ggcatgaatg ccacaagcac 87540
   attcagtggg ggaagccagg tagacactga agtcagggca ttcaggggga tgccagcaga 87600
   gagacgtgga ttaggaaaat gatcataaga taaagtctta agcagagctt gaattcaagc 87660
   tctgccattt attagctata tccttttggc cagtttactt aacctctctg accattaaca 87720
   tcttcagttg caaaatggag taatgacatc tagtgtttct gggattgttg caaatataaa 87780
   agaataatat atgcaaacct cttgccatgt tgaaggtatc taggaaatgt tccctatcta 87840
   gctgtggcag taataaaaat aagcatgcaa agccacagcc agacctacag gaggagccca 87900
   gagctatgat ggcagacttg ggagaagctg gaagccaata gagaggggcc cctgaatgac 87960
   tcttgttgtt gaaaacctag gaccaaagtc aggagaatag atttcagctc tttccttgcc 88020
   actggctggc tgtgtgacag atggtttgct agttgtcttt cctgggcctc ctttagtttc 88080
   ttctagagca aggttattgt tattgggtca ctttgtacca gtcattttat caccaggaat 88140
   attttggtcc cgataatatg aatgataatt gtccccatgt ttaagctatg atgttaaaaa 88200
   catgctacct ctttttggtc tgtttgccta cctccatgcc caatccttac cacacctgac 88260
   cctatgctgt cctcactccc actgcacccc agagccttgc ttacccttcc acccaggtaa 88320
   ggtgcaaaga ttccatggaa ctgtgtttcg ggcccagtag ttagtaacaa gggaaagaaa 88380
   atgtatcaaa gattcattcc ctagatggtt gtgttgttga ggtgggagaa tcggacttcc 88440
   tctgggtaga tagccgtcag ggataaagaa agctcaggga catgtttagg atgctcactg 88500
   caaaaattat ctaataataa aaaaattcac acataaaaat aatggttttt agagtcccaa 88560
   aatggtggag tttttatttc cctggttttc tttaaaaagg cacattttta ttttctatct 88620
   gtccatttag tcattcatgt atttaacaaa tatttattta gttcttctta tgcccttggt 88680
   gctgttttag gtgttgggga taaaacaaga aatcaacagt ggatacaaca ataaacaaag 88740
   agataactac ctatagcata tcacaggtat tccaaaagca ataaacaaac caggataaat 88800
   agttattgac gtgatggtaa agctgcgtat ctccaaattt cagtggctca tagcagcaaa 88860
   cattgttttc tcttgcacag tggcttgtgc ttggctggag tgagtgacct caggctgctt 88920
   tcacactgag ttctgctcca ctgtgcttat cctccttata ttagcagcca ccggtgctgc 88980
   tagtaaatgt tctcttcttg tagcatgata ggaacacaag ggggtgaagg gaaagtcata 89040
   tggcttctta ggcttcaaca agaaattgca aaattctatc tgaggcccac agtctagtag 89100
   acaattcatg ccacgtggat aagcccaaga tcaattggga tgtgaggggt acagtcagtt 89160
   catagtgggt ggtactgcaa tgtcatatgg caaaggcata aatgcgttat tctattaagg 89220
   gaaggagtaa agaactgaag acaacagtgc aatctaccac acagggaaag gatgatgagg 89280
   aatgtcaggg taaggggggg tggcattttg tatacagtgg tcagagaaag ccttcaccat 89340
   agatgacata tgaccagaga ccttgaggag gtgagctggg taagtaattg gaaggagagt 89400
   attacaacag agaaaagagc tagtgcaggg gtttgcttga gaggtcaatg gggccgaagc 89460
   agtatctgag ctggaaagat ggaggaggta aagtccaagg tatattaggg gaggagcatg 89520
   tgtgataggg ggctgaagca ggtaatataa gcccttataa aggtttcagt ctttactctg 89580
   aataaggtga gaagatattg ggggactttg atcagaagaa agatgtgaat tgataagtgg 89640
   taacaatata actgatggat gtattgagag tgcatgcaga gagaccaagg tggaaggaag 89700
   cagtacactt acaagtttat tgcattgatc cctatatgga ctgatgatag cttggtctac 89760
   ggtggtgaga aatgataagg ctctggatat attttggagg tggtgatgat aagatttcaa 89820
   ggtgtatcag atgtaggata tgagagacag aaaggagtca aaggtgattt ccaaatgttt 89880
   ggtttgagac tggaaaggtg cattgccaaa aaataaggtc aggaatattg tcagccacga 89940
   tatttagaaa atctgattct cattggtgag gataggaatc tttcttcccc tgctctgcaa 90000
   tgctgagaag tctttatgaa aacaacgcaa gtaccacaat aaaatactat tcaggttttt 90060
   atatgttcca cttaaaccaa accactgtat catataagct agaattatgt ccacattaat 90120
   ttttgttgta ctttgatttt aaaagtcatg ggaaatggat cctagaaata aataattcat 90180
   ggaaactaca ttattaagtt aattctaacc aatagcatgt cattaagata caaatgtgaa 90240
   aatagttcaa ttttattttt taattaactc ttcagaagta atagccgaca attgtccttg 90300
   atcaggtcaa atgctgtttt taaatgcctg acagttttta agtctttaat ggaaatcaat 90360
   ggcccaactt cctgaaatgt attaattaca tttatctttc taggcagttt ttagtaagga 90420
   ccaggactta aaaataactt ccaaacattc tctggctaaa gtctattttt ttcttaataa 90480
   attcacagtg ggcatttact attctggtat cttccccaca atatccaaac tttgttcact 90540
   aattcctttt ccttttctct ctacatccat ataccacttt atctttcaag catcagcgta 90600
   aatcccattt cctacattca gtgaccctgc ctccaactca cactgatctc atatacttca 90660
   atgtgatatt ttatatggaa gtcattgttt tttgcataag attgcaaagt gatttggaaa 90720
   aaaaaaccaa aaaccttggt aagcaaccat tcaacaaata ctgcattttt gtttttcatt 90780
   tctcattttg tatccctttt tatactgatt ctcggcagga gtatccaaga gtggaaacat 90840
   gcaaggtttc agaatctttt gcaacttaat taggctctcc gtggaatatt ctgaatccct 90900
   ggaccattgg agcagctgaa cctggagttt ggaggaaaga tgggactggg aggcagcgag 90960
   tggggatgaa ggagcagcct gtgtcccctt catttgatgt ggtcctggca tgctatataa 91020
   aaggagtggg gattgaggcg tcagctgttt tgctggtgtc catggcatca tttactggat 91080
   gaaacttgca ggacgctgag gtttggacag ggaatggctg agccaaaact gaatcttcct 91140
   tgctaatcgc tgtgcacaga gaggataaat acaaatggat gcaccccaag aggcagtcac 91200
   tgtcattgaa caaagggaaa ttagttaatt attgctatag tccatgaagg agggatctgt 91260
   ctgtcctctt tccaatcctc tcctcacaac agtggtagta gtaggaggag gtgagatgtg 91320
   gaaaagcaga ccccacctcc cctccatgcc aggctgcatg agctgcaagg ctagtcatat 91380
   ctggaagttt taaattgaaa ataaaattaa agttagtaag tgaacaacag tgagccagca 91440
   ctttgggagg ccgaggcggg tggatcacga ggtcaagaga tcaagaccat tctggccaac 91500
   ttggtgaaac cccatctcta ctaaaaaaaa caaaaattac cctggtgttg tggcgggcac 91560
   ctgtaatccc agctactcag gaggctgagg caggagaatc gctggaaccc aggaggcgga 91620
   ggttacagtg agccgagatt gtgccactgc actccagcct ggcgatagag ccagaatccg 91680
   tccccccgac aaacaaataa aataaaataa aataaaataa aaaagaagca ataactcttc 91740
   cagtaatcaa aaaagtctta attttatgaa ctcaggatga gattgtgtta agggaaccca 91800
   ccacccagtg agaaatgatt tgaccaaagg cagatagtgg tatctgtcag gacaaataaa 91860
   agcatctcat gcttatagcc caataagtta aattttctca ttaaattggt gattttctga 91920
   agctagtttc cttctttaga aaaaaaagca ataataacaa tctgttatta ttactatgat 91980
   ccactggctg gtaatcatga gccggacgtg ctgactctct atacctagca gtctcaaggg 92040
   cacctgtgag cggtaaaatg gctttcatcc atctcacaac acttgaactg tgcacatgtt 92100
   tagcgaacaa atgtttactt ctcacacttt gtttttatct gtagtattcc tgtaagctgt 92160
   gaacaggaat tcctgtaggt gtgaacactc caaggtgcca tacacttagt ttgacatcaa 92220
   aggaaaacta ctcttatctt tccccttgtg gtctttgttc tgcactgttt gtctcaacac 92280
   cacatacttc cctcttatac cgccaatgct cccaactttc tcaatcatta ttcatcctta 92340
   aatttcaggc cttggtgttc ctttccgctt gacacagatt tcctgcataa aaccttggct 92400
   tctcttcaat caccatacag gttttctggt agtcttacac tgctgtaggt aaatattatt 92460
   cagttctatc caacaaagta cacccaccag ttctgttcag gtttgttaga agtcaattgg 92520
   ttatgagtga tttttctata cagtcatatg taccaaattc tctcttatgc attgctgaga 92580
   agtattatag tctcaatact cacactgaaa aagttgcttg ctacttctca tctgcctgtg 92640
   attacatgaa aaagctttgc atgtaggtca gaaacaggga aacataagga gccaacactc 92700
   atttctaaca ttaatggaat ggagaataag gacattcagt ttagttgtaa ataaattaaa 92760
   agaaaataat ttaaaaagct attgtcttca gcatttaaag ttttctcaac acttgctcag 92820
   caatgctatt atttctcaga ttctgaaatg gctaccctct tatcatttta atgtaagtga 92880
   ctttctgtat ttctttctct ctttttaagt agttattctt caggaatgaa catcaggatg 92940
   caaagaacaa ttcaccacct aatcagtcat ttatggcaga aactgaatca aaatgattag 93000
   acttagtgac tctgagtgta tactttgatt attgcttttt caatttgacc tactgaagag 93060
   aacattattt tatgcctgaa tttactttgc tctacttttt tagttaaagt cttataaatt 93120
   aaaaaaggca tttaatagaa ataaaatata gttatattgg catgagggag attttccata 93180
   taaatcgatg gaaagaaatt gaatcaacac ttacaaacaa cattgttttg gttatttagg 93240
   attacgttta cccactgggc tagcaagtag atgacatttt gcctcatatt tgatattcga 93300
   ctaattcttt tttcagtgtg gaatgaggca ggtaaaatgt gcttaagatg ttctaagaag 93360
   tcacaaggta ggtaaaaaat tgtgatcttt gaaattggcc aaatctgtat ttgggttcac 93420
   atttcatcac tcaattgttg agtgtctttc ttgaccatct tgttggacca ctctgagttt 93480
   cactcttgct gttgaactga ggctaataaa cccttctcct caatagacat tgtctacaag 93540
   gtgagcagtc aaaggcattt atatgctatg caatgatttg aagcacactt tcaaatatat 93600
   ggttcaaaac aaactcacga tgtatatatt atattttgtc atcactcttt tgagctgatt 93660
   tgatgagagg cattttaatg tagccaaaat aaacaagcca ttgaacatta accttgatgg 93720
   acgatgcaac tcaaactaaa agttccttga gaagtaactg atctgctcaa ggtcacacac 93780
   taatcaggac agaggcaagg cctgactctt ggtctcatga gctaatattt atttatgttg 93840
   accatgcaag ctatatccaa aaatagtaca gaattttaga gaattttaaa ctctgtcata 93900
   gatggatgtt tgattaatcg agcggactgt ttttgtttaa tggaaacaat taaaattatc 93960
   tttctaaaat aatatattga aactgactca caatactaag ttatatgagt agaatgacat 94020
   ctgttatagc cacattctat ttattatctt taatatatta ttttaatata cttgacattt 94080
   tatttactct ttaagtaata aatatcaaat aagagcaata aataaagctg atttataaat 94140
   tactgttctt ctatatgagt taagatgcag tagaatatac cctcttgaga ttactagact 94200
   gggttttagt ataaaacatg agtgcaattt gcccacatag ataggttatg ggaagatagt 94260
   tttgaaactc aggaagagtc actctgaaga attgagactg gagttctgcc aagagatgtg 94320
   caagtcaaat ggaattctgc aactccagag agggagagag agagtagagg cttggaagga 94380
   ggaagagaca aagttttcag ttctcactag gcacagttga aatttctact ctgtgttttc 94440
   ttgaaattta agtgactctt tccaatcgac tcttatgtaa gtcagtctga atcacttttg 94500
   gtctttgcaa ccaaaggatg tattaaaacc atcttttgct tgtcagttaa acatatggct 94560
   gttgtttaaa tgtaagaatg gagagaaaag gtgccacgta attcaggagt ctcttcatct 94620
   ttgtcacaga gccttgaact atcttcccaa taaatatttg atttgaatct tcagcactga 94680
   ggggaagtgt cttctgaaat aatacaggct aaattgttat gtagcagcca tctatacaaa 94740
   gcaaattttt gtaatataca aaagagggga ttttgctatt atatcagttg gcaagaatca 94800
   ttatcgtgat tggaaataaa tagatacaga gagaaaagaa tgagatttaa aaaaaaaatt 94860
   cagcttcaag gaaattggat tttctgttga gtatggaatc gagactttgg aaactaaaag 94920
   gctagaaaaa gattgcttta ttaagaaaag taaaaataca tttttttcat ttgaaaaatg 94980
   cctactagac aataaaacat ctggctaaaa aatgaaatct tcagtgacac atctggatat 95040
   ttgctccata agatatcaaa catgaggata attgaaaata tagagctaaa ttacttaaat     95100
   gttaagctcc catccagatg tttgatgcta tgttagatga aaaaactctt tacatgccag     95160
   acattaaagc aatcagggat atctaaatgc caccataaat aaaatagcat tgcaaatatg     95220
   gttcactgga aagaatgtat taactgataa ttacagacta cacaagaaat ttgcttctta     95280
   gttaaaagtt tttttaattt ttttatttta ttttattttt ttccttctga tcagtagtaa     95340
   ctaataaaac atggaaacac gaaatagaca tccgtagacg acaactggtc aggcttattg     95400
   acaaacaagt ggatttcatt tccaaactat ttttttacta tatatagaaa tatcaatgag     95460
   ttagaggttt tggctcctaa ttagagaata taaaacttga tttttggaaa tgcattggag     95520
   aaataggaca aaattttgaa tttcaattct ttggcaatat ttataaggaa taaaatccaa     95580
   aatagatgaa actgatataa tttagcttca agaagtacca gatgacaact gaagttggct     95640
   cacaaattat aatctatggg agaatgggaa gcagtgagag ttaaacagtc aaggacagca     95700
   tcagtcgttt ggctgacact gcagatttat acagacacag ttttaaatcc aggcagatct     95760
   gttagtgcag agaagaaagc actgtatctc ttatgtggtt caggcaggaa gcaagggtta     95820
   taatctaggg agtcagaaaa gttaaagagt gccaccaagt tatgtgagtg gacagagttg     95880
   aatgttgcct caaggacaaa aaaacaatat cttctctgtt ctacttttta aaatggactt     95940
   ttaacattta ggccacatta aaatttttgc aatcaagaca gattcatcag gatgttggta     96000
   acaaattcta atacatctat tgtaagtgtc tactcaaaat gaagataaat attttaaatt     96060
   ccatttatgt aagaggaaaa caggggctca gagaattacc atgacacatc caagagtaac     96120
   actctgtcat gaggaagcag agtatgacta caaatccagg cctggctaat tcaaaagacc     96180
   acactttttc acttgtcatg ctgactctta aaacatcaaa acctgatcag ttgctttggt     96240
   ctgaactaag gtaaaaattg ctacagatga actgtttcct agattattgc tacacataac     96300
   tctttcctag attaatgcag atgtgtagat gacacaaagt gacttgagca acaaatctga     96360
   ttactgaaaa gatttaacat attgtacaga ctactgtgtg cctgaacaac agaaagtgct     96420
   gaataacaaa aaaaaattgt aggtttaaaa catttaaaac aatcaaattc tttaaaacat     96480
   gcatgtaact ccgtgatact gactcagtgt ttggcacaca gaaccctgtg aatataaaat     96540
   gaatgcccgc ctgaggaaac taattttata tctatatcta catctttatc tatagataca     96600
   tgtatatata tttttatcaa tatcaatatt tttcttgatg tcaaggtcag gatcgtggct     96660
   ggtgtgtcag ggtccacaaa tgtcatctca ttcaggaaag tatttcaggc ttaggtcaaa     96720
   ttgtcagaag catttgaacc aaagcaactc catcttaaag aggagcttgg taaaatgaga     96780
   ctgaaaccta ccgggctgca tacccagatg gttaaggcat tctaagtcac aggatgagac     96840
   aggaggtcag cacaaaatac aggtcataaa tacctggctg ataaaacagg ctgcagtaaa     96900
   ggaaccagcc aaaacccacc aaaactgaaa tggggatgag actcttctcc gatcgtcctc 96960
   actgctacac tttcaccagc gccatgacag tttacaaatg caatggcagt gtcaggaagt    97020
   taccctatat ggtctaaaat ggggaagcat gaataatcca ccccttgttt agcatatcat    97080
   caagaaatag ccataaaaat gggcaatcag cagcccccag ggctgctctg tctatggagt 97140
   agccattctt ttattccttt acttccttaa taaacttgct ttcacgttgc accgcaggct 97200
   cgccctgaat tctttcctgc ctgagatcca agaaccctct ctgggggtct ggatcaggac 97260
   cactttcctg taacaaaatc aagcatcttt ccagacaaag cgtggtttta cccattctag 97320
   aatccccttc ttaatttata aggccgagac aagtgtccag gtaggcctta gctgcctggt 97380
   gaatagtaga gaaacagttg gaattatatc tgcttggtta aaatccaaga aagcataagc 97440
   caggagtcaa ttggaacctc tcagctctta aaagtaatat tggaatattt tgttccagta 97500
   tttatctatt tcactttgca ctgcaggctc tccctgaagt ctttcttgcc tgagatctaa 97560
   gacccctctc tgggggtctg gatcaggacc cctttcctgt aacaaaatca agcatctttc 97620
   cagacaaagc atggttttac ccattctaga atctccttct taatttataa ggctaagaga 97680
   agtgtacaga aataaacttc tttgatagtt tttagctgca acagaagtgt gggatggggc 97740
   agtggagtga agagggaatt gtgttttagt ggtgaacttt ggatcagaat ctggcttctg 97800
   ccgatttgtt taattgctta actgattttt ttcttttttt tttttttgag acggagtctg 97860
   ggtctgtcgc ccaggctgga gtgcagtggc gcgatcttgg ctcactgaaa actccgcctc 97920
   ctgggttcat gccattctcc tgcctcagcc tcctgagtag ctgggactac aggtgcccgc 97980
   caccacgcct ggctaacttt tttgtatttt tagtagagac ggtgtttcac cgtgttagcc 98040
   aggatggtct cgatctcctg acctcgtgat ccgcccgcct tggtctccca aagtgctggg 98100
   attacaggcg tgagccactg cacctggcca gttgattttt tttatccttt tgtgatgcct 98160
   gaattttggg cttgaaaacc cagatgtcaa atacaccctt ttctttaata tttgcagaaa 98220
   gtcctcattc atagacattt gtagtttgcc gcttttatga gagatacagt aagaggaggg 98280
   ggtggaagag gggtactcca catctaaggt aaccatccca ctggcccact tttggccacc 98340
   cattgtgaga acttttccca tgccatctca tccctagcct tgaacctgct cccgctccac 98400
   tttctccctt ttcccttcac cagtcctcct cttttttcta tatcttcctc tcattttcat 98460
   atgcagtacc acttattcat cttttgtttg ctgctggctg ttgttttgaa ggttttaagt 98520
   tctattgcaa ggtgcttatg aaatagtttc ttaaattaaa aaaaaacttc taaagtaact 98580
   ttatggtgca actattattt tatcttattt tataaaaatg atatttttgg ggactaggca 98640
   cagtggctta cgcctgtaat cccaacactt tgggaggcca aggtcgttgg atcacttgag 98700
   gccaggactt ccagaccagc ctacgcaaca tggcaaaacc ccgtccctac taaacataca 98760
   aaaatcagct gggcctcgtg gcacacgcct gtaatcccag ctacttggga ggctgaggca 98820
   ggagaattac ttgaacccat aaggcaaatg ttgcaatgag ctgagattgt gccagtgcat 98880
   tccaccctga gcaacagagc gagactctgt ctcaaaaaac aacaacaaca acaacaacag 98940
   atctttttgg tattcaatca ggtattttta aaccatattt aatacatcta actagtaact 99000
   tttagatttt atctcattaa tttaagtgtt ttgatgtctg aatatttaac atctggacaa 99060
   aatggcttca gtaaaggcag gtgtatacag tattttgtgt aattccatta taattctatt 99120
   ttttccagtg caaattcttc aatgttctca tcaaaaaagt tatctatgga aaggaacacg 99180
   gcttcctttg cattctcatt tgaagcagga agtgtgaaag accgcgctag actcagtaat 99240
   tggctgtcat acactattta gaacgctgct ttgaagaaga cctgcttcct cctttactct 99300
   tctctgtttg caccacaaaa tattcaaatg gaaagatcaa agtggaatgt tcatttttat 99360
   tgcctctatt tcatgtgtga tacttattct aatccttctt ggagtaaggg tcaaagattg 99420
   ggggatttga aacataaaac agaatttgaa gggggatgga ttgagtctgg tagctgacca 99480
   ggagactgac ttaaattaga agaagctgaa ctgtagtatt caaaatacag gtaaaacttg 99540
   taatgttgtc tttatataca tgaaagcagg aattcatcat gtgagataat aaaatgcaag 99600
   gtctggaagg aaaaaaaaaa catgagagaa agatatcctc atgaatttgt ttactgactg 99660
   cttggtgaag ttatggatta catgcctgca gtccaaaatg gaattctgcc tctgagagct 99720
   tgcctttgcc tgacttctaa acacaattgc ctggaagaag atggtgatgc gtctgtaagg 99780
   agactggttt tacaggaagt gttacttccc gtcacactaa ttctcctgga gctaacattt 99840
   caaacacttg cacttatagg ttatttctct ttgtagaaca gaaatgagcc ttcattttgg 99900
   ctaccagtac tttagaatac ttggtgaaaa gtttctttct ctccctctct ctctgtctct 99960
   ctttctcttc tccacccttg cccatcttct ctcttaatac attttatttt attttatttt 100020
   attttagaat cttacctgga ttctccttaa aaatggtgca acataaatat atactcaatt 100080
   ttgtttttct ttgctgcata aaacagtttt ttttaattaa agagaaatag ctataagtgg 100140
   tctaccatca acaaaaacac aacaaacagc tcctttgtac cactgtcatg ttgccttcca 100200
   tcttacagtc ctcagtttct tactgctcac ccccagtggc catagatgaa actaaaatct 100260
   ctggatttcc ctggtttgtg actctcaact ggctcagcta gcagggattg ggcactggga 100320
   taaagcctcc cattttctgc tttctttgct attcttctct gagttggtct ctcagctgtc 100380
   tggtaggagt cagcatgggc acattgggta ggtctcagga aggcatatct cacaagaagt 100440
   ctcaggacac ttctctaatg aggcatccgg cagagttatg aatggagcca gaaacgactg 100500
   actgggctgg agaggggcac ctggcgattg gcagagcagt caaaggttgt caaaatgcac 100560
   aggctggaat cagattgcac ttgatatctt aggaccgaat gtttcatgag ttgagatact 100620
   gtgaagaaaa ataggtggag aatatttctg agcaattggt tggtttacag gattatttcc 100680
   cacgtctttt ttataacccc tctttccacc tgaaaaactc ctagttaacc ttcaagattc 100740
   tgccttgcct gatacaagaa ttaacttggt tgctccattc aggtcccttc aaggaaacgt 100800
   catattgcat aagtgcccca attgatcctt ttaatcatga cctggtcacc taggtgtcca 100860
   catcttccct tgacctcagt cttttccctg tgtttccagt gcaatcacag ttcctgatat 100920
   gaggattctt aataaatagc tgctgcatga atgaaaaaca agacatagag aggaagcact 100980
   tattagattc cagactccct gctatgcatt ggggattcaa caacaaatgt cacaaacttg 101040
   ttccaaaata ttatttgtac agtgaggcct gtttattgcc tggtatgggt actgttggca 101100
   gatacggggc ttctgggtgt ttaggaaagg tcaggtacct tcttcctgag agttttagaa 101160
   agactgtgtg gtctattttt taataggaag ccttaagagt ggagtccagc ggcacatggg 101220
   aaaatataac attgaaacta ttggaaagca tggcatcaat gtggaaaaac ggggtcctac 101280
   attgcaattt aagttgttgt agatgaaaac agtaaactct gaagagtggg gccaacatta 101340
   acctgctggt aggggttcct ctcaaggtca ggtagccctg agatttatgc taaaggacaa 101400
   ggccagtgct taccacattt agcaacttct ctctcggtga gtcctagttt agcacgatat 101460
   tgccacactc aacgactcga ccccacgatt tcaaatggca accccgttga agtggatatg 101520
   gagttttgct tctgtatgtc cattgaaggt catgtatgaa gatgttacca atgttaagct 101580
   attctgaata tccccagtat ctcttcaagt gcaaaactta cagcattata aaataagggg 101640
   aagatatacc tgaaaaaaca tgaagatttg gtaatttaaa aaaaagtttt aaaaacaaaa 101700
   attgcttaat taaagtccta tatgaattta ggcaaaatac acaaactagc tgatctccag 101760
   attccccata tagatgacag gcaaagaatg tctacaatta ttgataagat taaatgagat 101820
   aatgtgtata tttcttgcac attgcataaa acttcaataa acatttgcct acctaaattt 101880
   ccattttctt tcattatata cctctgtata aggcccagtc agaatgcctt tccagatata 101940
   aagcatggca ataactagga aatttggaat actggtaaaa atttcttgta tttttctttc 102000
   tttctcttta tggggttcca tcaaattttg ctggaccatg tcacttggat atttttagat 102060
   gagaccttaa aaaagtcata tatgggccgg gtgtggtggt tcatgcctgt aattccagcg 102120
   ctttgggagg ccgaggtggg tggatcacct gaggtcagga gttcgagacc agcctgggca 102180
   acatggtgaa acaccatctc tactaaaaat atgaaaatta gccaggtgtg gtggcgggtg 102240
   cctgtaatcc cagctacttg ggaggctgag gcaggagaat tgcttgaacc cgggaggtgg 102300
   aggttgcagt gaactgagat cacaccgttg cacaccagcc tgggtgacaa gagtgaaact 102360
   ccatcacaaa aaaaacaaaa aaacaaaaaa aaaccaaaaa agtcatatat gttacgccaa 102420
   atatggctga taaaactgaa gaggttgaac atgtaagtag tagttgtgct cagacattag 102480
   agtgcctgtg aagaactaca aagcttgcta tcgttttata catttgttat ggtctgaaga 102540
   attgtatttc ctcaagattc acatgttgaa acctaacccc caaggtcacg gtattaccag 102600
   gcgaggcttt tggctgatta ggtcaagagg gctctgccct taaaaatgaa attagtgccc 102660
   ttgtgaaaaa cgcttgaggg agcttgttca ccccttttgc catgtgaaaa tgcagcaaaa 102720
   tgtgttatct acaaggaatg ggcccttgcc gcatgttgaa tctgttggca tcttgatctt 102780
   ggactttcca gccttcagga ctgtgaacaa tgaattctgt tgcttataaa ttactctgct 102840
   tataaattac tctgcttata aattctgttg cttataaatt tgttacagct gcctgaatag 102900
   atgaaggcac cattcgagtc tctggatttt actttcccaa aattcagtga gttccagtat 102960
   cattctttga gaaatccaat aattgttttt aaccttaaga ttaacaggta ggaaagtgac 103020
   acacacctga aacctgttac tctagtggta gaatctcatg gtttgcaaac gtactgctga 103080
   aaatatttcc tgcatatgaa aggtactctg aagcccctca gaagacacca caccacccag 103140
   actgctacta gataacatag actccaagaa tggcatttgc taatgtgtgt ataaaatggc 103200
   attttagaat tgcctaaata tccttattaa cactttctaa agaattgctg ttaactcttt 103260
   taaaatcaat tagattgccg aaggaagagg gaggggatag gtttaagtga ggtactacat 103320
   cacatagtgt tggatacatt ttccaaaaag ataaatgtaa gtctttttga acacataatt 103380
   tttgtggcca tattgtctct gaataaaagg acaattgggt tgtcttcaat ctggagagtt 103440
   ctagttgttt tcattttttt tgcctttatt ctccccagtt tcttcatgat gatgcatttg 103500
   tttatagcct gcacaccaca tctgatacat ttttaatctt tagtgagcca tgacaaactc 103560
   agaaatgatt agaaattcca aaagaacatg ttttttaact tcactgagta cttttttttt 103620
   ttatttcact ctttgttttc tttggaaaac aaataaaatg tctgcttcct cttagccata 103680
   tctctctaaa gtgttctgtt gtgtgtcatt agccaacagg cagctgggta aatgctggcc 103740
   cacatccaaa atgatgattg cttttcataa gcactgtctt tgctgttggc tttctgacta 103800
   atggcttctg cttgtaatgg atacaatgta gtatgattaa ctctcaggtg attaatagat 103860
   ggtgtttctg agaactaaat aatgaagaag caattaaaca aatctgatat gctgtcttta 103920
   ccacaaatca aataacactt tttcttccta ttccttggac agtggtttgg aggtcataaa 103980
   gctgttctga ctcacaaatg ggagactttg gcaattgttt taggataaat aaagacagga 104040
   aacatagtac accctcttca ccatcatact cacctgtgtt tgagattttg tagtgtgtga 104100
   tatgaaaaat aggtgtcatt taatccgtga gccccaattt cttcatctct aaaacagtga 104160
   ttataataga taatttgtac ctttaaatga agattaatga agattaaaca acacaatgta 104220
   cacaaagcat cccacaaaaa tgccaaatac atagtattca gttaatatat gttggtttca 104280
   aatacctccc tttcttctag ccttagcact catttttctg gaagcaaaga atattaggag 104340
   ataggatgag gattgtcaga aactttcttt tcttctctga ccttcagtaa ttcagaaaag 104400
   aaaaagattc atccctctac taacatgaaa ataattgcca tttgcagccg tgataatcat 104460
   tttaagttat tagtattaaa aaaggcatat catagattat cgtatttcta gcctccaata 104520
   tcaatttcta tactccctgt tacttggcct tggctgtgta gaattttacc atctcactag 104580
   aaataataaa cagcaaacat caaaacaagc aaaaacaagt aagcaaacaa attataggta 104640
   attaatatat tttatacttt aaacatttga agatggtgag tttttttttt tatttttatt 104700
   ttttgagacc gagtcttgct ctgtcgccca ggctggagtg cagcggctcg atcttggctc 104760
   actgcaagct ccgcctcccg ggttcacgcc attctcctgc ctcagcctcc ctagtagctg 104820
   gaactacagg tgcccgccac cacgcccggc taattttttg tattttttag tagagacggg 104880
   gtttcaccgt gttagccagg atggtctcga tttcctgacc tcatgatccg cccgccttgg 104940
   cctcgagatt tatttttaaa cggcagaaat aggtggctaa tttgtcttgg aacaagtaac 105000
   accttttgct tggttatgtc tcatgaacaa acaatcccac aatctcagtg gcttatagca 105060
   acaagggcta acttcttgct cacgtcatac catagctgaa atcatttccc aattttatct 105120
   tttaccggtt ggagatgaga aataattaca cttattgaac ttgctaataa atttgttagt 105180
   ctttatctgc agctcccctt gttttctcaa ctttatgttg aagtataatt tacgtatcat 105240
   aaaataaata gttaagtatt tctattaatc agtttttgta atagcatgca ttcatgttac 105300
   caccacctaa acaacagagt tttttccatc acccacaaac atattctttg cttctttcca 105360
   ttcagtctct gctgaggcaa cttctactgt ctgttagttt tacctacttc agaagttgat 105420
   ttaaatagaa ttctacaata tgtgcttgta tatgtctgac tctgtctagt cagaataatc 105480
   tgtgtttttt acattcatga tttgtatgta tcagtagatg acatcttctt tttgctaagt 105540
   agttttccac taaatgaata tatataacat catattttat tttcttattc ttttttgatg 105600
   accattagaa ttgtttacag gtttggacta ttatgaataa gtcttctgtt aattttgtag 105660
   aagtcttttt tacacgtttt tattcctctt gagaaattac ctaggagtag aagtggtgaa 105720
   tgatagagta ggtgtatatt tgactctata aacaactgcc aaactgtttt ccaatgtggt 105780
   tgtgtcattt tacattgtta ccagcagtat aatagagttc tggttagtcc acttttcatc 105840
   atctttaagt tgtctttcat tatctcaaca gtgtctttta aagtgcatag gcttttaatt 105900
   ttgacaaatt taaatttacc agtggtggtt tttttttttt tttaataaat tgggcttttg 105960
   gtgttagttc ttagaaatct tttcctaatt cctgctttct tccagcagat ttttagtttt 106020
   tattatttca tttggaccta cgatataatt tgagttaatt tttgtatgtg gcacaagaaa 106080
   tgaatcaggg ttttacttgt gttttcgttt tttgggtttt tttgtttgtt tttgcttttg 106140
   cttttgtttg cttttgcaag tggatatgct attgttctag caccatttgt caaaagatca 106200
   ttctgtcttc actgaatagc ctttacatct ttgctgaaaa tcagttaacc tatatgtgtg 106260
   agtttctttc tgcaatctgt attctgtttc attaatctgt ctatatatat aacaaaacac 106320
   gctgtctttc ttactataag gcttgaaatc agatagtttt caaacttggt tcttttttct 106380
   ttttttaaag ttgttttggc tagtctagtt tctttgcctg tccaaataaa tgttataatt 106440
   aacttgtcaa tatcaacaat acaatcttat gtggttttta ttaagatagt attgaattgg 106500
   gattgtataa agatgaattt gaaaagaatt aacatcttaa caatattgca tcttccaatc 106560
   cataaaaatg gcatagctct tcacttactt tactctcttt acttttcttc cagcaatgtt 106620
   ttgcacagtt ctttcaaatt tttaaatcac atctatccct aaatatgtca tgtgttttaa 106680
   atggtacata taattttcgg aaaaaaacca tttttttaaa gtcatttaat gaaagtatat 106740
   ataagtaaaa tttattttta taacttttta atttgttctg tatgtcttga aactttgatg 106800
   aacatattta ttaattccag tgcctttgtt tgtagatttc actccatgtt ctacaaagat 106860
   gattaatgcc attagtgatt aaagaaaatt ttagttcttc ctttctgatt gtggtatctt 106920
   tttctcgtgc cttaatgtac tgatagggac cttcagtaaa gtgttgagta gaaatagtga 106980
   gagcagaaat ctgtgatctg ttcttgatct tattaaaaaa ttcaatcttt taccataaag 107040
   cttgatatta actgtggagt tttccctaga tgtctttttt caggttgaaa atgctccctt 107100
   ctatttcgca tttgttaaaa gtttatatca ggaatgaata ctagaattgt caaatgcttt 107160
   ctctctgtta agcggctttt ttctttggtg gtttagtaag atggtgaatt ttctttcttg 107220
   gctgtcaagt gttaaagaat taatgtttgc agtcccaaga aaaatccatt tggttatcat 107280
   gtactattat ttttatatat tgttgaataa atagcacttt ctaaaatttt tttcagaacg 107340
   ctgacatttt attcataagg tatgttagtc tatagttctc ttttcttgaa atatctattt 107400
   ctggttttgg tttctggatg atgctggctc atagaatgat ttggcagata atacccactt 107460
   ttcagttttc tgaaggtttt ttgtagattt gattttattt cttccttgaa tgctggtggt 107520
   ggcacttatt agtggtcatc tgggcctgga atgttctttg tatgagtaca atttctttag 107580
   taaatataag actatactaa ttatttgttt ctttttgagt gtactttggt ggtttttgtc 107640
   tttcaggaaa tttgttcatt tcatctaagt tgtcaaagat attggcaaaa ggtgttcatt 107700
   ctactttctg attattcttt tagtgttttt aaaatctgtg gtaatgtcac tacaccattc 107760
   tcttccttct gattttgata agttctgtct tctctcttcc tttcctaatc aatctgacca 107820
   aaactttaac agttttaaaa taagcttaat gatattctta aataactaac catcatttgg 107880
   tttcctttca acaatttgta taaatttaag gggtacaagc gcaattttgt tatatgcgca 107940
   cattacctcc taatgaagta ttgactttca gtgtatccat cacctgaata atgtacatcg 108000
   tactcattaa gaagtttctc aacgccttcc ccactttcac ctcaccccta cttccaaatc 108060
   tccagtgtct atcattcaca ctctgttgtt tttctgtttt gtatttcatt gctttttgtt 108120
   ttgatattta ctatttttaa aacttacttt gggtttaatt ggctcttctt tttctggttt 108180
   cttgtggtgt acactgagga cattgttctg agaccattct tcctttctaa tactgagact 108240
   gagactcaat gctctaaatt tttccctgag cactgtttat cagaatccca taacttttga 108300
   catttcattt ttattcagtt caaactactt tctattttcc atgttgatgt tttaaagctt 108360
   tgttatttat ttaatttgaa gctatttgga gattttccag tgacttctat ttttcattcc 108420
   aatttagtta ttttcagaca aaaaattttt tcacaatttg aatctttaag tttattgaga 108480
   attgttttat ggaaaaacat atggcctaac ttgaaaaatg ttccatgtac acatgaaaat 108540
   aatgtgtatt ctgctgtcgt tgggtagagt gttccaccca ttttaatttt gttaagttgg 108600
   ttgatggttt tcatatcttt aatagccttt ttaattctgg ctacttattc tatcagttac 108660
   tgaaagaggt gtattaaaat ttcctacaat aattatctgt ttctccatac aattctatta 108720
   ggttttgcct catatatttt gttattaggt acgtaaatgt cgtaaactag catggtgtct 108780
   taatgtattc agtctttcat ctttataaag tgacttttac ctctttgctc taaaatctcc 108840
   tttttctgat gttaatattg ccactccagt tcttttctct tggtctagtg ttatcattac 108900
   atatctatat ttttaaacta ttctttacct tttgtattta tatgtgtatt tgtaactaaa 108960
   gtggattttt tttttttttt ggtggggagc tgtatttatt tcccagggct gtcagaacaa 109020
   atatccataa attgggtgga ttagaacaac aaaaatttat tctctctaga gaagaacgtt 109080
   ttcttgccac tccctggctg ctggtcattg ctggcagtcc ttgtccttcc ctgactagta 109140
   gctacatcat tctcatttct gcctctgtct tcatatggct gtcatttcac tgtgtgcttg 109200
   tctctgggtc ttcaagtggc ctttttataa ggacactggt cattggatgt agggcctacc 109260
   ccaatccaat gtaacttcat cttaaataat cacatctgca aagaccctgt ttccaaataa 109320
   ggccacattc acatgtactg gagttaagga catcaacata tcttttggtg ggggcagaag 109380
   gggcaattca acccataata gtctgcctct aggctctcaa aatttatatc cttcctatgt 109440
   gcaaaactca tccacccccg tcctaaccta cacaaaatct tagccctttt cagtatcaac 109500
   tctaagttca aaacctcata taatgtcatc agtttttaaa agctccactt ctcattttct 109560
   aaatcatcta aatcaagtct ggatgagagt ctgggtatgg cctactctgc agtaaaattc 109620
   ttcatgtgtg gagctgtgaa attagaggat aaattatatg ctttcaaaat gcaagggtag 109680
   taatagccat aggattgaca cttccatttt aaaggagaga aattggaagg aataaaaggg 109740
   tcacagaccc caagatattt ggaaataaag cagaataaat cccattagtt ttcaaggcct 109800
   gcaaataacc cactgtagct tgtcactctg ccctctgggt ctacagaagc ctaattcagc 109860
   ttcagaatct gcaaccctag aggccccatc agcctctttg gtctcaaggc aaacttggag 109920
   ttattcttcc cttttcttga agaatcacac atgttcacaa ctcattagct ctatttgcct 109980
   ttttgctgcc tgtagatctc agaagtctga gagactctcc ttcatttctt ctcatctctg 110040
   ttccatcagt caaagctggc aatgtctttt gtgctatcac attaagtagt gaattcaaca 110100
   gttaagccat ctggtcctgg actttcctta gttgggagat tttatattac taattcaatg 110160
   tttttggttg ttataagttt gttgagagtc tctacttgag tcagtttagt tatttacatg 110220
   tttctaggaa tttttttatt tcatctggtt gtctaatttg ttgacatata atttttcata 110280
   gtgttttatt gtaatccttt ttcttccagt ggggtaggaa gtaatgtttc tactttcatt 110340
   tctgatttta gttatttgtg tctcctctct ttttatcttt tttgtaagtc cagctagaga 110400
   tttgtcaatt ttcttgttcc tttcaaagac caatttttga tctcgttgat taaaaaaaaa 110460
   ttataagcaa caaaacaaat aaaaacaaaa taaaatgaaa gccagagtga tagtgagagt 110520
   gagggcaagg tgggtggggg tacagggaaa ataaccactt ctcctggtct ttgcagattg 110580
   gttctgtgtc aggaccctcc tcaacactta gacaggtttg cactggctag acaacagccc 110640
   acgatgaaaa ctcaaggtct tctgaggcct ttgctgagca tgtgtctttt gggtacacat 110700
   gtagctttct gaatttctca gcatacttgt ctccttttgt taatttctaa agaaacgctc 110760
   cctagctttc agttttgact ttaggtagtc tattgtatgc tttgacagtc agcttttgtc 110820
   ataagcatct gagggtcctt actttggctt gcaatgttcc tggcaatact agctgccttt 110880
   ccagcctgag tttccagttt ggcaaaacag agataaagaa cttatgtcac tcctacaggt 110940
   agccctacac agggcagaac tgataaacac aatattttct aataagatct tattcttttc 111000
   actccagacc aatgggaaag ggtcccacac tgggaatgca agctatagac acttgaagac 111060
   caagactgtt caaagagggt aaggtggcag gtcgggagga agggagagta caaaggtttc 111120
   ctagcatttt aaagtaccat tttcttgatt ctgtattttt ttgattacta taatgtttta 111180
   ctgtattcct gaattctgac aaagttggtt ctgacaattt ctgcttgttt ccaatgtttc 111240
   tgttgggaga cgaaaggttg gagcttcctc atccatcatt tcgcacatgt ctaggtcaga 111300
   agtttcctga ttactgtctc ttcaagtatt tcctctgcct catcctgtcc ctgccaccct 111360
   ctgaagctct cattacactt ctgttagaca tttcatgttt tatctcagat atctgatgtt 111420
   ctgtttattt cattcttttt tgtctttgta tttcattttc aacagttttt atagatgtgt 111480
   tctcaagttc attgaacttt cctctgcttt gtagagtcag ttgttaagag cagctaataa 111540
   attcttcatt tataatattt tccacttcta atattttcat ctgtcctttt tatttctatc 111600
   tctctgctga agtttgcaat ttcttcaccc atgttgtcta cacatccact agatccttca 111660
   gcattttaat tacagtattt cattatcctg atttttaaca ggatgtgttc cctctacaat 111720
   tattctttgt atttaatgac tgaagttggt tttcctgatt gtgacctctg gtgttttaag 111780
   gactttggtt ttaatttgtt gtccaactct tggcttcaat ttgctctcca gggctgacag 111840
   gtgttcccaa gctctggcct gggactcatg cttagtcagt gagattcttt ttatacagca 111900
   tgtaatggag cacagcaaac cttctctgat ctatatcccc atccctctct ccgacaccct 111960
   tttgagactg gtataggccc cttcatgtag taggacactt agttatgatg tcgctacatt 112020
   gaaacacagt gaataatagt ataagcctta gaatagatgc acctgcactt aaatcccatc 112080
   ttcatccttt ataagctctt tgatttgggg aaaactcact tcactttttc aagccttggt 112140
   tccttaaatt gtaggttaga cagaaaataa tacaccttgt agaatcattg taaagattaa 112200
   ataaagaaca ggggaacaca gtgcctgaca ttcattaagc agtcactaaa ctgtaattgt 112260
   tattatttgc tttatgactc taacctcact gagctttaat ttctttatct gaaaaatgat 112320
   ggttatgttg tataatagta acacagtatc atgtgccttt ccctataaaa taacatgata 112380
   attttaatga ctaaaattca gaacacaatt tacaaattaa aaagtgttct tacacagatc 112440
   acatttcccc agaaactcaa aaggcaggtg ttgtgttccc ttcctgtaga tgaggaagct 112500
   gattctagga gactgctacc agggtgggtc ataagccagt gtctgtgtct ctttaatcct 112560
   ggttctgtgc tttttacatt gcatgagtct aaactctgca gggggaactc aaccgttcct 112620
   attctttagg ccaatataca catatggtct catgatttcc tttcatttcc cttaatgcat 112680
   tctaaacgct gctgatttgc tgttttatgg acttcttggc tgtgtttctg agtatttttc 112740
   catgctgcat ttcatttcat catcttctgt ctctatttcc aaatgtagct atttgttcag 112800
   cctggttttg ctctgtgagg tgttatttac aatacttact aatttaatct ttgttcgggg 112860
   aaatccaagg agcaattgca tacttctgtg aatcattgat aattcaagcc tggaaaaatt 112920
   cacctagcac taggatcctg ctcttttttt gttcagtgcc atatgtatat atagtgccat 112980
   acgcatacat atgtacagcc ttattccatt ctatactgta attgatgtta ttttgattgt 113040
   tttgtagccc tttgaagcag atcccattgc ctacaaaatg aaaatgggta aatatcattc 113100
   acaaaatggt gattgattgc aagcacggtt taccatacat ctaatatgaa cagtaatgta 113160
   ttccctaatc acaaattctg gatacaaact cagaagccaa aaatgtgtga cacttaaaac 113220
   ttgagaaagc caaaactaaa agggaattca cccagccatt ttcctgttcc cagcagatga 113280
   aacatacaca gcaacctgca gtaagtcaca tatgatgcac ggcacatgtg ggcagtcaga 113340
   gcctaccacc acactgcctg tccccagacc tttctggaaa gcatccttgt attaactctg 113400
   gatttcattc tagcattatt tatctgactg tttcttcatg tacatggagt tggaggcttt 113460
   gttaaaaaat ccacttaaaa tactaatttt aactactttc ccggtgtgta atgcatttct 113520
   aggatgcatt acttaatgca gtacatcaat tagattatag aatttaacac cggattcaaa 113580
   tatatattta tttggttaga atagcaagca attataggaa gaactcagcc tgaagaagga 113640
   gttttagttt cccatttctg tggttggcta ctactaataa tattatagtt gctatgattt 113700
   tagagtttga ctaaatatca ggcactctgc tatataaata cttgcttttt tttttttttt 113760
   gagacaggac tttgctccat tactcaagtt agtgtgcagt agcgtgatct caactcactg 113820
   caaccttcac ctcctgggct caagtggtcc tcctgcctca gcctcctgag tagctggaac 113880
   tacaggcatg tgccatcatg cctggctgat ttttgtattt ttgatagaga tggggtttcg 113940
   ctatgtcacc caggctggtg ttgaactcct agactcaagg gatcctccct cttcagcctc 114000
   ccgaagtact gggattacaa gcttgagcca ctgctcccgg ccaatacctg cttttcttta 114060
   atgggtgctg gtgaggttaa aatgaaatta tttctgtaca aggcaactga atggctcttg 114120
   agtaatcata aagaacattg tcatggttta gatgcgtgtc tccccaagcc tcatgttgaa 114180
   atgtgatccc caatgttgga ggtgggatct actgggaagc attaagttca caggggcggg 114240
   tcctttatga agggcttggt gccatcctca caataatgag tgagttctca ctctattagt 114300
   tcccatgaga gctgattgtt aaaaatatcc tggcatctcc tggtcacaca caggttcccc 114360
   ttcaccttcc aacatgagtg gaagcctgag gctttcacct gacacccaat cttccagcca 114420
   gcagaactgt gagccaaata aacatttttc cataaattac ccaacctcag gtattcattt 114480
   atagcaaaag aaagagatga aaacagacat taaatgttat atatattttt taaatttttg 114540
   tttaagattt tttttttacc agtaactgac aagctaattt agtatacact gttcacttaa 114600
   tatgtttttc agtcttcata acttagatct tgttggattt tcggcctcaa atattcttac 114660
   tcttttccat caagatgtct gtaaatctat ttaatccttc aaagtataat ccaaatcctt 114720
   ctatccttga agattttcag atttttccaa acaaggtaat ttctttttcc tctgaatatt 114780
   catagctttt aaatctctgc catagcaatt gttttctact ttaaattgcc actggctgcg 114840
   tgcctctctt atttgctctt gtgaactatg agcccctcaa agccagaact tatgctatat 114900
   aaatattttt tctctctctc tgaattaaaa aaacagtacc atatgactct cagtaaatag 114960
   ttgataggca ttgctgaatg attttcaatt ttatctaata tcggcaaggg ttgcaggact 115020
   gcaaaatatt ttttcactac tggttttgag aaaaggtaca acggatgaag tgttcaatgg 115080
   cacttcctca aaaacctgtt gaagttctat catttggcag ttggctagga atcatacagg 115140
   gcatttatct ttgaaatcag tgttctcttt gtcttgaaaa gggtaggcct gtcatgctgg 115200
   ctcacgcctc taatccccgc actttgggag gccaaggggg gcaaatcacg aggtcaggag 115260
   attgagacca gcctggccaa catggtgaaa ccctgtctct actaaaaata caaaagttag 115320
   ctgggcatag aggcacacac ctgtagtccc agctacttgg gaggctgagg caggggaatc 115380
   gcttgaacta ggagacggag cttgcagtga gccgagatcg cacccctgca ctccagcctg 115440
   ccatctcaaa aaaaaaaaag ggggtcacat taggaatttt tttttttttt gtaatgctta 115500
   tgtataagaa accagcttgg aactgagagg gaagtgtttt attagtcatg caaattgagt 115560
   ttattagcaa gatggtgccc ctgtagagtc aggtaaattt agtgtgtgtt tgtttatgtg 115620
   tgtttggtat ggcttttgtg tttttctcta catttggatt tatagtgcag tctctaaaaa 115680
   gtattgcttc ttttgggaaa tgtgtctctg aaactgcctt tgaagaatta taggtaatga 115740
   gataaatcta atatgaatga ctccaccttg cttctaacct cacagactat ttttattttc 115800
   ttaatctagc atggaggcca agataactat gagagaaatt tagttttagt taaactttga 115860
   ggctagggaa attgcccctc ctccttgctg gaagattgaa gccagattca tagggcaatg 115920
   ttagaattat ggcacaggtt gtacttttct aaagaatagg cattgttaaa ccataacctg 115980
   ccatggctta gcttgttttc ctgtaagttg tttgctgccc caaagtcatg taactgggct 116040
   tgggtaggtg gggggtgtta cttcccacct acatcatcac catagacaac atcaccactg 116100
   agatattttt cagatttagc atttcagcag accaagagct gccaccaagt tctgagactc 116160
   cctcccaaaa actgactcag ctgcatgaag gctgttttag acacctctgt gatttcatca 116220
   gttgtttcag tttcccagcc ccctgcttgc caaagtaccc taaaaaaccc acaccttcaa 116280
   attctcatgg aagggtgggt ttaagaagtt tctccctgtt ctcttcactt ggctggcatt 116340
   gtgatattaa actcatttct ttgctgtaat acctgttctt ctcagtacat ttgactttct 116400
   cggcagcagg ctagatgaac ctgttgcact attaccactc ctctatccaa aatccaccag 116460
   ggcagttatg ggtcagaact ttagaagatg acagaaggcg aaggtcatct ttactttgga 116520
   tcccagagga caacaactaa ccagttagca gtgccattgc cacgctggtt tgttctatac 116580
   taacccataa ggaatgcttt tcagtgaaaa acctaggctc taagaaaaca gtgatcaaat 116640
   actttgtttg cctccactag gtcagagagt aaggtgaggc acacttgggg cctggggtga 116700
   gagattggct atcgccagct gtgcattata gagaagcact ttgcctgcag aaggaaggga 116760
   ggcttctgtg ttgaggaaag tgctatcaag aaaattaact tagagtgttt tctgagaata 116820
   agcaagaggg aaagccaaat catttatagg ggaaaatatg gaaaaagttg aggggattta 116880
   gaaattttct agggatggat aatccctctt ccaaatgtga aaatggcctt tggcaactta 116940
   gaaaagcaac agaatagaat gatttttcaa ttcagagtca aggattgtgg ctaaggttct 117000
   ctctcttggt tttgggagga tgatgtctgc aggacagagg gaaaaagtca gaaagcggac 117060
   ataccagaac tgtgtggctt caagcttatg ggtagaacca ttgctgccag tgcatctagg 117120
   tatctgcttt ttcaactgtg accatatatt ttggtgttat aattattttc ttaagaaact 117180
   atacgttttt attggggctt actatgaccc cagtatttta tctaggtagg ttataacatt 117240
   aaatatagta ttaggctcac tttattttat tttttattat tttttaaatt tggggggtat 117300
   ataatagctg ttaaaatttg ttttaagtag tgttcctcaa cagagcctaa aacgcaagac 117360
   aaagtaagtg aaatcttaaa tctaattgct tcagatatgt aaacatttta tagaaaataa 117420
   aaacaacaat aaaacaaata aacaacatca ctaccacccc caacaaaaca accaaaccag 117480
   ccaaaaggat caagatcttg taagataaat ggacagtttt ttatattcat acctagagtg 117540
   tctgtgatat ttagtattaa tattattatc ttccattcag cttcgtttaa atatctttca 117600
   acatagtcta atatttcaat aaatgaatta tttttctggg aaaataccat gaaacattgc 117660
   attctgaggc tcatccaatt acattctgag caagtatcat cttacggaaa agaaacaagt 117720
   tctattatgc cattgagttg agcacttcat atccattttt taattcagtc tccttaacaa 117780
   ccctgcaagg ttgttgttgt tagcttcatt ttatagttgg ggcaagtgaa gcttgatgac 117840
   atttggtaac ttgtgcgaag agaactagag ttcataaatg gccaaactta ttctaaagcc 117900
   aagtctatag gcttccacag tgcatttcct gaaccgccat gctagacacc ccctgggtaa 117960
   gagcgcttct aactccaaac ttctgtgatc tggagaagtt tggaaaagca acagaataga 118020
   atgattattc aattcagaga caaggattgt ggctctcttg gttttggaag gatgatgtct 118080
   acaggacaga ggaaaaaagt cagagaaaaa agtcctgata atgagaattg acaaagcact 118140
   aaaagatttt tcacctttaa ccaaattttg ttctatgtcc ctggatcttt ctttaattaa 118200
   ttatgaaact tttgattgct taagtatcat gtatagtttt gactttattt gagaaatgtg 118260
   cattttgggt aaactcatct tgagtataat gattgttatg tttactagat ctaagaaatt 118320
   aaaatatcta agtctttgta cactaaagaa gtacaggatt atttttaaga aagcaactca 118380
   tatgtttggg aacatctgta tacatcatta atattttctt ttgtttgtag atattcatta 118440
   ttatgtagac accactctgc tttaaatctt tgaagatcaa ttttgagaaa tttgatagtg 118500
   tttggaacaa acagtattct gctcaactgg catgagtcaa aggtccacat tacccttagg 118560
   aaaaaaaaaa aaattattgc tttctgtgag gggttttaaa gatgtgacat ttttcaatat 118620
   ctgctttata acttttgatg ataaaaacaa aataataaaa aattacagca gaaaacattc 118680
   tttgaaggct tataatgtgt caagttctat taaaaatgtt ttatatgcat atttaaattc 118740
   atcctcatag catccccaag agatagaggc tattagaacc attttacagt tgggccaaat 118800
   tcagtcagct cagaagtgac agttctggga attttcttct ccagagccca agattttaaa 118860
   cattgagcat atttccttct aaagcatatt gccaaatact atagaaggac cgtgaattat 118920
   accttgtgat tcaggctacc atcttgaaac taaattatga gtgaaaacat tttgtttggt 118980
   tacatttttt atatttaaaa tacatttgaa aagaatttgg aatgtgttaa agtcagagtc 119040
   atatattgag acctcgtatc tgaattgaat tactttaaac aaagtggaaa gaactttttc 119100
   catcaatttg aaaagtaaag taaaagacag aaagaaacag agagattaaa tgtttttccc 119160
   ttattttgta tattgtgggc agtgccattt atcttagcta tgttaataaa cactggattt 119220
   tcagaaggtt tatgcttcat ccttgcttta tgctgtaatg tagccacaca gaatgtctct 119280
   ttatgttgac tgaagttaat atatgcatct taccttttaa tattgcatca actatagtca 119340
   tagtggtgga aaataataat gctctgtgtt ctgcactatg gtggatgttt tataaacagt 119400
   atctgcaatt attacaaact cctattagag agctatgtct aaccttattt tacatgtaag 119460
   acatagaaat tcgaagttcc cagaatcagt gagtagtaga tttgaactga gttttaacac 119520
   aactctaaag cccaagcctg gcctccttcc gctgcgcaat tctcctccag agggaaaggc 119580
   tagcaccatc cctaagaggg gccgaaagtc ttctaagggc acctgaacca ttgtgaagaa 119640
   ttgtgttaat aacccagtaa ttcacacttg ttcaaatttt cagacatttc ctgggatgca 119700
   gtcaaaactt ttaggagtct gaagcctgag gctgcctgct ctctgctgct gattatagag 119760
   ctctctagtt gatgagtggt gtcctggagc tccctggcca tacatacaca gtgctcatgg 119820
   gtcgactgta tggcaccagc ctctgctgct ctaccctttt cagccttttt ttctggaatg 119880
   catgctgagg tcctccttgt gcctttgaaa tttttatgaa gagggtgatt atttttggta 119940
   cagcaaaata tgtagaattt gcacttctta ggaattaaac atctcaattt ttttccccca 120000
   gagaaaataa ctcttgagca agggaacact catctcaccg ctcagacttt gaggtggccc 120060
   tacatgtata caagtttggg ggaacatgag aattccaatt aaattctggg aaatccagac 120120
   atatattcaa caagtattta ttatgtgcca ggcgttattt tgagtgctag aaatataatg 120180
   gcaaataaaa cgaaaagaac cctgatcctg atggagtgtg cgtctaatga gaaaaatata 120240
   caataaggac ataagcaaat aaattcataa tatagcatca gaggcgataa acagtagaag 120300
   gaaaagtacg tcagtgtaag gcacagagag ctcccggggg cagagtgttt tcattaggtg 120360
   gtcagagaat atcttgtttt tttgtgagca gcttcagcca aagtaagtaa tcaagaatta 120420
   tacttgaaac gattggtttc cagatgttaa gtataacaaa tattaaaatt cctcggtcta 120480
   acaccctact catcttccag ctgctttaat tagagtagtt tctatttgtt ctacaaatag 120540
   ggcttccttg taaaattttc tttggagatg ggtttcatat ctgagagcac tgcctcagac 120600
   caaataaata ttaacacagg aggaagctga gcgtcaaagg gaagcagtaa cttggccaag 120660
   gatgccagtg ctcccagtgg ccaagggaaa cccaggctgc ggtcctctct gtccaagctc 120720
   tgtcctcggc cctcaggtcc tggcctccag cactgagagc aacacagagt gcctgtgagg 120780
   cggtctaact tctgctgact ggtgaaggtg gatcccccag ccccaggaaa agtcaaccag 120840
   cagcttgaac agcgcttccc tcgcggaggc tcagcactca gctcatcccc cgccatcggg 120900
   ctgtaattca ggtgccagcg ttgctcctcc agcaggaaca taagcccaaa cttaacaaat 120960
   tcctaaagca gaacaaagaa agtgtcagag gggaatgggc acggagcctg tggaccgcac 121020
   tagaatcttc taattcttag gaatgcagat cgccctgtgt aaggggccgc tcagagcgcc 121080
   cgaggagcgc cggtggcctc ccttggatgc ttcgctattc atcccacctt ctggaagagg 121140
   ctattctttt gtggctgtct tggtgtttgg ctgagttgtt attgaattcc tccctgactt 121200
   cttttttttt tccttcggaa gacagaagaa aagaagccgg cagtggaagt cttctctggg 121260
   aagcctcctc ctcctgcatc tggctgctcc ctttggggca gcctggtccg cctctctggg 121320
   cgccaggtgc gtctgcagcg ctctcctgga ggccggctgc aggttcatta gtgctcacaa 121380
   taggcacgca gcgcagggga atatgtaaag aaagtggagg acatcggagt ggcattcaaa 121440
   gcggcaggcg ggagtaggag tttccctagc ttttcttcct ttgtttctaa tcaccttttc 121500
   tgtttcttca gagaggatgc taatagagtg aatacctggg cgttccagga gaagagttag 121560
   tggaggtttc agaaaaggaa atgcaatctg ggacgaaaaa tagcttttct tccccttggg 121620
   gtgtgtgtgt gtagtgtctg aatgctttgc caccgatgag gccgaggact aaaagcagga 121680
   taaaggctgt gattggcaca gaagacccct ggtgaaattt ctggctcagg tactagcttt 121740
   ctgtgagtcc tgtgcaattc ggcacagccc ttggggcttt gagtcctttt tttttttttt 121800
   tttttaataa aataagagac ttggctaata ggatttctag cctaccactc ctttacagtt 121860
   ttaagatatc attttaaaaa gttacatttc aagactgtag taaggaggtg acattatata 121920
   cttaaacata aaaatgtgga gtgatttgaa gagcactgaa attattccac tcttcaaagt 121980
   atatgcattt atgtatacat acatgtacat acacgtgtac ataaacaaac atatacatat 122040
   agtcattttc agatgcctga aattctgtcc ttaaagacag ttgtgaatcc cagttctatt 122100
   agtatttgac tgtgaccctc ttagttttgc ctactgtctg caagactcaa tttttctttt 122160
   tttcctcata atgggaataa atattgtctg tcttctaact gtgttatgat atttaaatga 122220
   ggaaatgtgt tgaatggtag ttcttcttgt ctgaaagata agaatacatg ggtgccatca 122280
   tccaatctca aagtcattgg gttttgacag aaatggtatg atgggtaatt atcccatttc 122340
   tccaaagtca ttaggaacct gaagtaattt ttttaaactt gtttttcatg cacctgtgtt 122400
   ggctagatca cagagcctgg gcccatcctc ttgaggaaat attatctgcg tcaaccttcc 122460
   tcaatgcctc ctggatgact ttaccaccat cttgcaaatg tctattattt gttagctcta 122520
   gaagactcag agcaatgatt gcaccaatct gaaggttatt tcttccatgc tggtcttctg 122580
   ccattggtga tattacagat gtttgtccag aacattactg atgacagcta cacacctgga 122640
   aaataaagtt tcttgtttgt tccttccaaa ttaattttct gaatgaacac cagcaactca 122700
   aaatgcattc agtttcagaa ttcaaagaac agaagagcaa atttgaggga cactctggca 122760
   cagtagaggg tatgtacaat cattgaatgc aaagtctttt acattttacc ttacacgtca 122820
   atctcagcta atcaggcttt atttttcagg aaagtgctgc agagcagaga agcaattctc 122880
   aacaagacaa agctactata atagcgactt gtggagtcac gttaagaaga aaataggcag 122940
   agaagtggac tcttgacggg agtctcatcc atcatttcct ctctgtgcaa cctcaggcag 123000
   ggtacataat cgatctaact tttagttttc tcttatgtaa tctggagata ataacatata 123060
   ttttatataa ttattgtgag tattaaatac caaaacccct ttatgtcaca tgtttagcat 123120
   agtgtctata tcacagtatg tgcgtcatat gaaactcagt gctttgtacc cgctgattcc 123180
   tggtgaatga aatacggtca cacatcacaa tgactgtctg ctgaaatgcc aaaatcctgc 123240
   ctgtagttca ggaaccagag taaaggaaaa tgaaccctgt agtgttttta ctttttctct 123300
   ctttttagag aaagatcatt tgcatgtggc tccaccttaa taattgcatt tgtaatgctt 123360
   tcatgttgtc acatggtcta acccccagga tcttatctta ataaaaattc agttaaacat 123420
   gtgttcaaat tggccagatc agccaaatct ggaacaggga aagttgtttt cctcctaatg 123480
   aactgtcccc tggacacggg cagtgccaca gaagttcagg aatatttatt gtccatggca 123540
   tttccaggag ttacactctt tgttgaaccc ttccatcgtg ccagggggag gctggtgatt 123600
   cattaattca ctgtgatgat ttgtcctaat tattctaatt actgccttgt ctcctcagac 123660
   cagctggtgc ctaaagcaga gggaaagtca ccagtcatga ggactgcggt ggtggctgca 123720
   gaatcccggg cactggatct gtaggattca ctgtcaccac atcaaaaaag ctttgctgct 123780
   gctgctgctg ctgctgctgc tgctgctgtt gctgctgctg ctgctgttgc tgctgctgct 123840
   gctgctgctg ctgagcccaa cttatccttg gtgctgctct ggcctgagtc cgggttcccg 123900
   gggccctgcc atgcatgcat tctctcctct gctcctcttg taccttttca gggagagaag 123960
   agccctgata caggttgctt ttctgtcttc ccagcttgct gccccaccaa ttccagacag 124020
   catctgctgt gtgttctttg tgcacccatc cctgtctgaa cgtatcttaa acccagtctg 124080
   gtttctaaca actgtcgata ataaaaatat ctatttctta ctgcacacta tcaagtgcca 124140
   agatggatgt tcattgccat atgtgcactg tctctaagtg ctacagtttg cagtcttagt 124200
   tcatatatta aggctctgca tgacctagag actagtttac attgcccagc aagggagtaa 124260
   cacatggaac tgaatcccag attgctgcat ccccaaagtc ggggggccct ttatgcgtct 124320
   ctcttttccc ctcccacaag ccttcctggt catctacagg actcctgctg aaaccttcct 124380
   tcggtctttt atccctgagc tcctttgctg tgataacata gactgttctg tctggcagcc 124440
   ctgggcagtc atcagtgttg gctttgcagg tcagcttggg gtccccaacc aggattcctg 124500
   aggccacaga atcttgctgc aaacacagct agtcttggag aactcatatc tgtgcctttc 124560
   tgatgtgctg tatcacccca gcctgccctg tgtttctttg ggatattttc acagctcata 124620
   cttcagtggt atttgcctct taagtcactt accccttatt actctgaaca aaactggaat 124680
   ccgaaaaaaa caaaaacaaa aacaaacaaa caaaaaacac tggaactgtg gctcatctct 124740
   cagggaatga gctgacactt agtagaatct atgacatgag ggctatgtta gagaccctga 124800
   aatgaagctg ggtgaaaaat atcaaggtgt tgtgagtgac aaagatgata ttccaaagat 124860
   tcaactgaga aagagtcagt gtatcttatt attaaaattg cttctgtaag agtcatcata 124920
   agcttgactt gtatttcttg accaactctt atttctattc atatctcagc ttaactgtca 124980
   tttcttcaaa gaggcagaca tcccaactct acaaactgta ggccaccagc ttctgtcatt 125040
   ttataagaac aggtgaatca cttcacactt gaacttattt acttattttc tttttctttt 125100
   ttttcttttc tttttttttt ttttttgaga cagggtctgg gtctgtcacc caggctaaag 125160
   tgcaatggca tgatcaggcc tcactgcagc ctcgatctct ccggctcaag ttatcttccc 125220
   acctcagtcc cccaagtagc tgggattgag ggcatgtgac aacatgcctg gcttatttat 125280
   ttatttatgg aggcaagatc ttgctatatt gccagggcta gtctggaatt cctgagctta 125340
   agtggtcctc ctgcctcagc ttcccaaaat gctgggatta caggagtgaa ctactgtgcc 125400
   tggccttgaa cttattttct taatgcttgc tttcccaagt aggctgaaag ctttgggagg 125460
   ctaagaggca acattttgta attacctgat attcttagtg ccaagagaag gctggctctg 125520
   ggcaggccgt ctgcccatgt gctcgacaag gcctgtattt atgtatctgc aggcaggccc 125580
   ctcatttgta ggcgactagg tcttcttagg gattcctggc catactagga ggtcatctgc 125640
   agggacgcct gcactgatgg ggagtccttg agagccagct gactacagct ctgggatgat 125700
   gagggtggga aaattgtcca gttagaagaa atactgaaaa cctcaatgga caaactggaa 125760
   cttcaggaag aatggtggtg gacagaaaaa aggaaaaaaa aaagagaaga gtgaatgaac 125820
   caaagtgaat gagcctcatt tctagatcta cagacttttg ataacacctg gcctgtttat 125880
   atcacaaagc tgctaggctt tacaaaacaa attcaaaaca aaaacaacaa aaagccatga 125940
   agagctatct acgtgcaaga ggttttatat ttagttgtgc tttattttac taaccaaact 126000
   gaagactagc ttgctgggtt ccatttcaca ctgccagctc ttcactgtgt gcattcatga 126060
   gcctgatgct aatgagacct agagtgatct cataggcaac ttgcaacgtt caggctggac 126120
   aggtaagtgt gatgtgttgt gttcaaaagg aacagatggt ttaaatttaa attcaaattt 126180
   tgaaggaaac acaaagacaa agtgaaactc tttgaatgtt agataaaaat gttgatattt 126240
   attaatgatg atatcagatt taatggaatt tggttgaact attaaactgt ccactaagta 126300
   gaaattaatg ccagttcttt gatcttttta tataattaca gtaataattt agacttttct 126360
   tgactgcaac tctggggaga tgtaatgata gaaaaaacaa accaaacttc agggcatatt 126420
   cagcatgatg tctgataaat taatgctctt gatcgtaatt tgaaaaggag ctcttgccct 126480
   gcaggaagct gaccgggaaa cagaagcttt attagtgcag agatggcatt gactacacct 126540
   acggtttatc tccactctgc tctgaagcag caaaagtagt gagacacgga ctgaaacagg 126600
   atgttagttt tctgaaggag cagatacatt taagcccagc ttcatgcagt ggaaagggca 126660
   caggactggg gctaaggaaa tgtgtgttct acttagagtg accaaggaat cagtcaaatt 126720
   cttttttgag agttaagggg aatcatttaa taattatgtt gaggtaatac gtgtgaacta 126780
   gaacgatcct tggcaaatca ggaccctaat ttggctcaag actagtcact taataattct 126840
   ctcatctcat gtgattctct tagctttctc ggatctcagt ttccttattt gttgcaattc 126900
   ttgcctgtaa acccttaaga gtgctaagag aataaggtaa gagaacatat gataatgaaa 126960
   ggttggaaat aggaaggtga aaatagtcat ttggtagcat tgttctgatg aagagggtaa 127020
   agctaattct cccataaatt atttgtgttt cccctgttga ccagaagaat gaagaataac 127080
   tgagacaaaa attctattta tttcatagca tgatgtggcc acacattcta aatgttaaag 127140
   ccttagtttt gggttaggag cttacatttt agcaccaagt ttccctgtta ttatttttcc 127200
   tataggactg tcaagattta ggcaatatgg ccgctttaaa acataaaaat taactttact 127260
   catcaaaagc acagatgact accaatctca agcttttagt ccaaatgaaa attcagtgta 127320
   aataccttta gtacacagaa ttccagctga atgggaaaaa aaactgatta actcatattg 127380
   tattatttgg tgagaaatta ttctttcttt ggggctattg ctgagtacta agaaaagaaa 127440
   atgaatatcc aggatctgct gctactcccc aaaaaatata ttattgtctt ctttcttctt 127500
   gaattttcta agggccccag tgagggctgg gcccttccac atgccgtggt agctaagctt 127560
   cagagccagt tgccagggat cggaggctca gcaaggctca ggagagtatc caaggtcaaa 127620
   cagctaacaa ggaaaggagc aaagactgaa gcaggtgaca aatcctctat gctctcctct 127680
   aatccagtca ctaaccctac ctttggtagg aatctggaaa taagaggaga gattaagtac 127740
   ttttcaaatg ggtacaaaag ggaagttttc tgggatttta ggatggtgtg catatatgct 127800
   gaagtgtgct tgaaaacaaa ataaaatgca ggtgtggaag aatttttaaa aaccattgta 127860
   accaaggggt tgattgacta atataatgtt agcaataata cttgccatct gcaattttgc 127920
   cattctcata agaattggtt tagcttaaaa acattgaata gctaagcatg tgtttaaaaa 127980
   actctacttt aaaataaaat agatttaata tgttatttga cttcagacag gtcttatgtg 128040
   atgatttgat atagatcatc aatactgaaa tgaacctatg tttcatttga gaatatctgg 128100
   aaaagagaag tgtgaatgtc agatttcaaa tcaaattatt tctattttaa aatataatca 128160
   tcacaatact tttaaagctc aatgagctta ttttatccta catatgcaaa ccagagtaag 128220
   gttaagtatg caacttcatc ttgccaacag ctgtaagggt gaccatcacc tgggaactcc 128280
   caaaaaccct aaaagcacct ggctgggctc gcttgcctga agcaaagatg cccagtggga 128340
   ggatctcgct ccatgaagag gcactgagag ggccagtgat gctagcagac agagaaaaag 128400
   gcattctgct gcaggaaaag ggcactagcc aaaccgggtg tcccacagat cactagcaca 128460
   ctatcaagtt ggtgggactg taaactagtt cactagttca accattgtgg aagtcagtgc 128520
   ggcgattcct cagggatcta gaactagaaa taccatttga cctagccatc ccattactgg 128580
   gtatataccc aaaggactat aaatcatgct gctataaaga cacatgcaca catatgttta 128640
   ctgcagcttt attcacaata gcaaagactt ggaaccaagc caaatgtcca acaatgatag 128700
   actggattaa gaaaatgtgg cacatataca ccatggaata ctatgcagcc ataaaaaatg 128760
   atgagttcat gtcctttgta gggacatgga tgaaattgga aatcatcatt ctcagtaaac 128820
   tattgcaaga acaaaaaacc aaacaccgca tattctcact cataggtggg aattgaacaa 128880
   tgagatcaca tggacacagg aaggggaata tcacactctg gggactgtgg tggggtgggg 128940
   ggaggtggga gggatagcat tgggagatat acctaatgct agatgacgag ttagtggggc 129000
   agcgcaccag catggcacat gtatacatat gtaacaaacc tgcacattgt gcacatgtac 129060
   cctaaaactt aaagtataat aattaaaaaa aaaaaaaaaa aaaaaggaga gcctcgtcaa 129120
   caagatggag tgaaggtaga cgggtgcttc agtccctgta agacaaggca gggaccggca 129180
   gggctcactg agcacttgca ataagttatg cacagagacc ttccctatat tttttcatat 129240
   atgttttact gtaacccgat ttagtagata cccttttata aatgatgagt tcctggaatt 129300
   cttggaataa agagcgatta gattgcttgc ccaagatgac agttataagt gatgaagaca 129360
   agatttgacc ttctgtagta ggatttctct ttctgaattt ttaaccactc acaaaaataa 129420
   gtgctttaaa acctagagga tttgaggatt ctaatagtta ctagaggaaa gagatgaaga 129480
   agaggaaata gggcaaattt ttttagtatt atattctagg ccgattaaaa aaatctatta 129540
   gtggacttga tcatttgtta caaaatttcc tgactttttt ctaatgaaaa cacagagact 129600
   cagaaacatt aaacaagatt tcaacatcac tcagctaaaa caaagatgaa aggaattttg 129660
   catctgtgat tgcagtcagt attctctcca ctattatatg ctgtggagat atgactctct 129720
   gagaaatagt gaaacagaaa atattggcct catatctctt cttgagtggt actggctggt 129780
   gcacccactt tcttccattc tgcatttttg aatattgaaa ggcactgtgt ttcaacattt 129840
   ctctaactct gccctcctaa aataaaagcc tttgagtctg ctcctcacct gacggtcaaa 129900
   atatatctgt gtcatgccat agtccctcgg acttttacta ggggacttat tgcctaatgt 129960
   tacagacaca ttttatatgt gtttagtatg gtttaatata acgtgctcca tcgggggaat 130020
   tggttttaaa ttgtgtcttt atgagaggaa atgatttatt tattcaacac tattggttga 130080
   ggtgtctctc tggaccaggc actgggtacc ttacatgttg gcatgtctga caccgtggca 130140
   tatttagtgt ggcatctagg ggtcatattt tctattttgg tttttttttt ttttttgaga 130200
   cagagtctcg ctctgtcacc caggctggag tgcaatggtg agatctcggc tcactgcaac 130260
   ctccacctcc caggttcaag cgattctctt gcctcagcct cccaagtggc tgagactaca 130320
   ggcacgtgcc accatgtctg gctaattttt tgtattttag tagagatggg gtttcaccgt 130380
   gttaaccagg atggtctcaa tgttctgacc tcatgatctg cccgccttgg cctcccaaag 130440
   tgctgggatt acaggcgtga gccaccgctc ctggccatat tttccatttt aatattaggg 130500
   agtatattag agacatagct gtttctcaca tagaattatc tgtccctact gcataagtta 130560
   acataccagt taggtttccc caaagctccc atccttgttt ctcattcctg cttttagaga 130620
   tactggggac aggagaagtc tggtcacctc tctaccacca aggagccaaa tgtcaattac 130680
   acaagtcctc cgatttatag tttctccatt aatagagtat ggctaagtgt ttatatttct 130740
   gatttcttag gtttttcatg aagatcatcc aggattgtag atgaaaaagt actataaata 130800
   ctgaaatgta cttatcctat gtaacatcat tattttatgt aataaatcaa ggcctctgtt 130860
   atggtttgat tgtggtttgt tcctgccaaa atttatgttg aaacttgatc cccagtgtgg 130920
   tagtgttaag aggtggggcc tggtcttggg catgcaaccc tcatgaatag cttaatgccc 130980
   tctcaagggg tgagtgcatt gttgctctca tgagaatgat tagttctcct gtgagcaggt 131040
   tgctaaaatg agtctgcctt cctaggtttc tctctcttga catatgatct cttttcatac 131100
   atccccctac ttttgttttt cccattagtg gaagcagcct gagacccttg ccagatgcag 131160
   ctgcccaatc ttagactttt aagtcaccag acttatgagc caaatcaatc tcttttcttt 131220
   aaaaattacc cagcctcagg tagtgtgtta tagcaacaca tagactaaga cattctctaa 131280
   gccacatccc tgaaaactgt tttagaacct gcatctatat aagggctttt taatatactt 131340
   ggtgtcagaa acccaccctt tcctctagaa aatatatgaa gagaatagat accttgtgga 131400
   tgacttctct cccacttaaa cttcaaactg atcatagtaa tcaactccct gcccattcct 131460
   ctttcttaca tctggatttt cagtaatcaa taataacaat ccagtaatgg tgcagctggt 131520
   atggcagaaa aattattgca gcaaaacatt cacaatgagg ctaatgagat catgtgagag 131580
   agggaagagg gttgaatcga tgttgaaact aattttattt cattagcact agatgtaaat 131640
   tgcagtataa tggaattaat catttaaaat ggattgttca cttagaagtt acaaaaatac 131700
   caacattcca tttggtgcta tctttttcct ctgatgaatc aatttggttt taaaggattt 131760
   gtttgtattt aaagtaaagg gaaccaaggg gtttatacag caattcttgg gaacaggaat 131820
   gagataagaa aaacagctag agcagaagga catcaacaaa caacaaaaac aacaacaaac 131880
   tgagcagatt atgaaagggc aggagataat ttctacctgg agtatgtgaa catggcaaaa 131940
   tcaacttaaa aaataagtct tctaaagact taatccattc tgagccaacc tgaaaacagt 132000
   gattcttaat ccttcatata catcataatc tcttgggaaa cttttattag atagcaatgc 132060
   ctaggcctca tcctaattta attaaatcag aattgttaag agctagtact gcagaacata 132120
   tataatgcca acttcctatt tttatttttt atttatttat ttattgagac ggagtcttgc 132180
   tctgttgccc aggctggagt gcagtggctc catctcggct cactgcaagc tccggctccc 132240
   gggttcacac cattctcctg cctcagcctc ccaagtagct gggactacag gcgcccacca 132300
   cccggcctgg ctaatttttt tgtattttta gtagagatgg ggtttcacca tattagccag 132360
   gatggtctcg atctcctgac cttgtgatcc gcccacctcg gcttcccaaa gtgctgggat 132420
   tacaggcgtg agccattgcg cccagccaac tttttaacaa taaaaatcac gtgggatttg 132480
   aacaaaaagt atactaatca aaggaataaa tatttatata tctcctgagc tgcataagga 132540
   attatgggat taaagtggta caagattctc ttaaacaggg caattactag cagattaggg 132600
   atataaaaat atatgcatta attgtccgtt ggtggtgcaa atgttatata ccagagatat 132660
   ttagaagaag gagaaatgac tttggaccca ggagaactag ggaaatctta gagttaatag 132720
   gagatgaagc agaatttaaa ggaccatgta tcagcaaact attgctacac agaaactcat 132780
   cttagtctta aagactttct aaacaaacat gctttaaaga gtttgcagcc acctcaaagt 132840
   tggtctcagc ttgggttgag ttgctaatga tcataaattt ttgtgatgaa caatctattt 132900
   cagaaagatg ctcacaaatt tctgtgacaa ttgtgactca aagtcatttc acaaatctta 132960
   ctacaaagag atctaaacat tcttttctct ggccaggcac agtagctctt gcataaattc 133020
   catcttttgg gaggccgagg cgggcgaatc acttgaggcc aggaattcaa gaccagcctg 133080
   gccaatgtgg tgaaaccccc atctctacta aaaatacaaa agttagccgg gcgtggtgac 133140
   acgtgcccag ctattgagaa gctgagacat gagaatcgct tgagcctggg aggtgaaggt 133200
   tgcagtgagc caaaattgca ccactgcact ccagcctggg taacagaagc taactctgtc 133260
   tcaaataaat aaataaataa aaacaatttt ttatcagcag ccttgacgac aggatcatta 133320
   atgaaataat tctgaggaac agactcgcta catgcataat tttatagaaa atgaagctga 133380
   tttaaagtgt ataaatgatg cagggtgtga tcaatttaag acttgaaccc aacattcatg 133440
   gctccaaatg cagtgctcat tttcctgtgc aattacacat ggttttggac aaaggtcttc 133500
   gtgttctatt ttattcatac aataatttca atttaattaa cctcaatttt aataagcagt 133560
   tattcccaag acagaagcat taagagaacc aacatttttt aagagctcac taggtctcag 133620
   acaattacaa gtcattattt aatcttagat gttgtgccct aagtgccttg cttgcttcac 133680
   cactgccatg gccctaccag caacaagaac ttatccaagg ttttgtagac atttgtaccc 133740
   aaagagggac aaactggaag gcagtacttg atgtatgttt tcatctatca ctgtcaaaaa 133800
   cacgcgcctg aaacttccat ttacatacct gtccccacat ctctccttcc cagtccccac 133860
   tgtgcttcgt gctcctattt tgatgatatt caatctgtta cgttctctcc accccagtcc 133920
   catgatttct tcacttttac ctcattagcg tccagacttt acatgtgtcg tggataaaat 133980
   acactttttc taagtctgtt gagaaaatga taaaagtggt atctcaaaca gcccaaggag 134040
   acagcccaat gggcgctcga gccctggaca ggcatgttgc aaacaacgtc catcagtaat 134100
   caggactgag agagcataaa gcctcagagg tcagaggtca atgggaggtt ccagttgggt 134160
   cagaaatgaa aactgcagag aatgcagcag atacatggca ctgttgaaag agaggcctag 134220
   aagcagaaga caggtgagtt atatttagct ggggacctgt gggaggttgc aggtgatgct 134280
   cagtggagat atatgaatca tcctacctgt tacataggcc agctgggatc aaaggaaaga 134340
   ttagagtgtg acaaatggct ttgtatccat aggagtttgg gttgataaga cattaagagg 134400
   atttaacttt aatagcctaa actagtttta taaacagtgt ttccagataa gacacagaat 134460
   gcccatttaa tgctgaattt gaaattaaca atgaatattt ttaatacaaa tatgccctaa 134520
   atatagcatg agacatacta tactaaaaaa aaactgtatc tgaaatttaa atctaattgg 134580
   acatgctata atttatttgt taaataccca accctgtgta tagagttgga aatcaccctc 134640
   tttactgatt ataggactgg atattcctgt ctaaatgcta atagtgaggg tagagagaaa 134700
   gaaaacttgg ccacatactc tgacaatact ggtataattg taaataatgt taaactccgt 134760
   ttgctagcaa gaattaaggg agctatgtct gaaatggcac tgttttgtca gtgaattctg 134820
   tttacctttt tcttctgtct ggagtgtatt tgtgaagagt cccttattat cagttatgtt 134880
   ttatgaaaat cagcacacta ccacaatgac atttaagcac agaatcatta gtccatgttt 134940
   ttaataaaca catcgattaa gaaaaaaaaa acttggccac ataaaagtta tttgaacttc 135000
   agtttttttc tccgcaaaat gggcataatc taccaacgcc tcccgtagtt gcaaggggat 135060
   gtgcatgaga taacgtttaa agtactgaac acaggcctgg aaaggtagca agtattcaat 135120
   tgctttcaac cactgctcaa gtcaaccttt tatgaaggcg gggaaggctg agtgaatggg 135180
   taaataaatg ttcttggtaa agcagcacac catcagaatt tcactaatat tcatcacaat 135240
   ttattttcta cccgaagctc aggatccacc ccattccgta tgggaaacaa tcgaaacaaa 135300
   gtggcatagt aagcagtccc atgacagttg tgtggaaatg ccaccctttc tttttaaggt 135360
   gaagcagtaa agcttagcaa gtagcatttg tgcctttgac aaatggtagg ctgacaacac 135420
   ttggacgtgg ttgacaagta caaaatagca ttcagtaggc aggtgccagg gaggggaaaa 135480
   caactcttac gtattgctat ctgtattgag ccagacttta aagaactgga aaactctcta 135540
   catgggaatt aagaacatga agatatttca gtaaaatctg ttattatttt taagatacat 135600
   tgttttcaaa agctcgtttt ttggccatat gggattgtat tggaaaaata ttcctagaaa 135660
   atataattca gcatattttg atggaaatca tttcccttca tttataagta tccaaataat 135720
   ccattgtgaa catcagaaat tattgaaatc tctaatgcag ctttaataaa ttccttcctg 135780
   tggaatatgg gcgtgttttc tgaagcttta ttgaaaaatg aaagtctcac ataaaatgta 135840
   tttcagatca tttaactttt ctatcttatt ttatgatcca gtgaatttta ttcattctag 135900
   caagtcgtgc tgaatctaaa cactttagga ttaagattaa aatactgagt atataaatta 135960
   gggttaataa aaaaatccaa tatatgtttg gaaatgtagc tactgtcact taaaggaaat 136020
   ggtgcagctc ggaagtagag tgtagataaa ttaaccaatt ttttgaaaaa aattgatgtc 136080
   cacagaaata agactcagat ggtctcattg ttaaaaatta aggctgatca aagtgactca 136140
   actccctagc gaatattttc tctagacact aaatcaagtg cctgtaattg catttctata 136200
   gttttattcc atggtgtaat cagggtgaaa aattaagaca agtctctgga caatagaaaa 136260
   tatctttcag atagatttca atttcagttg gttggattag ctataggaag cttgtccaac 136320
   ccacagcttg tatgtggctc aggacagctt tgaatgtggc ccgatacaaa tttgtaaact 136380
   ttctgaaaac attatgagtc tttttgtgtg attttttttc ttagctcatc agctattgtt 136440
   agtgttaatc tattttatgt gtggcccaag accattcttc ttccagtgtg gcccagggaa 136500
   gcccaaagat tggatgcccc tgagctagag aatataagta caaaagagaa aagctatgaa 136560
   atgagtaata agcacctgaa ctttatacct gaactctaat gaaattccac aagtaggggc 136620
   cgggcatggt ggctcatgcc tgtaatccta gcactttggg aggccaaggc aggcggatca 136680
   cgatgtcagg agttcgagac catcctgacc aacatagtga aaccctgtct ctacccaaat 136740
   tagccaggca tgatggcaca tgcttgtagt cccagctact caggaggctg gggtgggaga 136800
   atcgcttaaa cctgggaggc ggaagttaca gtgagccaag accacaccat tgcactccag 136860
   cctgggtgac agagtgagac tctatctcaa aaaaaaaaaa aaaaaaaaaa aaaaaaaaaa 136920
   aaaaagaaat tccacaagta gggtaggtac tgggcaaaag ggaattaaga ctatttgtca 136980
   gacttagtac gtgacctgag ttaagacact gtatatatgc aggtacatat ataaacctgt 137040
   atgtatcttg ggagatctcc tctgtttgtc ttcactggac aacaaaatca tagagaaaaa 137100
   atgcttgtga cacacacaac aacctctccc agccttgcac aaacaggcac accctagaga 137160
   aacaacatgg ttagaaaggc agccttgacg tcaaggtgac gaaaggataa gtataagacc 137220
   agaggtaaga tcactgcttg agaagttgtt aaaataatct gtcaagaaag aatcaatacc 137280
   tggactatga tagtgacaac tcaagtggag agaagtcaag aaagttgaga aaccaaagtc 137340
   agtggaataa caggacgcag aagaaagagg gtggaattca agagcagtca gggaccaggt 137400
   gtttcatgat gctactcaac aagataaaaa agatataaaa aggaaagggt gatggggaag 137460
   acaatgactc agttttgcat aagttcaatg taagatactt gtgggacaca ctaataatca 137520
   ctcttagcaa tgcttttgat agttttagac attcagattg tccaaggtca ctgaaacaat 137580
   ggaagtcaat gagacaggct ggaggactaa ataaattgga tgcagggtgt gagacacggt 137640
   atgcacaaaa gagaacttat ttgtcatttt aagtggatcc actgaagttc tgatgtttac 137700
   gatgatgctg aaaaatacac agagcaatag agaagtgtaa gtaccatggc gtgttggagc 137760
   caatagataa cttcttggta attttgtaag ctgggtgtta gaaacaacta ttattcgata 137820
   ttaaattgta taaatttaga atacataaat ttatattaag cataaagata atacaaactt 137880
   acaacttacc atttcttatt ttattttaat ttataattta caactatcta tgtttgtgaa 137940
   gctatttaca tctattttgt ctatattgtg gaaaaaccat aatggtgtga tgttgtgtaa 138000
   ttctttccag ctctgctttc agtagcatca ccttgacaac ttgaaatcac actaaagtaa 138060
   tcagcaagca ctacaaatca gaacttccca tcccagggag ctacctgtga atcatctacc 138120
   agcacaagac tagttaagta ggaccatcca tatccaagga aggaatagta gaatacaaat 138180
   gtgttccact tactattgct gaattagaaa ttcactccat aatttattag tagcttaaaa 138240
   taacaattat ttcataatta aagctcagct tctgtttctg gaaagatgtg actggatagt 138300
   cctggattgg ggtgtcatgc agttgtaggg ctagagctgg agggtcaggg ctgatcaagc 138360
   atctctctct tcatgtagcc ttctccacac ggtctctctg catgaactat tttggacttt 138420
   ttataacaca atggcctcat ggtagttgtc cttcttacat ggtgtcccag ggctccaaag 138480
   gcaagtgtct taaagaagaa ggtaaaagct acattacttt tcatttcctt gcctcagaag 138540
   tcacagaatc atttccatca cctcttattg gttaaagtca caagcctgcc cagactcaaa 138600
   aagagggaaa ggagactcca attctcaaaa acggagtggc aaggtttaag aagaacatat 138660
   gggattggag atattttggg gccatctttc aaaatacaat ctgctgcaga tcccaaatca 138720
   caaaagaggg agacaactat atgtagctac cagtaggagg gctactctag aggatgcttg 138780
   agaattattg ctttcatcaa tctgagacaa aattcacaca catgagctag aatatcattt 138840
   ccaaatgata ggttggtagg atttggggga attttatgga gtagagaaaa ccaaacttag 138900
   atgataatta acaataaata ttgtctgtat ctttatggct taggaaagtt tcatgtctgt 138960
   aaccaggaac tcactacttt ttatttttgt tttgaacatt tcttctcaat tcattccttg 139020
   catgagattt tgtccccatt ttgaccatca tacttgtttg cattctcctg cttttgtctt 139080
   taatgcttta atttggggat atatatccat atgtctatgg atacatgtat atggatatgt 139140
   atgatttggg gatatatatc catatgtata tacatatgta tgtgtataga gatagacaga 139200
   tgatagatag atagatagat agatagtaga ttcttctcta cttgcaatgg ggtgatgtcc 139260
   tgataaaccc accaaaagct gaaaacatta tcaactgaaa aattatggtt atttagcctt 139320
   gtgcccatgg gctaaagaaa aactgtggct catccagaat cataagagct tatcctaacc 139380
   tggaaaagat caaaattcga aattcaaagt acagtttcta ctgaatgtgt attgcttgca 139440
   ccatcgtgaa aacaataaat cacatggcaa accctcataa gttggagacg ttctgtgtgt 139500
   acacgtgcgt gtgtgtagtt tgttcgtttg cttttttttt tttttctgtt ttcctgataa 139560
   ctttagtttc tatggacagc atatttagtt ctcacctgct catgctgcct caggttttct 139620
   gatgataggg tgaagaagaa aatgcctctg tggcctaagt tctgacccct ctattaagtc 139680
   caaaagccca aaaccgtatt tctggacttc caaaagtcca gaaataaaga tacaaagaag 139740
   aaattctgag cgggcaaaat gggtactgca ttgttgaata aataaaggga aagttctaag 139800
   cagagtttaa attaactaac aatttttttg agacaagacc acattttgga aattttagaa 139860
   aattgtttta agaaagaaca tgttatggta tttattttgt attgaaaata gaataacaga 139920
   aaagcttaga ggcagaggtt agaatctata gaaaattctt tagggccggg cgcggtggct 139980
   cacgtctgta atcccagcac tttgggaggc cgaggcgggc ggatcacgag gtcaggaaat 140040
   cgagaccatc ctggctaaca cggtgaaatc ccgtctctgc taaaaatgaa aaaaaattag 140100
   ccaggcgtgg tggcgggcac ctgtagtccc agctactcgg gaggctgagg caggagaatg 140160
   gcatgaaccc ccgggggacg gagcctgcag taagccgaaa tcgcgccact gcactccagc 140220
   ctgggcgaca gcaagactcc gtctcaaaaa aaaaaaaaaa aaaaaaaaaa aaaaaaaatt 140280
   ctttagctgt tctctaaatt tgataaaaat tattgagagt ctgccaggtg ccaggttcca 140340
   tgctaggttc ttgagacaga gcgagaagta agatagaaac aagcatctgg taggcactta 140400
   aaatatatgt tgaaaattac atgatgcagt agagcctgcc aattagaggc tcatcctcta 140460
   attttggaga tgtgacagct ttttatggga attaaaaggc caaagaaagc tgaaggtcag 140520
   gtctagacac agtccaaagg tgaaatataa ttaaaaatgc tcgcttgttc aacaatatcc 140580
   aggttgcctg tttgctcata gttattagat gattaagtat ctgtaattca gttaagcatt 140640
   ctaagctgca gaaaatgcta taaaccagtg ggtttttttt tcattttcat tttctatttt 140700
   tgaaatagcg tcttactctg tcaccatggc tatagtgcag tggcgtcatc acggctcact 140760
   gcagtcttga actccttaaa ccagtggtgc ttcaaaccta gctttttctc agaaaaacta 140820
   ggcagcttaa aagtgatact gaccaatggg taccaaacta aatcactcaa atcagaatag 140880
   ctattggtat ggccagaatg gatttttgtc aaagcttctt gttatttcta gtgtgcaacc 140940
   agcactgcaa actcctgacc taaccaactt gtgttttctc tttcccctta tgtgctatcc 141000
   catctgccac cagatagctc ttggtaagat gttacctggt gtgttagtcc gttttcatgc 141060
   tgctgataaa gacatactgg agactagcaa gaaaatgagg cttaactgga cttacagttt 141120
   cacatggctg gggaggtctc agaatcaggg caggaggtga aatgcacttc ttatatggtg 141180
   gtggcaagag aaaaatgagg aagatgcaag agcagaaacc cttgataaaa ccatcagatc   141240
   ttgtgagact tattcactac catgagaaca gtatggggga aactgccccc atgattcaaa   141300
   ttatctccca cagggtccct cccacaacat gtgggaatta tgggagtaca attcaaggtg 141360
   agatttgggt ggggacacag ccaaaccata tcacctggtt atctgaattc tgatcaaggg 141420
   cacttcagga atttgggaaa ttttcaggta ctgttgttct caaacgtagc tacatattga 141480
   aataacctag agaattaaaa aacagaaact cacttatgcc tgagtgctat accctcctct 141540
   gcttacaagt atgttcagtt catatggggt atggcctgag cataacaatg tctgaagctt 141600
   cataaatgac tggtgtgtgg tcaagttgca gaaccactgt gctagaacat cctgatgata 141660
   aagatctgac tgtcagttca gatacaatct aaatatagag aaattcttaa cagaggatgc 141720
   agagtaaatg acttcaggtg gaaagcacgt aaagtaaatg tgagaagaag gtcaggtggt 141780
   gagtgtgggg agctggggaa agcatgctcc tgggctccct ccaacagaag ccacctgtag 141840
   cctgggacca gtgtgctaga ggaacctgtt ttcaaagaaa gccagaagta tggatgcatt 141900
   tttatttgca gcctgatgat ttttatattt tggcaactaa ttcactttat taaaaaatac 141960
   tgaataagct caagaaatct gtgagctgga ttcagccaaa gcgaatgcca gtttatgact 142020
   ttcctcttgg aaagaaaagt ctttcatgtt gatgtatcac attttcgcca gcttttctgg 142080
   ggttatgttc tcagaagttt cgtttagttt gctctcatga aaggtgacgg taatgttcct 142140
   cagctactta taaacatttg aagaaacttt tcctctgtat tcttagtttt atactcagtg 142200
   aaaatataag tcaagtattc ataggtattt cactgttaga tcaccatatt gaggaatgag 142260
   tgttagttca ttaatgccgt acacaaacac ccaaaccaaa atgtgcagtg cttgtggaat 142320
   cacgatgaga taattagcaa tttcttcact tcccactgag caggatgagt tgaggattag 142380
   cttctctcag tagattacac attagagaga aaaatgtcaa acagcaaagt aaaatgggac 142440
   tctacgtctt cccacacaag catctccagt gcactaatga gctagcttcc ttgtcacatt 142500
   cacaactagc agcagatgac ctaagaataa gtcagagaag ctttctatta gaggaacttt 142560
   atcttgcctc taaatatata gtaaaaaagt gggtcaattg gtacttacag aagatatttt 142620
   agttcaactg gagaaactta actattttag aaataaagaa cccctggaaa agttagtctc 142680
   ccttattatg catggaaata aaagcacttt cttaatttga aataagtgaa ttttttactg 142740
   aagaaattcc tttatgctgt cctcagtcca catattaacc ccatgtatca aggcctctga 142800
   tctgccatct caaccacctt gtcactgtcc actaacctct tgctgcttcc cttctctcct 142860
   tgttccacct agggttcaca gtctctgctt tgaatcagac attcacagga tcagacatgt 142920
   gattcaggga cttagcaaga gggagtgatc agtgcattgg cctctgtttt atgacaaacc 142980
   accaaaggac atgtgtcctc acacaacagg tacacttgct ttggcataca gaacctcccc 143040
   agatggctgt gctggaggtg agtagatggc gtgaaggaat gcctctgcac atatttgtca 143100
   tttcctgttt cccattgatc aaaatctctc caaagagcac actgtctctc cttctttata 143160
   cttatggact atgacacttg gccacactga gaactgcaag ggtaatcaga tcccatacac 143220
   tgcagtatgt attttcattg aagtgtacaa atgacagagg agttaacact tttggactgc 143280
   cccagtcctc tggcagaagt actaatccct ctgagaaata catttctgtt ctttatcaac 143340
   tgcccagtct caagtatttt gttacagaag tacacaacag attaagacat atgtcttctt 143400
   tttaagaaat gcctaatcag gtcctttatc catttcagta ttttattttg ttttgttgtg 143460
   ctactgagtt gcatcagttt cttatatatt ttgggtatta accccttatc agatatatgg 143520
   tttgcaaata tttttgctca ttatgtagat tgctttacag tctgttgatt gtttcttttg 143580
   ctgtgcagaa tatttttagt ttgatgcaat ctcagtttat ttttgcttgt gttgcctgtg 143640
   cttttggtgt cataaacaaa atattttttg ccaagaccaa tatcaagaag ctttttcaga 143700
   tatattttct tctagtagtt gtacagtttc attgtcttat atttgttttt aatatgatgt 143760
   gagagaaagg tcaaatttta ttttttttgc attggattgc cagttttgcc agcatcattt 143820
   attgaagaga ttatcttttc actgttgtgt gttcttggta tttttgtcaa aaatcagttg 143880
   actatcagcg tgtgaatatt tctaagctct ctattctgtt ccattggtcc atatgactgg 143940
   ttctatgtta gtaccatagt gtttttatta ctatcgcttt acaatacatt ttgaaaatgt 144000
   gatgccttca gctttatcct tcttgctcaa tattgctatg ggtatttgtg gtcttttgtg 144060
   gttccaaatg aattttaatt tttttatctt ctgtaatgaa tatcactgag attttggaag 144120
   gaattgcatt gaatctgtag atcactttgg gtagtatggg cattataaca acattaattc 144180
   ttttgatcca tgagtggaga atgtcttttt atctgtgtct tatgcaaaac aatatggaac 144240
   ttctttaaaa aattaaaaat agaagtacca tatgactcag aaatccaatt tctgggtata 144300
   tatctgtatt agtccgtttt catgcctctg ataaagacat acctgagact gggaagaaaa 144360
   agaggcttaa ttggacttac agttccacat ggctggggag gcctcagaat catggcagga 144420
   ggcgaaaagc acttcttaca tggcggcagc aagagaaaaa tgaagatgca aaagtggaaa 144480
   cccctgataa aactatcaga tcttgtgaga cttattacct accatgagaa cagtgtgggg 144540
   gaaactgcct ccatgattca attatctccc actgggtccc tcccacaaca tgtggcgatt 144600
   atgggagtac aattaaagat gagatttggg tgggaacaca gccaaaccat attgctctgc 144660
   ccctggcccc tccaaatctc aagtcttcac tttgcaaaac taatcatgca ttcccaacag 144720
   ttccccaaag ttttaactca tttcagcatt aacccaaaag tccacagtcc aaagtctcat 144780
   ctgagacaag gcaagtccct tctgcctatg agcctgtaaa ataaaaaaca agctagttac 144840
   ttcctagata caatgagggt acaggtattg gtaaatacag ccattccaaa tgggagaaac 144900
   tggccaaaac aaaggggtta cagggcctac gcaagtccaa aatccatcag ggcagtcaaa 144960
   ttctaaagct ccaaaatgtt cttctttgat tccctatctt acatccaggt caggccactg 145020
   caagaggtag gttcctatag tcatatacag ctgtgcccac gtgaccttgc aggggataac 145080
   ccccctggct gctttcatag gctggccttg agtttctgcg gcttttccag gtgcacggtg 145140
   caagctctcg gtggatcaat cattctgggg tctggaagat ggcggccctc ttctcacagc 145200
   ttcactaggc ggtgccccag tagggaatct gtatgggggc tccaactcca catttccctt 145260
   ctgcactgcc ttagcagagg ttctccatga cagccccacc cctgtagcaa acttttgcct 145320
   gggcatccaa gtggttccac acatctgaaa tctaggcaga ggttcccaaa tctcaattct 145380
   tgacttctgt gcacccgtag gctgaatacc acatggaagg tttggggctt ggaccctctg 145440
   aaaccatgga gcgagctgta ccttgacccc ttttggcaat ggctggagtg gctgggacac 145500
   agggcaccaa gtccctagac tgcacatagc acggggaccc tgggcccaac ccacaaacca 145560
   tttttccctc ataggtttct gggtctgtga tgagaggggc tgccaagaag atatatgaca 145620
   tgccctggag acatattccc cattgtcttg ggattaacaa tttggcttct tgttacttat 145680
   gcaaatgtct gcagccagct tgaatttttg ctcaaaaaat gagtttttct tttctactgc 145740
   atcttcaggc tgcacgtttt ctgaattttt atgctgtttc ccttttaaaa cggatgcttt 145800
   taacagcaac caagttacct tttgaatgct ttgctgctta gaaatttctt ctgccaggta 145860
   ccctaaatca tctctctcca gttcaaagtt gaatctctag ggcaggggca aaatgccacc 145920
   agtctctttg ctaaaacatg acaagtcacc tttactccat ttccctacaa attcttcatc 145980
   tccatctgag accacctcag cctggaccat attgtccatt ttgctatcag gcttttggtc 146040
   aaagccattc aaccaatctc taggaagttc caaactttcc tacattttcc tgtcttcttc 146100
   tgggccctcc aaactgttgc aacctctgcc tgatactcaa tttcaaagtc actcccatat 146160
   tttagggtat cttttctgca tcaccccact ctactggtat ttattttaca atttacaaca 146220
   atctatgctc ttgaagctat ttacatctat tttgtctata ttgtggaaaa accataatgg 146280
   tgtgatgctg tgtaattctt tccaaatcta ctttcagcga tgtcaccttc atactaatgc 146340
   ccaggtctca ctgtattagt tcattttcac gctgctgata aagacatacc agagactggg 146400
   aagaaaaagt gatttaattg tacttacagt tccccatgcc tggggaggcc tcagaatcat 146460
   ggtggaaggt gaaaggcact tcttatatgg tggtggctag agaaaatgag aaagatgcaa 146520
   aagcagaaac ccctgataaa cccatcagat cttgtgagac ttattcacta ccatgagaat 146580
   agtatggagg aaacttatcc caagattcaa attatctccc accagatccc tcccacaaca 146640
   cgtgggaatt atgggagtac aattcaagat gagatttggg tggggttgca gaatgaaacc 146700
   atatcaatat ccaagaaaat aaaacttgga tctttaagta gtatctgcac tcctatgttc 146760
   attgtagcat acatatatat atacacacac acatatatat agatatgatg ggatatttct 146820
   ctgctttatt tttagcctta gaaaaagaag gaaactctat aattggcaac aatatgtacg 146880
   aacccgaagg aacttatgct aagtggaata tgccagacac agaaaacaaa tactgcatga 146940
   ttagatgtga aactgaaata gtcaaactca cagaaggaca aagtggcaac ctggtgattg 147000
   ccagggtcag agtggggagg ggaaaattgg gagatgtttg tcaaagaata cagttttagt 147060
   tatgcagaat gacattccgg tgatctaatg taaagcatgg tgactacagt ttacaataat 147120
   gtattatatg cttgaaattt actaagatgg aagattgaag gtgttctcac cattccaaaa 147180
   aaaaatggta attatgactt cactatatat atataatata tatataaaca ttacattgta 147240
   tactttataa gatttctata tgtcaattat ccctaaataa agctgaaaaa atttaaaaat 147300
   aatcccccga aatatatgta atatgaacaa taaatataag ccacacatgt tttttaaaaa 147360
   tttgtcatgt ttttaccctg acagcaaatc tccaattgga tagcctcatt tcaagtgctc 147420
   cacaggcaca acgaacaacc gactaccatg tgggccactg cagccctagg aaatacgaat 147480
   gatgggctct gaaccaatct aagtggaatg actttaaaca gaaataatga ctcaagttct 147540
   cgaacttaag tatgcttcag tatcatgtgc agagcttgtt aaaacataaa tcgctggact 147600
   ccacacccag agactgattc aatagttctg aagtggggat cccaaatttc cttttctaat 147660
   aattttccag gtgatgctaa tactgccctt caagagccac gggtgatgag gctttgggaa 147720
   atcagctttg taacttttaa gtggaaaaag ggacatatag ttcctaaaca tgatataaat 147780
   ctctcctcca ttacagtaac aattagtgct atagtagaaa agacaggtgg tcaaattgta 147840
   cttaagatta caatttggtc actaaatgga ataacacatg tttttttttt tttttttttt 147900
   ttttttttta gtcttgttaa ccaattgcag ttgaagatgc aggtatttga atattatata 147960
   cacacatatg gcacatttaa cttagtataa tttgtgcatt aaagtaaaaa tcgagtcatc 148020
   tacatttcca ttcactgaag gaaacatcta ggcataattt cagtaaacaa actggggagt 148080
   ggaacgttat aacatgtgca taattcaatt tatttgaaat tgactgtaag tcctcattag 148140
   gccttaagtc ccttttcagg tgaactatgt ctcacattta ctgttgcatt tctagttttt 148200
   aatatcttgt atactgtagg tctctaataa atagatgcat gagtagtttt atttgtttca 148260
   tatatttttg ggaattcagc cttcagttat cctgaaattt taatacagtg ttgaagtccc 148320
   attgtgaggt caggagagtc tgtatgtatg taaagataga catctctcta aatcttttgt 148380
   tcctttctgt aataagtatt aggaatataa tgcacttcag agtaaaagtc cagatgctga 148440
   caagctactt ttaaccttgt gattctggat ggtctgatta tagagtccat ttccaaactt 148500
   cctttcacta cacaggagcc tggagatgaa ttaaaaatta cctgatgctc tgaattgaca 148560
   tttcacagtg cagtatcata acatattgta agaagagatg ctacacacga atttttaaac 148620
   gattaaaaat atatctctac tgatgcaaga aaggaacttt atgaatgtaa cattttctca 148680
   aaattggtct cataaatgaa gatcaaaaac ctttagtcaa gtggaagcca atttagagtt 148740
   gttactgtca agcatctata acttttcaaa taaccaaatg ctattgctaa catcaggctg 148800
   tagagtttaa attttcataa attcgactat ggagcctact tcattgccgg actagaatga 148860
   caaatacatg ataacctaca tgggataatt ctttaatatt gttgtttggg gttgacaatg 148920
   agaatccacc cattgactga gttccaactc ttttccaggc acttaatttt tactcactga 148980
   aatacttgtc acagccacct tataaagtaa aaattattat ttatgcccat tttctataga 149040
   gtatactgaa gctcagggag cttaagtagt tttctcagtg acatggatgg taattggtca 149100
   aatctgtttt gaaaagttcc tcagactctg cagcctgcat tcctatgact gagctatgct 149160
   aattagcaag agacagaggc ctttggtaca caccttttta tcagggaaaa gacttcttgt 149220
   gaataaataa acactaagtc gtatcagtat acaactaaat cggccctaac gcaaggaaat 149280
   ccttaatgat agcatcatta tgactctctt aaactatttc actggaaagg gaatttttaa 149340
   ctctggggct ttgagagctg gactcagtct gaaggagctg ataacatcaa ggatttgtgc 149400
   tggcaagggc aagtgcagtt gctccactat ggagatcata ggttggccct acagctcttt 149460
   ccttacaaaa aaatctatag aaggtgattt ttatggcatt atctctcttg caatagacag 149520
   tgaggaaatc ctcaattgtt ctatttctac agtttcgtgg atcagagtac aattcttgct 149580
   tccttcgtgt ttaggctccg gaaagggatt tatcatgaga gcaaacaaat aaaaagaggt 149640
   agttcagata agacaaaaga caaaagaaat agaagtatgc cccagaggag tagatttact 149700
   atttattctt tcctgctttt gacatgagat ctataccatg atctttggcc ctgactacat 149760
   tctggacaca cattttatat agtgtttgtg ccttagggga acagtggcta ctatgcctcc 149820
   tacctgttat cattaatttt tctttttcaa aaaaaagtca atgaaccata tttcttaaaa 149880
   acattcacca agctcctcag agaaaatggt ttactctgct agaagttatg tctcaagatc 149940
   tcccctctac cccagacaaa ctaatctcca tcattatgac aattctccca tacttacact 150000
   tgatgacagg tcacaaattt atgaagttga ctgagttatg gctcaacaat ttagtcttta 150060
   ttttctttgg ggagaaaaat gatatgaaat catataagat aaaaattttg gagcaataaa 150120
   tcactcaatt tgaaataaac atcagtacag gacataaaga tgtcatgaaa catttccacc 150180
   caattgttta atccaccctc ttagtcaagt ccagagtatg acattatctt tcttttctgc 150240
   tttgtgaagc caagctcatg tttgaaatga gtttaaaaac aaatgaattt cagacttaca 150300
   gaatgttcat cttttagtgt tgttattaag aaagttaatg aaaacagtat tcttaccctg 150360
   atgctccagg aaaggcatta gatccaaatg agtacataac attccatcat tctaatttcg 150420
   aggttgatat ggatagccaa tgaaatccta caattgcaaa ttgcttcaaa gtatttcacc 150480
   ataatgacta gactgcaatt ttgatgggtt actgataaac accaaaaatt aataataagg 150540
   aaatagatga aatcacttgc attattgtat tcaatacaat cttagtaatt aatataattt 150600
   ggtcataata tattcatttg tgaacttacc atatgcctat tatgcataga gcactgtgta 150660
   aagcactgga aatttgtatt tgttcaacaa ataattactg agtacttctc tgaatagaat 150720
   attactgcag aactggggat ataacagtaa aaagtcacac atgaaaaagg cctcccttca 150780
   tgaaacacat tctaatggag aataggcaaa gcacatgata aataaattag agtatattgg 150840
   aaagtgttaa gtattgaaga ggaaaaatat aaacaggcaa ggacagagac cattttaatg 150900
   agatggtggt ggtgacactt tagactgtct gtagggtagg cctctcatga aaagaagtga 150960
   catttgaata aagattggga agaagccagg aaattatcca tgagggtgtg tatggaaagg 151020
   gcacacaagt agatggaaag aagggaaacg agctgagcat tttgtggaag agtaaggagg 151080
   ccggggtcct ttactggatg ctgtgagggg gcgagaggta gaagattaag ccagagagat 151140
   aaagggaatg gctgatatgg aaggtcatat gaggattctt gagagtcact ggaggatttt 151200
   aggcagagta gagagctgat ctgatttggg aggacgactc tgactattgt gataagacta 151260
   gaatgcaaaa ataaataatt tatggtccta atgaactcac acttcatttg aaaagttgaa 151320
   aaggatatgg attcagcgta gtgagataag ttctattaga gtacagaggg ttgatagtaa 151380
   cgggtaagac tcctgctcac tcctcagcct actccaatct ggcttctagc cctgtcatta 151440
   cactgagaca attcttgaaa ggattctgat gacttttagg ttgccaagct ccatgtcctt 151500
   cctctagcct catcttactt gacttttctg cagcaattgt aacattttat atcgccctac 151560
   ttcttgaaat actttcttcc cttgttgacc atggctctac cttgtccata tttttttctt 151620
   gtttctctgg gtaggcactc acttttcagg ctcactttcc tctccctagt aattgggtct 151680
   tacaatttct gtccatagca gccacattaa caactccaaa gggctcactc acaacatagt 151740
   tcaagggact ggtggttttt tgaagttgta tattgctgtc ttgagctgcc taaaatgaat 151800
   aactcatttc tgtcttattt tttgtctcct tgttttgatt ttctttacat aaacaatttt 151860
   aaccatgcat gaggcttcaa ataggactta attgcaagtt tatgtttcca tccaggattc 151920
   tttgttgtgt aacatactca tattcagttg actgcttgac atctctggtt aagtgtcttg 151980
   aatacgcctc aagtttataa tgtcccaagg tgaactcaag ggcctccttt tgtacagggt 152040
   tcttctccag tgttctctgg cttgatggat aacactacca cccatgcatc ctgctatgca 152100
   agacagaaac ttaagtattc ttctggatgc ccctcacttg cgtaccctcc aaatccaact 152160
   cattatttat tatccaatct attgatattt ttcctcaagc catcaccata gtccatgtga 152220
   ccatcgtctc ctgccctttc ttcctccagg agttttttct ccttactaca gtcacagaga 152280
   ttttctaaac tgttaattga gtcatgtcac caatcaccta cctgtttaga acactttaat 152340
   ggatttcctt ttttatctta gagaaacatg atcatagtct ttcagtttcc aaatatccta 152400
   accattgcct acttccccaa gctcctcttt ctcttaggct ttcttcctct ttccacttta 152460
   cattcaaact gctactagcc ttttaaaatg tttttgaaca gggctgttac gcatatgagt 152520
   tgctctacca aagacgtggt gcttctttct tctatttctc atgcaaacat attagcttct 152580
   actttattct agattttagc atagttatta catcttcttc taaccctgcc ttcaattccc 152640
   aggtgaaata aattaccctt tataagctct catagtatat atacctctcc tgcagagaac 152700
   ttttcattga gacctagact gctgccaagc acagtgcagg tcctcaaaaa taattgctca 152760
   atataaggca agaggatttt ttggcaggtg gtgccatgct cttatttaac caggatatca 152820
   gatgataccg tcatttattt acatattttt ctgaagcaaa taatcactga actacatcaa 152880
   gtatcgatga ctatctggac cttgaaattt taggcaaaaa tattacttat agggtttatt 152940
   tgggatattt aatattttaa ttagatttga aaatattatg gagacaagtt aaaacataat 153000
   gtgcttagga gcttgtttcg aaggttacac aggtgttgct tgtactctaa tctcagaact 153060
   ctcaagttct tttttttttt gagatggagt cttgctctgt cacccaggct ggagtgcagt 153120
   ggtgtgatct cggctcactg aaacctccac ctcccgtgtt caagcgattc tcctgtctca 153180
   gcctcctgag tagctgggat tacaggcacg tgctaccatg cccagctctt ttttgtattt 153240
   gtagtagaga cagggtttca tcatcttggt caggctggtc tcgaactccc gactttgtga 153300
   tccactcacc gtgacctccc aaagtgctgg gattataggc atgagacacc actcccggct 153360
   agaactacca agttctaact gtgattctag acaaaaattt ccatgcttca ctttctcctt 153420
   ctacaaagtt tcagtgaatg ttaaaaagtg aaaatacaca aataaaacat agttaacata 153480
   cacccgacta gctcttcaaa aacattagtt ctgttttcct gcacttccca catgttgata 153540
   gaatttctcc ttctcaagtg ttcttaccag ttaaggcagt gcttctttgg cttcggatac 153600
   agcataacgt ttttgtctgt aaagatcttc tctccctgaa tctgataaac ttaaatttaa 153660
   tagcatgtac acaaagtgac atagatcaag caggagaagc tagacaggat tctccacacc 153720
   actgttgagg aaaggaaggc ataatgccat cacctgcaat tagaaaagtt gtctgagcca 153780
   ttacaaggca ttttcaagtc tcccataaca ttttagcacc tgtaggaaag cttctctttg 153840
   taagcaaaag gttaatagct gtgcttctcc agctgtttca cgtgggccag atgtctgagt 153900
   agcccaaact acttaactgc atattagtgg cacataaact gcaggctgcc gcttgtcaga 153960
   gagttgttaa tttccataga atgttgcact gaaagtcatt actatgtgaa ttacaacaaa 154020
   tttgttaagg aggttatggt aagcattggt acaaggatta gtgcgtttga aaaaaaaata 154080
   gaaggttcat cctctacgtg atctgagcga ctctctaagg tcttagtcac acacacacat 154140
   gccccatgct ttattttgtg tctgagtttt agcttccaca ggttgccagt atccatgtat 154200
   tgtgtgtggc caagacatcc tatcaaaact gaattgtata aaatgtcaaa aatcttatga 154260
   aacttgttag atgtttatgc ttattgagga tattttgtta tgatagtcaa ccataatatg 154320
   gagttctata acttaaaaat aacgatcagt gcatgtactg aattcaaact aagcttcaga 154380
   ttcagtactc agttttctaa gtgtttatct cttgtaggat agggtctgca aggtgaatct 154440
   atgggtcaca tctcacatat ggcccattct cacacacacc atctcatgta atcctggtaa 154500
   tgtttgcaaa aggccattgt cgtcgtcgtc gtcttcttct tcttcttctt cttcttcttc 154560
   ttcttcttct tcttcttctt cttcttcttt cttcttcttc ttcttgttct tcttcttcct 154620
   cttcctctgc tttcttcttc ttcttcctct tcctcttctt cttcttcttc ttctatcttc 154680
   ttcttcctct ttttttttct gaaggtggaa cagacctaga agctggagtt cagtgtttgt 154740
   tggtgacaca ggattttaac ttcacattta tgacttcaca gtcgctgata ttagtgttga 154800
   tttttttgtg gattagtata gagtattttt ctacaactac tgatatcaga caattgccag 154860
   aatgaggatt ttgttggcag ggctagcaag gaggaagggg agcatttcaa agctgtgtcc 154920
   agctgaatga gactgcctag ggcttaaggg ggcagcctca ctcagggagg ttctaatgat 154980
   cagtcccttc tccttgacca atgtgtgtgt gcatttgccc caataaacag tcctggtcat 155040
   tttgaccgaa gatatttttg aattagtctt atatgctatc tacataaatt gatgctatgt 155100
   aaccactaca tacaatggag tactattcag ccataaaaaa agaatgagat cttgtcattt 155160
   gaaacaagat aaatgaaact agaggtcatt atgttaagtg aaaaacctat aattttaaaa 155220
   cagaattgtt cttttaatga gacttaagga atattaattt tatttttaaa atttcacagg 155280
   gagaggagag ctaaagtaag tgtgtgtgtg tgtgtgtgtg tatgcataca aacataggtg 155340
   gaagatttgc taatggcaga taattatttt tccttaatta cctcacttag ttattttatt 155400
   taaacattga aacaattttg ctaagtcggt attatctaca ttttatagaa acatttagaa 155460
   aatgtgaata ccatgcctgg ggttgaaaat agagcatttt gttttgcttt ctttcacctt 155520
   cttcactttg cttcttaaag gatataatct tctttttaat caattgtggt ataacatata 155580
   tattttaatt ataatagaaa tgtttatttc atatgttgtg atcataaaaa cttggaaaat 155640
   aaaaatagaa acaaattcaa attacgattt gtagtttctt ttttttaaca tgtcatatag 155700
   cttattgctt atttctgaaa aaaaaattgt cttggaacct tccaagttgc tgcccactct 155760
   gaaatgtttt actctataca gcctttgttt ggggcatgta actttctttc tttttttatt 155820
   attatacttt aagttccagg gtacatgtgc acaatgtgca ggtttgttac gtgtgtatac 155880
   atgtgccatg ttggtgtgct gcacccatta actcgtcatt tacattaggt atatctccta 155940
   atgctacccc tcccgcctcc cccaacccca caacaggccc cggtgtatga tgttcctctt 156000
   cctgtgtcca agtgttctta ttgttcaatt cccacctatg agtgagaaca tgcagtgtct 156060
   gctttttttg tccttgccat agtttgctga gaatgatggt ttccagcttc atccatgtcc 156120
   ctacaaaggt cataaactca tcatttttta tggctgcata gtattccatg gtgtacatgc 156180
   gccacatttt cttaatccag tctatcattg ttggacattt gggttggttc caagtctttg 156240
   ctattgtgaa tagtgctgca ataaacatac gtgtgcatgt gtctttatag cagcatgatt 156300
   tataatcctt tgggtatata cccagtaatg ggatggctgg gtcaaacggt atttctaatt 156360
   ctagatcttt gaggaatcgc cacactgtct tccataatgg ttgaactagt tcacagtccc 156420
   accaacagtg taaaagtgtt cctatttctc cacatcctct ccagcacctg tttttttcct 156480
   gacttgttaa tgatcgccat tctaacttgt gtgagatgat atctcattgt ggttttgatt 156540
   tgtatttctc tgtggtataa tatatcttaa gagtcttgtg tctgtcatta caacttaaat 156600
   aaacttatta gaattgtaaa cttaagcatt tacatgtatt ttacctagat attgaagtat 156660
   tcagtcttta gaaaaaatga taaagagggg gaaaacatta gtacaaaata agatatacta 156720
   acaaaagttt gaaacgatac aaatgaggca atttaccagt tacctaagaa tgcacaaatg 156780
   cattaaacta acagggatgt gttttaatag ctgtgatatt ttaagaagtg ccgatacagc 156840
   tattccattt cttattcaag tatctctaag gcacttctag gtacacataa ctgggagaaa 156900
   attttaaaaa ccaaccaaac aaaagaaaac ttttcctatc tgttccaaac acacatgaca 156960
   gacagattaa aatctatttt atgttaaaaa tataattgcc tttaaaaagg agagaggaaa 157020
   tatctgctaa taccaaagac aaagggtaaa taaatttaat gcctatgagg gggggaagaa 157080
   ggcagatttt ccacaaagta acagcgatta ctttgacaat agattttatc actaaaaata 157140
   aataaggtca ataaactatt atccttaaag tattcagttt agaatccttt atgatagtta 157200
   taccattata ctatgtggtg ggtgtcagag tgaaggcatt taagaaatat aaagactata 157260
   aaagcttatt atccacccat ctggaaagga ccactaaggt tgtagctgag gaaacaatac 157320
   tgtagtttaa aaaagtaagg ttcaaaataa aatcttaaca gcaaagtgtt gaaacatgct 157380
   ttattagttt ttcaatactg tggtaataca aactaccaca catgtagtaa cttaaaatta 157440
   acccagattt tttatcttat agttgtggag gtctaaagtc caaaatcctt ctcactgggc 157500
   caaagtccag gtgttggcag gactggcatt ttgggaataa ccttggatag aattcttttt 157560
   cttgcctgtt tcagcctctc aattctactt gtaattctta ggtttttgct cctatctcca 157620
   tcttcaaagc cagcagtggt gcatctttta acctccgtga cttctgcttt catggccaca 157680
   tctccttgtc tcactctttt gcctccttct tttctgtata agagcctttg ggcccatgca 157740
   cataattcag gacagtctct ccattgaaat atccttaatt taatcacatt ttcaaagtct 157800
   ttttgccatg taaggtaaca cgtttacagt ttccacagat tagggggact aatccaactt 157860
   tgggggacta cttattctgc ctaccacaca tattgataga gctaaaaaaa agtactatac 157920
   aaaaaaaaag aacagtgatc ctgttttttt ttaaaacaga gcaagagaga ggaactacat 157980
   tagtaatgtt agtaaataaa catcaaacta actgtaaaga aaataaaagt taaaataaaa 158040
   gttaaaaaag ttattcaaag tcaaagtatt aatacactat tgagaaaatc aatagagcaa 158100
   agagttggtc ttttgagaaa acaaatatgt ttgataaact gctagaaaga ataaccaaga 158160
   caaagcatgt ataatgcaca aatcatccat atcacaatac gcatacataa aaagttaata 158220
   agagattatg aattgttata tatgaatcaa tttggaaact tagatgagat ggataaaatt 158280
   tcccaaaaac tgcaacttag tagaaaatac accagaagaa atataagttc taaatagtcc 158340
   tatatctatt taaaaaatga atacctaatt aatgaagaaa acttttgtga gattagaaca 158400
   aacttgaagc ccaaaccagt taagacatta taagaaagga gaattgctga cctgtctttc 158460
   tcataagcgt agatacaaag aattctggat ggactagcta tttatttatt tccagggata 158520
   tattaatagg atgatacacc aaaaataaat tggttttatt ccaggaacac aaggttggct 158580
   caacatttaa aaattgaaca atttaactta tcatagtcgc aaaacaaatg agagaataca 158640
   tctgattatt tcaacatttg caaaaagttc acttgttgta atttagtacc cactcatgac 158700
   ttgaatgtgt acttagaagg tataaataaa tgtatagata aattatgaga actactgagt 158760
   gaatttagta agatcattgt actcaagatc gataaagaaa aataattttt gtatgtatat 158820
   tgccacaaag aactataaaa tttgaaaatc ggatgccatt tataatacca taatataact 158880
   ctagaaaaaa aactaaaaaa cccttaaaag gtattaacaa ggagaactac aaaatattac 158940
   tgacagaatg tacaaaagac ttaaatatat aaagttatat tctatgttaa gcagtaaaaa 159000
   actcagcgaa attaagttgt gtttttccct aaattgatcc attggtttac tacaatcata 159060
   atttaaatct aagctgcatt tttggctgaa aaatttcaaa ctgaatctaa actatactgg 159120
   gaaatgcaaa agacctagga aaggcaaaac actcttgaag aagaaggtgg acaatttata 159180
   cttgtgaagg tgttgcattt attatgatac tctagtaagg aagacagtgt agaattagtg 159240
   taaaaataca caaatagaac aataaaagaa aaaaaataga ctttccaaca aattatgctg 159300
   tgtcaattaa atattcctat agaaaaaatg aatcttctat tttacaccat atgtcgaaat 159360
   caattttagg tgaattgtaa atctaatatg aatggcagaa caataaagtc ataaaagata 159420
   atatgtgatc atagttttat aatcttgggg taagcaaagt tgtttttttc aaaactggac 159480
   ataaacaatt ctaaccgtaa agaaaattat tgatatattg aattatgcta aaaagacatt 159540
   ctgctcataa aaatatatga ttaagcctgt gagagtcaaa ccacaggaga gagaagattt 159600
   ctgaatttct agcaatacca agacaaaggc atacaagaca attttaaaac tgggcaaatg 159660
   cactttacaa taaatctatc aaaactgcta gtaaatgtga caatgtgctt ggcatcatta 159720
   attattctag aactatgacc tgatgctaat gcctgcatat taagagtgaa tacaattaaa 159780
   aacagaagaa aaaaactctt tattatcaaa atttctcata tcttactggt gggagtgtaa 159840
   attggtacag tccctcaaaa atctctttta cagatccact aaagctaaac atttgcgtac 159900
   tgtataaccc agcagttcca ctgtcaggta tatgccccac agaatgttat gcatatgttc 159960
   acaaaaatgt atacacagga atcttcatag gtgcaatgtt catgtgactg taagtgtaaa 160020
   caattcaagt gtttataaac actagaacag ataaaaaatt ttggtatgtt catataatgc 160080
   aatactataa agcaatgaaa atagacaaag aattgccata tccaacagaa taagcagaaa 160140
   tgaatacaga caacatgatt ccatttatat caattttatt tatttatttt tacttatttt 160200
   ttcttttatt tatttatttt tttgagatgg agtctcactc tatcacccag gctggagcgc 160260
   agtagtgcaa tcttggctca ctgcaacctc tgccacccaa gttcaagcga ttctcgggcc 160320
   tcaacctccc gagtagctgg gattacaggc acatgccacc acacccggct aatttttgta 160380
   tttttagtag agatggggtt tcaccatatt ggccaggctg gtctcagact cttgacctca 160440
   ggtgatccac ccaacttggc ctcctaaagt gctgggatta caggtgtgag ccaccacacc 160500
   cagcctcatt tatgtccatt ttaaatgaaa ttataattaa tctatagttc tagtaactta 160560
   tatgtttctt taatttctct ttcttatatt gacattaata atacccttga tttttattag 160620
   aacatgccta ctttatcttt ttttaaaata accttattta caactgacct atttaattta 160680
   ggcagcatat aactaattat ttttggtttg gttttatttg atataatctg ataatctttt 160740
   taactcattg agttgagttt aactcatttt tattttaatt ataattatag gtatattttg 160800
   ggtcagaagt tagaaagtat ttctctcttg tgataattag aatttgagca gaacttccat 160860
   gttgatataa tatttcttga tcagagtggt aataaaattt atcgagatgc acatttaata 160920
   ttatgcacgt tttgaaacat acataatatg tatgccaata aaatattcaa cattttcata 160980
   aattaaatgc cttaatgaga taaataatcc aagaactgaa gaaaccactc taacatacat 161040
   agataatgtg ttggatgtga tattataatt ctaaataaaa taatatcctc cccacattat 161100
   aagcaaaaaa aaaaaagata aatttaaaag gaaaatatga aagaggtcac atcaaaagta 161160
   ttagaagaaa taaagaagaa tgcatatgtg gcatcaaggt aaataatata ttatttagga 161220
   agacagcaaa agaaaaatca ctaaccagcc gagtgtggtg gctcacgcct gtaatcccag 161280
   cactttggga ggccgaggtg ggtggatcac ctgaggtctg gagttcgaga ccagcctggc 161340
   caacatggtg aaaccccatc tctactaaaa atacataaag tagccgggtg tggtggcggg 161400
   cgcctgtaat cccagctact tgggaggctg aggcaggaga atcacttgaa cccgggaggc 161460
   agaggttgca gtgggctgag attgcgccat tgcactccag cctgggtgac atagtgagac 161520
   cctgtcaaaa aaaaaaaaaa aagaaaaatc actaaccata gaagaagtac agtgcaatct 161580
   aacttcattg aaatcacagg cttttttaac aacaacaaga tgccataaac atacttaaag 161640
   gaaataggat caattaggaa aatatattta ccatgtatat aataaggtta gaatttttga 161700
   tagaaatgta aattagtata aaatttgaag gacaaatttt aaatatctat taaaatagaa 161760
   aatgaacagc aaaaaatata tgtatgcatg tgcatgtatg tgtgcatata tatgtgtgtg 161820
   tgtgtgtttg tgtttgtgtg tatatatatt tatattcttt aaatatgctg tacacaggta 161880
   caatggaatg tttgttggag caatatttgt aaacctaaaa atacatatgg cctaaatagt 161940
   cttcaaaact gctaattact ataatgttga atacaattta aattcctata tatcaggtga 162000
   aaagaagaat gtgtctcaat agttacatag gaatagacat tacagtacat tttgaatgtg 162060
   gcgtaatatg tctatagtga tcttatttat ataaattaaa aataatcatg ctcaaaaagt 162120
   tatacagatg gtccctgact taagatggtt ccacctacag tttttcaact ttacaacgat 162180
   gcaagaggag gatctgtatt tgtaaatagg ttgtactaaa tttaaacgaa tgctatacag 162240
   ccatccctgg gaatctgcca ggtattggtt tcagagcacc cctaccctat accaaaatct 162300
   atggatgctc aagtttctta tataaaatgg tgcagtattt gcatataacc tatgcacatc 162360
   ctcctgtata ctttattttt tgtagagaaa aggtcttgct atgtcaccca ggttggtctt 162420
   gaactcctgg cctcaagtga ccctcctgct ttggtttccc aaagtgataa gattacaggc 162480
   atgagccacc attccccacc ttgtacactt taaatcatct ctagattatt tgtaatacct 162540
   aatacaatgt gaatgcaatg ttgctacact gtatttttaa aatttgtatt tttaattgtt 162600
   gtaatgctat tttttattat tttttccgaa actttcaatc tgctgttggt tgactttggg 162660
   aatgtggaac ccattgatac agaaggccaa ctgtgtgtat atagatatat atacacacac 162720
   acatacatat atatatatgt gtttctcaga aatgactctg tgtgtgtgtg tgtgtgtgtg 162780
   tgtgtgtgtg taaaaagcta tgaaatgcag tcataatgca ggttgcttct gacagggctg 162840
   aacagtggga aaggaacaag ggagaagaat gaaacaggaa tgacacagag tacgatgcta 162900
   cataaaaact taaattagct tttttttttc ctaatttgaa aatatgaagc aaatatttaa 162960
   gaattttgaa tgtctatact tcttgaaggc atgttttaac atgtgataat gtagttttta 163020
   cttataattt aattcaataa ttatcagagg gatttttttc tgaaataaat agggaaattt 163080
   aggtagctgt gagcattgaa gaggttattt ggagagaaga tatgaaaaaa tcaactcctg 163140
   gacctgggta aggaaagata acttgtgctg aaacaaaagt acagagagcg cagatacaca 163200
   ccccccaaga tttcagctta atcttactta tatcatcatc gagttgggag aaggagaaga 163260
   aaaagagggg aggcctgtga caataattct tttattcata tatttactca tcaaaaaata 163320
   cttattgaga aagtgctaga taccctgtcc taggtattat caatttactc atgaaagctt 163380
   cattctgtct caaggaattg tgatgattcc tgtaataaca gcaagtgcaa agtgctacca 163440
   aatgatagaa gaaatagtct atttgtggat attgaagaag accgcacatt ccagagggct 163500
   gttgaaaagc acagatggaa agagtggaaa aaaactgcag actaccccat acaaagacac 163560
   acagtcatta aaggacaggg acatttggtc agtgaaacca tcttgttctg ctggaacata 163620
   gacagtcctt gtgaaaacca agaaccagca gaattgaggc tgaaaatcag gtgaggtctt 163680
   gttgccaact aacccttcat actgttccca gttttatggg tgatgggaag ccactggtgc 163740
   atttccaaac tggaaatgac acaaggaatt gctgttttcg aaagagaatc caatcagctg 163800
   tgcaaaggaa gttctcagga gagaccagat atagaaagac aaatctgact aatacatttt 163860
   tttttcttaa aaaagagact agaaaggtaa aggtaagtgg atggatagga agagagaatt 163920
   tatgaacaaa agtagtatgg tatggtgaga tttgatggct atttggttat gaggaggcag 163980
   ggagaataaa caagtgataa tgactacatt ttttagtttt ggaaaagcac ttgtgagtca 164040
   ttcagggatg ttcagttggc agatggaaat agaaaaacca attatgagtc ttctgctcat 164100
   acattttagg aagtctgcat tgaagctagt tctcctttta ctcagtgatc ccatttgagc 164160
   taataagctt cataaccact actcacttca taaccactac tcacttcatg tcatggctct 164220
   ggcctgtgaa agggaggttt acctaatgaa gtaaaagtta ttcttctaaa gtatagtcac 164280
   tgttgataca tttctcagga acattctttt tcttttgaca gcatgctatg aaataaaaat 164340
   tgcgtgcaga cctggaggaa tattaacata tgaagacatt gggggaaggt tattgattga 164400
   tttactgaat ggtctgattc aaatgaactc ctaatatata tagaccatct cagaattaat 164460
   caccttctct actcttagag tgttccttat cattcaggta gcaatcattt gaacacccat 164520
   catttttaaa aaatgaaaaa gaaaactgta actaccttga caactggaag catgatttta 164580
   agtagctgaa ggagttactg gcttaagtta gaaacaaacc agattggaaa ctaggtataa 164640
   agtaattaat ttcttccatt ttgagaagtt ggaaggtctc actgagcttt tgttggttac 164700
   ttattcatat tgagaaattg attatttctt ttgcctaaag tcaaagagct ctgaagagac 164760
   ctagtcaaga tttggatgtt ttgaatacag tcacaattat ggtctcagaa atactttaaa 164820
   acacattcat cagtcatgct cttattcaga atcagggcac aaattttaaa caataaaatc 164880
   catgtgaact taaaaatatg ccttcgagtt attgaacatc taattaaatt gcttatttct 164940
   cattatgcct tctctaacaa taaatgattt tcattcctga atcccaatgg gattttcctg 165000
   gtccacttgt aagttgttta tttagataca caatgtgaat ctatatgttg atttattttg 165060
   taatggttca caggactggg ctactttcct ctgagtgagg gtaaaattaa tcagtaggaa 165120
   atgaatattg tcagtgtcaa tgattataag aggaaattat atgtgggagc aagagagaat 165180
   gaagcagatt atttgtggtt aggagttaat tcagagggat ccatgcataa tctccatctc 165240
   cgccaatttg gctgttttac tcctgttgcc atcatgccag catgggatgc tcaatatcag 165300
   aggctggctg acatcaactg gatgaaacca tttgttgatt gatggtttat tgtttcttcc 165360
   atggtgaatg attgctactg ggaaataata tgggatacaa agatcttcaa acattatgct 165420
   cacttccata tatctctctc cattcagatc tccttttccc cagtctctaa tctttctccc 165480
   tgcaggcacc tgtcccattg tccagtccgt tggtcatggc tcatgaattc acacataatt 165540
   ctaatcccag actatttctc ttcccacaca caatagatga ccagttttac tgtttgaagc 165600
   tctgcccatt gggagcaatt ttctttacca ctctatttta aggtcactcc tgagaaagga 165660
   ggtaatgcaa ctacagacta tttttacctt gcatccacat atctaagcaa attatccatg 165720
   agctaagcta ggggtatttc ctcctccttc atttggttgt ggatctctca tagggccact 165780
   ggagtgatct caagagagtt ggcactggtg cagcagtgag gagtgtcaca gggctctgag 165840
   atgcttgctc atgcagcttg cttgtgccct cttgtcttgc ttatgcttag ccattgatgt 165900
   gactacatac attcatctga caccatgtgc catataagcg atgcttaatc ttaggactca 165960
   atctggacag aacccactca tggcagggct gcttacatca tagtctagat ctctgaagag 166020
   cccttttctg ttctgggtca ccctcaaatc tgccagcctt ctgcacgatt aaattatagg 166080
   ccatagcaat attctcgagt gtagaaaacg ctgtttccag atcctgaaga agtctaccag 166140
   gtagaatgat tccttctctg tggtggtagg tgagagatgc aataatttgc tttttacatt 166200
   ggctaagatg tcccatcata tacctaacct atgtgtccta aaaattataa tgagatgaga 166260
   ggcccttgaa tcttagcagg attcatctcc ttcactcaaa agtgcacaca tttactaagg 166320
   tttctgacat gtcattgaaa ttgcctttca aaaattgtaa cagttagata aatctaacat 166380
   agctgactct gtcttgtttc taacctcgca agcaaactgc ctttgcccat ccgtacatgt 166440
   aggacaagct aactatggga agaatttagt ttatagttta aagcaagaat gatagtagtc 166500
   tcttcccaaa actaatctcc cacttattca ggtactgaag tcgctccttg taaagctaat 166560
   aaaaggccat aattttagaa ttacagtaag atcctgaatt ctgctaatgc taggcatagt 166620
   taaataataa ctagtcattg tctcctaata gatttactgc tcaggagtca tgtagccaga 166680
   ggtcacaaaa tttgtaactt ccctaattgc cccgagagac agcattgcta ttgcaaaacc 166740
   tgactggtgt ttgcaatatt ttttagagtt tgcattcttt gggccaacag atgccaacca 166800
   ggctcatgac ccatatcaaa aaactgactc aactgctcct gtgacaaccc agaaactgac 166860
   tcagtgcacg aaggcaattt tgatgcccgt atgatttcat ccccaaccaa gcagcaaatc 166920
   aaattccata gccccttgcc caccaaactg tccttaaaaa aaacctcacc tccaagttct 166980
   tagggaggca gatttgagaa ttatctccca ttctcctcac ttggctggcc ctgtgattat 167040
   ggaactctgg gctgcaacac ctgctatgct caatgtattg gctgttttgg aaacagtggg 167100
   caagaagaac ctgtcaggct gttatgttcc cagacagaac tggggtccag ctgcttgttc 167160
   tcatggttca atcataagat gcaaacagac tgggcaagaa gggagtttct gcaaccagtt 167220
   acagggagaa aaccagaata actcaacaga ccaactcaaa gttacaagtt tttctccagt 167280
   gcttataata cattcaaacc atatgcctac gtgtgggaaa gcacctacaa actgaagtgt 167340
   ttcactctgt ctaatcttta actagggttc agatctggaa attacttcag agcctaggaa 167400
   caattatgta atctaaagtg ggtcttggta caaggtgtgc aagaatgcct ccttttatta 167460
   attaaactct aatgtctgag aaagcccagg tggggtctta atggacttgt ttttacattc 167520
   taacccttgt actaaggcac cagtttctcc agttctttaa tgcttaagtt atacactcat 167580
   cagatttaca ctaaggtggt ggtgaaggat gactgccctg gttgctaatg gagacctggc 167640
   ctgccacaat tacactagtt atttcttact cattcctcag ttagcatcat atcattgatg 167700
   gagtggatca tgtgatgttg agtgagatgc atagataatc tagatttcct tggactatat 167760
   tttaacagag gttgggggca gttaacataa ttgtgggaca aaacttgaaa catttgctgt 167820
   cgttcctgcc ttgtgaatgt gaattttttc agaactgctt tttctgctaa gaatagaaaa 167880
   gagtgtattc aataaatcaa tggccaatta taggtacctt agaccaagct aatgtgcttt 167940
   tgcaaggatt ccatatttgg catggtggct gcaattgtgg ctactattac tttgtacttt 168000
   gtagtggcag cttaatatct tccgttggga tctgtttggt tactgcagga atcaaactgg 168060
   taaatcatgg tgtttgttgg gaccaccagc ctatgtcctt tagatcctga aggtgcttct 168120
   attaatactt tctgccatcc atcccctgag agtcaccagc tgcccaatgc aatattgttt 168180
   tagaaatacc atctagataa tgtaactctg tgtcccatca tgcaaggctt ggtaatatat 168240
   ttacttttgt gtttactttt ctttctaatc cattatctat ttctcagcca atatgagctt 168300
   tttaaaacac aaatgagatt atttaatttg tctgctaaaa atccctcaat ggctttctac 168360
   tgcccttcca ataaaatcag aatctctgat taaaaactaa caattcttat gatttttgac 168420
   cctgactaga tttttggcct taaaatacat gatcgtccac ctctagcaca agcctccagt 168480
   cataggattt tttgcatctt aaacacagca cctttcccat cagggcattt ggacttgcct 168540
   ttccctccgc taagagtgct tttcccagca cactgaagga gggcttgcac ttcatctttt 168600
   tattttcaac tcaaataata catcttcatg aggtcttacc tgaccacata gtccctgaaa 168660
   ggttcaccga aaagtcactg gcatgaggca cattgattaa taggagaaga gacataaaca 168720
   tttatttaac ttctatacgt gggagcctta agaatgaaga ccagcttccc aatgagttac 168780
   agaaatgtat ataccatctt gaggccacag taaagaatac agactcagaa catggccaga 168840
   aactggttat attggtaaat caggcttagt gaaaagacag gttatgagag agagaaagaa 168900
   gcttgactag caaatgtgac cttgttatgt agatgaagct tcccttagaa tagagggtaa 168960
   atgtttctct tcatacttta aaggtgtcag actctccagt ttcttcagga tctggaaaag 169020
   cacaggaaag ggaggcagcc tggctgcatt gaaggaaatt ctctacagat ccaaattttc 169080
   cccacaaaag acagctttgc agggccactt ttgtttgcta gccaagtggc agccatttca 169140
   aaatatgtca aaaaatacat tttgagggta agatcattaa atttccttta ccaccatgat 169200
   taaggtagca gctccattta tatttccttc agctagtttt tctgcgtatc cacttgcctg 169260
   tttgtgtgtc cacactgact agaatatgag ggcagcagca ggaacaacag caggacctag 169320
   gagtggttgt gacttctttt gatctcaagg aggttcttct aaatgttgat tctactggaa 169380
   tatgacatac cagtagacag atggacagac atctaatctg catagatggg atttggagtt 169440
   attgtaatct caaattagga agtcagagag gttctttata tcctaatggg tgaagctagg 169500
   tatacaagtt tcatagatat ttttgtacgt atcaactttg gtttaaaatt aatctagtac 169560
   ttctattttt aaataatgat aaatgtatga catatagaca tgcacttgcc acccacaatt 169620
   aatgaatgtt atattttttc caaacttttt aatgaagccc tttattccat atatatgaat 169680
   aaagggcttc attaaaatat atatatataa aattttatat atataatata tatatttttt 169740
   aaagaaaata attccaggta tagttagagc aaatctccct catgtcttac aactaatttt 169800
   ctacttcccc acgcataatg gatctcacag cattttgttg cattcctata cacattcaaa 169860
   tttttaaaaa atatatatat atccataatt tcttgtattg ttttgtttaa taatttatat 169920
   aaatgcagtt acatcaaata tattatgctc caacttgctc atcttaaata atagctttat 169980
   attctgagag aaatccacgc tgagcatgtg actctattta aatcttacct gttgaaaggg 170040
   cccttatgcg cagagacaga ctgtccccct gtaggtccta agaggagttc agtaactgcc 170100
   ctgatctctg aactcttggc aggtctccca gactcccagt tcttgtcagg acgccctcct 170160
   cccaattgtt ctgccctgaa cggattgtgt tttctccact atctgccctg caaccaagca 170220
   agcgttatct ccccattttt atcatcacag aaaggccagc cttccaggac atctctaatc 170280
   cttcaattct aaccccacat gcattcaata catcaacgtt tctcaatatt taatgtgcat 170340
   attaactact tatttttttt ttttttgagt ctcactctgt cacccaggct ggagtacagt 170400
   ggtgtgatct tggctcactg caacctccgc ctcccgggtt caagttattc tcctgcctca 170460
   acctcccgag tagctgggat cacaggcgtg tgccaccatg cccggctgat ttttgtattt 170520
   ttagtagaga cggggtttca ccatgttggc caggctggtc tctaactcct gagctcaggt 170580
   gatctgcccg cctcggcctt ccaaacttct gggattacag gcataagcca ccacacctgg 170640
   ccttgaacta ctttttgtca catgaccttc tccctcttca agtccagcaa cagagaatgc 170700
   tcctcacttt agtttcctct tgtgcttaaa gtctctgtca acaggacaaa cttgtccata 170760
   ttgacgactc acctgattgc gtcagggtcg ctagaggagg atttcattat cttaagatta 170820
   acttgggagg ctgaggcagg agaatcgctt gaacccggga ggtggaggtt gcagtgaact 170880
   gagatcgggc cactgcactt tagcctggga gacagaggga gactctgtct caagaaaaaa 170940
   aaaaaaaaag aataactcgg tgagaggtgg ccaagatggc ttattagaag cagctagtgt 171000
   gtgtggctct cacagagagg acgggaaagg gcgagtaaat acagcacttc aactgaaaca 171060
   tcaatctact cgcattggga ataatcaagg gaacaactca acccacagag aatggagaag 171120
   agcaaggcag gacactggac cacctgggag tgacacagag caagaagaac ctcccctgcc 171180
   cagggaagca gtgagtggat gtgtgaccct gggaagccat gcttttctgg tgagtctttg 171240
   cagccctcca gtcaggagat ccccttgtga acccactcca ccagggcctt cagtgtgaca 171300
   cacagagctg ccgtggagtc tcggcaaagc agccactcag gcatatgcaa acagcaggaa 171360
   gccttagatg ctcaggcttt ctgggcttct gggcaaaagt agctgcaact ctagcaaagc 171420
   aggagggtag atccctgtac atatccctag gaaaggggct gaatccagaa ggcctagcag 171480
   tgacagtctg catgttccac ttccacggga ccttgcagga taagacccat tggattggaa 171540
   ttccaaccag tcaccagtag cagcgttaca cctccctgag acagagcttc caagaggagg 171600
   ggcaggccac catttttgct atgtgggtga cttagtcatt ccaggcttca agctttggag 171660
   agttcaagcc aactaggggt ggaaaggatc ccacagcaca gcacagctgc tcttaaaagt 171720
   gtggccacat tgcttgttta agcaagtcca agatcccgtt actcctcaca gggtgggacc 171780
   ttgcaactgg ggtctccagc caccttctac aggtgtattt gggctggcaa cgggcttgta 171840
   acttcctggg aaggagcttc cagagtgagg ggcaggccac catctttgcc attttgcagc 171900
   cttcactggt ggtaatctcc aggtaatgga aaatccaagg caactaggga ttggagctga 171960
   cctgcagcat actacagcaa ccctataaaa aagatgtcag actgttacgt gggtgcccat 172020
   tcccatatct tctcaccagg tgcatcctcc aggcctgagc ctccagccac cctctgccag 172080
   agctatcaac tccctaggac agtgccatca ggagcaagtg aaatcctctc tgtcactgcc 172140
   tctgcagtcg aactgccttt cctagtctca gactgatgaa ggattgaaga ccataagtgc 172200
   cttatgcaca cctccaacaa ggtgcagtca acccaaggag aggaggccag ttcatcaccc 172260
   acgggtcccc cacatacccc actgctcatt accagactgg gaacccctgg cttgggccca 172320
   cagcacaggc cctccatccg gggctgattg cactgagtga ttgctgacct taaatctctc 172380
   tggggtggag ttcccaggag aaaagcaaaa gactcgctgc cataaccagt actaagttct 172440
   cttcctttac tgactctaag ttgggaagga acataaacac tgagattgcc acagagtggc 172500
   agtggacagc ccaggcatgc caagctggta tctacagcag cactcaaggg ggagaggaac 172560
   ccatactttc aggacattga gaggcaacat ggctacaact gaaggaaaca gagcaagagt 172620
   ctatcagctg gcaagtatgc ctgagtgaca cctactaaat cacaccccaa agcttcaaca 172680
   ccaaaaatac cttgctaaca cgcccccctc tgaaaccaga gacaaggagt cagcttcaaa 172740
   taaagacccc gcacaaagcc tttacccagt gagaatatcc ggcaaagtct attgactgta 172800
   ctcaatctat actgcagtta aaggaacaca cacatgcaga gatgagaaag aaccaatgca 172860
   agaactctgg taacccaaat ggccagtatt ctatgtcctc caaatgacca caccatttct 172920
   ccaacaagag ttcttaacta ggctgaactg gttgaaatga cagaaataga attcagaata 172980
   tggataggaa caaagctcaa tgaaattcag gaggatggca gaagccaatg taaggaaact 173040
   aagaatcaca ataaagtgat gcaggagcta aaggatgaaa tagcgggtgg aaaaacaaac 173100
   aaacacaaca cccaatgggt ctgtcagagc tgaataactc aatacaagaa tttcacaatg 173160
   caatcaagta ttaacagcag aataaaccaa gctgaggaaa gaatctcaga acttgaagac 173220
   ttgctctctg aaataagaca gacaaaaata aagaaaaagt ttataaaatg tgataaacaa 173280
   aacctttcag aagtatggca ttatgtaaag aggccaagtc tatgagtcat tggcatactt 173340
   aaaagggatg gggagaaagt aaacaacttg gaaaacatat ttcagggtat catccacgaa 173400
   aactttccct atcttgccag agtccaacag tcaaattcag gaaatacaga gaatgcctgt 173460
   aagattctac atgagaaggt catcccaagg tacataattg tcagattttc caaggtcaaa 173520
   atgaaagaaa gaatgttaaa ggaaactaga gagaaaggtc agtttaccta aaaagagaaa 173580
   cccatcagcc taatagtaga tctataagct gaaactttgt aagccaaaag agattgggaa 173640
   cctatgttaa acattctaaa aaaaaaaaaa aaaaaaaaat tcaaccagaa attttatatc 173700
   cagctaaatt aagctttcta cgtgaaaaag aaataaggtc tttttcagat aagcaaatat 173760
   tgaggaagtt cattacctcc agacctgcct taaaagagat cttgaaaggc ggaaaggagc 173820
   actaaatata gaaaggaaag accactagca gctaacacaa aaccactgaa acacacagat 173880
   cagtgtcact ataaagcaac cacacaaaca aaccaataca ataaccagct aacaacataa 173940
   tgacacaatt aaacccacac acatcagtac taaccttgaa tttaaacaag ctaaattcct 174000
   tcacttaaaa ggcacagagt gacaagctgt ataaaaaagc aagacccaat ggtatactgt 174060
   cttcaagaga cccgtctcac atgcaatgac acccataggc tcaaaataaa gggatggaga 174120
   aaaatctacc aagcaactgg aaaacagaaa aaaaagcagc agtgagaatc catatttcag 174180
   acagaacaga tacagatttc aaaccaaaaa aaagcgggca ttacataatg gtaaagggtt 174240
   caaatcaaga agaaaaccta actatcctaa atatacatac acccaacaca ggaacaccaa 174300
   gattcataat gaagcaagtt cttagagatg tacaaagaga cataaacctc catacaacaa 174360
   tagtgggaga ctttgacact ccactgacag tactaaatca tcaacataga aaattaacaa 174420
   gatattcagg gcctaaactt agcactggat caaatggatc tgattgacat ctacagaact 174480
   gcaccctaaa acaacagaat attcattctt ctcattgcca catggcacat actctaaaat 174540
   tgaccacaca aacgaacaca aaacaatcct cagcaaatgc aaaataatca aaatcatacc 174600
   aagcacattc ttggaccaca gcacaataaa aatagaagtc aatactaaga aatctctcaa 174660
   aaccatgcaa ttacacgcaa attaaacaac attctcctga atgactttgc gtaaatattg 174720
   aaatcaaggc agaaatcaag aaattctttg aaactaatag aacaagggta caacatgcta 174780
   gaatttcggg gacacagtga aggtggtgtt aagagggaaa tttatagcac taagttccca 174840
   catcaaaaag ttagaaaaat ctcagattaa caacctaaca ccataactga aagaattaca 174900
   gaagcaagag caaatcatgc tagcagaaga caagaaataa ccaaaatcag aactgaactg 174960
   aagaaaatca agacatgaaa aaccactcaa aagatcaaca aattcaggag ttagtttttg 175020
   gaaaaaatta ataagataga taggctgcta gctagaagaa aaaaagaaga aaagagagaa 175080
   gatgcaaaaa aaaatacaat tagaaataat gaagagaata ttaccactga tctcacagaa 175140
   ataaaaataa ccatcagaaa ctactacgtg cacttctatg cacacaaact agaaaaccta 175200
   gaggagatgg atgaatttct ggagacacac catcacaaga ctgaaccaga agaaattgat 175260
   tcagagaaca gaccaataat gaactccaaa aataaatgag taataaatag cctaccaacc 175320
   aaaaaaagaa aagtccagta tctgatggat tcatattcat agccaaattc taccagatat 175380
   acaaagaaga gctgtatcat ttgcacagaa actattccaa aaatttgaag aggagggatt 175440
   cctccctaac taattctatg aagccgatat cattctgata caaaacctgg cagagacaca 175500
   agaaaaaaag aaaacttcag gccaatatgt tcaatgaaca ttgatgcaaa aattctcaac 175560
   aaaagacctg caaaccaaat ccagcagcac atccaatagc taatccacta cgatcaagta 175620
   ggcttcatca ccagaatgca aagttggttc aacatacaaa aatcaattaa tttgattcat 175680
   catataaaca gaactaaaga caaaaaccac atgatatctc aatagatgca aaaaaggctt 175740
   ttgataaaat tcaaaacttt ttcatattaa aaaccctcaa taaactagga attgaaggtg 175800
   cattagtcag ggttctctag aggtacggaa ctaataggag aggagtatat atgaagggga 175860
   gtttattaaa gagcattgac ttacactatc acaaggtgaa ggcctacaat aggctgtctg 175920
   caagctgcaa gcaaggaagc cagtctgagt accaaatgta aaaggtaggg aagtcaacag 175980
   tgctgccttc agtctgtgac ttaaggcctg agagcctctg gcaaatcact ggtgtaagtc 176040
   catgggtcca aaagctgaag aacttggagt ttgatgttca acagcaggaa gcatccagca 176100
   caggaccaag atgaaagcca ggagactcag caagtctagt cctttcacat tctttcacct 176160
   gctttttttt ctagccatgc tggcatctca ttagatgata cccacccaaa ttgagggtag 176220
   gtcttcctct accagtcccc tgactcaaaa tgttaatctc ctttgacaac gccttcacag 176280
   acacacccag gaacaatact ttgaatcttt taatccaatc aagttgacat tcaatattaa 176340
   ccatcacaga atgatatacc tcaaaatgat aagagccata tatgacaaac ccacagtcaa 176400
   cattataata gattggcaaa tcctggaagc attccatcga aaaccagcac aagacaagta 176460
   tgccttctct cactactcct attcaacata ttattgtaaa ttctagccag agcaatcagg 176520
   caagagaaag aaataaagaa cattcaaata ggaagagaag aagtcaaact atctggttgc 176580
   agatgacata attctatatc tagaaaacca catagtcttg gcccaaaagc tccttcagct 176640
   gataaacaat ctcagcaaag tttcaagaag caaaatcaat ggacaaaaaa tcactagcat 176700
   tcctatacac caacaacagc caagccaaga accaaattag aaaggcgatc tcattcacaa 176760
   atgccaccga aagaataaaa tatctaggaa tagagctatc caagaagata aaatatctct 176820
   acaataaaaa ttataaaaca ctgcccaaat aaatcagaga agatataaac aaatagaaac 176880
   acatcccatg ctcatggata tgaagtaata atatcattaa aatggccata ctgccaaaag 176940
   caatttacag attaaatgat attcctatca aactactgat gaaattcttc acagacttag 177000
   aaaaaactat tttaaaatcc acagatccta aaaagagctc aaatagccaa gcaatcccga 177060
   gcaaaaagaa aaaagctgga ggcataatgt tacccaaatt caatctatgc tataggggta 177120
   cagtagccaa aacagcatga tactgctgtt acagaaacag gcacatagac taagggaaca 177180
   gaatacagag cccccaaatt aaggccacac acctgtgacc atctgatctt tgacagagat 177240
   gacaaaaaat aaggattggg gaaaagactc cctatttaat aaatggtgct gggataactg 177300
   gctagccata tacagaagat tgaagctgaa ccccttcctt aaaccatatg caaaaatcga 177360
   ctcaaggtgg attaaagacc tcaatgtaaa actccaaact gtaaaaatcc taaaagacaa 177420
   cctagccaac accatcatgg acacagaaat gggcaaagat ttcatgacaa ggacaccaaa 177480
   agcaattgca acaaaagcag aaattgacaa atgagatcga attaagctta agaacttctg 177540
   cacagcaaaa gaaactatca acaatataaa cagatagtct acagaatggg aaaaatattt 177600
   gcaaactatg catctgacaa aggtctaata cccagaatct ataatgtaca taaacaaatt 177660
   tacaaaagaa aaaaacacat taaaaagtga gcaaaggaca tgaacagaca cttttcaaaa 177720
   gaagacatac atgcagccag caaacaggaa aatttcagta ttactgattg ttagagaaat 177780
   gcaaatcaaa accacaatga gatgccatct cacaccattc agaatggtga ttattaaaaa 177840
   gtcaaaaaca agagattctg gcgaggttgc agagaaaaag gcacactttt acactgttgg 177900
   tggaagtata aattagttca acagttgcgg aaagcagtgt ggtaattcct aaaataccta 177960
   aaaacagaaa tacttttcga cccagcaatc ccattacagg gtatataccc aaaggaacat 178020
   aagtttttct atgttcctac ccatatatga ctgtgggttt gtcatatatg gctcttatca 178080
   ttttgaggta tatcattctg tgatggttaa tattgaatgt caaattgatt ggattaaaag 178140
   attcaaagta ttgttcctgg gtgtgtctgt gaaggcgttg tcaaaggaga ttaacatttt 178200
   gagtcagggg actggtagag gaaacatgca cacatatgtt tattgcaaca ctattcacaa 178260
   tagcaaggac atggaatcaa cctaaatgcc catcaatggt agagtgaatt ttaaaaatgc 178320
   ggtgcatgtg catgatggaa tactatgcag acataaaaaa caataagatt atgtctttta 178380
   cattgttgat ggaagtgtaa attagttcga ttgtggaaag cagcatggat tcctaaaaga 178440
   gctaaagtac agaacaacca tttgacccag caatcccatt actggaaata tactcagaag 178500
   catataaatt attctattgt aaagaacacg cgtgcaaatg ttcattgcag cactattcag 178560
   gagagcaaag atatgaaatc aacctatgcc catcaatgac agattggata aagaaaatgt 178620
   ggtacatata cactatagaa cagtagacag ccataaaaaa caagatcatg tcttttgcag 178680
   gaacatggaa ggagctgaag gcctattctt agcaatctaa tgcaagaaca gaaaaccaaa 178740
   taccacatta tcacttacaa gtgggagcta aattataaaa tcctatgaac ccaaaaatga 178800
   aacaacagac agtagggtct tcttgagggt ggcggctagg aggctagaga ggagcagcaa 178860
   aggtaagtat tgggtactgg gcttaatacc tggatgatta aataatctgt aaaacaaatg 178920
   cctgtaacat gagtttacct ccataacaaa gcttcacatg taccccgaac caaaaataaa 178980
   agtttttaaa aaagaataac tcatttgtgg tctaattata tcttcaaaat cccttcacaa 179040
   caacacctag attagtgttt gactgaataa ctgggagaag gtgtgtgtat agcaagtgct 179100
   gggaattacg gggtctatct tagaattttg ctagcacacc ttctgagtca aaggtgggtt 179160
   cccattagag tagaaatcag cactgcttct gaaatttccc cgaattgaaa ctatgggcga 179220
   actatacaag aaagatcttg gaggacttac ttttacccta tcgttcacta aatgtttatt 179280
   gtccacatta tatttgctga gcacgattct attcatgaaa gacatggtgc tgaataaggt 179340
   gatcacattc aagaaattca gagagtttag tctttagtga ataagaggag tgtaggaata 179400
   tagagtctaa tagagtacat gttttaaagt tacttacaaa tttatctcaa ttatatatat 179460
   atgtgtgtat acatataatt gagtatatat atatatatat atatatatat atatatatat 179520
   atatactcag tgataagtgc tatgaaggga aacctgcgta cagaaaatga tgatgtagtg 179580
   ggaagtattt taaatgagct attgacagaa ggcctctctg aggaagtaat atcagagcct 179640
   aaacctttaa gaagtgaagg agtaaaccag aaattgcttc cagtacctct gtaaaagccc 179700
   aaagctgtaa taagctcagg cttcataaag ataacaagga ggctgatgca gatacaatta 179760
   aaatgagtga aagatgagta gtgtaaaatg aggtaggaag aaggagacta aagccagata 179820
   atgtaaaaac ctgctactaa tagacgtcag tcattgtcca agcaaaataa aggtattttc 179880
   aacattcaaa aataacaaga gatcatggtc gctaacaaag aggctgatgc agacagaatt 179940
   aaattgaatg aaagatgagt agcgtaaaat gaggtaggag aaggagatca tggcagatgg 180000
   gaggcaggat agatcgcagc tccaactcgg atggacagag caatgggcag aggcctacat 180060
   cgtgaatttt agctccgaga ttgattgcaa gaatgaacca gaaatccaga gaggacccac 180120
   agaccctctg aaggaagggg acttctcttg cagcacccag gagacaccca aatactgtga 180180
   gtgtcccagc tgcagaagtg gggaaatgag atcctcctct cccaaacaca cacccccact 180240
   ggggaaactg aaggtttgct tgttggagaa gtttctgacc ttaactggag ctgagtcaat 180300
   ctagagagtc cagtgaaata caggggaaaa agaagcagca ggaaaggccc tgggagctca 180360
   ctgggttccc aggcagtcca ttcctgcctg gcaccacagg gatccttggg agggtggcca 180420
   gaggggcaga ggaaaatgcc acaggaagaa ggaagtctct ggctaaattt tgaaacagtt 180480
   ctagctaggc aagaggcctc ttggccagaa ttccagggag ggtgtaaatc tggtgtgcag 180540
   actctatagg caggggaaga accaagacct tttctttcac agctgggagg tgggtggcct 180600
   ggggcaagtt ctcaagccct gctctccaac tgcctggaaa cagatttggg gctgttcaga 180660
   tgtgtatggt gggagtaaga ccgtcccttc aggttgcgtg ggagctgggt aaggcctgtg 180720
   actgctggct ttcccccact tccctgacaa cctgcatgac tcagcagaga cagccataat 180780
   cctcctaggt acataactct attaacctgg gaatatcacc cccatcctcc acagcagcca 180840
   cagcaagacc tgcccaagga gagtctgagc tcaggcacgc ctagccacac cctcagccgc 180900
   acccccgcct gatggtcctt ccccaaccac cctggtagag gaggacaaag ggcatagtct 180960
   tgggagttct agggccttgc ccactgctgg ttcctctcca tactaccaca gctgatgctc 181020
   cctggaaaga accacctcct ggtaggaggt caaccagcac aaaaatagag cattaaatca 181080
   ccaaagctaa gaaccctcac agagtccatt tcacccccct ggcacctcca gtggaacagg 181140
   tgctggtatc catggctaag agacccatag atggttcaca ttacagggct ctgtgcagac 181200
   aacccccagt accagttcgg agctgggtag acttgctgaa tggctagacc caaaagagag 181260
   ataacaatca ccggagctca gctcacagaa agtgacagta ataggaaaat ggggagagta 181320
   caacatcaag agaacaccct gtgggacaaa agaatctgaa caacagcctc cagccctaga 181380
   ccttccctct gacacaggct acccgaatga gaaggaacca gaaaactaac tctagtaata 181440
   tgacaaaaca aggcttttta acacccttca aaaattatac tagttcacca gcaatggatc 181500
   caaaccaaga agaaatccct gatttacctg aaaaagaatt caggaggata gtcattaggc 181560
   taatcaggga ggcaccagag aaaggctaag cccaatgcaa ggaaatccaa aaactgatac 181620
   aagaagtgaa gggagaaata ctgaaggaaa tagataacat aaagaaaaaa aatcaaaact 181680
   tcaggaaaca ttagacacat ttatggaatt gcaaaatgct ggaaagtctc aacaatagaa 181740
   ctgaacaagt agaagaaaga aattcagagc ttaaagacaa gatcttcaaa ttaacccaat 181800
   gcaacaaaga caaagagaaa agaataagaa aatatgaaca aagcctccaa gaagtctagg 181860
   attatgttaa atgaccaatc ttaagaataa ttgatcttcc tgaagaagaa gagaattcct 181920
   aaagcttgga aacacatttt aggggaataa ttgagtaaaa cttccccagc cttgctagag 181980
   acctagacat ccaaacacaa gaagcacaaa gaacgtgttg gaaattcatt gcaaaaagat 182040
   cattgcctag gcacattgtc atcagattat ctaaaggctt tacatgcata tggacaccaa 182100
   aagcaagcag gggtagctat tcttatatca gacaaaacaa actttaaagc aacagcagtt 182160
   aaagagacaa agagggacat tatacagtgg caaaaggcct tgtccaacag gaagatatca 182220
   caatcctaaa cacatatgca cctaactctg gagctcccaa atttataaaa caattactaa 182280
   cggacaagaa atgagataga cagcaacaca ataatagtgg ggtacttcaa tactccactg 182340
   acagcactag acatgtcatc aagacagaaa gtcaacaaag aaacaatgga tttaaactat 182400
   accttggaat aaatgaactt aacagataga tacagaatat ttcatccaac aaccacagac 182460
   tacacattct attcaacagc acatggaact ttctttaaga tagactattg ataggccata 182520
   aaacaagcct caatgaattt aagaaaattg aaattatttc aagcactctc tcagaccaca 182580
   gtggaataaa actgaaaatc aactctaaaa ggaaccttca aaaccatgca aatatgggga 182640
   aataaataac ctgctactga aagaacgttg ggtcaaaaac aaagtcacga tgaaaattaa 182700
   aaaaattctt caaactgaac aaaaatagtg acacagccaa tcaaaatctc tgggatacag 182760
   caagggtggt gctacgagga aagttcatag ccctaaacgc ctacatcaaa aagactgaaa 182820
   gagcacaaac tgacattcca aggtcacacc tcaaggaatt agagaaacaa gaacaaacca 182880
   aatcaaaact cagcagaaga aaggaaataa acaagatcag agcagaacta aatgaaattg 182940
   aaatagaaaa ccactactaa aaaaaaaaaa aaaaagaaaa gaaacaaaaa gctggttctt 183000
   ttaaaagatg aataaaattg gaaggacatt accaagatta accaagaaaa gaagagagac 183060
   aacccaaata acctcattaa gaaacaaaat gagagatatt acaagggagt gacaccactg 183120
   aaatactatg aacataaact agaagagatg agtaaattcc tagaaagaca caacccacct 183180
   agtttaaatc aggaacaatt agatatcctg aacagaccaa taacaagcat tgagattaga 183240
   atggtaatta aaaaattatg gtaaataaaa aattaccaac aaaagaagtc caggaccaga 183300
   aggattcaca gcagaattct agcagacatt caaagaaaaa ttggtaccaa tatttttgaa 183360
   actattccac aaggtagaga aagaaggaac cctcgctaat tcattccatg aagccaacat 183420
   taccccaata ccaaaaccag gaaatgacat aaccaaaaag gaaaactaca gaccgatatc 183480
   cttgatgaac gtagatgtta aaatccttaa ccaaatacta gctaaccaaa tccaacaaca 183540
   tatcaaaaag ataataggtt tcataggtaa aagccctatt tatatttagg gtaaagaggg 183600
   acacggagac tatgacatcc cttcccagtg taagagttgc agtaacaaag tgtttctcag 183660
   tggaggataa ttcttctcag cagaacattg tcagcttctg gagggccagt ggcatggcag 183720
   cagggctaag aagctggagt gaaagcacta gatactctac ttcctgagca gtggtagact 183780
   attccctcca gaatttcaga aaaaacaaaa aatagggaca ctttgaggag aaagctaatt   183840
   tctgataaca gcatcataga cttacactga cgtagttgag ttacaaatgt aaaatgattg 183900
   gcaaaatgtt tagcacacag gatgcaacat gtggtacttt ccttccctct cttttacagt 183960
   gaaattttca tctaaataag taagtaaata gattagtggt ctccactttg gctcctgtgt 184020
   tcagctacaa tttctattta gaataggtag acacttttat ctgattggtg taataacttg 184080
   gctaaagctg tggtgtgttg gtcatgcaga atagattatc caggatgccg aaggaatgga 184140
   gctttgtatt tggggctttg ctgactgcct gattattcta cagaccaatg aagagtctct 184200
   gcatccacag ctattacact gcctcctgaa gaggaggcca aatatcttat aaattacaag 184260
   attcaaaata ttattcaaaa taatcccact tgtcttcaaa tacacatttc ctccaaaaat 184320
   taaaacacac agaaagaaaa tgcaaaactc cacaaaattt aagaaacact ccaggccatt 184380
   atttcaactt cgccgtgaac aattcaggga aatgacaacc caggtggtat tttccatttg 184440
   actaacttat gttttagtac ttggcacttg tgtgtctact cccataacaa gcaactatgt 184500
   ggtgattaaa aattaaatgt tttgccttgt ttgaagagct gctgttcaga gtattgcatt 184560
   ggaaagcact tacctgaaag gagagcttat gttcggttag tggtggagag cagagtgatt 184620
   tccatttgct ctgtgcatgt cactggagag gttctaaact gtaaatgctc actgtccatt 184680
   tatggggttt atgtatgcaa tttctgcaga aatcgtcttt gtaaatggga atgaaaatta 184740
   tccttgcact tattccacag ccttttctca tctccagcaa gtgaaaatga gcctttgggt 184800
   ctcaaaatat ctcctgtgtg tttgattttg ttgggcagat ttgaaatgat cctccagctc 184860
   caaagttggt aaaaacagaa tggttttctt cttattaact gagcaactga tatccttaaa 184920
   aatgatgtct gtatatttct tattatatga cacaaattcc agggaaagac cacgggaatt 184980
   gtagtcaggc ataacaacct tttttctctc tctgctgttt actggctgga taatacaggg 185040
   taaattattt aaattctctg aacctaaatt ttctcatata taaagtgaaa ttgaaaatat 185100
   ttttcccaaa tttctgcttc cagaaaatgt ggtccactaa cctaaaactt gagagcatgg 185160
   tagtgtatat aaaacaaaat tctaagcctg acaaccaact gattggaccc tttctctcag 185220
   tcaagagcgt tccaaaatta acctgaaaaa tcagttaatg tcataatgtg aagggggaat 185280
   cagacacgcc ttatactctc catcctttgg aattcaagca cagctgacca gtatcagcat 185340
   taaaacagag atcttaagac taacaaaaca gactatttgt agcaataaga tacacaattc 185400
   caaccgaact ctagtatagc atcatacgac agatagcagg ccctgaaaga aatcaaagta 185460
   tttcacccca aaatatatct tactaagtct tttcaggaaa gtcaggcacc ttttaaggtc 185520
   tgataaaaag acatttacca tttaccatcc attgtctctg aagtcttctc cctagaggct 185580
   tcacctgtat aacaagagcc ttggtttcca caaccctcct tatcttaacc ctaagcattt 185640
   ctttctgatg acttcaactc ttcaaacaaa acttaactct ttcaaccaac tgccaattgg 185700
   gaaaactttg aatccaccta tgacttggaa gccaacctgc ctcctttaag atgtgtcacc 185760
   tttctaaacc aaaccaatgt acacattaca tgtattgatt gatgtctgcc tgtaacttct 185820
   gccccccatc ccttaatgta caaaatcaat ctgtgacctt acttccttga gcacatgttc 185880
   tcaggacctc ctgaggctat ggtgtgggca tttcctcaac tttagcaaaa taaactttca 185940
   aaattgatgg acacttgcct gagatacatt tttgtttaaa gcacaaagag gagatagaaa 186000
   aagagagatg agagtaatga atgtaaaatc tggaagaaaa tattgtggag acagaaaatg 186060
   attgagtccc attgagggga aagcatctgt cttgagcaaa aaagatcctg acagatggtc 186120
   agagaatatg ttgggagaca cttttcattg tgactttttt ctccccaaaa ttttccataa 186180
   taagccattt ctcatccctt gacccaacat ggttggtctc tggaacagga gcactgttca 186240
   catgctgctc aagaatggtg gtggtaacag cagagcagct cttgagactg gttggtggaa 186300
   aataagtatc aatgagggta tgcaggacag ctcatctcta aaaattactt tgcctgcata 186360
   aaacataact ccaagagcta gaggattact ctttggttct ggtttttatg gcagcttcat 186420
   tacgtagcca tgattgatta agtgattggc cactggtaat caacttaatt tccagcctct 186480
   ttctcctttc ctgaggttgg agggtggggc tgaaagtacc aactcactaa tcacacggtt 186540
   cccctggcca ccagcccctt catccttaga gattttccaa acgtcacctt attaacataa 186600
   actcaagtgt aatagaattg ggcttgttat gaataataaa agaccctcct ttaaacttta 186660
   taattttgaa gctatttcag gaatgggaga aaaaacaatt attctttgaa aaaaggtatt 186720
   cctcttattc ctataactca ggaatttgca agagttttac aagttctgtg atgggaacca 186780
   gggacaaaga ccaaatatct gtttcttata tcacaagttt tcaaaaacca attaatacat 186840
   tatttattca tttattcatg tatagataga tggttactgt caaaaataaa cactttgtgt 186900
   ttataggtaa tgcagcaatt acaatatata gggcacgaat gaagcttcac aggtgtcaag 186960
   actcgaaatc ttatagacta agttctttgg ccagaatgca atacaattgg aaatcagtga 187020
   taaaagagcc acttcaaatt tttattttag acaattaagt aagaaatcaa agagattttg 187080
   aaaagtatta gaatattatg agctgttata tgatactata tttgaaaact tagctgaaat 187140
   ggtaaattcc tatagaaaca tgaaatttca aaaatcgttg ataaagtaaa gagcataaaa 187200
   cattcagtat gtgttaaagt aatggaaatg gtcatcagaa gagcaacaaa taagttctac 187260
   aaactttcac agaatagcta tttattaact tacaaaagtt gttccaggac aaaaaaataa 187320
   agctttccca gctcatttta tgaagcaagg gtaatcttta ttccaatata cataaggttt 187380
   ctaaaaagcc attcttgagg gtgtgtggcc aatatgctgc ttgttcaaga ttaactgagg 187440
   gattagcaaa gaaagctcag aaatataatc atgtatttgt agcagatatg ctaagtgaca 187500
   gaactggaat gcccagttag tagggaatat gtaaaggata aaggccacta aatggagaaa 187560
   agttacgttc aatttctttc attcaatata taaatgttaa ctccagagtg cttaaaaacc 187620
   ttaaacacta tcaagctaaa gtaagaaaat gtagaatatc attgagccct ggcgtaagga 187680
   tgggggaaag agatagtata ccagattctc aaacaaaaga ctataaaaaa agagttaatg 187740
   gattgtatta cggaaatatt aaatatttca ttaaagaagg aaagtatagg tgatgttagc 187800
   aaacataggg cagattgaaa tgagggattt gcagcaacta aaaccaatta aaagccaatc 187860
   tgttaaatat ggaagaaatt ctacaaatcc ataagaagaa cacaaggaaa tacagagaac 187920
   aaacctaaat atctagaatt atatgaaaag atactcatcc tcaccaatag aaaactgcga 187980
   tattgcctta tacatattag gagatgcatc ttaggaaaaa atagagcttt gaattatacc 188040
   aagcattgtt atggatggag tggtgtgtgt ggcagtgacc agaatggatg caaactgata 188100
   aggttgatct ggagagcagc tgacagaagt tacattgcat atgtgtacac cctgttacca 188160
   acaagcccat tcctaggttt atattcccaa gaagttaatg aaaaatcatg tgtgaagctc 188220
   attcttttta tcactgtttg tgatagctac tggttgtagc tatctctaca tttatgtatc 188280
   tctaaataca taaaatatga ttgatgtatt cagtgtggca agcacttagg agcagtcaat 188340
   taggtgtacc tgtgaccaca tgaagattac ttaatgctac cacgttacac gcacatacaa 188400
   ataggctcca tggcacaatg tcatttacat atactaaaac acatacaaaa acccaacact 188460
   ctttttataa ggttatatac aaatccaaag acatgtaaca aacacattag attgggtgtc 188520
   tgtgttggaa catagaacag gattgggggt caggaaaagc aaaaagaaaa taaaagagaa 188580
   tgaggtcttc catgaactaa tgataattat gtgccataaa ctggaagtta tacttaatta 188640
   aactctatag ctgagaccaa atagaaagaa aaaagtgtgc agcagcaagg aaatgtttaa 188700
   gttaacaatg ctcaacatat ttgtgcatct ctgcagttta gaaaattgaa attttgaagg 188760
   gattggttaa taccatattc atttgctttt tgtttggtta ctttttatct cagttcctcc 188820
   ttaaggagaa gagcttaatc cttgctgtga atttcttttg tattatttgg gcaaaatctt 188880
   caagtgtctt aataaatata tgatctttct ttagtaccgg atgggtggga ggtttttttc 188940
   tctttctcat attcagctcc atgttctttc cagcagatgc tgctatcaga gcgtgatcct 189000
   cagttttcat aaaaatgcac acacttacaa caaaaattga ccacactttg gcagcttatg 189060
   tattaagtgt tcctaccaca ctgcgatgta tcagtgggca aacactgcat taaatgagct 189120
   atagcaaagc aatccagaag agaactatgt ggtcattaga atgatggaaa taactttaac 189180
   aagacggtga ctgcaacaat atgagtggaa acaatgccca aacaacaata ttccacgtat 189240
   agtatttata gtaaaaatac gtttgaaaga aaatatgaaa cacgttggta atgatttttc 189300
   actgtggtga gaagattggt cctggctgta acagaaacca tcagtaccat catccccaca 189360
   gccctccaac ccttccctgt gcttcgcagt ttccagctgc cagggtcggc atctttgtgc 189420
   ctgagggctt tggaaagcct ctctgctgat gtgcccatac accaaggaat tgacacctcc 189480
   tgggggcagc cctcaacaga gacccatggg agttcatgta taacaccaac agaaaaacaa 189540
   tgcaacataa aaaattgtgc tgtgacaaat taatgattcc catcagtgtg actgacagag 189600
   tttaaaaatg tttacaatat gactttctga cttcctcagg tggtgtcaat tcctaagaca 189660
   aacaccttgt aattcattac aaatgcaaat atgtcaaaca ttgttctttg aaaataaata 189720
   aataaaaatt gatatggtat taaaatactg ctgtttttcc tgtctgatac ataaagaaaa 189780
   tgtaatgtga atacttatag tttccaagag tatgtggaaa ttgacattca catatacttt 189840
   ggtacgtaag taaatttata gcaaaataat ttaaaatatg aacaactatt ggtatgttta 189900
   ttgtggcatt atttgtaata tcaaattaga aacaaagtaa atgtccaaca actaaggttt 189960
   taaaaaatgt gttacagttg cacaacaatg tacttaatgc cattgagctg tgcacttaaa 190020
   aatcgttaaa atggtaaatt ttgttaggta tattttatca caataaaaca tgtattatag 190080
   tgcacctaac aaagaaatga gttattaaag atgatatcgc aaagcatttt atcaatggag 190140
   aaatgtattt ctatgtgtgt gaatcatgct ataaaaatgt gcttaatatt tacattgtat 190200
   attatttatg ttatatatat gtaaatgcat atccatttat atttatagac attgaaaaag 190260
   gttacatttt aacatacact tcatgtaatt atagttcttc acacattttt cttttgctta 190320
   tttaattttt ttcaatattt ctctaataag ccaatcttac ttaagttaaa tgaaaatgac 190380
   cctgtgatct caacactttg ggaggccaag gtgggtgaat cacttgagtc caggagtttg 190440
   agaccagctt gggcaacatg gcgaaacccc acctctacga aaaataaaaa aaattagctg 190500
   agtgtggtgg tccaagtctg agctactgag gaggctgaag taggaggatt gcttgagcct 190560
   aggaggtaga ggttgaacaa tgagccaaga tgatggcact gcactccagc ctgagtgaaa 190620
   gagacaggcc ctggtttatt gatttttttt tttttttaag aaaatgacct tgatgtggaa 190680
   acatttggaa taacttcaca aatatgttct tgggtgtcta atccaaaccc gagaatcaat 190740
   aatgcttata aaatgggaga aatagggcga aaatttattt ttcttaaatt tttctttctc 190800
   atttctctga ttcaaacttt ttgacctact tttttttatg aataagaagc aattgattta   190860
   aaatacctta attttatctc atttcttaat tacaaggaaa acataggctg atttgtccat   190920
   ttttatttaa agtcattctc agaaggcttt ctgtgcttca catttaagtc actttttacc 190980
   tgataatatt atttatgact atgttttctt ctattgggtt gtaagtttta tgaaaactgc 191040
   attctggcta atttatttaa tgtatttccc acagtgtcca actaaatgtg tgtttgtaca 191100
   taatagattc ttagtatatt tgagggataa caaatggcag taaattaacc ttttcttttt 191160
   ctaccgtttt tttccatttt tttaaaaaaa catatggggc cttaatgggt tcctcccttg 191220
   tgtgtttctg ttcctttcac tcaagctccc tcctctcctc tattagaacc attttcaggt 191280
   atttttcctc tcttagggaa catgtaacat tcaatggtta agatttatgg aaatgtgccc 191340
   cccaatatgg ctacatgccc attttgtatg gtgtgaagac cataatttta taagctagcc 191400
   attccattat tggactgcca aagctattaa tatgttcttt ctaacacgtg agctgcattt 191460
   ttttccccca tgaatctagc cattgaatcc agctctgccc ctactgaaac ggggaaaggg 191520
   tatggtctct catctacatg gcactctatg gttgaagagc atgacctggg ttcctcagag 191580
   gctcctttac tgggctaggt ggccctgggt tctcacatag cccagttttc tggaccttgc 191640
   actgtgatgg ctgttcatga ccacactgtt ggttagccat ggtgctccca agacagggca 191700
   cagtgctcct gatttaacta gccgcaaaca gaagacaggg gggctttgcc attttagaga 191760
   ttataatcct atcataatca tctaagtgct ttggctttat ttcagcaagt gtggccattg 191820
   ctctgttatg cagatcttat gcctctttgt cctgtttctt gatgtccatt tatattctga 191880
   gtgtgtgttc tccagccctg caagcttgtg ctgctgactt cataggtaag aattccatat 191940
   tttgtttttt taacccattt gttttcaccc aggttatgct ttataaacat taattgataa 192000
   taaaacttga aagttaattt tctctctcta cgaatttccc ccaccctagc actcctgaat 192060
   cattgtggca tcagaacaga gctggcatgg tttctatggc acagaaaaca ctgtgggaaa 192120
   agggtctatg caagacttcg ggatggacac tgcagaagtt agggaaccaa aagaccacat 192180
   gttaaaactt gctcacaaaa tatcaccact tggcagtcag agagaagggc caatgccagt 192240
   ctgaggcacc gacttgaggt atagctttat gttagcacaa aattaatgct gattaacttt 192300
   atattataat taattcttca gtacattatc tactctaatc ttctcaacca cttagtaaga 192360
   aaattttgtt tattttccct attctacatt tcaggaaaat aagtattgga aaactacgta 192420
   cttccccaaa ggatactctc aagttttggc aaaaaagacg tctttcttct ttgccccatt 192480
   gtcctgtctc tgtccttagg gaaagaaaca tgaagaaatg ctccctggcc ttttaactgt 192540
   tcttgtggtc acagctccca aagagagtaa ggactcctaa tctgtgtgga cagagcctaa 192600
   atttctgggt gatgtttcac taggctgcag aaaaagcagg tgcccaacct ccacgttcac 192660
   ttcctgcgtg tgtctctccc caggcttctt gcagttttgg gctccatgag aaaagccaga 192720
   atgggggccc catctgtgca gagtgcagcc aaatgggcac tctctgctcc agattccaga 192780
   cctgctggga caaggtctct gactaacatg tccactggcc gtttgtctag gaaacagttt 192840
   gtgtatctac taggttggtg caaaagtaac tgcagttttt gccatttaaa gtaatggcaa 192900
   aaccattatg tagtaatggc attactttaa atggcaaaaa ccgcaatcac ttttgcacca 192960
   acttatagct cttctagatt caagggtgac aagtagaatg ctttacattg attcctgaac 193020
   cttagccttg agtactgatg ggaagagatc tgctccagat ggccatggtg ttgtaatcaa 193080
   ggtctggccc aagtactaag agaaagtagg gttttgtttt tgtttttgtt tttgtttttg 193140
   tttgtatttt ccagtttctc tttggctgtt gtgctcatgt ccaggcatag gtaatgtctg 193200
   agcgagtaca atttcctctc tgtaaaactg taaaggagtg agtagtaggt ttcttgtccc 193260
   aaagccactg ccttatattc ttaacattaa ccttatactc caggtgccag cctgtcccag 193320
   gtatagaggc atggggctta gttgattcat gcaggaagaa atcctaggta gtttcttgga 193380
   aatcttctta gctttaatta atccagggaa aatctccatg gatactaagg acttttctta 193440
   tcctctgtct taggacacac atgtgtggct taagttctgt ctagtttccc ctactgggga 193500
   tttttttttc aactatcata ggcattaagt cggaaaggac ccctgataca catactgatt 193560
   aataagagaa taacagacat tcattagcat attatttcat atatatggga gacacccaga 193620
   gaatgagtag ttctcaaaga ggtggctttg aatgtcagct tatagagcat cttcgacaaa 193680
   gtgttctttg ttggtgagga gtaaatttat agagaagtga caagaaaaag gaaagggacc 193740
   tggagtttcc agccagggta acttgtggga aggcaaataa ctgatagata aagtctggtt 193800
   agcaaatgtt gtcaacaaag attcctctcc agatggtaag aatctaaagc tgtcttcagt 193860
   agttaacctt tgttctcccc aagggaggtc aggataccct ttacctttgt aaatctgtgt 193920
   cttgttttta ggcaaacaca gggagggcag agagtttctt gtatctgctt caacttgcct 193980
   tcagctcaac ccttcatatt tcagggaggt atattcggct gcccctctgc catactccag 194040
   cattcctctg cttttctctc caatgtactg cgcctttctt ttccatccta tcctcacatt 194100
   ttgatgtagt gtgtgttgcc ccaggccagg tagcagaaac atcccctgtg caatctctcc 194160
   ttactctcgt caagctacac aagcttccct ctgtgaaatg accctctcct cctgcatttt 194220
   atttcatgtt gcactttctg ctgggcagaa accaattttg agcctgctcc tgagagctgg 194280
   gtgaagccct ccctaacact atgggtgcat cccagttcca tgggtgccca tgagcttgtg 194340
   atactgtatt ctgtttattg gtttacctct gtctctacag cattacacta ggagtttctt 194400
   aattattcca ccctttattc attcaatata tattgtgttg agcatgcccc atgtgctaag 194460
   agccatggca aggtcccaga gtaaaaagga tcaaaacaaa caaaagaata aaacatggtt 194520
   actgccctca agtaattcac aatgtaatgg aaaatccaga ttagtggaca cacacactgc 194580
   ggcgatggtt caagagtcag ctaatgtctg tgaatccctg gaagagatgc ggtttatctg 194640
   gatgagaagt gaggagactc agactctagc agatcacttt acctggtgcc ctgaaaggtt 194700
   agcagttact ctccagaggc agcaggccag ggatagaaag gggtgggggg taggaacagg 194760
   aaccacgcac acactgagga atgaggctga aatagcagtg caatgtcttt gtggctggtc 194820
   aaagcacagg aaattggaag caattcagtg tggtgggaga agagagtatg gggggagggg 194880
   ctgggagttg aggacatgtg gattggagac agaggctcaa ttcagctatt cgtctttgca 194940
   tctactctat cactccacaa ggcataggct ctcttttctc agtctctctc tctctctttt 195000
   tctctttctc tcagccgtta ttttctaatg agcaaatgaa tgaagaatga agtagacatt 195060
   ccaaggctgc ctccaaatgc ctcttagtaa cataaatatg tatggatgat ggctgttgaa 195120
   atcaccagga aaaaatatga acattactgt ttaagtctca ttattcctta agtgcctgtg 195180
   tgtgtttgct taattacttt gaatggaagc atattttaac attgctatga catctcctac 195240
   ggaataaatg agctaagaaa gagacttaag ctgtagacca caaatggccc ttatatcatg 195300
   tgaagttatt aatacctgcc cttgttcatt gattatttaa gtaatttatt tccatttggg 195360
   acattttacc tcaggtattc tagtctgtgc taaaagctgc aggaaagtgc acctgtccgg 195420
   cctgcttggt gtgatgcacc gttcttgcac atatctgtcc caagctctgg ggtcactggc 195480
   acccattagg ttctagaaaa actgtaggaa ggagcccagt gagcctggga ataggccagt 195540
   ggggaagtac gaagaatgag aagcacaggg gagaattagg gacgcactgg agagtttctg 195600
   ccactgaatt ggacaaaata tttgagttgt gtcagaaaat caaaattgaa caaaaaattt 195660
   tgagaggcac ttactctgtt tttacccctg ggctaaacaa agacatgttt agatataaga 195720
   atatattata ggtagataga tagacagtcc ctggcattgt ggaacttagg gtttacttaa 195780
   agataattac ttcaggcagt attggaaatc atagtggtca atgaagatag tagatcttta 195840
   ctatgtgttt tatcaaactg ctcttattca ttcatttcat aacatcgtaa tagctctcac 195900
   ggaaaacaaa tttagtggga ataaaattgt tagatagtta ggcagacatg agcagggcag 195960
   gagaggtgcc ttccaaccca ggaatgtcaa gtgaccatca ggtgatggtc aggcagttgt 196020
   taaactgtct ctctaaaata aaaattggtc acagccagtg ctaaggaaag tctgtctccc 196080
   aaaagataga aagtacttga agttggtgat ctgctgcttc ctgataagat ctcaggagtt 196140
   gggcaagtgt gttcagacat gtgcactaag aggcaaaatg gcagtttaac tggtatatga 196200
   ccttcctcta ggaacacttg gctggtaaag gaagaacgcc tcaagtgagc atgcccacaa 196260
   catcagtaaa ccactgggca tgcggcccct cccacatgct ggcaggccac tgaacatgag 196320
   gatagcccct cccaaggaaa aatcaaggga ggagaaatgc aaacactgga accaagccaa 196380
   tgtgtaaaac cacaagtcaa gggccaaaca gcacacttag atctcttaaa tgtccacttg 196440
   gcccccttcc aagtatactt cctttcgttc ctgtctaaaa cttgcctcag tctctctctc 196500
   tgccctatgc ctctcacctg aaatctttcc tccaaggagg gaagaatcaa gttgctgcag 196560
   atccgccaga tttgctgctg ctaacacaat gaactttgtg aagatgaggg aggaaagaga 196620
   tttgagaact atgctctgat tttttttatc ttgcccaaat tcctatttaa gggtcctagg 196680
   ggggtcatgc cctacaaatc ttaagttctc atcctatggg ttttatttaa ctctatatat 196740
   tttgacttac tttccaacct gactctggcc ataaattaca agacaaggaa gaaaaataaa 196800
   aatattttac catgaaacat atttctttgc catattttga aatggccctg caaagctgtt 196860
   ctttgtggag gaaaatgttt catctgtaaa gaatctctat taacatagct agatcttttt 196920
   ctttcagatg cttccaatcc taaagaaacc tctttaagat taactaagat ttgaatagga 196980
   aacaatttgc catctattgt ctctaaaggc agccacaata agacttcaaa aaactttggc 197040
   ctccacagtc ttttatctta ccctgaacat tccctttcta aggatcccag gtctttagac 197100
   aaactcaacc aattgtcaac cagaaaatgt ttgaattcac ctatagctga gaagcctccc 197160
   ccaactcccc tacccagttt tgagttgtcc tgcctttcta gaacaaacaa atgtattttt 197220
   taaggtattt gatttatatc tcatgccttt ctaaaatgta taaaccaagc tgcgccccga 197280
   caaccttgga cacatgttct caggacctcc tgagggctgt gtcacggacc atggtcactc 197340
   atattttgct cagaataaat ctcttcaaat attttacaga gttcgactct ttttgttgac 197400
   agaataacct gaagaattgg accacactta atgagaagcc gaatgtttgg aaagattatc 197460
   gatttgtagt tgacgtttgt ttaattctag ggcagtatcc cacataaaat agagagaagg 197520
   ataattttca tgattatcct taagcttaat aagatcacac atgcaaagtg ataggaaaat 197580
   tgttgttgtt atttcaatca atgtagctat tatacttatg tcactcaata gagagaaact 197640
   gcatgcttat gaaaggtcat atagtagctc atgggaagaa cttgagctga aacccagttc 197700
   ctctgactca tattcaaatt atctttctag gaaatcacac aatatactca gttctcttta 197760
   aaaacaccag agtccccatc ttgtgctcag aatgttctct tacactccct ttgcttcctc 197820
   ttttctacta ggtgaaataa cagcatcact ggacgcaaga aggaaaccct taccccaacc 197880
   ccactgaaat gatttagatg tccaggtaaa gtgtggcctc cctaaaacgt gtttcccaag 197940
   ggtgactgaa atagcaccat tgaaaattca gcactctggt ttcagtgttg actttttcct 198000
   ctcttttcaa agttgtcttt tgctgtgaaa cataaatgct ggtcacttca tcaacacttt 198060
   atcataaaga ttattgaatt cattcaagtg actgagccct taggaatcct tcctgccttc 198120
   agggaacact ctgacatttg gataaatgtc tcacattaaa gtaagtaaat aaaccagaca 198180
   ccatttcaca ctagatccta gtgagaaaaa gaaagattcc aatcaaaggg acaaaatgga 198240
   aatgacctct cccaactttt catatgtgta gattcagctg gggtaatcga aacaatggtt 198300
   ttatcatcat gttccttcct tccagttcag gcctgatagg aatatctaaa gacctctgtg 198360
   gaatcatagt aaaatgggca gatattggag catggtagta attattgatt gagaaaatat 198420
   caatggcaaa tattacttta ataaccaggt tagtgagtca atccttgggt tgagaccata 198480
   aacccaaatt catctttgtt taaattatag tatggcatat ttgtatcatg ttggaaaagt 198540
   catttacttt ggtataatac acagatatat ttggtctttg ttctgaattc ctggcaccca 198600
   gttcctagaa cgcttggaat ttcctgagtg atagaaatat cttttgttat tcataaggaa 198660
   gctgtttcca tcacacctga gtttatgctg atatgtgact tcaggtggag cctttagata 198720
   gcctcaggat gggagtggtc atcagaaaga ccaagtattt aggagactgg aacttctagc 198780
   cctatatggc cactgacctc cagaaaatag ggtgagacaa ctggagttta tgctctataa 198840
   aaagtcttga acaatgagat ttggagacat tttggggttg gtgaacatgt caatttgttc 198900
   agaggtggca catctagaga gggcatggga gctctgtgtc acccactgtg ttatgccttg 198960
   ccttatgaat ctattccgtt tggctgtttc tgagttatat cctttaaaat aagccagtac 199020
   atagaactga agaagcagga gagaatcata ggaaccccag atctatagtc agttggacag 199080
   aagtattaag tggcctagga ctgcaactgg tgttggaagt ggggacagtc ctatgggata 199140
   gagcccttta atttgtggca tctgatgctc agtccaggta gatagcatca gaattaaatt 199200
   gaatttttgg ttactcagtt ggtgtcagag cactggggaa tttgttgttg gtgtgagaaa 199260
   acacgtgata cctttcaggg tttctcagtt tatttgtaaa atggaaatac taaagtctat 199320
   ttcttaggtt gctatgtgga tttcattaga tatcataaat gaagtgcctt ctattttgtc 199380
   tttcataggg tagttttttt ttttagaaaa agctaggtat tcttaaaaga ttttatataa 199440
   aacagtttta gttccatatc ttatgagaaa ttttttattg cctaaataga gaggctcata 199500
   gctctgcccc catagttagg atgtctcaac ccttgtcact gacctaagat tctccaagaa 199560
   ttccaaagac ccagctatac cagggaatac agaccagcag cgatcttttg tttgcacaac 199620
   atttttttgt aattgaaatt atttaaaaat gggtctatag taaggagatt cacatcgaaa 199680
   gctttgatga tgtgcatctt ctgaaaaact aaaaggtact gcagagtagt cttcatagtc 199740
   acgatgcgct gtgctgaggc tgctgcttag aaaggtgggg cttcttctcc ctgcttaccc 199800
   acgtgtctgg ctgatctctc agctcagctg caggagcttg ttggtcctga agagtggtct 199860
   gtctttattt gctgatactt aaactttcct tcttgaaaac tgaatcactg ctatttggtg 199920
   cacattttca cccagtctga gtgccacctt acatctttca ttctgcatct tgagaattct 199980
   gttttacagt cagaggtgac aactagatca gagcattgac tgctcccaat ttcgctttta 200040
   caaaagagag caaaggaagt ggtgatgtta ggtttggggg tgatgataaa gtcagagagg 200100
   ggaactccca ctgaagccca cattatgaaa agagctgaac caacttgtac tcttgatctc 200160
   cccttaaaag ctggttttgt acctgaatgg agagaagtgc agatattaga gacctggctg 200220
   atgtctggtg cttcattgcc ctgaatagac agaaaaatgc agacccttgg gaagcaaagt 200280
   gcttgacaga gctgaaagtg aacagcagag gctgggagga agcaggacca aacaaatcca 200340
   gaaatcctcc aaggctgtag ccctaggccc agcaggtggt ggtcaggaca aagtaggagc 200400
   atgtacagtg ttgagatcag agtcttggga aagcacctgt tacaggtcag agtgaaggat 200460
   gatcccagaa acaagtcaga caagaggcag gtaagtgaac aaagtgatca caagtcaagg 200520
   gaaatggtga gtccataagt ttgtcagtag caatttactt taactacttc atcttaggcc 200580
   atattgtgta catgcgtata ttggtttctt tcaattgcat gtcaaggaga tctgctcctt 200640
   tttttttttt tttttttttt ggtacatgtg ttgctgaagt gaacacagat ctgctagctt 200700
   ttaatcagaa aatccacact ccagggctgg ctactacaag ttgttttgct ttcttgctta 200760
   ctaaatacta tcagaagcta tattaattaa gtataagggt tgatagatat tagaaaggtt 200820
   gttgattagc aataatgagc attagaaatg tgcttacatt tttgtgcatt tttgatattc 200880
   aatgtgtgtg gataaggtaa aacattttga aaacactttc tgcttttaaa ggagcagaca 200940
   ttaaaatcta gtgtctattt caccatatac ccttagcgat aacatggaag gttcgatatg 201000
   taaataatct tgtgttgtgc tgttgcttta gaaggttatt ggtcacagta ttccaagagc 201060
   tgatttgctg aaatacataa tgagaatatt gtaacatttg gtttcaaaga actttccatg 201120
   caacacattt tcagaacctc aaaacagaat agtgggattt tattaaggct gcatgcttac 201180
   ttgtaaattg tgtacatttg tagcataatt cagtcttggg cttaatttta taatttcctg 201240
   gaagctaaat aatgaaagag tgttaattct cacctgtctt caattactgg gcagcactgg 201300
   tgagattctc aaaagaaatc tattacctgt gtgtgactac agtactcagg aactgtatgg 201360
   atagagaaga acaagaatga cctgtggatt aaaaagacag aacagaggtc caaagggagt 201420
   gagtgagacg ccagcaacag ggacaccttc tggctcagta aaaacacttg ttctgtggtg 201480
   gggaatggtt agggttgcac tagctgtacc ttctgtgggg aataggcaac cttgggtatg 201540
   gaaaatcact gagacaagat tcaggagacc cagttcagtg ctagccctaa ttggtcatgt 201600
   gaacttgggt aagaccctta tcccctctaa gtcttggctt cctgatgggt tagatgaaga 201660
   gttaaacaaa atattcttgg ctggggtagc acagtgtggt gaaggcagtg agatttcggg 201720
   atctttagtc tctcttacaa tcttaggctc actctgaata tctagggctt cttgtgcaag 201780
   ttgtttaatc catttaagct ttaatttttt tatctattaa atgcaggcga taacacctat 201840
   atcaaaaagg ctatttttag tattaagtaa aaggtgacat gtgaagtgtt taaaaaactg 201900
   cttaataatg ttaccccatt cctctcatct ttccctttac actcacacat tctacaattc 201960
   tatgaattaa ggggaaatga ttgatggaaa tttgtggcac aggcagagat ggaatgggga 202020
   tgttatttcc tcagtattgg gcttgagtga tagtaaacga tgaagataaa gttcactttt 202080
   gaagtggaat ggttaaagta ccattgaggg aatacatgtt taacctaata aactggagac 202140
   ccattttgta gttgtattct accccatggg aagtgcaaga gacacagcac accaaaaact 202200
   aggaaagctc tacgattact agtcgggact ctggatccaa ctggaaacat cactgtgtga 202260
   tggatcagaa tttgaagcat gaggagaatc tgggctaggc gagcacagaa atcatgccta 202320
   caccaccagt gggaagagga aactctgtct ctctgtatgt atatatgttt gtgtgtatgt 202380
   ttttgtgtat gtatgtatat acatgcatat atattatgtg tatatataac atatatttag 202440
   tagatttaaa aattatgaaa ttatgataaa attgtggctt cagtcatcaa caattctttg 202500
   ttaagtcatt aatgaatgac tttttttttt ttttttgaga cagtctcact ctgttgccca 202560
   ggctggagtg cagtggcatg atctcggctc actggaatct ctgcctcccg ggttcaagtg 202620
   attttcctgt ctcagcctcc cagatagctg agattacagg agcccactac tatgctcagc 202680
   taatttttgt gtttttagta gagatggggt ttcaccatgt tagccaggct ggccttgaac 202740
   tcctgacctc agatgatgca tccgcctcag cctcccaaag tgctgggatt acagacgtga 202800
   gccttcgtac cttgcctgaa caaattttct tttataaaaa attgggttgc tccattcctt 202860
   atttgacatt ttattttaaa aagtgattta tttataatta taaacttaag caatatagag 202920
   gatgcaagga aaaaaaactg aaaaagcaga aatgaatccc aatctaatga aggggaatta 202980
   gtcaccgtta gtatttgata aacatgtaga tggacatgtg gatgcagaga tagacctgga 203040
   ggcagattgg cagaaagata gataattaac tagaggtgtc tgaagattgg agttgggagg 203100
   ggtatcatgg acttgtcctg gaacaagtct ctttaggtga ctcacccagg ggatcagtgt 203160
   gtctcacaat attgttcttt aagtcactag ttctctctgt taggatctta tgacatctgc 203220
   tttcttattt ttcctcttcc caatttcatt tccaacagtt tcttagccag aaggtaggct 203280
   gataataaga caatgacaag tgagagtgct tcctccttca cctccacctg ttttgaaaag 203340
   gagtcttgta tgtggacatt cccctctttt ccttggtacc cctcacccct ccttgcggca 203400
   tcacatggct atttgagcac catgaacctc cctttcaggg agatctaaat cctgcctaag 203460
   gtctgagtta agactgcctg tctttcagcc tcctacctca cttgtcctca atataactgt 203520
   atttggcaaa tattcttcca agtgtgaaat ttggagttgg ggcatattct tgttagtgat 203580
   gaaggtggaa ctttcttctt gatattttct tgctgctttt atagatttta gcaagttttt 203640
   tggaatgaga catcaaaaaa gaatggcttt attcaatgtt cttgagccag aaatctccta 203700
   cctgtgaaga ttctggtact cagatttaat acaatcaagt gtctcttgaa attttatgtc 203760
   ttagcatgcc ttttattttt atttttgggt gtgctatacc ttatttcagc actactaata 203820
   gaaaaaaaaa ccaactatat agacaagaat aatcaagaaa aatacctcaa gggccaatgt 203880
   gaaaagattt ttagtatttt ataatttata acattttata atttaatttt tctaagtagt 203940
   aaaatatatt taaaatctgt gtatttttaa gaggaagttc caaatgaaag aaatatgaaa 204000
   caaacaaagt tagacatggg aaggaaaatg atcaccccat tgctatgttc atcatgtctt 204060
   attctcacta ttttaggagt gtcaaggtgg cgtttaggca ccgtgtgact cctttaccgt 204120
   gaatcaccct ctgcaacccc caggtgcagg ctgctaatta ttctcttcaa attttctcca 204180
   ggagttccct gtttctctgt cttggatata tatttggctc tgcatggtag accaggagca 204240
   ccagaactga gacattaaaa tcttttgagt taaatagtat taggagatta ttttcacagg 204300
   cgcctttaaa aatagatgta tccaattact gtaattctaa tttttcaaat gctagtcctt 204360
   ttgacaagtt gcagctctgt ttaccagctc ttgtaggagt ctcatgattt cccccaaaag 204420
   agcagcatga agctgaaatt gtaagtttat caaagcaaat tgcatgcatt ttccaaaggc 204480
   aaattgcaga ccaaagccat ttgtagatga atagaaaact acatgaaaga gaacactctg 204540
   ctgtgtttgc ctactcataa cttcaaccca aattaccttt taaaacacta tttggaaatc 204600
   tatagaactt caaaagggcc cattaatgga tgtgttgaaa atagaaccca acttcttcaa 204660
   ctccaaacac agagtttcac tattcaacaa ggaagtttcc cagaataagg tttaagctta 204720
   agttcctgaa gttcataggc taaaaatata caaagtttac gttgtagttt ctcatcactg 204780
   taagactcac cacatattca aaagaccatt aggaagtcct gaatgtatac tgtgtcctac 204840
   cctgaagagg gcacagttta ttaaaactat tatcatatat atctaaacaa actcttttac 204900
   ataaaaaagg taagtaaaaa tattatgtgg attaagggat agagggttga aaggactatt 204960
   tcaacggctt ttaacaaact cagcagattc aaaacttgaa ttcaggtctc aacttatctt 205020
   tcaagaggaa aatgctgaaa gtgtacagcg agtgtaactt cgggtttctg tataatattt 205080
   aatgagataa atgcctccct ccctgtagtg tgggttactc agcatttgcc ttgcattttt 205140
   attctccagc ttattctctt ctgtgtccag aaggctgatt tccatagggg gcatcaactg 205200
   cattccctcc ccagtttcaa ttttgctagt aaaaagcatc agagggagat cagaggatga 205260
   aaagtagaaa gattagagca tttattcctc ccaccccacc ccagcttgag ttcaggtctg 205320
   acagtgtctg cagcactggc actatggccc tgagggccac ctttttccat gtccagctct 205380
   taactaagct ccagtaacac caatgcctcc atttgttcca ccaggcctag gagtggcaaa 205440
   ggctcttcac ccttgctagt ccctaggtgt ctacatatcc cttgtaggtt gttacctctt 205500
   accacgcctt ggtaaacact cccctcatta agaagcttct ttgaaaaatt ccaactaact 205560
   gagcatgcca tgtgttttag gctggggtcc tgacacatac aaatttgtgt atctaatttt 205620
   aaaagcttat ttcacctcct gctcttgcat gcaattatag cgcataatct tgtggttaga 205680
   gataagcaaa cacgctgaaa ctaaaatgca aatgcaccct ggaatactgt aacttaaaag 205740
   ctgccaccaa aataattcta cagcagacaa gtccatcctg cacttaggag atgagtctac 205800
   gtatgcacac aggtgctttt cttagcaaga acttcttctt atgtgggtaa ctctaggcct 205860
   tctctctggt gtggagtctc tgtcttgttt ggtggtcctg atcgtttcac ttatcatacc 205920
   tcctgctgaa cctttccagg caaggcagtg attcaggaaa gaccgctgac agtgtagaaa 205980
   gtacacggtt ttgtttctgc tggccagcta gcggggaatc ctggtattca gaaggggaat 206040
   caattctttc tttttgattt agggatatgt ttggcagtta ttaattaaca tgtgtgagag 206100
   atcctgagaa tttcaaacat ttatttttta aaggagtcta agtttaaact taacttcatt 206160
   atataaaact tccttctcct tcctcaacct cacttctttg aattatttta ctttagaaat 206220
   ggcaagtttt ctcaaagatt aatgcaaagc tcatgtcaaa atagagctct ctctctctca 206280
   attttatttc tactatttta actatttcat ctatttattt atttttttat agttttcgtg 206340
   gtccattagt tccatgattt aatttactat tttactttaa aagattttcc cttgccagtc 206400
   aagtagtatt ccatagaata catgagagaa tacatttttg aagaaaactg gtaaacaacc 206460
   aattaaaact ctttatggat tttcttccac atataaatta ctattctata catgaaataa 206520
   taaaaataag ctttggaatg aaagtatata aaaatgtcaa tagaaattct aagtatgggg 206580
   taatcagcct tgaggtaatt gatagttgcc tatacttaca tgatttgtaa tctgtcaatc 206640
   atttattcaa caaatatttt ccacagctcc tatacacaaa gcattttaag ggtagtatta 206700
   aaaacaaagt actttccctt ccctaaaaat ctttataatt tatttcagaa gaagagaaag 206760
   gtactttttt tttggcagac ggggacagag tctctctgtg ttgtctaggc tggagtgcag 206820
   tggtacaacc tcagctcact gcaacctctg cctcccaggt tcaagcaatt ctcctgcctc 206880
   agcctcctga gtagctcgca ctacaggtgc ccaccactac gcctggctaa tttttgtatt 206940
   tttagtagag acggggtttc accgtatttg ccaggctggt ctcgaactcc tgacctcagg 207000
   tgatctgcct gactcagctt cccaaaatgc tgggattaca ggtatgagcc accttgccca 207060
   gcctcgaaga aaggtacttt ttaaggaagt gcaacgatag tggcaaataa gagttccaag 207120
   cagaatgaag cctcttcctg gtagtgggaa ctggggcttc tggtctcagc cattgatcaa 207180
   ttatcactga caggaaccat gagacctgtg caaatgaatg actgtgtaca gttctagtca 207240
   ttgatctgat cctcaaaaaa agatgtgata aatgtggatt aattgaataa gggattgtgt 207300
   gtgtgtatga gtatgtgtgt gtggaaggga gtgtaaagta tgcaactact ttgctatttg 207360
   aagatagatt aaaatcacct ccatctcttc cttcagcata gaaagacaaa acgtgggaga 207420
   agtgtgaatg caagtctatt caccagtttc taggatttgc tctccgggtt tggtgggtta 207480
   ggatggggat caaaatttca aagaaatatt gcacagccaa gaaaataaaa gtctagtata 207540
   cctattcagt tgctaggcag ttactaatag gttgttgata attcaactac tggtacgctt 207600
   aggtaggctg aaatgcaatt aatttgaaga attacgcaga aaaaatcctg gatgagagac 207660
   ctagaagtga ttaaggaaat aagagtggct caggcacttc tctaaccatt catcaactgt 207720
   gtaatggaac acaagcaatg cttttcctca acctttaaca gagtggactg ctgggaagat 207780
   gcattatgac acagatccag gaaagcatca tctctgagtc tttctgcctc tttgtgttgg 207840
   ttgggagagg ctaaaagagg tttctgaaga ggaatgacca gtgttagaag aagtgtagca 207900
   tgtgtgctac catttcaggg cattatagca gagaactcag aaaggccaga atcttgtacc 207960
   aaactgtaac cacctctttt ccaggtgaga cagccagcaa agaaagacac atgaagcaaa 208020
   tggagccaca cgtgatgatc atactgatct ctgctaaatc aagctacagc acacaggagt 208080
   cccaaagctt gctgggcctt ctctcacctg catgttcact cttattaaat tatgatgagc 208140
   cctctgacaa actgtcatat ttgaaggacc ttttaccaga gaagaggagg gggaggaaga 208200
   gagaaatcaa aaaagataag caaaagggct gggcttgctg tgtgatcaca gataaaatac 208260
   caaacagtta aataatgctg gagggtgacc tcttatgtga cccaatcaga gatttgacat 208320
   ttatctcctg aatgtgacat cccatctcat caaggatttg ggacagtggg gatgaggaga 208380
   cacgttctct ctccctttcc ttgctcagcc ttgttctctc tgtttactct ttgggcttca 208440
   gtgcactttt gtggccaagc ccagtttgtg gcctgcccct tgcatggcca tctctgcagt 208500
   atcagtgaaa actcaacagc ccctctgtgt ggccacactt tttttctttt ctaaaaacaa 208560
   tgagatactt atatccaccc catgcaaatt cctccaggat attgctcttg agtcatcctt 208620
   gagaaatttc cgtcttgaaa catgtctatc tccatgttgg gaaatttaat tagtactcct 208680
   attgtaggga ttagggaaag cacaactaca cttttcatca gcttcaatga tttcaccaaa 208740
   gggcatttat ttacttagtt ataccagctg tttgttcacc agataccgtc tcctttgacc 208800
   ctatgtgcac acaaacccac gcacagcgga tgttgactat gagaactgat aatatcacac 208860
   ccattttaga tacatcattg aggcaaaaag ggacatttat gggagatatt tttctttttg 208920
   ttgtggaaat tctgacaatc tcattttatt ttctcattca aattattaaa tgattctctc 208980
   tctctctctc tctgcttaca taggggtcat catttgttct tttaatccat ttcaagagca 209040
   tgtccccttt tgccgtattc agggatgatt gccccctacc tgaaccccat cttgctccaa 209100
   caaacaagtc atataatcag agatcgcact cttagtcaaa agtctgacca gttaaagaat 209160
   taattgcctg cgccagggag tatggaaagc aaaaaagaaa ctctgttatg atttctatag 209220
   atggaaggag aaatgcacta tttgaatgat agagtttaat tgtcattatc tagaaaagat 209280
   acttcattta aacttctcct ttacttaaaa taaacacctc aatgctcaca ttcctgcctg 209340
   gaacattttt agcaataact gctggttgtc aatgacaaca atcatattta attgttcatc 209400
   ctgacatgct ccagcactct attaagcaca gattactata gattgaataa atgaatagca 209460
   aagatgctgg ccaagagctc tgaacgtatc agtggaaata atgaattgca tcatccttcc 209520
   atgaaaatgc aggaagggcc aacacaggtc aagagcttgt tagagtttca ggttgtccat 209580
   tttgactgaa ttaaaaagaa agcaagttta ttatggatta tacatgtaag cagggagagg 209640
   atgaaggtca aatttcttga ttaggtatta tttagctatt ttaccatatc tcaaagttat 209700
   aattcagaaa tgaaatgaag tgtatcttgt ttcttatatg aatttttaaa aggtacacag 209760
   tagatagtct tactgaattt tagtttaatt gcactttcat ctcaatatta ttattttatc 209820
   aagccagata agtaaataat atcctttcat caggtatcag agcacagttg taatataaat 209880
   gggattagca taatgaatgc ctttggagaa aattataaaa tgtcctttca tgcacataca 209940
   gttttataaa catgactttc ctttgtttta attcaatctc tgatggaaat gcacaaattt 210000
   taaaattctg ggaagaacaa acttatctga tcatttttaa gtaattgata gatcacagta 210060
   gaattatgtt tttcttttaa gtgatgagga tagaaaattt gagttgttta attagacatg 210120
   tgactgaacc cagacgtaag ggatcacttt tggtgccctg gaaagtactt tgtactgaat 210180
   ggggaagtcc agatatcata ttctggtgtt gggctaaata ctaattacaa aaccatcaga 210240
   caaaattatt gttattatta ttttgcagca agtgactgtt aacattgaat gtgtgttatc 210300
   aacttaaaag tctattcttt cttgaaaata tgtgtcataa atattttctc ctcaaagaat 210360
   aaacacacct tttataaatg agttgattta ttactgaact atctatttat gacagcatgc 210420
   ttcttccatt tatccaataa ttatttggtt aacaatcaca tttcataggc ttaatgtttt 210480
   ataattgttt tggtaaacct cctgaatatc tctatgtgta cagaaagcag ggaatgggca 210540
   ttttaagggg ctgagatttg caaactgtcc tttagaaaat ttgtaacaat gtacccagcc 210600
   caataataaa caagaatgtc tttccattga ctataccaaa tttctacttt cctttaaagt 210660
   ttggcaaaat tataagaaat attaaattat tattatttat ttatcatttg ttgtactgat 210720
   aaataaccaa tccatccaat aacaaataat ttatccaaat tataaaatat taattattat 210780
   ttatcattta ttaataaatt atttattcaa taataaataa ctcatccatg ttatatatca 210840
   tgtgttattt attttaataa tgaataataa ataatattta gtatttatta tttgattatt 210900
   taatttttaa ataaatgagt ttgcattttt aaatatattt attgacaatt tgtcaagttc 210960
   tcttgctaat ttccttgcct tcttattcct agttagactt gcatgctatt tgtgttgcaa 211020
   atatttctca gttggtcaat tttcctttct ttttattata gtcaatgcaa acttactaca 211080
   aaagtagtaa ttttaaggtt tctctggcac cagaataaat gcatgctaac caataataaa 211140
   tgatacagta taattttaaa gtccacacat tgtgtgggag ccaaatggaa cactgtcttc 211200
   acaatgtggg gcacagtctt tgtgaggaca actcctctat gcccagcttc aattatgtag 211260
   atttttgaga gtgtcagaag tctgaagtag gatcagtgga taggattcta tggtcttatt 211320
   attattttaa ttatctggtg ttccaaaaag tgtttcctga aagttaaaca atgagagttg 211380
   gtttccttcc actttactaa gtggtgtttt attttttcct tcttagacta tatgtatgat 211440
   ctgtaattat gcatgaggtc aaattgatga gggctagcca aatatgctaa aatgtcagtc 211500
   tgagaatttt aagtgtaact ctagcagaaa ataagagcaa acttgtccta ctaatactga 211560
   ctaccctgga ggacacctgt actttaatgt ctaatgaatt gtttttcatt gagtcaattt 211620
   tttttacatc aatgttcatt gaatatttat tctgcatcaa accctgtatt cagccctggt 211680
   tggttaaaga ttatctattt acttatcttc tatgtatcta tctatctatc tattatctat 211740
   ctgtctgtct atctatctat ctatctatct atctatctat ctatcatcta tctattcacc 211800
   tatcatctat ctatccattt atctataacc atctatctat ctatatctgt ctatgtctat 211860
   ccatctgtca tctatctatc tatcatctat ctattgtctc tattcattat tcatctatct 211920
   accctatcta tctatctatt agctacatat ttagctagct ggcatctatc tatatgtctc 211980
   tatcatctat cttgatatag atatacagat agagatacag ttttttcctc aggaaagtca 212040
   gtgttaaata aggctctcaa ctgagcataa ggatgaggac aggtaaacga taggggaatc 212100
   taattcaaat gagtagttca gggaaggctt cttcaggaag gggatcatta acactaaaac 212160
   ttgaagggca catgcactta tgtgttatgt ggataaatta ccaggggaag tactggagag 212220
   aactagagag aaagaagcaa gattcaacat ggttttttaa aaaagcgtga tgttaaaaaa 212280
   aaaaatttac aggaaattac cctaagtcag tcagtgtttc ttaaggctga gcattaagag 212340
   aaataattct tggggatgtt aaaagctcag atgtgtggga actatcttag gcctgttgaa 212400
   aagaaactta atgaggccac aacaggtctg aagcagtggc aatcttgaaa aaaaacacag 212460
   aaaaagacag agactgaaga ccaatgcaag caattctaat tccagaccca aagaaatgag 212520
   ataataatta taatttctaa ataaagtcaa ctttctacta atgtacagaa agagaaaccc 212580
   agggcaatat ttaaagtgaa gaaatgaaaa aaagataaca tatctgtaaa actgtgcatc 212640
   tataacttaa ttttgtaaga ataaatactg cttttactag taacttaaag aaaaaggttg 212700
   tgatttagac aaataataag gaagaagtaa attgaattca gttagtgacc gattagaaat 212760
   atcaagtttc ttgactcaaa caccctattt atttttctaa gttagagtaa ggcttagaaa 212820
   acagcaagta tggtcttaaa aattattagc aaaactcaga agttctccct caaatcagga 212880
   attcctctct ctctttctta ctcacatgca tgcacacaca cgagcagatt ctaatcacag 212940
   tgaaaaatac caagatcgat aaatatgcta ttcagatata agaaacttta atattttata 213000
   atatttgccc tatattttta aaggaataat agggttatag ttaaaacctg ttttgcatta 213060
   ccatctcagt cttacttctt tcacttcttc cctttgcaaa aaatgtcatt ttactgaaca 213120
   ttaaatattt agatctaggt catttccttt aaatgctgtc tgaacattta cgtatgaatg 213180
   tattacaatt tatataccca ttcctctaat aatagaaatt taatattatt cccagttttc 213240
   gatattacaa aacattgcaa taaatatcaa tatttgtatc tctttgtgga ggttatcaaa 213300
   gttccccttt agcgttccta tttagaaatg ggagttctaa gttgcaacag atactggtgt 213360
   tactaaatct catccttttc ctctattttt tagttcatgt ttctgccccc acttgtacct 213420
   aatcacactc accaaaaggg tggtaggtgt ggctgggttt cttgcctgtc actgtgtgat 213480
   ggtttgaagc agctaatcct ttcaaaagct aaatggaatg caaagactta gccctatttt 213540
   agagataaag aaggctaagt tagaacacac caagtatgtt ttttagatat catcaagcaa 213600
   atggaatatc cattattgta actgaaaagt ctgggctgtt tcaactgagt tcaaaagcca 213660
   cagtaaaaca tcaatttaga agcaagctgt tagagaaaat atgcctgtga atgtgtatgt 213720
   ctgtgtgtgt ttgtgtataa aaatatatga tgtagcttat aacacaaata agtcagcctc 213780
   ctacccacta cacatgtgtg cacgtgtaca cacagacaca cagaaaaaca tgcattgtat 213840
   cagggctatt tgcagaccac ctataatatt ggattataaa ttactgcctt taataacata 213900
   gttttgccgg cagcttggta ggaagtcccc agtaagaatt aatttgaagg atatagttgc 213960
   aacgtctttc tactttccaa atatttccct tgtatacttc aacagtgcct ggtatttcaa 214020
   cattagtgat ttattcactt gttttcaggc actgaaatct ccaaaggtcc tactttcaaa 214080
   ataaaatttt aaaatttata gaagaaatgt attatgccac taagatcaga atgattgaat 214140
   agatctaaag catttataac catttccctt tcaggggtgt ccaagggaga taatagagtc 214200
   atagattctt agttctgttt ctggttgggc cagcgaaata ccttcctcat cccttttctg 214260
   cttgtcacta gagacagaaa ctaaaagcca tggctcaggc tgccaaaagc ttaaaacaaa 214320
   acaacaataa caataaaata aggcaggttg gacaagcttg tgggatggtg agggtgtaac 214380
   aagtcctgga gaaggtatgt tccaatagac agcaggcaag agtagatggg agagggcttc 214440
   ccacagtggc tcacctagaa cacatctcta aatgtcttct ttaaacacca tttcctttcc 214500
   ccagcatagc tgataaactc tctctatatt ctcacccatc gaggcctctt tttatcacac 214560
   ttcatttcct tccttattct aactctctgt gccctcaggt tctgagctac tcctgccatg 214620
   ggcagcccct ccacaccctc aacctcaaca aggctcatgt gattctctct tcattccagg 214680
   gcaatatttc tcaatcttgg catgattgcc agtttgagca gattaattct ttgttgtaga 214740
   ggcaatcttg cgcattatag gatgcttgct agcatccttg gcctctagta attagatgtc 214800
   agtaggatct ccccagttgt gacaaccaaa aatgtcccta gacatggtca aatgtgccct 214860
   gggggacaaa caattgagaa ccacttttct ggatataatc tctatctcat tacactggta 214920
   attgcaatgc agagtagtgt caaacataaa taatgagatt cagaaactat gtgaagtata 214980
   gtgtttatct gagtgcaaag cttgaagatg accacctgga aaacacatac tctaaaacaa 215040
   tgggtcttcg ttcctaagtg gataagtcaa ggtttcattt atatagggag agttgcaaca 215100
   ttttccatac aaggtcagtg cataggttat ggcaacttga taagttacag attgccataa 215160
   taaaaggaag attgccttga cattccatga ggaggggtga tgatctgagt ggggtcttat 215220
   ctctgctgcc acaaggcctt tcttaatcat ttataggaag gggaagaagt tccagctgca 215280
   cactatgtga ctcatgccac atagccagaa tcctctctgg gttcaaaatg ccttaaagtt 215340
   ccaacagttt caagtttgaa ttatttaatt ttacagtaga aaaagatgtc aggaagagta 215400
   ctgagaaagt atctaaccca gaataaagag ttcggagagg gatccctaaa ggatgtcaga 215460
   aatgtcaacg catatggttg ggaaagagtt ttctaggttg agatacatca catacggaag 215520
   cctgctgata aatgataatg aaacatcagg ggaaatatta agtaattcag agtaccagca 215580
   gggaagtggt gaaatactgc tggaggaggt gggtgaaagg aacaggatga agagtgttgt 215640
   gtactaagat gagtttgacc ttctcctgag gtcaagaggg gaccaccgaa gcattttaaa 215700
   cacacaaata gtttcatttt agtaaagttc ttctggtagc ctgtggaaat caaagtcaac 215760
   aaacatagag actattagga gactattata catcttagat aacattcgtg taatgtttag 215820
   ggcaatgtgg tcattgacta ggtcaactag gtaaatctta gtgtcatcct ctttctacat 215880
   cataatttca acaaagccat ggctgttggc gattcaaaaa ggaaatatca ttgtcacata 215940
   gtgagtccgg aaagaaggta aaacatgctc atgagctctt actcatattt ttcattgtat 216000
   ttttttagaa tttaattttt acttattatt ttctcaatct ataaaatttg aaattctcac 216060
   aagccttagc atgtaaactc tgttactgtc ttcacacctt tgggagatct catccagtct 216120
   cagggccata aatataacct atattttgtt aactttaatt tacatctcta gcttgaattc 216180
   ccccacttaa ttcccaaaaa caactcaaca tatcaactta gatgcttaat agggacccct 216240
   cacaatctat cagtaaattc tgaaagcact ataattaata gacatatacc tatacctaac 216300
   tctctctctc tctctctcac acacacacaa atggttagtt tccaacatta tttcttctta 216360
   ctttttctct aaaactggtt acagtgtgag tttccatttt ataatcaatc tttactcagc 216420
   taacactagc tattgcgctt tatagttaaa cattagactg gccgttatgg cttactatta 216480
   tgagaatcaa tgttttattt gacactttgt gcctcaaagc tgcttattat taaattgtgg 216540
   cagcactatt acttcccaaa tgtgttggcc tcaacctaat ccccaaatcc ttgtaggcac 216600
   atgtccatgg gcatgttatt aactgaaagt acttactcac acagccttac caactttaaa 216660
   aaaacaagga aaatctgaag gaaaaaaaag tcatgtaata ttaatagcac atattgtatt 216720
   ataaattatt ctgtaacaaa taagcaaatg taaaaatgat gctgtattca ccccccaact 216780
   taatcttgtg tagtggcata ctgtgaaata gcatatctca tttcccagtc taatgagtta 216840
   ttgtaaatac tgtttggtca aaattacatg tgtataaaac cttaggtctc gtgtttcttg 216900
   atatttctgt gtttgaagtc tgtggtaact tgtcatggca gcctgaactg actaagagag 216960
   gacccattct gatgctatca aatattaaca aataattgag atcctgctcc actgaagact 217020
   tctttgtcct caattgtaga tggagggaat gggctgttaa tagaaatgat tgtaagaaag 217080
   tccaaagtgg tttggtatgg tacatttaat aatttaaatc tgattatgct attttccctt 217140
   ttggtttaac atgtatcaaa caaacaaaaa gtcatgtcct ggtcatatgg tgccttagat 217200
   ttagggaaaa aaataaaaat agaaaataaa agagaggaaa agagatacct caatcctttc 217260
   atcccactgt gcttaactga aagaactgca tttgataact ttactttgct atcgtttagg 217320
   agactctcct taatttgatt ctgttcaact tatccagatg ctatttaaca catgtcttct 217380
   gtagaagctg tcttacttcc cacactccct cccactttta tacattagtc aaaccattac 217440
   ctcatccctg aaatgacctc tctccctgaa tgcctaagtt tacctactcc tcaagacctc 217500
   ctcctatttt atttatatta tgcttttact ttttaattca tgatgttcag tgtgaaaaag 217560
   ttgtaagatt aaaaacatta atttccattt taaggaattt atagtattct aatcggtaaa 217620
   acactttcaa tacaaactga tatattatac aaacaataat atagccttat acatgtatac 217680
   tattagtatt ctaattggtt aagcacactt tcaatataaa ctaatgtatt aacatagctt 217740
   atacatgtaa tatatatata aaatgtataa ggctatgtta taattattaa tactatatta 217800
   aatgtattaa gcaaacataa aatagcctta tatgagtaaa aagtagaagc gtctggaggg 217860
   aataggtgaa attcagagag gaatgtttaa caattttgta gaatgggaaa ttaggagatt 217920
   gggaggagaa gttcaggaag aattcacata actgcaatga tcctcccttc tgatactctt 217980
   atctgcccta tcatttgtga catcttatac attggacccg ccattttctc tgtgtacttt 218040
   atctttgtaa tgagattgtg aattcatgtg aagagcagac atgagttacc tattttctct 218100
   atttgacaga atgcgctgca gagttcctat catttttctt ttcttttctt ttttttgaga 218160
   tggagtcttg ctctgtcacc caggctgtag tgaggttccg cgatctcgga tcactgcaac 218220
   ctcaacctcc cgggctcaag agattctcct gccttagcct cccaagtacc tgggattaca 218280
   gacacgtgcc acaacaacag gttaattttt gcacttggag tagtggtggg gtttcaccac 218340
   attggccagg ctggtctcga aatcctgacc tcaagtgatc tgcccatctt ggcctctcaa 218400
   agtactggga ttacaggtgt aagccaccac gcctggtccc attttgttaa agttttgaca 218460
   ttttctgaaa tctggggtct tgacactaat ccattttcag caattgaaat aaaaaaatta 218520
   cttgatgaca gggaaggcaa taaatattat taaccaaagt attcttctaa tgcactagaa 218580
   aatgatggga agggcagaga atagtctcaa aaacttttat caactagaac tgtgtgatgc 218640
   aaatgctcag cttcatattt gcctcaaatg aagctgagct attctctttg ctttcccatg 218700
   tagggagctg gacttggact acccacagcc tttggtcggc tgatggctgt ttaatcacaa 218760
   acaaagagat tccaggacaa gtaacaatag cttttgttta aaaaggactt catatgtcac 218820
   aaaattcatt atcacctgtg cattggttag ctcaggcttc cataacaaaa tacttcagac 218880
   tgtgtggatt aaacaacaga tcttatttta tcatcgttct ggaggctgta tatccaagat 218940
   tagcctgcca gagtggtaag ggtctggtaa gggccctctt cctggcttgc agatggtcaa 219000
   cttatcactg tgtcctcaga tggccttttc tctgtgtgca cacagaaaag agagagagag 219060
   ggtgagatag acagagagtt agatctctgg tttctattct cataagaaca cttaattact 219120
   ctcttatggt ttccatttct aaatacagcc acactgggag ttagggcttc cctatgtgac 219180
   ttttggggag acacagacat tcaatctata acaacccgtt accacctgga atcctcagat 219240
   tagtaaagaa ggaaggacaa gaattaatat aaccatttgc caatgaagca ctgaatatca 219300
   gggagcttac agattgttcc aaatcataga agcagcaaat cttagaattt ttgaaaccag 219360
   aagttgtgtg caagtgtttc ttccccatgt ttctgtccat tactgatgtt ggtgccaact 219420
   tggagttcaa tgcagaagtc ttgtggaata tgagggtagg aaagctgggt tatgtgcatt 219480
   ttagaatgaa aatgtgaacc atctagaatt ttatctttat cttggctttt aaaaaaaaga 219540
   attttccaga tgcacttaat gctctaggag gtaggcagag gattagaaaa gaagaataag 219600
   acaaagttga atgataatga gcttcagggc atcagaataa gacctctgga tctgaatgtt 219660
   tgttatagga tactttttag aaaaaaaatg tgttatttta aaccatgttg tttgtcatag 219720
   tgaaaggatt atttatattc attacttgtt aacattggtc ttttaacgtt tttgtagctt 219780
   cctgtaagca ttcatgaaca acaaaaatgt tttcttttaa gagagtaaga aacaaaaagg 219840
   acatgcctat aattcttatt ttgaaataag ataaaattac ttagatacaa ggagatattt 219900
   tgggagaaga tgtcttttac tacttaataa aatttacttt cttcctcaaa gataagaaaa 219960
   caacagtaat aggaaccaga acaaaacaac cttcttgatt tctaagaaat tgaattaatt 220020
   ttcccttcaa tgataatctt tttcatcccc ttcttttttt tttttctttt ttttcttttt 220080
   tttttaacaa gacctagcat cagctttggt agagcaactg actaaggagg tacacacctt 220140
   tctttgaagt taggaccttc tggctttccc taggtcatga agacatgtca ttttgcccag 220200
   cacctacttc acctggctca gaaggaggcc aatgcacaca ctctgagtgc ttatctcact 220260
   ttccctccat tctgcatatt caggcactgt tcttagcaac acagcctttt ccacaaggag 220320
   agttgcaaca catttttctc aggaggaggg agagggaagt cgactttcct actacttgct 220380
   taaaatactc aattgctccc cagtgctcat gggatcagat acccactttg taaacttctc 220440
   caggaggttc tgtgggggcc tgccttctct ccctccccag cctccatttc atcgcttccc 220500
   tattcacatt atatgcccta cctcagcaaa agccaggctg tttcttgact ttttgctggc 220560
   tgctttgtgt ttttgttttt gttttggtaa aaacacataa tgtcaaattt accatcttag 220620
   caatttctaa gtgcacagta cagtattgtt aactatatgc acatcgttgt gcaacaaatc 220680
   tataaaaatt ccatcttgca acactgatat gatctggctg ggccctcacc caaatctcat 220740
   attgaattcc catgtgttgt ggggtgaaac tggtgggagg taattgaatc atgggggcag 220800
   gtctttccca tgctgttctc atgatagtga atatgtctca tgagatctga tggttttaaa 220860
   aataggagtc tccctgcaca agcgctcttt tcttgcctgc tgccaatgtg agatgtgcct 220920
   ttcactttcc accatcatta tgaggcctcc ccagccatgt ggaactgtaa gtccaataaa 220980
   cctcttcctt ttgtaaattg cccagtctcg ggtatgtctt tatcagcatc atgaaaacag 221040
   actaatacaa acactaaaat tctatgccca ttgagcatca aatttccttt tcaccctttg 221100
   ctatcccctg gcaaccatca ttctattttc tgtttctaag agcttgacta ctttagatac 221160
   ttaagtatct catatgagta ggattatgca gtatttgtct tttagtgaat ggcttatttc 221220
   acttagccta aggtcttcaa tatttatcca cattatagca tatgacagga tttccttctt 221280
   tttcaaatgt aaaaaatatt taattacatg tatataacat ataccacaat ttgcttatct 221340
   attcatccat ctatgaacat ttaggttgct tccacctctt ggctagtgtg aataatgttt 221400
   ccatgaagat ggtatgcaaa tatatctttg agactttgtt gtcaactatc ttggataaat 221460
   acccagaagt gggattgctg aatcatatgg tagttctatt ttcaattttt ggaaaacctc 221520
   catacaattt ttttatagca gctgtaccat tttgcattcc tacaacagca tgcaaatgtt 221580
   ccagtttctc cacatccttg ctaacactct ttgtcttttg tttctgaaaa tagccaccca 221640
   gacaagtgtg aagtggtatc tcatggtttt agtagcattt ccctggtgat taataacatg 221700
   gagcatcttt ttatgtattt cttcaccatt tgcatgtctt tatggagaaa tgtctattca 221760
   aagacaaaaa acaaaaaaca aacaaacaaa aacaaaaaaa cacactaaat taattaatta 221820
   attaattaat taaaaaatta gccaggtatg ctggcatgta ccttgggagg catgctgatg 221880
   tatacctaat cttctcggga ggctgaggta gaagaatccc ttgagcctgg gagtttgatg 221940
   ctccagtgag ctatgataat gccactgcac tccagtcgtg ggtgacagag caagttcctg 222000
   tcacaaaaag aaaaacaaaa actttaaaaa acttatatac tttgttattt ttaattttaa 222060
   aataattcta tagctttttt tgtgtatgtg gttaccttgg agcttacaca aatatcttat 222120
   tgtattacag tctatttaaa gccgatagaa acttatattc aattatgtag taagactaca 222180
   cttcaatttt ctcccataca ctttgttatt tttatcatga tttatattta ttcatattat 222240
   gtacctttca acatattttt agttatagtt attttaatac atttttcttt taacttttat 222300
   acaagtatta gagcaattta caatgattac agtaacagta ttatgtactt gtctacatat 222360
   ttacctttgc caacgagttt tatattttcg tatactattg tgttgctatt ttgcatcctt 222420
   tcatttcaac cttaaggact cccttttgcg tttcttttaa ggcaggtttt gtgatgatga 222480
   actccttcag cttttgtttt tctgtgaaag tctctatctt ccctttttgt tttaaagaag 222540
   agtttttcca gatgtagtat tcttgattat cagtttcatt tttttctttc agcactttga 222600
   atatattatt ttactccttc caacccaaaa agtttctgct gaaatgtcta tttatagtct 222660
   catggaagtt ccttgtatat gacaagtcac tctttttctc ttgccgcttt cagaattctt 222720
   actttgtctt ttacatttga caatttgatt ataatgtgtt tcgatgtgca tgtattcatc 222780
   ttatttcgtg tctctgggct tctgggacct ggaacttact tctacagatt tgataagttt 222840
   tagtcattat tacattaagt cacttttctg gttctttgtt tcttcttttt ctgggacttc 222900
   agaatgtgtg tgttggtcaa cttgatggtg tcccataatt cctgtaagtt ttcttcactc 222960
   ttttgcagtc tttttgtctt tatgctcctc taaattattt ccagtgatct gtcattggat 223020
   ttgctaatcc tttcttttgc ttggtctagt ctgctgtgga acctctctag taaatttttc 223080
   agtgtacgtt gttcagccca tgatttctgt ttggtacttt ttaagatttc ccatctcgtt 223140
   gttgaaattc tcactatatt tttgtgtcct tgatgagcat cttgtaatta tttttaatta 223200
   tctattggat aaatcatgta aggttggttt ctgtatgttt atcttgttcc tttgtttaga 223260
   acatttttac ctgattcttc attttttctt gaccctctat gttggtttct gtatattaac 223320
   aaagcaagca cctctcccag tcttcatgga ctggttttat aaaagagaac actttcacca 223380
   acaaatacag ccaaaaataa tggtggccta taataattct ttccctcctg aggaggaatt 223440
   aggcagctgt gatttttgtc catttattct gtgttgagcc agagagaggg tagctatggt 223500
   ctacaagccc aaaacaccat tttcatactt ctctgagcag cttgactgtg ctgggcctgc 223560
   ttaagtcagc actggttaga aatgctagtt attttggcat ccatggagaa aagttagggt 223620
   gctggacaca tggataaatt ctttcctttc ctatggagaa tctttcagct tgttttttaa 223680
   aacctacttg ctatgtgctg agcaaggggt ataatcactg gcatctacca ggctaaactg 223740
   ttgccaacat tctcccctgg gtggttggac tgtgccagac ccatcacagc cccaatattc 223800
   ataggacagc aaccagtttg ctgagagccc ccttgcagaa gtggagtgct gaacacacaa 223860
   accaatctct tctctccctt gggtgaagct agaaggaagg gcatatgttc ttgatccaat 223920
   ggcactgtgc aagggaggga tttctggtta gaaagtatcc tgaatttccc tactagcttc 223980
   gacaaatctg gttaagcttt cttctgaggt gcaggagtct tttaattcgt ttgtgattac 224040
   tcacagagtg ggctgatttt tgaattgttg cttaatcagt gtgtttgtgg gagaaaatag 224100
   gaagtccagg tcttcctatt ctgccactgt gctgatgtca ccttttgctt ttgtcttggt 224160
   aaccaaaaag aaaataccta tagaacataa acattataaa aaaaaaaaaa aagaaaaaaa 224220
   agaaaaggtg acatcagcaa gatggccgaa tcagaagtcc tggaccctat ctcttttctt 224280
   ctgggacttt tataatggat atacttgttt acttggtggg ataatataag ttttttattc 224340
   tttctgtctt tattgttttt ccttctgatc cccttggtca ataagttcaa ataacttgtc 224400
   tttgagttca ctgattcttc taataaatga agagtgtttt tgaaactctc tagtaaagtt 224460
   ttcaattaag ttgttctatt cttcagcttc agaatttcag tttgaatctt ttttgtagtt 224520
   tctaactcct tattgatact ctcattctgt tcatgctttg tttggttgtc cgtctattta 224580
   tcttgtagct cattaagatt ctttaagata ttttgaatta tttttttaga aaattcacag 224640
   agctctcatt ctttaaatat taatttttag agatattttt tcttttgatt gagccttatt 224700
   tccctgtttc ttcatatgct ttttttggcc aggatttact tatttggaaa aaaagatcta 224760
   aaattctcag gccataagga ctggcttcat gcaggggaag atctttacta attattctag 224820
   tcagagattc tttgggcctc tcaaatcttt tctaaaaatg tgtcttctct gtgcttgttt 224880
   gtgtaatttc ctaattacaa tggtatatag gattttaaga aaatctcata atttcttgct 224940
   ccttccactg tctgtctgtg ctgctgctgg ttctctggtg ttgcaacaaa ccactccttt 225000
   gttctcagtg gctctcaggc atccaatcta ttgcagctca tcatcaatgc tcttcaccag 225060
   gtgaaacaga aaccagttcc atagacagct tcactgaaaa gtcagaaaat tttatgtaca 225120
   ctcacactcc actcttctat ttccctctca aggaaaaatt tgtgagttgg gcatgttctc 225180
   ctgattgcac taattgtgct ggcttggtgg gagggctgtt agtgaaatgc agcaggtttt 225240
   cttactcatt tcaatatagc tattgtttgc tctggggtat tgtgacttct taattatttc 225300
   cagagttctc ataaagactt tttgaacata tattattgtt aaatttgtat ctctgttgga 225360
   taatgactct ggggttctta atctactatc ttgctgatgt aacccttttc aaaacttcag 225420
   acttttaaaa cactttttat tgtgataaat tggagacatt gtatcattta cagtgcataa 225480
   aaatttagaa caacccataa ttattctctg ttttagtatc ttccctttca atatttttcc 225540
   atgcttttta taatgttgct attataatga acaagcttta tttaacatta tattctaagc 225600
   actcaccgta aaattataaa ctactcatag aattatttta gaagttttat gaattctgct 225660
   ctgagagcat tcacctacat atatatatat atatatacaa ctttttgctg ttatcaataa 225720
   tagctaatat tattgctaat gatacgattc tatacctaaa aatctctaaa gacttcacca 225780
   aaaggctcct aggcctgatg aaccatctca atgaagtttt gggctaccaa attaatgtac 225840
   aaaaattagt agcatttgca tacaccagta aggttcaagc tgaaagtcaa atcgataacg 225900
   caaacacatt tacaatagcc agaaaaaaat aaaatatcta ggagcatatc tagccaagga 225960
   ggtgagacct ctcaaagaca accacaaaac actgctgaag gaaatcatag atgacgatat 226020
   cattaaaata tctacactgc ccaatctata ctttcaatgt tatatgtacc aaattaccaa 226080
   tgttattttt taaaaaagaa ttagaaaaaa gcattctaaa atttatatgg actcccaaaa 226140
   gagcctgaat aagaaaagca atccttagca aaaaaaaaac aaagctgaag gcatcacatt 226200
   gcccaacttc aaaatatact ataaggccat agtaatcaaa acagcatggt actgttaata 226260
   aactagacac atagaccaat gaaacagaat agagaattca gaaataaagc agcacaccta 226320
   gaaccaactg acctgtgaca aaatgggcaa aaataagcaa tgaggaaatg actccctatt 226380
   caataaatga tgctgggaaa actggctaac catttgtgga agaatgaaac tagaccactc 226440
   cctctcacta tatacaaaaa ataactcaag atatattaaa gacttaaata taagacctca 226500
   aactataaca attattgaag aacacctaca aaatactctc tcaagtatca gcataggcaa 226560
   agagtttatg actaagtcct caaaagcaat agcaaccaaa acaaaaactg ataattggga 226620
   cctagttaaa ctaaagagct tctgaacagc aaaagaaact atcaacagag taaaaaggca 226680
   ccctacagaa tgggacaaca tatttgcaaa ctatgaatgt ataaaggact aatatgcaga 226740
   atctataaag aacttaaata aatcaacaag agaaaaagaa agtacaccat taaaaagtgg 226800
   gcaaaaagta taaaaagtat tctcaaaaga agacataaaa gtggccaaca gacatataaa 226860
   aaatgctcaa catcactaat tagagaaatg caaatcaaaa ccacaatgag aataatacct 226920
   cacaccaagt gacacgcaca atcaggatgg ctatcattaa aaagtgaaaa aataacagat 226980
   gatggcaagg ttgcagagca aagggaatgc ttatacatag ttggtgggaa tttaaatgac 227040
   ttcggtccct gtagaaagca gtttagggat ttctcaaaaa actaaaaata gtactacaat 227100
   ttgacccagc aatcccttta ctggttatgt aagtggaaaa taaattgtcc tgtcagaaag 227160
   acacttgctc ttacatgttt attgcagcac tattcagaat agcaaagaca aggaatcact 227220
   ggataaagta aatgtggtac atatacacca tggaatacaa tgcaactatg aaaaataata 227280
   aaatcatgtc ttttgcagca acacagctat aactggaggc cattatccta agcaaattaa 227340
   cacagaaaga caaaaccaaa tattgcatat tatcacttat aattgggagc taaactttgc 227400
   ctacacatgg acataaggat ggcaacaaca gtcactcggg acttctaaag gtaggaggta 227460
   ggtggggaga agagttgaaa aacttcctat tgaatgacaa gatcagtaga agtccaaacc 227520
   tcagcatcac acaatatact ccataacaaa cctacacatg tgccccctga gtctgaagta 227580
   aaaaaattaa aaactaatat ttgtataaca gtatctctgt gttaacatct accaagaatt 227640
   ctgtctttta tctcatattc ctatatgtgg aagatttcat ccatccaaga gtggtaagca 227700
   attgaaatat gatcatgttt acatagaaaa actagcatat ttaagatatt tttagcttaa 227760
   aagagcagaa cgtcctggtc ttttgaaagc cctgcaagat aatttcaaga agatgtatca 227820
   gcacatttta aaaagataga ccatctagga cagattttga gatctcaaca aggtcactaa 227880
   ttctttatta cacagtatcg ctaactagta aggttgccaa aatggaaata tcaatacgaa 227940
   catccataat cagtaacaaa aaataattga gttgattgtg gagcttttca ttgaagtcag 228000
   aatactggaa ttatgataaa ctaatcgact atagataaaa attatccatc tatctatttt 228060
   gtgtacgtag agttctgatg cttgtaaatt cttcaaggga gcaaaatgga catattcaca 228120
   agaaatataa aagaatattt cctctattaa agaaaagttt tgacatttct ccctgtacta 228180
   tatttacaaa gcaaatgcaa attattaagg attaaaaaaa gttttaaaat agaaactact 228240
   atcatatgag tgtatattaa atcctttgct acaaaagaga atatgcatat tctaaaacag 228300
   attggaagat taaaatattt accactgtag aaagcaaatt tagatttagg gaagtagaga 228360
   tcttattgac cgaaattatt gtagtaggaa gaatgtttca actacaggat ctgaaaatgt 228420
   ccaaaagtca cacagaaaaa aactcttctt ttatatggag gggtaaagta gcctagaaag 228480
   aataaggtat ggggcagtgg ttgagcatgt catgatctac agttattcaa cgaatgattt 228540
   atctgaagct cagcctgctc ttgacagggg ttgttaagga gaagttgttt catattacct 228600
   tgcccaagag tggtccaaag ttgaggaact tgaaggaagg aatgaagctt aacttaaagg 228660
   gtgataaagt caaaataaca atcacatttc atatcgatca gtggggcaaa aatctcagga 228720
   acccttgata agacaaaaaa tgggaatttg atgggtctat atctggcttt tttctgtgta 228780
   aacaggggac tctcattaaa tttaatgaag tcccacagga aaatatgctt ctttgcagta 228840
   agctgttacc aaggaaacaa aggtttgggg gataaagttc cttaaacttt gcaattttcc 228900
   aggattatac agttggagta aatttcaaca ttgtcactaa aaaaaaatcc ttgtctagac 228960
   ctatgttttg aagcattttc tctatgttat tctccagtat ttcatagttt cagatcctaa 229020
   atttaagtcc ttaatctatt tgagttgatt tttgcatgtg gtgagagaga gagagagaga 229080
   tctagtttcc ttctttgtca cgtggatatc aagttttccc agaacaattt attggggaga 229140
   cagctttttc tccagtagat attcttggtg cttttgttga caattagttc atggtaaata 229200
   tctggattta tttctgggtt ctttattttg ctccactggt ctatgtattc gtttttatgc 229260
   cagtattatg ctgtgttagt tactatagct ttgtactatg tttttaagtc agatagtgtg 229320
   attcctttag ctttgttctt tttgctcaag gctgttgtgg ctatgtgggt ttttttcaaa 229380
   ctaaaagctt ctgtaaagca aataaaacaa tcaagagtga agagacaaca tatagaatga 229440
   gagaaaatat aaacaaataa tatgttttat aagagatcta tatccaaaat atataaggaa 229500
   cttctataat gcaacaataa aaaacaagca acccagttca aaaaataaaa ataaaataaa 229560
   gaacttgaat agacatctat taagagatac aaaagatata caaatagaca ataagcccat 229620
   gaaaagatgc tcaacatcat taatcattag gaaaatgcat atcaaaacca cagtgagata 229680
   ccacttcact accattagaa tgatgatatg ggttggctct gtgtccccac ctaaatctca 229740
   tgttgaatgt aatttccagt gttggaggag gggcctggtg agaggtgatt gaatcatggg 229800
   agtgaacttc ccccttgctg ttctcatgat agagttctca caagatgtgt tttttttttt 229860
   aaatgtgttg cacctcctgc cacccctacc ctgctggcca tataaagatg tactggctgc 229920
   ctctttactt tccaccatga ttgtaagttt cctgaggcct ccccagaagc agaaacctgt 229980
   tcagcccata gaactgtaag ccgattgaat ctctttcctc tataaattac tctgtctcag 230040
   gtagttcttt ctagcaatgt aagaatgaac taatacagat gactattaca aaaacaaaag 230100
   caaaagtaaa acagaagtaa taagcgttag caaggatgcg gagaaattgg aattcttgca 230160
   cattgctggt gagaatgtaa aatggcatag ccactgtgaa aaacagcatg gcaattccaa 230220
   aaaacaacaa aaaaacaaaa caaaaaaatt atcgtaacca tatgatccag taattccact 230280
   tctgggtggc aggttctagt ttcaatcacc tcttacctaa gggcaagggc actatgcatt 230340
   cctgacaaca tatgtgagca tcctcttccc tgtccagcag cacattctct tggttatatc 230400
   tttcctagat ctattctgat cctaattctc tccactaagt cacttcatag aggagagaaa 230460
   taacaagtat tccataggcc aactccatta ctacaggaca gaatttttca ttctggactt 230520
   caccaggcct gcctgtcttc agcacagtgc tcagcctgaa atgaaggcct caacagatat 230580
   tggtataccc atgttcattg tttacaatag ccaaaagttg gaagcagccc atatgtctac 230640
   taatggatta gtgggtaaac ataacgtggt acataaatat agatggacta ctattcagct 230700
   ttaaaaggaa ggaaattcag acacatacta caatatggat aaacattatg ctaaatgaaa 230760
   caaaccagtc acaaaaggac aaatactgca tgattcaaac ccatatgtga tattcaatgt 230820
   agtcaaattt atagagagaa aagtagaatg gtatttgcca ggagctgggt gatagggaga 230880
   aacagagagt taatgtgagt agtttaagct ttgcaagact aaaatgttct gtgggttgat 230940
   gatggtaatg gtttcacagc aatgtgaaag tacttaatgc tggtgaaatg gctaaaatag 231000
   taaattttat attatgtata ttttaccata agttggacaa aataaatttt tttttgaaaa 231060
   ccaaaactga caaatttttt tcatgtttga ttgggggaaa aagatacaaa tagtaccttt 231120
   attatataag aaaggagata taaatttaaa tactgtggaa tttttaaaac atgaaagatt 231180
   atcatataaa atgacattac aacatttgaa aatacaggtg aaacagataa agaaaaatga 231240
   ttaaaattca cttaagaatg tatgaaagct ctgagtaggc taatatgctt tagagaattg 231300
   aatatgtagt aaacatcact aagtaaagaa aaaacctgca taggtttgtg agtaaattaa 231360
   aaatttaaag caaagaaagc aaatatattg acaaaaatgc tttcagcaca caaataagtt 231420
   cttattataa aacattttat aaacctaaaa atactttttc attaaacata ttttctagct 231480
   cagtttacaa gtctcgcatt tttataaaaa aatgaacaaa agtagcacac aaaatgtaac 231540
   tgtgaggtta atttgtttta aagggttata atgaagaaat tatatgataa tatcaagaca 231600
   cacccaagct attattaata aattctcatt aaaatataaa aatcaaaaag agactgctgg 231660
   caagatggcc aaaaaggaac agctctggca gctccagcga gaccaatgca gatggcaggt 231720
   gattgctgcg tttccaactg aggtaaccta ttcatctcat tggaacttgt taggcagtgg 231780
   gtgcagcaca cggaaggcaa gcagaagcag ggtggggcat tgcctcatct aggaagtgca 231840
   aagagaggga ggcatccctc ccccagccaa gagaagccat gagggactgt gctattcagc 231900
   ccagatacta tgcttttccc acagtattta ccacagacca ggagattccc tcatgtgcct 231960
   acaccatcag ggccctgggt ttcaagcaca gaactggaaa actgtttggg ctgacactga 232020
   gctagctgca ggtttttatt attattgttg ttattttttc ataacccagt ggtgcctgga 232080
   acctcagtga gacagaatca tttattcccg tggaaagggg actgaagcta gggagccaag 232140
   tggtctcact cagggggtcc cactcccaaa aagcccagca agctaagaac cactggcttg 232200
   aaattctcac aggaagcaca gcagtctgaa gttgacctgg gatgatcgag cttggttggg 232260
   ggagaggttt ctgccattat ggaggcttga gtaggtggtt ttcccctgac aggactaaaa 232320
   aggcctggaa gctcagactg ggcagaattc aacatggtgt ggcaaagcgg ctatggccag 232380
   actgcctctc tagattcctc ttcacttggc aagacatctc tgaaagaaag gcagcagctc 232440
   caatcagggg cctatagata ccactcccat ctcactggga cagagcgcct gggggaaggg 232500
   gtggctgtag gcacaacttc agtggactta aatgttcctg cctgctgact ctgaagagag 232560
   cagcagatcc tgacaaggag ggtttttctc agtacagcgt ttgagctcta ctaagggaca 232620
   gacttccttc tcaattgggt ccctgaccgc caaacctcct gactggaaga gacctcccaa 232680
   caggggttgt cagagacttc atacaggaga gctctggctg gcatcagcct ttgcgtcaaa 232740
   gcttccagag gaaggagcag gcagcaatct ttgctgttct gcagcctcca ccactggtga 232800
   tatccaggca aatagggtct ggagtggacc tccagcaaac tgcagcagac ctgcagaaga 232860
   ggggcctgac tgttagaagg aaaacaaaca aacagaaagc aataaaatca acatcaacaa 232920
   aagaaccccc aaaaagaaac cccatccaat ggtcttcagc ctcaaatacc aaaggtagat 232980
   aaatccatga agatgaggaa aaatcactgg aaaaatgctg aaaatttaaa gaaccagaat 233040
   acctgttttc gtccaaatga ttgcaactcc tctccagcaa gggcacaaaa ctggacaaga 233100
   aatattttga ttaattgaca gaagtaggct tctgagctaa aggagcatgt tctaacccaa 233160
   tgcaaggaag ctaagaacct tgacaaaagt tacaggaact gctaactaga ataaccagtt 233220
   tagagaacaa aataaatgac ctgatggagc tgaaaaacac agcacaagaa cttcctgaag 233280
   catacacaag tattaatagc caaaatgatc aagcagaaga aaagatttca gggattgaag 233340
   atcaacttaa taaaataagg agtgaagaca agattagaga aaaaagattg aaaaggaaca 233400
   aacaaagcct ccaagaaata tgggactatg taaaaagact aaacctacga tagattgggg 233460
   tccctgaaag tgagaagaag aatggaacca agttggaaaa cgcacttcag gatattatcc 233520
   agaacttccc caacctagca agaaaggcca acatttaaat tcaggaaatt cagagaacac 233580
   cactaagata ctcctcgaga aaagcaacct caagacacat aatcatcaga ttcttcaagg 233640
   ttgaaacaaa ggaaaaaatg ttaagggcag tcagagagaa aggtcaggtt acctacaaag 233700
   agaagccagt cagactaata gtggatctct ctgcagaaac cctacaagac agaagagagt 233760
   gggaaccaat attcaatatt cttaaagaat tttcaaccca gaatttcata tcaagccaac 233820
   taagtttcct aagtgaagga gaaataaaat tctttacaga caagcaaacg gtgaaggatt 233880
   ttgtcaccac caggcctgtc ttacaagagc tcttgaagga gcactaaata tggaaaggaa 233940
   aaactggtac cagcaactgc agaaaaaaca aaaaacaaac aaacagaaca ccaaaatata 234000
   aagaccaatg acactatgaa gaaactgcat caactaacag gcaaaataac aagctagcat 234060
   cataatgaca ggatcaaatt cacacataac aatattaacc ttaaatgtaa atgggctaaa 234120
   tgccccaatt aaaatacaca gactagcaaa ttagataaag aatcaagaac catcggtgtg 234180
   ctgtattcag gagacccatc tcacatgcaa agacatacat aagctcaaaa taaagggatg 234240
   gaggaatatt taccaagcaa atggaaagca aaaaaaaaaa aaaaaaaaag caggggttgc 234300
   aatcctagtc tctgataaaa cagactttaa accaacaaag atcaaaaaac acaaagaagg 234360
   gcattacata atggtaaagg gatcaatgaa actagaagtg ctaactatcc taaacatata 234420
   ttcacccatt acagtaggat ccagattcat aaaacaaggt cttagagacc tacaaagaga 234480
   cttagacttc cacacaataa aagtgggaga ctttaacacc ccactgtcaa tattagacaa 234540
   atcaacgaga cagaaaatta acaaggatat tcaggaattg aactcagttc tggaccaagc 234600
   agacctaata gacttctaca gaagtcttca ccccatatca acataatata ccttcttcct 234660
   gcaccaaatc acactattct aatattgacc acataattgg aagtaaaaca ctcctctgca 234720
   aatgcaaaaa gaacagaaat cacatcaagc agcctctcag accacagtgc aatcaaatta 234780
   gaactcagta ttaagaaaca cactcaaaac cacacaacta catggaaatt gagcaacttg 234840
   ctcctgaatg actactgggt aaataacaaa attaaggcag gaataacaaa ttttttgaaa 234900
   ccaatgagaa caaagagaca atgtaccata atctctggga cacagctaaa gcaatattaa 234960
   gagggaaatt tatagcacta aacacccaca tcagaaagtg agaaagatct aaaattgaca 235020
   ccctaacatc acaattaaaa caactagaga agcaagagca aacaaattcg aaagctagca 235080
   gaagacacga aataattaag atcagagcag aactgaaaga gatagagaca cgaaaaactg 235140
   ttaaaataga tcagtgaatc caggagctgg gtttttgaaa agattaacaa aatagataga 235200
   ctgctggcca gacttataag gaataaaaga gagaagaatc aaatagacac aataaaaaat 235260
   gataaaggag agatcaccaa tgatcccaca gaaatacaaa ctactatcag agaatactat 235320
   aaacacctct acacaaataa actagaaaat ctaaaagaaa tcgataaatt tctggacata 235380
   cacaccctcc caagactaaa ctaggaagag gtcatattcc tgaaaagacc aataacatgt 235440
   tctgaaattg aggcagtaat taatagccga ttataaaaaa aaagcccagg accagatgaa 235500
   ttcacagcag aaatctacct gaattataaa gaagagctgg taccattcct tctgaaacta 235560
   ttacaaacaa tagaaaaaga aggactcatc cataactcat tttatgaggc cagtatcatc 235620
   ctgattccaa aactgggcag aaacttcata caaatatccc tgatgaacat cagtgcgaaa 235680
   atcctcaata aattactggc aaacagaatc cagcagcaca tcaaagagct tatccaccat 235740
   gattaagaca gtttcatccc tgggatgcaa ggctggttca acatatgcaa attggtaaac 235800
   gtaatccatc acttaacaca accaatgaca aaaaccacat gattttctca atatatgcag 235860
   aaaaggcctt caataaaatt caacattcct tcatgctaaa aactctcgat aaactaggta 235920
   ttgatggaac acatctcaaa ataataagag ctacttatga caaacccata gccaatgcca 235980
   tactgcatgg acaaaagctg gaagcatttc ctttgaaaac cggcacaaga taaggatgcc 236040
   ctctctcacc actcctattc aacatagcat tggaaattct ggccacagcc atcaggcaag 236100
   agaaagaaat aaagggtatt caaataggaa gagaggaagt gaaattgttt ctgtttgtag 236160
   acaacatgaa tgtatattta gaaaattcca ttgtctcagc cccaaagttc cttaagctga 236220
   taaacaattg cagcaaagtc tcaggataca aaaccaatgt gccaaaatca caagtaatcc 236280
   tatacaccac taatagacaa gcagagagca aaatcatgag tgaactcctg ttcataattg 236340
   cttcaaagaa aataaaatat ctaggaatac aacttacaag agatgtgaag gacctcttca 236400
   aggagaacta tgagccactg ctcaaggaaa taagagagga cacaaacaaa tggaaaaaaa 236460
   gttccatgct tatgtgtagg aagaatcaat attgtgaaaa gtataagtaa tttataggtt 236520
   caatgttatt cccatcaagc taccattgat tttcttgaca gaactagaaa aatctacttc 236580
   aaatttcata tggaaccaaa taagagccca tatagccaag aaaatcctaa gcaaaaataa 236640
   caagctggag gcatcacgct acctgatttt aaactatact acaaggctac agtaaccaaa 236700
   acagcatgat actggtacca aaacagagat atagaccaat ggaacagaat agaggcctca 236760
   gaaataacac cagacatcta caaccatctg atctttgaca aacctaacaa aaacaaggaa 236820
   tggggaaagg attctctatt taataagtca ttctaggaaa actggctagc catatgcaga 236880
   aaccagaaac tggacccttt ccttatacct tatacaaaaa ttaactcaag atggattaaa 236940
   gacttaaatg taaaacctaa cactataaaa accctagaag aaaacctagg caataccatt 237000
   caggacatag gcacggacaa agacttcatg aacaaaacac caaaagcaat tgcaacaaaa 237060
   tccaaaattg acaaatgtga tttaattaaa ttaaagagct ttgctcagca aaagaaacta 237120
   tgatcagagt gaagaggcaa cctacagaat gaaagaaaat ttttgcaatc tatccctctg 237180
   acaaaggtct aatatcctga atctacaagg aactaaaaca aatttacaag aaaaaaacaa 237240
   acaagcccat caaaaagtga gcaaagtata tgaacagaca cttctcaaag aaagacattt 237300
   atatggccaa gcaaacatga aaaaaagctc accatcactg gtcgttagag aaatgcagac 237360
   caaatccaca gtgagatacc atctcacacc agttaaaatg acaatcatta aaaagtctgg 237420
   aaacaacaga tgctggcaag gatgaggaga aataggaatg cctttacact gttggtggga 237480
   gtgtaaatta gttcaactat tgtgagagac agtgttgcaa ttcctcaagg atccagaagc 237540
   agaaatacca tttgatccaa caatcacatt actgggtata taccaaagga ttataaatca 237600
   ttctactata aagacacgta cacatgtatg tttactgaag cactatttac aatagcaaag 237660
   acttggaacc aacaaaatgc ccatcaatga tagactggat aaagaaaatg tggcacacat 237720
   acaccatgga atactatgca gccatgaaaa actgagttca tgtcctttgc agggacttgg 237780
   atgaagtggg aaaccatcat cctcagcaaa ctaacacagg aatagaaaac caaacaccac 237840
   atgttcttac tcgtaagtgg gagttgatca atgagaacac acggacacag ggagggtaat 237900
   atcacacact ggggcctgtc agggggtgaa gaacaaaggg atggagagca ttaggacaaa 237960
   tgcctaatgc atgtggggct taaaacctag atgacaggtt gataggtgca gcacaccacc 238020
   acgacacatg tatatgtatg taacaaactt gcacattcag cacatgtatc ccagaactta 238080
   aagtaaaatt aaaaaaatta aaaataagaa agcaaaaaac aaaaagatac tcactgttat 238140
   atttttaaat atgaatgaaa aatataacat tctctaattg caagggttgt ttatcatgta 238200
   tcattttaat gacttaacaa gattagcatc ccatttagaa ttaagaataa gaaagaatat 238260
   tgaagaagat aggaaaagtt atttgcagat gatacgaatg tctattcaaa caaatctagg 238320
   aggatcttca tgttattaaa attaataatt tgtttcagca acgtggccaa aatataagaa 238380
   taaaaacgta aaaagcaatg tatgttttaa agtctgatca gaaaccaatt tgaatttttt 238440
   tcttgaggta catttgcagt agaaacagag atattaaata tctaggaatt atttatagat 238500
   gagatagctg tacatataga tatagacata tacatacaca gaataaaagc ttttcacttt 238560
   aagacttcaa tgtcctttaa aagaacaaaa atatgtctag tttcagactt ttctgaaata 238620
   caaaatgtta taagacagaa aagcaatttg taatggtgtt tacaagatca gatttttact 238680
   gaaaatttta atataacact tacttattat taaaataatt taatgacctc ttcaaaaata 238740
   tacatggcta ttgcctaagc catacactct tctaccttta aaaaatcact taaaaacata 238800
   ctccaaccag ttgaaagttg aatccagtta atttctcaaa gactgcttag ttaatttata 238860
   taattatttg cctttgaaat tttcttaata tctttttaga atgtttatgt aaacacttac 238920
   aatccaaacg gaagtagtag tacacttaaa acacctgaag caagttcagt gatgcaatta 238980
   tacatgagaa tgcttagaaa attccattca aaatcctcag tgcaagttaa tcctccggaa 239040
   tttgtacaaa gataaaatta gcactatcac actaatgatg tacaaaaaat atttcctttc 239100
   tttggagtca tatgactagc tacattttat ttgcatttac tattaataaa tgacggttct 239160
   tctgctcagc atggttaatt gactggatgc acagtactca tcccctgtta tcagtacaga 239220
   agtgaggata aatgaaaagg aaggaaggga gggaattagc caaagtgatg aatgacacat 239280
   gaatccaagg ggaattggaa cctgatgatg gctttgatac ctacctagga agagggcact 239340
   tgactttagg ttccatggct ttgggtagct ataactgagt tatattcgta aggttagaag 239400
   tcacaaagtt ctacctcatc tctgaaacaa gttctgagaa aaaagtctac caaacagcac 239460
   taggaagtcc caggaatact ttccaactcc caggaacgat ggctggaaat tgtatcacac 239520
   acaaaatatc aggagcccag gcctacagct ttcacactta taaagtaaat ttatactatt 239580
   ggcatggtga atgaatatca agataattaa tgtccacatt ttttgctcac attgcttctt 239640
   aatcttcctc tttcctccta aaattacatt aaaattgttc tgcaactggg aaaccagaag 239700
   gctgaatcat ttttggaaca acagcaagca tgcccactac aggccaggca catcaaggaa 239760
   cctcactggg ggaactcaca ggaataaaat tataaatcaa aggaaaacac aaaacattat 239820
   aacacctttt aaaaatattc aataaaaatt gtaccttata actgaagata aaaccactta 239880
   ggcaatttta aataagtgta tttaaagtgc tcaatgatac agtggaagga tatgaaatta 239940
   cgaagaaaag aacagtgtga aaaaaaagtc aaccagaaaa agaaattttt ttcaaataaa 240000
   tgcaaataaa attatagaca ttgaaaatat aatcactcaa attaaaaaaa tctcagtgga 240060
   tggattaata tctaagaaag aattagtaga tttgaagaga gatctgaggc aatcacccag 240120
   aattaacaca gaggaatatt tagagatgaa agatactgat aagaatttag gagatgggaa 240180
   agataagtag aaaataacca tcatgcataa agtagggttt ccaggaacat actattatca 240240
   ataatgagga aataacattt gaaaacaaaa tgctttcaaa ttttctaaaa ttgcagtgtc 240300
   atataggagg aagtctctgg gtcctgataa gctaattcca catcttggct atcaaatgat 240360
   ggtacagaac ttgaaaagca atgaaagacc tcttaaaagc ttcttaaaag aaaacaccat 240420
   acataaaagg aagaagaaaa tgtgattcac atttttcagc aacaatgtat aagagaagac 240480
   attgaaacat cagcctcaaa gttctgacgg gaaaaaaaaa atacccgacc catgactcta 240540
   tatctaatgg aaactatatt aaacactgtg tctacaatag gaatattttc agaaatagga 240600
   atgaaagaaa atttactaat cggatgcttt acaaaatgta aattgacttg actcacatgt 240660
   cttaaacatc tcccaatttt aataaaaatt aagtaaatct aaattcttaa ttaagttttc 240720
   ataatttatt gtgtcctaga tggctggtta atttaagttt aagaaatctt tatttatcaa 240780
   ctatcaaata tcaaggagtg tacagcatac aaagaaggga ttaaaaaacc atatgagttt 240840
   aatgccctga aagaagacca ctacaaataa cagctaaatt ttttgttggc ttaactatgt 240900
   atgccaggca cttagagtta catttacttt agagttatgc ataaaacttt cttgaggtag 240960
   ctattatttt acagataatg aaactgaagt tgaaagtggt taaaaacttg tataaagctg 241020
   ttaagaatag agacagaatt ctaaaattct aagtaatttt ccctcatgct gttttccatt 241080
   ataattcatt gttttttcat gatgctttga atttataagt tatttatgaa agtttaagcc 241140
   ttcattcata tgatgaccct gataatatag agaaatagaa gagttctcta attcattgat 241200
   gtggaaaata caagaaaaag caataaggag ttttaccaaa gtcacagtgg gcctcctata 241260
   aagttggaat aaaaattcat atttcaagaa attcccatat gggagcttat ttttacagta 241320
   gaccagagtg gtaccttaga attaagagca cccgttgctc acctgtgtgc attgcacaac 241380
   tcttagcatc attgtgcctt gacttggcca cttgtagagg ctcagtttga aagaatacat 241440
   gaatggacca gtcatcccgt caccaacgtt tctttctttt tctttttttt ttctgtactc 241500
   gagttgctta aattttcttt tatatatata tatatatatt ttttttatta tactttaagt 241560
   tctaaggtac atgtgcacaa tgtgcaggtt tgttacctat gtatacatgt gccatgttgg 241620
   tgtgctgcac ccattaactc atcatttaca ttaggcatat ctcctaatgc tatccctccc 241680
   tcaaccctcc tccccacaac aggccccagt gtgtgatgtt ccccttcctg tgtccaagtg 241740
   ttctcattgt tcaattccca cctatgagtg agaacatgcg gtgtttgctt ttttgtcctt 241800
   gcgatagctt gctgagaatg gtggtttcca gcttcatcca tgtccttaca aaggacatga 241860
   actcatcatt ttttatggct gcatagtatt ccatggtgta tatgtgccac atttcttaat 241920
   ccattctatc attgatggac atttggattg gttccaagtt ttgctattgt gagtagtgct 241980
   gcaataaaca tacatgtgca tgtgtcttta tagcaacatg atttatattc ctttgggtgt 242040
   acacccaata atgggatggc tggatcaaat ggtatttcta gttctagatc cctgaggaat 242100
   tgccacactg tcttccacaa tggttgaact agtttacagt cccaccaatg gtgtaaaagt 242160
   gttcctattt ctccacatcc tcttcagcac ctgttgtttc ctgacttttt aatgatttcc 242220
   attctaactg gtgtgagatg atatctcatt gtggttttga tttgcatttc tctgatggcc 242280
   agtgatgatg agcatttttt catgtgtctg ttggctgcat aaatgtcttc ttttgagaag 242340
   tgtctgttca tatcctttgc ccactttttg attttttttt ttcttgtaag tttgtctggg 242400
   ttctttgtag attctggata ttagcccttt gtcagataag tagatttcaa aaattttctc 242460
   ccattctgta ggttgcctgt tcactctgat ggtagtttct tttgctgtgc agaagctctt 242520
   tagtttattt agatcccatt tgtcaatttt gtcttttgct gccattgctt ttggtgtttt 242580
   agacatgaag tccttgccca tgcctatgcc ctgaatggta atgcctaagt tttcttctag 242640
   ggtttttatg gttttaggtc taacgtttac atccttaatc catcttgaat tgatttttgt 242700
   ataaggtgta aggaagggat ccagtttcag ctttctatat taggaaaaga ggaagtcaaa 242760
   ttgtccctgt ttgccgacga catgattgta tatctagaaa accccattgt ctcagcccaa 242820
   aatctcctta agctaataag caacttcagc aacgtctcag gatacaaaat caatgtacaa 242880
   aaatcacaag cattcttata caccaacaac agacaaacag agagccaaat catgagtgaa 242940
   ctcccattca caattgcttc aaagagaata aaatacctag gaatccaact tacaagggat 243000
   gtgaaggacc tcttcaagga gaactacaaa ccactgctca aggaaataaa agaggataca 243060
   aacaaatgga agaacattcc atgctcacgg gtaggaagaa tcaatatcgt gaaaatggcc 243120
   atactgccca aggtaattta cagattcaat gccatcccca tcaagctacc aatgcctttc 243180
   ttcacagaat tggaaaaaac tactttaaag ttcatatgga accaaaaaag agcccgcatc 243240
   gccaagtcaa tcctaagcca aaagaacaaa gctggaggca tcacactacc tgacttcaaa 243300
   ctatactaca aagctacagt aaccaaaaca gcatggtact ggtaccaaaa cagagatata 243360
   gatcaatgga acagaacaga gccctcagaa ataatgccgc atatctacaa ctatctgatc 243420
   tttgacaaac ctgagaaaaa caagcaatgg ggaaaggatt ccctatttaa taaatggtgc 243480
   tgggaaaact ggctagccat atgtagaaag ctgaaactgc aaagacaaaa gccacatgat 243540
   tatctcaata gatgcagaaa aagcctttga caaaattcaa caacgcttca tgctaaaaac 243600
   tctcaataaa ttaggtattg atgggacgta tttcaaaata ataagagcta tctatgacaa 243660
   acccacagcc aatatcatac tgaatgggca aaaactggaa gcattccctt tgaaaactgg 243720
   cacaggacag ggatgccctc tctcaccact cctattcaac atagtgttgg aagttctggc 243780
   cagggcaatt tggcaggaga aggaaataaa gggtattcaa ttaggttttt aaaatatttt 243840
   tgagacttgt tgtgtggtcc aacgtgtcat cagtcctgga gaacgttctg tgtgctgatg 243900
   aaaagactgt gtgttcttca gctgttgggt gaaatgttct gtaaatgtct gatgggtcaa 243960
   tttggtctat ggtgcacttt aaatccaatg tttctttgtt gactttttgt ctcgatagat 244020
   gatctgtcca atgctaaaag taagctattt aagcccccaa ctattattgt attggaacct 244080
   atctctgctt ttatatctag taatatttgc cttatatatc ttggtgtcct gttattgggt 244140
   gcatataaag taatattgtt acattctctt actgaatcaa tctctttatc attatataat 244200
   gttctttttt gtctcttttt atagttttga acttaaagtc tatttcatct catgtaagtc 244260
   tagctatttg ggctcacctt tgaattacgt ttgtgtggat tgtctttttc cattccttcc 244320
   gttttagtct atgtgtgtct ttacagagga aatgagcttc ttgtagacag catttagttg 244380
   agaaatccaa ggcaatgaga tatcatctcg cttgagttaa aaatgtctat tttcaaaaag 244440
   aaaaaaatag caaatactgg caaatatgtg gaaaaaggga actccaattt gctgttggta 244500
   ggaaggtaaa tgagtacagc tattatggag aacagtatgg gagttcctca aaaaactaca 244560
   ggtagaacca ctatatgatc cagcaatccc actacagggt acatatctaa aggaaaagaa 244620
   aacagtagat aaaagggtta cttgcaccct cgtgtgtact gcagcattat tcacaagagc 244680
   aaaaatatgg aatcaccata agtgttcatc aaaagacaaa tgatgcaaaa tgtggtatat 244740
   atacacaatt gaatattatt cagccataaa aagaatgaaa tcttgaaatt tgcggcaacg 244800
   taaatgagca tggaagacat tatattaggt gaagtaagac aaacagaaag acaaatacca 244860
   cattttctca ctcatatgta ggaactaaaa aattaatgtt atagtagagt gttgaatagt 244920
   ggttaccaga gggtaagatg gacaggagag atggagaaat aaggagaggt taggtaatgg 244980
   gtaaaatatt acagttagac aagagaaata agttcgagtg ttctacacca cagtaggatg 245040
   actatagtta acagtaattt actatatatt ttcaaatagc tagaagaaag aattttgaat 245100
   atttgcaaca taaagaaagg ttaagtattt aaggtcatag gtatgataat agccctgatt 245160
   tgattattaa atattatatg tattaaaata tcacaatata cccataaata tgtacaatta 245220
   ttatgcatca attaagaaaa gaaggaaaaa tctaagggaa aacaaaattg aggtggtaaa 245280
   taatatatta aattttttgt gtatataatt ttaaaggaaa atgatttcag ggtgtgtata 245340
   cataaaatat cacttggtat actttaaatg tgtgcagttt atgtatgtca attacacctc 245400
   aataaatctg tcaaaaatgt gtgaccataa taatctaagc caaaattgta gatgaagacc 245460
   atgcagcagt agaattgggt ggagaagtcg agaaggagaa caaattattg ggaaagagaa 245520
   gacataaata acatagacat caatataagt atgaaagaaa taataaagca tgaatttaat 245580
   taatataacc aacagttaaa tgaaaatgaa agattatttt caaaacagta gaagaaaatt 245640
   tgatatgcac attggaatat aagaaaggag gggaaagaaa aactataaca aagcaaaaaa 245700
   gaataaacac agattaaaat ggcaaattaa aataataata ttagctataa taaatataaa 245760
   tgacagatag aaaaagaaaa caggatcagg cagaagggct ccagaggagg actaatctcc 245820
   cacagaattc cagaaacacc tgctgtcagc aatgagtctt tgtagggtgc tcaggtagcc 245880
   aatcagacag ttctttcttt catataattg caccacacgg ctggcacccc atctctgatc 245940
   acacctgggg aggtgcatag tgagctgaca gtacagactg aggccagaga tctggtgcac 246000
   cacactgcca aaatcccagt ccgtttccca gacccatcct ctggagcttg gtgttgtctt 246060
   ctgctggcat ctgtgctata ccacatgtag gtcattaggg caataacatg gctattgctg 246120
   tttctgtctc tgagattcca agaaatgtga atcaatacct ggttattaca ctttgtttgc 246180
   gccatgacat ctctaataga aatatgtaaa gtgaaaagtt agtaaaaaga gattaatatt 246240
   tcagtaatag gatgaactag caattctaaa tgattctcca ccagtagaaa aactgtatgt 246300
   actgggtaaa taattacagc caaggtttta aatgaatcat tggcttcaca agaaggtatg 246360
   ataaatctct aagatttaga ggtgaaaagg aagaataaat taaggaagag tgaacataaa 246420
   ttcagaagaa aatattgccc tgggaatcta ttgcagggat tggaccagat tgttagagta 246480
   gctctatcca cattggacag atatagtcac acttccttac acatctgcca tctccatgag 246540
   gggaacaggc cctgactagc ctgctgttcc aaagaggatg agagatgcat aggacaccct 246600
   tggatatgat ctggagcttg gcgtcaagcc aagcagacca gcctagatca gcttctcact 246660
   tggtccattt ttgtcttctc aggtttatcc caataaattt tttgcatgta attgtctgag 246720
   agcctcaata agtctagatc agccaacccc aagacacatg ctttttgatt caagccactg 246780
   agttttggag tggtttgtca catatcttta ttgtggcaat tgctaaccaa tatgttactt 246840
   ttaaaactgt agctggagtc tcttggtagc aactatgttt acaggatttg attgagaagt 246900
   aatcatcctt gcttctcaag aactagaata tgagttgtgg agacatgagg tgcttctcag 246960
   tttgggatgt agaaagaaag aactgtgatc tggtggaagc aaagcagaaa aggcagcaga 247020
   gagggagcct tcaggagggt gttaagcctg tcagtgtgtg atgggtaata cttgtgctat 247080
   ggactgagcg tttgtgtccc tataattcac atgtggaagc ctaaaccccc agtgtggcta 247140
   tattttgaga tgggacctct aaggaagtaa ttaaggttaa atgaggccat atgagaggga 247200
   ctctgattca ataggataaa aagagatatc agagagttca ttctctctct cttcatgaac 247260
   atgcactgag gaaaggccat gagaggacac aacaagagcg gggccatcta caaaccagga 247320
   agagagcctc accagaaaat gaattgtcca gagcatggtt cttcaacttc tagcatccag 247380
   aactgtgaat ggatctattt ctcttgttta agccacccac tctgggtatt ttcttatggc 247440
   agcttgaact gactgagaca atggggatct actcagttct ctgcttcagc ttcaacttac 247500
   tcacagctac agagaatagt gctgctttag gctcacagcc tctcccagga tattaccttc 247560
   tgtcactgat atggtttggc tgtatcccca cccaaatctc atcttgaatt ataactccca 247620
   catgttatgg gagaaaccca gtgggaggtg atttattatg ggggcaggtc tttcctgcac 247680
   tgttctgatg atagggaatg agtctcaaat gatctgatgg ttttaaaaat ggaaatttcc 247740
   ctgcacgagc tctctttttg cctgctgcca tccatgtaag acatgacttg cccctccttg 247800
   ccttctgcca tgattgtgag gcttccccag tcatgtggaa ctgtaagtcc attaaaccct 247860
   gttttgtgaa ttgcccagtc tctggggtct ttatcagcag tgtgaaaatg cactaataca 247920
   gtgtattggt accagtagag tagggtgctg ctaaaaaata ctctaaaatg tggaaacgac 247980
   tttggaactg ggtaacaggc agaggttgga acagtttgga ggggtcagta gaagacagga 248040
   aactccagga aagtttggaa tttcctacag actttttaaa tagccttgac aaaaatgctg 248100
   atagttatat gaacaagaag atccaggctg aggtaatttc agatggagaa gagaaacttg 248160
   ttgggaactg gagcaaaggt gactctggct atgttttagc aaagagactg gcagtatttt 248220
   gcccctgccc tagagatttg cagaactttg aacttgagag agatgattta gggtacctgg 248280
   aggaagaaat ttctaagtag cgaagcattc aagaggtaag ttgggtgctg ttaaaagcat 248340
   tgcattttaa aaggaaaact tagcattaaa gtttggaaaa tgtgcagcct aatgatgtag 248400
   taaaaaagaa aaacccattt tcttaggaaa aatttaaact ggctgcagaa ttttgcataa 248460
   gtaacaagga gtggaatgtt aatccccaag accgtgggga aaatgtccac agggcatgtc 248520
   aagagacctt tgctgcagcc cctcccatta cagacccaaa agcttaagag gaaaaaatgg 248580
   tgggccaggc ccagagtccc catgctgtgt gcaggctagg gacttggtac cctgcatccc 248640
   agccactcca gccactgcta aaaggggcca aggtacatct tggcccatgg tttcagagag 248700
   tgcaagcccc aaaacttggc agcttccatg tggtgttgag cctgcagatg cacagaagtt 248760
   aagaactgag gtttgggaac ctctgcttag atttcagagg atgtatggaa atgcctggat 248820
   gtccaggaat aaatttgctg caggggtggg gctctcatgg agaacctctg ctagggcagt 248880
   gaagaaggga aatgtgagat cggcccccga cacagagtcc ctactgggca ccccctaatg 248940
   aagctgtgag aagacagcca ctgtcctcca gatcccagaa tggtagatcc actgatagct 249000
   tgcgctgtgt gcctggaaaa gctgtagacc ctcaatgcca gcctgtgaaa gcagccagaa 249060
   gggaggctgt accatggtaa gccacagggg tggagttgcc caagaccatg ggaacccacc 249120
   tcttgcatca gtgtgaactg aatgtgagac atggaatcaa aggagattat gttggagctt 249180
   taaggtttga ctgccctgct ggattttgga catgcatttg gcctagagcc cctttgttct 249240
   ggccaatttc tcccatttga aatggatgta tttactcaat gcatttaccc ccattgtatc 249300
   taggaagtaa ctaacttgct tttgatttta cgggctcata ggcagaaggg ccttggcttg 249360
   tctcaggtaa gaggttgaaa tgtggacttt tgagttaatg ctgaaatgag ttaagacttt 249420
   ggaggactgt taagaaggca agattagttt tgaaatgtga ggacatgaga tttgagaggg 249480
   gcctggggtg gaataatatg gtttggctgt gtcctcaccc aaatctcctc ttgaagtgta 249540
   acttctacag ttcccatgtg tcatagaagg aacccagtgg aaagtgatcg aattatgggg 249600
   gcgggtgttc cctacactgt tcttctgtta gagaatgagt ctcacaatat ctgatggttt 249660
   taaaaatggg agttttcctg caaaagctct ctctctttgt ctgctgccat ccatgtaaga 249720
   tgtgactggc tcctctttgc cttccaccat tattgtgagg ctaccccagc cacgtggaaa 249780
   tgtaagttca ttaaatctct tttttttttt cataaattac ccagtctcag gtatgtcttt 249840
   atcagcagtg tgaaatggac taatacagcc acagaaaatt atcttataca agaatgtgtc 249900
   ttttctcagg gtgtggctta tagtcaagaa tatcaaagac cccggccttt cctgcaaatt 249960
   ggaacaaggc taaacagtat ccaaggtgta gagctcccta tagaatcagc agagcttgca 250020
   tttgcaagtg tattaataga tcagcttctc cttctgccca atcttgcctt cctcactttt 250080
   tatttcagaa ggacatttac tagtacaatt tctgcatcta cttcttagtc tcagaatttg 250140
   tttccagaga acccaatcac aggcagagtt tttgagtgat tttctgcatt gcactttcat 250200
   tccaatagtt ctgttgtacc ctctcattaa ataatggaag cgtctgacta ctgtatattc 250260
   aagggcaaag gatcatcccg tgttttgctt gaagtggtac aaagaaaaac agaaaacact 250320
   tgagctttgc aagacttctg cctcagaaag aagtaggtag acaagtgtaa gctgggatga 250380
   atttaagata tggaaccatt tactaggcaa cctgggggtg actgaggaga ttaacttgtg 250440
   gtcaaaaaca aggattcttt cagtaacagt tgatgagcac ttgggcaatt gtaatttgac 250500
   acaaaggaaa tcaagacttc caaggccagt aaattatgac atccacagga tatctttatt 250560
   tatatccata catttgcaaa attgagattt ttctgcatat aaaaccttat gcattgcttt 250620
   atttaacttc taaaaatatt ttaggttata aaataatttt cagagtatag cttttgatga 250680
   ttgcataatt ttccatgctg gagatatact gtaaattatt taccctactt ctctattttt 250740
   tgatatttat atggttttat ttttttctat tataaataat actgagttga ctatcattac 250800
   acataaatct taatgtgtat ttctgattac tttcttggga taaacctatc gaagtggatt 250860
   taatgactat tttcagtaat aaaaagaaag attattttga aagcttgtga tacatattgc 250920
   agaaccttga tctgtaagtg tgaaatatgc actccttcca gcctttcaat cagtatatgt 250980
   ttattgactt gtacgatgta agcagaactc tttttagatg ttagagaaaa gaaacaggta 251040
   gagtcttgtt ctcagggttt tcatgttcaa tacaacacaa cacaaagttt tgtgtatgtg 251100
   tatacataat gtgcaattgc ctatgtaagt cctcattatt ttatcaactg tagcaggcat 251160
   gatttaaagt atttttttta attttctggc atgctaacaa caaaaagttt atcaaggaaa 251220
   catcattttc ttgcaaagca aggacagtat atttgatatg agctttttac cctgctctga 251280
   taagaacata gaagatatgt tgctattgaa ctttcttctc cacatatatt tttagttact 251340
   ttttatttca tattctgcag ttacacttcc tatacttcag catcagttta tattcaacta 251400
   catctcaaac ttgaaatcct tacacttcct tcagaatctt cttacaaatt cctttcctgt 251460
   ctttctaagt tatatgtacc ctccaggttt tcacctcata aaagggtaaa gaaaaaaaaa 251520
   atagaaagca acatcttttg tttttacagc ataaggagtt cagctcctgg cagttcttct 251580
   gccagcataa cagttgatga acactggaca gcctgtttca agtgagatgt aaaagaatca 251640
   agactgtatc ttaactgttg tatctgattc ttttaataat tttcacccaa gtgaagggca 251700
   tgtttgcagc agttatttgc atgagccgca tcatcagcac cccgaacatt agcatcctgg 251760
   gcattactga agactcttgg tcctttccct gacgaacgat ggtgcatggc atgatctact 251820
   ttttctataa agcctggaat gacatagctg tggcaaacac tctaggagga aaatataaat 251880
   gtccttcttt tctgatattc tcattttcat cgagtaataa aggaagtgga aaggtagaag 251940
   atacatgcaa aatcataaaa gtaattctaa ttcacaagat aatctatcat tttcaagcaa 252000
   gtggaatata aatactgtag gttttagaga tatttagccc caaataaaac cacaggtcta 252060
   cacatgagtc actgtagcac tttaacctct ctgggctcta tttccttctc tttaaaatgc 252120
   acagagggtt tttgtgaaat cagtttagat tatgcatatg ccatttctaa aatagtgcct 252180
   tctctactgt agttgttgaa taaaaaagta acaatattag agaaacaatt ttagattttt 252240
   ctttcagtaa ataagcttac aaataaaata tctactttgg cttttatgat aaactttggt 252300
   ttgaaattat ctgaaaagaa atccttagtg ttctcatgta ctactcttat tcctgtccat 252360
   gaccgttttg aacagtgaaa gaggtcaata ttttctaaag agcctatgtt ttgacgactg 252420
   tatatttgct tggcaatgtt ttatgaaata aataattagt aatggtttta gctatatttt 252480
   aggtactcac tacaatactt tttttgttct acatttttct aaataccaaa taaagaatta 252540
   gaaaatcatt caattatagg atgtgacctt atcttgctga ttacaccaaa tccaaagtca 252600
   attttagcct ctctttcaga ggttgtaaat agaaacccaa attggataac acatttcccc 252660
   ttcaagacaa acaggtggag cccatcattg aaagtaaatt atcaggtgag gactcaaggg 252720
   acccgggcca gttcagattc tgtccagtca ttaaactttt tagatctcac tgtcttcatt 252780
   tgtcaaaggc agatagcgac cctgacttca ctgatacaca gagttatcat gaaggctgcc 252840
   tggtacaatg ggcagaaata tgctctaaca aagagatgta aatgcagtat gtatagcatt 252900
   gagacttctt agaagggagc tcgctgctgc caatgctctg ctatgagttg cctcaggttg 252960
   tacaattcct ggtgaaggcc tagaatttaa gccattgaat aggaggatta tttgggaacg 253020
   atataaaatt ggagataggt taatctaggg tcaaatatag gtgagaagca aggaaataat 253080
   actgtggtaa gaaaatagat taagcagaga aagattctat gattctgact gaaataagca 253140
   ttgctaacta taactggttt ttgtgcccct tggagcatga ggttttgaag agctcattgt 253200
   gttggactgc ctctttggag aaaccatcaa tggaccttca atctcatcaa tggaaatttt 253260
   acccaaaaac acaaccttga tgcttcagaa gagaggagga gggaaggatc atgtgcccat 253320
   gagttttacc cctttgatac gcagtagagt attgtttggc ttacaaagta tttgggggaa 253380
   gatatgagca agtaagaata taagtgcctc atctctcaga tagatatgat ttgaggcatt 253440
   agttgacatc ctacagaaga aatatttttt aaaaagtcca gcttcaacta ctggacccat 253500
   catttatcag ctatttgact ttgggaaagc aacttatttt tctaaacttt atctctgaaa 253560
   tgaaattaag gatactgtat ttgttatggt tctccagata aacagcagat agatagatag 253620
   atagaaagat agatagatag atagatagat agatagattg atagatagat agatagatga 253680
   gcaggagaga ttttaaggaa taggttgaaa agattacaga ggctgacaag tctgaaattt 253740
   ataggccaaa ctggcagcct gaaaattcag gtaagagttg atattgatgt catagttttt 253800
   agtgcgaaat ttgtagggca ggtcagcagg ttggaaactc aggtgagatt tctgtgttat 253860
   agtcttgaaa cagaatttct tatttttcat gaaacctgag tttttgaact gaaggccttc 253920
   aattgattgg atgaggccca ccctgcagca gatgttgcag ggtaatctgg cctattaaaa 253980
   ctcagctgac catagtatta atcacatcca acaatacctg tatagcaaca tcgacaccag 254040
   tgtttgatcg tacaactgag catcatagtc tagccaagtt gacacataga gtcaaacatc 254100
   acagatactt acctgtggtt cagtgggata atggtctgaa cacttagcaa ggaagttgag 254160
   atgatttcct gtgtcagcat tttatttaag tggctgaggg tgtgggagtg gcaggggata 254220
   tatttcaaat atatcaagcc taaaatatct tcttgttgac tagataggaa tatgtgttac 254280
   tttaatacat acaactttac tttatcttaa agatattgat gattcaggtc aaggacccag 254340
   caaaaacaat gttgtgtaga aagaaagagc acaaaatttg agtgtgtgag aactgggttt 254400
   gaatctaagt tatatcctct ctatcacttt gggaaatcta ctcttgtctt caatttcctt 254460
   atctgtcaaa tgaatttaat aacaccactt ctttgagttt gggggatgtg tgaaacacat 254520
   gaaacataga gtttctcaga aaatgtagtc taatatttgt aatgatcttt tttcttcatt 254580
   tactgttttt tcaacctgct gagtagaagc tcatttttcc atctgagaaa tacattttca 254640
   aattccacaa ctttcttgtc atttatggtt actaaaatca gttgaggtag tttataaatc 254700
   caatcccata tcaattgtaa aatctttgtc tccttcttta ttggacaaaa ctgagggtca 254760
   gagacttgga atggagtttg tgcatggctt tttctttcgc ttctttttct tcccactttt 254820
   ggatcttgtt tcttcaagtt tgggacaagt gaagatctat catgagatgg taatggtcat 254880
   atgcctcttg ggctgcttct gtgatgcaga agtgctcctg gcctgttcct gatgttccct 254940
   gatatgtgag gcaatctgca gatatgttgg tttctgtatg gtgtgtatgc ttttcttgga 255000
   gaggtgtcca gatttccaca gcatgcctct tattgatgac ccacttttag agacaaatgt 255060
   gtttctttat tggttgcccc catcaaaaat ccatacctcc aacctctttc tagctgcaga 255120
   cactgaggct attgaggtat tcttccttcc atatcttctg ggagctttag ggatgtggag 255180
   tggggagatg cattttttca tactggttag ggctactctt gcaattctgc catattcaga 255240
   taacatgtgg cagaaacagg cagacctctt taattacatt ccaaggggca caggcaccaa 255300
   gttccccagg aactctttca ccctgcagtg ctcctgtgga agcaataaga gggtagcccg 255360
   gcagatgtgt aatgcaatga acatgacctg gtaagccaaa attcaagctc tctttctttc 255420
   aggaatatcc acctgacttg cttaaagctc tcatccccac cattccttgt cttcggggat 255480
   gtgatggtga tggaagggaa tcacagggca agagcatagg gaaagtattt tgcactatga 255540
   gtattctatt ccctaaaaga aagaagatgt ttaaaaatta cctctgtatt tcccctcttt 255600
   cgttctttag ccatccatcc tatactccag gatgacaagt tatccagtgc cctgggagct 255660
   gtatctgacc ccatttgatg gtcactctct tctgaataca ggctttaatt gtcaattaca 255720
   aggcaactaa aaaaagtttc attccatatc ctaatacagt agctattatt attatcagca 255780
   ggaacagtag ctcctttatt cacactgagc tcgtagcact ctgtttttct cctttatgac 255840
   cattgtataa tggatgcttt ctggatggtg gcatattggg ccgtaaatac tgtaacttcc 255900
   tcctacatag attaccagtt agagctcctt tacaatcatt ttatggtttt aaatatagcg 255960
   ccatacttgc ttcaagatct tgtttcctgt ttcacaaaga gcgattggtc ttcctcgtgc 256020
   tgagaaaaaa taacatttga aatgcagaat ccagcctgtt ccccagggct ggctttccac 256080
   caggttcctg tggggaggtc aagcaggtat ttcagcaggg cagtgtcatg ttgctccata 256140
   tcctggccac atctcccttt agactccaca tcccaatatc tgctgttcaa ttccctgggt 256200
   aattaattac cctacaggag ccaatagaat cctgtataag ctggtgggat tccattgtat 256260
   gagatgaaat gcagcactgc cttggggggt gctcttttca ttaggcccat gctattctgt 256320
   cttagttgaa cacaatctat aggtatctct taattagaac cccaaaagca ctgacagtga 256380
   cgtcctttgg ataggcctgt agtaagtgaa ctaagcggtg tccacctgag gccaaacata 256440
   atctcccctc tccttcaagg taaacttcta taaaatagaa aacaaagaaa ctggattttg 256500
   atgaatctga aatgaatccc tcttcatata tttattactc gtatatagga ggttttcttt 256560
   tatacttttt gaggattccc caaaacaaaa ctgcacttgt atagctagga ggactaggga 256620
   acaatgcaga cgtgggagaa agggtgagtt cttgtgaccc agaactgggt ttgaagcagc 256680
   aacttccgtt caatcaagca tgcctctcag cattgcaggt gccaagttgc cattctctct 256740
   ctttccttcc atgcgctcag gtttcccagc tctcgactgc tttttttctg acaccttttt 256800
   ttatttcttc ctctccacag gtctctcagt tttgctgata aagatatttc actctacccc 256860
   agtttcttta ttttgcattc cgcatctatt ccttaaaact gttttctgaa gtggcaaata 256920
   cttttgtttt tgaggatgaa gattttggtt tttttcatgc tcactaaagt aaagcagatc 256980
   ttgttgccag gattctccct cctctgaaga cagtgggtta taacaagtga caaatatttt 257040
   cttcctgtct catagtaata ttcaaaataa gagatttatt gtgtctactc agtgtgaggc 257100
   aagacagttt ataatttacc atttatttga gttgtgtgca gaaacctacg ttaactcaca 257160
   ccatatttct tatttatttg attgatattt cagcacccct tgtagattga tgcctccagc 257220
   agatactcag aagttcagct cattactatg tgtctgggcc actctgaccc tgagctctac 257280
   ttagcctact catctctacg catagtgctc agagcaaata aaaatggatt tgaccattac 257340
   agaagtctca gatcaatgca aagacacagt ttttactctg agcttggctc tcttctactt 257400
   ttacttggtt tttagccttg atttcatttg tggttggtcc tgaggttgct cctcagattt 257460
   catgaaacag tttaacttcc tagtttggac cccaagatcc ccttaagaca ctctctatag 257520
   taaagtccat gggggcagaa atcatagatt ggaatcatag gtcagccact tgctagcttt 257580
   gcgatcttgg gagattcacc tggcatctca gagtcaggct tctcatcagt aaaattgggg 257640
   caatttagag acatttgtta caaggattaa attagagaag cagttaaatt gctttagcac 257700
   aatatgagta acatggcaat gtttaataca tattatttgc aattataatt tcatcagttt 257760
   attagtaaaa ttatatctca ttattgccat ttctagcaat cacccagtct ttctcatcac 257820
   tgggctctca cccaggcatt tgcagtcctg gaatccatgg aaaagctcac cctcatccct 257880
   ggtgacaggc atgttggaca gctactttcc agcatattat cacttgagaa ctatcacttg 257940
   agagtcccct gctcagaaag atctcttcag atgccatttt gcattaactg tcagaggctt 258000
   tataatcctt aaaacagtct tgctacatgc tatgagatat attttagaca tattttctca 258060
   tttgatacat acaaactgtg taagattttg ttttcatact ataaagaaac caaacttcaa 258120
   taggattaaa taacatatct atgggttatg gaactagtat taaaatctgt tcgtgataac 258180
   tccctagaca tggtgctacc attatagcat gagctatagt gagtgaagat ttaggctgga 258240
   gagtggtggg gcagaggctg caatatgctt cacttaaggg tcctcggaac gagtcaggac 258300
   caagtaaaca gcttggctga agctcaggct ttcttgtaga tggagaattt ttttaaggag 258360
   aagtgcattt catgaatttt caagcagaag caatatatgt aggcagtacg aagcatttct 258420
   taaggaccca aagcaagtgg atgagaattg gagattgatc catcatacac tagctggttt 258480
   ggtcttagga cattgcttag actctcaaaa actcagtatc ataaataaaa ttaggaaaat 258540
   agtagtactt atcccataag aatattttga gaattaaatg agttaacaaa tataaatcat 258600
   ttagtacatt atctggtgca tagtaagcac cgaagctatt gtcatttagc tctcattaat 258660
   tcaagacaca aatataaagc ttaagcatat aataatgtct tgagagatac gacatttgac 258720
   tataactatc attttgaagt tgtcatacca aggataaata cagcagaccc tggaacaaca 258780
   tagatttggg ctgtgcaggt ccatttacac aaagattttc ttccacgtct gccaccttga 258840
   gacagcagtg ccaacccctc ctctttctcc ttcccctcac cctactcaat gtgaagagga 258900
   tgacgacctt tatgatggta cacttccact taataaataa taaatatatt ttctcttcct 258960
   tatgattttc ttaataacat tttcttttct ctatattatt ataagaaaac agtgtgtaat 259020
   acacataaca taaaacttat gttttaattt actgttgaaa ttattggtag ttaatggtgg 259080
   ggcaagttag tgtccaaccc caatgttgtt ccagggacaa ctgtgtttga tgtaatataa 259140
   tgtttttgtc ttaagagctc attagaaatt atcttttttc actttccaat tggaaagtat 259200
   ttttttaaat ctcatgacaa tatatgaatt tgtcagtttg agctctgttt ccagaaaatg 259260
   agcatctttg attttatgat atttgaaatt attgctctgt gtgtgcacat atatacatgt 259320
   aatttataac taaatgacct tcttttgttt tcatttgtgt gtatcccttt gctagggctg 259380
   ctgtaacaaa gtaccacaca ctgggtggat taagcaagag caacatagtg tttcacagtt 259440
   ctgggggctg ggagtctgag atcaagaagt tggcagggcc atgcttcctg tgaaggacct 259500
   agggcaggaa ctgttccagg cctctctcct agcttcttct ggtggtttcc tggcaatctt 259560
   taatattccc tggcttgtag atgcatcacc ccaatctctg ctttcatgtt tttgaggtat 259620
   tcttcctttg tgcatgcttg tttccaaatt tcctcttctt ctcagggcac cagtcatatt 259680
   agggtagggg ccaccacact ccaggatgac ctcattttaa ctaattccat tccaacaacc 259740
   ctattgtcaa ataaagtcac tttctgtggc acttgggatt agaacttcaa tatatgaatt 259800
   ttcagggtgt ggggacacca ttcgactcat gcacatgatc ttcatttatt tcacatctat 259860
   acattatata taattattta ttttatgtat gttatatatc atatgtatat atcacatgca 259920
   tacatgcatt tccttaccat cacattacag tctactagat tatgtcctta agtgtcatgc 259980
   ttttgccttt attcattaat aattatatcc ccagtttgtc agtactcagt gctgtatctg 260040
   tcacactgta ccaatgcagc aaatgttgtt gtgaatacac aaaattctag agaaaataga 260100
   aagggtgaca aatagttatg aataggtatg ggaacgaatg ggtataagga ttgtgtagaa 260160
   agatgtgggg aataaatact cacacctgcc tagtttgggc aattgtacct aaagacaaca 260220
   attctgctgg cttctctgtg actggtaggg taagccctaa gcacatgttc ctgcagtgta 260280
   agggggatat tggggaaggc ctccggtttc aaattccctt tgctttatgc tctgcaagtt 260340
   agcgcacatc agttcactag tttgaatgag attccaagca gccttctgac agcttcccag 260400
   ttagggtcag tcccttttgg atcctatcaa tgatggctac tccatggtac ctggagaggg 260460
   cttgctctga accactcaat caaccaccat gtgtcacact accctcattg acaaatccac 260520
   tctcattcca tatttgggat tatacaattt taggaattct tattcctctc tttgtaaagt 260580
   caaagattta tttataaatt atctgcaaag cattcctcag caagcatgtc ctttttgcaa 260640
   tgctgtagca taacctactc ggtcaagttc aatttaacat tttgtgccat cattttctta 260700
   gcttgaaaat gtcagccatt ttctacacaa acttggcatt cttctggttc aacacttctg 260760
   tgagcttaag gcaattactg cagctgtgtt actttattca aatgtagcat acaactcaag 260820
   ccaaagatgt tcctggtgga caaggtctcc tatgacactt tacctataaa ttatcatatt 260880
   cttcacttac aaaccttaca cttatattac tattttaaag tactaaatat tttcaaatcc 260940
   tattttttaa ataaacccaa agacaggact ataaaatgct atggaaaggg agaaaagaac 261000
   acttaataac aagcagatgt cctcactgta tcctattctc ttggtatttc cttttacata 261060
   tgtcatctta tttattcatt aaaacaacca tgcaggctgg gtgcagtggc tcacgcctgt 261120
   aatcccagca ctttgggagg ctgaggtggg tggatcacaa tgtcagtagt tcgagaccag 261180
   cctggccaac atggtgaaac cccatctctt ctaaaaatac aaaaattacc cggacccggt 261240
   ggcatatgcc tgtaatccca gctacttgga aggctgaggc aggagaatct cttaaaccca 261300
   ggaggtggag gttgtggtga gccgagattg tgccattgca cttcaccctg ggtgacagag 261360
   tgtgactctg tcaaaaaaaa aaaaaaaagc aaattaagtg ttacagagca agaaattgag 261420
   tatcagatag gacaaaatta atgtcccttg gattcacaac tacagggcag agatgggatt 261480
   aaaatccaca agcaaaagcc atctaagctt cgttgcatta taccacagtt aggagtattt 261540
   cattctgagt ttgaatggaa tatggtaaat attgttaaac acaggtaata catgaacgat 261600
   ctgcaatttt agaggacgtt ttaataaact acctaaggac ctacagccct acagccaggc 261660
   cgtggtagac tcaaggccaa tatttgccta ccaattaaaa taaaatcaat tcaatgtgta 261720
   ttgttaatag ctcaaaattt ccatttaaca cagcttttac tattcatata taaaggtgaa 261780
   attgtgtttg ataaaattgt ctttatatct cttaatagaa agatgatttg tttttcctat 261840
   tatagctagt gctgaaatca ggcctattaa caaaaatgag ctgcagtcac agttttagta 261900
   aataatacac tctcttgaat gtactttgag aggaaatata tttggaaaac agccctttat 261960
   catttctgac atgatgtaaa catgtcacca ctctcaactt gtgcacttgg ctccaatttc 262020
   tgagagcatt tcattctttt tttaaaaatt ggttacataa aagtaaagta ccttgattta 262080
   tgcttggttt aattagagag aaaatgaaag aggcagattg acaaggaaag aaagttattg 262140
   gagtagctgg accaggaggg gcagatgcca catttgcttg aggccactgg ggagaatgaa 262200
   cccccaactc aggtctaagc agtacttttt ccatttgtag gaaaaaaaaa acaaaggagc 262260
   aaaccaaaag ttagcaagtt gtgagggact ctctgggctc tgagattgga agtaggttgc 262320
   agcaggaagg aactcgctaa gcgccctagt ctctgcatgt atttgggaca cacatctccc 262380
   ttcttggtta gaacattgct atctcagctg actcttctat ctgcttttcc cttgaattct 262440
   aaactttgtg tttctggcgt ttatatgtag tagttaaaat aatgaggtcg aagccagtgt 262500
   tctgctgcaa atcttgacta tcctacttcc aagctttatg agctttggta agtcacttcg 262560
   cacctctaag cactagtttc ttcatcagta aaatgggcgt aataatggct acctactcat 262620
   gacttagtac attaagatac tgacaaactt catttgtagc aattgttaaa gatatttcta 262680
   atgacaacat aatgcctgct gcagtgaaaa ttcaagatga atccctcagc tgagtgctgt 262740
   gctgctgagg aagatcacac agccatccta agtagcttcc tagaaaagtg taatataagc 262800
   caaaactggc actcagtatc atcaggatat cttctgtgtt tttctagaaa aatcagttta 262860
   tattctcaaa ataaatactg tattatgact gtcttcacaa aactgctgta ctactcctcc 262920
   tggctctact ctcaagtatg acattgttca aatgatattg agagaataga aatccataca 262980
   cactatcttt ctcattttcc tgttaccaac tggtaagtct ccctgctcct cagcctttct 263040
   ttcttaatcc ctactacaaa ataggaattg ttcattcccc tatcaaaggt cagtttttcc 263100
   atcaagggcc tgtgtctcac accttcctgc tctgttgggt tatcctcttt ctcctgaaac 263160
   atatgcatgc atatttctgt tggataattt tcaataggac aaagacatgc tctggtacca 263220
   ttaatattta aaatgaaaaa ctccagtgtg gtatccagga gaaaagtaaa taaataataa 263280
   ataaaatttt aaaaaacaca aaacaaccca ttctcttttt ccttcattcc catttgatta 263340
   ctgttttatg tttttgcttt aaattcagag aaattttttc caaagaggtt taatcttcct 263400
   gctatccatt ccctactaaa ccaattccaa cagtcttctt cttgcctcac tccatttgtt 263460
   cgtttatgta gcgaatgaac aggctaaatc tatgctaggc tttagagctg caatagtaag 263520
   cgagacaccg attcttcatg gcaattatat agtctagaat taaaaaggat tttaaaaagt 263580
   gatcacatat aatcttttat tatagaagat atttacagga cagaaaccat taataaatat 263640
   agatagtgac cgaggttcac ctggagaaga ctgtagggcc tgtgggaaaa gctcactgga 263700
   atccttctgg cacagggaag tgaagttctg ggtttggttc caggtctctg tggaacgtag 263760
   ggacagacac tttaggtaat gtggatcttt tcaaaaacag aaaatattaa cagtttaata 263820
   aaaaagagga cctagtcaag agcagaagaa acagtattca aggaagggca aatgagcata 263880
   tgccaagacc tgatggagga aagtttgaac gatcaggaat tatgaccaag acacttaata 263940
   ataatttgga atggaagagt gaagacaaaa aattagttga gaatatttaa tgctttcttt 264000
   ttttaagaaa tagtcctact tattaggctt tctgctgtgg tggtgaaagc agagatggag 264060
   gtgtatacct ggaagagatt tcatttttct agataattat agaagctaaa tcatgagctc 264120
   atcactctag ttttttgtat gtttgaaaat tttcataatt cttaaaaatt attttgtaca 264180
   aaatcatcat gtaaattttg aaatacctct gagatatcca agaggaaaca tctcatagaa 264240
   gcactatgta tattcactaa tgtttcacgg tgatacaatg atgagagact tacatttgta 264300
   aattatattt actggaatgt tttcgatcac ctcgggaggt tgtgttggga gaagagagac 264360
   aagtaaagca agactgaatt ctgaggaata ctgtacagac attctgcaaa gaagactctg 264420
   ggttaaaaca gctggtgagg tcagaggaaa cagatatacg tgtgtttatg aataaagaca 264480
   ggagaagaag gtgctttaaa aagaaagcac atttcagcag tgttgacttt tgcaatgagg 264540
   ttatgatgaa gctgaaaaat acgcactgag tcagtcacca tcgttgtctt ggggactcca 264600
   tgttaaaaaa attcttttgg tcagggtagt aacactccct ttgcctggct tagaatttag 264660
   ttcatcgctt ctttactgtt ccaaatgtcc acatttgcag atgaatatga ggttatagca 264720
   attcttccat agtaagataa gaccaatatc attgctctct tctatagcta cataaagtgt 264780
   aggtaccagg gccctaactt gagtataagt tgtgaagaat atgaaaactg aggtccacat 264840
   agataaaccc gaatggccta tgggaaaagc ccatgaaacc tcctggcaca gaaaagtgag 264900
   gtttccaggt taagttccag atttttgttg aaaataatga caatttttat tgtctttgtt 264960
   gaagataagt ccagtagagg aaattctact caggctgtct catagtaagt gattctagca 265020
   cagaaaatag cacagtgcct agctcacaga atatgatcaa taaaaaatat gtagtgactg 265080
   atgcttgctg gtaattaatc ctcagttgca attaaaacac acaacaactt catcaagtat 265140
   ttacccatcg taattttttg ggcctggaaa tggagacttt tgagaggcta atgacacatc 265200
   caacatctca tagttactag cagatttcat gttctaaaca aattgttctg acctgtcagg 265260
   caacaaggag gtagtaaatt agccttaaga tcattatgtt acttgagtgt catacatgta 265320
   ctggtatatt tctcattgta ttgaagtttg tttctttttt ttcttcatct ccaaatgtcc 265380
   tctgattgct attgtttccg tagctgagtc acgcctgaga catctcactg actttctttc 265440
   ctaactactt gatagtctaa tgaaaggaaa gagagggagg tgaagaaata gaataagata 265500
   aattagtaac ttctggacaa ttctggaaaa ctgaaactat aggattaatg ttcagagcga 265560
   agtacaataa atggaccata agtcaaatat ttcataataa ttctgtagaa agtgacattt 265620
   gaaaactcaa attgtacaaa accaaatctt tccttcatga tggcaaaggg atgggtcaca 265680
   ccttaaattt cttcccagtc tccatgctat agccacactc tatggggaaa aatagccggg 265740
   gggggcgggg gtcattccat tacttgaaat gaaagacacc attctaaaga aatttcaaat 265800
   ttctttacat tattaataca ttattaaccc tatacatgtt tagcaatatc tttctcaaag 265860
   ttagtaaaag gaatgcacca ttattttgtg agaggggctg tggaaaacag ttcttgctta 265920
   ttggacatat ctgatgggga gaggtcagag gtgttaatgt aaccagagca tgtgcttctc 265980
   ttatacagtg ggatccacga gggggataga tgactggttg tctggtgaat gcagtgttac 266040
   ttttattagg ccataaaaag gaaagtttca aggccaacta attgtattgt ttgtcatagc 266100
   agtgtccctg taaaaggagc atattttcca acctcggcct gggaagatgc aacatgaact 266160
   gtcactaaaa ttaaggaaac atctctaaat caaactaatt tagactaaat ttagactaca 266220
   tttagtcatc aggtagttta tggtgacccc attctctgca tgtgctgaaa aactatggct 266280
   gtccatctct catagcctgg gcaggtttgc acaaaatgta tttgtgctag aaatccagaa 266340
   aaacaaacta ccacacacca gcataaaaat tgaagctcag aaaagtgaat tgaagtcctc 266400
   agagcatcag ttttcttact ggttgtatcc attttctgga cttgccataa aagttgccac 266460
   aaacttggtg gcttaaaatc agaggaatgt gtcgtcttaa tggtttttga ggccagaagt 266520
   ccaaaatcaa agtgtcatca gagtcacact ctctccgaag gctctagggg agaatccttc 266580
   cttgctcctc ttggctttgg gtggctcctg agagtcctca gctgtgacag cctcactcca 266640
   gtctatgcct ccattttcac gtgtccctct ttcctgtgtc tgtgtgtcca ctcctattct 266700
   tataaggact ccatttatta aatttagggg ccaccctaaa tctagagtga tttcatttca 266760
   agatatttaa ctaattaaat ttgcaaagac cctatttcca aataaggtca ggttctcagg 266820
   ttctgagtga acatgtattt tgggactatt caatccacta cacccgtaga ttgagctggc 266880
   agttcttaac ttataagact tattgtgact gaataacttt tctgtaaagt atttggtagc 266940
   taataggtga tcaacaaatt ctttgttttt aagaatacca ctatagttat ctttaatcct 267000
   gaccatgact gtctgaatat tctcattttc ttaaatatat tagtatgttc aattcttaca 267060
   ctggtctagg cattcattat ggaagcaacc tgctatatgt aaataagcat gattttaatc 267120
   ctatctagta ggctgtgtgt tataaatgat ccctagaaac atagacattc cgttgtggga 267180
   gcacggaaaa gggagtgatg atttctgaga agatatgact tgtaactttc caataacact 267240
   tttctagaag ggtctcatgt taaaataaat aaatgtcaaa ctaaactaag agacagcact 267300
   ataatgtact aattggctcc cagattaggg ctcttaacca tcaggctatt ctgcctccca 267360
   aattaaaatt ggtagcggga catgaattta ttgtttgaaa tgagcacagg ttaagtaaaa 267420
   tgagaactat ggaaaaacaa tgacaggaag aacgggagaa ttatactttg ctaacagtaa 267480
   tagtaaatag tgaacattta ttgtaaataa gtttaacaat acgcatttaa gacaaattta 267540
   agagcttata ttttttgacc agtaattcct tatttaagaa tctaactttg gaaatcatac 267600
   aggatttgta gaaccttttt acagaaagat actcattctg gaaatatttt taacactgag 267660
   atttggaaac aatataatgc ccaacctcaa aggattaata aattaaattg tgatattatc 267720
   ctcaattgaa tactatgtgg tcattataat tcatgtttca aaagagtata ataaaatagg 267780
   agagtcctgg gcttttactc tgagtgatgt tggaagccat tgaaggtgga ggagaaagaa 267840
   atgagaagaa cgtttccaaa caatgaagat gtccagccag tcattgacaa aaatgttctg 267900
   ttaatcatgg tggatgggaa gtaggactag attgcagctc tgtatagatc agtgtgcaga 267960
   ggctcgcatt gtgaatttta gctccaggtt gactgcaaga acaagccagc aatcccgaga 268020
   gtacccacag actctctgaa ggaagcgaac tgtttctgca gtacccaaga gacaccacaa 268080
   atactgtcag tgccccgacg ctggaagtgg taaagggaga cccccctctc tggaacacac 268140
   agccccactg gagaaactga aggtctcttt gtgggagaag tttcctacct tctctggagt 268200
   tgaatccatt tagagagctg agcaaaatac agggtttgag gaagcagtag aaaggccctg 268260
   ggagctcact gggtgcccaa acaggccatt cctgcttggc accacaggga tccattgtga 268320
   gggtggccag aggagcaggt gttgaaactc cacaggaaga aagaaatcta tagttgaact 268380
   ttgtaacaat ttgaacaggg cgagaagcct cctggccaga actcatggga gggcacaaat 268440
   ctgttgtgca gactccacag gcaggggaag aaccaagccc tttttttcca tagttgagag 268500
   gcaggtagcc tgaggcaagt tttcaagccc atcttgccca ccacctggaa acaggctcca 268560
   tgagaactgc tctttggttt gcatgggagc tgggtgaggc ctatgactgc cggctttccc 268620
   ccacttccct gacaacctgc atgactcagc agaggcagca taatcctcct atgtacacaa 268680
   ctccattaac ctgggcatct cacccctatc ccccacaaca gccacagaaa gacccacaca 268740
   aggagagtca gagctcagac acacctagcc ccacccccaa ctaatggccc ctccctatcc 268800
   accgtggtaa ccgaagacaa agggcatata accttgggag ttctaggacc cccaaccccc 268860
   accactggtc cctatacata ctactacagc tgatactccc tggagctgat tcctcctggc 268920
   aggaggccaa ccatcacaaa actagagcat taaagctaag aaccctcacg gagtccattg 268980
   cactccccac cacctccact ggaacaggaa ctggtatcca tggctgagag accaatagac 269040
   ggttcacatc acagtactcc gtgcagacaa cccccagtgc cagcccggag ctgagtagag 269100
   tcactaggtg gctagaccca gaaaagagac aacaaacact gcaatttagc tcacaggaag 269160
   ccacatctat aggaaaaagg ggagagtact acaacaagta aacacaccca tgggacaaaa 269220
   gaatctgaac aacagccttc agccctagaa cttccctctg atagagccta cccaagtgag 269280
   aaggaaccag aaaaccaacc ctggtaatat gagaaaacat acctctttaa cagcccccaa 269340
   aatcacccta attcaccagc aatggagaca aaccaagaag aaatccctga ttaacctgaa 269400
   aaagaattta ggaggttagt tattaagcta atcagggagg caccagagaa gcgcaaagct 269460
   caatgcaaga gaatccaaaa aatgatataa aagtgaaggg aaaaatattc aaggaaatag 269520
   atagcttaaa gcaaaaacaa taaaaaattc aggacactct gaacacactt tcagaaatgc 269580
   aaaatgctct ggaaaatctc agcaatataa ttgaacaagt agaagaaaga aattcatagc 269640
   tcaaaaagaa ggtcttcaaa ttaacccaat ccaagaaaga caaagaaaaa ataataagaa 269700
   aatatgaaaa aagcctccaa gaagtctggg attatgttaa atgaccaaaa ctaagaataa 269760
   ttggtgttcc tgaggaagaa ggtaagacta aggaaagaat cttaagagct gtgaggcagg 269820
   agcaccaggt aacctataaa ggaaaaccta tcagattagc agcagatttt tcaacagaaa 269880
   ccctgcaagc tagaagggat tggggcccta tcttcagcct cctcaaacaa aacactattc 269940
   agccaagaat tttgtatcca atgaaactat acatcacata tgaaggaaag atacagtctt 270000
   tttcagacta acaaatgctg agagaattca ccattaccaa gccaccacta caataactgc 270060
   taaaaggaac cctaaatctt gaaacaaatc ctggaaacac tcaaaacaga atatttttaa 270120
   ggaataaatc acacaggatc tattacaaaa atactagcta aaaagcaaaa aaaaaaaaac 270180
   caaaatacac aggaaacaaa gagtatgatg aatgcaatag tacctcgcat ttcaatacta 270240
   acattgaatg tatatggcat aaatgctcca cttagatata taaccgcaga atggaaaaga 270300
   acttaccaac caatcatctg ctgccctcag gagactcacc taacacgtaa ggattcacat 270360
   aaacctaagt acaggggtgg aaagaggcat ttcatgcaaa tggacaccaa aagctagcag 270420
   gggtagctat tcttatatca gacaaatcaa actttaaagc aacagcagtt aaaagagaca 270480
   aagaaggaca ttaaaggcct tgtccaaaag gaaaatatca caatcctaaa catatatgca 270540
   cctaacactg gagctttcaa atttataaaa caattactaa tagacctaag aaatgagata 270600
   gacagcaaca tagacaccaa tagtggagga tttcaatact ccactgacaa cactagacaa 270660
   ttcatcaaga cagaaggcca aaaaagaaac aatggattta cactatgcca tggaacaaat 270720
   ggacttaaca gatatgtaca gaacatttca tccaacaact gcagaataca cattctattc 270780
   aatagcacat ggaactttct ccaagattca ccatatgata ggccataaaa caagcctcaa 270840
   taaatgtaag aaaattgaaa ttatatcaag caccctttca gactacagtg gaataaaacc 270900
   gggaatcaac tctaaaagga atcttcaaaa ccatacaaat acatggaaat taaataacct 270960
   gctcctgaat gagcactgag tcaaaaacaa aatcaagatg gaaatttaaa aattcttcaa 271020
   cgtgaatggc aataatgaca caacctatca aaacctctgg gatacagaaa aggcagtgct 271080
   aagaggaaag tttataaccg taaatgccta catcaaaagg tctataagag ggccaggttc 271140
   ggtggctcat gcctgtgatt gcagcacttt gggaggccga ggtgggtgga tcacctgagg 271200
   tcaggagttc aagacgagcc tgaccaatat tgagaaactc cgtctctact aaatatacaa 271260
   aaaattagcc aggcctggtg gcacatgcct gtaatcccag ctactcggga ggctgaagca 271320
   ggagaatctc ttgaacccag gaggcggaag ttgaggtgag ctgagattgc gccattgtac 271380
   tccagcctgg gcaacaagat taaaaaaaaa aaaaaaagtc tgaaagagaa caaacacaca 271440
   atctaaggtc acaccacaag gaattagaga aataataaca aatcaaaccc aaacccagca 271500
   gaagaaagga aataaccagg atcagagcag aactaaatga aatggacaca cacacacaaa 271560
   tatagaaggt aaatgaaaca aaatgctggt tctttgaaaa gataaataaa actgatagac 271620
   cattagcaag attaaccaat aaacgaaggc agaaagtcca aataacctca ataagaaacc 271680
   aaacaggaga cattacaact gacgtcactg aaatacagga tatcattcaa ggctactatg 271740
   aacaccttta tgcatataaa ctagaaaacc tagaagagat ggataaattt ctggaaaaat 271800
   acaaccttcc tagcttaaat cgggaagaat tagataccct taacagacca ataacaagca 271860
   gcgagattga aatagtaatt taaaaaatac aaacaacaac aacaaaaaaa gtccaggacc 271920
   agacggattc ccagcagaat tctaccagat attcaaagaa gaattggtac caatcctttg 271980
   acactacttt acaaaataca aaaagaagga accctcccta attctttcta tgaagctagc 272040
   atcaccctaa taccaaaacc aggaaaggac gtaatcaaaa aagaaaacta cagaccaata 272100
   ttcttgatga acctacatgc taaaatcctt aacattatgc tagctaactg aatccaacaa 272160
   catatcaaaa agataattca ccatgatcaa gtgggtttta taccagggat gcagggatgg 272220
   tttaacatat gcaagtcaat aaatgtgatg caccacacaa acagaattaa atacaaaaat 272280
   cacgtgacca tctcaatagc tgtagaaaaa acattcaaca aaatccagca tccctttaat 272340
   attaaaactc tctcccagct actcgggagg ctgagacagg agaatctctt gaacccggga 272400
   ggcggaggtt gcagtgagct gagattgcac cattgcattc cagcctgggt gacaaaagct 272460
   agactttatc taaaaaaaaa aaaaaaaaaa aaaaaaaacc agctctcagc aaaatcggca 272520
   taccagggac atatcttaat gtaataaaag ccatctatga taaacccaca gccaacataa 272580
   tgctgaatgt ggaaaagttg aaagcattcc ctctgagaag cagagcaaga aaaggatgcc 272640
   tactgtcacc actcctcttc aacatagtgc tggaattgct agccagttca atcagacaag 272700
   agaaagaaat aaagggcatc aaaattggta aagaagaagt caaactgtca ctattttctg 272760
   atgatatgat tgtttacctt gaaaacccta aagactcctc cagaacactc ctagaactga 272820
   taaaggaatt cagcaaagtt tctggataca agattaatgt acataaatca gtagctcttc 272880
   tatacaccaa gagcaaccaa gcagagaatc aaatcaataa ctcaacgctg tttataatac 272940
   ctgcgaaaac aaaaataaaa tacttaaaag tatacctaac cgaggagcct aaagacctct 273000
   acatggaaaa ctgcaaaaca ctgctgaaag aaatcataga cgacataaac aaatagaaac 273060
   acatcccgtg ctcatagatt tgtagaagca gtattttgaa aatgaccata ctgccaaaag 273120
   caatctacaa attaaatgga atgcccatca aaataccacc atcattcttc acagaattag 273180
   aataaacaat tctaaaattc atatgaaacc aaaaaagagc ccaaatagcc aaagcaagac 273240
   taagcaaaaa gcacaaatct ggaggcatca cactacctga tttcaaattc tactataagg 273300
   ccgtagtcac caaaacagca tggtactggt ataaaaatag gcacatagac caatggaaca 273360
   gaatagagaa cccagaaata aatccaaata cttactgcca actgaatttt gacaaagcaa 273420
   acaaaaatat aaagtaggga aaggacaccc ttttcaacaa atgatgctgg gataattggc 273480
   aagccacagg taggagaatg aatctggatt ctcatctctc accttataca aaaagcaact 273540
   caagatggat taaggactta aatctaagac atgaaactat aaaaattaga aaccaaaccc 273600
   tttagacatt ggcttaggca aggatttcat gaccaagaac ccaaaagcaa atgcaataaa 273660
   aacaaagata aatagctggg acctaattac actaaagagt ttttcatggc aaaaggaaca 273720
   gtcagcagag taaacacaca acccacagag tgggagaaaa tcttcacagt ctatacacct 273780
   gacaaaggac taatatccag aatctacaac aaactcaaat cagtaagaaa aaaacaatcc 273840
   catcaaaaag tgggctaagg acacgaatag acaattccca aaagaagata tacaaatggc 273900
   caacaaacat atgaaaaaat gttcaatgtc accaatgatc agggaaacaa aaatgaaaac 273960
   cacaatgcga taccacctta ctcctgcaag aatggccata ataaaaaaaa taaaaaaaca 274020
   ggagatgttg gcatagatgc agtgatcagg gaatgcttct atactgctgg tgggaatata 274080
   aactagtaca gacactatgg aaaacactgt ggagatttct taaagaatta aaagtagaac 274140
   taccatttga tccagcaatc ccactactgg gtatctaccc agaggaaaag aagtcattat 274200
   gcgaaaaaga tgcttgcaca agcatgcaca attcacagtt gcaaaattgt gaaaccaacc 274260
   caaatgccca ccaatcaacg agtgggtaaa gaaactgtga tgtatatata tatataaaat 274320
   atatatgtat aaactgtgat atatatataa agaaatatat atgatggaat atatatatac 274380
   atatatatat atatgatgga atatggtgaa tatgatggaa tactactcac ccataaaaag 274440
   gaatgaatta acagcatttg cagtgacctg gatgagattg gagactatga ttctaagtga 274500
   agtaactcag gaatggaaaa gcagacactg tatattctca ctgatatata ggagctatgc 274560
   tatgaggatg caaaggcata agaataatac aatggacttt gcggatttga ggggaagaat 274620
   tggggggagg tgagggataa aagactagaa atatggtgca gtgtgtatgg cttgggtgat 274680
   gggtgcacca aaatctcaca aattaccact aaagaactta ctcatgtaac gaaataccac 274740
   ctgtacccca ataatttatg gaaaaataaa taaataaaat aggaaaaatt actttcactc 274800
   tcaactatgt gtagcttaga ttgagtgtcc ctagaagacc ctgagacaag aatttgtgca 274860
   agttgtttat ttgagaaatg atagtaacaa gcacagtaag agaggcaaag aagtgagaca 274920
   gacaagggaa agatgccact aaaaaatggt ttaaaaaaac cccataaaac aaaacaaccc 274980
   ccccaaaaaa ggctatgttt tttcccaaaa atatatcatg tttattgagg tataacatac 275040
   agggttaaat aaagggtttt agtcccatat acacagtcac ataaccacaa tcaagatgta 275100
   gtataatttc ttcacttatc aaaatgtccc acattcttgc tcctgatagt caatccccca 275160
   ctccctggag aacactgatc tgttctttgt ccctctattc ttgccttttc cagaaagaca 275220
   tatatatgga attatacagt agccttttga gtctggcttc ttcacttaat aaaatgcatt 275280
   taatattgaa caatgttgtc gcatgaatca tgttttcttt tttaattttt aaattgttga 275340
   gcaatattcc cttgcatgaa tgcactacca cagcagttgc cattcatcag ctgaaggaca 275400
   gttaggttgt ttccagttct tggtgattat gaacacagct gctataaaca ttcatatgga 275460
   cataagtttt cttttctctt gagtaactaa ctaggaatag tattgccagg acatatggtg 275520
   ggcatatgcc taaacttatc agaaactgct cagtgggcaa ctgggactca ctcctcctgg 275580
   agacttcttg tagactgcac aaaactatgc ttcacagttt ttccattcaa gggaccagga 275640
   agctggggga ttcatttgtc ctccaactct gccattagtt gaaggttgct ctcaagagta 275700
   ttaaccctag agcatctatg gcccatctgg tacagaatat gcatgttcta aaggacatag 275760
   taaaacctag gttagaagtt gaagctgttt tcagtaggaa gccttggggt tgatatgcac 275820
   agaatgttga ggcctgccag caccttctac atcatgtggg gaaacatgtt gccattcaaa 275880
   ttctagtaat aattattttt gtgggggtgt aaggctttaa caggaacatg actttccctt 275940
   gtctcagaat cctcaaatat aagaaatctt atgttagtat attttgtaaa taatcacaat 276000
   aatactgatt attaccgtga gagagtttat gaagcactta ctatcaggga actatcatta 276060
   tctctgtttg cccagggtga tattgctagg agatagtgaa gccactattc agttaagcag 276120
   cctggtttca gaaaaggtac ttctaaccac tatgttatac tatataatcc tgttataact 276180
   taggttatga gaatttctac aaataaaaaa aagagaataa acaagagttt gtatataggt 276240
   cactgaactg ttcatatata ataaaatagt aaacaaaatg taatgttcta caaaatactg 276300
   tgcttgtgtg gtgggttgta gggattttgc ttctgtttcc ttgttttgga taaatttaat 276360
   ttaaagatca tgtgtttgtc tatggaaaaa tattgaaatt ttactttttt aatgtaaaga 276420
   atgtgtgttt tttaattaaa aaatttaaat ggactattaa taaattataa ttatatatat 276480
   ttatgaggta cacatatgta caattataat ttattgtata tatcatgttc acttatacat 276540
   atgaggctaa gacatatgta tacaaagtgg aatgatcgac tcaagctggt taacatatcc 276600
   atcacttcag atactattaa atgatggtgg gaagaggtca aaataacatg gggaaaactg 276660
   aaaactgaat cctgattctg cagttaagaa gcagttgtaa tggtgagatg tcaactgcca 276720
   agagacaaag gcagagaagt attaaactct ctgggtgctg tgttctttaa gagcatctgt 276780
   ctttgctgga aagattgatt ctggcttgca ggcagtcatt accacagtgt ccaacatcaa 276840
   aaagcctcct tgctctaccc ccagcagttc atcgattctg gacaaccctg ggtggtcttg 276900
   gactcttacc agcatctgct tagcctcgaa gcccctccaa atctcacttt gcctccacag 276960
   gccacattaa gttcaaatta acgaattcct ctaggcagaa ctttttcaaa gagttgaagc 277020
   caaaataaaa gttaatgaat tccagcaaag gcaaaagacc tctggataga tgttttgccc 277080
   tttttattca ttgaactcat gcagttttta ttttctcagt tttctctgtg taaaactctc 277140
   tgttccacca atgtttccaa aattaatttt gaccttctgg caacatttta atttcacatg 277200
   ccccagtaga ttctctttat tcaacaactt atttcatata ttcattattc cttttagaaa 277260
   atagcagtat cgtgctcaac ataagtattc attatatgcc taattacact gagaactgtg 277320
   cacagagaca agagcctggt gcagaggaag gagcacttgt ctgaggggag ccgacccagc 277380
   ctcatgcagg gcggcgaggc tgaagtgtaa ccctgaggac agggggcgag gcaggagctg 277440
   aggacagaga gctgacttgg gacgaggttg ctcaatgccc tgggtaacat gtgaaggagt 277500
   ttagatttta ttcctgagcg tggaagaaaa ccatctcctc ctctaatttt aaaccttctt 277560
   gagggcaggg catgtgtaat tctgaatatc tcttagtatc ttctagtaag tgttgaggac 277620
   aagttatcat taatagcaac tacttatata ttgtggctca gcaccttcca aataccctaa 277680
   caacaccatc cccaaatctt cacaaaacac taaaagttgc tatcatttaa ttgaatgtat 277740
   ggatactcaa aaataagact cagagtagat gagttgcaac tgctttgttg agtatctaac 277800
   gtgaaagatg tagagagaag caagaccatt ttatgaaatt gtcactgtcc cataatagat 277860
   tagagtaggt tctaactcca gcttttccct tctccatgct tgtgactttt gacaaatcag 277920
   ttttttacat atcaatgggc atttgttaaa aatctcctga gattgaggca tgatgcttgg 277980
   ttctacgatg gagaggcaac aaaaacagtc tttactctca aggggctcat ggtcttgtag 278040
   tagaattaaa atttacaagc aagtaatagt aaatcaatgt gaaaactaca ttaataaatg 278100
   tatacaattt attaagggac aaattaactc tacagggttt tataataagc tgacatgaaa 278160
   atgactaatg gtttgccagg cagaggagaa tgagacagac attccaaaaa agaaagaata 278220
   tccacaaatc catggatgtc caagtgtgag aagactggtc cgcccagatc atgtgggcac 278280
   ttatagcctt caatcactgt cttcacctcc agtctacaga tgtgtggctg ttaaaatggg 278340
   ttcctgggaa ccccagacgt cctgccagtg ttgcaaacat aaccctgtga ggcccagcat 278400
   ttgcaagacc agcaaagaga ggtgataagc agacaaaatc caggcctctc atcatattaa 278460
   ctgtgtgaca ttgggtaagg gtccaaatct ctctgtgcct cagtttccac catatgtgcc 278520
   attgggaaaa taagagtaac tatatcacta ggttgttgag gagataaatt atttgaatat 278580
   atgtaatgtt tttggaacaa ggcctggtgt agagtaaatt tcatgtaagt gttagctaat 278640
   tttatgatgc taatcacact ttaaacagaa ggaatcttct ttacctattt tataccttca 278700
   gctttcatat aagatatatt ttgaaaaagt gtttgcacaa aatctttcta aaaaatttta 278760
   atataacccc tataggtcca ttctttcaac aaatatgtaa tgagtatctt tttgttccag 278820
   gcaatgtgct cgggataggg cagcaacaca tttaacaaat acttctgcca tcatgggctt 278880
   tcttctgaat ggggtaaatt aaaaagttat atatatatat atgtgtgtgt gtgtgtgtgt 278940
   gtgagagaga gagagagaca ttatattgtg ttaaaatatc tgagaaaaat aataaaaatg 279000
   ttggagaaat aatgctgtga gtggggtgtt caacaccaag tagggctgat agagaaggcc 279060
   ttggtagaag gtgatgtctg ggagaagtca cgaagcaggt gagggaagga gttagtagat 279120
   tcctgggcat ggtgggagca ggccctgtgt gttgaggagc tgccagaagg ccaaggaaag 279180
   caggaacgaa tgacatgaag aagcatcagg gagggaggga ccggtagcca aaggtgagag 279240
   gctatacaga ttgaggtaac attggtaact tggaagactc agggttttat tccttgaaga 279300
   tggggaagat gggaaattac aggagggttt ggagcagaag catgacatgg gtgattaagg 279360
   ttttatgcaa atactaggct gtgagtggtt ggtgggagca taggggtatc ccaaaggcta 279420
   ggaaataagt tactcagcaa ttcacagaag agaccatggt ggttttgatg agagtggtag 279480
   caataggagt agtgagaagt ggtgaaattc ttggtaaaat ttccaaaggt tatataaggt 279540
   tgtatatagt gagactgtgg cagagtgtat ccattaaaat cttttttgtt gttgttgttg 279600
   agacagaatc tcgctctgtc acccagcctg gagtgcagtg ctgcaatccc ggctcactgc 279660
   aacctctgcc tcgcgggttc aagcgattct cctgcctcag cctcctgagt agctgggatt 279720
   acaggcgtgt gccaccacgc ccggctaatt tttgtatttt tagtagagat ggagtttcac 279780
   catgttggcc aggttggtct cgaactcctg gcctcagggg agctgcccac ctctgcctcc 279840
   caaagtgctg ggattacagg cgtaagccac caagcccagc ctaaaatcat ttttaactga 279900
   tgcctttggc gcttccattt ctgggaaatt gttcagtagg ttcctgatta cctagtcaag 279960
   aaagggttat gggagcagag cagggtctgg ctcttattcc aagcacaggt gcagcccaat 280020
   ggttttcctg tctttagaca agaatgctta gtaattccct tgcttaaatt gttcctccat 280080
   ctctgtcatt gaatagaatc tgaccctgag taatgtgact taatgagggc agtttgactg 280140
   tgcattcaaa gaaccactaa atatatgttt actttatatt tagtctcagc cattatgcat 280200
   aattatattt aaaagggcac tgaatagata agttacaagc cacaacaggt gtgactggag 280260
   aaacacagag aacgcacata aaatctggga gagagagcag gttacataaa tttcccatca 280320
   gggcagagaa atataaggta tatttgaact ttgaagccaa tcagtgtgca accaggtaat 280380
   tacaggcagc ctacaccagg atgggagatg ctggagtgtg agcttgggaa tgcccccata 280440
   actcattcat cttcaaagtg cccttggggg cccggcctca ccagccagga aatccaggat 280500
   cgtcagttgg aattatatca acatctgaga ggccatggag ggaatgaacc tcatttacca 280560
   tgattaaagc agatgaactg caggtttagc tcttctccat taaattcctg ttaaacttga 280620
   gaacttccac aggcattatc ttattaatag gcaggacagt cattaacagg tgatgaaagg 280680
   agggaagaaa tgactgtgaa atttctgaga atttaagaat gtctgcttca tgtacagtta 280740
   agaaagaaac taacattcaa catttttatc aggaatttgc atgcattatt tcattcattt 280800
   ctcatatcaa atctgttctt tattggcatc aatctaaata aggatactga cggaaaatta 280860
   atataaaaat tattttaggc ccacgttgag gattgaagcc tgggaaatac ctggaaatgg 280920
   cttcattcag ccttcgttac aagcaggttt ttagaagtga aagggaataa agaatgtgct 280980
   gatacaaagt tttttgttag gaattttcaa tggtttacag aaataacatt gattagtgat 281040
   tggctataca ttttagagct ctgggttatg agttatggtg tccagcgtat ggcattgtta 281100
   ggttaattta tggctacttg tcatcaagta atccagagcc cacccagcaa gtggcttcaa 281160
   aaggtgatta cttagctcaa ggttggggtg gtggggaatg agacctcact gctgtctcac 281220
   cttaatggcc cttttcagat tgctcagata atagtttctt ctctttctca taaataaagg 281280
   aagagagact gaaagaatct tagtaatttt ctagaggtgg tattttctag attcagaatg 281340
   aactacacaa atattgagtc ccctgttatt tccacactat catttgtcct ccaaaaggtg 281400
   aaatgcattg tctgacatca tattttagtg acagagcctg tccttgtacc aaggtctctt 281460
   gtggttttct ctatagtctc atgccagctg cctagctatg ggcccacaga atcaaagcct 281520
   ggagagttcc tatgaagacc tagataacca ggtttcaaca tagctctgtc tctggagtga 281580
   gtggtgaagg aaggagggga gcagaccacg tagccgactt ctctatttgc attttttact 281640
   tattgttttg tagtgcaagc atcaaacact ggaagcggct gagcattttt ctctaagtat 281700
   ggcgaaagct tgcctttttt caggtcttct tcttctttct cattaaatca ctttggtctc 281760
   acacaacaaa atagtcttca gggatggcct taggcaaagc tgtaaaatct atcctctgtt 281820
   cccagaactt ctccttgttt gtcctctctc tcattctgtg aatctctcat cctcttctga 281880
   tacttaggaa atttctctca tacaagctca aaaaaggatc cagatcattg tatgcatgaa 281940
   aacaaggcct catttgcaat tcaaagggca ggccaactgt gattatgcct cttatgtgtt 282000
   gtgaatccaa tgaagaattt taataaaagt tctaaattga ggcatagcca cattctaaaa 282060
   gcaatggaga atgatttgtg ggacatgaaa acgtgtcacc ctttaccatt gctttcttta 282120
   acgttattca gttaagtctt aattctcaag tttctatatt tgattttttt caaattatgc 282180
   tccaacctat agactaaatg gactctctgt ggctgagatg ctcaaggtaa agcagaacca 282240
   ggcagccaca gcaggatgag ggggtcatca tgcatactgt gcgctcggaa aatgttgcaa 282300
   aaatgtcaca agacatccct tcctgcaatc aagccaaact gaaataggct acagctggaa 282360
   attcccaaac agaccaacaa ttgtccacca agagtcagcc aacaaagaga gacctgtggt 282420
   tttacactta caggtcatcc attcaaggct ctgttcctca ctcccccagg ccccttcctc 282480
   ggcctttcgc tttttacctt tataatctct aactctccaa cccttcctgg gagtgcactt 282540
   tcactttatc ccaaaggcta ctcgtctcca atctataggt cactgtcaga aaataaagcc 282600
   cctttctgcc tctgtagatc tcatcttttg ttaatagctt taatattcta aaaagaagat 282660
   tcacaaacac ctagacccgt ggctgaacta agtttaaaag acgattgatt gagtgttgtt 282720
   ttctggatcc agttgaagtg tgttactcac ctactgagct agtaagaatt cccctaggaa 282780
   gaacagtaaa aatgaagaaa acctgagctc cagagtcaac gtagctgtga taggctcaat 282840
   aatagcccct ccacagttgt ccacgtttga atactaaaaa cctgtgagca tattacctga 282900
   catggccaaa gaggctttgc agatttgatt aagttaaaga tcttgaagtg gggagaaaat 282960
   cctagattat gtggatgagc ccagtgtaat tacaagagtc cttataagag gaaagcagaa 283020
   gagtcagatc ctgagaaagg gagaggtgac gacagaggca aagatcaaag aatcagagag 283080
   acagacgttt cagatgtcat gctgctggct ttgaaattgg aagaaaggtt tgcaagctaa 283140
   tacgtgcagc ctctagaaaa gacaagggaa cacttttttc cctagagcct ccagaaagaa 283200
   ggctactcca ctgataccct gattttagga cttctgacct ccagagctgt aaaagaataa 283260
   atctacgttg ttttaagtga ctatatttgt gatcatttgc tacaggagca gtaagaaact 283320
   aatacagtgg tcggtgaaga tgatcttccc tagtaatggt tagtatttga ggcaattaga 283380
   ctttgtagag ttgcagctgt tgtaggggaa tattataaga tcgtgttaga aaatacttca 283440
   ggagaataag gtgaaaatgc ccacggcgtg taccagcagg ctccaaagcc tttgaaaaca 283500
   gaaatggtag cagtaaacaa acggcacttc ttggagtaaa tattagcgtt ctattaaggg 283560
   ccttaagatt gtaaattgtc acattccacc aaaatgctct tttttctcct tctcttttta 283620
   aaggtttgtc caatagaaca taaaactgtg acgcttgaag ataaacgtga ccttgaccag 283680
   ccctgaggtg agcagtgatt tctttcaggt gtcaatcctc agcaaatgca agagaaatat 283740
   taacctgtgg caaaatgtgt gctggtgtga aagagagaat gatgtttcct gaagaatcta 283800
   acattttttc tccatgtgta gaattcaatc aggcttatga tgagcaaaaa atgaaatagt 283860
   tcccttctct ctgtagaaga aggtgatgat tttttttttt tgacttacag tgcatgcaac 283920
   ccagatttaa atccaggaag aaacaaaatt ttgtgttcta taaaacattt tggaaaaaga 283980
   aaagtgctac tgtaggtaac atgcacagtg gtgtcctggg acccccaggt caaatccttt 284040
   gcatccactc ctgtgttttg ccacgccctc actccatcaa aagtctgcca ccaccatcct 284100
   cctaaatgct gtaaatgaaa gtagcatagg gtgccaagac tcccaagata ctctccttta 284160
   cttgtccaat cattatccca attccagcta aattccttaa cgggcataac tgcctaagtc 284220
   aaatttaaat gtaaaaactt acatgtggcc agtggcattt tatcggacag cactgatcta 284280
   aacaaatatc tgggattggt tttctcacct tgttcactgg gttggaggag atggtgaaga 284340
   acagcctcaa atctcccagg acggacatta ttttcagaaa gggggcagat ggagggggaa 284400
   ggaacagctc gtctatcatt ctttctctcc tacagtaatt atgccaagat acaaagttat 284460
   aatttcttag agttcaacag gagtccaaag actcctggtc tttctttgtg tgagccagtt 284520
   gatttgcatc ctttccaggc tacataaaca cagaagccca cagggcaatc cagctgtgtg 284580
   tattacctga gccgtgggga gggatgctgt gtgatcacca ttacctgtga gaaacttgcc 284640
   ttactatctt cttcctactg tttttgctgt acttacccag cagttgccaa ttagatgggc 284700
   ctcacaagag taaaaatcgt caggtcaact gtggttacaa caagaatcct tgcatggaga 284760
   tgccataatt ttagacgcac ctatcatggt ggaggaaagg tgacctttgg gatgattgtg 284820
   tctcactgct ttacagttgg gaaagcaggt caaaagacaa taaattactt gtccaagatc 284880
   ccatggtaag attgttccag aagcacatac ataagctgtc tgtactacac ctatcctgaa 284940
   attgtgctct ctctgcaagg ccattttctc tagcaatttg caagaactgt aggaatgttc 285000
   ttgctcaaat atgaagcatg gacgttcttt ttattgccct caaaataaaa tttttcttta 285060
   tatcttctgt tactaccact gaacaaatgt tttccaccta ctcccaaagt aaaggaagat 285120
   aaaaagagaa gttagaaata attgcattac aactaccact attatgacta ctatcatttc 285180
   cgctaatgct acatgagaaa ttctttctat aaagtacatt atttaattat attaacagcc 285240
   ctatgtggta ggtattgtta tccccaatag tcccccaaaa gaagcaaaga aacagaaaaa 285300
   ttaagtaatt tatataagtt cccagagcag gtaagtagtg atgttggggt ttgatcccta 285360
   aggtaagtat tcccaaagct cgttattttc caataatgtg tttgaatatg ttaaatacct 285420
   tcacttcaga tctctccctt ctttcccatc ctcattctaa ttttgtagag tattggtccg 285480
   tctttgaata tatagaaaaa cagttcttat taatatttta ctaaatgtaa tcaactgatg 285540
   ctatgtattt ccagtttcaa atgagttgaa cataattcct ttaaaaagag tggcttgaaa 285600
   agagtatcag aatgagtcaa gtgacctgca tttctcttcc gagctctatt ccaaacaaat 285660
   cacaagcaac aataggttgt ttttgtacaa tttcagaatt tgtcaatatc tgtggtctca 285720
   gttgaatgtt tctggaagcc gaatcagaca cacagcaatc aaaatcacca gggaatgctt 285780
   tttcagcatt ttatacctgc attctccaat cctctaatcc ccaagtaact acagaacatc 285840
   aaggacagtg ttggtgtgga atgtgacatg cacattttta aaccagtctc taaggaattc 285900
   taatttacac ccttaacccc tggaaaagat aatcttcgag atttttaaaa gcctatttta 285960
   atagacttgt gagcacttca agctctaagt cagaagataa aataatatat agaataagtg 286020
   agcctttaca aagctcattt ttaccaaatt actgttcact ttaccaagtt accctactga 286080
   tatatttaga tgagccatta cacattcatg cctctgctcc tctctgacta gtcacctgtg 286140
   ccaggtgcac tcacaggttt tattgaagac ttggaagtgc tatatgaaat tctgaaatgt 286200
   tctcaaagtg cctgtaaaat gttttctttt tcctgaaagc aaagatttgt agaaaggtca 286260
   cttaactgtt ttcttacaga aagttgatat tcatgaatcc ataccagttc tggatgaaat 286320
   aaacatcttg actgtcctat tgagaagaca tgatattggg gtgaagtcca aggcatgtct 286380
   tcagttttgg ctttatattt aaagttataa accgcactca aaacagaaca aaatatgtat 286440
   atgtatttga gaagttgtaa atgtagtaca aagaataata ataatgaaaa caaaaagcac 286500
   tttaggctct aattcaacat tcgtggcata gtttataaca gtgaaaattg aaaacatatt 286560
   aaatgctgaa cagaagataa gaaaactgtg gtaaatccac tagatggaat attatatagc 286620
   catacacatt acagttatta cttctgtata ataatataaa atatcttata tcataatagt 286680
   aagcaataaa aacatgagaa aaaattttat ttaccactta aataaaatta gcttgaatta 286740
   catgtatgaa aaaagatcac ttaattatgg aaaataacat aactaacagg aacataatct 286800
   taaggatgag tgttttgaat tggttctgga gtttctggaa ttgattctct aaattaatta 286860
   gatttaaagt caccaatgac tctatttcca gcagtgaaga gagcactgat agtctataat 286920
   gtgatacagc aatagagatg ggcaaagcat caccattggc tactcctaat cagatagcta 286980
   tgagaggcca ggttctgggt gaccatatat ataatacttt ctaatctttt tgtcaaactg 287040
   acagctgtaa tgatattcac tagttgctcc taattttgct agaggaagga aaagaagagc 287100
   tcaggggttc aaattcccaa ctcaagaatt atataaatga cctgaaaatt tctaagtctt 287160
   ccctgcagga aatctttatc ttctatagct gcagatctga gattgctcaa aaatcaaacc 287220
   cagaatctca tcctgtgaga ggctgaatta taataccagt tgaatttcca acctcacggg 287280
   gtgtttattg ttaaactagg gcattgattt gcaaggaatg ggatcctgag agttggaatg 287340
   aagaaataag agaaggccct gatgagattg gggacactga tttcctcaac tctgataagt 287400
   cttctttgcc agcaaaagct gttattccat ccccatctga gtagatcaac ctggctttgc 287460
   ctaaggaaac tgtaatggcc tcccctgagg cagttgctat gcaaattgta gacctataag 287520
   tatactcaag tcccagcagg cccctaaagg tgaggtacag aatgtgaccc atgaggaggt 287580
   gtgccattct ccaaaacaaa caaacaaaca aacaaacaaa caaacaaaca ccttgatctt 287640
   tttcaatata tatagacaga aattttagga atatatgtgg gaatgaatat ttatgtaaaa 287700
   tggcagaaga aacataaatt tggacaaaac agacttatat tgacatgagt ccactaatta 287760
   gagattctta tttaaatgtt gcagtttgga gaattagaag ggactctaaa cagttagttg 287820
   aagtggtaaa ctatattatg gcccaaaagg tggtccaccg tgaataaacc agaatgacat 287880
   tctagtttaa gtgttgcagt ttggagaatt agaagtgggc tctaacagtt agttcaactg 287940
   gtaaactaaa ttatagctca aaaggtggtc cactgtgaat gaaccaggtg gtacccctga 288000
   ctgttcattt tacgtggaag gagaaatagc cagacatgta ttatatattg cttcatggcc 288060
   atgaccaatg gtttgcctgc atggtaaagt acttggaaga aacatgattg gaaatgctgg 288120
   acctgatttg gtatactgca aaggaagaca gtgaaggctt agggagatgg gaatgttagt 288180
   tttttcactt acaacctact cacccacatc gggagggtcc agcagacata gctttcatca 288240
   atactgtgag aaatacattt gtgagggagc cttagcctcc ttgaacagct ctgtgatcgc 288300
   tctcctctac tgactagaac taacagtgga aactgatgtc actgatatgg gaagcctaaa 288360
   tgcagtgaga acaattgcat ctaggagtgt caggggtcaa gtggcagcac tcaaccaaca 288420
   aaaggtgggc atcgttgctg taatggacag cagggatcaa ctctccagct ctatgtcata 288480
   gtagggttag cagggatctt ggtcaccttt ctctttcaga atacatcaca ctgtttcact 288540
   acattgatga cattaggcgg attgtacctc gtgagcaaga agtagcaact acactagaca 288600
   tactggcatt acttggagat attgtgggtt ccgttctaga ccaacatata aaagcaaata 288660
   tttcaataaa aggagtcaca caaatttttg ggtttcccag tgcatataaa aattatattt 288720
   actctttatc atcatctatt aaatatgtaa tagcattatg tctaaaaaac atacatacct 288780
   aatgaaaaaa tatttcattc ctaaaacatg tccaaagtca caaagtgagc acatgctttt 288840
   agagaaatga cattgataga cttgcctgat gcagggttgc cacaactctt gtatttgtaa 288900
   aaagcacagt atttttcaag gtgcaataaa gtacagcaca ataagataaa gtatgcctgt 288960
   actggtaaga catttgcatg ttagaggttc gtacataaat tcagaggcct ttccaaatca 289020
   gcaaaatttc taggacttca gttgtatgga gtatgttaag agatcccttc taaggtgaag 289080
   gttaagttgt tgtatcactc agtaaagacc ctagactatc aaacccagat gagtaatata 289140
   ttgactccaa aattcagaaa ggcccataat aatgactagt gggactcttt gaattttgga 289200
   gtcaatatat ttctcatctg ggtgtgatat tctaggatct taactgagtg acttgaaaat 289260
   ctgctagttt tcagtgggtc ccagaagaag agaagtctat acaatagatt taggttgcca 289320
   tgcaagctgc tctgcccttg ggtcatatga tgcagtagat ccaatggtgt ttgaagtaac 289380
   agtggcatat atggatgcca tttggtgtca tttacaggcc cctataggta agttttatag 289440
   caaagtcctg ccatcctctg cagataaata ctctcctttt gagaaacagc tttaccctgt 289500
   tattggagct tagttgagac tgaatactta actatgggca accaagttac catgtgacct 289560
   gagcttccca tcatgaagtg gatattttct gagccaccaa accttaaagt tgggtatgca 289620
   caactgcact ccatcatcaa atggaagtgg tatagaccag atgggactca agcaagccct 289680
   gaaggcacaa gtaacttaca tgaagaaatg gctcaaatac ccatggtttc taatcctgct 289740
   atgctgcccc ttctctctcc tagccagcac ccatggcttc atggggagct ccctaaattt 289800
   aggtgacaga ggaagtgaga atgcatgcct tgtttacaga tggttctgca caatatgcag 289860
   gcaccatctg gaagtggaca gctgtaacac tacagtcctc ttctgaaata tccctgaagg 289920
   gcaatggtga agggaaatcc tctgagaagg tagaactttg agcaatatac ctggttgtta 289980
   attttgcttg gaaggagaaa tggccaaaca tgtgattata tattgatgca tggtcatggc 290040
   caatggcttg actgcatggt aaaggatttg gagggaacat gattgaaaaa taggtgatag 290100
   agaaatttgg aaaagaggta tatgaatata cctctctgaa tgggcaaaaa cgtgaagata 290160
   tttgggtcca gtgtgaatgc tcatcaaagg gtgacctcag cagaggagta tttttatcat 290220
   caaatggata gagtgaatca ttctgtggat accagtcagc ctcttttcat tgtcattaaa 290280
   ccaatccacc cctgtcgtta ctcaaaaagc tcatgaaaaa agtggccatg gtgacagaaa 290340
   tggaggttac gcatgagttc ggcaacaggc acttctactt accaaggcca acctgacccc 290400
   agctgttgac aagtgcccaa cctgccagca gcagagacct acactaagtt cccagtatgg 290460
   caccattccc cacaattata ggtcagctac ttggtggcag gttacttaca ttggactact 290520
   tacattattg aaagggcagc attttgtcct ttctagaata aaagctttct ctgcatgcca 290580
   atttcttttg tttgcttgca atgcttattc acagtgccct ggtggttact attattaact 290640
   ttattctcta aacaaagaac ataaggtaca gagggtataa atcagctgcc ctttgctacc 290700
   tggatatgga tagcaatttt ttcctttctt gtcaactctt cagcatctct cattaatctg 290760
   tctcctcatg caagaacagc actcttttca attttgaaag tttatattgt gtttaatatc 290820
   acctaggatt aatcttccct cattactctt atttttcata acttttcaag ccattcttgt 290880
   agggttttag gatcctgtaa gcattttgaa aacaaatttt acttcagaaa catattggaa 290940
   atgtactgca gaagcataaa tttgtatgtt aatagagatc atttaaatag ttttcttctt 291000
   taaatacaat aaggcttccc atttgatgat gtctttgttt ttgtgtgcct atgatcatca 291060
   atatcatttt caataccgac cctgtttcag tgcagacctc gtagataatt catgttgttt 291120
   cccactacct ccttctcact gatactcatg atgtgaatgt ggtttttttc tcccattatt 291180
   tcttttaacc acttaataag aaagttatta atttctatat gtttcatata tagcaactgt 291240
   cctgagtttt ttgatcattt gaataaattt gctcatttga atctagcagg tgtttcacgt 291300
   ataaggatat atctctgcaa atgatgacta ttctgccatt gttttctaat ttttgtattt 291360
   ctgttttgtg tgatagcatt tacttgcaac tgcaggacca ggtgaaataa tagtggtgat 291420
   aatgaaaacc cttgtcttct tcctgcctct aatgagaata attttaattc ctccttgtaa 291480
   agcctgaggg gagcgtttga gtttgacata ttaaatgttt attcacacac atgcatgtgt 291540
   ggataaagaa gggacagtaa tatctacctc atggggtgtt gtgaagatta agagacataa 291600
   tgtatggaaa gcacttagaa cagtgcctac ctcgtagcaa actctttaca atgatgaatt 291660
   tctatgttca tatgcaagag ctatccagct gtttactctg taaaaccgca gccataaatg 291720
   ggtgagttaa tacacacaag acatttggaa tggttcctgg aacatagtaa gggctcaaga 291780
   aatgtctaag ccattaatat actgcattat taaggcattt tgtcaagaac tgatgctgat 291840
   ttttatcaaa aggttatgct gaggtttttc tacttttgat ccatcagtca aatgaattgg 291900
   tctggaatat tttctagtgt taattcgttt gctttcgtgg aattagctcg tcttgcactt 291960
   acattaaatc agctgccaga gtttgcaact atttcgttta caaaattttt aaattaatac 292020
   tgaaataaaa atttgatctt agtttttttt agttgagctt tagtaggaac agttcgctaa 292080
   cttcacagaa attattggaa tcttttccta aatagaaaaa taatacaatg caagagtata 292140
   aataaattat atatgtatat atctaatatt tggctcccta agtatttaga aaatcaatat 292200
   cttccttaaa gagttagtga aaatcccctg tgaaacaccc tggcctgctt attgtttttt 292260
   gtcttatttt attctttggt agaagtagct tttgacactt ttaatgattt taattatttt 292320
   acttgcttta tttgacagtt taggtttcta cctctttagg ttccctgcct catctggagt 292380
   tagttttaat agtttatata ttccataatg agcatacatt gtattctttt cagtttattt 292440
   ctatggagtt gaacaaagtg atgtcttgtg aatacaatat tttcatctgt atcttcatta 292500
   ccttcctccc ataaatttgt attgttttat ttcactttgc tttttatttt ttcattaact 292560
   ttggttaaat taggtaatgg tttattttcc cagagtttta tgtatttttt tcttttattt 292620
   ttaatttatc agtattatct ccttcattat aacttatata ttttgttttt ccttttcctg 292680
   atttttttgg atggggtggt atggaaacca gtagtttgca ttcttttcta tttactaagt 292740
   ataagactag ccatgtttct ctgaatattt tcactgaacc actgatttta gttgcatact 292800
   attttatgct aatttatttt tgcattttct gtaattttag ttttaatttt ctctttgatc 292860
   aaaacttgct tacaaaagag ttttcattta tcgattggtt aattttttaa tatttaaatt 292920
   ataaataagt tttttattaa catatctcac ggcagataaa tcagataatg ttttctgtat 292980
   tatttctgct tttaattgtt tttgcttaca gtgcttttat ttttgcaaat gtttcagaca 293040
   tccatgaaaa gaatatatat tctatgattt tagtcttagt ggttgctatg tacatagtta 293100
   aatgtgcctt aataattagt ttaattataa cgatcttaag tgttttatta attagttttt 293160
   ttttaagttt ttttcttgtt ctatactaaa tcactcacaa gctgtgaagg atgaattgaa 293220
   gtccccctct actaaaagaa ttctatttct atttggtttt cttgccatgt tactcattcc 293280
   tctcttaggt tatgttattg tggttactga ttttttttta agtttaatat tcaatttgga 293340
   ttatccttgc ttgcttaata tattgccttt cttggtctca tttaattctt tatgtcctga 293400
   aatattttta aaacaccttt attgagatat aattaatata caaagaactg tgcatattta 293460
   aagtatacaa tttacaaaaa aattaattaa aggaattcaa atagaaaata aataaataaa 293520
   attatacctg tttgcagatg tcatgattct ataaccagaa aactaaaaac accacacatg 293580
   gacatgaaaa gtgtttgaga gaatacataa atacagttaa gttttgggtt acacagtaaa 293640
   catataagaa ttagtgacat tacactaaca acaagccatc tgaaaaataa attgagaaaa 293700
   caatcccatt tatagtagca taaaatagag tcaaatactt aaaagtaaac caaagatata 293760
   aaatacttcc atactgaaac atacagaaca ctgatgaaag agattagagg agacacaaac 293820
   aaatggagaa acaattttgt gttaatggat tggaagactt aatattgtta aaatgcccat 293880
   actactccaa atattctaga tattcaattc tatctccacc aatcccaaat gctttatcta 293940
   tagcaaaaga aaacacaatt ttaaaattta tacggaacca caaaagtcct agaatagcca 294000
   aaacaatctt tgggaaaacc aataaagctg gagacattac acttcctgat ttcagaatat 294060
   attacaaagc tgcagtgatt aaaacaatat aatactggca ttaggacaga catttagatc 294120
   aatggaagag aatagaaagt tacagaaata aacccacata tatatggcca actgatcttt 294180
   gacaaggctg ccgtcaatat acagtgctgg ggaaactaga tatcaacatg caaataaaaa 294240
   aatctgactt tttaatattg aaatgccctt tctttttttg tttcccttta gaaacaaaaa 294300
   gattttctat cattttcctt tcaatctttc ttaaacattt tgcttatatt ttttgaaaga 294360
   atatttgagt tttgattttg tgacccaatc tttttattta aactattaat tgtcactgat 294420
   tagcactttt tcttctctgg tttgtagata gttttgagtg tgataacatt atattaatta 294480
   aagctattct ttctgatttt ggagcattta ttttgtttta tcttttactg tgctgtctgt 294540
   gatggttttg ccaggggagt ttgcatatac tgagctaatt attaccttta tgacaatgag 294600
   tttgtatgta tcacatttgt ctctctttct ttacacattt tctaatatat acttgccaga 294660
   gataatagaa aaacacatgt caccactttg cctgcttcta cctttgaaac tcttcatctc 294720
   taaattttag atgattatgt tttttctgta tgtttaggct tatgccctta tgtactcaat 294780
   tattttattt ctttaaagta aattttttga cccctggcta taaaatatgg ggaaatctca 294840
   taaaatctta caccttctct ttcccatcat actcccatta gttatacaat ttttatcgca 294900
   tcagagttta taattttcac atttatattg aagacataat gctgcatttg tttcagcttt 294960
   tattcttgag ttaaatggca tcaaccctca gcactggccc ttcatgacaa tttctccaac 295020
   catctctctt tttttttgtt caagtttatt ccatcattgt ttccttaaga aggcgtcatt 295080
   taaaatattt tctctgttct tggaaattct aaacaatgca acaatgtgtg tctgtcattt 295140
   tcattctcaa actactatat gccttgtata aaattcttgg gacacttctg ctcactaagg 295200
   aatttaggca ctcttctagt gttttccagc agtgctgcaa aagttagatt aaaatattct 295260
   ttatttaatt taatgttcag taactttatt aggatatgtt tcagggttaa tcattatgtg 295320
   tcatgttttc caggtctcaa tatacttagt gatatctaaa aacaagattt tttaatatct 295380
   gagaaaaaca ttgaattaca ccttaatttc ctttgtttct tttctcctct tcagaaataa 295440
   ctattttcct tcaaaaacaa gtattacaag aatctgtctt ctatagctat tattataatt 295500
   tcagttcaga tcatatcttt tcagttcttt tatagcatat ttttcctttt gcttattttc 295560
   ttttattaat taattaatta attttttttg acagagtctt gctctgtcaa ccaggctgga 295620
   gtgcagtggc tcaatcttgg cttactgcaa cctctgcctc cccagttcaa gtgattctca 295680
   tgcctcagcc tcccgaacaa ctgggaataa ggcaggtgtg tgccaccaag cccggctaat 295740
   attttgggtt tttagttgag atgggatttt gctatgttgg ccaggctggt cttgaactcc 295800
   tggcctcaag tgatgcaacc atctctgcct cccaaagtgc tgggattaga ggcatgagac 295860
   actgcagcag gccatttatt ttcttttttt tagaaacaaa gtctcatttt gtcacccgga 295920
   ctggagtaca gtggcgtgat catagtccac tgcagcctga gatcaagtga tcctgtcacc 295980
   ttggccttcg aaaatactag aactacaggc atgagccatc tttgcttact tttcttaatc 296040
   ccgtgctcca tatagcatcc tctgtttttc tgtaacgttt atttcccctc atattgtttc 296100
   caaggttacc tttatttttg cattttgtat tgatcttatt tttatttcac cttttttttg 296160
   gttctgccac tcatatttca agtccttgtt tcgcttcacc atttccctcc ttaaatgttg 296220
   tttgtcagct ttgactcatt ggtttttttt gggagattac tccattgatg tttttctttt 296280
   tccttttatt ttatggtaat ggcaacagtt tttatctatt caatgggaac attttttggg 296340
   ggaagtgtgt gtgtgtgtaa attataatat atttttatag acccagtgct aatttttttt 296400
   ttttcatttt gaatcaggtg atttttagtc aggctaacag atttttattc tctatgcaag 296460
   ggagtcctta gttaactgag taatatggct gtaggctcat cacatccact gtgttctcac 296520
   tcccccaaca ccttacccaa aggcggcttg ctgtaggtcc aagttgagtg tatgctgcct 296580
   cctatgcccc tacctcctgt gcttattgtt ggtaccaaac aggttccctg gaagctctca 296640
   ctttcattct cattctttca aaatgagctt tttaagtgtg gctcctgaac gtggtccatt 296700
   gactttgtct tctacaaaga tgtgctttct acagactctg gaccacctgg gtggccgtga 296760
   cagcactttc atcttgttct tcactgaatt acatatcttg tgttttctag ttttatgaaa 296820
   aagtggcttt acactgttgc tttcattttt tttctattgt ttctatatca tatctgtgag 296880
   agaattaggg aaaactgact ttagagtttt aaaatcaggt ggcctgttat tcttgtaact 296940
   atgttaccgt aatcatattt caaaaaattg gcatttccaa aatgataatt aaacaaatat 297000
   tttttccatg aatttagcaa tgtaatagct tcatggagat ttggaggcaa taagtggctc 297060
   acttctttga aaacttacct tacaccttgt catgtgtagt gtgccaattt ttataagagt 297120
   gggagaaaga atcaactgca aataaataaa aagaataaag agaagacatc aggtaatcat 297180
   gaaacacctc ctaccaactt tataaaatta atcaggaaag aaggaaggac aagaaacaaa 297240
   actgaacaca gcttgcagca cattcagcat tgatcaccag gtcagctgct ctccgaccta 297300
   cttcctcata gttgtttcat gcctcttgcc tcagaatcac atagaccatg ttacaagatt 297360
   atagttctta actgctctac agataacaac ttgaacatta tgagatggta agttttctat 297420
   ttgagatatt cttgcatgtc ctgcatactg atgaaactac tgacattagc tggtctgaag 297480
   gaccccacaa gaagctgacc aaggaatgca gtttacatat tctgataatt ttatctccct 297540
   taccccaacc aatcaatgat ctgaattctc cagcccttca ttatccatga acccctttaa 297600
   aaactccagc tacaaactcc ttggggagat ggatttgagg gtcttctccc atctctttgc 297660
   ttggtgccct gtgaccatta aactctttct ccgctgcaaa cccttctggg tcagtataat 297720
   tggtttgtta ctgcatatcg gacatatgaa cttgttgatc ctgtaagaaa ttatggcaag 297780
   cctagtcagg agcagtttgt gggcatttgc ctaaggctca gtgttctgtc attattggga 297840
   tggacccagg gataagccca aatggccact tagctccagt gaactagact gtctctgctg 297900
   ccttgcctat caatggggca cataagtcac ttgtgcatag aaacagtttc tggttgtgaa 297960
   aaacattgtt ggtaagtttt atttgtgcaa ctggcacccc ctttgtcttc tccctatttc 298020
   ctggcctcct tggatgtctt gtgacctctc ctgaggtctt gtgaactcct tggatgttct 298080
   gatgtctcat gtctcttctg atgtcttgtg gcctccttgg aggtcttgtg gattctcctg 298140
   ggtttttgac gaccctcttt agtaatagga agggccttat ttgagggaat ttctcttaaa 298200
   ttaaaaggat taaaaagggc agtgcttggg agaaatactc ttgatttctg aaatttggaa 298260
   attgagtttg gaagtccttt tgtttgtctt tgtcctgtta tgtatgtttg tatgtgaaag 298320
   agatccctga aagaattggt agtgaagctc ggcaggccta actcggggtt accatccact 298380
   tttccacttt gcctcaagac cacctgctga actcctggtt ggaggtcatt ccccccgacc 298440
   ttgagtaggt caaaggtgcc aaggctagct gaggcaaatg tgagccttgc taggtcaccc 298500
   atgggtacta agtagagtga ttagtgtgta cattttgttg tgcatgcatt atttaggcca 298560
   gaatgaaaaa tgttcatttg gtttctccaa gcagcccacc gggctttatg caaaattggg 298620
   aagacaagag agatctttat taggtagtgc aataggtaac ttacccacta agggcacgaa 298680
   ctcctgtgaa aaaactatgg aaaagtgcaa aggcatgcca ggttttctag gattccagtt 298740
   ggttataaac catggctcag tcttgtgcac atttgaaatt gatgggcaaa ttacagtaaa 298800
   gtaaaaattc agagctcaaa tgagtttatc tgaaaacata aaattaaaag ttctctgtct 298860
   gtctttattt tcttttctgc ctgctttgta tctttcataa accactgagt ttgtattgct 298920
   atctcatgac tagaatttca aagcaaaacc tatagcatct ttgtattgtc tgtatgtgca 298980
   tttagataaa tttatatgta tgcacatgta ttgtgttata tatcatgttc atatggtact 299040
   aaactggctt ataattaaaa gaacactcat aaataaagtt caaatatttt tcaagttcat 299100
   gtgacttaaa tcattaatta aaaagctggc tttaaaatta ttgataaaat aaaaatagaa 299160
   atgtctccag aattgtcagc atatattttt gtctgggctt actgatgatt ttatatgttc 299220
   ctcttctaga tattttaagg tatcagattt tggcacaaag gttataagac tataaaccca 299280
   tccaaaaaca gaatggtctt cgttggtgta attttttgac aaataagact aatttaatgt 299340
   tttttgttca atgaaaacag ctaaatcatc taagttattg gcaaaagtac tcataagttt 299400
   aagtttctta cttaggtgaa ctaacacagg ctataaaaat gtcaaacaga aataaattta 299460
   aatgatgact agctttgtct aatatctcag ttatcataac taatctagat aaactgctaa 299520
   aaataaatga gtaaatatga atgggctaaa tacttaaagg taaacttttt gtttgtttaa 299580
   aatcttaaaa ttattttgaa tgcttgttag atatctgggt aattttcagt taagaaacag 299640
   ttacgatata gggaaacaag tttctaaaca ttgttaaatc atttcaacta taaaacacta 299700
   acatctaaca aacagttcag gatttcttgc ttcctaggtt ttcactaaaa cttaaggtta 299760
   cctgaaataa gatttctagt aaatatatag ttctgcatgt aaaacgtgtc aaaaaagatg 299820
   tgtttctcat tagaaaaaaa attaaaaaaa tataaagttg ggtttttatt aagaaaaaga 299880
   ataattttgt ctaattcaga agttatctga agattgattc aaattatgaa cttgaaaaga 299940
   ttatgaaaca aggtaaaaag gccagtaagt agaggagaaa tatgtaaaaa caattacaaa 300000
   tataaaaata tatttttcat aaggagggtt ataaaaacaa agataatact tttatatgaa 300060
   aaaatatttt gtgtgatata tttttgttct aaaataaaat gactagttat ttagaaaaaa 300120
   aaagaaagtt cagacatgtc atagatggcc tgtataagtt gtgacaagac tcatgaaagg 300180
   gaatttatga aagaaattta gtatttgatt aggttgacta taattgaaaa tattattcat 300240
   aatagtcctt ctaaagattg gttatcctct gataaaacaa gatttcctta aggtatgaat 300300
   ttactctaaa taaaattaca agaggtggct gggcacagtg gctcacgcct gtaatcccag 300360
   cactttggga ggctgaggcg ggaggatcac gaggtcagga gatcaggacc atcttggcta 300420
   acatggtgaa accccgtctc tactaaaaat acaaaaaaca aaaaaaaaat tagccaggcg 300480
   cgatggcggg tgcctgcagt cccagctact cgggaggctg aggcaggaga atggcgtcaa 300540
   cccgggaggc ggagcttgca gtgagccgag attgcaccac tgcagtccag cctggacgaa 300600
   agagcaagac tccgtctcaa aaaaaaaaaa aaaaaaaaaa aaaaattaca agaggtttac 300660
   cttttaattg tgttacctgt ttcattttga aaactcagat ttatatctca gaagttcgac 300720
   gtttgctgtt tctcattgtt ttcacccttt tctccctttg agaaggccag agatgaaaac 300780
   tctcttcttc atttttattt tattttactt tatttatttt attttatttt attttatttt 300840
   attttatttt atattttatt ttattgccag ctcctgtaac attctgtctc tgtttttcac 300900
   tttactgcta tggcctgaca ataaaatatt tatcttaaag ctctagaaaa gcaatgtttt 300960
   tctttagaat aatttgattc tgtatacttg acttttcttg ctatgtctgg atttttccat 301020
   gtaacagaaa acttctcaag gtgttactag gagtcatgca ttactagttt tctagttttt 301080
   tgtttcctct ataatataat ttataaccat aatcctggac acattcttcc tgtattggat 301140
   tgattcaagt gcccttttca tcagggttga ctttaaggtt ctctaaatgg gtttcccata 301200
   aggagaagca atcacacttc actctgcagg tggttttcct tggtgttttt gataacaggc 301260
   ctaagaagca agattttata ttttatcaat ataattccta tgttgttagt tttttgatta 301320
   cttaggaaca ttcagcttta agaaggaaag tttttacttc tgtataactt tctgtagtgc 301380
   ttttaaagtc ttgattatca ctttggttaa attaataact attattttac aatgacctgt 301440
   gattctgttt tgatcaaatg ttttggcctt ttaacatctt tgatatacat cctcaaaatc 301500
   aaattctaaa ttaagtctct gacttagact tattgctgat acttatcaaa gctgtaaaaa 301560
   ctgatccctg caagatttta gcatcttttt acagcttcca gtcaaaccat gaactccagc 301620
   atcaccaact ccagcctgat aattacatgt actgaaagag atatccattg aaagactccc 301680
   tcgacccccc ctttaaaggg actttcatca ggtgctatta attaatcctt gtactgttaa 301740
   gttaaagggt tttgattcct gggtgcacat atctcatctg aaaaaggcac caactcctac 301800
   taattcttaa aatattgata ctattatctg acaccatact caggttaaca aaagccttgt 301860
   cttcagaccc agaagaaagg tagcatgaaa atgatctgct tttgtgagac ataagactag 301920
   aaattaaaac tattcaatct ctctaggctc agtgactact gcaggagaga tgggcatgtg 301980
   agattgtaag ggcctgtttt gagggataca attagttcag atgctccaaa tcaaggatgg 302040
   cacagagtta cctaaacagc caacaagttg aggaattttg ccactctgaa ttccttaact 302100
   caaaagggtt taaccaaatg cttatgtttc gtattgctaa atggctacaa gtcagtatct 302160
   aagaccagaa ttcatttcta taaccttact ttttggctct tatatgctga aaaggttttt 302220
   taagcattat tgagtgtctg cccacttcca ttcccatctg gcccagaatg tttaattgac 302280
   tacatgtctt ttgactgaaa gtcatttggc cataggggtc tcaccaaggg acaagatgga 302340
   cccaaagcag gtgaccacac caacctgaca atgatatggc acaaaataaa aagctttgca 302400
   atccatgctg cttctggcat atcttgacca aaaatactga acccagaact ttttggggaa 302460
   atagatttga gagtctcctc ccatctcttc acttggcacc ctatgatcac ggaactcttt 302520
   cctgctgcaa atcctgctaa ctcagtgtaa ttgatcagtg ggcaaacaaa cctgttggtc 302580
   ctataatagt caaagtcctg tgatgcaaat aaaccttaat ggagagacag ggttagtttg 302640
   tgtttggtgg tttaagaggt tgtcatcaaa gagtgagtaa ttaagatggg cattgaatgc 302700
   taagagagag gcatttatgt gcagttgttg gggaggaaca ttctaaaaat aaggcccagc 302760
   aagtgcaaag tcccaacttt ggaaagataa aagctacttg ttttatggga taaaagaaaa 302820
   ggaaaaaaat acacataatg agagagatct gcaggaacca ggtagagtca agttgtgtag 302880
   gcctgagtca aggacttggg cttcattaaa gatttttgag aaaagaaaaa cacttcttac 302940
   aatgaaatca gatgacttca actgatattc ttaatgatat actgtatata aaaatataga 303000
   cattcttgat tggtcaggta ctaataaaat gttttccaca catacagttt tgtgaatttc 303060
   agttacaact gaaaacctgt ggattcccct gacctcacgt gctcttccat ctagagagtc 303120
   tgaattgtaa aggagaggtc caaaaccagc attgatgaag acctaaatga cctatcactc 303180
   atgaaggctt aggtctattg tcaaaggctg acactctagc ctgatggagt gcttccttga 303240
   aacaggttct tacaaacatt gtctgattta attttcacaa caatccattt gaaataggtt 303300
   taaattgctt ctattttaca gttgaagaaa cacactttaa aaggtttaag gaattttcct 303360
   gaggttactc agctagtaag tggtagagac caaaaaaaga acttgggtct gttcattgcc 303420
   cagcctgggc tctcccgcaa agcctgccac ccacaaggct tgagatggag agaatctggg 303480
   gtaggaaata cctttctgtt tttttcaaaa ccactccctg ttggcagaac tctccttaag 303540
   ctacgcaaga gggttgtccg gttaagccat ttatctttct ctatcagaga gcaggctctc 303600
   ttttctcata gctgtgtagt cttagcagtc agaattgata acagctgctg gacacctgag 303660
   ctgtattatt ttgccaggag ataacagagt gaatgacttg tagcagactc agaacactga 303720
   tgagttcaag gcccacagta gagattgtga aatgtaattc ccagataatt caaggggaag 303780
   agaagctaaa caaatctcgg tttcactctt cgaatttgta ctctgagaac aaattgagaa 303840
   atctgagaag aaaagtcgtc atatttgaag tgcttcagag aaatttttgt cttctttttc 303900
   ctttactctt tggggcttga ataccaacaa gactcatttt tgaaattaga atttctcttc 303960
   catagggagt gagatttaac ttaagataaa atttgatgct tccttcagag agagcaaatg 304020
   tcttagatac aggtcaggga agcttttgct ggccactcat gtgcatggat ctcattcttc 304080
   tgctttgcct ttttagtaaa tgggtggcgg gaatgtaggg gttttttgat aattgaaacg 304140
   aggtgtttcc tttcttgcaa agtgctaaat gcattaagta cttttgcaaa ggagatagta 304200
   cgtgcctcaa tttagacaga aattttactt taaagaagtt aattcgtgat gaaatgaggg 304260
   tctatcatgt gccatgtgtt gaaacttagg gatgagtcag atgctggtcc tgctctcacc 304320
   aggttcacct tcaccttggg agacagacgt gtagccaagt aactccagtg tgcaatgagt 304380
   gaggctccat acactttctt gaatttttag gagtcccctc ctatctttca tcattactcc 304440
   atcttgttag aaaggaggca tagctgaata tcattttaga agagagactt gccagtggat 304500
   gtatgacgat ttatatggag ttactaaggg gttcttagtg ggcttttgag acagatctga 304560
   aggtaaattc taatgcttct gcttcttgtc cctggcacct tgcatatagt tcatatttga 304620
   ctcctcaggg agatgagaag ggattaaagg gaagaacaca gactttgtga ctagttccag 304680
   cacagtagcc gccagtactc ttagttatgt ggctgtctag aactgacttt gcttattaca 304740
   tcaggcactt ctgtgacttt ctcagggacc ccatactagg aaattccagt ggcaggtcct 304800
   gtgatatggg tgatgttttc tcctagaagg ccacataatg attctgtacc ccaacccctg 304860
   ctataaccca ttctgaagtg gccccattct aagagcctca ccaggtagct cttacaggtg 304920
   aagttcattt ggacttgcat caaagctgga actttcctac atgcctcaca tcaggttcac 304980
   ccttttcacg gctgttagta gaagtcaatt taagtcttaa tccaacccct tatctctctg 305040
   tcatgtcatg ggcagtcaat agttacagtc taaattttgc agtttttagc tcactggtta 305100
   ataaagacat cacattaggc tgggtgcaat ggctcatgcc tgtaatccca gcactttggg 305160
   aggccgaggt gggtggatca cctgaggtca ggagttcgag accagactga ccgacatgga 305220
   gaaaccctgt ctctactaaa aaaaaaaaaa aaaaaaatat atatatatat atataaatta 305280
   gctgggtgtg gtggctcatg cccataatcc tagctacttg ggaggctgag gcaggagaat 305340
   ggcttgaacc tgggaagtgg aggttgcggt gagccgagat cgtgccattg cactcctgcc 305400
   tgggcaacaa gagtgaaact ctatctcaaa aaaaaaaaaa aaaaaaaaat gacatcacat 305460
   cagatgtaaa agactcaaga cacattcaat atgtgtagga tcacaaaaca aacacaacaa 305520
   caataacaac aacagcaaaa tacagagtct attaaagaca aaacagcaga aacaaagtga 305580
   aaaatacata catatgcagt atggaaaaga tcaatgaaaa gcccagagag taataaatat 305640
   tattttccag gcagttttgg ctccacagga catgcatcaa tgtccggaga tgtttttggt 305700
   tgtcacaact gaaagggaat ttgctcctgg tatctaatag gtaaaagcca gagatgctgc 305760
   taaacatcta atacacagga cacatctccc atccacctac tcacccaccc acaacaaata 305820
   attaccctga cccaaatgtc aatcatgctt aggggaaaaa ccctagtgta gaggaattta 305880
   ataagaacat gagtgaataa aacaatgcaa taataaaatg acaaaacaag agaacgaaga 305940
   acgggtaata gggagataag gaggaacatt aagaatccct acaataacat tgctagaata 306000
   aaatagacct ggttgaaaaa cagatgccta ctttcatcat tttattttat ttgagacaga 306060
   gtctcactct gtctctcagg ctggagtgct gtggtgctat tctagctcac tgcagcctca 306120
   acctcctggt ctcaaatgat tttttatttt taaggttgga tgtgattgga catgatcgca 306180
   cattgatttt cccacctgca tcaccaccac taacttggaa acatgagtct tgtcattttt 306240
   ctcaaggact cacttctaca aatttctgag tgaggccttg ggaacaaatc cagtccctca 306300
   ggacactagg tctgggaccc ccaaactttc cctgactctc taacaagtca agggaaaaaa 306360
   ataatacaat attaagggtt aaactacttt tcattcccaa taggaaatgt tatttttctc 306420
   atcttgtggc tattggggtt ttgagcacca gcaagggaca cttaataagt tgtagatgca 306480
   tcatgtgaag gggcagtaat aattatttca tctgaaataa accagagtgg gcatgtgtct 306540
   gctcatagat ggaaaagtat gcattttgca cttgggtaac agttggcaac tctgactaga 306600
   aaaccttttt tgactatggc aagcatcctt cttacttgga ggaaaaattt atgcaaaaga 306660
   cctggcaaag ttcctggaac atatagtgtg cctgatcaat gaacatatta ctttctcacc 306720
   ttaaacaagt ttactgtatt tcattattta agaaaaagcg ggccgggtgc aatggctcac 306780
   gcctgtaatc ccagcacttt gggaggccaa gttgggcgga tcacttaagg tcaggagttc 306840
   aagaccagtt tggccaacat ggtgaaaccc cgcctctact aaaaatacaa aaattagttg 306900
   ggtgtggtgg cacacacctg taacttggga ggctgaggca tgagaatcac ttgaacttgg 306960
   gaggtggagg ttgcagtgag ccaagatcac accactcact gcactccagc ctgggctaca 307020
   gagtgagacg ccatattcaa aaaaaaaaaa aaaaaaaaaa aaagagagag agaaagaaaa 307080
   gactcatatt caaatcttgt cttacttaat atacttttcc taagtgtaca ctcaactcct 307140
   aactctttaa taagactaga tctctttaaa gaagataaac aaacagcaaa gcaatatgta 307200
   tgcagttaat cctcaggaaa ggataagata ggaagtaaat ggaaatgata tcttgaagca 307260
   aatccttctt agactccttc gctgcctctc agaagaatgc taaagtgtga cattttttct 307320
   gatcacactt aatttatatt gcatcactat tggtgtagcc attctttaat tgaatttatt 307380
   tagtctcttc ttcttgcact ttgataggcg aaaataatac agcggggacc aaactgcagt 307440
   tgtttattca aggcacttaa ggggcaaata agttgataat tatagtattg tttgataatg 307500
   ataaattatg cttaatgaaa aatgcaaatt aagtataatg ataaattatg cttaaataca 307560
   gatgagcaca tagggagagt tcccacaaat tattgggcca cagaaactaa cattttccgg 307620
   agaaatatac atcttgtgga ggcctgaagg gtgaatagga gttgtcagga ggcaaaagag 307680
   gaaaaaccca tggagaggaa ggattaacat gttcaaaagt ccagcagtaa gtaagagcat 307740
   agtacggctt tccagtgttt ctttatgcaa atgtaaacag atacacatat acccttctta 307800
   cctactcaaa agatagtgaa ctatgcacac tccttttagc cttgctttat tcaaataaca 307860
   atatattttg cagaccttcc catgtaggta cttaactttc cagctgctta tgattttaat 307920
   gagtacagcg gaattaaacc agactcaact gatggtcact tggatgattt cttgcaataa 307980
   tcttgcaaaa atgttgctat gaataaaatt gaaatagttt cctagcttgt catttgtctt 308040
   ttacatatgt tttggggcct aggcataaag gtgtttattt atacatagta aaaaaatact 308100
   ttattttatg gcttgaaatg cctcatttgt tagatataaa attcttataa tatctgaatc 308160
   tatttctaaa ctttctttct catttccttc gtttgacttt ctattcattt gccagcacta 308220
   caaggtgaaa ttcttgagtg tttatagtat atttaatatc ttttaaggct atatctcctt 308280
   attgcctttt tacttttagt gttattatgg tcatattgtt catttttttt acataagatt 308340
   tagaatcagc atatctaatc ctgaagtaaa tcctgattat tttcaatggc ttaatgttaa 308400
   tttatattac agagagagaa ttgacatctt tatgagactg agtcattctt ttgaaaacat 308460
   gttatatatt tctacttgtc atgtctattt ttgtgttcct tgaggacttc taaaaaagtt 308520
   tctgcataaa gttgttcttt gcacatatct tgttatgttt attcctatgc ttgtttttgt 308580
   ctcttattaa aagtgaagtc ttctttttca ttatgtctcc ttttagattt tttttttaat 308640
   atgagggcca ttgattcttc tttattagtt ctatatcctg ctatctcatt gtttttagta 308700
   gttgttaaaa tttttacaaa ctctctaagg cctggtaaga cagttacatc atctacaaac 308760
   tatgtgtgag ggtgttttaa cctccttcct accactttta aacgaatata ttttactctt 308820
   ctgatacatt gctagtgcct ttagcagaaa agtttgataa taattgtgaa agctgagaga 308880
   gtgtcttgtt cttgacttta ataattgact atgtcacaaa gtatgtccct gcattaatct 308940
   agatttgtgt atgtgtgtgt gtgtatacac aagcagtcac atgtaagtat gtatgtaatc 309000
   attcatttcc atcatgttgc atttgttgtt gttgttgttg gatgaagaat gtagctgaaa 309060
   gttgtcaagt acctttcaat ttctgtagag atgctcatat aatttctata attaagtaaa 309120
   ataacataat taattatatt aatatatttg ttaatagtga atcattcttg aattcttagc 309180
   atgaggttac ataaaagaga tgagtcttgc agttttgttt ttctgcaatc attatttttg 309240
   gaatcaatat ttcgtcatta caataattca gaaatggccc ttttgtcctc taatctctgg 309300
   aacagtttaa ctagtatggg aattgtggtt tgttcagtag gattctccta tgaaaccatg 309360
   tatttttgat attaccttag cagtacacca ttgtcagagt tttctccatt tttcgtggaa 309420
   attaatctat tttcgtttct attcctatga agtcagattt tggtaaataa tattttgtca 309480
   aaatagttta catttcattt agttttctat ttatttgcct tgagtttacc aaaacgaaac 309540
   tttctaactt aaaaaaaaag ttgttatttt tatatcagtg attgttttcc ccttatcata 309600
   accgtgtttg tttcttttgt gttttttctc tttttttctc aggtaattta atcttttatc 309660
   aattttggag ttcttcaaag aagtaatttg tttatttatt gctgaatttt agcttgaagt 309720
   attctttctt ttcttttcca ttaattggtt atttttcaac cttttgttct aatttttagt 309780
   ttgatgctta atttgttaac ttttattatt ttttgtatat tttatgaaga tttaaaacaa 309840
   tatttcactt gagtatttgt tttgctgcat cttgattctg aactatagtg ttttcactat 309900
   tactgttttc cagaaatttt gcaattttga tttgtatttc tctttttacc taaaagttgc 309960
   tgataacaga ctaagttttt catgacgatc aaatattagg tttatatgta taatataaac 310020
   atcaggattt taaacacatg agaaaactta ctctctgcat agcagagaat aaatcagagg 310080
   ggaccaggct gtagaaccct ctttgtatgt gaaatatgct tatgttcaaa gataaagttt 310140
   aggttgcctg agattgagca gtagtttggg caacaacaat aaaacaagca gatggatttg 310200
   agatatataa agggagtata ataaactttt catggtgatt cattaaaagt gctacctgaa 310260
   gctgaggaaa gagttaaggc caacaaagtt cagacatggg caactccatg ggtggtagag 310320
   ctacttactg aggcaggaac tgatggacca agaacaggtt tggaaagaaa gacatgaaga 310380
   ggtgtgagag tttggatttt agatcctgag aagcatacaa gcaatggtgt tatcaaagca 310440
   gttggatata taaaagactg tatggtgaga gacaaattgt gaagtcatta gtactgaggt 310500
   gataatttca tatatagaaa cagctgatgg aagaagttta aagtaacaag aaaaaaagca 310560
   ttgaaaaggg aatcctgagt gctccaggca tttatgggac aagcagaaga agggtaatct 310620
   ttgatagatg atgagaagga gccagtctgt catcccactg aattgtggct gccttgagga 310680
   aaacatagaa tctgaattat ggaagactgc tgaaaaagta ttaattgcac ataaatgaat 310740
   gcatgggcaa agcaattttc caaaactgat acagattatt aaaactatta tactcattta 310800
   attctagaaa actgcatcat gaaataagtg gtggttgagc tggcacttct aggaatggac 310860
   aaacctctgt gtagaaccca tggctccatc actggagtcc gtattcagca gtaaaacagc 310920
   ctctctcagt catgcttgct gtgagttaca cccagccagc aggatcatct tgacagctag 310980
   ctatttaact aagttatcag taagcaggta atagtccctc agttcagtca aaacagtcaa 311040
   attagttgta cctgtagaca tagcctttct ttgccaacat ttatgacacg gacaggaatg 311100
   aattaggaaa tagaacaata gctccctgta tgtgaaagat gtgtcgtctg tctaaagaga 311160
   aaacacattg tgtttattga gtgggaattg tcatggaagc tttgcatagc tttaaaaaaa 311220
   aaaacacaat atgctaactt cattccagtg attgaagcac acaattttgg aaggttttat 311280
   ggaaagaaag aggaaaaata aatagagaag ctgaagtccg aaataaatgt gtgagtcagc 311340
   cctcatccaa atacaattat taccttcacg ctttgccctg ttttgtttga cctaggaaaa 311400
   tatattcaat gggatttcaa agaaaaatca aataattttt ttttcaaaca ctgtggcaaa 311460
   aacccatctg gccaaaattt tacataattg cattctcaat gcaacccaaa tacatatttt 311520
   taaaaaagcg gccaggtgca gtggctttca catgtaatcc taacactttg ggaggccaag 311580
   gcgggcagat catgagctca agagatcaag accatcctgg ccaacatgtt gaaaccccgt 311640
   ctctactaaa tatacaaaaa gtagctgggc atggtggtgt gcgcctgtag tcccagctac 311700
   ttgggaggct gaggcgggag aatcatttga ccctggagac gcaggttgca gtgaactgaa 311760
   atcgcgccgc tgcactccag cctggctaca gagtgaccct ctgtctcaaa aaaaaaaaaa 311820
   aaagaagcaa aaaaaaaaaa aaaaagaaaa caatcgatta tatacacaca atgctgtgtt 311880
   cttaggaatc cactgtccta ttctctgtca gtgaaaataa aaacacaaac acaaaagcac 311940
   tctacccatt taaagctgcc tgattatttc ccctctgaca aactgctgcc cctttgagat 312000
   agctgttaaa tctcatggcc tttatctttt gacattttat catattttgc ctaaggagat 312060
   ttatagaatt gtgctatgat tttgtgccat gctttaactg taatgttgtg ctgtggtttg 312120
   gctccaacac ttgatttcct cctatgtctc ttctatgcat gaactcaggc tgttctgcac 312180
   attaaaaggg cccagttatt tattggatga gtaactgttg ctttcttcct ctcctccctc 312240
   tctctcttcc ttcccgccct actttctgta tacttatctc taaacaaatg gtttatggat 312300
   ctctgcccac ccctctgcat gggtgacttc cacgggcagg gaccgtgcag tactttggtc 312360
   ttcctgccat gtttcccaat taaatctgtg actttttctt tcccttggtg ccatccaggg 312420
   ctgcagtcag ttaactaaaa ttcaggacca gcgtgtggtg gactgcttcg tttatagaca 312480
   tggggaattc aatctcaaag tacagtttca gttttaattg ctaatattaa gtggtttttg 312540
   aatttctaat gagtacctaa ttagagcaaa tgttttcctt actaacaagt aattatagca 312600
   ttccaaacac gtttacttta attaaacaaa actggttgac taattacgaa tggcaaggca 312660
   ggacacagcg gatttctatc ttcaaggaac tccagcagac attcttgtgt gggtaactac 312720
   acattaaagc tcacttttgg gtgctctcag ggcggctcct gatagtacca gttacgcaaa 312780
   gcgaagttgt tcaactggag gctcagtgcc tcctccatgt aatagagaca tctccgtggg 312840
   gttcttctca actcttttca aaatttcccc attgaacgaa aacaaattat gcctgagtgc 312900
   atgtggtggt tgcctgaaac agctgtcaga atgtaaagag tttttaaaga ctcaagttta 312960
   atttaaaaat tatataccat ttgcattata tgcaataata acctgtttta attctagttt 313020
   ttaaagaaac attttcctca acccctcaat cctcccaaag ataacaaaca ttcaactgga 313080
   gcttactgac ctcctagcta tagttgtggt ttgagagcag gaatttcggc gactgaatgc 313140
   aaaatccatt cttttaaagc tatggtatat gcctgtgggg tatactccaa agtatggatt 313200
   ctgtttggcc atcagtattg tggatggcta tacttcagtt tcctcttatt gatgttacat 313260
   attgaactac tgctgaaatt tggaattaga ctctgaaatg ttactattgg cctttatgca 313320
   gtatctggca actgtgaaaa cagataatta ggtatttgtt cacacttgtc tctttgaatg 313380
   aagctgtcat tgtagtctga gtagggagtc acttaaagga aatgtactcc taaccctttg 313440
   aagaggccaa tcatggtaca caaaccacat gagtcaccaa agctgatatg gacacatcca 313500
   tctgtatgtg cttgggtaaa aagttcatcc gggagagatg tgactgagga aagatgatca 313560
   tgaaaagcag ttgtgagaga atgcttctgg ggaaaatgga tttctcaagt cctgaggcca 313620
   ggttcacaac ttagtaagaa aaagagaata ttcacacaca ggaattgact tgcatgatgg 313680
   cattgagcgg aactggttat ggtgtaggtc aacaagtagg aggaacaaaa tagtacggaa 313740
   gatgaaaatc aaagatgaaa atggaagaga ggtagaacat agagcttaac agagcccagg 313800
   cctaaaattt gaggttgtac aaaaccatgc ctgagtgttt cattgtgtta cttaacacat 313860
   gcccgtccat gcattctatg agtatttcac tgcccttttt caggtgctgg tcatgtaggg 313920
   ataatgacat attcataaac gggtgtaata tgaggtaaga aacgacctgc cttataaaag 313980
   agaagcaggc aaatgcaata ggtgttcaga gatcccagcc tggccttgct tctcagcttc 314040
   tttgcctgct caattctgtt ctgtgactct cctcagagtg ggttcatgct gattgttttc 314100
   actagtcctg cttcgccttt tcccttaaaa tccacatcac caactcagag tgttagaatc 314160
   tttttttttt tttttttgag atggagtctg gctctgtcac ccaggctgga gtgctgtggt 314220
   gcgaactctg ctcactgcaa gctccgcctc ccagggtcac accattctcc tgcctcagcc 314280
   tcccgagtag ctgggactac aggtgcatgc caccatgcgt ggctaatttt ttgtattttt 314340
   ggtagagacg gggtttcacc atgttagcca ggatggtcta gatcttctga cctcgtgatc 314400
   ctcccacgtt ggcctcccaa agtgctggga ttacaggcgt gagccccttg tcaggcagtg 314460
   ttggaatctt accccagtga aatacaatag tgacccatgg gccctgaaca ctcacatgtg 314520
   cttagcactg ttctaagggc tttttagata ttaatgaatc caatttgacc aagggtgaac 314580
   agctactaca ccaaagagct agtatctgac ccaaagaggc tcatttccta gccgaagtct 314640
   aaaaacaaac aaacaaacaa acaaaaactc catagtatct atttgatgcc tcctttgtta 314700
   tcatgaagta tctctgattt atccatatgg aggggtctct gtctcctctg aactcacaaa 314760
   gcattcttta tgcctctcat caaatacctt tctcactatc attctacatt tggggagggg 314820
   gctcttggaa gtatgactca tggagttttc attgttttaa atacattttg aacttttgaa 314880
   gatgttttag cagaaaattt tagagtaata tcaataaata tttgtaatgg aatgattgat 314940
   cctatatcta acatacaaag aaaatgccta aatagtctca gattctcatt ttaatgtttt 315000
   ttaattttat atttttatat ttttgttggt ccttggaatg ataggactta gggatttttt 315060
   tccttggctg atcttgcttt gaaggcaaag ttataaaaat atactttgaa tgatttgtga 315120
   ttaatttatt ttagtataac ttttttcttt tcttttcttt ttttttttaa taaacagagg 315180
   ctaagcaaat ctttggagat tttctctagt tccctcttag ttctcttctg tggtcctctc 315240
   caatgtccta gcgtttgcta tttacttggc tagagcctcc agtctggtat ctgatccagg 315300
   cctgcctttc ctaaaaggcc ctactatgct tagctctttt taaaaagcca gctttaaagc 315360
   ttttattagt attctaacag ataaatttat acttaagtat taataattgc ccacttctca 315420
   ttattattat tattgttgca attgttatct cagacagtta ggaataatgt ttggaatggc 315480
   aataatggta ttctttagtt agagatagta ctcctgcttg atacttaaag tgcctcataa 315540
   aattcaaagg aaatcagaca tccaatttct tactttagtc gatgtaacta ttgttcactt 315600
   aaaacactca tgagttcttt tcatgagtcc taactcagag gaaacttcct agagctcatt 315660
   aaaactttag gctctgttta ggtgacctat aggctgtgtt ggcatataat gattttgtct 315720
   tttgagctcc aaagatatac cattaatcta atgttatcaa gatttgaaaa atggatttat 315780
   aaaaagtaaa cacagctgag cgcagtggct cacacctata atcccagcac tttgggaggc 315840
   caaggtgggt ggatcatgag gtcaggagtt tgagaccagc ctgtccaaca tggtgaaacc 315900
   ccgtctctac taaaaataga aaaattagcc gggggtgatg gtggccgcct gtaatctcag 315960
   ctacttggga agctgaggca gagaactgct tgaacccagg aggcagaggt ttcagtgagc 316020
   tgagatcacg cctctgcact ccagcctggg tgacagagcg agactctgtc tgaaaaaaaa 316080
   aaaaaaaagt aaccacaagg gtattattct cctgaagaat cctttgcttt ctgatatgat 316140
   aacatttgct tatcagaatt cagtttctgg tatgatggaa taaattacaa agtcactgga 316200
   caaattagag ggtaaattct taaaaatgtg aattgcaagg tgtagatttc aagtagcaca 316260
   gaacccagat gaatttcatc cactacctgc attataatca ggaacagaaa aatggagttt 316320
   actttaacac tatgttactt gagtgcaaaa tcccagctcc acaaaactct gatgtcctga 316380
   aatttgttca catgccttag ttgagctaat tactagaatg agtttgggca agttactttc 316440
   ccctcagcag gcttcacttt tcctcactgt aaaatgaaag tgtgggtctg tgaatctctg 316500
   agatacttcc gattttgtgc attagtgatt ccaagtgaga tttccttcag ctagcatgcc 316560
   attaaatgct acatttttat ttgctttctg atttccctct tttcatttat gaaaagagct 316620
   cacaaaacct ttttcctgtc tatttgctct tggaaaatta tgggctcttg gaagttttca 316680
   atttaaatta tatttacttt tcattctcac tccgtcagag gcatgtgcat atgaaaatgt 316740
   ttgtcttcca tccataccct actgactcat taggaacaat cagagtgagc tgttcttttt 316800
   cccctgctaa taataacaaa ggaaccaaac atatatagat gagattgtat cttcaaagga 316860
   aatttacgtc ggatttgata tttattttca caattatatt ctgacgtaga aagcgtcaac 316920
   accaatttcc caaatgagaa actctattat agaattgaaa tgggagctac tctgcaaaac 316980
   ccctaggtgc catgggtaca atcaaccatg gttatgtgcc ttccccacag ccatttaccc 317040
   ttaccctgtc aaaagaaaaa ctgttgtttt cctatgtact caaaatactt ctgacaccaa 317100
   acatgtgggt tttctaacca agcaattttc catttctctg tggacaccaa ccgagtgttc 317160
   tacaatttaa ctaaattctg aaactaactc cctggagtca gtgtgggcct acagtttaag 317220
   tgctcattcc cacaaggctg cccaccactt cagataccaa ttgcaaggcc cagattgtta 317280
   cctgtaattc tcataaactg gctataaata tgaggctcct ctaacccctt attgtttgat 317340
   agttggctat aaacactcac aaaacccaag aaatggctta tttaggttta ctggcttact 317400
   ataaaggata cagacaaaca atcaaacgaa agagatgcac agatcaagat gtagggtgca 317460
   gcacagagct tttatccctc tcccagtgtg cactctctca gcactgacgt gttcatcaac 317520
   ctaggagccc tctgaactcc attgtttggg ttttcttttg aggtgtcatg acataggcaa 317580
   gattggttaa atcactggcc ttgtgattga gctcaatctc cagccgctct tcccttcctg 317640
   agggaggtta gaggtggggc tgaaaatttc aatcctctga ttacatggtt ggttcctctg 317700
   gcaaccacgc ctcattttcc aagagccatc tcaatctcag gtatgtttga aaaggactta 317760
   ttatgaaaaa caatagacgc ttccctcaca ctgatcactc aggaaattcc aagagtttta 317820
   ggaacttgtg tcaggtacca gggacaaaga ccaaatatgt atttcttatt atgtcatacc 317880
   ttgcccgcat acaccctcac acacaaacac acaattcaca catgtacaga atgaaaaggc 317940
   aaagagtaac atctttatat ctgtgtaagt caattagttt aggatgattg caaaagttaa 318000
   aacattccat agaacaggaa catagtgaaa gctcaacaaa tataataatt gagcctgctt 318060
   tctcacctat aaattatgat agacactacc ttgaaatggt tgtaagaact atacatgtaa 318120
   gattatttaa tacttgatgc ccagtggcgt ctcaggggat gatagcctct ctttctcttt 318180
   ctctttctct ttctctccct tcccttccct tcccttccct tcccttccct tcccttccct 318240
   tcccttcccc tcccctcccc tcccctcccc tcccctcccc tcccctcccc tcccctcccc 318300
   tcccctcccc tcccctcccc ttcctttcct ttcctttcct tctttcagca attccaaact 318360
   tgcagactgt taggaacttt ggataatttc tccatctcga aagtaagctt gtgatattcc 318420
   ataaatcact tagcttagac ttaattgtgc tgtaaacaaa caaacaaaca tatctgaatg 318480
   gcttccaaca gcaaatgttt acctcttgcc ctctgttcat gtcttctgta agtttagctg 318540
   aggtttggta tactctgtat ccaagactcc tgtgaagaaa agtccccatt tgggacatac 318600
   tcgtctcata aaacagggga aaaacagata atggcaccat gcagctgttt ttgacatatt 318660
   tgcttagagg tgggtagcat catttctgct tcactgtatt ggccaaagca aatcagatgg 318720
   ccccgcctgc tgccaaaagg ctggaagaag taagattgtc acacaagagg agatagccaa 318780
   catttggaaa tagtaccaca aaccatcata tttctaaaca tattttaatt gctctcatac 318840
   acatgaatga ttgcaaactc gggttcctca tttgctcatt tgtttatgca tcactcacca 318900
   gacagtcatt gaagaatcgc tgtgttctag atactgtgtt ttctgatgta ggtgatacag 318960
   agagaaagaa aacgcaatct acgtgtagct agaaattttt tgtcattatt tcttttttca 319020
   gctcaaatga attctgatag aaaatttttg gtagagagca aaaggaaaaa atactgtatt 319080
   ggggaatgtg tgtgtgaatg tgtgtgtatg tgcatgaatg tgtgtgtgaa tgtgcgtgtg 319140
   aatgtgtgcg aatgtgtgtg tgcatgaatg tgtgtgcgtg tgtgtatgcg tgaatgtgtg 319200
   tgcgcatgtg tgtgaatgtg tgcgtgtgtg atgaatgtgt gtgactgtgt gtgactgtgt 319260
   gtgaatatgt gtgtgaatgt gtgtgactgt gtgtgaatgt gtgaatgtgt gtgaactgtg 319320
   tgtgaatatg tgtgtgtgaa tgtgtgtgaa tgcgtgtgtg agtgtgtgag tgtgtatgtg 319380
   tttgcgtgaa tgtgtgtgta aatgtgtgtg tgcatgtgtg tgtgaccgtg tgtgaatgtg 319440
   tgtgtgaata tgtgtgtgaa tgtgtgaatg tgtgtgtatg agtgtgtgtg agtgtgtgtg 319500
   aatgtgtgtg tgtgagtgtg tgtgcttgga agatgagcat gatgtcaaac aaacatacac 319560
   ttccaaaagt agtgggcatt cctgcttagc cttttgaaag cgatatgggg aagagagaaa 319620
   agaacatgag ccatgcctca ggtactccat gtccagggaa tgcaaacttc ataaaggcag 319680
   aggtcacatc tgcttctgct cacttctgtg tctaaactca ataactgctc tttggaaaag 319740
   aggaaaccaa gtgacccaac acatggatac acagagtcag gggacagcag ttcccacaga 319800
   agaccaccgc agcaaagctg ctcccaagta gagatagtga tgcctgctca cggtcacagg 319860
   ctgtcagagg ttatgtgccc agcaagaaca aaaggaccat tggaaactgg gtagcagttg 319920
   gggccgttct gagaaggcaa cagttcctct cttctgtaag gacaactaga gggccagagg 319980
   gaacttcgtg ttgagatggg gctgtacggc acccagggtt tagtgatgga gggctaggag 320040
   gctttgatcc aggagcagaa gaggcaggac atgctcatgg agctcatgag gaacaaatca 320100
   catttgagag agcagtcaat ggtaattgca agtctgaggc cgagcatggt ggcttacgcc 320160
   tgtaatccca gcattctggg aggctgaggc gggtggatca cccggggtca ggagttggag 320220
   acaagcctgg ccaacgtggt gaaaccctgt ctctactaaa aatacaaaaa ttagccaggc 320280
   gtgatggcgt gtgcctgtaa tcccagctac tccagaggct gagacaggag aattgcttga 320340
   actcgggagg tgaaggttgc agtgagccga gatcactcca atgcactcca gtctgggcaa 320400
   caagagtgaa actccatctc aaaaaaaaaa aaaaaaaaaa aaaagaaaaa agaaaaagaa 320460
   agtctgagca ggttttctgg gacttagcag tgttcctatg caggctatac atttacattt 320520
   aatgttaatt cttttgtgga tgtgttttcc ccattaattt atcttcttgg tatgactttc 320580
   cccagagtgc atgtaagaaa taatgtgctg ggcaaagcaa tggcttggta gaagaaaccc 320640
   agcacccctg catggactgc atacttcaaa cataacttca aacctcctac taggtttgaa 320700
   gatccttaaa gaaagggata tctgagtatc ctgcatatat taggtgactg gtgaatgctt 320760
   ggggaatgga tatgtgagta agtgacggtg tcattgtcat gagaaagaga aatattcctt 320820
   gcattttctt attctatcaa tcaagtctat aagatgtcta tttactctta tactttagca 320880
   cagtgctggt gagatgaata aacaggcatt aaagagcctt ctagagcata ttaaaacaaa 320940
   ttacttgctt tgcaagctca atttaatttt atttccttaa aaatccaaca aaagtgcatt 321000
   gaatcacctc taagcattaa gtaatgtgca aggttctagg gtttcactga tgatgaatat 321060
   gtgattccta ctttcaaaga ccactttcaa ctgaagaaac aggttatcta attttaaaaa 321120
   ggtagcataa tttgtactct aacaattgta tttgtataga gccaagacag cattgttgtt 321180
   ctctctgcaa agttctcagg gacgtgttgt tgacccagtt gttagggtgg ttaaagctgt 321240
   gaatgaaccc tattttaagg cgttagcaag aattcagtct ctttcatcat gagaagcaat 321300
   gatcaaggaa cagatacatt tataaagttg aagaaaatac cgaaaaatgt attgaatcta 321360
   tgttttccca ggaagattgt cttttcttta atttatccat aaaaaatatc tgattttaca 321420
   attttactct gtgcctttaa gagatagaaa ggaatgaatt ctattacatt gcattgtttg 321480
   gtgccaagcc ataaatttac atagttgcaa cataaacagt ttcaaattat aactctacaa 321540
   tatccaaacg aaacactatc aattcttttg ttattgactt gttttatagt tgtaatgcag 321600
   actccatata tttgttattc agttttttat tatattattt ctcaggcaat tacattttta 321660
   ctgcaaattt gggggattta cttgatataa tcattgagta caagcatagt gctaagtatt 321720
   taaatgagaa tgttatcaaa tgaacttaat ttttcttgtc ctgttgcaac cagattattt 321780
   ttactatgta agatttatta aaacatattt tcaattatgc tgggaaattt aaatgaggca 321840
   agcattccac acataatatt gcctcccaat aaggttgaca tttacaagat ggtgagtaac 321900
   attttgtgtg cttatttctt aactactttt gattttgtca atacaagata aattagccaa 321960
   aatcaggcaa ctatgtttag gcatttgtat taatcaggtg ctccatcttg tctgcaatat 322020
   tgataggaat gtttagcaac tgtgccaagt agactgagtg gtaaaaatga atacagattt 322080
   tgctataaat tgcaaataag tacaaaatta atctgattga cttaatcagg aatacagaaa 322140
   aacattagca tttcaagtac ttctattttg atttgcagga ttcattggcc atgtaaaaca 322200
   gcaattctat aaatcctcag aagaaatagc agaactgctt ttacaaatta ataatgttta 322260
   tgttgtgtaa agtcatttta gacagtgcca gtttggtgtt attgtttagg aaaaaactga 322320
   gccttgtaat gaaaaataat tgttcctctg tcataggagg ggtttaagac atattccttg 322380
   attgaatgtt tgatataggc tcaagcactg gtgggaagtt aaagcgttct tggtatgacc 322440
   tcttctgctt gtccctgttg accaagttct ttaacttgga cacaactcac caccccacaa 322500
   accctctcct ggtattttgt gttggggttc ctacagtaaa tatcagttac tggagaaaag 322560
   gacttttcaa agaagcatgc ctagtactaa cactcaacat tcacattcca tattttcttt 322620
   taaccaactg tctaaataag tatatattgc acgaatattc agttttaatc tatcgtaatg 322680
   ttctcaatta ttctttgaat tagctagttg ctattattga gctgatttag aattaatcag 322740
   tttttctgaa cacatttgta agaagaaaca atttaatctt ttccccattt ttacacctca 322800
   aaattatctt aagaattcat ttacttttga gacagaacca tttctagttt cagtaccact 322860
   tttgaaagag tacacatata ggtagatcaa ctttgaaaaa ataacacaat aggtagtgta 322920
   tataggatat attgtataga aataggagga aactttcctg taataggaaa ggaaacttcc 322980
   gtcttctgtt tgttaagaaa tatttgagag atctcaccat cctgccttct tgctttggcc 323040
   aaattgaata aacctttctc tatctctcag cacctatgtg tcatgtttgg cattagctac 323100
   acatcccata caggagcctg aatttgggct tctacaacat gagggtggcc tctagaaaca 323160
   accttaagag ccccttgcag taacccaaac aggagataat ggtaaaagaa cttttccctt 323220
   tctaggatgc tttgtagaat aaaacattta gatgtgttgg tgacaggctg ggaggctggg 323280
   agaaagggta agcaagacca cttcaaagga tttattgcct ctgaaaacca ggaatgttaa 323340
   agctgttatc tactgtgaga gggactaggt tgtaatgagg gtaggatcag gagttgggtt 323400
   ttggacttgc aacatttgga atgactactg ccatcccagt ggcattcagt gagctccacc 323460
   catgaacacc aagcaaaagg aggcaaacag gagacagtgt cagaaacaga ggatgcaccg 323520
   gcagaggtga aagcaaacag cctgaaactg aaacataact ggaagagtca agctgccgct 323580
   tctcatgatt aattttagtt ctgtgtgggc taaggctaat tagtaatgta agaattacat 323640
   gagcaggact gaaaagtatt tccccagccg tggcatctgg ctccttaaag ggttgtattg 323700
   gcagctctat ggtgctctcc aaatctggca tatggctgaa attgggtttt ggcaggtgtg 323760
   acttgtgccc ttgtaccttt tcattaatgc atcctgtgtt tattggaata aaaatggtat 323820
   tgaacataat ccatttctaa gggaaagcaa cctcaaaatg atatatgttt gcgaacataa 323880
   ataacattta ataattaaat gtcatttcta actattaaaa gtgcttgcat gtggattatt 323940
   aaccagttga caatcattca tgggaaggtc actatgcata tctcgttatt gtaaaggagt 324000
   gataaaaaaa aaggttgatc attattgggt attggatctg cacactgatt agataggagg 324060
   ggtcccaagg gagaggcgaa gttggccagc agtggggtga gcacggggag tgttggggca 324120
   gtggctgtgt accagtcata tagtcagatt atgacacttg agcagccctt tgtttctagt 324180
   gttttccagc taatacttac actggtttaa gcaaggtatc ttacaggatt ctctcaacaa 324240
   tctggtggtt tgtgtttctt tggcaattga aatagatgcc cagaaaagtt gtgattttcc 324300
   taagaacatc cagctggctg tgagcagtac tgaactcaca tccagatgaa ctggactaga 324360
   agagcaggct acatgttttt gaaatttcta aggattgtta atattcaaat cttctttctc 324420
   aacctggtca tataatctct gatatcacct ccttaaactc atctagtaat atttcccatc 324480
   ttcagagcct aaatgtcaaa tgtcatcagt tttatgcttt tatcttacaa taatataaaa 324540
   taaaatgact ttactcaaac cacattgcaa aagcattttt ccagcatatt aaccttttta 324600
   taattgttga tatggaagag ttttaattcc gtcataggtt ttatgcattt taaatattga 324660
   gtcttggact gtgctgatat ttttggtaaa taaacagaaa gatgtgcaac tgtgctgaca 324720
   ttacagtctg gagaagtgag aaattaactc tgtaggaagt caacatgatg tcagatcaat 324780
   gtgaccacta aatagatgag agttgccagg gcaacttttc acatgctcta agcaatgtga 324840
   tggtttgttt tcctattgat gcatatgcta ccctattttg gctaacatcc aattcaatta 324900
   cgtaagtatt tattgcaccc tttctacata tccagcatat tttataattt tctgacatca 324960
   ttcatatatc tattcaaaaa cgaaggctta cacataatga actggcttag actttatcta 325020
   ctttatctac caggaaattg attctttttg taaaaatgac gagttccatc ggccaggatc 325080
   tataccaaat tccgaagcca aggatatgtt agaaaatatt ttgacacacc agcctttact 325140
   tgatctgtag tataaagcaa gagtttctaa cttgcctact atcttttttt ctgcctctga 325200
   gataacatat acaggttgct gttatggccc ctttaaccat gagtatacca agggcaaaga 325260
   ggtagaagtg agtacaggca ctttagctaa acatttagtt gagttgagtt taacttttgg 325320
   tttagttgaa gtgcctgcac ttaatcaact aaaagttggg ctgctttttg ttattactgt 325380
   gcatggccaa tccaactcag tgtatttaac tcatagactc ataaatcagt gcattaactc 325440
   atagacaact gtgtatcttg tttgattgat tgattgattg attgatttgt ttattttgag 325500
   atggagtctc actctgtcgc ccaggcttgt gcgttcttgg ctcactgcaa cctccacctc 325560
   ccgagttcaa gtgattctcc ttccttagcc tcccaaatag ctggtactac aagtgtgcac 325620
   caccatgcct ggctagtttt tttctttttg tatttttaat aaagatgggg tttcaccatg 325680
   ttggtcaggc tggtctcaaa ctccagacct caaatgatcc acccgcctca gcctcccaaa 325740
   gtgctgggat tacaggcatc agccaccgca cccggcagta tcttgttttt tatttttttt 325800
   ctttaacttt ctaagtatga aggaagctac gattattctt tccaaatctg tcaggtcatt 325860
   ccattcctct gcaccaaacc ctgcaatgat ccccctctcc gtgcaggaaa aaaaacaccc 325920
   acagtccctt caatacatcc aatatgctgt gtgtgctgcc ctatggtctc acctcctccc 325980
   cactgctccc tttcttcctg tgctcattta ctgcccctca ggcctttcac catttaaaca 326040
   gctgtgccca ctagcctacc tgaccacttt gtaaggatgg ctcccaccac ctgagtcact 326100
   ctttcatcta atatccatgc agctccacag ctcttctttc agttttacac aaatactgta 326160
   ttttcattgt ggttgccctg actgctgggt tcaatacttc agatcccaca cctagcatgt 326220
   ccagctcccc acatcctgtt tcatcatatt agatcactta acatcttaaa atacactaca 326280
   tattttgctc acttattgtg tttactttat ttttcctccc actagaatat aagaccaaaa 326340
   ataaagatga ttgtctgttt tgcttttttg tctccctgat gtatctcaag tatctagaag 326400
   aatggcacag agtaggtgct catttttgtt gttaaatcaa agagtgaagg aatgaaagtt 326460
   aatcctgaat cttgtattct gtcctatcat tattttaaat atcttaagga tgctcttaca 326520
   aataagtact aaaaaatagc aggagaaatc tatgccatcc aagaaaatct ctgacaagct 326580
   gtcacatgtt tggacacctt tagtcagttg aagaagaagg ggagttgtgg tcttctgggt 326640
   aaagaagttg ccctgggagg tgactggaga ccatcttaca caaaataact tgccttgatg 326700
   cgagagaagc cggagcaaag gatagtcctt ttcagatcag gtcaaaagac cacctacaac 326760
   gaaatctcca gccatagaga ggggcattat gtatggcaga tgccagacgc ccactattta 326820
   atccaaagaa gaccagaact ggaccaagtg cactagaata tctgcagatg ggacatggag 326880
   acctgtactt cctagcaacc caggagaccc tggcttgacc taaactttac ctggataatt 326940
   ctattttctt ttttgttttg agatgaagtt ttgctcatgt tgcccaggat ggagtgcaat 327000
   ggagcgatct tggctcactg caacctccac ctcccgggtt caagcaattc tcctgtctca 327060
   gcctcctgag tagccgggat tataggcatg ctccaccatg cccagctact ttttgtattt 327120
   ttaatagaga cggggtttca ccatgttggc caggctggtc tcgaattcct gaccttatga 327180
   tccgcccacc tccgtctccc aaagtgctga gattacgggt gtgagccact gctcccagcc 327240
   tgccctgaga attcttacca atggtgaggc accaagcatc ggattggagg gagaagggtt 327300
   ggtataaaaa cagaagaaaa aggaagaaga tgggagggac ataaatcaca aatgggctga 327360
   gaagctctat tattattgtt attatatatt atttattatt attagcttca ttttgcaaat 327420
   aaaataaacc tcaccaccaa gctctagcag caaaacattt gcggagcacc actgtgtgct 327480
   tggccccagg cacacctgcc atctcatctc tatcgacaca gattgttcat tttgcagatg 327540
   agaaaagttg ataataggag agataaagca attttcataa gattgttctc tcattaagca 327600
   aatatttatc aaacaccttc tatgttgtct gtgggtgggg catcctgaga ggaataaggg 327660
   agatgaggtt tctgctctta cagagttcac attcagctgg gagaggcaga ccacaaaaag 327720
   cgactaatca atgatgaaga agctttgagg tcacggagaa gaaagtgcgt ggagagctgg 327780
   gatgcagtgt ctggagcctg gctctggcct ttcctctcct ctgctaggct gcagttctgg 327840
   gcaagagcag acttcactga aacccacgct ggctgtgtta gccaatgtgt tgacagcacc 327900
   ctgtgtaaag aagtcaccca acactgcatc agtgtgtaaa gtggaaacct aacaatctca 327960
   caatgttcac gaaacgctaa ggacatcaca aggaatgatg tagtaaagtt tttccactga 328020
   tgtcatcttt ggaagtagat tttgctcaat tcttattgga acatgtgcac agctgaactt 328080
   caagatcctt gggttctcag ctcaaatcta tataaatgac ttaaccaatt gcttaacttt 328140
   ctggaattca gatgctttaa ctgtaaaatg gtttggagct ctgaatggag tatttccaaa 328200
   ttatatctaa ctgtgaaaat tcaatgacat agaagattta ggatggagag gaaaaaaaat 328260
   aacctacata acgtacaaat gaataaaaat tataacaata ataatataaa tttattaagg 328320
   tgagatttaa aagaaaaaag attgctattt ttttattgct agttaaagct attcttggaa 328380
   gatttccagg tactttttaa gtgaaacgtt gtgtgcatga gatgtcctta tatctttttc 328440
   aagtatatag atgggctcta ttcaaaaagg gcctctcttc tcgtcttatg cccacctgcc 328500
   atctgagcaa ctatatctat tttttgctgg gctacgtgga atgattgttc tttttatgtt 328560
   cttgcctaag aacacacaca accacagcac tcctgacact tagaaggaga tgaattatat 328620
   ttaaccgaaa ggacacttgc ttgactagga ctgctaaatg tgttagctgc agggaaggcc 328680
   agctgccttc ttggccattt ggacatgccc aatcttctct tactgatgca cagatgtctg 328740
   aggccttgtg ggtgaatccc agtgtgcttg cagccaggag aaggtattgt gagcggtgct 328800
   gagcatgtga ggtgacatat ggttctcctt ctccatttga caactttatc agcttctttc 328860
   catagttcaa gtttgcagct ctgcagaacc aaaggctgct attcaaaata gcctcatcac 328920
   ctcttcctta ctccctcaac ctccagccct tgtccaattg cctggattgg gaaagttctg 328980
   cagtggtttg agcaatcact taataagacg gctgtcggtg gtaaaactgg aattggttgt 329040
   tgctattgtt gcggctttgg tgagtatcac aagtaagcac tggaaaatta gaagcaccca 329100
   gaggttttag ataacatata tgtaatggac aatcatgtca gggacccatt gattcctata 329160
   tagaaacacg ctgtgccgta agtctcttta cttgtatgta ttataaatat ttagaaagga 329220
   aaagtgtgtg tgggggtgtg tgtgttttat actcaaaatg aactttttga tcactacaaa 329280
   attcatttgg tatggtaaaa tttagaaata atctggaagt taaaatttga agaaatatca 329340
   ataatggccc tttaatatat aaacatatta ttactattat aaagattata tagcaatatt 329400
   aaaataataa tcatctaata ttcaatgaca atggaaaata taaaatatct atactcatga 329460
   tatctctaac agtgtgtgta tatgaacaat gtgtggaaag aaacacattg aattgttgat 329520
   atgtgatagt ggtaggactc ttgttcttat tttaaagctt aatttcatta gaatagcatc 329580
   tgcaaggcat atatcaaaat atatgtttat aaggttttaa taaataagaa tatgaaataa 329640
   ggtgcattga tctcttatta tgtgtcaagt ctttgggccg ttcatgctca cagccatgtt 329700
   gtgaggcaga cagtatgccc attttacaga tgaacagacc aaggccgatt aaccaaggtt 329760
   aaattaattg tccaactgta gctacaaagt tacgattaga aataagttct ggtgttctgt 329820
   tgcatactag gtttagtgtg gttaacagta aggtattgta tattacagaa tagctagaag 329880
   agaggctttt gaatgttctt accacaaaga aatgataaag acatgaggtg atgaatgagc 329940
   taactaccct gatatagttt ggacatttgt cctctccaaa tcttatgctg aaatgtcatt 330000
   cccaatgttg taagtgagac ttggtggaag gtagtgtatt atgagggtgg atccctcatg 330060
   aatgacttgg caccttccgc ttggtgatga atgagttctt aatcacatga aatagttcac 330120
   acaagatctg gttgtttaaa agtctaggac ctcctgtctc tctctctctc tcttgctccc 330180
   tttcacacca tgtgatactc cagcttcccc ttccccttct gccactattg aaagctttcc 330240
   gaggctccca ccagaagcca agcagatgtt gttcacagcc tatagaacca tgagtcaaat 330300
   aaacttcttt tctttaaatt acccagcttc aggtatttct ttataccaat gcaaaaatgg 330360
   cctagtacat accctgattt gatcagcata caacacatat acaaattaat gtaccaaatc 330420
   atactccaca aatatataaa attacaatgt gtcaacttaa aaagaattac atatttgatt 330480
   tacaaatttt ttattccttt taattctatc tcagtatttt ttacaatctt tgattacttt 330540
   ctcatttttc taattccatc ttttatttgt tttaaataaa tattttaacc aaaactgtta 330600
   aaagaagagc aacgtgcacc aaattaagtt cagtgctctt tcaacaacat gaaatttcta 330660
   ttatgcaaag tactgaaatg aggtacacaa aaaactcttg ttatcttaga gcgagtctat 330720
   tgtcaatgtg tctggagaga cagagaacaa aaatcctagg tctcaaaata gatgattctt 330780
   tttttggaat agtttactcc taaacaactg atatacatgt ttttctattg tatttcaaat 330840
   aggtgaagtt attttcacta atcctgtcgg tgctcacagc taagtcctac aatgacagat 330900
   catctaccag caatctagag aaaatcagtt ttgtgtatag agaaataagt tcccctggcc 330960
   caccatggcc agcatatgac aaggaaatga cagaggggac tgactatctg ggccagggga 331020
   acaaagggag gtttgcagag attctgaggg tacacgcaag agagtaggga gcatttctgc 331080
   agcgagaggt acttggccat ggctacagca gacaaaggat ggtcaggaat actttagcca 331140
   caacgttagc acagacatta gcagaatgct aaatcgtggg atccaccata accagaagaa 331200
   tgctagatca gtcccacctc agcaaggact ggcaaggacc cccaaaacaa agaaaaaatt 331260
   caaggactct gtcactccat accacaagtt cacaggagcc cttcatcctc caaacagtgt 331320
   gatgccacac tgcggacata cagatgaggg cagaattgat gagactgaaa aagtgcctaa 331380
   agggattatt tacaatattg gtttggacac agttttgaac caaattagac taagtaaatt 331440
   tccattttta tcatcaaata aaggatcatg aaggaacacc acgtcagttt tagacaaaat 331500
   gcgttctata tcggcataca cagatccccc tccatactgt gtatatgtgt ttactcatat 331560
   atagatgtgt gtacatgcat agtcacatac cttattgaca ttacaactat catcacaatg 331620
   gttttcacca tactatagca cagacaaata tttttatttt attttatttt agtttagttt 331680
   agtttattta tttatttttt ttatttttca gacaagttct cactctgttg cccaggctgg 331740
   agtgcagtag caccatctca gcttactgca gcctcaacat tccgggttca agtgatcctc 331800
   ccacctcatc cgcccaagtg gctgggacta tacgtgcata ccactacgcc cagataattt 331860
   ttttattttt tggtagagac agcgtttcac catgttgccc aagctggtct tgaactcctg 331920
   ggctcaagcg atccacctgc ctctgcctcc caaagtgctg ggattacaag tgtgggccac 331980
   tcaccaggcc aaatcttttg attacaaatc tatttgctca attcctagtt ctgtattcaa 332040
   cactgatatt gttgttgttt tagatcacac acaattcaca ttgagccagt aatgtctttg 332100
   aagcattact gaaataaaaa tttcagcata ttctcaataa ttttgcagct cctccaaatt 332160
   taatcactat atagaatctt tatttacagt tttaaaattg cactagccat ctcttccttc 332220
   accttttttt aaactgtaca aaactgagaa ggctgaaatt atatttttct cataatttta 332280
   caaacatacc tactttttct acctaaaatt atccagaaag tgctttcttc tctatgtgtt 332340
   gtgtggaaag gcttctatat gcctattttt gaagaaaata gttcactttt actccattat 332400
   atgaaatttt ttcattattt aaatcctgtg attagttttt tcactgaata gtcacttcca 332460
   tagcagtttg gttaaaaatt ctacagtaaa agtagtaagt gttggccact taccacttgt 332520
   aaagtgtatg ggcatagctg tatgtgtgtg tttatacatt tatatacaga tgtgtgtata 332580
   ttaaatatgt aatgtaatat aatacaaatg gtctctgact aatgaaggtt aaacttatga 332640
   ttttttcacg ttacaatggt gtgagattgg tgcacattta gtagaagcca tactttgagt 332700
   acccatacaa catttctatt tttcagtgca gtattcaact aattacatag ctcttcaata 332760
   ttttatttgt gtattttatg cacatattta ttttaataca aacgtacagc tttggtcaaa 332820
   tgtttaatat tcactgtaat tttatgggtg acattagcaa gatctatttg tctaaaaact 332880
   ctttcttaca aaaaaaccca ctttaaactt ctcaaatgcc cctccatccc atttttttaa 332940
   gtttctaata aacataaaac atatttttaa aagaagaaga aaggctccag aagatcagaa 333000
   caatacaact ggttttacca aattcaggca tttagaaatt gagaaaagag gcaattttta 333060
   aacttatgac atgacaacag gcataaaaca gcctgctatg gacaaagcag caagtgtagt 333120
   cagcctgcta aaattcagcc tctgtgagca cagcacttaa ttttacccct tattttttag 333180
   tagaaatagg atctcagaaa tcaccggact tctgtattgg tctcgttgga agctgcagac 333240
   cggaactgtt tctatttggc catcttggcc ccccgccgcc aattctactg taaagacaca 333300
   tgcacacgta tgtttatagc aaagacttgg aaccaattca aatgtccatc aatgacagac 333360
   tggataaaga aaatggggca catatacacc acggaatact atacagccgt aaaaaagaat 333420
   gagttcatgt cttttgcagg gacatggatg aagctggagg ccatcactct cagcaaacaa 333480
   aggaacagaa aaccaaacac tgcatgttct cactcataag tgggatttga acaatgagaa 333540
   cacatggaca cagggagggg aacaacacac accgggtcct gttggggcgt ggaggaaaag 333600
   gggagggaga ggattaggag aaatatctaa tgcatgtggg gcttaaaacc tagatgacag 333660
   gttgataggt gcagcaaacc accatggcat atgtatatct atgtaacaaa cctgcacatt 333720
   ctgcacatgt atcccagaac ttagagtaaa gtaaaaaaaa aaaaaataag aatcgcacta 333780
   tgtttcctag gctggtcttg aactcctagc cttaagtgat cctcctgcct cagcctccca 333840
   aagtgctgag attacaggct tgacccacca tgaccagccc aatatggctt ttaagaagaa 333900
   gctgagccag ccccacctgt ttcgtttggt tctggtgctg gcctgtgttt tgacaacatg 333960
   cttgtaagag ttttaggttc tgtcagcatc agcagacaca gcccatcctt ctctccaaat 334020
   gactgttccc ttaggcactt gagtcacttt tcagtaaaca tctgcatcat acttcaaatc 334080
   ctgttactaa catttgtgtc tccaaaattt attctccaaa ccccaaaccc cgcacacttt 334140
   aatgcaggcc tgaagtagat ggaaaagggg aaataaatga tgtcaccata gcttatcaca 334200
   tgacctaaat taacttttca tgattgacca gcattaatat tcacagattt aacaatgctt 334260
   tttaagaaaa aacaacagtc cagaagtgat tctttctgtg tggtagatat aggtctgttt 334320
   atttttaaat gtgtttttct ttgtactttt tcgttctgaa aactcttgca ttttcattta 334380
   atatcactca ttataaagtt tatctctgaa gtaggatgta ttttctgatc atattttcat 334440
   ttcttagaaa tatattgtat tttaatacct caattttatc agtattttta tatatacttg 334500
   tttgcttggg ggaagatatg agtgagagaa gctttgcact tctttctcta tgggtaaaaa 334560
   aataaaaagt taattttagc attagaattt attttagctt actatattca ggggaatgca 334620
   aatagcaaaa tttctattct tctcaacact ctttccaaga tgaaatgtgc tcagattgtt 334680
   caaacctaac ttaatagcta ttagaatctg tcttgtcgaa agtctcagat tctttatttt 334740
   actctttgta gcagcaggac tctttggtcc aactgcccga aaaactattg tcaagaaaat 334800
   gcccagtgac ctccaaactc ctgacctttc aattacctca gtggatggac cgagagaaag 334860
   gtgactttta ataaaacaca aataatatca ctaaaccatc aaaaattgaa tcccaagacg 334920
   gtttctcact aaattttatt ttccctgcct ccctcaaagg aactgtgtat tgaagttctc 334980
   catctgctga tgatctctta ggcttatctc ggtgtagtac atccatttag caatgcagat 335040
   aatcagcatt tgcagaatgg cactcagttt tgcagctgaa aagattagag tatacaatcc 335100
   accattcatt ctcaagggaa ggagtcatcg cattgaagca gtctaattat ggccatcagt 335160
   ccaaatggca tggtggccaa ggatcaaccc caccagcata ggaagttcca ggctccaaga 335220
   aagcccagag gttttggaat tagtgagcta agtgagtttg cctcattccc acacatcttg 335280
   ctcctcactt cctttactgt gccctgtggt tggtgacatg actgcatatc tgccacttct 335340
   caccactttg tatattcacc ctgccttgtc ttgatttctc atcttgataa ttctcccatc 335400
   ttttgctcaa gacactttta aacaactctc agagtaacat ccttctttgg ccctagtgag 335460
   catttgaaat atgttctata ttctaaagat ccagaacatg tgggcatttt cacgatccag 335520
   cctgtcacca tgtgttgttt agccacacgt gttcaaagca gatttcgagt tgagttattt 335580
   ctagctgggc acaaacaggg ttccattttc ataggcctca ctcttcccta ctgcattaca 335640
   tggtttcttc ttcacttcta ctacactact ccttctttaa attacagaag cttaaacata 335700
   aacaaaagta gaacaaagtg gcataatgaa acaccacaaa tccattactt tgcttcaaaa 335760
   tttcttagta caaagctatt attttttata cctacatgcc tgtgctgtaa aatagatgga 335820
   ctactttgag ccaaatcaaa gacatcttat aacttcattg gtaaatagct tgtatttata 335880
   agtaggtaag agacgttcac tttaaaaagc acaaatcaaa tatcaattaa acattcaaat 335940
   ctcaagaatt ctcacaatcc ccaccccacc caccctcctc tatcttctcc atccccatta 336000
   cactatcact cttcatttga ctggttcaaa tcagaagtca aatgaagttg acacgttgtg 336060
   tttggttgat atgtcaacaa agtcacttat aatttatcat ttcttatctt gtaatttctt 336120
   tttgaaaatg ttgagtttat cacatttgcc cttttcccag caaaaggcat cccagtgtct 336180
   cctcactatg gagtggaagg agcaagaggt ttataatagc atcttgttat attgaagtca 336240
   caagctacca gcatcttctt atattgaagt cacaggcagg atttaaattc atcttatatg 336300
   taagtatggt atcccagtgt gtcagtgaag agacagatgt tactgagatc accttgccca 336360
   gggacgcatc tcctaatatc tgcaaggatg tcctgtatac caaaagtata gcttcattcc 336420
   ttctaatccc aaagactctc aaataaagca aatctgcggg aggacaaaga tgatggtaag 336480
   cacagaggag aatgggtatc aatcacttac atcattagga aggagtgaaa tcttacaatg 336540
   atctttccct ctgctttctt cctttcaggt gatctaactt cctgtgccag catcatcatt 336600
   ttacaataag atttgacttt ggttaaagtt tgaaattctc ctctgaggaa acacatttca 336660
   ggatgttatc tttcatgcat accttatcat gaagaaagga tccaactatt cataatctag 336720
   atttccactg atttctttaa ccacagtttg ataacaacca ccagtgaggt agtcaggtcc 336780
   aggtcttatg gaactttatt ctgttcattg ggaatgtggg ttttattgat gagggagtga 336840
   taaactattg gagaattctc aattgtggcg tcatgtcttc tgagttatac ttttacagga 336900
   atgattctgg ttgctctgaa ggaacagata gatgtgggga gacaagaata aaagcaagga 336960
   ggcttgtaac tggctgtttt catactagag gtgagtgaga gagtttgaga gtttgatctg 337020
   ggctttagag gccatagtct tactttatca cttttacaag gaaagaaaga aaggcccatt 337080
   gtagtaaagt ggccccttta agaggccata gtaaaaggat ctcagtcctc aaatatataa 337140
   aatagtatat attttttaaa aattgtaaaa acctttgcca ttcattttct gacttaaaat 337200
   gcctgatgag gttgtcttag ttctcagatt tcaaatcttc tgatctatgc atggcacata 337260
   agttcctcac tgaacaattg atgattaatt ataaatgcca ataatggtta ttgtttgaga 337320
   ggagatgact gtggatatgg agaagtaaac agaacactct cctttctctt tgacttcatt 337380
   taaccaattt tttctttcaa atcattttct agtaagaaac cacctacaaa tgcccagagg 337440
   attgctgcct tatttaaagt attataaaac ttattgcata tgcataacgt gactgtgacg 337500
   tggaaacttc tcagccttgc ccagtccttt tgaaatgtta agctcactgt aaaaatgata 337560
   aaatattgtt attttctctt aagaaataat aaaattaaaa cccaagaagg aaaagactct 337620
   gctaataaat aacagtgtca aatttaaaga agatatttgc ataaatatta ggtttaacca 337680
   aatgaaattg ccaatattca aaagttttta accttcaaag catcggtttc atatggttcc 337740
   gtctattaca aactcttgct ccttccaatc cactgtggca accttcagaa agatggggga 337800
   tattaaatgt ccttttgttc tcacagagta tcctttatgt tgtgaacatg gacttagtta 337860
   tcactgccac tctgtgggct tttatgatct attataacct ttactatagt gcaaatatct 337920
   gtatatataa acacatatat atatagccta ttagaccctg aatatcttaa aggcagagaa 337980
   agtctcttat tttcctttta taactagcaa ctggcatagt gacagacaag aatggatgct 338040
   ccatatgtct ttgtttagaa tgatataaat gcaggggttg atgaatcata ttcttttaat 338100
   aatcttataa gaagcagtaa tgagaagcca ttttgacaac aggcttttaa atcagacacc 338160
   ctgggtttag atgtgaatcc gatatttact tcctatggaa gttgggacaa gttgctcaaa 338220
   cttgaagtgc ctcagcttcc tcatatatgt aacagacaat acttacctca cagagatgta 338280
   gtgataattc aataagttat tgcttagaat agtgagtggc atgtaataat tgccatatac 338340
   atgttatatg tgtttgattg ttatttttaa taattgtttg ttgaattgtg attcctgtgt 338400
   gtgtgtgtgt gtgtgtgtgt gtgtgtgtgt gtcttgtgga ggtagtgatg gcagtgggag 338460
   gctgttctca ctagtgttgc tttcagaatt cgttagcact aatttgccag ccaattacct 338520
   gggaagtatg gcatctggtt gaataaagga gtcataattt ttataacttt tgtttttgtg 338580
   accctttgca attttttgaa catccacagt gagtctcagc aaataaagca tctggaaatg 338640
   aatattagtt gcaaactaat tgaagcaata acactaaaaa agattaaaaa aggataattg 338700
   tcatgtttga gatcccacac aatagtgcca atatcctgtg ctgaatcttt tctgaggcct 338760
   gaaaacaagg taaggggtca ttgcaggcat catgtgattc tttttcacag gaacttcagg 338820
   actgaatcct gcagtagaag caagaacagg ctattgggag actctggtcc tcaactgcac 338880
   aaagaagggc ccatatcctc aactctgcca cagacctctt tgattccagt tggaagcctg 338940
   gagatttcac tgctgctgag gagggttcca gggactggga aacctgagct gtgagctgag 339000
   cttgggtgat ctgtttccat ctgtggtctg gtgctgatgg tttccaaggc aggaatcaag 339060
   tcagtccttg aagagctaca acagagcaaa agtgcaggta tgagagcata gatggacaaa 339120
   aaaaagcaag taatcataat cagagaagag ctggagcagc taggaaattt agaagaagag 339180
   aacaggttac aatagatgcc gtgaacacag cgatggttct aagactatgc ccatcttgtt 339240
   tacttctgtg gcttcagtgc ctgacaaata catagtcagc actcatcaaa tattaaataa 339300
   atacatgcat gaattgtcaa aaggaggtga cactatccaa ccacttaaag tataaatttt 339360
   tccagataga tacaatgcta cagatacatt atcaaatagg atggaggaac tagaagatta 339420
   agagaagcat tagattgctc tactctggaa gtaatctcca gcaagataag gaatcaattc 339480
   tctttgccca tttaattacc atatttcctg aagatttaga aatattttat ctggctccct 339540
   ggtcctaata tttgaggaat ctatgatttg ataaatggaa caaatgcctt acagagatat 339600
   gatatagcca gggtgtcttg ggcagaaata cgtaattggt aaagtcaaga tggtggaagg 339660
   taagctctgt ttctgaagac aaatcacatc cctgtacttc agacttgatg ggtgtctatg 339720
   agcaagaatg ttttctctat ctgagcccca gggtaaaaga caagacatgg agcactctaa 339780
   tgtagccaat cgactcagaa tatctcattt taaatatatt ttttcatatt agatgtgcga 339840
   gtgggaaagt aattatgagg tcacacatcc tttccttagg taaaccaaca tttttttaca 339900
   tttaggaaga aactgaataa aactgtcttt tgtccaagtt aatatacctt gtgaacccca 339960
   gctttttgtt aagaaaacaa agtgctgtga cttattttaa acaacagaag ggaaacatga 340020
   cttctgattc ataacttccc ttaggcttga gggtctctga aaggagagct ggaaatgctg 340080
   agagatgtga agatgctttg ggagggcaca gtttcccctt tgccttatgg gagattataa 340140
   agtttcacag actatcacca ctttacttta gaattttcct caatacttcc ctctagaagt 340200
   ttgcagggaa gatctcttct tggttattaa tatgtttcag aaatgacagg ctaaagttct 340260
   acctacccag atggaagcaa actgattttt ggcttctctg tttgagtggc agccagtgta 340320
   accctgggtt tctggttgaa attccaatgc agagattccc aagccatcag ctggaagaca 340380
   caactttcag caacaactct gtttgtgccc atcaggaagt tggatgaaat aattttaaaa 340440
   tatggatatc tgtacatcca gatattttca gacaagttca gaaagattat tttataaggg 340500
   atgcaattag aaaaacaatt attacaaaaa ttgtactata tttaataaac ctcctcctat 340560
   gtctcctaca atgtgtgtgt tattatcaga ttgatgggaa catcactgaa taatcagatc 340620
   aagtgaattt gattggtgag aaaaaaatat gctcgcttat gaaattaaca attggtcatg 340680
   acacagagta acaaagattt tatttattat ttattctatt ataatgttta aatatataat 340740
   tttatgcaga ttatgcattt tgcataaatt ctgttatgca aaatataaat tctgttaagt 340800
   tctatagagg gcagaagttt gatttaagac aaatatgaga aaatgttctt tcatgactga 340860
   ataatatgat tctggtaaaa taagtgtctc tgagagaagc ctcagacttg tgtttttatg 340920
   ggaagagcga aattcttctt gcttgagggc aaccttttgc agagaggaaa aataatagca 340980
   agaaggaaag gtgaggacta ctttttgata gtggaaatca atctgtgatt catcacaaaa 341040
   tacaagtcat ctggagctac aaatttatgg gtggtttaaa tagcaagaag cagaaagcat 341100
   atacttgggg ataactggaa agaagatcag aggtgaccca agtttcagga attgaactaa 341160
   agagatggtt ggaataaaac tttttcagtg gaaaggcaga agaagggaag tgatttttat 341220
   tgtctacttt acaacacgct tagccaacat tatctcactt taatccacac cacagccgta 341280
   gcctttcaca gtaatatcat ccacatatca cacagggata gatggggtta tgttagataa 341340
   attgtccatg accacccagt gagtaagcaa cagagccaga actcaaacct aggttgtcat 341400
   ccaatgtcag gcatttttca agtttggaaa aggggtctat acttctaaat atatagagca 341460
   atattctgaa gagaaggtta aataatatgt tgaatggaga gaactttatc ctgtctgaac 341520
   aacctctttc gatctattta gattacatgt gaaaataaaa atgttctgtt ttttctctat 341580
   atggtaaaga ataaaagtac atatgtttat caaagtgtac aatattctaa atcaggagtt 341640
   cttgaataaa ttaattacta catgctagaa ttcttagaaa tttagattac acactttgtt 341700
   tttataaaca gatatttaaa taacatcggt ctttgcgtat ctatgcacac gcgatgtaga 341760
   ggatattttc accaaatgta agtgggatgg aggcggcagt ttgttgactt ttcctccaat 341820
   ctttattcta ttttaaagcc tttagggaat tgttccttca gaaacaacgt cagatactgt 341880
   ctcaaagaag cttttctgga aagccctgat attaaaaaca aaatgagagc cacaaacaac 341940
   aaaaagtcca ggatgtccag agaaaatggt ccatcttcag gcattcctga aagcagacca 342000
   tcaccaactt tgtcatcatt attattattt gctcaggcat ttctaaggag ataacatcat 342060
   atctcccagg aactgcctca aaggcccaat ccacacaagg aagccctatg caaaattttc 342120
   aatgaaaatg cattgcctag agtaaagatg gagcattaag agaagatgga ggcagaagta 342180
   aggatctatg agtaaatcaa tggccatgaa aactggaggc actaaaagaa gctggaacaa 342240
   aacaggttga ggtcaagatc agaggtaggg aatgcactgg tgaaatatac aagctacgta 342300
   gggttagatg gttcaaagga aggccttttc atgcagggtt gtacctaatt gtagacttaa 342360
   aattgaggct tatggaatca cagtagtggt tgaccctaaa tgattatgtt attattctta 342420
   tcccctcata tgaatttttt aaatgacttt ctgctaaaga cgcatttcta tttcaaaatt 342480
   tagtatcatt atctaactct gactctcttc ctaattcttg tccttctaaa tcatttttga 342540
   gagctaatta ggttgagtgc cattttcaac tgctactttg gaagataagg tacatgcctg 342600
   catattgtgc gcttactgct gtgcacatct gtgctcaata tcaatctgca gggattctga 342660
   aaattaacat ccctttaaag gcaaacagga ttacagggaa agctatgtat tattacttgg 342720
   tgtttggttt tacggataca gcctggattt caattgaaaa tagtaagaaa attagaaaat 342780
   attttctctt ttctaaaaat gctcccctcc tccacacaca catagaaaaa tataattaga 342840
   aaaaatcata tcttaaaaag caaataagta ctttaaaaat atattcatag ctttgaaaga 342900
   tgcactatgt caatataaaa ttaaatccat ttttattgtc aagaaaatgg gctaatagaa 342960
   ttttctacca attttatgat aattggcata tggataaaac agaaaaaaac aaagtcttga 343020
   ttagaattac tgtatctgga ggttttaagt taggggaaaa aaagtcaact tttttttttc 343080
   ctctctctct aacagtttat cacaagcaca gtttcccccc actactttca attttatatt 343140
   ctaggtttta gacctctgaa atctgagaag atctgtgcct aggggaaaga gactgccttg 343200
   aagaaacatg tgtttgtgac tgagtcttat cagagtataa ataacacaac tgtttccaag 343260
   aagaaaagat cagataacat atagtcaatt gtagcctgca gggttgaaag aattgacagg 343320
   ttttattact tgctcaaatg aatctttaat actttttgtt ttgttttgtt ttgtttttga 343380
   gacggagtct ctctatgtcg cccaggctgg agagcagtgg tgcaatcttg gctcactgca 343440
   agctccacct cccaggtcac gccattctcc tgcctcagcc tcccgagtag ctgggactac 343500
   aggcgcccac caccacgcct ggctaatttt ttgtatattt agaagagatg gggtttcaca 343560
   gtgttaccca ggatggtctc gatctcctga cctcgtgatc cacctgcctt ggcctcccaa 343620
   agtgctggga ttacaggagt gatccaccgc acccggccaa tactttgaag atagtaagaa 343680
   aaggagcaag atcggtagtt aaaaacacca gagaatgttg ctagaatgag ctccacaagt 343740
   gtatgttgaa ttctctggtg ttatagttgg gacgacagag tagacagagg gagtgattaa 343800
   agaacagaaa cttaacacac cggagcaccc ttgtttgtcc cgtgctttac agtcttcatt 343860
   taacttatca cacacatagc aagtacagta tagacagaaa ccaagacaat atgctataag 343920
   attgagtaaa aaggaaagtg atttaaaaga ataaggaagc caaaaaggaa gcagtttgtg 343980
   gtcgcctgag agatttgtga ccataaagca ggggctggtt aatattttct gcaaatatcc 344040
   agatcgtaaa tatcttagat tgagcaacta ttcaattctg ctttgctggt aggaaagttg 344100
   cctagataat atatcaacaa atgggttttg ttccaataac actttattta ggaaaactga 344160
   aatttgaatt ttatattatt ttcatatatt acaaaatgtt tttcaagcac taaaacatat 344220
   gaaatccatt ctgtactcct ggattacaca aaggcaggca ctgggctgga tttgtccagt 344280
   gggctaccat ttgctgatcc ctgcattagg gcctgtgctt ataccttgac ttcaggtatc 344340
   tgcaaggata aagaggagga accctataac accctcaagt tctctctgac cactgtgatt 344400
   tttaaaacac actttctttg aatggctatc tttttccttt aagagataac ctccacattt 344460
   agaaacatag acactagaag gtctggcaaa tgaaagagaa ttgtggatta tttttccatt 344520
   gaatgattta aaaataaata aaacaacttt gattgacatc tggtagatta aaaaggcaag 344580
   atttatttcc tacaaaataa agaaactgac acatatgtca gacacacgtg gcatttctga 344640
   tgtggaaaga aggaaagaca tcaataagac caattaattt actttcttta gctttgtggc 344700
   aagtcaccta actcgtcaat ctgtgtgcag gcaaatatgt attacattgt tctgtgctag 344760
   gatggtatta aacattgcca ctttttaatt tactctttat tatactgaca ggtaaacaaa 344820
   gcaacacgaa ggctaaataa aagtcatttc cccaaggtaa gtttacaatg ttggttcatt 344880
   tacagtttag tggtcttgaa agtgaagagt ttattttgta ttgttcaact agcttgcttt 344940
   gctgtggata cacttgctca atattagtta taaatgtaat ggacatgtct atataagaat 345000
   gagtataggt cctataatgt ttatttaacc aggctgaata atgagaaaca tccttgtcat 345060
   ctatcacacc cttcaaataa aaagatgagc aaccctgagg gtgcgttgct ttttcttttt 345120
   ttttttttct ttgagatgga gtctcggtct gtcacccagg ctggagtgca atggcatgat 345180
   ctcggctcac tgcaacctcc aactcccggg ttcaagcgat cctcctgcct cagccttccg 345240
   agtagctggg attacaggtg cacgccacca tgcccagcta atttttgtat ttttagtaga 345300
   gacaggtttc accatgttgg tcagagtggt ctcgaactcc tgacctcgtg atccacccac 345360
   ctcagcctcc caaataaagc tcagcggtac attgctttaa caacattaaa ttgcaaggtc 345420
   tatgtctcat aagagagcag gttaggacta gtctccacag gtccagtggc aatatcctta 345480
   gtatcataaa caatacactc agaccattaa gacatactgg aaacaacatt ttggttgcca 345540
   ctatttccac tgatgtgtac cagagacata tgaagacact tttacacaag ttgcataata 345600
   aaaccaaagc aatgaataaa ctttgaacat ttgtgactaa atgataccta cattgtattt 345660
   aattatatta tgcatatata ctgcatttga ctctcactcc aaatatgtta gaaacttttc 345720
   atattaaaat ataaagctct attttcattc aatagatgat ccttagttaa ttcaatgagt 345780
   cccattcatt gatgaattgt ttgccttcat tttgtgttaa tttttttgtc atttttttct 345840
   tctcatgagt tttgttacct gatatgataa cctttcccag tgaaatattt taattttatc 345900
   tagctataca ttcttctaga tcttttttgg ttataggcat ttttagtttt caatctgtga 345960
   tttttctgta attaattttt gtaagaacta aattaaatat tatctttttt gaattatctt 346020
   aatgtctcac atcacttatt gagtagcctt tctatcctcg ctagtttaat attactgcaa 346080
   tttttacaaa ttgaatagcc atgtgcattt gtatctattt ctacctctag attctactga 346140
   actgtccaga tatgaaataa ctacaataaa caggctatta caatattaat aaactatctt 346200
   aattgtcttg ttactatttt ttctgttaca gcaaaataag ggtagggacc ttcagatgga 346260
   cagattagcc tggattaagc aggtaagccc aataaatcac atgagtcatt gactatggtg 346320
   aacttttttt ctggctggga gcaagagacg caaagatgga aaggttagag agatgctatg 346380
   tgagaagact caatattctg tctctggctt tccagatgga gaagccgagg atcaaggatc 346440
   gggccaggag ccaaggaata ctgccacctc tagaagttgg gaacggccct cagcttacag 346500
   ccactaagaa aagggggatt ttggccctct agctgcaagg aatctaattc cacttgaatt 346560
   aacaaggaac atccaggcag gaatgtagcc ctgacaacac cctaatttag gccagtcagg 346620
   cccggacaga cttctgaccc acggaattct aaaataattt gtgtttctta aaccatcaaa 346680
   tttgttgtaa tttgttatgg tagcaacagg aatcaaatat acctctctac ttttacaaga 346740
   tattgttgtc tgcagcctgt ttgaagtcgg tgactaaaca cagctggtta ctatggcagg 346800
   cccattctga agagttgcag gacaccctcg gg 346832
<210> 39
   <211> 10584
   <212> DNA
   <213> Homo sapiens
<400> 39
   cccgcagctg cgccccaccc gggccaccaa gcacggtgga gggggaacag gacactgcct 60
   tcttgcttct cttctctctg gcatctccct cttccgcccc cttcctttta cccttccctg 120
   ttttcatcta ctcgctgtga gcttctctct ccttacaaac cctttttcct gggctagaca 180
   ctctgggtca agttcagagt atcgtgtcct caaggcctct tctctgcaag aaaccccgat 240
   gattagaggg ctggagggaa aaactcatgg ggaagaaagc atggggtgtt ttcaccaaat 300
   gccaaattca tctcagtttt attgcgcgcc aactgcatgt gaggcaccag gtgcagggag 360
   acaggagaca gtatccgagc tccaccatcg cggtatagag ggtgagacga acagtaacca 420
   agaaattaat agaataggca ctaaagggct tggcgaatgg aggctccacc acactttcct 480
   ggagcgagtg agttggacaa gatgaggttt ctctgggatg gcgatgtctg agctggggcc 540
   agaagtaaga gttggggatt caggggtccc atcacctgtg tgtgctggaa ggaaatcttt 600
   gctattctta gcacggtggt gtggggccag ggcgaggcag gaggtggaat ggaaataatt 660
   aaataggaag caaaaattat cagtccacag gatcctggtg tgatctgcga gggagaggtg 720
   gatgaaccca ggagtgaggt ccttctgctt ggaggttcct gctctgcact catccaaaca 780
   cccagcagga aaccccagtt tactgcaggg agaacctgaa gttgtatact cgttttggct 840
   acctttgccc ttcagctggg aggaaaagca gagtttagaa acaagtatac tctccgtctc 900
   tgggactgtt tcattatttg taaaataggg tggctgaagt tggaaattca cgggtccttt 960
   cccgttaggc gatttgagga gaaaggagca tttggacact atggctccct cggttttccc 1020
   acgcctaacc tggaagaaag ccatcggccc agctgcttgg ggggctgccc cctaactagc 1080
   aaccttggtg acaccagatc gggtgttttc agcttctggc ttttggtgag ctggacggtg 1140
   ggagcacgtc tgtccgtggg gtggcttagc caggtcgcag gagtgccgtg agcgggtcag 1200
   gcccgtaatt tccatcttta gtcaggagct agatttctgg ctagctctat gtggccagtt 1260
   tgtcctaggc cagaagacgt ctgagaatag atgttacgac tgaagcttgc aagaaggccc 1320
   ggggaaaaga gcagcttctt ggtcctaagc ccttttcaaa gtaagatgtt ttctctttca 1380
   ctgtatgtcg gaagctttcc tcctgccata ggagtttctg cgcgattttt gtccgctgag 1440
   acacttgaga gagccgagca gcggacagcc tcttggcctc gggaagccac agaattccaa 1500
   ggacgctcac agtttgacaa gagcctgctg gagccaggtg gctccttttc cttggaacaa 1560
   gaccgtgaga caacagccct gtcccagccc aggaagctgg ggtctcctgc accgctgtca 1620
   ggccgctgct gaggagctct gcgtgccgca cacttaaggg ctctccggac ttcgacccca 1680
   cacgcagctc gagcaaaggg atcttttgtt gatacctcgg atagcatccc ttccgcgggt 1740
   tgctggaaca gcagagtctc cttttgggga gcagctctgg gaccgtgccc gttgggcact 1800
   tgcggctcct gggaaccgat cgaggggagg ggcccttcag ggactcccgg gacaaatctt 1860
   gggcgcataa cgcgaaccct gagcacaaaa aatgagccag gccctcactg ggtgagctcg 1920
   ctacagcccg cagacccgcc gccacaacct caggttcagc gccaggggac gccaggggct 1980
   tggccagtgt cttcggcctc ggcgagccag gttccacgca acctgggaca aaacagtctc 2040
   ggaggctgag cgaagcgccc cgagcctcac gcttccgcat tcaggttgtg cttggcgagg 2100
   gtagaaagta cttcccacca cctaggccac agccggacgc ggcggcccag gccgcaattg 2160
   ctgcctagag cgcagacagg gagctcgggt cgcgtgtctg ttttggagag tggcgggctg 2220
   cggccgccgc ggaggatcct gggggattcc aagcgcggca ctgggccggg gtgcagagct 2280
   ggggccgcgg tggggagggg ggcgcgctct cctcttcccc tacttccccg gaggttaatt 2340
   ccgacaagtt ttgcaggcgc tcagtggcac cggcgggggc cggaacgcgc gcgctccagc 2400
   tgcctgcaga ccccctctca agaaaaacag atcgtgtaac caccctggca ccaaccagtc 2460
   ggtttcaagt ttccgcgagt aatttttttt tgttgttttt aaacaactac tgcactatcc 2520
   aaatattggc tggcgctcag gacgctggca ggcaaatcat tgccccgggc ttagtgtaat 2580
   tatctcaggc gtatttggtt taccccttag gcaattgttg gcgacgctat gctaggaaga 2640
   ggggttccat gcaagctttt attgcgttta attggctcta tggaggggag taaaaaaaga 2700
   tgtggaactt taggagcggt tatttattca ttctttctcc tccatgcccc cagcccctgg 2760
   agggtctcac gcgctccttg ggctggatct aggccaggcc ttcccacggc ttctgcggac 2820
   agctgtgccc agggcgagga ggaacacaag ttaccgatag ggctaggacg acgtacagcg 2880
   ctagaaaaaa ggcaaaggtt taggcgcaca tctcaatttc catgggttct ccctttacgc 2940
   actgacttag ctgacgtgcg gaaggttgcc tgggttagtt acgtttgggg tcctcccagg 3000
   agccttcctg gtcacgctag cctggcactt gcctgacctt cggctgactt ctcggcgcat 3060
   acttttccca gacgaaactc ctaaagtgga tttgaagagg gtaggagaaa gccagcccca 3120
   ggactcggaa acgtggcttc cagctcgagt ccaggcctgg atgaccctgg aggcatcatt 3180
   tcaccgcttt gggcttcttt ttctttccag gaaatactca gtagtcagaa ctgcgccttc 3240
   gaaggctcca aggagttgag gtttgaagct gtgatctcga cggtggagtg gcgctggggg 3300
   tctgggcgct cggagcgggg agctcaggag ctgcgcagct ggggacgctg cagggcagca 3360
   gcgccacccg tcaacccctt ataacgtttt aggaatccgt cgcctccagc gtcaccaacc 3420
   cgacctgaca tctctgcgaa gtgcatccct gccccgtttc cgacccgtcc agtccccgcg 3480
   ccttgcccgg caggcgccca ggacacaggt ttggctccgc cctacttgga tttgagtgca 3540
   taagctcgat ttgacttttc ggaatgcccg agggtggagg gcagaaggga aaagcctgag 3600
   gtcaggggca tttatttctt ggagaaaaaa agcactggcc ttgaatttag gagacgatac 3660
   tcgagctcca gcttcgccac ctactggctg tgcgactgcc tcttctctga gcctcagttt 3720
   ccttttctct aaagtggcga tgaccctgtt gccttaaaag tgcgattctg agacgtggga 3780
   ttttgagagt gactgctgga atactttgat ccagtaaacc tctatcaaga gcctactgtg 3840
   tgccaagcgt cagaaaacgc cttgtaaagt gtgaagcatt gtaaatacat aagatgctat 3900
   tatcgttttt gctgttagaa gtcatcatct tttccttcct ccaaatctaa ttgtacctaa 3960
   gaagagctcg acaaaatgaa gcccgacggc ggtacatgag cgggtggatg tatctggcgt 4020
   tggagtggtc agccgcgggg tgcacacggc tttctctcgc agtgccaccg gccaagaaag 4080
   acagcccagc tttcgtcccc caaagtttct agcacctccg ctcgcgggcg agactggctg 4140
   ggttgtctga gccgtaggtg tgcgagtttg agggaaggag gatacaaggg ggtccagcca 4200
   gcagcggaga gccaggggat agcgccgggc cgcgcaggct cagggttgcc gtgccagcgt 4260
   cgctccacct cgacattgac cccaggctct cgtctgaccg aaactcactc cgcaaagacc 4320
   ctacgtgctg cccagagtca gaaacctgac ttttaaatct ccactcttcg aggcgagatt 4380
   cgtctcgaac accgcggctg tgtacactgc ctgtggcccg gggcgcgcaa tcagcgtgga 4440
   gatgggaagc gtttgtgtca ccctagcaga gcaatttccc ctttacagtc cacccgcgcc 4500
   tcacacctct tggccaacat ccagaagctt tcctacgggc cgtgtctgtt taggtagcgg 4560
   ccactgacaa ccataaccac caaataaata accaacgatt aaaaattaaa taactttctt 4620
   gcgctccctc cgacgactaa taacaaagac tccgcacatc agcaaccagg tgtcgcggtt 4680
   aaactcctga cagtaactgt cttccttttc agccagccag ttctggaata cattacatta 4740
   attaagtctt tggtgattta gcctccttgg agagaagtcc aacaatgtaa ttaagagcat 4800
   actggctata ttagatatga atattcaaaa cagtgtcaat taattagcca acagatctag 4860
   ctcactatgc ttcgaaagcc cctgaagtcc tctaagatac atttaaaggg tcatcggcgg 4920
   ggaaaggaga ggggggtaaa gaatattaaa gaggtgggtc cagatgatct cttcgttcca 4980
   cataaacatt ttctaatgat ggcgatttat aacgtccctg acatcacata ccgcaccttt 5040
   gggcacgtgg gtgcccgccc atgtctgtgt gtgtctggtg gtggtggtgg agttgggggg 5100
   gcggggcgga tgttgttgat ccatcagagc aaagatcttt tataattaaa gtgaggaggc 5160
   atccaactgg aagggccttc ttagaggcac caagaggcta tccattgtac atcctctcag 5220
   acatgttaga aagataaata ttaaatctgc tatcacaaag ggagctgtta aatagatact 5280
   aagctgatat gcataaaaaa ttaattaggt agttttctca gcatcaaact cctggacaaa 5340
   taactacttg taaagtacac cttctgggca tattgaacat atgctggaat tatccttaat 5400
   tgactaatta cataaattac tcattaattt tacagcacat caattataaa agatatatca 5460
   attaattttg cataaattaa gactggaatc tccctccccc caaaaaacac aagagagagg 5520
   ctgctgtaac aaaatatgca gagaggggag aaaaacaatg tttgtgtatt tgtagattgc 5580
   aataataatt tgttctgctc tgggcagaga ttttcttaag gaaaaacatc cttggcaatt 5640
   aaaatagtcc taaagaccac ttacgatgtg atcatgcgtg caccaaggcg aaacagacca 5700
   tttaaatcaa ttaggactgc tctgatggaa aatgagggcc tggcaatgac aaagtactca 5760
   gacattagta atcacaaatg atttaacatc cacattaaat gcctgactgg gtagtaaggc 5820
   ttattttcct aagtcagcag ggtgcccgta acgaagttat ctctgggtat cccaaagtag 5880
   acttcggcaa cgatttcaca cacaattggt ggaaattaat ttgacacctc tcttcaacct 5940
   aactcctcca gcctgctcgt ttcgggctct cctaagcggc tgttggcctc ctcacctcct 6000
   ccctcgtttc cttaaaaagg tcattaaatg cttcgtcacg agcccagccg tggggccctg 6060
   ctccttctgt tttgggtttc tcctcccctg gtgggactgg cggctttcag aatacagact 6120
   gtgtctcggc ctaggctagc gggtcgagaa ggtgatttcc aaggggtgag caaagtctga 6180
   gacccccggc tccttgactc ttcctggccc gccctccgca gtctgccagg ctgaggctga 6240
   aaacacgagt ttccagaaag gctttaggca ggctgcgggc gccgggaggg aagccgggag 6300
   accgctgcgc ccgctgcgcc cgctgcactt tggcaacgag gcctgcgtga ttttagtcag 6360
   cagggggcgc tgtctcccct ggtcgccgcc ttagagagga agccgccttc ggtgcgggtt 6420
   ttctccgcac agcgatggag gtggagagat gtgagtttcc tggggtagcg gcacttctag 6480
   aaatgccagg ctatggttca ctggtttccc ttctgtgtga aaaccccaat ctaccttgat 6540
   ttgtgccaac catctgtaat gcttttcata gataacttta gacaacatca aatcaaacgg 6600
   tacttgaaat acatcccaaa tttcctcttg gaagttgtga acttcgaagt tgcacctaca 6660
   aatatagagg tgcttttcca aaagaaacaa aaacttcctt gaaaggaatc cgtagtcatt 6720
   atttaatgcc agccccacgt gtgtattgat aattattgac aatagaaata tagaattttc 6780
   tatgttgtta agaagccttt caacgatata agtgggagat tcaagttata tagaatcata 6840
   tggttcaaaa attttgtttt tatattcttt taagtatttt gtattcatag tgatcattca 6900
   taactctgaa gtaaaataaa tcagaatgac agacaactgg atgttggaga aacagaaagg 6960
   ttttactttc aagagatgcc tagtttggtg gatagtaagt ttatgatcag cagattccca 7020
   atcccacact cataaattgg ttgtgttaaa aatactttgt tttaaaacca ctatgtattt 7080
   cactcaaagt gaaatttccc ctaggaataa tactgtaact ggtagatagg atcccagcta 7140
   gttggtactg ttccgcttta agtcctgtta aaccagtaat gtatctaaaa atttgaattt 7200
   ctcctttgag actgatcaca accctaaaag gtccttttct tatccccttc tgttgctagt 7260
   ctccaaagat actggctccc acccccatat tggaccatgc cttctggaat tcatgccctt 7320
   gtgcagtttc ttctcatatt aaatctgacc cagtcccatg ggaacaagta gcatgtggca 7380
   gaagtggtgc agtatgattt caagccaaat cagaaggacc cttgcagctt ttgccttgcc 7440
   cttttaaaac acacactctg gggtggtggt ggggattggg gggtgggggg gcagtgccag 7500
   ccaacaggta agaagtccta cggctcacac tgtgaggaaa tccagtttag ccaaggagag 7560
   agagagagag agcttggcgg gggagagaga gagagagcgc gcactcttgg agagagagag 7620
   atgcctgatc agccaccagc tctgccaacc atcccaagtg agatgtcata tgtggtgagt 7680
   aaaggaactg tcctggatgt ccagtccagt taacttctag ataatttcag cctcagttct 7740
   acctgactgc aactgcatga gagaccttag tcaataacct ccctgctgag ctcagtcaat 7800
   ctacagaaac gcatgggcta acaaaccatt gctgttcagg cattatgttt aggtgtgatt 7860
   tattacacag cagtagataa ccagaacata cattgtcctg tctcccattc tttttcctat 7920
   actatttttt cttgaatctt atttctgcta cagtttgaat ctggctactc atgtgagcta 7980
   gcctgggaat ctgaacacta gttagaatat cgaggctggg attgtgtgcc aaagagattg 8040
   gcaaggagag tagatgaagg agactgcaac ataattagac atttctattt acgcttagga 8100
   aacagttacg agaaactaaa tactgctctt cctcggagct tctcaggggc tggcactgga 8160
   ggatacaact gtcagtaagt tcctaagtca cgtgctcaaa gagaagtgcc tctattagaa 8220
   tttctatctc aatcatgtgc attgttttaa ttaaaggttt ttttctaata ttctcaaaca 8280
   acagatatat aattgtattt atagtatgga agacaattaa attagaatct ttttcaagat 8340
   aaaaatagtg tctttgaaat tgctcacatt tctagagaat aatggtgcta tgatcagtag 8400
   tttctgttcc attagcttct cctttccaac taggcaaatc aataaacttt atagtgtttg 8460
   tttttcacct tttattgcca ccatttattc aagtgcagac ttgccattgt aacttgtact 8520
   tctggatagg cagtcatata tttgctttgt tatattgata agattttttt tttttttttt 8580
   ttgagatgga gtctcgcttt gtcatccagg ctggagtgca gtggtgtgat cttggctcac 8640
   tgcaacctcc acctccctgg ttcaagcgat tctcctgcct cagcctcctg agtagctggg 8700
   attatagaca cacaccacca aacccagcta atttttgtgt ttttagtaga gacagagttt 8760
   caccgtgttg accaggctgg tcttgaactc ctgacctagt gatctgcctg ccttgacctc 8820
   ccaaagtgct gggattatag gcgtgagcca ctgtgcctgg ctaagttttt tttttttttt 8880
   tttttttttt tttagcgcaa cttgtgctgg tccttttgtg aagcaagaat attcaagagt 8940
   ttatagatat aaaaatatcc tcagctctta ctaaagaaaa atcatcttcc tattgttcat 9000
   tgtagtctca tctttcagat ggaaaaacag ataaaatccc agacattccc aaagaatggg 9060
   cttcataagg ttcaagtaga tagctggtat ggattttaat tgtcttctaa atcatccttt 9120
   atgcagggaa agcctaacat gtttgagtga ttactttttg tttggcatcc aatagtgcca 9180
   aaatggactt tggcatcaga ctgatttggt taattttagg tgcttttttt tttttttttt 9240
   ttttgagaca gaagcttgtt cttgttgctc aggctaggtg caatggcatg atctcgactc 9300
   actgcaacct ccacctcctg ggttcaagag agtctcctgc ctcagcctcc cgagtagctg 9360
   agattacagg cacccaccac cacccctggc taatttttgt atttttacta gagtcggggt 9420
   ttcaccatct tggccaggct tcagtcttga actcctgacc ttgtgatcca cccacctcag 9480
   cctcccaaag tgctgggatt acaggcgtga gccaccgagc ctggccctag gctcttatta 9540
   tttagtgagt gaccttgggt atgggaccta acttttatag cctcaaatta ctcatatgta 9600
   aaatggggat gatagaacct gaaatagagt gattttgatg attacatgag atactggacc 9660
   taaaatgcac acggcgaatg catcataaat gttagcaacc tgtcacttac tcaggagatt 9720
   tctggggtaa attgctgttg tattttcact tgttgatcac tactttggaa aggttaaaag 9780
   tgtactttta aaaatctcag ttggcagggt cttaagatga cttgaatcta tcaggttgta 9840
   cctgaagaat ctggagtatc cttcctcatc taattctcca gtttttctcc cctcgaattc 9900
   ctctgtcaga agcacaactg taagaacacc accacttctc tgctcagaaa ttttcaatag 9960
   cagtttggag atgaccttag gcaagtgcac acctcttagc ttcacattca aaaagccttc 10020
   tacaattggg cctctgggtc tatccttcta gatttatcac attccctctg ctcctaccaa 10080
   aggaggtcag ttgcatgctc ttccgaatac atagccgcat tttcccccct gacttcactc 10140
   ccattgttca ctctttctga aatgtgtatc ctcttctatt tttacctccc caaactctac 10200
   tcattcatca aataagacct cattcaacaa ttgcctcctc cacaaagcct tctgttacct 10260
   ttccttgtag aatcatcttt ttcctctaag tgtctagaac atgttgttta cacttcaatt 10320
   atgcaacaat cacaacctgc ctttcttatt aatatgcaaa ctttatgaaa gcagggatca 10380
   ttttttatcc aatttttgtt tgccacagta gttagtacag ttaaatcagt gcttcccaaa 10440
   ccatctttga aaaagaacca gctttgaaaa aaaaaagcaa tctgttgcag attgagatgt 10500
   ttgtaaaaca caataaaaat gaaagattag aaaactaaaa taaaaatacg gggcaaaata 10560
   caagcccaaa tgtctctctc tctt 10584
<210> 40
   <211> 4241
   <212> DNA
   <213> Homo sapiens
<400> 40
   atgtgccacc acacctggct gattttttgt atttttagta gagatgggat ttcaccatat       60
   taaccaagat ggtctcgatt acctgacctc gtgatccgcc cgcctgggcc tcccaaagtg      120
   ctgggactac aggcgtgagc taccaagcct ggccaagtgc aacaagcttc taaagcaaga      180
   atgtggactc cacgttctga tttgcccact tactagttgt gtggcttcgg ttggacttgt      240
   gttagtcatt tttgcttctg ctcctctgag ttctcttatt tttaaaatgt ggataatggt      300
   acctagctca aagggtttct gtgagagtta actgagttaa tcactgtaaa gtacttagaa      360
   cagcatgctc taaagggaga agaagaggtg tggagaaatg gagtagggtt gaagaaactc      420
   ctaaagctcc cttgagttca gaagggagat gaggccagct gggtatagtg tgtgtgtgtg      480
   tgtgtgtgtg tgtgtgtgtg tgtgtgttta ggggaggttg gtgttggtct cagtgttatc      540
   taagtgtgtt gtacaggcaa ggtaactgga gaaaagggca aggaaaggca agtcttaaaa      600
   tttcttagaa agacctttca caagccaggt gcagtggttc atgcctgtaa tcccagctct      660
   cagagaggca gaggcaggag gatagcttga gcccaggagt tcgagacctg cctgggcaat      720
   atagagagac cccgttctcc acaaaaagga aaaaaaaaat gacaaaaaaa aaagaaagac      780
   ctttcaagaa taatttctac caatgagtgg atcaagaaaa tgtggtatgt atgcaccagg      840
   gaatactact cagccataaa aagaatgaaa taatgtcttt tgcgacaacc tggagggagc      900
   tggaggccat tattctaagc gacgcacctc ggaaaaggaa aaccaaatac catatgctct      960
   cacttacaag ggggagctaa gttattagta cgcaaaggca tacagagtga tttaacagac     1020
   tatggagact cacaaggggg aggataggag gggcacaaga gataaaaaac cacatattgg     1080
   gtactacata ttgggtagag tgacaggtgc gctaaaatct caggcatcac tactatacaa     1140
   ttcatctatg taaccaaaaa ccactggtac ccccaaaact attgaaatta aaaaatattt     1200
   ttaaaaaaga actatatcca cctgtggcag agaaaatggg gtatagggtc tggacagaaa     1260
   cagacctgca ttctccagag accaagaaag ctattaagag agtttaattt ggttctatgt     1320
   tagtgtggtg tcaaagcagg gtgaatttgc attaaagaag tgtgaaagtc taaaggcaaa     1380
   gtaggtcatg agacacgaga attaaatagg gagatatgga gactgtttaa tgatttaaac     1440
   ctctatcaag cgaattgaac aaagagagac tgtttctctg tgtttgcatg gtcatggtca     1500
   ttgtgatcac tgctcacgtt ggttgcagca ggctgtcttg ccaatggctg ctatagcagt     1560
   aacaggggac tggtcagcaa atgtgcattg catcaatgac atggagccca tgccctttca     1620
   ctggcttgtt ggtagtggtt gcatggggct ggggaccccc tcttggatct ttattgctga     1680
   cctcacttct ctgtatcaca caagtctcaa gaagatagag aagacaataa tacttaataa     1740
   aatgagaaaa ctgttttatc aatatgttaa gtgaaaaaag cagaatatga aactatgtat 1800
   atgaacctaa ttggttaaac atatgtgtag aaaaatgact gaaataaaac agattaaaac 1860
   agtaaataat agttgtctaa ggatggaaat tacagatgac aatctttcct ttatatttta 1920
   tatcttcttt gctttcattg ataaaaatgt tttgcttttg caattggaaa aggtgaaata 1980
   aatgtaatta acttataaga aatgtaagtt cttccttgtt cagagcttta cacattgctc 2040
   agcaacaaat ggttgtttgc taattggtat gtaaagggca ataaacaagt taattgctgg 2100
   ctacattctc gaatagctag aggaagttca aagcttctca accttatttc agctccaaga 2160
   aagaggacct ctgatgtggg tcaaagacct aatggagtcc tcaaaagccc accatgcgcc 2220
   atagtgggtg gaggctgggc aggagggaga agcttcaaag ccccctcttt ccatgacatg 2280
   cttcttcttc acatcaattt ccatcacttc cctttccctc tctcagctac tgttaaaaga 2340
   tggacttttg tattaggttg ctggggttgc caaagtaaag tgccacagac ttaaatcaca 2400
   gaaatttatt ttctcaaaat tctagggtct agaagtctga gatcgcagtg gcagcagggt 2460
   tggtttcttc tgagacctct ctcctagctg gcagatgccc tttttcctgt gtcttcacat 2520
   ggtcttccct ctctgtgtgt ctgggtccga atctcctctt cttatgaaga tatcagtcat 2580
   attggattag aacccacact aatgacctca tttaacttaa aggctctatc tccaaatata 2640
   ggcatttgga aatactgggg gttagggcca caatgcataa atttttgggg gacacagttt 2700
   agcctataac aggattgaag tgtctccttt cttaaggagc catgtctctg ctcatagaga 2760
   ttcaaaagaa agccaaggtt gggcttagga gaaaataagc aaaagtagag gctgcttaaa 2820
   gtttttgggt ttggtatagc ccaccaggtt aagaaatctg acaggaattt gagtaatctg 2880
   aatccattct gcgtttgccc agagtctcct tctggacaca tagccctctt aaatctctgg 2940
   ctgcattgag gctgcttgag aatttgaaat ctcagtcatc tcctccttgg ggacatggtg 3000
   aatatgtcat tctgttgaac tgatcaccat attatcggat gtttctacaa aagcaaaaca 3060
   gagttgagag cagaaatatt agaatcttag aaagtgacag gggctggagg atgaagaggt 3120
   ggcagggtaa aaggatgggg tactttggtc atattatcct tgataggctg gttgacattc 3180
   tcattctctg ctaagaacaa gcagaagcat cccaggaaac tcttatggga ctttaatgtg 3240
   atgaaatgag agagattatc tctctcctct gcctaaggga tgactatcca tagaatgtac 3300
   tcagacttct ttccaatcac agagataaat cctgaaagtt ttgcagtatg gtactgattt 3360
   ccttgaccta ccagagtcca ggtctataaa gtttgccttt gcccagccag cagatggtgc 3420
   catttttcta ggaaattgag ccagattcag cttgaactgt catttggcct ttagtgttgt 3480
   aggcactgtg ctcacagcat gttgtatagg gcttcacagg taaccagttt ctgttcgagg 3540
   aaagtctagc gctcttacct gcagttgaac acactgaaat cttcaagttt ctcagtttaa 3600
   gtgaaaactt ttatttttaa aatttcggga taattagaag aaaatcttag tatttccttt 3660
   agaaaaatct tcccccaccc ctcaactctc tctttctctc tctctctctt tctcgcatct 3720
   tattccaaca taccaggtgg agccatctca tctttccttt gaaaggccct ctcacaatcc 3780
   ttcacgctta cacatggtgg gtggactaaa ttctatagtg aaattgtagc ttttgaggaa 3840
   tgagtacttc tgaacagctg ctttactttt tgggggacat ctagctgtgt atttatcttt 3900
   taaggtataa atgctttcct tcaaaaaaat atatacatat tctgaaatat ttggaagcca 3960
   gttgcagatg ttatgacaca tcagccctaa atattcagca tataactcct aacaataaag 4020
   actttcttct atgtaactgc taaaccatga ccatatctaa gaaaatcaac agtaatttcc 4080
   aagtattact taatatccat ttcatattca aaatttccta gttatttccc atataccttt 4140
   catagctggt tttattcaac tgcatccaat caaagctcac acattacatt tagttgtttc 4200
   atccattcat atatacatat atatgtgtgt gtgtgtgtgt g 4241
<210> 41
   <211> 601
   <212> DNA
   <213> Homo sapiens
<400> 41
   cctgtgcatc taatttagtg gggggcagac ctgtttcaca agccaaaata acaggctgca 60
   ataactgagg attttatata taccctgacc aaagaagttt gcattctcat agcaccatct 120
   agtggaaatt tggggatttt tgttttacat cgaacagcct aaaatggaga tagtttatgt 180
   tattatgaca gggagagaag actgcacgat ttgctaattg aggtgcaata cagacaagta 240
   aacaaaaaca ggaaaaatgt ggcttatgtg aaagacattt tttaaccata tacaacatgt 300
   tctataaaaa cagagcaaag ccgttagctg aggaacttgt gtgcacaaag aaatatgtac 360
   aaatagcttt tccagaacag tctagtaaaa atatgtagta aaagtgggaa gattatgtta 420
   tatagaaata tatgtttatg tgctgtgttt atatgccaca gcctcaaacc ttcttataac 480
   ttattaacaa ttatatgaaa atataggaac aaagctcatg ttctgtatga atacaacagg 540
   attcaaataa ccacatacat tacaaagagt tgaataaatt gaattctcta ttttctactg 600
   t 601
<210> 42
   <211> 252
   <212> DNA
   <213> Homo sapiens
<400> 42
   attgtggaac tgctctttct ccctgcgatt cagaggggaa aagataaagc cacacagccc    60
   tggggcctct tgcttaagaa cacatctcag tttaaccacc aaggccgtta tgataacaaa   120
   tgtcagtatg gaggtgtgaa catcaggggc tattcaagga cgcttctctg atcactgcca   180
   tgtctatctg ctgtgatcac gactatttta taaacaccct tcctacccaa tgccatcaga   240
   attttaggga cg                                                        252